(19) Europäisches Patentamt
European Patent Office
Office européen des brevets

(11) EP 2 468 873 A1

(12) EUROPEAN PATENT APPLICATION

(43) Date of publication:
**27.06.2012 Bulletin 2012/26**

(51) Int Cl.:
***C12N 15/82*** *(2006.01)*

(21) Application number: **12151921.9**

(22) Date of filing: **13.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **03.01.2007 US 88316807 P**
**15.12.2006 EP 06126279**

(62) Document number(s) of the earlier application(s) in accordance with Art. 76 EPC:
**07857537.0 / 2 069 504**

(27) Previously filed application:
**13.12.2007 EP 07857537**

(71) Applicant: **CropDesign N.V.**
**9052 Gent (BE)**

(72) Inventors:
- **Sanz Molinero, Ana Isabel**
  **28035 Madrid (ES)**
- **Vandesteene, Lies**
  **B-3010 Kessel-Lo (BE)**
- **Ramon, Matthew**
  **B-8500 Kortrijk (BE)**
- **Rolland, Filip**
  **B-3020 Winksele (BE)**
- **Van Dijck, Patrick**
  **B-3271 Zichem (BE)**
- **Thevelein, Johan**
  **B-3052 Oud-Heverlee (Blanden) (BE)**

(74) Representative: **Popp, Andreas**
**BASF SE**
**Global Intellectual Property**
**Carl-Bosch-Str. 38**
**67056 Ludwigshafen (DE)**

Remarks:
This application was filed on 20-01-2012 as a divisional application to the application mentioned under INID code 62.

# (54) Plants having enhanced yield-related traits and a method for making the same

(57) The present invention concerns a method for enhancing yield-related traits in plants, particularly increasing seed yield, by modulating expression in a plant of a nucleic acid encoding a Class III Trehalose Phosphate Phosphatase (TPP) polypeptide. The present invention also concerns plants having modulated expression of a nucleic acid encoding a Class III TPP polypeptide, which plants have enhanced yield-related traits relative to control plants. The invention further concerns novel Class III TPP nucleic acid and polypeptide sequences. The invention also provides nucleic acid and polypeptide sequences, and constructs comprising the same, which are useful in the methods of the invention.

EP 2 468 873 A1

SEQUENCE LISTING

<110> CropDesign N.V.

<120> Plants having enhanced yield-related traits and a method for making the same

<130> PF58635

<150> EP 06126279.6
<151> 2006-12-15

<150> US 60/883,168
<151> 2007-01-03

<160> 131

<170> PatentIn version 3.3

<210> 1
<211> 1125
<212> DNA
<213> Arabidopsis thaliana

<400> 1
```
atgactaacc agaatgtcat cgtttccgac aggaaaccca tcttgggttt gaaaaccatc     60
actgtctctg tctctaactc tcctctgttc tctaattcct ttcccactta ctttaacttc    120
cctcgtcgaa agctcttgaa acttcttgaa gcagccgaca aaaacaactt agttgttgct    180
ccaaagatta cgtctatgat cgattccatg cgtgattctt cccctacacg tctcagatcc    240
tcttcctatg actctgtttc agataacgac gacaaaacat cttggatcgt tcgttttcct    300
tcggctttaa atatgtttga tgagattgtg aatgctgcga aagggaaaca gattgtcgtg    360
tttcttgatt acgacggaac tctttctccc atagttgaag atcccgacaa agctttcata    420
acccatgaga tgagagaagt cgtaaaagac gtggcttcga atttcccgac tgctattgtc    480
accgggagat ccattgagaa ggttcgtagt tttgtccaag taaacgagat ttactacgcc    540
ggaagccacg gcatggacat tgaaggtccg accaacgaaa atagtaacgg ccagagtaat    600
gaaagagtgc tattccaacc tgctcgtgaa tttttaccga tgatcgagaa ggtggttaat    660
attttagagg aaaaaacaaa atggatccct ggggctatgg tggagaacaa caagttttgt    720
ctgtccgtac attttcgacg tgttgatgag aagagatggc ctgcattagc tgaagtagta    780
aaatcagttc ttattgatta tccaaagctg aaactaaccc aaggtagaaa ggtacttgaa    840
atccgcccca caatcaaatg ggacaagggc caggcactca attttttact aaaatcatta    900
ggatatgaaa attcggatga tgtcgtgccg gtgtatatcg gggatgaccg tactgacgaa    960
gatgcgttta aggttttacg tgaaagggga caaggttttg ggatttttgt ctcaaaagta   1020
ccaaaggaca ccaatgcctc ttattctctt caagaccctt ctcaggttaa caagtttctg   1080
gaacgtttag tagaatggaa gaggaagaca gtgggagaag agtga                   1125
```

<210> 2
<211> 374
<212> PRT
<213> Arabidopsis thaliana

<400> 2
```
Met Thr Asn Gln Asn Val Ile Val Ser Asp Arg Lys Pro Ile Leu Gly
1               5                   10                  15
Leu Lys Thr Ile Thr Val Ser Val Ser Asn Ser Pro Leu Phe Ser Asn
            20                  25                  30
Ser Phe Pro Thr Tyr Phe Asn Phe Pro Arg Arg Lys Leu Leu Lys Leu
        35                  40                  45
Leu Glu Ala Ala Asp Lys Asn Asn Leu Val Val Ala Pro Lys Ile Thr
    50                  55                  60
```

```
Ser Met Ile Asp Ser Met Arg Asp Ser Ser Pro Thr Arg Leu Arg Ser
65                  70                  75                  80
Ser Ser Tyr Asp Ser Val Ser Asp Asn Asp Asp Lys Thr Ser Trp Ile
                85                  90                  95
Val Arg Phe Pro Ser Ala Leu Asn Met Phe Asp Glu Ile Val Asn Ala
            100                 105                 110
Ala Lys Gly Lys Gln Ile Val Val Phe Leu Asp Tyr Asp Gly Thr Leu
        115                 120                 125
Ser Pro Ile Val Glu Asp Pro Asp Lys Ala Phe Ile Thr His Glu Met
    130                 135                 140
Arg Glu Val Val Lys Asp Val Ala Ser Asn Phe Pro Thr Ala Ile Val
145                 150                 155                 160
Thr Gly Arg Ser Ile Glu Lys Val Arg Ser Phe Val Gln Val Asn Glu
                165                 170                 175
Ile Tyr Tyr Ala Gly Ser His Gly Met Asp Ile Glu Gly Pro Thr Asn
            180                 185                 190
Glu Asn Ser Asn Gly Gln Ser Asn Glu Arg Val Leu Phe Gln Pro Ala
        195                 200                 205
Arg Glu Phe Leu Pro Met Ile Glu Lys Val Val Asn Ile Leu Glu Glu
    210                 215                 220
Lys Thr Lys Trp Ile Pro Gly Ala Met Val Glu Asn Asn Lys Phe Cys
225                 230                 235                 240
Leu Ser Val His Phe Arg Arg Val Asp Glu Lys Arg Trp Pro Ala Leu
                245                 250                 255
Ala Glu Val Val Lys Ser Val Leu Ile Asp Tyr Pro Lys Leu Lys Leu
            260                 265                 270
Thr Gln Gly Arg Lys Val Leu Glu Ile Arg Pro Thr Ile Lys Trp Asp
        275                 280                 285
Lys Gly Gln Ala Leu Asn Phe Leu Leu Lys Ser Leu Gly Tyr Glu Asn
    290                 295                 300
Ser Asp Asp Val Val Pro Val Tyr Ile Gly Asp Asp Arg Thr Asp Glu
305                 310                 315                 320
Asp Ala Phe Lys Val Leu Arg Glu Arg Gly Gln Gly Phe Gly Ile Phe
                325                 330                 335
Val Ser Lys Val Pro Lys Asp Thr Asn Ala Ser Tyr Ser Leu Gln Asp
            340                 345                 350
Pro Ser Gln Val Asn Lys Phe Leu Glu Arg Leu Val Glu Trp Lys Arg
        355                 360                 365
Lys Thr Val Gly Glu Glu
    370
```

<210> 3
<211> 2027
<212> DNA
<213> Triticum aestivum

<400> 3

```
cccaatccaa ggctctgtca tactaagtga caccttctga acaacggagt tttctcgaaa      60
aggaggttga agcccgggcc tctgcatcat tctgaacatt ggagttaagc ggagctggct     120
ttgcatggct tttcctatgt gccaatccac tcttctgata tagatgtgtc atctctctca     180
gcatgacaag ttcacaggta ctctgctttt gatgaacttc ctccacacct gcagtgacaa     240
gaaaacgctg aagaagtggt tcttcatcga caagacagtg ggctaagaga atccagctga     300
aagagagacg accgccgttc tgaccacatt ttttcctcgt cccacccgat ggatttgagc     360
aacggctcac cggtcatcag tgatccgatg tcaatgagcc agtcgttgat gggagtgctg     420
ccttccaaca tgatgccgtt ttcagtcagg cccgggggtt actccggcgc tggtggcgcc     480
ggcgtaaacg tcagcaggcg caagatcgag gaggtcctcg tgagcgggct gctggatgcc     540
atgaaatcct cgtcgccacg taagaagcac aacgtggtct tcggccagga aaacctgccc     600
gaagaagatc ctgcctacag tgcatggatg gcaaaatgcc cctctgctct ggcttccttc     660
aagcagatcg tagccagtgc acaaggcaag aagattgcgg tcttcctgga ctacgatggc     720
```

acactgtcgc cgatcgtcga cgaccctgaa aaagccgtca tgtcccctgt gatgagagct 780
gctgtgagaa acgtcgccaa atacttcccc gccgccatcg tcagcggaag gtcccggaag 840
aaggttcttg aattcgtaaa actgaaggaa ctctgctatg ctggaagtca tgggatggac 900
ataatgacat cttcttcagc acattatgaa cacaatactg aaaagggcaa agaagccaac 960
ctcttccaac ctgctcgcga ttttctgcct atgatcgatg aggtttccaa ggccctcttg 1020
gaagtcacga gcggaatcga aggcgcgagc gttgaggaca acaagttctg cgtgtctgtt 1080
cattaccgca acgtagacga gaaggactgg gagctggtcg cacggctcgt aaacgaggtg 1140
ctggaggatt tcccccctct caaagtgacc aacgggcgaa tggttttaga ggttcgtccg 1200
gtgatcgact gggacaaggg gaaggccgtc gagttcctgc ttcagtcgct cgggctgagc 1260
gactccgaga acgtgatccc aatctacatc ggcgacgacc gcaccgacga agacgcgttc 1320
aaggtgctcc gggagaggaa ctgcgggtac gggatactgg tctcgcaggc gcccaaggaa 1380
accgaagcct tctactcgct cagggacccg tctgaagtga tggagttcct gaactccttg 1440
gtgagatgga agaagcactc gctatgaaca aacaggagat gactgtagtt tccgaggcga 1500
cagttttgca gcgttggaaa ccatagctag ggtcgaatga tgttgccgtc ccctgttaat 1560
tcgtttaggt cgattgatat tcttgtactg ctagtcatgt tctttgccac cgagaaattt 1620
gatcggtggc tgtgttggtt gttatacata atgctttagg ttaacataaa ttggatctgg 1680
atctcttgat tagaacacca atacatgtgc ggtagcgtgt gaacttacaa gtgattgacg 1740
aaatcccata ctcttgatgc aggccctagt cgatgcagca cacgggaata gttggcgaag 1800
cggaacagca ccgagtgaat agcccatggc cgaaagcgtg aacgtagcca gcaataggta 1860
gaagacgccc agaaggctac cggcactaga gatggccggc atcaaccagc gactggtcgg 1920
tgtagagcag caggaagccg ccggcacatc cttgcggtgg ccggctgtgg tgatggcctt 1980
ctgatccctt tatgcaccct tttaggatcg gtagttagga gtttgtt 2027

<210> 4
<211> 372
<212> PRT
<213> Triticum aestivum

<400> 4
Met Asp Leu Ser Asn Gly Ser Pro Val Ile Ser Asp Pro Met Ser Met
1 5 10 15
Ser Gln Ser Leu Met Gly Val Leu Pro Ser Asn Met Met Pro Phe Ser
20 25 30
Val Arg Pro Gly Gly Tyr Ser Gly Ala Gly Gly Ala Gly Val Asn Val
35 40 45
Ser Arg Arg Lys Ile Glu Glu Val Leu Val Ser Gly Leu Leu Asp Ala
50 55 60
Met Lys Ser Ser Ser Pro Arg Lys Lys His Asn Val Val Phe Gly Gln
65 70 75 80
Glu Asn Leu Pro Glu Glu Asp Pro Ala Tyr Ser Ala Trp Met Ala Lys
85 90 95
Cys Pro Ser Ala Leu Ala Ser Phe Lys Gln Ile Val Ala Ser Ala Gln
100 105 110
Gly Lys Lys Ile Ala Val Phe Leu Asp Tyr Asp Gly Thr Leu Ser Pro
115 120 125
Ile Val Asp Asp Pro Glu Lys Ala Val Met Ser Pro Val Met Arg Ala
130 135 140
Ala Val Arg Asn Val Ala Lys Tyr Phe Pro Ala Ala Ile Val Ser Gly
145 150 155 160
Arg Ser Arg Lys Lys Val Leu Glu Phe Val Lys Leu Lys Glu Leu Cys
165 170 175
Tyr Ala Gly Ser His Gly Met Asp Ile Met Thr Ser Ser Ser Ala His
180 185 190
Tyr Glu His Asn Thr Glu Lys Gly Lys Glu Ala Asn Leu Phe Gln Pro
195 200 205
Ala Arg Asp Phe Leu Pro Met Ile Asp Glu Val Ser Lys Ala Leu Leu
210 215 220
Glu Val Thr Ser Gly Ile Glu Gly Ala Ser Val Glu Asp Asn Lys Phe
225 230 235 240

Cys Val Ser Val His Tyr Arg Asn Val Asp Glu Lys Asp Trp Glu Leu
245 250 255
Val Ala Arg Leu Val Asn Glu Val Leu Glu Asp Phe Pro Pro Leu Lys
260 265 270
Val Thr Asn Gly Arg Met Val Leu Glu Val Arg Pro Val Ile Asp Trp
275 280 285
Asp Lys Gly Lys Ala Val Glu Phe Leu Leu Gln Ser Leu Gly Leu Ser
290 295 300
Asp Ser Glu Asn Val Ile Pro Ile Tyr Ile Gly Asp Asp Arg Thr Asp
305 310 315 320
Glu Asp Ala Phe Lys Val Leu Arg Glu Arg Asn Cys Gly Tyr Gly Ile
325 330 335
Leu Val Ser Gln Ala Pro Lys Glu Thr Glu Ala Phe Tyr Ser Leu Arg
340 345 350
Asp Pro Ser Glu Val Met Glu Phe Leu Asn Ser Leu Val Arg Trp Lys
355 360 365
Lys His Ser Leu
370

<210> 5
<211> 2002
<212> DNA
<213> Glycine max

<400> 5
actatttttt tcaggagaat attattgcgc ttttcgtgtt tctccccgaa agcaccaata 60
acgcattgcc ttccctatgg tcgctgtgtt aaagtttggc tgcccataat attaccccat 120
ttttagcttc tgccaaacaa caacttgtgc cttttttttt gtctcctttc ttatttgttt 180
ctttttagcc tttttgtttt tttattggcc gccaaactaa aaacaatatc agtatttgcg 240
tcttttaatc tggtattcct ctcttctgac tggtggcttc gttttcgcct ttgaagatat 300
ttagacgaaa acgagaatcc gcattctgat tctgaagatt catgtctaat ctcatttctt 360
tcttcccttg ggttgagctc gtttttagg agctcggcag ttgatggact gttaccccaa 420
gagcagtgac aagaaaacat taaagaggtg gtttttatt gataaaagag ttgggtgaag 480
tggaaatccg ataagtatta tttcaattgg tgctcaatac tccttaaagt tcttacattg 540
ttgctggact gtaaattgat ttaaaatctg gatcccatgg acttgaagtc gaatcatact 600
cctgttctca ctgatgctgc acctgtaaca aagtctagac tgggtgtgca ttccagtttg 660
ttgccttact cccatgctgg gacaaccttt acacatggaa tgttattgac tatcccgagg 720
aagaagacag gaattcttga agatgttcgt tctagtggtt ggttggatgc catgaaatca 780
tcttcacctc cagccaggaa gataacaaag gatgttggcc atgggtttgc atcatctgat 840
tctgaaactg ctggtgctta ttttagctgg ctgctaaaat acccatccgc acttgcatct 900
tttgatcaaa tcatgaacta tgcaaaaggg aaaagaattg cgctgtttat ggattatgat 960
ggaactcttt caccaattgt ggataatcct gactgtgctt tcatgtccga caatatgcgt 1020
gctgctgtta aaaaggtggc agaatatttt ccaacagcaa taattagtgg aagaagccgt 1080
gataaggtat atcaatttgt aggactaaca gaactctatt atgctggtag tcatggaatg 1140
gacatcattg ggcctgttag acaatctgta tctgataatc acccaaattg cattaggtct 1200
acagacaagc agggtaagga agtaaattta ttccaacctg ctgctgaatt tctgcccatg 1260
attaatgagg tacttaattc tcttgaagag tgtacgaaag acattaaagg agctaaagtt 1320
gagaacaata aattttgtgt atctgtgcac taccggaatg tagatgaaaa gtattggaat 1380
tgggtggggc aacatgttca tgatgttctg aagggctatc cacgtttgcg cttaactcat 1440
gggcggaagg ttttagagat ccgacctgtg attaactggg ataagggcaa agctgtcacg 1500
tttctacttg agtcacttgg gctaaacaat tgtgatgatg tgcttcctat atatattgga 1560
gatgatcgga cagacgaaga tgcatttaag gttttgagag agggaaataa aggttatggg 1620
atcttggtgt cttcagcacc aaaagaaagc aacgcaattt actctcttcg tgatccatca 1680
gaggtcatgg aatttctcaa gtcacttgtg ttgtggaaat caagcacctt aaaaagcctt 1740
atatagtgta tagaggagag aatatactct gctatactat actatatcat aaaaaaaaaa 1800
aacagctttt ttcgattttg gtttttctca gaaacattcc aagaggttta ctggagcaga 1860
tttacgcaca gaagaacctc ttttggtaac aacagcgttg tcgctaccca gtttttttgc 1920
tcgaagctct tgtggtagct gtaaattcgt caactcataa ttataaatat cttaaagaag 1980
taaaaaaaaa aaaaaaaaag cg 2002

<210> 6
<211> 389
<212> PRT
<213> Glycine max

<400> 6
Met Asp Leu Lys Ser Asn His Thr Pro Val Leu Thr Asp Ala Ala Pro
1 5 10 15
Val Thr Lys Ser Arg Leu Gly Val His Ser Ser Leu Leu Pro Tyr Ser
20 25 30
His Ala Gly Thr Thr Phe Thr His Gly Met Leu Leu Thr Ile Pro Arg
35 40 45
Lys Lys Thr Gly Ile Leu Glu Asp Val Arg Ser Ser Gly Trp Leu Asp
50 55 60
Ala Met Lys Ser Ser Ser Pro Pro Ala Arg Lys Ile Thr Lys Asp Val
65 70 75 80
Gly His Gly Phe Ala Ser Ser Asp Ser Glu Thr Ala Gly Ala Tyr Phe
85 90 95
Ser Trp Leu Leu Lys Tyr Pro Ser Ala Leu Ala Ser Phe Asp Gln Ile
100 105 110
Met Asn Tyr Ala Lys Gly Lys Arg Ile Ala Leu Phe Met Asp Tyr Asp
115 120 125
Gly Thr Leu Ser Pro Ile Val Asp Asn Pro Asp Cys Ala Phe Met Ser
130 135 140
Asp Asn Met Arg Ala Ala Val Lys Lys Val Ala Glu Tyr Phe Pro Thr
145 150 155 160
Ala Ile Ile Ser Gly Arg Ser Arg Asp Lys Val Tyr Gln Phe Val Gly
165 170 175
Leu Thr Glu Leu Tyr Tyr Ala Gly Ser His Gly Met Asp Ile Ile Gly
180 185 190
Pro Val Arg Gln Ser Val Ser Asp Asn His Pro Asn Cys Ile Arg Ser
195 200 205
Thr Asp Lys Gln Gly Lys Glu Val Asn Leu Phe Gln Pro Ala Ala Glu
210 215 220
Phe Leu Pro Met Ile Asn Glu Val Leu Asn Ser Leu Glu Glu Cys Thr
225 230 235 240
Lys Asp Ile Lys Gly Ala Lys Val Glu Asn Asn Lys Phe Cys Val Ser
245 250 255
Val His Tyr Arg Asn Val Asp Glu Lys Tyr Trp Asn Trp Val Gly Gln
260 265 270
His Val His Asp Val Leu Lys Gly Tyr Pro Arg Leu Arg Leu Thr His
275 280 285
Gly Arg Lys Val Leu Glu Ile Arg Pro Val Ile Asn Trp Asp Lys Gly
290 295 300
Lys Ala Val Thr Phe Leu Leu Glu Ser Leu Gly Leu Asn Asn Cys Asp
305 310 315 320
Asp Val Leu Pro Ile Tyr Ile Gly Asp Asp Arg Thr Asp Glu Asp Ala
325 330 335
Phe Lys Val Leu Arg Glu Gly Asn Lys Gly Tyr Gly Ile Leu Val Ser
340 345 350
Ser Ala Pro Lys Glu Ser Asn Ala Ile Tyr Ser Leu Arg Asp Pro Ser
355 360 365
Glu Val Met Glu Phe Leu Lys Ser Leu Val Leu Trp Lys Ser Ser Thr
370 375 380
Leu Lys Ser Leu Ile
385

<210> 7

<211> 1402
<212> DNA
<213> Tagetes spp.

<400> 7
cgaccattgg caaaccgaga ttaggaattc atgattcatt actaccatac gcatcttctg 60
gagtcttctc tcaagctcca tttcttaaaa aacctggact acttgatgat gttcgttcta 120
ctagctggtt ggatgccatg aaatcttctt ctccaaggaa tgttctaaaa aataacgcca 180
ctgatttgtc aaatgatgtt gcttaccgca attggatgat taagtatccg tctgcacttg 240
catccattga acagattgca aataatgcaa agggaaagag gattgccttg tttctagatt 300
atgacggaac attatccccg atcgtggaca atccagatca tgccttcatg tctaatgcta 360
tgcgtgctgc agtaagaaat gtggcaaaat acattccaac agcaattatt agtggcagaa 420
gctgtgaaaa ggtacacaag tttgtaggac tgaaagaact atattatgct ggtagtcatg 480
ggatggacat aatgggtcct gttcagcctc caactgatca tagaattgag gctatagaag 540
gaaatttgta ccagcctgct agtgaatttc tacccatgat caacgaggtt tttgtgtccc 600
ttgttgagat aaccaaggac atagaaggag caaaagtcga agacaacaag ttttgtgtct 660
cagtgcacta tcgtaatgta gatgagaaga actggacaat ggtagcacaa tgtgtcgaag 720
acactttgaa aaactatcca cgtctgagat tgactcacgg gcggatggtt ttagagatcc 780
gccctgtgct taagtgggac aaggggaagg ctgttgagtt cttactcgaa tcacttggct 840
taagtaattg tgatgatgtg ctccctatat atgtagggga tgacagaaca gacgaagatg 900
cattcaagtt tttaagagag ggtagtcttg gttatggcat cttggtgact cctgccccaa 960
aggaaagcag tgcatattat tctctcaggg atccatccga ggtgatggaa tttatcaact 1020
tactagtgat gcataagaag tcggttccat gattcgactg attcgtacaa taggaagacg 1080
ttctgaaatt ttcatttaaa agaataaaac aaggcatctg aagtagaaat gagcacttct 1140
tcaacctccc tacctggcaa ttgtaaatcg cacgatatta ctttcttcat gtttgacata 1200
cgggaaagat atatgtgtat atacaatttc ttattattga atcccatgta cgtacatatc 1260
cggtcggttt tgttaataca aagatccggt attgttagta taaagttaga cgaaaatcaa 1320
gttgattgat gtaaaatttt ctttgtatga atgttcggta ctttactttt aatgtttcta 1380
tgaaattttg atattgtgtt tt 1402

<210> 8
<211> 304
<212> PRT
<213> Tagetes spp.

<400> 8
Met Lys Ser Ser Ser Pro Arg Asn Val Leu Lys Asn Asn Ala Thr Asp
1 5 10 15
Leu Ser Asn Asp Val Ala Tyr Arg Asn Trp Met Ile Lys Tyr Pro Ser
20 25 30
Ala Leu Ala Ser Ile Glu Gln Ile Ala Asn Asn Ala Lys Gly Lys Arg
35 40 45
Ile Ala Leu Phe Leu Asp Tyr Asp Gly Thr Leu Ser Pro Ile Val Asp
50 55 60
Asn Pro Asp His Ala Phe Met Ser Asn Ala Met Arg Ala Ala Val Arg
65 70 75 80
Asn Val Ala Lys Tyr Ile Pro Thr Ala Ile Ile Ser Gly Arg Ser Cys
85 90 95
Glu Lys Val His Lys Phe Val Gly Leu Lys Glu Leu Tyr Tyr Ala Gly
100 105 110
Ser His Gly Met Asp Ile Met Gly Pro Val Gln Pro Pro Thr Asp His
115 120 125
Arg Ile Glu Ala Ile Glu Gly Asn Leu Tyr Gln Pro Ala Ser Glu Phe
130 135 140
Leu Pro Met Ile Asn Glu Val Phe Val Ser Leu Val Glu Ile Thr Lys
145 150 155 160
Asp Ile Glu Gly Ala Lys Val Glu Asp Asn Lys Phe Cys Val Ser Val
165 170 175
His Tyr Arg Asn Val Asp Glu Lys Asn Trp Thr Met Val Ala Gln Cys

```
            180                 185                 190
Val Glu Asp Thr Leu Lys Asn Tyr Pro Arg Leu Arg Leu Thr His Gly
        195                 200                 205
Arg Met Val Leu Glu Ile Arg Pro Val Leu Lys Trp Asp Lys Gly Lys
    210                 215                 220
Ala Val Glu Phe Leu Leu Glu Ser Leu Gly Leu Ser Asn Cys Asp Asp
225                 230                 235                 240
Val Leu Pro Ile Tyr Val Gly Asp Asp Arg Thr Asp Glu Asp Ala Phe
                245                 250                 255
Lys Phe Leu Arg Glu Gly Ser Leu Gly Tyr Gly Ile Leu Val Thr Pro
            260                 265                 270
Ala Pro Lys Glu Ser Ser Ala Tyr Tyr Ser Leu Arg Asp Pro Ser Glu
        275                 280                 285
Val Met Glu Phe Ile Asn Leu Leu Val Met His Lys Lys Ser Val Pro
    290                 295                 300
```

<210> 9
<211> 1207
<212> DNA
<213> Arabidopsis thaliana

<400> 9

```
atgaagatta cggatatttc cggaaagatc gagactttgg ttgattcctt aagggatatg      60
tccccgaccc gtgtcagatc ttccttctct gatgaacatg tatccgagaa cgatgacgag     120
agaagctcct ggattgctct tcatccgtca gcattggata tgttcgaaca aatcatgcgt     180
gatgctgaag gcaaacaaat tattatgttt cttgattacg acggaactct ctcgctaatc     240
actgaagatc acgacagagc ctacataacc gacgagatgc gagaagttgt aaaggaagtg     300
gctacatatt tcaagacagc gatcatcagc ggacgaagca ctgacaaagt acagagtttt     360
gtgaaactca ctggtattca ctacgctggg agccacggca tggacattaa gggtccgaca     420
aataccgatc agagtaacca agaagaagtg atgtttcaac ctgcaagtga ctatttaccg     480
atgattgacg aggtggttaa tgttttgaag gaaaagacaa aatctatccc tggagctacg     540
gtcgagcaca acaagttttg tctaacagta cattttcgtc gggtcgatga aacgggatgg     600
gctgcattag ctgagcaagt gagattggtt ctcattgatt atccgaaatt gagactgaca     660
caaggcagaa aggtcttaga actccgacct tccatcaaat gggacaaggg aaaggctctc     720
gaatttttgc taaactcatt agggatagca gaatctaaag atgttttacc ggtttacatt     780
ggagatgacc gtactgatga agatgcattt aaggttttat gtgaaagggg acaaggtttt     840
gggattatcg tctcaaaaac tattaaggaa acgtacgcct cttactctct tcaagaccca     900
tctcaggtta aagaatttct ggagcgtttg gtaaagtgga agaaacaaac acttggagaa     960
taagaagaag aactggttca taagagatat tgagtttcgt gttcttaatt cttataaccc    1020
gtcctgaaat atatgggact cttagcaaaa taaaaatgga gaccttaatt tttagatttt    1080
aaatttattg tttcaatgaa taatactgaa aagattaatt tttcgttaaa atggtcttgt    1140
aagatattct aatgattttt ttttatatga attaagaagc atgtttcgtt gaatttatag    1200
gttaaca                                                               1207
```

<210> 10
<211> 320
<212> PRT
<213> Arabidopsis thaliana

<400> 10

```
Met Lys Ile Thr Asp Ile Ser Gly Lys Ile Glu Thr Leu Val Asp Ser
1               5                   10                  15
Leu Arg Asp Met Ser Pro Thr Arg Val Arg Ser Ser Phe Ser Asp Glu
            20                  25                  30
His Val Ser Glu Asn Asp Asp Glu Arg Ser Ser Trp Ile Ala Leu His
        35                  40                  45
Pro Ser Ala Leu Asp Met Phe Glu Gln Ile Met Arg Asp Ala Glu Gly
    50                  55                  60
Lys Gln Ile Ile Met Phe Leu Asp Tyr Asp Gly Thr Leu Ser Leu Ile
```

65 70 75 80
Thr Glu Asp His Asp Arg Ala Tyr Ile Thr Asp Glu Met Arg Glu Val
85 90 95
Val Lys Glu Val Ala Thr Tyr Phe Lys Thr Ala Ile Ile Ser Gly Arg
100 105 110
Ser Thr Asp Lys Val Gln Ser Phe Val Lys Leu Thr Gly Ile His Tyr
115 120 125
Ala Gly Ser His Gly Met Asp Ile Lys Gly Pro Thr Asn Thr Asp Gln
130 135 140
Ser Asn Gln Glu Glu Val Met Phe Gln Pro Ala Ser Asp Tyr Leu Pro
145 150 155 160
Met Ile Asp Glu Val Val Asn Val Leu Lys Glu Lys Thr Lys Ser Ile
165 170 175
Pro Gly Ala Thr Val Glu His Asn Lys Phe Cys Leu Thr Val His Phe
180 185 190
Arg Arg Val Asp Glu Thr Gly Trp Ala Ala Leu Ala Glu Gln Val Arg
195 200 205
Leu Val Leu Ile Asp Tyr Pro Lys Leu Arg Leu Thr Gln Gly Arg Lys
210 215 220
Val Leu Glu Leu Arg Pro Ser Ile Lys Trp Asp Lys Gly Lys Ala Leu
225 230 235 240
Glu Phe Leu Leu Asn Ser Leu Gly Ile Ala Glu Ser Lys Asp Val Leu
245 250 255
Pro Val Tyr Ile Gly Asp Asp Arg Thr Asp Glu Asp Ala Phe Lys Val
260 265 270
Leu Cys Glu Arg Gly Gln Gly Phe Gly Ile Ile Val Ser Lys Thr Ile
275 280 285
Lys Glu Thr Tyr Ala Ser Tyr Ser Leu Gln Asp Pro Ser Gln Val Lys
290 295 300
Glu Phe Leu Glu Arg Leu Val Lys Trp Lys Lys Gln Thr Leu Gly Glu
305 310 315 320

<210> 11
<211> 1396
<212> DNA
<213> Arabidopsis thaliana

<400> 11
atgtattaag acttgacaac ttgtctttct cacaccaaac ccctctcctc tgtttcataa 60
catctgctct ttcttttttt tcctaagccc ctaatgacaa accataatgc cttaatctct 120
gatgctaaag gcagcatcgg agttgcggtt agagttccaa accaatctct gttttctccc 180
ggaggtggcc gatacatcag cattccccgg aagaaactcg tgcagaagct agaggccgac 240
ccgagtcaaa cccgtatcca cacttggatc gaagccatga gggcttcttc cccaacccgt 300
acccgaccgg ggaacatatc tcccctcccg gagtccgatg aggaggatga atactcttct 360
tggatggctc aacacccgtc agctttaacc atgtttgaag agatagctga agcttcaaaa 420
gggaaacaaa tcgtgatgtt tctcgactat gacggtacat tatcccccat tgttgaaaac 480
cctgatcgag cttacatgtc tgaagagatg agagaggcag tgaaaggcgt ggctagatat 540
ttcccgaccg cgattgtcac tggaagatgc cgtgataagg ttcgtagatt tgtgaaactt 600
cccggacttt actatgcagg tagccatgga atggacatca aaggaccttc caaaagaaac 660
aaacataaca agaacaataa aggagttctt ttccaagcgg cgaatgagtt tttgcctatg 720
attgacaagg tctctaagtg tctagtagag aaaatgagag acatagaagg agcaaacgtc 780
gagaacaaca agttttgtgt ctccgtacat taccgttgtg ttgatcaaaa ggactgggga 840
ttggtagcgg aacacgtgac atcgatattg agtgagtatc cgaaactgag gttgacacaa 900
ggaagaaaag tcttagagat tcgaccaacc atcaaatggg ataaaggcaa agctctcgag 960
ttcttgctcg aatccttagg attcgctaac tctaacgatg ttttgcccat ctatatagga 1020
gatgatcgta cggacgagga tgctttcaag gttttgagaa acaaaggaca aggctttggt 1080
atacttgtgt ccaaaattcc aaaggaaacg agtgctacat attctctaca agaaccttcc 1140
gaggtaggag agtttttgca gcgactcgtg gaatggaaac aaatgtcact aagaggaaga 1200
tagccaattt cctgacataa atttattttc aattaataaa tgaattagtt ttcactatgc 1260

aacaaaaatt gttgtatata tgatcaatgt ttttttaatt attttactct tcatgaacaa 1320
atgtaagttt ataggaactt tcttaaccaa gaaaaaagta agtttgctag tataatattt 1380
tcatcattct cttttt 1396

<210> 12
<211> 369
<212> PRT
<213> Arabidopsis thaliana

<400> 12
Met Thr Asn His Asn Ala Leu Ile Ser Asp Ala Lys Gly Ser Ile Gly
1 5 10 15
Val Ala Val Arg Val Pro Asn Gln Ser Leu Phe Ser Pro Gly Gly Gly
20 25 30
Arg Tyr Ile Ser Ile Pro Arg Lys Lys Leu Val Gln Lys Leu Glu Ala
35 40 45
Asp Pro Ser Gln Thr Arg Ile His Thr Trp Ile Glu Ala Met Arg Ala
50 55 60
Ser Ser Pro Thr Arg Thr Arg Pro Gly Asn Ile Ser Pro Leu Pro Glu
65 70 75 80
Ser Asp Glu Glu Asp Glu Tyr Ser Ser Trp Met Ala Gln His Pro Ser
85 90 95
Ala Leu Thr Met Phe Glu Glu Ile Ala Glu Ala Ser Lys Gly Lys Gln
100 105 110
Ile Val Met Phe Leu Asp Tyr Asp Gly Thr Leu Ser Pro Ile Val Glu
115 120 125
Asn Pro Asp Arg Ala Tyr Met Ser Glu Glu Met Arg Glu Ala Val Lys
130 135 140
Gly Val Ala Arg Tyr Phe Pro Thr Ala Ile Val Thr Gly Arg Cys Arg
145 150 155 160
Asp Lys Val Arg Arg Phe Val Lys Leu Pro Gly Leu Tyr Tyr Ala Gly
165 170 175
Ser His Gly Met Asp Ile Lys Gly Pro Ser Lys Arg Asn Lys His Asn
180 185 190
Lys Asn Asn Lys Gly Val Leu Phe Gln Ala Ala Asn Glu Phe Leu Pro
195 200 205
Met Ile Asp Lys Val Ser Lys Cys Leu Val Glu Lys Met Arg Asp Ile
210 215 220
Glu Gly Ala Asn Val Glu Asn Asn Lys Phe Cys Val Ser Val His Tyr
225 230 235 240
Arg Cys Val Asp Gln Lys Asp Trp Gly Leu Val Ala Glu His Val Thr
245 250 255
Ser Ile Leu Ser Glu Tyr Pro Lys Leu Arg Leu Thr Gln Gly Arg Lys
260 265 270
Val Leu Glu Ile Arg Pro Thr Ile Lys Trp Asp Lys Gly Lys Ala Leu
275 280 285
Glu Phe Leu Leu Glu Ser Leu Gly Phe Ala Asn Ser Asn Asp Val Leu
290 295 300
Pro Ile Tyr Ile Gly Asp Asp Arg Thr Asp Glu Asp Ala Phe Lys Val
305 310 315 320
Leu Arg Asn Lys Gly Gln Gly Phe Gly Ile Leu Val Ser Lys Ile Pro
325 330 335
Lys Glu Thr Ser Ala Thr Tyr Ser Leu Gln Glu Pro Ser Glu Val Gly
340 345 350
Glu Phe Leu Gln Arg Leu Val Glu Trp Lys Gln Met Ser Leu Arg Gly
355 360 365
Arg

<210> 13
<211> 1398
<212> DNA
<213> Arabidopsis thaliana

<400> 13
ctcctcacct tctctctctc aacatctctc tctttctctg tcttctctct gtctcaacga 60
gaatatgact aaccagaatg tcatcgtttc cgacaggaaa cccatcttgg gtttgaaaac 120
catcactgtc tctgtctcta actctcctct gttctctaat tcctttccca cttactttaa 180
cttccctcgt cgaaagctct tgaaacttct tgaagcagcc gacaaaaaca acttagttgt 240
tgctccaaag attacgtcta tgatcgattc catgcgtgat tcttccccta cacgtctcag 300
atcctcttcc tatgactctg tttcagataa cgacgacaaa acatcttgga tcgttcgttt 360
tccttcggct ttaaatatgt ttgatgagat tgtgaatgct gcgaaaggga aacagattgt 420
catgtttctt gattacgacg gaactctttc tcccatagtt gaagatcccg acaaagcttt 480
cataacccat gagatgagag aagtcgtaaa agacgtggct tcgaatttcc cgactgctat 540
tgtcaccggg agatccattg agaaggttcg tagttttgtc caagtaaacg agatttacta 600
cgccggaagc cacggcatgg acattgaagg tccgaccaac gaaaatagta acggccagag 660
taatgaaaga gtgctattcc aacctgctcg tgaattttta ccgatgatcg agaaggtggt 720
taatatttta gaggaaaaaa caaaatggat ccctggggct atggtggaga acaacaagtt 780
ttgtctgtcc gtacattttc gacgtgttga tgagaagaga tggcctgcat tagctgaagt 840
agtaaaatca gttcttattg attatccaaa gctgaaacta acccaaggta gaaaggtact 900
tgaaatccgc cccacaatca aatgggacaa gggccaggca ctcaattttt tactaaaatc 960
attaggatat gaaaattcgg atgatgttgt gccggtgtat atcgggggatg accgtactga 1020
cgaagatgcg tttaaggttt tacgtgaaag gggacaaggt tttgggattc ttgtctcaaa 1080
agtaccaaag gacaccaatg cctcttattc tcttcaagac ccttctcagg ttaacaagtt 1140
tctggaacgt ttagtagaat ggaagaggaa gacagtggga gaagagtgaa agacatgcaa 1200
cataattaca caaataacac ctgagttttg agtagattat taatttatat aaacaccctt 1260
tttaaattag tagatgcctg gagggtgttt cggccagaag tttttctttt aatctacatc 1320
gaaccgaaaa ttgtaaattt cgtgtaacga tttagaaatg gaaaaatata ttaataattt 1380
actcattctt atttgacc 1398

<210> 14
<211> 374
<212> PRT
<213> Arabidopsis thaliana

<400> 14
Met Thr Asn Gln Asn Val Ile Val Ser Asp Arg Lys Pro Ile Leu Gly
1 5 10 15
Leu Lys Thr Ile Thr Val Ser Val Ser Asn Ser Pro Leu Phe Ser Asn
20 25 30
Ser Phe Pro Thr Tyr Phe Asn Phe Pro Arg Arg Lys Leu Leu Lys Leu
35 40 45
Leu Glu Ala Ala Asp Lys Asn Asn Leu Val Val Ala Pro Lys Ile Thr
50 55 60
Ser Met Ile Asp Ser Met Arg Asp Ser Ser Pro Thr Arg Leu Arg Ser
65 70 75 80
Ser Ser Tyr Asp Ser Val Ser Asp Asn Asp Asp Lys Thr Ser Trp Ile
85 90 95
Val Arg Phe Pro Ser Ala Leu Asn Met Phe Asp Glu Ile Val Asn Ala
100 105 110
Ala Lys Gly Lys Gln Ile Val Met Phe Leu Asp Tyr Asp Gly Thr Leu
115 120 125
Ser Pro Ile Val Glu Asp Pro Asp Lys Ala Phe Ile Thr His Glu Met
130 135 140
Arg Glu Val Val Lys Asp Val Ala Ser Asn Phe Pro Thr Ala Ile Val
145 150 155 160
Thr Gly Arg Ser Ile Glu Lys Val Arg Ser Phe Val Gln Val Asn Glu
165 170 175

```
Ile Tyr Tyr Ala Gly Ser His Gly Met Asp Ile Glu Gly Pro Thr Asn
            180                 185                 190
Glu Asn Ser Asn Gly Gln Ser Asn Glu Arg Val Leu Phe Gln Pro Ala
        195                 200                 205
Arg Glu Phe Leu Pro Met Ile Glu Lys Val Val Asn Ile Leu Glu Glu
    210                 215                 220
Lys Thr Lys Trp Ile Pro Gly Ala Met Val Glu Asn Asn Lys Phe Cys
225                 230                 235                 240
Leu Ser Val His Phe Arg Arg Val Asp Glu Lys Arg Trp Pro Ala Leu
                245                 250                 255
Ala Glu Val Val Lys Ser Val Leu Ile Asp Tyr Pro Lys Leu Lys Leu
            260                 265                 270
Thr Gln Gly Arg Lys Val Leu Glu Ile Arg Pro Thr Ile Lys Trp Asp
        275                 280                 285
Lys Gly Gln Ala Leu Asn Phe Leu Leu Lys Ser Leu Gly Tyr Glu Asn
    290                 295                 300
Ser Asp Asp Val Val Pro Val Tyr Ile Gly Asp Asp Arg Thr Asp Glu
305                 310                 315                 320
Asp Ala Phe Lys Val Leu Arg Glu Arg Gly Gln Gly Phe Gly Ile Leu
                325                 330                 335
Val Ser Lys Val Pro Lys Asp Thr Asn Ala Ser Tyr Ser Leu Gln Asp
            340                 345                 350
Pro Ser Gln Val Asn Lys Phe Leu Glu Arg Leu Val Glu Trp Lys Arg
        355                 360                 365
Lys Thr Val Gly Glu Glu
    370
```

<210> 15
<211> 1259
<212> DNA
<213> Arabidopsis thaliana

<400> 15

```
acattcactc tcatttccat caaaattata acttcatttt acattttgat taatatccta     60
aaatggttag attcatcgaa gaaaatatta ctaaaatgtt ggagacaaag gccatctcaa    120
actcggaggt tttatatgtc ggaggagacg acggagacac gtcaccaacg acgaaagttc    180
ttcatgattt tcagatcaac agcggaggag gactgataag atcatgggtt gattctatga    240
gagcttgttc tcctactcgc cctaaatcct tcaatagcca atcttgttgg attaaggaac    300
atccatctgc tttgaacatg ttcgaagaaa tacttcataa atctgaagga aaacaaatag    360
ttatgtttct tgattatgat ggtactctct ctcccattgt tgatgatcct gatcgagctt    420
tcatgtccaa gaagatgcga aatactgtga ggaaacttgc aaagtgtttt ccaacagcca    480
tagttagtgg gagatgcaga gagaaggttt ctagttttgt gaaattaact gagttatact    540
acgctggaag tcatggcatg gacatcaaag gaccagagca aggatctaaa tacaagaaag    600
aaaatcagtc tcttctttgt caacccgcaa ctgaattcct cccggtgata aacgaggttt    660
ataaaaaact agtcgagaac actcaatcga ttccaggagc gaaagttgag aacaacaaat    720
tttgtgcgtc tgttcacttt cgatgcgtag aagaaaataa atggagtgac ttggcccatc    780
aagttcgatc agtcttgaag aattacccca agctcatgct tacccaagga agaaaagtat    840
tggagattcg tccaatcatt aagtgggata aaggcaaagc actcgagttt ttgttagaat    900
cactaggata tgataattgt accgatgttt ttcctatata tattggagat gatcgtaccg    960
acgaagacgc ctttaagata ctgagagaca aaaaacaagg tcttggaatt cttgtgtcca   1020
aatacgcaaa ggagactaat gcttcttatt ctttgcaaga gccagatgag gttatggttt   1080
ttttagaacg tttggtggaa tggaaacagt caaggtgtgg ggcatgaaga gtttacaagt   1140
acaggagaaa cttgttcttt ttctttttttc tttttactat gtagtattgg taaagattct   1200
aacgtataag agaacgagaa taagtttatt aatataacaa gaagacaaat attgaggtt    1259
```

<210> 16
<211> 354
<212> PRT
<213> Arabidopsis thaliana

<400> 16

Met Val Arg Phe Ile Glu Glu Asn Ile Thr Lys Met Leu Glu Thr Lys
1 5 10 15
Ala Ile Ser Asn Ser Glu Val Leu Tyr Val Gly Gly Asp Asp Gly Asp
20 25 30
Thr Ser Pro Thr Thr Lys Val Leu His Asp Phe Gln Ile Asn Ser Gly
35 40 45
Gly Gly Leu Ile Arg Ser Trp Val Asp Ser Met Arg Ala Cys Ser Pro
50 55 60
Thr Arg Pro Lys Ser Phe Asn Ser Gln Ser Cys Trp Ile Lys Glu His
65 70 75 80
Pro Ser Ala Leu Asn Met Phe Glu Glu Ile Leu His Lys Ser Glu Gly
85 90 95
Lys Gln Ile Val Met Phe Leu Asp Tyr Asp Gly Thr Leu Ser Pro Ile
100 105 110
Val Asp Asp Pro Asp Arg Ala Phe Met Ser Lys Lys Met Arg Asn Thr
115 120 125
Val Arg Lys Leu Ala Lys Cys Phe Pro Thr Ala Ile Val Ser Gly Arg
130 135 140
Cys Arg Glu Lys Val Ser Ser Phe Val Lys Leu Thr Glu Leu Tyr Tyr
145 150 155 160
Ala Gly Ser His Gly Met Asp Ile Lys Gly Pro Glu Gln Gly Ser Lys
165 170 175
Tyr Lys Lys Glu Asn Gln Ser Leu Leu Cys Gln Pro Ala Thr Glu Phe
180 185 190
Leu Pro Val Ile Asn Glu Val Tyr Lys Lys Leu Val Glu Asn Thr Gln
195 200 205
Ser Ile Pro Gly Ala Lys Val Glu Asn Asn Lys Phe Cys Ala Ser Val
210 215 220
His Phe Arg Cys Val Glu Glu Asn Lys Trp Ser Asp Leu Ala His Gln
225 230 235 240
Val Arg Ser Val Leu Lys Asn Tyr Pro Lys Leu Met Leu Thr Gln Gly
245 250 255
Arg Lys Val Leu Glu Ile Arg Pro Ile Ile Lys Trp Asp Lys Gly Lys
260 265 270
Ala Leu Glu Phe Leu Leu Glu Ser Leu Gly Tyr Asp Asn Cys Thr Asp
275 280 285
Val Phe Pro Ile Tyr Ile Gly Asp Asp Arg Thr Asp Glu Asp Ala Phe
290 295 300
Lys Ile Leu Arg Asp Lys Lys Gln Gly Leu Gly Ile Leu Val Ser Lys
305 310 315 320
Tyr Ala Lys Glu Thr Asn Ala Ser Tyr Ser Leu Gln Glu Pro Asp Glu
325 330 335
Val Met Val Phe Leu Glu Arg Leu Val Glu Trp Lys Gln Ser Arg Cys
340 345 350
Gly Ala

<210> 17
<211> 1690
<212> DNA
<213> Arabidopsis thaliana

<400> 17
tttcttctct tcctcctaca ttctcgactc tctcttttta ttagggtttt caattttaat 60
caaaaaaaaa ccctcaaatc tcatttttgg atttggtttc ttctgcgatc tctgagattc 120
aatttcttct atcaaaggaa acaaaaaact tcatctttcc gaagattttt tttccttcct 180
tctggagctc ttttgaatca ggtgaatttc taattgaatc tgaaagttta cgatgaacaa 240

```
tttgatatga tgataagttt tgtttgatgg agctttcacc aacaagcagt gacaagaaaa    300
caatgaagag atggtttttc atagataaac gagttggata gagtgttatt gaagataaag    360
aaagcaaaaa acagaagttc ttgcattgaa gcattggttt tagagtttat tgaagcattg    420
tttagagttt tgattaatgg atttaaactc aaaccacaaa tcctctgttc ttaaagatcc    480
ttcaccatca gttaaccaat caagactcgg cgtgtctagt agatttatga tgagtcaatg    540
gaagaaacct gcgaaactcg atgatgttag atccaatggt tggttagatg caatgatttc    600
atcttctcca ccgcgtaaga agcttgtcaa agattttaat gttgaagttg ctcctgaaga    660
tgattttgct caacgtgctt ggatggtgaa atatccttcg gcgattagct cgtttgcgca    720
tattgcagct caagcaaaga agaaaaagat tgctgtattt ctagattatg atggtactct    780
ttctccaata gttgatgatc ctgatcgtgc catcatgtct gatgcaatgc gttctgcggt    840
taaagatgtc gcgagttact tcccaaccgc aataattagc ggtagaagcc gtgacaaggt    900
ttatcagttg gtaggactaa cagaacttta ttacgcgggt agtcatggaa tggacataat    960
gacttcttct gatggtccga attgtttcaa atccactgac caacagggta aggaagtgaa   1020
tctgtttcag cccgcgagag aattcatacc ggttatcgac gaggttttta gaacccttgt   1080
tgagaaaatg aaagatatca aaggtgcaaa agtagagaac cacaagttct gtgcatctgt   1140
acattaccgt aacgttgacg aaaaggattg gcctattatt gctcagcgtg ttcatgacca   1200
cttgaaacaa taccctcgtt tgcgtctaac tcatgggagg aaggttttag aggttcgtcc   1260
tgtgatagac tggaacaaag gaagagcggt cgagtttcta ttagaatctc tcggattaag   1320
caataaagac gatttgcttc ctatctacat tggtgatgac acaaccgatg aagatgcgtt   1380
caaggtactg agagatggga accgaggttt cggcatccta gtatcgtcta taccgaaaga   1440
aagcaatgcg ttttactccc ttagagatcc atccgaggtg aagaagtttc taaagacttt   1500
ggtgaaatgg gcaaagttgg agaagaattc tactggtttt tgaataatat agtggaattt   1560
tatgattaaa tgcttattta cacttttttt tgtttcttta atcttttttc atctctaccc   1620
agtttagtc acagcttaat ttatgatttt ccatttatct ggtggaagtt aagttattaa    1680
agttacatgt                                                          1690
```

```
<210>  18
<211>  368
<212>  PRT
<213>  Arabidopsis thaliana

<400>  18
Met Asp Leu Asn Ser Asn His Lys Ser Ser Val Leu Lys Asp Pro Ser
1               5                   10                  15
Pro Ser Val Asn Gln Ser Arg Leu Gly Val Ser Ser Arg Phe Met Met
            20                  25                  30
Ser Gln Trp Lys Lys Pro Ala Lys Leu Asp Asp Val Arg Ser Asn Gly
        35                  40                  45
Trp Leu Asp Ala Met Ile Ser Ser Ser Pro Pro Arg Lys Lys Leu Val
    50                  55                  60
Lys Asp Phe Asn Val Glu Val Ala Pro Glu Asp Asp Phe Ala Gln Arg
65                  70                  75                  80
Ala Trp Met Val Lys Tyr Pro Ser Ala Ile Ser Ser Phe Ala His Ile
                85                  90                  95
Ala Ala Gln Ala Lys Lys Lys Lys Ile Ala Val Phe Leu Asp Tyr Asp
            100                 105                 110
Gly Thr Leu Ser Pro Ile Val Asp Asp Pro Asp Arg Ala Ile Met Ser
        115                 120                 125
Asp Ala Met Arg Ser Ala Val Lys Asp Val Ala Ser Tyr Phe Pro Thr
    130                 135                 140
Ala Ile Ile Ser Gly Arg Ser Arg Asp Lys Val Tyr Gln Leu Val Gly
145                 150                 155                 160
Leu Thr Glu Leu Tyr Tyr Ala Gly Ser His Gly Met Asp Ile Met Thr
                165                 170                 175
Ser Ser Asp Gly Pro Asn Cys Phe Lys Ser Thr Asp Gln Gln Gly Lys
            180                 185                 190
Glu Val Asn Leu Phe Gln Pro Ala Arg Glu Phe Ile Pro Val Ile Asp
        195                 200                 205
Glu Val Phe Arg Thr Leu Val Glu Lys Met Lys Asp Ile Lys Gly Ala
```

```
    210                 215                 220
Lys Val Glu Asn His Lys Phe Cys Ala Ser Val His Tyr Arg Asn Val
225                 230                 235                 240
Asp Glu Lys Asp Trp Pro Ile Ile Ala Gln Arg Val His Asp His Leu
                245                 250                 255
Lys Gln Tyr Pro Arg Leu Arg Leu Thr His Gly Arg Lys Val Leu Glu
            260                 265                 270
Val Arg Pro Val Ile Asp Trp Asn Lys Gly Arg Ala Val Glu Phe Leu
        275                 280                 285
Leu Glu Ser Leu Gly Leu Ser Asn Lys Asp Asp Leu Leu Pro Ile Tyr
    290                 295                 300
Ile Gly Asp Asp Thr Thr Asp Glu Asp Ala Phe Lys Val Leu Arg Asp
305                 310                 315                 320
Gly Asn Arg Gly Phe Gly Ile Leu Val Ser Ser Ile Pro Lys Glu Ser
                325                 330                 335
Asn Ala Phe Tyr Ser Leu Arg Asp Pro Ser Glu Val Lys Lys Phe Leu
            340                 345                 350
Lys Thr Leu Val Lys Trp Ala Lys Leu Glu Lys Asn Ser Thr Gly Phe
        355                 360                 365
```

```
<210>  19
<211>  1687
<212>  DNA
<213>  Arabidopsis thaliana

<400>  19
gtgagctctt tgcaattttt agggttttct tcgaataaag aaaaaaaaaa tccctgatct      60
tcctcttagt gatttctcct ttcgagatcc ctgagattcc gagaaaatct gcattttttt     120
ctcgtgattc tctagctctg ttttgtgttt tacacagctc ttttgaatca ggtttgtgtg     180
tgatagcgtt gttgtggtaa aagctttgta catttgaaga tttagagatg ataagttttc     240
aggtaaccta cttttaatgg acagttcaac aacaagcagt gataagaaaa cactaaagag     300
gtggtttttc attgacaaaa gagttggata aagtgtgttt agaagaagcc gtcgaatttc     360
ttagtttgaa gcattttatt gtttggagag ttcgatggat ttgaatataa acaagacgac     420
ccctgttctt agtgacccta ctacaccagt aagtaaaaca agacttggat catccttccc     480
ttcagggaga ttcatgatga attctagaaa gaagattcct aaactcgatg atgttcgatc     540
taacggttgg ttggacgcga tgatatcttc gtctccaccg cgtaaaaggc ttgtcaagga     600
tttcaatatt gagattgctc ctgaagatga tttttctcaa cgggcctgga tgctcaagta     660
tccttcagcg attacctcgt ttgcgcatat tgcagctcaa gcaaagaaca agaagatagc     720
tgtgtttcta gattatgatg gaacactttc tccaatagtc gatgaccctg atcgcgccat     780
catgtctgat gcgatgcgtg ctgctgtgaa agatgtcgcc aagtacttcc caacagcaat     840
aattagtggt agaagccgtg acaaggttta ccaattggta gggctaaccg aactctatta     900
cgcgggtagt cacgggatgg acattatgac tcctgtaaat ccaaatggat cccctgaaga     960
ccctaattgt ataaaaacca ctgaccaaca gggtgaggag gtaaacctct ttcagcctgc    1020
taaagagttc atacccgtca ttgaagaggt ttataataac ctcgttgaga taactaaatg    1080
tatcaaaggt gcaaaagtag agaaccataa gttttgtact tctgtacatt accgtaatgt    1140
tgacgagaag gactggccac ttgttgctca acgcgttcat gaccacctga aacgataccc    1200
tcgtttgcgt ataactcacg gtagaaaggt tttagaggtg cgtcctgtga ttgagtggaa    1260
caaaggaaaa gcagtagagt ttctgttaga gtctctcgga ttaagcaaca acgatgagtt    1320
cctcccaatc ttcatcggag atgacaagac cgatgaagat gcattcaagg tactgaggga    1380
agggaacaga ggatttggaa tattggtatc gtctgtacca aaagaaagca atgcatttta    1440
ctctcttaga gacccttccg aggtgaagaa gtttctcaag actttggtga aatgggggaa    1500
gatggaaagt tcaaaaacaa gtttttgatg attgtgtagg agaatcagaa gcttcattct    1560
gcactgcttt ttaatttctt attttttttc ctttttaaa gtttgtactt ctatatatta    1620
tcatttttcc atattatcca ttaagcaaac gggaaaaaca agaatataac gattgtcttg    1680
acattt                                                               1687

<210>  20
<211>  377
<212>  PRT
```

<213> Arabidopsis thaliana

<400> 20

```
Met Asp Leu Asn Ile Asn Lys Thr Thr Pro Val Leu Ser Asp Pro Thr
1               5                   10                  15
Thr Pro Val Ser Lys Thr Arg Leu Gly Ser Ser Phe Pro Ser Gly Arg
            20                  25                  30
Phe Met Met Asn Ser Arg Lys Lys Ile Pro Lys Leu Asp Asp Val Arg
        35                  40                  45
Ser Asn Gly Trp Leu Asp Ala Met Ile Ser Ser Ser Pro Pro Arg Lys
    50                  55                  60
Arg Leu Val Lys Asp Phe Asn Ile Glu Ile Ala Pro Glu Asp Asp Phe
65                  70                  75                  80
Ser Gln Arg Ala Trp Met Leu Lys Tyr Pro Ser Ala Ile Thr Ser Phe
                85                  90                  95
Ala His Ile Ala Ala Gln Ala Lys Asn Lys Lys Ile Ala Val Phe Leu
            100                 105                 110
Asp Tyr Asp Gly Thr Leu Ser Pro Ile Val Asp Asp Pro Asp Arg Ala
        115                 120                 125
Ile Met Ser Asp Ala Met Arg Ala Ala Val Lys Asp Val Ala Lys Tyr
    130                 135                 140
Phe Pro Thr Ala Ile Ile Ser Gly Arg Ser Arg Asp Lys Val Tyr Gln
145                 150                 155                 160
Leu Val Gly Leu Thr Glu Leu Tyr Tyr Ala Gly Ser His Gly Met Asp
                165                 170                 175
Ile Met Thr Pro Val Asn Pro Asn Gly Ser Pro Glu Asp Pro Asn Cys
            180                 185                 190
Ile Lys Thr Thr Asp Gln Gln Gly Glu Glu Val Asn Leu Phe Gln Pro
        195                 200                 205
Ala Lys Glu Phe Ile Pro Val Ile Glu Glu Val Tyr Asn Asn Leu Val
    210                 215                 220
Glu Ile Thr Lys Cys Ile Lys Gly Ala Lys Val Glu Asn His Lys Phe
225                 230                 235                 240
Cys Thr Ser Val His Tyr Arg Asn Val Asp Glu Lys Asp Trp Pro Leu
                245                 250                 255
Val Ala Gln Arg Val His Asp His Leu Lys Arg Tyr Pro Arg Leu Arg
            260                 265                 270
Ile Thr His Gly Arg Lys Val Leu Glu Val Arg Pro Val Ile Glu Trp
        275                 280                 285
Asn Lys Gly Lys Ala Val Glu Phe Leu Leu Glu Ser Leu Gly Leu Ser
    290                 295                 300
Asn Asn Asp Glu Phe Leu Pro Ile Phe Ile Gly Asp Asp Lys Thr Asp
305                 310                 315                 320
Glu Asp Ala Phe Lys Val Leu Arg Glu Gly Asn Arg Gly Phe Gly Ile
                325                 330                 335
Leu Val Ser Ser Val Pro Lys Glu Ser Asn Ala Phe Tyr Ser Leu Arg
            340                 345                 350
Asp Pro Ser Glu Val Lys Lys Phe Leu Lys Thr Leu Val Lys Trp Gly
        355                 360                 365
Lys Met Glu Ser Ser Lys Thr Ser Phe
    370                 375
```

<210> 21
<211> 1283
<212> DNA
<213> Arabidopsis thaliana

<400> 21

```
gtctctctca aagcctacac actcattctc actctgtttc tacaacccta aatctctttt      60
```

```
catttacaga cagaaatggt tagattcata gaagaaaaca caaaacttgt agaaaaagaa     120
accgggaaca aatcaaacaa cgatgtaaca acaacgaaga agaaagctct tcaagatatc     180
attatcaaca atggagtagg attgatcaat tcatgggttg attcaatgag agcttgttcc     240
cctactcatc ttaaatcttt gttgaaacaa agctcttggc tcacggaaca tccatcagct     300
ttggatatgt ttgaagagat tcttcacctt tctgaaggaa aacaaatcgt tatgttcttg     360
gattatgatg gcactttatc tcccattgtt gatgatccag atcgagcttt catgtctaga     420
aagatgagaa gaactgtgag gaaactagca aactgtttcc cgacggccat agttagtggg     480
agatgcatag aaaaggttta taactttgta aaactaactg agttgtacta tgctggaagt     540
catggaatgg acatcaaagg accagagcaa gggtccaaat atgagcaaat tttacaggat     600
agtaaatctc ttctttgcca accagctaca gagttcctcc ccatgatcga cgaggtttat     660
cataaattag tggagaaaac aaaatctact cccggagccc aagtagagaa caacaaattt     720
tgtgtctctg ttcacttccg acgcgtagat gaaaataact ggagtgattt ggctaaccaa     780
gttcgatcag taatgaaaga ctaccctaag ctccgtctta ctcaaggaag aaaagttttg     840
gaggttcgtc cgattattaa atgggacaaa ggcaaagcac tcgagttttt attagagtca     900
cttggatacg ctaattgtac cgacgttttc cctctttata tcggagatga tcgcaccgac     960
gaagatgcat tcaaggtatt gagagaacga agacaaggtc ttggcattct tgtatctaaa    1020
tttccaaagg agactagcgc gtcttattca ctgcaagaac ccgatgaggt tatggagttc    1080
ttgcaacgtt tagtggaatg gaaacaattg agatctggag catgatgaga gctataacta    1140
cagaagaatc gtttcttctt aattagtaac ttgttaattt tattttattt tcttgctctc    1200
atatattgta ctcaacgcgt atgtaaaatg tttataagaa aattgtagat ccatcattct    1260
caaggaaaat atgttttata tat                                              1283
```

```
<210>  22
<211>  349
<212>  PRT
<213>  Arabidopsis thaliana

<400>  22
Met Val Arg Phe Ile Glu Glu Asn Thr Lys Leu Val Glu Lys Glu Thr
1               5                   10                  15
Gly Asn Lys Ser Asn Asn Asp Val Thr Thr Thr Lys Lys Lys Ala Leu
            20                  25                  30
Gln Asp Ile Ile Ile Asn Asn Gly Val Gly Leu Ile Asn Ser Trp Val
        35                  40                  45
Asp Ser Met Arg Ala Cys Ser Pro Thr His Leu Lys Ser Leu Leu Lys
    50                  55                  60
Gln Ser Ser Trp Leu Thr Glu His Pro Ser Ala Leu Asp Met Phe Glu
65                  70                  75                  80
Glu Ile Leu His Leu Ser Glu Gly Lys Gln Ile Val Met Phe Leu Asp
                85                  90                  95
Tyr Asp Gly Thr Leu Ser Pro Ile Val Asp Asp Pro Asp Arg Ala Phe
            100                 105                 110
Met Ser Arg Lys Met Arg Arg Thr Val Arg Lys Leu Ala Asn Cys Phe
        115                 120                 125
Pro Thr Ala Ile Val Ser Gly Arg Cys Ile Glu Lys Val Tyr Asn Phe
    130                 135                 140
Val Lys Leu Thr Glu Leu Tyr Tyr Ala Gly Ser His Gly Met Asp Ile
145                 150                 155                 160
Lys Gly Pro Glu Gln Gly Ser Lys Tyr Glu Gln Ile Leu Gln Asp Ser
                165                 170                 175
Lys Ser Leu Leu Cys Gln Pro Ala Thr Glu Phe Leu Pro Met Ile Asp
            180                 185                 190
Glu Val Tyr His Lys Leu Val Glu Lys Thr Lys Ser Thr Pro Gly Ala
        195                 200                 205
Gln Val Glu Asn Asn Lys Phe Cys Val Ser Val His Phe Arg Arg Val
    210                 215                 220
Asp Glu Asn Asn Trp Ser Asp Leu Ala Asn Gln Val Arg Ser Val Met
225                 230                 235                 240
Lys Asp Tyr Pro Lys Leu Arg Leu Thr Gln Gly Arg Lys Val Leu Glu
```

```
                245                 250                 255
Val Arg Pro Ile Ile Lys Trp Asp Lys Gly Lys Ala Leu Glu Phe Leu
            260                 265                 270
Leu Glu Ser Leu Gly Tyr Ala Asn Cys Thr Asp Val Phe Pro Leu Tyr
        275                 280                 285
Ile Gly Asp Asp Arg Thr Asp Glu Asp Ala Phe Lys Val Leu Arg Glu
    290                 295                 300
Arg Arg Gln Gly Leu Gly Ile Leu Val Ser Lys Phe Pro Lys Glu Thr
305                 310                 315                 320
Ser Ala Ser Tyr Ser Leu Gln Glu Pro Asp Glu Val Met Glu Phe Leu
                325                 330                 335
Gln Arg Leu Val Glu Trp Lys Gln Leu Arg Ser Gly Ala
            340                 345
```

```
<210>  23
<211>  1280
<212>  DNA
<213>  Arabidopsis thaliana

<400>  23
atgtcagcta gtcaaaacat tgtcgtatca gagactacaa tgtcaagtat catccccaac      60
aacaacaaca acaacaacaa ctcttcttca cagaaactcc ctccttgttt aatctcaatt     120
tccaagaaaa agcttctcaa gaacatcgac atcatcaatg gtggtggaca aagaatcaac     180
gcttgggtag attcaatgcg tgcttcttct cctactcatc tcaaatctct tccttcttct     240
atctccacac agcaacaact caactcatgg atcatgcaac atccttcagc actagaaaaa     300
ttcgaacaga taatggaagc ttcgagaggg aaacaaatcg taatgtttct tgattatgac     360
ggtactctct ctcccattgt tgatgatcca gacaaagctt tcatgtcaag caagatgaga     420
agaacagtga aaaaactggc taagtgtttc cccactgcta tagttactgg tagatgcata     480
gacaaggtgt ataactttgt gaagcttgct gagctgtatt atgctggaag ccatggcatg     540
gacattaaag gtccagcaaa aggcttctcc agacacaaga gggttaaaca gtctcttctg     600
taccaaccag ctaatgacta tcttcccatg atcgatgaag tttatagaca actcttggaa     660
aaaacaaaat cgactccagg agccaaagta gaaaaccaca agttttgtgc ttctgtgcac     720
tttcgctgcg tcgatgagaa gaaatggagc gaactggttc tacaggttcg gtcggtatta     780
aagaaattcc ctacgctgca actgacccaa ggtcggaagg ttttcgaaat ccgtccaatg     840
attgaatggg ataaaggaaa ggctcttgag ttcttgttag aatcacttgg atttgggaac     900
actaacaatg ttttcccggt ttatattggt gacgatcgaa ctgacgaaga tgcatttaag     960
atgctacgag acagaggcga aggctttggc attcttgtct ccaagtttcc caaggatact    1020
gatgcttcgt attctttgca agatccatcc gaggtgatgg atttcttacg acgattggtg    1080
gaatggaaac aaatgcagcc aagaatgtga aatgataata taataaacga gtgacaatat    1140
tcgaaatgcc aatggatgta tataatgaat atgttcatgc ctaacttttt taggaacgtt    1200
gtaattggta aaggatatgg cagtgtcttt aacgtacccc cattaggtta atcttcttgt    1260
tcgtcacatt gtatttttgt                                                1280

<210>  24
<211>  369
<212>  PRT
<213>  Arabidopsis thaliana

<400>  24
Met Ser Ala Ser Gln Asn Ile Val Val Ser Glu Thr Thr Met Ser Ser
1               5                   10                  15
Ile Ile Pro Asn Asn Asn Asn Asn Asn Asn Asn Ser Ser Ser Gln Lys
            20                  25                  30
Leu Pro Pro Cys Leu Ile Ser Ile Ser Lys Lys Lys Leu Leu Lys Asn
        35                  40                  45
Ile Asp Ile Ile Asn Gly Gly Gly Gln Arg Ile Asn Ala Trp Val Asp
    50                  55                  60
Ser Met Arg Ala Ser Ser Pro Thr His Leu Lys Ser Leu Pro Ser Ser
65                  70                  75                  80
```

```
Ile Ser Thr Gln Gln Gln Leu Asn Ser Trp Ile Met Gln His Pro Ser
                85                  90                  95
Ala Leu Glu Lys Phe Glu Gln Ile Met Glu Ala Ser Arg Gly Lys Gln
            100                 105                 110
Ile Val Met Phe Leu Asp Tyr Asp Gly Thr Leu Ser Pro Ile Val Asp
        115                 120                 125
Asp Pro Asp Lys Ala Phe Met Ser Ser Lys Met Arg Arg Thr Val Lys
    130                 135                 140
Lys Leu Ala Lys Cys Phe Pro Thr Ala Ile Val Thr Gly Arg Cys Ile
145                 150                 155                 160
Asp Lys Val Tyr Asn Phe Val Lys Leu Ala Glu Leu Tyr Tyr Ala Gly
                165                 170                 175
Ser His Gly Met Asp Ile Lys Gly Pro Ala Lys Gly Phe Ser Arg His
            180                 185                 190
Lys Arg Val Lys Gln Ser Leu Leu Tyr Gln Pro Ala Asn Asp Tyr Leu
        195                 200                 205
Pro Met Ile Asp Glu Val Tyr Arg Gln Leu Leu Glu Lys Thr Lys Ser
    210                 215                 220
Thr Pro Gly Ala Lys Val Glu Asn His Lys Phe Cys Ala Ser Val His
225                 230                 235                 240
Phe Arg Cys Val Asp Glu Lys Lys Trp Ser Glu Leu Val Leu Gln Val
                245                 250                 255
Arg Ser Val Leu Lys Lys Phe Pro Thr Leu Gln Leu Thr Gln Gly Arg
            260                 265                 270
Lys Val Phe Glu Ile Arg Pro Met Ile Glu Trp Asp Lys Gly Lys Ala
        275                 280                 285
Leu Glu Phe Leu Leu Glu Ser Leu Gly Phe Gly Asn Thr Asn Asn Val
    290                 295                 300
Phe Pro Val Tyr Ile Gly Asp Asp Arg Thr Asp Glu Asp Ala Phe Lys
305                 310                 315                 320
Met Leu Arg Asp Arg Gly Glu Gly Phe Gly Ile Leu Val Ser Lys Phe
                325                 330                 335
Pro Lys Asp Thr Asp Ala Ser Tyr Ser Leu Gln Asp Pro Ser Glu Val
            340                 345                 350
Met Asp Phe Leu Arg Arg Leu Val Glu Trp Lys Gln Met Gln Pro Arg
        355                 360                 365
Met
```

```
<210>  25
<211>  1558
<212>  DNA
<213>  Arabidopsis thaliana

<400>  25
gatacaatct tagtaatcac acttgagtat ttttcttaac tggttttgtt tcttggcatc      60
ttcatttcag ggtctatatt tctctgagga attggattct tatagaagtt aaaccgcaga     120
gcttcttcta tccaatagtc ctctgagaat cgtcgatata gtttgctgat atagttgtta     180
tggacatgaa atctggtcac tcgtctcctg taatgactga ttctccacca ataagcaact     240
caagattaac cattcgtcag aatagacttc cttactcatc agcagcagcc acggctatct     300
cacagaacaa caatctctta ctaaccgttc caagaaagaa aactgggatc cttgatgatg     360
ttaagtctaa tggttggctt gatgcgatga aatcttcttc tcctcctcct acaatactta     420
acaaagataa cttaagcaat gatgctacgg atatgactta tcgcgaatgg atgcagctca     480
agtatccatc agctcttacc tcttttgaga aaatcatgag ttttgcaaaa ggcaaaagaa     540
tagcattgtt tcttgattat gacgggacac tttcgcctat tgttgaggaa cctgattgtg     600
catacatgtc aagtgctatg cgtagtgcag tgcaaaatgt tgccaagtat ttccctaccg     660
cgatcattag tggaagaagc cgggataagg tgtatgagtt tgttaatttg agtgaacttt     720
attacgccgg aagccatgga atggacatca tgagtcccgc aggagaatct ttaaaccatg     780
aacatagccg tactgtatca gtttacgaac aggggaaaga tgtaaatcta ttccagcctg     840
```

```
ctagcgagtt tctcccgatg atcgataagg tgctttgttc tcttatagag agtacaaaag     900
atatcaaagg ggtaaaagta gaagacaaca agttctgcat ctctgtgcat taccgcaatg     960
tagaagaaaa gaactggaca ttggttgcac agtgtgtaga tgatgtcatc agaacatatc    1020
caaaactacg gctaacacat ggccggaagg ttttagagat ccgtcctgtg attgactggg    1080
acaaagggaa agctgtgaca tttctacttg aatcactcgg cctaaacaac tgtgaggatg    1140
ttcttccaat ctatgttggg gatgatcgaa cagacgaaga tgcatttaag gtactacgag    1200
atggaccaaa ccacggttat ggtatattag tctctgctgt gcctaaagac agcaatgcct    1260
tttactcgct tcgtgacccg tctgaggtga tggagtttct gaagtcattg gtgacatgga    1320
agagatcaat gggttaaagt agaagaagaa gatgtagaag aaaagtggtt aacgttaata    1380
tatattacaa atatgcaagt tagaggttta ttgttacttt tcggctttat acaaaaacta    1440
ccgttgagat ttaacttaac ggtatcggag gagagatcac atgatggctt aaaacatgcc    1500
ggagatactt tttccgacca atctctttat cttctacaac tgtaaattcc tccatctc      1558

<210>  26
<211>  385
<212>  PRT
<213>  Arabidopsis thaliana

<400>  26
Met Asp Met Lys Ser Gly His Ser Ser Pro Val Met Thr Asp Ser Pro
1               5                   10                  15
Pro Ile Ser Asn Ser Arg Leu Thr Ile Arg Gln Asn Arg Leu Pro Tyr
            20                  25                  30
Ser Ser Ala Ala Ala Thr Ala Ile Ser Gln Asn Asn Asn Leu Leu Leu
        35                  40                  45
Thr Val Pro Arg Lys Lys Thr Gly Ile Leu Asp Asp Val Lys Ser Asn
    50                  55                  60
Gly Trp Leu Asp Ala Met Lys Ser Ser Ser Pro Pro Pro Thr Ile Leu
65                  70                  75                  80
Asn Lys Asp Asn Leu Ser Asn Asp Ala Thr Asp Met Thr Tyr Arg Glu
                85                  90                  95
Trp Met Gln Leu Lys Tyr Pro Ser Ala Leu Thr Ser Phe Glu Lys Ile
            100                 105                 110
Met Ser Phe Ala Lys Gly Lys Arg Ile Ala Leu Phe Leu Asp Tyr Asp
        115                 120                 125
Gly Thr Leu Ser Pro Ile Val Glu Glu Pro Asp Cys Ala Tyr Met Ser
    130                 135                 140
Ser Ala Met Arg Ser Ala Val Gln Asn Val Ala Lys Tyr Phe Pro Thr
145                 150                 155                 160
Ala Ile Ile Ser Gly Arg Ser Arg Asp Lys Val Tyr Glu Phe Val Asn
                165                 170                 175
Leu Ser Glu Leu Tyr Tyr Ala Gly Ser His Gly Met Asp Ile Met Ser
            180                 185                 190
Pro Ala Gly Glu Ser Leu Asn His Glu His Ser Arg Thr Val Ser Val
        195                 200                 205
Tyr Glu Gln Gly Lys Asp Val Asn Leu Phe Gln Pro Ala Ser Glu Phe
    210                 215                 220
Leu Pro Met Ile Asp Lys Val Leu Cys Ser Leu Ile Glu Ser Thr Lys
225                 230                 235                 240
Asp Ile Lys Gly Val Lys Val Glu Asp Asn Lys Phe Cys Ile Ser Val
                245                 250                 255
His Tyr Arg Asn Val Glu Glu Lys Asn Trp Thr Leu Val Ala Gln Cys
            260                 265                 270
Val Asp Asp Val Ile Arg Thr Tyr Pro Lys Leu Arg Leu Thr His Gly
        275                 280                 285
Arg Lys Val Leu Glu Ile Arg Pro Val Ile Asp Trp Asp Lys Gly Lys
    290                 295                 300
Ala Val Thr Phe Leu Leu Glu Ser Leu Gly Leu Asn Asn Cys Glu Asp
305                 310                 315                 320
```

Val Leu Pro Ile Tyr Val Gly Asp Asp Arg Thr Asp Glu Asp Ala Phe
325 330 335
Lys Val Leu Arg Asp Gly Pro Asn His Gly Tyr Gly Ile Leu Val Ser
340 345 350
Ala Val Pro Lys Asp Ser Asn Ala Phe Tyr Ser Leu Arg Asp Pro Ser
355 360 365
Glu Val Met Glu Phe Leu Lys Ser Leu Val Thr Trp Lys Arg Ser Met
370 375 380
Gly
385

<210> 27
<211> 1530
<212> DNA
<213> Arabidopsis thaliana

<400> 27
gaacacttca aaaaacatag caacttcact tcttcctcct ccttcctagt ttctcgtttg 60
gtctcattct ctttaattac ctcctcctct gttctgctca tcccctgttt ttatagtttt 120
atataaaatg gtgagccaaa acgtcgtcgt atcagacgcc aagaccggga tcataacggt 180
ctctacagtc tctaactcct ctgtctttac tcctaccgct caaaaaccac cgactgctcc 240
tggttacatc tcagtttcca agaaaaaact cctcaaaaac cttgaaatca atggagctga 300
tcaaagtcaa agacttaact cttgggttga ctccatgaga gcttcttctc ctacccatct 360
caaatcactt tcctctttct cttccgaaga agaacacaat tcttggatca aacgacatcc 420
atcagcactt aacatgttcg aacgaatcat cgaagaagca agaggaaaac aaatcgtcat 480
gtttcttgat tatgacggta ctctttctcc gatcgtggat gatccagaca gagctttcat 540
gactagcaag atgagaagaa cagtgaaaaa aatggctaag tgttttccaa cttccatagt 600
tactggtaga tgcatcgaca aggtttatag ctttgtgaag ctagcagaac tgtattatgc 660
tggaagccat gggatggata ttaaaggccc aactaaaggt ttctcaagat acaataagga 720
taaaccatcc gttctttacc aaccagccgg cgactttctt cccatgatcg atgaggttta 780
taaacaatta gtggagaaaa ccaaatctac accaggagcc aaagtggaga acaacaagtt 840
ctgtctttct gtgcacttcc gttgtgttga cgagaagaaa tggagcgagc tggcttcaaa 900
agttcggtcg gtggtaaaga actatccgac gttgaaactg tctcaaggtc gaaaggtttt 960
tgaaatccga cctataatta aatggaacaa aggcaaggct cttgagtttt tgttagagtc 1020
actcggattt gaaaactgta acgatgtatt tccaatttac attggtgatg ataaaaccga 1080
tgaagatgca tttaagctac tacgagggag aggacaaggc tttggtattc ttgtctccaa 1140
gttccccaaa gacaccagtg cgtcgtattc tttacaagac ccacctgagg tgatgaattt 1200
cttgggacgg ttggtggagt ggaaacagat gcagcaataa acgtgcaagg ggcaatgaag 1260
tacatttagt agtcttttta ataaaacgag aatattcata actataggtg catatataga 1320
tataatatgt acatccctaa cctataatgg aacattgtac aagatcctct tatagattga 1380
tcttgttcat cacaaatctt ttcttttagt aaaagagatt atatgaacaa gaaaaatggg 1440
gaacttttat cgatttgggg atatattatt atagccttct atctttattg cttgtaggca 1500
atttcttact tttcaagtta ggctattgta 1530

<210> 28
<211> 370
<212> PRT
<213> Arabidopsis thaliana

<400> 28
Met Val Ser Gln Asn Val Val Val Ser Asp Ala Lys Thr Gly Ile Ile
1 5 10 15
Thr Val Ser Thr Val Ser Asn Ser Ser Val Phe Thr Pro Thr Ala Gln
20 25 30
Lys Pro Pro Thr Ala Pro Gly Tyr Ile Ser Val Ser Lys Lys Lys Leu
35 40 45
Leu Lys Asn Leu Glu Ile Asn Gly Ala Asp Gln Ser Gln Arg Leu Asn
50 55 60
Ser Trp Val Asp Ser Met Arg Ala Ser Ser Pro Thr His Leu Lys Ser

```
65                  70                  75                  80
Leu Ser Ser Phe Ser Ser Glu Glu Glu His Asn Ser Trp Ile Lys Arg
                85                  90                  95
His Pro Ser Ala Leu Asn Met Phe Glu Arg Ile Ile Glu Glu Ala Arg
            100                 105                 110
Gly Lys Gln Ile Val Met Phe Leu Asp Tyr Asp Gly Thr Leu Ser Pro
        115                 120                 125
Ile Val Asp Asp Pro Asp Arg Ala Phe Met Thr Ser Lys Met Arg Arg
    130                 135                 140
Thr Val Lys Lys Met Ala Lys Cys Phe Pro Thr Ser Ile Val Thr Gly
145                 150                 155                 160
Arg Cys Ile Asp Lys Val Tyr Ser Phe Val Lys Leu Ala Glu Leu Tyr
                165                 170                 175
Tyr Ala Gly Ser His Gly Met Asp Ile Lys Gly Pro Thr Lys Gly Phe
            180                 185                 190
Ser Arg Tyr Asn Lys Asp Lys Pro Ser Val Leu Tyr Gln Pro Ala Gly
        195                 200                 205
Asp Phe Leu Pro Met Ile Asp Glu Val Tyr Lys Gln Leu Val Glu Lys
    210                 215                 220
Thr Lys Ser Thr Pro Gly Ala Lys Val Glu Asn Asn Lys Phe Cys Leu
225                 230                 235                 240
Ser Val His Phe Arg Cys Val Asp Glu Lys Lys Trp Ser Glu Leu Ala
                245                 250                 255
Ser Lys Val Arg Ser Val Val Lys Asn Tyr Pro Thr Leu Lys Leu Ser
            260                 265                 270
Gln Gly Arg Lys Val Phe Glu Ile Arg Pro Ile Ile Lys Trp Asn Lys
        275                 280                 285
Gly Lys Ala Leu Glu Phe Leu Leu Glu Ser Leu Gly Phe Glu Asn Cys
    290                 295                 300
Asn Asp Val Phe Pro Ile Tyr Ile Gly Asp Asp Lys Thr Asp Glu Asp
305                 310                 315                 320
Ala Phe Lys Leu Leu Arg Gly Arg Gly Gln Gly Phe Gly Ile Leu Val
                325                 330                 335
Ser Lys Phe Pro Lys Asp Thr Ser Ala Ser Tyr Ser Leu Gln Asp Pro
            340                 345                 350
Pro Glu Val Met Asn Phe Leu Gly Arg Leu Val Glu Trp Lys Gln Met
        355                 360                 365
Gln Gln
    370

<210>  29
<211>  1178
<212>  DNA
<213>  Populus trichocarpa

<400>  29
atatgcagag tttagtttac acaagcggag atgacaaacc agaatgtggt tgtgcctgat      60
gccaaacagg gtattgatat caccatcgca atggtgctgc ccaagtctct gttttcacct     120
gttgtgccaa agccattgcc tgccgctcct ggtgggtatt tcacaataac ccgaaagagg     180
ttctcgaaga agacagaaac tggaggcaaa atcagttcct gggttgattc tatgagggat     240
tcttcaccta cccgtgtcaa atccaccact tctttatcag aaactgaaga gaaaaattct     300
tggattgtaa accatccttc tgctctgaac atgtttgagc aaatagtgaa aggctcaaag     360
ggaaagcaga ttgtgatgtt ccttgattat gatggtacac tgtcacccat tgttgaagat     420
cctgacagag cattcatgac caatgagatg agagaagctg tcagggacgt tgctagatac     480
tttcccacgg ctatagtgac gggaaggtgt agaaaaaagg tgtatagctt tgtaagattg     540
gcagggcttt attatgctgg tagccatggc atggacatca agggaccatc caaaaataat     600
tgcaaatacg aaaaaggtgg tgtactcttt caacctgcca gtgaattttt acccatgatt     660
gatgaggtgt acaatgtctt gttggagaga acaaaatcta tcccaggagc taaagtagaa     720
aacaacaaat tttgcgtatc cgtacacttt cgatgtgttg aggaaaagat gtgggctata     780
```

```
ttagtagagc aagtccgatc agttctcaat gattatccca agcttagatt aactcaaggg    840
aggaaggttt tagagatccg acccaccatt aaatgggaca agggcaaagc tcttgaattc    900
ttgttagagt cattaggata tgccaattct actgacgttt ttccggtcta tattggagat    960
gatcgaactg atgaggacgc attcaaggtt ctaagaaaca gggggcaagg gcttgggatt   1020
cttgtttcta aagttcccaa ggaaacaaat gcctcttatt ctctacagga accaaaagag   1080
gcaagttctt taatgtaaca agcaagttgt tatactttta tttgtggtaa taattattgt   1140
taaattttca gttttgcaag ttcgactaat aaatcata                           1178

<210>  30
<211>  355
<212>  PRT
<213>  Populus trichocarpa

<400>  30
Met Thr Asn Gln Asn Val Val Val Pro Asp Ala Lys Gln Gly Ile Asp
1               5                   10                  15
Ile Thr Ile Ala Met Val Leu Pro Lys Ser Leu Phe Ser Pro Val Val
            20                  25                  30
Pro Lys Pro Leu Pro Ala Ala Pro Gly Gly Tyr Phe Thr Ile Thr Arg
        35                  40                  45
Lys Arg Phe Ser Lys Lys Thr Glu Thr Gly Gly Lys Ile Ser Ser Trp
    50                  55                  60
Val Asp Ser Met Arg Asp Ser Ser Pro Thr Arg Val Lys Ser Thr Thr
65                  70                  75                  80
Ser Leu Ser Glu Thr Glu Glu Lys Asn Ser Trp Ile Val Asn His Pro
                85                  90                  95
Ser Ala Leu Asn Met Phe Glu Gln Ile Val Lys Gly Ser Lys Gly Lys
            100                 105                 110
Gln Ile Val Met Phe Leu Asp Tyr Asp Gly Thr Leu Ser Pro Ile Val
        115                 120                 125
Glu Asp Pro Asp Arg Ala Phe Met Thr Asn Glu Met Arg Glu Ala Val
    130                 135                 140
Arg Asp Val Ala Arg Tyr Phe Pro Thr Ala Ile Val Thr Gly Arg Cys
145                 150                 155                 160
Arg Lys Lys Val Tyr Ser Phe Val Arg Leu Ala Gly Leu Tyr Tyr Ala
                165                 170                 175
Gly Ser His Gly Met Asp Ile Lys Gly Pro Ser Lys Asn Asn Cys Lys
            180                 185                 190
Tyr Glu Lys Gly Gly Val Leu Phe Gln Pro Ala Ser Glu Phe Leu Pro
        195                 200                 205
Met Ile Asp Glu Val Tyr Asn Val Leu Leu Glu Arg Thr Lys Ser Ile
    210                 215                 220
Pro Gly Ala Lys Val Glu Asn Asn Lys Phe Cys Val Ser Val His Phe
225                 230                 235                 240
Arg Cys Val Glu Glu Lys Met Trp Ala Ile Leu Val Glu Gln Val Arg
                245                 250                 255
Ser Val Leu Asn Asp Tyr Pro Lys Leu Arg Leu Thr Gln Gly Arg Lys
            260                 265                 270
Val Leu Glu Ile Arg Pro Thr Ile Lys Trp Asp Lys Gly Lys Ala Leu
        275                 280                 285
Glu Phe Leu Leu Glu Ser Leu Gly Tyr Ala Asn Ser Thr Asp Val Phe
    290                 295                 300
Pro Val Tyr Ile Gly Asp Asp Arg Thr Asp Glu Asp Ala Phe Lys Val
305                 310                 315                 320
Leu Arg Asn Arg Gly Gln Gly Leu Gly Ile Leu Val Ser Lys Val Pro
                325                 330                 335
Lys Glu Thr Asn Ala Ser Tyr Ser Leu Gln Glu Pro Lys Glu Ala Ser
            340                 345                 350
Ser Leu Met
```

355

<210> 31
<211> 1184
<212> DNA
<213> Populus trichocarpa

<400> 31
gtgtgcagag tttagtttat acaaacagag atgacaaacc agaatgtggt tgtgcctaat 60
gccagaaaag gtgttgatat caccatcaca atggccctgt ccaagtctct gttttcacct 120
gttgtgccga agccattgcc agctgctcct ggtgggtatt tcacaatatc ccggaagatg 180
ttcgcaaaga agactgaaac tggaggcaaa accaattcct gggctgattc tatgagagat 240
tcttcaccta cccgtgtcaa atccactacg tctttatcag aaatcgagga gaaaaatact 300
tggattgtaa atcatccttc tgctttgaac atgttcgagc aaatagtgaa tggctcaaag 360
ggaaaacaga ttgtaatgtt tttagattat gatggtacac tgtcacccat tgttgaagat 420
cctgacaggg cattcatgac caacgagatg agagaagctg ttagggacgt cgctagatac 480
tttcccactg ctatagtgac gggaaggtgt agggataagg tgtacagctt cgtaagattg 540
gcagggcttt attacgctgg tagccatggc atggacatca agggaccatc caagaattgt 600
tgcagaaaca aaaaagatta tcaaggtgta ctttttcaac ctgccagtga tttttaccc 660
atgattgatg aggtgtacaa tgctttgctg gagagaacaa agtatatccc aggggctaga 720
gtagaagaca acaaattttg catatccgta cactttcgtt gtgtcgagga aaagatgtgg 780
gctgcattag tagagcaagt aagatcagtt ctcaatggtt atccaaaact tcgattaact 840
caagggagga aggttttaga gatccgaccc accattaaat gggacaaggg caaagctctt 900
gagttcgtgt tggaatcatt aggatatgcc aattctactg atgttttacc agtttatatt 960
ggagatgatc gaactgatga ggatgcattc aaggttctaa gaaacagggg acaagggctt 1020
gggattcttg tttctaaagt tcccaaggaa acaaatgcct cgtattctct acaagagcca 1080
acagaggcaa gttctttaat ctaacaagca aaggggttat gattaatttc tggtgctaat 1140
tatggttgat tttcaatctt acaagatgga ctgatgaatt atgt 1184

<210> 32
<211> 357
<212> PRT
<213> Populus trichocarpa

<400> 32
Met Thr Asn Gln Asn Val Val Val Pro Asn Ala Arg Lys Gly Val Asp
1 5 10 15
Ile Thr Ile Thr Met Ala Leu Ser Lys Ser Leu Phe Ser Pro Val Val
20 25 30
Pro Lys Pro Leu Pro Ala Ala Pro Gly Gly Tyr Phe Thr Ile Ser Arg
35 40 45
Lys Met Phe Ala Lys Lys Thr Glu Thr Gly Gly Lys Thr Asn Ser Trp
50 55 60
Ala Asp Ser Met Arg Asp Ser Ser Pro Thr Arg Val Lys Ser Thr Thr
65 70 75 80
Ser Leu Ser Glu Ile Glu Glu Lys Asn Thr Trp Ile Val Asn His Pro
85 90 95
Ser Ala Leu Asn Met Phe Glu Gln Ile Val Asn Gly Ser Lys Gly Lys
100 105 110
Gln Ile Val Met Phe Leu Asp Tyr Asp Gly Thr Leu Ser Pro Ile Val
115 120 125
Glu Asp Pro Asp Arg Ala Phe Met Thr Asn Glu Met Arg Glu Ala Val
130 135 140
Arg Asp Val Ala Arg Tyr Phe Pro Thr Ala Ile Val Thr Gly Arg Cys
145 150 155 160
Arg Asp Lys Val Tyr Ser Phe Val Arg Leu Ala Gly Leu Tyr Tyr Ala
165 170 175
Gly Ser His Gly Met Asp Ile Lys Gly Pro Ser Lys Asn Cys Cys Arg
180 185 190

```
Asn Lys Lys Asp Tyr Gln Gly Val Leu Phe Gln Pro Ala Ser Asp Phe
        195                 200                 205
Leu Pro Met Ile Asp Glu Val Tyr Asn Ala Leu Leu Glu Arg Thr Lys
    210                 215                 220
Tyr Ile Pro Gly Ala Arg Val Glu Asp Asn Lys Phe Cys Ile Ser Val
225                 230                 235                 240
His Phe Arg Cys Val Glu Glu Lys Met Trp Ala Ala Leu Val Glu Gln
                245                 250                 255
Val Arg Ser Val Leu Asn Gly Tyr Pro Lys Leu Arg Leu Thr Gln Gly
            260                 265                 270
Arg Lys Val Leu Glu Ile Arg Pro Thr Ile Lys Trp Asp Lys Gly Lys
        275                 280                 285
Ala Leu Glu Phe Val Leu Glu Ser Leu Gly Tyr Ala Asn Ser Thr Asp
    290                 295                 300
Val Leu Pro Val Tyr Ile Gly Asp Asp Arg Thr Asp Glu Asp Ala Phe
305                 310                 315                 320
Lys Val Leu Arg Asn Arg Gly Gln Gly Leu Gly Ile Leu Val Ser Lys
                325                 330                 335
Val Pro Lys Glu Thr Asn Ala Ser Tyr Ser Leu Gln Glu Pro Thr Glu
            340                 345                 350
Ala Ser Ser Leu Ile
        355
```

<210> 33
<211> 1337
<212> DNA
<213> Populus trichocarpa

<400> 33

```
atttggttt ttgtttttc tagtggaaag atgacaaacc aaaatgtggt agtggctgat     60
acaaactctg gactcaactt ggcaatcaca gtgcatgtaa caaactcctc tatcttcaca    120
acggcggccc agaaacctcc agcggcacca ggtggttaca tatccatttc tagaaagaaa    180
ctcttaaaga atctggaaat caatggagga gcaagaatta atgcttgggt tgattccatg    240
agagcctcat ctcctactca tatcaagtcc acgccttctg ttaatgaaga ccaaagctca    300
tggattcttc accacccatc agcactggag atgtttgagc agataattga tgcctctaaa    360
ggaaagcaaa ttgttatgtt cttggactat gatggcacac tttctcctat tgttgatgac    420
ccagataaag ctttcatgtc caagcaggtg atgagagcaa cagtgagaaa gcttgcaaga    480
tttttcccta ctgcaatagt gagtgggagg tgcagagaca aggtgtataa ctttgtacgg    540
ttagcagaac tgtactatgc tggaagccat ggcatggaca ttaaaggacc agcaaaaggc    600
tccaaataca agaaagtgag tgagctttac cactgtattt gcaagtacca tccaggcggt    660
gatggtgttg tctttcaggc tgccagtgaa tttcttccca tgatagatga ggtttacgaa    720
gaattggtag agaaaactaa aacaactcca ggggccaagg tggagaacaa caaattctgc    780
ctctctgtgc actatcgctg cgttgatgag aaaaaatgga gtggactggc acaagtagtt    840
aagtcagtgt tgaaggagta cccaaagctt cgacttactc aaggaagaaa ggttttagaa    900
atccgcccta ccattaaatg ggacaaagga aaggctcttg aatttttgtt agagtcactt    960
ggattcgcca attgcactga tgtttttcct gtttacattg gagatgatag aacagacgaa   1020
gatgcattta aggtactaag agagagagga caaggttttg gtatcttggt ctctaaaatc   1080
ccaaaggaca ctagtgcatc ttattcccta caggaaccca cccaggcaag ttatggattt   1140
cttgcgacgt ttggtggagt ggaaacggct ggcctttcaa gggcggtcaa gggtggtgta   1200
aagagtagtg ggaagattga gacagtacag atatacccac cccttcaaga aatgtaatta   1260
agggtgggat gttagactat gtgatcatct cttgttcaag attaaaaagt atgaacaaga   1320
aaagtaggga aatataa                                                  1337
```

<210> 34
<211> 408
<212> PRT
<213> Populus trichocarpa

<400> 34

```
Met Thr Asn Gln Asn Val Val Val Ala Asp Thr Asn Ser Gly Leu Asn
1               5                   10                  15
Leu Ala Ile Thr Val His Val Thr Asn Ser Ser Ile Phe Thr Thr Ala
            20                  25                  30
Ala Gln Lys Pro Pro Ala Ala Pro Gly Gly Tyr Ile Ser Ile Ser Arg
        35                  40                  45
Lys Lys Leu Leu Lys Asn Leu Glu Ile Asn Gly Gly Ala Arg Ile Asn
    50                  55                  60
Ala Trp Val Asp Ser Met Arg Ala Ser Ser Pro Thr His Ile Lys Ser
65                  70                  75                  80
Thr Pro Ser Val Asn Glu Asp Gln Ser Ser Trp Ile Leu His His Pro
                85                  90                  95
Ser Ala Leu Glu Met Phe Glu Gln Ile Ile Asp Ala Ser Lys Gly Lys
            100                 105                 110
Gln Ile Val Met Phe Leu Asp Tyr Asp Gly Thr Leu Ser Pro Ile Val
        115                 120                 125
Asp Asp Pro Asp Lys Ala Phe Met Ser Lys Gln Val Met Arg Ala Thr
    130                 135                 140
Val Arg Lys Leu Ala Arg Phe Phe Pro Thr Ala Ile Val Ser Gly Arg
145                 150                 155                 160
Cys Arg Asp Lys Val Tyr Asn Phe Val Arg Leu Ala Glu Leu Tyr Tyr
                165                 170                 175
Ala Gly Ser His Gly Met Asp Ile Lys Gly Pro Ala Lys Gly Ser Lys
            180                 185                 190
Tyr Lys Lys Val Ser Glu Leu Tyr His Cys Ile Cys Lys Tyr His Pro
        195                 200                 205
Gly Gly Asp Gly Val Val Phe Gln Ala Ala Ser Glu Phe Leu Pro Met
    210                 215                 220
Ile Asp Glu Val Tyr Glu Glu Leu Val Glu Lys Thr Lys Thr Thr Pro
225                 230                 235                 240
Gly Ala Lys Val Glu Asn Asn Lys Phe Cys Leu Ser Val His Tyr Arg
                245                 250                 255
Cys Val Asp Glu Lys Lys Trp Ser Gly Leu Ala Gln Val Val Lys Ser
            260                 265                 270
Val Leu Lys Glu Tyr Pro Lys Leu Arg Leu Thr Gln Gly Arg Lys Val
        275                 280                 285
Leu Glu Ile Arg Pro Thr Ile Lys Trp Asp Lys Gly Lys Ala Leu Glu
    290                 295                 300
Phe Leu Leu Glu Ser Leu Gly Phe Ala Asn Cys Thr Asp Val Phe Pro
305                 310                 315                 320
Val Tyr Ile Gly Asp Asp Arg Thr Asp Glu Asp Ala Phe Lys Val Leu
                325                 330                 335
Arg Glu Arg Gly Gln Gly Phe Gly Ile Leu Val Ser Lys Ile Pro Lys
            340                 345                 350
Asp Thr Ser Ala Ser Tyr Ser Leu Gln Glu Pro Thr Gln Ala Ser Tyr
        355                 360                 365
Gly Phe Leu Ala Thr Phe Gly Gly Val Glu Thr Ala Gly Leu Ser Arg
    370                 375                 380
Ala Val Lys Gly Gly Val Lys Ser Ser Gly Lys Ile Glu Thr Val Gln
385                 390                 395                 400
Ile Tyr Pro Pro Leu Gln Glu Met
                405
```

<210> 35
<211> 1253
<212> DNA
<213> Populus trichocarpa

<400> 35

```
tcacaagtac taccaagtaa tttggctacc atggatatcg agtcaaacca ttcttctcct     60
gttctcactg atcctgctcc aataaacaag tcaaggctcg gcatccattc taatttgttg    120
tcttacgcac catcgggagg atcactctcc tctagcaagt atagaaacgt taacattcct    180
agaaaaaagc ctgggaaact tgatgaagtc tgctcgaacg catgtctgga tgccatgaaa    240
tcctcatcac cccctcgcaa gaagctcatt aaggatggtg ctgacacagc ttacggcacc    300
tggatgctca agcatccatc agcactcaac tcatttgagg aaattgcaaa ttttgccaaa    360
aacaaaaaga tagcaatgtt tctagactac gatggtactc tctctccaat agtagatgac    420
ccagataatg cccttatgtc tgatgatatg cgttttgcag taagaaactt cgcaaaatat    480
ttcccaacgg cgattattag tggaagaagt cgtgacaagg tttatcagct tgtaggacta    540
acagaattgt attatgctgg tagtcatggg atggacatct tgggccctgt acgaaaagcg    600
gtgtccaatg accatccaaa ctgtaatgaa tcaactactg accaacaggg caaggaggtg    660
aatctgtttc agcctgctag agaatttata cctctgatcg atgaggtttt tagaaccctt    720
gtcgaggata ctaaggggat caaaggtgca aaagttgaga atcataaatt ttgcgtctct    780
gtacatttcc gtaatgtaga tgagaagaac tggcaatcta ttgcacaatg tgttcaggat    840
attttagata agtatcctcg tttgcgaaaa actcatggac ggaaggtttt agaggtccgt    900
ccaatgattg actggaataa aggaaaagca gttgaatttt tgcttgaatc tctaggtcta    960
agtaatagag atgatgtact cccaatttat attggtgatg atctgacaga tgaagatgca   1020
ttcaaggtgc tccgggaggg gaatcgaggc tgtggaattt tggtgtcatc tagacccaaa   1080
gaaaccaatg cagtttactc tctcagagat ccatcggagg tgatgaaatt tctcaattcc   1140
ctggtgacat ggaagaaggt ggatgcatcc tgaataatag gaggaacatc tgatgacatg   1200
cagagataat ttagaaattt gattttgaat atttataga tattttgcaa aat            1253
```

<210> 36
<211> 380
<212> PRT
<213> Populus trichocarpa

<400> 36

```
Met Asp Ile Glu Ser Asn His Ser Ser Pro Val Leu Thr Asp Pro Ala
1               5                   10                  15
Pro Ile Asn Lys Ser Arg Leu Gly Ile His Ser Asn Leu Leu Ser Tyr
            20                  25                  30
Ala Pro Ser Gly Gly Ser Leu Ser Ser Ser Lys Tyr Arg Asn Val Asn
        35                  40                  45
Ile Pro Arg Lys Lys Pro Gly Lys Leu Asp Glu Val Cys Ser Asn Ala
    50                  55                  60
Cys Leu Asp Ala Met Lys Ser Ser Ser Pro Pro Arg Lys Lys Leu Ile
65                  70                  75                  80
Lys Asp Gly Ala Asp Thr Ala Tyr Gly Thr Trp Met Leu Lys His Pro
                85                  90                  95
Ser Ala Leu Asn Ser Phe Glu Glu Ile Ala Asn Phe Ala Lys Asn Lys
            100                 105                 110
Lys Ile Ala Met Phe Leu Asp Tyr Asp Gly Thr Leu Ser Pro Ile Val
        115                 120                 125
Asp Asp Pro Asp Asn Ala Leu Met Ser Asp Asp Met Arg Phe Ala Val
    130                 135                 140
Arg Asn Phe Ala Lys Tyr Phe Pro Thr Ala Ile Ile Ser Gly Arg Ser
145                 150                 155                 160
Arg Asp Lys Val Tyr Gln Leu Val Gly Leu Thr Glu Leu Tyr Tyr Ala
                165                 170                 175
Gly Ser His Gly Met Asp Ile Leu Gly Pro Val Arg Lys Ala Val Ser
            180                 185                 190
Asn Asp His Pro Asn Cys Asn Glu Ser Thr Thr Asp Gln Gln Gly Lys
        195                 200                 205
Glu Val Asn Leu Phe Gln Pro Ala Arg Glu Phe Ile Pro Leu Ile Asp
    210                 215                 220
Glu Val Phe Arg Thr Leu Val Glu Asp Thr Lys Gly Ile Lys Gly Ala
225                 230                 235                 240
Lys Val Glu Asn His Lys Phe Cys Val Ser Val His Phe Arg Asn Val
```

```
                245                 250                 255
Asp Glu Lys Asn Trp Gln Ser Ile Ala Gln Cys Val Gln Asp Ile Leu
            260                 265                 270
Asp Lys Tyr Pro Arg Leu Arg Lys Thr His Gly Arg Lys Val Leu Glu
        275                 280                 285
Val Arg Pro Met Ile Asp Trp Asn Lys Gly Lys Ala Val Glu Phe Leu
    290                 295                 300
Leu Glu Ser Leu Gly Leu Ser Asn Arg Asp Asp Val Leu Pro Ile Tyr
305                 310                 315                 320
Ile Gly Asp Asp Leu Thr Asp Glu Asp Ala Phe Lys Val Leu Arg Glu
                325                 330                 335
Gly Asn Arg Gly Cys Gly Ile Leu Val Ser Ser Arg Pro Lys Glu Thr
            340                 345                 350
Asn Ala Val Tyr Ser Leu Arg Asp Pro Ser Glu Val Met Lys Phe Leu
        355                 360                 365
Asn Ser Leu Val Thr Trp Lys Lys Val Asp Ala Ser
    370                 375                 380

<210>  37
<211>  1271
<212>  DNA
<213>  Populus trichocarpa

<400>  37
aacaactctg ctgcaagtaa tttggctacg atggatgtcg agtcaaatca ttcttctcct      60
gttctcgctg accctgcatc gataaacagt tcaaggctcg gtatctattc taatctgttg     120
ccttactcac caccaggagg atcactctcc tctagcaagt atagtaggaa aaagccaggg     180
aagcttgacg aagtctgctc gagtggatgg ctggatgcca tgaaatcttc atcacccccct    240
cggaagaagc tctttaagga tggttctgac accgcttaca gttcctggat gttcaagcat     300
ccatcagcac tcaattcatt tgaggaaatt gcaaattttg ctaaaaacaa gaagatagca     360
atgtttctag actatgatgg gactctttct ccaattgtag atgacccgga taatgcgttt     420
atgtctgatg atatgcgttc tattgtaaaa aacgttgcga agtatttccc aacggcgatt     480
attagtggaa gaagtcgtga caaggtttat cagctggtag gactaacaga actatattat     540
gctggtagtc atgggatgga cattttgggc cctgtaggaa aagcttcaat gtccaatgat     600
catccaaact atagtgaatc tactactgac caacagggca aggaggtgaa tctgttccag     660
cctgctagag aatttatacc tatgatcgat gaggttttta gaacccttgt cgagaatact     720
aagggaatcg agggtgcaaa agtcgagaat cacaaatttt gtgcctctgt gcatttccga     780
aatgtagatg aggagaactg gcaacctatt gcacaatgtg ttcaggatat tctagataag     840
taccctcgtt tgcggagaac tcatggacgg aaggttttag aggtccgtcc aatgattgac     900
tggaataaag ggaaggcagt tgaattcctg cttgaatctc tagggctaag taacagagac     960
gacgtgctct caatttatat cggcgatgat ctgtcagatg aggatgcgtt caaggtgctc    1020
cgggagggga atcgaggtta tggaattctc gtatcatcta gacccaaaga aaccagtgca    1080
gtttactctc tcaaagatcc aattgaggtg atgaaatttc ttaactcctt ggtgacatgg    1140
aagaaggtag aagaaggtgg atgcatcctg aataacagga ggactatctg atgacatgca    1200
tatagcttag gtttttgatt tcgagcattt taattattga tattacataa taaagaggct    1260
gttggtacag c                                                         1271

<210>  38
<211>  386
<212>  PRT
<213>  Populus trichocarpa

<400>  38
Met Asp Val Glu Ser Asn His Ser Ser Pro Val Leu Ala Asp Pro Ala
1               5                   10                  15
Ser Ile Asn Ser Ser Arg Leu Gly Ile Tyr Ser Asn Leu Leu Pro Tyr
            20                  25                  30
Ser Pro Pro Gly Gly Ser Leu Ser Ser Ser Lys Tyr Ser Arg Lys Lys
        35                  40                  45
```

```
Pro Gly Lys Leu Asp Glu Val Cys Ser Ser Gly Trp Leu Asp Ala Met
    50                  55                  60
Lys Ser Ser Ser Pro Pro Arg Lys Lys Leu Phe Lys Asp Gly Ser Asp
65                  70                  75                  80
Thr Ala Tyr Ser Ser Trp Met Phe Lys His Pro Ser Ala Leu Asn Ser
                85                  90                  95
Phe Glu Glu Ile Ala Asn Phe Ala Lys Asn Lys Lys Ile Ala Met Phe
            100                 105                 110
Leu Asp Tyr Asp Gly Thr Leu Ser Pro Ile Val Asp Asp Pro Asp Asn
        115                 120                 125
Ala Phe Met Ser Asp Asp Met Arg Ser Ile Val Lys Asn Val Ala Lys
    130                 135                 140
Tyr Phe Pro Thr Ala Ile Ile Ser Gly Arg Ser Arg Asp Lys Val Tyr
145                 150                 155                 160
Gln Leu Val Gly Leu Thr Glu Leu Tyr Tyr Ala Gly Ser His Gly Met
                165                 170                 175
Asp Ile Leu Gly Pro Val Gly Lys Ala Ser Met Ser Asn Asp His Pro
            180                 185                 190
Asn Tyr Ser Glu Ser Thr Thr Asp Gln Gln Gly Lys Glu Val Asn Leu
        195                 200                 205
Phe Gln Pro Ala Arg Glu Phe Ile Pro Met Ile Asp Glu Val Phe Arg
    210                 215                 220
Thr Leu Val Glu Asn Thr Lys Gly Ile Glu Gly Ala Lys Val Glu Asn
225                 230                 235                 240
His Lys Phe Cys Ala Ser Val His Phe Arg Asn Val Asp Glu Glu Asn
                245                 250                 255
Trp Gln Pro Ile Ala Gln Cys Val Gln Asp Ile Leu Asp Lys Tyr Pro
            260                 265                 270
Arg Leu Arg Arg Thr His Gly Arg Lys Val Leu Glu Val Arg Pro Met
        275                 280                 285
Ile Asp Trp Asn Lys Gly Lys Ala Val Glu Phe Leu Leu Glu Ser Leu
    290                 295                 300
Gly Leu Ser Asn Arg Asp Asp Val Leu Ser Ile Tyr Ile Gly Asp Asp
305                 310                 315                 320
Leu Ser Asp Glu Asp Ala Phe Lys Val Leu Arg Glu Gly Asn Arg Gly
                325                 330                 335
Tyr Gly Ile Leu Val Ser Ser Arg Pro Lys Glu Thr Ser Ala Val Tyr
            340                 345                 350
Ser Leu Lys Asp Pro Ile Glu Val Met Lys Phe Leu Asn Ser Leu Val
        355                 360                 365
Thr Trp Lys Lys Val Glu Glu Gly Gly Cys Ile Leu Asn Asn Arg Arg
    370                 375                 380
Thr Ile
385
```

<210> 39
<211> 1307
<212> DNA
<213> Populus trichocarpa

<400> 39

```
ggtttttttt tttttttttg tagaggaaag atgacaaacc aaaatgtggt agtggctgat      60
acaaactctg gaatcaactt ggcaatcaca gtgcatgtaa caaattcctc tatcttcacc     120
acggccgcgc agaagcctcc ggcggctcct ggtggttaca tctctatttc cagaaagaaa     180
cttttaaaga atcttgaaat cagtggagga gcaagattta atgcttgggt taattccatg     240
agaacctctt ctcctactca tgtcaagtcc acaccttctg ctaatgacga ccaaagctca     300
tggattcttc accacccatc agcactggag atgtttgagc agataattga tgcctccaaa     360
ggaaagcaaa tagttatgtt cttggactat gatggcacac tttctcctat cgttgatgac     420
ccagatagag ctttcatgtc caagaaggtt gatgagagca acagtgagaa agcttgcgag     480
```

```
atttttttcct actgcaatag tgagtgggag atgcagagac aagcagaact gtactatgct   540
ggaagccatg gcatggacat caaaggacca gccaaaggca gcaaatacaa gaaaggcagt   600
gaaggtgttg tttttcaagc tggcagtgag tttcttccaa tgatagatga ggtttacaaa   660
gaactggtag agaaaactaa aacaactcca ggggccaagg tggaaaataa caaattctgt   720
ctctctgtgc actatcgctg cgttgatgag aagaaatgga gtggactggc tcaagtagtt   780
aagtcagtgt tgaaggagta cccaaagctt cgacttactc aaggaagaaa ggttttagaa   840
atccgcccta ccattaaatg ggacaaagga aaggctcttg aatttttgtt agagtctctt   900
ggattcgcca attgtactga tgtttttcct gtttacattg gagatgatag aacagacgag   960
gatgcattta aggtgctgag agagagagga caaggttttg gtatcttggt ctctaaattc  1020
cccaaggaca ctagtgcatc ttattcccta caagaaccca cccaggcaag tagtatccaa  1080
tacatgcata attgctgtca tggatttttt gcaacgtttg gtgcagtgga aacggctagc  1140
ctttcaaggg cagtcaaggg tttaagatgg agtaggaaaa tcgaaacagt atatatatat  1200
atatacccac cccttcaaga aatgtaatta agggtgggat cgatgttaga ccacgtaatt  1260
tcttgttcag gttaaaaaaa tatgaacaag aaaataggaa gggaaaa                1307

<210>  40
<211>  398
<212>  PRT
<213>  Populus trichocarpa

<400>  40
Met Thr Asn Gln Asn Val Val Val Ala Asp Thr Asn Ser Gly Ile Asn
1               5                   10                  15
Leu Ala Ile Thr Val His Val Thr Asn Ser Ser Ile Phe Thr Thr Ala
            20                  25                  30
Ala Gln Lys Pro Pro Ala Ala Pro Gly Gly Tyr Ile Ser Ile Ser Arg
        35                  40                  45
Lys Lys Leu Leu Lys Asn Leu Glu Ile Ser Gly Gly Ala Arg Phe Asn
    50                  55                  60
Ala Trp Val Asn Ser Met Arg Thr Ser Ser Pro Thr His Val Lys Ser
65                  70                  75                  80
Thr Pro Ser Ala Asn Asp Asp Gln Ser Ser Trp Ile Leu His His Pro
                85                  90                  95
Ser Ala Leu Glu Met Phe Glu Gln Ile Ile Asp Ala Ser Lys Gly Lys
            100                 105                 110
Gln Ile Val Met Phe Leu Asp Tyr Asp Gly Thr Leu Ser Pro Ile Val
        115                 120                 125
Asp Asp Pro Asp Arg Ala Phe Met Ser Lys Lys Val Asp Glu Ser Asn
    130                 135                 140
Ser Glu Lys Ala Cys Glu Ile Phe Ser Tyr Cys Asn Ser Glu Trp Glu
145                 150                 155                 160
Met Gln Arg Gln Ala Glu Leu Tyr Tyr Ala Gly Ser His Gly Met Asp
                165                 170                 175
Ile Lys Gly Pro Ala Lys Gly Ser Lys Tyr Lys Lys Gly Ser Glu Gly
            180                 185                 190
Val Val Phe Gln Ala Gly Ser Glu Phe Leu Pro Met Ile Asp Glu Val
        195                 200                 205
Tyr Lys Glu Leu Val Glu Lys Thr Lys Thr Thr Pro Gly Ala Lys Val
    210                 215                 220
Glu Asn Asn Lys Phe Cys Leu Ser Val His Tyr Arg Cys Val Asp Glu
225                 230                 235                 240
Lys Lys Trp Ser Gly Leu Ala Gln Val Val Lys Ser Val Leu Lys Glu
                245                 250                 255
Tyr Pro Lys Leu Arg Leu Thr Gln Gly Arg Lys Val Leu Glu Ile Arg
            260                 265                 270
Pro Thr Ile Lys Trp Asp Lys Gly Lys Ala Leu Glu Phe Leu Leu Glu
        275                 280                 285
Ser Leu Gly Phe Ala Asn Cys Thr Asp Val Phe Pro Val Tyr Ile Gly
    290                 295                 300
```

Asp Asp Arg Thr Asp Glu Asp Ala Phe Lys Val Leu Arg Glu Arg Gly
305 310 315 320
Gln Gly Phe Gly Ile Leu Val Ser Lys Phe Pro Lys Asp Thr Ser Ala
325 330 335
Ser Tyr Ser Leu Gln Glu Pro Thr Gln Ala Ser Ser Ile Gln Tyr Met
340 345 350
His Asn Cys Cys His Gly Phe Phe Ala Thr Phe Gly Ala Val Glu Thr
355 360 365
Ala Ser Leu Ser Arg Ala Val Lys Gly Leu Arg Trp Ser Arg Lys Ile
370 375 380
Glu Thr Val Tyr Ile Tyr Ile Tyr Pro Pro Leu Gln Glu Met
385 390 395

<210> 41
<211> 1274
<212> DNA
<213> Populus trichocarpa

<400> 41
ataaaaaata ctcttacgaa tctgaatccg atggacctca aatcaaatca taatgctcct 60
gtgctcactg attctgctcc cctaagcaag tcaaggctac gcgggtacca ccatggtttg 120
atgctgccgt actcaccctc aggtgcacct ttctcgtcaa atctattact atctattcct 180
aggaggaaaa ctggagtgct tgatgatgtt cgctcctgtg gttggctgga tgccatgaaa 240
tcatcatctc ctactcataa gaagtttgcc aaggatatta accatgagct ttccgcacct 300
gatccagaag ttgcctatcg cacctggctg cttaaatatc catctgcgct tgcatctttc 360
gagcaaattg caaactttgc aaaaggcaag agaatcgcct tatttctgga ttatgatggt 420
actctttcac caattgttga aaatcctgac aatgccttca tgtctgctga tatgcgttcc 480
attgtaaagg aagtggcaaa atatttccca acagcaataa tcagtggaag aagccgtgac 540
aaggtgtatg agtttgtagg gcttacagaa ctctactatg cgggtagtca tggtatggat 600
atcatgggcc ctgtcaggca atctgtatct gacgaccacc gaaattgtat caagtctacg 660
gacaagcagg gcaacgaagt taacttattc cagcctgcaa gagaattttt acctatgatt 720
gacgaggttt atagttccct tgtcaggatt accgaagata ttaaaggggc aacagttgag 780
aacaataaat tctgtgtctc tgtacattac cgtaacgttg atcaagataa ctggaaatca 840
gttggggagc gtgtccagga tgtcataaag aagtatcctc gcctgcgatt gactcatggg 900
aggaaggttt tagaaatccg tcccgcgatc aattgggaca aggggaaagc tcttgaattt 960
ctacttgaat cactagatct cagcaattgt gatgatgtgc tgccgattta tgttggagat 1020
gaccggacag atgaagatgc atttaaggtt ttgagagaga ggaactgtgg ttatggcatt 1080
tttgtatcaa aatcaccgaa ggaaagcaat gcgtattact ctctcagaga cccagcagag 1140
gtcatggagt ttctcaagtc cctggtgaca tggaagaaat cgagtgcttt ataaatgtta 1200
caaggaatta cataaagagg agaatactat aacatacaca gacaacatga aaggcatttt 1260
atattcttaa gttc 1274

<210> 42
<211> 387
<212> PRT
<213> Populus trichocarpa

<400> 42
Met Asp Leu Lys Ser Asn His Asn Ala Pro Val Leu Thr Asp Ser Ala
1 5 10 15
Pro Leu Ser Lys Ser Arg Leu Arg Gly Tyr His His Gly Leu Met Leu
20 25 30
Pro Tyr Ser Pro Ser Gly Ala Pro Phe Ser Ser Asn Leu Leu Leu Ser
35 40 45
Ile Pro Arg Arg Lys Thr Gly Val Leu Asp Asp Val Arg Ser Cys Gly
50 55 60
Trp Leu Asp Ala Met Lys Ser Ser Ser Pro Thr His Lys Lys Phe Ala
65 70 75 80
Lys Asp Ile Asn His Glu Leu Ser Ala Pro Asp Pro Glu Val Ala Tyr

```
                85                  90                  95
Arg Thr Trp Leu Leu Lys Tyr Pro Ser Ala Leu Ala Ser Phe Glu Gln
            100                 105                 110
Ile Ala Asn Phe Ala Lys Gly Lys Arg Ile Ala Leu Phe Leu Asp Tyr
        115                 120                 125
Asp Gly Thr Leu Ser Pro Ile Val Glu Asn Pro Asp Asn Ala Phe Met
    130                 135                 140
Ser Ala Asp Met Arg Ser Ile Val Lys Glu Val Ala Lys Tyr Phe Pro
145                 150                 155                 160
Thr Ala Ile Ile Ser Gly Arg Ser Arg Asp Lys Val Tyr Glu Phe Val
                165                 170                 175
Gly Leu Thr Glu Leu Tyr Tyr Ala Gly Ser His Gly Met Asp Ile Met
            180                 185                 190
Gly Pro Val Arg Gln Ser Val Ser Asp Asp His Arg Asn Cys Ile Lys
        195                 200                 205
Ser Thr Asp Lys Gln Gly Asn Glu Val Asn Leu Phe Gln Pro Ala Arg
    210                 215                 220
Glu Phe Leu Pro Met Ile Asp Glu Val Tyr Ser Ser Leu Val Arg Ile
225                 230                 235                 240
Thr Glu Asp Ile Lys Gly Ala Thr Val Glu Asn Asn Lys Phe Cys Val
                245                 250                 255
Ser Val His Tyr Arg Asn Val Asp Gln Asp Asn Trp Lys Ser Val Gly
            260                 265                 270
Glu Arg Val Gln Asp Val Ile Lys Lys Tyr Pro Arg Leu Arg Leu Thr
        275                 280                 285
His Gly Arg Lys Val Leu Glu Ile Arg Pro Ala Ile Asn Trp Asp Lys
    290                 295                 300
Gly Lys Ala Leu Glu Phe Leu Leu Glu Ser Leu Asp Leu Ser Asn Cys
305                 310                 315                 320
Asp Asp Val Leu Pro Ile Tyr Val Gly Asp Asp Arg Thr Asp Glu Asp
                325                 330                 335
Ala Phe Lys Val Leu Arg Glu Arg Asn Cys Gly Tyr Gly Ile Phe Val
            340                 345                 350
Ser Lys Ser Pro Lys Glu Ser Asn Ala Tyr Tyr Ser Leu Arg Asp Pro
        355                 360                 365
Ala Glu Val Met Glu Phe Leu Lys Ser Leu Val Thr Trp Lys Lys Ser
    370                 375                 380
Ser Ala Leu
385
```

<210> 43
<211> 1271
<212> DNA
<213> Populus trichocarpa

<400> 43

```
tatcttacga atctggatca tctggatccg atggacctca aatcaaatca cagtgctcct     60
gtgctcactg attctgcacc cctaggcaag tcaagactag gtgggcatca tggtttgttc    120
ccatgctcac cctcaggcgg tgcagctttt tcgccgaatc tatggttatc tattcctaag    180
aagaaaactg gagttcttga tgatgttcgc tccattggtt ggctggacgc aatgaaatca    240
tcatctcctc ctcacaagaa gtttaacaag gatattaaca tggagctttc ctcgcctgat    300
ccggaagctg cctaccgcac ttggcttctt aaatatccat ctgctcttgc atcttttgag    360
caaattgcaa actttgcaaa aggcaagaga atcgccttgt tcctggatta tgatggtact    420
ctatcgccga ttgtagaaaa tcccgacaat gccctcatgt ctgatgttat gcgttctgct    480
gtaaagaaag tggcaaaata tttccccaca gcaataatta gtggaagaag ccgtgacaag    540
gtatacgagt ttgtaggact cacagaactc tattatgcgg gtagccatgg aatggatatt    600
gtgggccctg ttaggcactc cacatctgat gatcacccaa attgtatcga gtctacggac    660
atgcagggaa atgaagttaa tttattccag ccagctagag aatttttacc tatgatcgac    720
gaggttttta gctcccttct caagagtacc gaagaaatta aaggtgcaac agttgagaac    780
```

aataaattct gtgtctctgt acattaccga aatgttgatg aagataaatg gaaagcagtt 840
tgggagtgtg tcgaagatgt cattaagaag taccctcgcc tgcgattgac ttttgggagg 900
aaggttttag aaattcgtcc cacgatcaat tgggacaagg ggaaagctct tgtgtttcta 960
cttgaatcac taggtctcag caattgtgat gatgtgctcc ctatttatgt tggagatgac 1020
cggacagatg aagatgcatt taagattttg agagagagga actgtggtta tgggattctg 1080
gtatcaaaat cacccaagga aagcaatgca tattactctc tcagggaccc atccgaggtc 1140
atggagtttc tcaagtccct tgtgatgtgg aagaagtcga gtgctcaata aatgctacaa 1200
ggaatacaca aggaagagga gaatactcta atatacacat acaacatgaa agactttata 1260
tgtttttttc t 1271

<210> 44
<211> 386
<212> PRT
<213> Populus trichocarpa

<400> 44
Met Asp Leu Lys Ser Asn His Ser Ala Pro Val Leu Thr Asp Ser Ala
1 5 10 15
Pro Leu Gly Lys Ser Arg Leu Gly Gly His His Gly Leu Phe Pro Cys
20 25 30
Ser Pro Ser Gly Gly Ala Ala Phe Ser Pro Asn Leu Trp Leu Ser Ile
35 40 45
Pro Lys Lys Lys Thr Gly Val Leu Asp Asp Val Arg Ser Ile Gly Trp
50 55 60
Leu Asp Ala Met Lys Ser Ser Ser Pro Pro His Lys Lys Phe Asn Lys
65 70 75 80
Asp Ile Asn Met Glu Leu Ser Ser Pro Asp Pro Glu Ala Ala Tyr Arg
85 90 95
Thr Trp Leu Leu Lys Tyr Pro Ser Ala Leu Ala Ser Phe Glu Gln Ile
100 105 110
Ala Asn Phe Ala Lys Gly Lys Arg Ile Ala Leu Phe Leu Asp Tyr Asp
115 120 125
Gly Thr Leu Ser Pro Ile Val Glu Asn Pro Asp Asn Ala Leu Met Ser
130 135 140
Asp Val Met Arg Ser Ala Val Lys Lys Val Ala Lys Tyr Phe Pro Thr
145 150 155 160
Ala Ile Ile Ser Gly Arg Ser Arg Asp Lys Val Tyr Glu Phe Val Gly
165 170 175
Leu Thr Glu Leu Tyr Tyr Ala Gly Ser His Gly Met Asp Ile Val Gly
180 185 190
Pro Val Arg His Ser Thr Ser Asp Asp His Pro Asn Cys Ile Glu Ser
195 200 205
Thr Asp Met Gln Gly Asn Glu Val Asn Leu Phe Gln Pro Ala Arg Glu
210 215 220
Phe Leu Pro Met Ile Asp Glu Val Phe Ser Ser Leu Leu Lys Ser Thr
225 230 235 240
Glu Glu Ile Lys Gly Ala Thr Val Glu Asn Asn Lys Phe Cys Val Ser
245 250 255
Val His Tyr Arg Asn Val Asp Glu Asp Lys Trp Lys Ala Val Trp Glu
260 265 270
Cys Val Glu Asp Val Ile Lys Lys Tyr Pro Arg Leu Arg Leu Thr Phe
275 280 285
Gly Arg Lys Val Leu Glu Ile Arg Pro Thr Ile Asn Trp Asp Lys Gly
290 295 300
Lys Ala Leu Val Phe Leu Leu Glu Ser Leu Gly Leu Ser Asn Cys Asp
305 310 315 320
Asp Val Leu Pro Ile Tyr Val Gly Asp Asp Arg Thr Asp Glu Asp Ala
325 330 335
Phe Lys Ile Leu Arg Glu Arg Asn Cys Gly Tyr Gly Ile Leu Val Ser

340 345 350
Lys Ser Pro Lys Glu Ser Asn Ala Tyr Tyr Ser Leu Arg Asp Pro Ser
355 360 365
Glu Val Met Glu Phe Leu Lys Ser Leu Val Met Trp Lys Lys Ser Ser
370 375 380
Ala Gln
385

<210> 45
<211> 1768
<212> DNA
<213> Oryza sativa

<400> 45
agcactccat cttcctccat ctgcgtattt tccaacacat ctctcttgca gttcgtttca 60
agtctttcat taagtaaaac tttctgaaca tttgaagtgc aacggagctg gcgttttatg 120
gcttttccta tgaagcaatc cacatctctg atatagatgt gtactcgttc tgaacatgac 180
aagttctcag gtatttctct gttgatgaac tgcctccaca cctgcagtga caagaagaca 240
ctgaagaagt ggtttttcat cgacaagaca gttggctaaa ttcagcagtg cactcgtcct 300
taaagcgatc gatcatactt ctttcttgat ctcatcatcc taaattttcc caaatggatt 360
tgagcaatag ctcacctgtc atcaccgatc cggtggcgat cagccagcag ttgttgggcg 420
gcctgccttc aaatctgatg cagttttcag tcatgcccgg tggctactcc agctctggca 480
tgaacgttgg tgtcagtagg ctcaaaatcg aggaagtcct tgtcaatgga ctgcttgatg 540
ccatgaaatc ctcgtcacct cgcaggaggc tgaatgtagc atttggcgag gacaattcat 600
ctgaagaaga agaccctgct tacagcgctt ggatggcaaa atgtccttct gctttggctt 660
ccttcaagca aattgtagcc agtgcacaag ggaagaagat tgctgtgttt ctagactatg 720
acggcacact gtcgcctatt gtggatgatc ctgacaaagc agtgatgtct cccgtgatga 780
gagctgctgt gagaaatgtt gcgaagtact tccccactgc aattgtcagc ggaaggtccc 840
gcaataaggt gtttgaattt gtaaaactga aggagcttta ttatgctgga agccatggta 900
tggacataat ggcaccttca gcaaatcatg agcacagtgc tgaaaagagc aaacaggcca 960
atctcttcca acctgcacac gactttctgc caatgatcga tgaggttacc aagtccctct 1020
tgcaagttgt cagtggaatt gaaggtgcaa ctgttgagaa caacaaattc tgcgtttctg 1080
tacattatcg caacgttgca gagaaggatt ggaaactggt cgcacggctc gtaaacgaag 1140
tgctggaggc ttttcctcgt ctcaaagtaa ccaatggacg aatggtttta gaggttcgtc 1200
cggtgatcga ctgggacaag ggaaaggctg tggagtttct gctccagtca ctcgggctaa 1260
atgactctga aaatgtgatc cccatctaca ttggagacga cagaactgac gaagacgctt 1320
tcaaggtact tcgacagcga aattgcggtt atggaatact agtttcacag gttcccaagg 1380
aaactgaagc cttctactcg ctgagagatc catctgaagt gatggagttc ctcaatttct 1440
tggtgagatg gaagaagcac tcagtgtgaa aaaaagaaga cagtctgagc aggttttact 1500
aatactactc cggcagaaat aactgtagtt tccgaggcga caattttgaa gcattggacg 1560
ctgtagatag atgtaataac tctctagata tgtggttcca attcattctt attgatcgat 1620
gttattgtat tgttcatgtt ctccaccgct gagaaaaatt aatcagtgat ggttgatccg 1680
gtctcttgcc attgtaagtt gtaagcaaga aggccctctt cttagtatcc atgctgatga 1740
acgaaattaa aaccttcatt tttcgtgt 1768

<210> 46
<211> 371
<212> PRT
<213> Oryza sativa

<400> 46
Met Asp Leu Ser Asn Ser Ser Pro Val Ile Thr Asp Pro Val Ala Ile
1 5 10 15
Ser Gln Gln Leu Leu Gly Gly Leu Pro Ser Asn Leu Met Gln Phe Ser
20 25 30
Val Met Pro Gly Gly Tyr Ser Ser Ser Gly Met Asn Val Gly Val Ser
35 40 45
Arg Leu Lys Ile Glu Glu Val Leu Val Asn Gly Leu Leu Asp Ala Met
50 55 60

Lys Ser Ser Ser Pro Arg Arg Arg Leu Asn Val Ala Phe Gly Glu Asp
65 70 75 80
Asn Ser Ser Glu Glu Glu Asp Pro Ala Tyr Ser Ala Trp Met Ala Lys
85 90 95
Cys Pro Ser Ala Leu Ala Ser Phe Lys Gln Ile Val Ala Ser Ala Gln
100 105 110
Gly Lys Lys Ile Ala Val Phe Leu Asp Tyr Asp Gly Thr Leu Ser Pro
115 120 125
Ile Val Asp Asp Pro Asp Lys Ala Val Met Ser Pro Val Met Arg Ala
130 135 140
Ala Val Arg Asn Val Ala Lys Tyr Phe Pro Thr Ala Ile Val Ser Gly
145 150 155 160
Arg Ser Arg Asn Lys Val Phe Glu Phe Val Lys Leu Lys Glu Leu Tyr
165 170 175
Tyr Ala Gly Ser His Gly Met Asp Ile Met Ala Pro Ser Ala Asn His
180 185 190
Glu His Ser Ala Glu Lys Ser Lys Gln Ala Asn Leu Phe Gln Pro Ala
195 200 205
His Asp Phe Leu Pro Met Ile Asp Glu Val Thr Lys Ser Leu Leu Gln
210 215 220
Val Val Ser Gly Ile Glu Gly Ala Thr Val Glu Asn Asn Lys Phe Cys
225 230 235 240
Val Ser Val His Tyr Arg Asn Val Ala Glu Lys Asp Trp Lys Leu Val
245 250 255
Ala Arg Leu Val Asn Glu Val Leu Glu Ala Phe Pro Arg Leu Lys Val
260 265 270
Thr Asn Gly Arg Met Val Leu Glu Val Arg Pro Val Ile Asp Trp Asp
275 280 285
Lys Gly Lys Ala Val Glu Phe Leu Leu Gln Ser Leu Gly Leu Asn Asp
290 295 300
Ser Glu Asn Val Ile Pro Ile Tyr Ile Gly Asp Asp Arg Thr Asp Glu
305 310 315 320
Asp Ala Phe Lys Val Leu Arg Gln Arg Asn Cys Gly Tyr Gly Ile Leu
325 330 335
Val Ser Gln Val Pro Lys Glu Thr Glu Ala Phe Tyr Ser Leu Arg Asp
340 345 350
Pro Ser Glu Val Met Glu Phe Leu Asn Phe Leu Val Arg Trp Lys Lys
355 360 365
His Ser Val
370

<210> 47
<211> 1423
<212> DNA
<213> Oryza sativa

<400> 47
ctctcaaact ctcggagaag cgagcacaca cactcactcc ctgtctcctc ctcctcctcc 60
tcccttgagg ctgttcgtct cgacgagtgc ttggctttgt gtttgtggtg tgtgatcatg 120
acgaaccagg acgtggtggt ttctgagatg ggcatcgcgg caggcgcggc gctgccggga 180
ggaccggccg gcccggcggg cggcctcttc gcgtgccgca gcgcggccgc gtcgatgagg 240
cagacgtacc tcgacctcgc cgcggcggcg gtggcggcgc gctccgcgag ctgcaccagc 300
tgggccgacg ccatgcgcgc ctcctccccc acccgctcgt cccgctccgc ctccgacgtc 360
gacgagttca ccgcctgggt gaggaagcac ccgtcggcgc ttagcaagtt cgaggagatc 420
gccgccaagt ccagggggaa gaagatcgta atgttcatgg actacgacgg cacgctctct 480
cccatcgtcg ccgaccccga cacggcctac atgagcgacg cgatgagggc ggcggtgcgc 540
gaagtcgcca agaccttccc gacggcgatc gtcagcgggc ggtgccgcga caaggttcgc 600
aacttcgtcg gcctctccga cctctactac gccggcagcc atggcatgga catcaaggga 660
ccaagctcca acccggagtc tgccctttgc cagccagcaa gcgagttcct ccccatgatc 720

```
gacgaggtgt acaagacgct ggtggaaaag acgaaatcta cacctggagc caaggtggag     780
aacaacaagt tctgcctgtc cgtccacttc agatgtgtag atgaaaagag atggaatgct     840
ttgggggagc aggtcaaggc cgtgatcaag gaatacccaa agttgaagct tacccaagga     900
aggaaggttt tggagatcag gccgagcatt gagtgggaca agggcaaggc tctggagttc     960
ttgcttgaat cgctgggatt cgccaactgc ggcgacgtca tgccggtgta catcggtgac    1020
gaccgcaccg acgaggacgc cttcaaggtg ttgagaaaga gaggtcaggg cctgggaatc    1080
ctagtctcca agtgccccaa ggacaccaac gcctcctact ctctccagga cccaacagag    1140
gtgatggagt tcttgctcag gctggtggag tggaagcgga agtcgtcgtc gtcgtcgttg    1200
atgattcgtc cgagagtgta gcggcgacac ggtgtagagg ccgtaagaaa ctgaaactaa    1260
gatggtcaat catgcgaaca tgcgaacata ccccaccaac accacgtact acccccattt    1320
ttttccgatg atttctgtgg ttggcctctg cgacggactc gtggtggtta cacgctagct    1380
gttcgattcc gtcccctgtg ccgccgagga aaaggagacc acc                      1423
```

```
<210>  48
<211>  367
<212>  PRT
<213>  Oryza sativa

<400>  48
Met Thr Asn Gln Asp Val Val Val Ser Glu Met Gly Ile Ala Ala Gly
1               5                   10                  15
Ala Ala Leu Pro Gly Gly Pro Ala Gly Pro Ala Gly Gly Leu Phe Ala
            20                  25                  30
Cys Arg Ser Ala Ala Ala Ser Met Arg Gln Thr Tyr Leu Asp Leu Ala
        35                  40                  45
Ala Ala Ala Val Ala Ala Arg Ser Ala Ser Cys Thr Ser Trp Ala Asp
    50                  55                  60
Ala Met Arg Ala Ser Ser Pro Thr Arg Ser Ser Arg Ser Ala Ser Asp
65                  70                  75                  80
Val Asp Glu Phe Thr Ala Trp Val Arg Lys His Pro Ser Ala Leu Ser
                85                  90                  95
Lys Phe Glu Glu Ile Ala Ala Lys Ser Arg Gly Lys Lys Ile Val Met
            100                 105                 110
Phe Met Asp Tyr Asp Gly Thr Leu Ser Pro Ile Val Ala Asp Pro Asp
        115                 120                 125
Thr Ala Tyr Met Ser Asp Ala Met Arg Ala Ala Val Arg Glu Val Ala
    130                 135                 140
Lys Thr Phe Pro Thr Ala Ile Val Ser Gly Arg Cys Arg Asp Lys Val
145                 150                 155                 160
Arg Asn Phe Val Gly Leu Ser Asp Leu Tyr Tyr Ala Gly Ser His Gly
                165                 170                 175
Met Asp Ile Lys Gly Pro Ser Ser Asn Pro Glu Ser Ala Leu Cys Gln
            180                 185                 190
Pro Ala Ser Glu Phe Leu Pro Met Ile Asp Glu Val Tyr Lys Thr Leu
        195                 200                 205
Val Glu Lys Thr Lys Ser Thr Pro Gly Ala Lys Val Glu Asn Asn Lys
    210                 215                 220
Phe Cys Leu Ser Val His Phe Arg Cys Val Asp Glu Lys Arg Trp Asn
225                 230                 235                 240
Ala Leu Gly Glu Gln Val Lys Ala Val Ile Lys Glu Tyr Pro Lys Leu
                245                 250                 255
Lys Leu Thr Gln Gly Arg Lys Val Leu Glu Ile Arg Pro Ser Ile Glu
            260                 265                 270
Trp Asp Lys Gly Lys Ala Leu Glu Phe Leu Leu Glu Ser Leu Gly Phe
        275                 280                 285
Ala Asn Cys Gly Asp Val Met Pro Val Tyr Ile Gly Asp Asp Arg Thr
    290                 295                 300
Asp Glu Asp Ala Phe Lys Val Leu Arg Lys Arg Gly Gln Gly Leu Gly
305                 310                 315                 320
```

```
Ile Leu Val Ser Lys Cys Pro Lys Asp Thr Asn Ala Ser Tyr Ser Leu
                325                 330                 335
Gln Asp Pro Thr Glu Val Met Glu Phe Leu Leu Arg Leu Val Glu Trp
            340                 345                 350
Lys Arg Lys Ser Ser Ser Ser Ser Leu Met Ile Arg Pro Arg Val
        355                 360                 365
```

<210> 49
<211> 1511
<212> DNA
<213> Oryza sativa

<400> 49

```
cacaaccaaa tcaacaacca cgccattgcc gttctctcct cctttccttc cacaacccat      60
ctcttcctca tcctcctcct cctcttcctg ctgctctctc gctctcgttg gattcggttt     120
ggtttggttt gggccatgac gaagcacggc gcggtggttg tcccggtcgc cgccgccgcc     180
gccgacgcgg agcacgacga gtggatggag aagcacccgt cggcattggg gaagttcgag     240
gcgctggcgg cggcggcgaa ggggaagcgg atcgtcgtct tcctcgacta cgacggcacg     300
ctgtcgccga tcgtcgagga ccccgaccgc gccgtcatga cggacgagat gagggacgcc     360
gtgcgcggcg tggcggcgcg attcccaacg gcgatcgtca gcggccgctg cagagacaag     420
gtgttgagct tcgtggggct ggaggagctc tactacgcgg gtagccacgg gatggacatc     480
caagggccca ccaacgccgc cgcctccaag ggaggagagg aggaggagga gtcggtgctg     540
tgccagccgg cgagggagtt cctgccgatg atcggggagg cgtacgcggc gctggtggag     600
aaggtggagg gggtgatccc gggggcgaag gtggagaaca acaagttctg cctctccgtc     660
cacttccgcc gcgtcgacga gcgccggtgg ggcgccgtcg ccgaccaggt cagggcggtg     720
ctccggggct acccgcgcct ccgcctcacg cagggccgca aggtgctcga ggtcaggccc     780
gccatcaagt gggacaaggg cgaggccctc cgcttcctcc tctccgccct cggcttctcc     840
gccgccggag acgtagaaga cgacggcgac gacgacgacg cgttccccat ctacatcggc     900
gacgaccgca ccgacgagga cgcgttccgg gtgctccgag cgagggggca cggcgccggc     960
atcctggtgt cgaggttccc caaggacacc tgcgcctcct tctccctccg cgacccccggc    1020
gaggtcaagg acttcctccg caagctcgtc acctgcgctg ccgcatgacg tcaccttcac    1080
ctgaccacag tgacactaat tggcttcctc ctcctccacg ggaaagagaa agagagagag    1140
agaaataatt aaggatcttg attagtcaat tagcggttac taatccctgt attaatttta    1200
gccggtcact tcatttcttc ttcttctttt tttatgtgtt tctgttcttg ctctgcggct    1260
gccatcttga ttgatcgatc gatctcacag gccacagtgt gacagctcga tctgaaggtt    1320
tctctattat ccttaacctt cctccagctg atccaaggcc ttgtaattag tcgtcaattt    1380
ttagcagttt gttgtctgtc tgtctgtcac tgaaacggga cgaattaatc cgaattaata    1440
cgatccattt ccacctaatt tcagtttgta ctgtaccgtt taatttccat ctaaattcag    1500
ctttgcactg t                                                          1511
```

<210> 50
<211> 310
<212> PRT
<213> Oryza sativa

<400> 50

```
Met Thr Lys His Gly Ala Val Val Val Pro Val Ala Ala Ala Ala Ala
1               5                   10                  15
Asp Ala Glu His Asp Glu Trp Met Glu Lys His Pro Ser Ala Leu Gly
            20                  25                  30
Lys Phe Glu Ala Leu Ala Ala Ala Ala Lys Gly Lys Arg Ile Val Val
        35                  40                  45
Phe Leu Asp Tyr Asp Gly Thr Leu Ser Pro Ile Val Glu Asp Pro Asp
    50                  55                  60
Arg Ala Val Met Thr Asp Glu Met Arg Asp Ala Val Arg Gly Val Ala
65                  70                  75                  80
Ala Arg Phe Pro Thr Ala Ile Val Ser Gly Arg Cys Arg Asp Lys Val
                85                  90                  95
Leu Ser Phe Val Gly Leu Glu Glu Leu Tyr Tyr Ala Gly Ser His Gly
```

```
                100                 105                 110
Met Asp Ile Gln Gly Pro Thr Asn Ala Ala Ala Ser Lys Gly Gly Glu
            115                 120                 125
Glu Glu Glu Glu Ser Val Leu Cys Gln Pro Ala Arg Glu Phe Leu Pro
    130                 135                 140
Met Ile Gly Glu Ala Tyr Ala Ala Leu Val Glu Lys Val Glu Gly Val
145                 150                 155                 160
Ile Pro Gly Ala Lys Val Glu Asn Asn Lys Phe Cys Leu Ser Val His
                165                 170                 175
Phe Arg Arg Val Asp Glu Arg Arg Trp Gly Ala Val Ala Asp Gln Val
            180                 185                 190
Arg Ala Val Leu Arg Gly Tyr Pro Arg Leu Arg Leu Thr Gln Gly Arg
        195                 200                 205
Lys Val Leu Glu Val Arg Pro Ala Ile Lys Trp Asp Lys Gly Glu Ala
    210                 215                 220
Leu Arg Phe Leu Leu Ser Ala Leu Gly Phe Ser Ala Ala Gly Asp Val
225                 230                 235                 240
Glu Asp Asp Gly Asp Asp Asp Asp Ala Phe Pro Ile Tyr Ile Gly Asp
                245                 250                 255
Asp Arg Thr Asp Glu Asp Ala Phe Arg Val Leu Arg Ala Arg Gly His
            260                 265                 270
Gly Ala Gly Ile Leu Val Ser Arg Phe Pro Lys Asp Thr Cys Ala Ser
        275                 280                 285
Phe Ser Leu Arg Asp Pro Gly Glu Val Lys Asp Phe Leu Arg Lys Leu
    290                 295                 300
Val Thr Cys Ala Ala Ala
305                 310
```

<210> 51
<211> 1791
<212> DNA
<213> Oryza sativa

<400> 51

```
atgacgaacc aggacgtggt gatgccggac atcgcggcgg ccgcggccat gccggggtca     60
tccggccgcg cgccgctctt cgcgtgccgt ggcgccgcgg ccgtgtcggc gtcctccatg    120
ctcggcggcg gcggcgcggc gtaccaggcc gcggtggtgg cgcacgtggc gccggtgcca    180
gctatcaggc cgtgcgccag ctgggtcgtc gaggccatgc gcgcgtcgtc gccgacacgg    240
cccgccgccg ccgcggtcga cgccgagtac gacgcgtgga cgcagaggaa gcacccgtcg    300
gcgctgggca gcttcgagca ggtggccgcg gcggcgagcg ggaagcgcgt cgtcgtgttc    360
ctcgactacg acggcaccct ctcccccatc gtcgccgacc ccgacatggc cttcatgtcc    420
gacgagatga gggcggccgt gcgcgacgtg gcggagcact tcccggcggc gatcgtgacc    480
gggcgctgcg tcgacaaggt gcagagcttc gtcggcctcc cggagctcta ctacgccggc    540
agccatggca tggacatcaa gggccccagc tccaacgagg aagaagacac caagatcctc    600
ttgcagccag ctcgcgagtt cctcccggtg atcaacaagg cttacaaggc tttgatggag    660
aagacgaaga gcacgcctgg ggccagagtg gagaacaaca agttctgcct ctccgtgcac    720
ttcagatgcg tcgatgagaa gagatggaat ccgttggcag agcaagtgaa ggccgtgctc    780
cgggactacc ccgagctcaa actcacccaa ggaagaaagg tcctggagat ccggccgtcg    840
atcatgtggg acaagggcaa ggcggtggag ttcttgctga aatctctcgg attcgacgac    900
gaccgccgcg acgtcctgcc ggtgtacatc ggagacgacc ggaccgacga agatgctttc    960
aaggtgttga gaaagagagg tcaagggttg ggaattcttg tctccaagtg tgccaaggag   1020
actgacgcct cgtactctct ccaggaccca gcagagaagt acaccaacgc cggggcgcac   1080
gtgttcgtga cgatgctcct gaccgtggtg ttcacggcag ctgtggcgtt ggctcttgtc   1140
aacgccgtca actcgcatga cttcgcagca cacctcgccg gcgtggactg ccgcatgggg   1200
ctagctggtc cagtacgatg cccagcctcg ggcttcgtgg agctcctcgt gctggcgcta   1260
catgtgatgc gtgctcgcca tcttagatcg cctgcatgcc tgcctaatgt cgccctcgct   1320
acagctttcg attgcatctc catgtacgtg cgccggtggc agcgcggcga agtgaccaat   1380
gtacatgaag tatacgtgct aagtaggatc gaatttgttc cttatatggc aagcaccggt   1440
gaacaaagaa gtactggcga gacggccgtc ggcgccgccg ccgccggaat gtatcggatg   1500
```

```
gtgcccttgc acgcggcaaa atggggagac aaatggagaa tgagtgggga aagaatggcc   1560
gccggagtag actgccggcg gcgcgaggat ttgccgagca gtggagatgg ctaccgtcag   1620
gaggagatcg acggtggtca tggagttcct tgttcgattg gtgcagtgga agcttcggcg   1680
atcatcgtca gcgatgcgcc cgagggttta attagtacac tgacgacgga tcatatatac   1740
tatatgacag tcttccctgc taagctagct ctgacttctg aagaagtttg a            1791

<210>  52
<211>  596
<212>  PRT
<213>  Oryza sativa

<400>  52
Met Thr Asn Gln Asp Val Val Met Pro Asp Ile Ala Ala Ala Ala Ala
1               5                   10                  15
Met Pro Gly Ser Ser Gly Arg Ala Pro Leu Phe Ala Cys Arg Gly Ala
            20                  25                  30
Ala Ala Val Ser Ala Ser Ser Met Leu Gly Gly Gly Gly Ala Ala Tyr
        35                  40                  45
Gln Ala Ala Val Val Ala His Val Ala Pro Val Pro Ala Ile Arg Pro
    50                  55                  60
Cys Ala Ser Trp Val Val Glu Ala Met Arg Ala Ser Ser Pro Thr Arg
65                  70                  75                  80
Pro Ala Ala Ala Ala Val Asp Ala Glu Tyr Asp Ala Trp Thr Gln Arg
                85                  90                  95
Lys His Pro Ser Ala Leu Gly Ser Phe Glu Gln Val Ala Ala Ala Ala
            100                 105                 110
Ser Gly Lys Arg Val Val Val Phe Leu Asp Tyr Asp Gly Thr Leu Ser
        115                 120                 125
Pro Ile Val Ala Asp Pro Asp Met Ala Phe Met Ser Asp Glu Met Arg
    130                 135                 140
Ala Ala Val Arg Asp Val Ala Glu His Phe Pro Ala Ala Ile Val Thr
145                 150                 155                 160
Gly Arg Cys Val Asp Lys Val Gln Ser Phe Val Gly Leu Pro Glu Leu
                165                 170                 175
Tyr Tyr Ala Gly Ser His Gly Met Asp Ile Lys Gly Pro Ser Ser Asn
            180                 185                 190
Glu Glu Glu Asp Thr Lys Ile Leu Leu Gln Pro Ala Arg Glu Phe Leu
        195                 200                 205
Pro Val Ile Asn Lys Ala Tyr Lys Ala Leu Met Glu Lys Thr Lys Ser
    210                 215                 220
Thr Pro Gly Ala Arg Val Glu Asn Asn Lys Phe Cys Leu Ser Val His
225                 230                 235                 240
Phe Arg Cys Val Asp Glu Lys Arg Trp Asn Pro Leu Ala Glu Gln Val
                245                 250                 255
Lys Ala Val Leu Arg Asp Tyr Pro Glu Leu Lys Leu Thr Gln Gly Arg
            260                 265                 270
Lys Val Leu Glu Ile Arg Pro Ser Ile Met Trp Asp Lys Gly Lys Ala
        275                 280                 285
Val Glu Phe Leu Leu Lys Ser Leu Gly Phe Asp Asp Asp Arg Arg Asp
    290                 295                 300
Val Leu Pro Val Tyr Ile Gly Asp Asp Arg Thr Asp Glu Asp Ala Phe
305                 310                 315                 320
Lys Val Leu Arg Lys Arg Gly Gln Gly Leu Gly Ile Leu Val Ser Lys
                325                 330                 335
Cys Ala Lys Glu Thr Asp Ala Ser Tyr Ser Leu Gln Asp Pro Ala Glu
            340                 345                 350
Lys Tyr Thr Asn Ala Gly Ala His Val Phe Val Thr Met Leu Leu Thr
        355                 360                 365
Val Val Phe Thr Ala Ala Val Ala Leu Ala Leu Val Asn Ala Val Asn
```

```
    370                 375                 380
Ser His Asp Phe Ala Ala His Leu Ala Gly Val Asp Cys Arg Met Gly
385                 390                 395                 400
Leu Ala Gly Pro Val Arg Cys Pro Ala Ser Gly Phe Val Glu Leu Leu
                405                 410                 415
Val Leu Ala Leu His Val Met Arg Ala Arg His Leu Arg Ser Pro Ala
            420                 425                 430
Cys Leu Pro Asn Val Ala Leu Ala Thr Ala Phe Asp Cys Ile Ser Met
        435                 440                 445
Tyr Val Arg Arg Trp Gln Arg Gly Glu Val Thr Asn Val His Glu Val
    450                 455                 460
Tyr Val Leu Ser Arg Ile Glu Phe Val Pro Tyr Met Ala Ser Thr Gly
465                 470                 475                 480
Glu Gln Arg Ser Thr Gly Glu Thr Ala Val Gly Ala Ala Ala Ala Gly
                485                 490                 495
Met Tyr Arg Met Val Pro Leu His Ala Ala Lys Trp Gly Asp Lys Trp
            500                 505                 510
Arg Met Ser Gly Glu Arg Met Ala Ala Gly Val Asp Cys Arg Arg Arg
        515                 520                 525
Glu Asp Leu Pro Ser Ser Gly Asp Gly Tyr Arg Gln Glu Glu Ile Asp
    530                 535                 540
Gly Gly His Gly Val Pro Cys Ser Ile Gly Ala Val Glu Ala Ser Ala
545                 550                 555                 560
Ile Ile Val Ser Asp Ala Pro Glu Gly Leu Ile Ser Thr Leu Thr Thr
                565                 570                 575
Asp His Ile Tyr Tyr Met Thr Val Phe Pro Ala Lys Leu Ala Leu Thr
            580                 585                 590
Ser Glu Glu Val
        595

<210>  53
<211>  3384
<212>  DNA
<213>  Oryza sativa

<400>  53
ggttgatcga ggtgagcgtt tcctgaccta cttgctcgga ttaattacaa attgatttga     60
tcacgtagat aattacatca tcatcatatt gatggatgga tgcttgcaca tatatgcatg    120
cgttttgaat ctggtgatgt aatgatatcc aattaataat cttgctgcaa cacgaaagct    180
cagccagttt aattgatttg atttagttaa tgccatcgat ctcaggcttg attatcgatt    240
ggctgttgaa tgcacggatt aatcaaatag gttgaaggca cgcaatgcaa aattccaaat    300
tgattgtgga cctcttcggt ttctccttgt ataagacctt gaatttagaa attattagtg    360
agctatcgaa agttttcagt ggcatcacat attatctgct tacttggaac atatagaaaa    420
atagaattat taattaataa attaattgta ttgagcatta tacttcctta gtctgagagt    480
atatagcatt gggcataaag ttagtattaa ctattcacca tgtaactagc ttcttagtaa    540
aggtacaaat taaggactga agagaaactc cagactttta atcttcaaac tgaaggcaag    600
taatacattt tcagcccttc catttagttc cttggaggat caaattcaaa tgttgcgacg    660
gatgaagacg cacgaaattt gatgctttta tcgcacgttc atgtagtatt tggtcaatcg    720
atctctgcat catgccttga ccaagatgca tatttagttt gatgatctct gttgcttcca    780
attgcactcc attaattgtt tgaactaata gtcgttttgc ttcatgtaat cgaatgcttc    840
ctgcgttcga ttggtgagag aagtgcactc caatgctgta ccaacagtac tcttgggttt    900
ttctcttagg agagctttat ttagagtgaa agggacttcc taattaagat ttctttattt    960
tgaaagaagc tagtaattaa gatttcttaa catatgcaca cgtgaagtta taaaacatga   1020
ctcaaatctg atgttgatgt gtgttcgacc aagtccctaa tcagtttttg cacttttct   1080
tctgatttct cacgccaact gatcaattaa ggcaagtagg attttagttg tgttcttttc   1140
ttattgtttt cctttctctt gcttttcgct ttctcacctc ctacctcctt ggagacagtc   1200
cacaattgtg aattttcaac attagaaaaa taatcacaat tttgtggaaa tgattgaaaa   1260
actaattaat aggttgcatt tcacatgaac atagttaatt agagaacaaa atgcatacac   1320
taggtgttag agcagtacaa aagttataga agatgtcact gcttactgca tgagtatctt   1380
```

tcttcacaat cccattccat cataaatatt gcctaaacaa ttccactcaa ccttgttgct 1440
tgttcaactt taattctttc ataacttgat atgaaggtca ggacaattac tgtagactag 1500
tttcttcatg gttgcacgct gaataatcgt acacagatgc ccttgctgtc tctgaattct 1560
caaacactga acatatattg tagaacattt tacacctatt cctgtctgat ctcttgctgt 1620
gtgtgtacgg agcatgctgc cgttcttccc aagactttgt gtctcaattg gttatttttg 1680
acttgaagcg atccgtttcg gtagtccctg aggcagctag cgtaagcctc ttttgacttc 1740
caagcacaca agttacttgc tgcctcagat tcatcagcaa gtcactagta cttttcggta 1800
tctagatgaa gcatatatat aaaccacaag aaaagcggct gcccatttgt gttctgatca 1860
tgaggagatt caggtggttt cttgaaagga tgtgaatctg tggttgcaag atactgaaga 1920
aagtaataag gtggttcttc aatcttcata ggcttcagag ttcagagttg agttaatgtt 1980
tttgcaaaca aggtgtctgc tgacagtttg acagtaaaaa gatctgtata tatgggttcc 2040
tgcggtaacg gtagaagctc tgaatatgat gatcctgcat cattagagaa gatggaggaa 2100
ctggttttac ctctgaaatt gatgccctta cacaccaatg gccggttgta tgacatgagg 2160
ttgtcctcac ctactgctac ctgtgtgatc aatagttcat ctggttcatt cgacccgatc 2220
taccgagctt ggacgaagaa gtatccttca gctctaaatg cctttgacca cattgttgcc 2280
tatggcaaag ggaagaagat agcgctgttt ttggactacg acggtacact gtcgccaatt 2340
gtcgatgaac ctgacaatgc tatcatgtct gaccagatgc gcgaggtggt gaggaatgct 2400
gccttgcatc tacccactgc gattatcagt ggaaggtctc gtgataaggt gtttgatttt 2460
gttaaactaa cagaattgta ctatgctggt agccatggaa tggacatcat gggtccagtt 2520
ggggaacatg attcagttac taaccatagg agctccatta actcaaatag aaagcagggc 2580
aagggagtga agatcttcca ggctggtact gaattcttac caatgatcaa tgaggttttt 2640
agattgctca tcgataagac aaaggcaatc gacggtgtga aaattgagaa caacaagttc 2700
tgtgtatcgg tgcactaccg caatgttgag gagaagaatt ggcaacttgt ttctcaatgc 2760
acaaatgatg tcctaaaagt ctaccctcgc ctccgattga ctcatggacg aaaagttttg 2820
gaaattcgtc cagtgataga ctggaacaag ggaaaggctg tggagttctt gctggactcc 2880
ctagatctag ctagctgtaa aaatgttctc cctatttaca tcggggatga ttgtaccgac 2940
gaggatgctt ttaaggttct tcgtgacgat aaaaggggtt tcgggattct ggtgtcatct 3000
gtaccaaagg attctcatgc cctatattct ctgattgatc catctgaggt tatggaattc 3060
ttgaaaagac tagtgatgtg gaagaacgaa gaagcatcac ataataagta gattataaat 3120
atatctgatg tttactctag ggtagatttc tgaattctga caaagagaat aagcagaagg 3180
gcaacagaat taacttggac atagtggaca gttgtacatc atatagttaa tccattttct 3240
tagcctagca agtggcaaaa taaaagtaat tgccatgttg tacaagaatg cgaagcattc 3300
tgcacaagtc cagttctttg aataatggtc tcagttgtag aatggtgtaa cttgttaatg 3360
aactgtgata taaatttttg ggct 3384

<210> 54
<211> 359
<212> PRT
<213> Oryza sativa

<400> 54
Met Gly Ser Cys Gly Asn Gly Arg Ser Ser Glu Tyr Asp Asp Pro Ala
1 5 10 15
Ser Leu Glu Lys Met Glu Glu Leu Val Leu Pro Leu Lys Leu Met Pro
20 25 30
Leu His Thr Asn Gly Arg Leu Tyr Asp Met Arg Leu Ser Ser Pro Thr
35 40 45
Ala Thr Cys Val Ile Asn Ser Ser Ser Gly Ser Phe Asp Pro Ile Tyr
50 55 60
Arg Ala Trp Thr Lys Lys Tyr Pro Ser Ala Leu Asn Ala Phe Asp His
65 70 75 80
Ile Val Ala Tyr Gly Lys Gly Lys Lys Ile Ala Leu Phe Leu Asp Tyr
85 90 95
Asp Gly Thr Leu Ser Pro Ile Val Asp Glu Pro Asp Asn Ala Ile Met
100 105 110
Ser Asp Gln Met Arg Glu Val Val Arg Asn Ala Ala Leu His Leu Pro
115 120 125
Thr Ala Ile Ile Ser Gly Arg Ser Arg Asp Lys Val Phe Asp Phe Val
130 135 140

```
Lys Leu Thr Glu Leu Tyr Tyr Ala Gly Ser His Gly Met Asp Ile Met
145                 150                 155                 160
Gly Pro Val Gly Glu His Asp Ser Val Thr Asn His Arg Ser Ser Ile
                165                 170                 175
Asn Ser Asn Arg Lys Gln Gly Lys Gly Val Lys Ile Phe Gln Ala Gly
            180                 185                 190
Thr Glu Phe Leu Pro Met Ile Asn Glu Val Phe Arg Leu Leu Ile Asp
        195                 200                 205
Lys Thr Lys Ala Ile Asp Gly Val Lys Ile Glu Asn Asn Lys Phe Cys
    210                 215                 220
Val Ser Val His Tyr Arg Asn Val Glu Glu Lys Asn Trp Gln Leu Val
225                 230                 235                 240
Ser Gln Cys Thr Asn Asp Val Leu Lys Val Tyr Pro Arg Leu Arg Leu
                245                 250                 255
Thr His Gly Arg Lys Val Leu Glu Ile Arg Pro Val Ile Asp Trp Asn
            260                 265                 270
Lys Gly Lys Ala Val Glu Phe Leu Leu Asp Ser Leu Asp Leu Ala Ser
        275                 280                 285
Cys Lys Asn Val Leu Pro Ile Tyr Ile Gly Asp Asp Cys Thr Asp Glu
    290                 295                 300
Asp Ala Phe Lys Val Leu Arg Asp Asp Lys Arg Gly Phe Gly Ile Leu
305                 310                 315                 320
Val Ser Ser Val Pro Lys Asp Ser His Ala Leu Tyr Ser Leu Ile Asp
                325                 330                 335
Pro Ser Glu Val Met Glu Phe Leu Lys Arg Leu Val Met Trp Lys Asn
            340                 345                 350
Glu Glu Ala Ser His Asn Lys
        355

<210>  55
<211>  1517
<212>  DNA
<213>  Oryza sativa

<400>  55
accttcccct gtcatctcct cctcctccac caccacctcg ccgtcgccgt caccgcgcca      60
ccgtctcctc gatctctcac tcccacagct cgctggttgc ttcggcttcc gctgctcggt     120
cgcggttgat tttgggggat gacgaaccac gccggcttcg ccgcggacga cgcggtcacc     180
gcggccgtgc cggtgcaggc ggcgcagggc gggcggcatt tcccgccgtt cctggcgccg     240
tcgtccaggc tcaccgactg caagaaggcg gcggcgcacg tggacctcgc cggcgcgggc     300
ggggtggcga cggtgcccgg atcttggccg cgccacgcca agcccgtctc cggcgccgag     360
ctcgacgact ggatggagaa gcacccgtcg gcattggcat ggttcgagtc cgtcgccgcc     420
gcggcgaagg gcaaggagat cgtcgtgttc ctcgactacg acggcaccct ctcccccatc     480
gtcgccgacc ccgaccgcgc cttcatgtcc gacgaaatga gagaggcggt gagaggcgtg     540
gcgaagcact tcccgacggc gatcgtgagc gggaggtgca tcgacaaggt gttcgacttt     600
gtgaagctgg aggagctgta ctacgccggg agccatggaa tggacatcag ggccccccacc     660
gcggcagcgt cggagtacaa ccacaacatg aaggcaaagc agggtgatgc tgttactttc     720
cagccggccg ccgatttcct gcccgtcatc gaggaggtgt atcatgtgct gaaggagagg     780
atggcgagta taagggggttc gctggtggag aacaacaagt tttgcctttc cgtgcactac     840
cgctgcgtcg acgaggcgga atggggcgtg ctggacggca aggtgagggc ggtgatagag     900
ggctacccgg atctccgtct cagcaagggg agaaaggtgc tggagatccg ccctgtcatc     960
gactgggaca aaggctccgc actccagttc ctgctcaaat ctctcggtta tgaggggcgc    1020
aacaatgttt tcccgatata cattggagat gaccgcaccg acgaggatgc tttcaaggtg    1080
ttgcgcaaca tgggacaggg cataggaatc cttgtgacca aggtcccaaa ggagacagct    1140
gcatcctaca ctctgcgaga gccatccgag gtgaaggagt tcctgcgcaa gttggtgaag    1200
attaagatca acggggacaa agggctgatt ggcaagtagc tgatccaagg catatagcta    1260
gctagctagc tttttgttcc ttaatttgtc tctttccttt tggtccatta gaagaagaag    1320
aagaacaaca acaagagtta tatgcatgga tggtaactga tcatgcatga tcgatgcatg    1380
cggtggtgaa aacccgggct tttggctttg aaggtgtcta ttaccgtatc cttctggcca    1440
```

```
aggtcctgta attaaatttt gctgccgtga caacagaaat ctcagcaaga gggacacaag   1500
tgtggcccct tttcttt                                                  1517

<210>  56
<211>  366
<212>  PRT
<213>  Oryza sativa

<400>  56
Met Thr Asn His Ala Gly Phe Ala Ala Asp Asp Ala Val Thr Ala Ala
1               5                   10                  15
Val Pro Val Gln Ala Ala Gln Gly Gly Arg His Phe Pro Pro Phe Leu
            20                  25                  30
Ala Pro Ser Ser Arg Leu Thr Asp Cys Lys Lys Ala Ala Ala His Val
        35                  40                  45
Asp Leu Ala Gly Ala Gly Gly Val Ala Thr Val Pro Gly Ser Trp Pro
    50                  55                  60
Arg His Ala Lys Pro Val Ser Gly Ala Glu Leu Asp Asp Trp Met Glu
65                  70                  75                  80
Lys His Pro Ser Ala Leu Ala Trp Phe Glu Ser Val Ala Ala Ala Ala
                85                  90                  95
Lys Gly Lys Glu Ile Val Val Phe Leu Asp Tyr Asp Gly Thr Leu Ser
            100                 105                 110
Pro Ile Val Ala Asp Pro Asp Arg Ala Phe Met Ser Asp Glu Met Arg
        115                 120                 125
Glu Ala Val Arg Gly Val Ala Lys His Phe Pro Thr Ala Ile Val Ser
    130                 135                 140
Gly Arg Cys Ile Asp Lys Val Phe Asp Phe Val Lys Leu Glu Glu Leu
145                 150                 155                 160
Tyr Tyr Ala Gly Ser His Gly Met Asp Ile Arg Gly Pro Thr Ala Ala
                165                 170                 175
Ala Ser Glu Tyr Asn His Asn Met Lys Ala Lys Gln Gly Asp Ala Val
            180                 185                 190
Thr Phe Gln Pro Ala Ala Asp Phe Leu Pro Val Ile Glu Glu Val Tyr
        195                 200                 205
His Val Leu Lys Glu Arg Met Ala Ser Ile Arg Gly Ser Leu Val Glu
    210                 215                 220
Asn Asn Lys Phe Cys Leu Ser Val His Tyr Arg Cys Val Asp Glu Ala
225                 230                 235                 240
Glu Trp Gly Val Leu Asp Gly Lys Val Arg Ala Val Ile Glu Gly Tyr
                245                 250                 255
Pro Asp Leu Arg Leu Ser Lys Gly Arg Lys Val Leu Glu Ile Arg Pro
            260                 265                 270
Val Ile Asp Trp Asp Lys Gly Ser Ala Leu Gln Phe Leu Leu Lys Ser
        275                 280                 285
Leu Gly Tyr Glu Gly Arg Asn Asn Val Phe Pro Ile Tyr Ile Gly Asp
    290                 295                 300
Asp Arg Thr Asp Glu Asp Ala Phe Lys Val Leu Arg Asn Met Gly Gln
305                 310                 315                 320
Gly Ile Gly Ile Leu Val Thr Lys Val Pro Lys Glu Thr Ala Ala Ser
                325                 330                 335
Tyr Thr Leu Arg Glu Pro Ser Glu Val Lys Glu Phe Leu Arg Lys Leu
            340                 345                 350
Val Lys Ile Lys Ile Asn Gly Asp Lys Gly Leu Ile Gly Lys
        355                 360                 365

<210>  57
<211>  1395
<212>  DNA
```

<213> Oryza sativa

<400> 57

```
atggcgaagg cgagcgtggt ggtgcctgag caggtgggcg cggcggcggc ggcgcaggtg      60
gggtgcccct gtccgggcac gacgctgttc ccgtacccgc cgccgcgcgc cgggatcgcc     120
gtgcggcgca agtgcctgca ggcggcgcag cagctggagc tcggcgccgg gctgcgcggc     180
ggctgggtgg agtccatgcg ggcgtcgtcg cccacccacg ccaaggccgc cgccgccctc     240
gccgccggcg tcgacgagga gcacgccgcc tggatggtcc gtttccgttc accgattgat     300
cgatgttcgt cgcgttcttg gcgcgcgcgc gctgacactg acatgaaccg tgcatttccg     360
ttcgtctttg tgcaggcgag gcacccgtcg gcgctgggcg agttcgagaa ggtggtggcg     420
gcgtcgaagg ggaagcagat cgtcatgttc ctcgactacg acggcaccct ctccccccatc    480
gtcgacgacc ccgacgccgc cttcatgagc gagacggtga gcttgagctc ccctcccctg     540
tcacctactc tgctcctcca ctcatcatca tctcacacct ctctccttcc tcatcagatg     600
cggatggccg tgcgcagcgt ggcgaagcac ttcccgacgg cgatcgtgag cgggcggtgc     660
cgcgacaagg tgttcgagtt cgtgaagctc gccgagctgt actacgcggg gagccacggc     720
atggacatca agggccccgc ctcccgccac gccgccgcca agtctcctcc ccacaacaag     780
ggagtcctct tccagccggc cagcgagttc ctccccatga tcgagcaggt gcaccagcga     840
ctcgagcagg ccaccagctc catcccgggc gccaaggtcg agaacaacaa gttctgcgtc     900
tccgtccact tccggtgcgt cgacgagaag agttgggggg cgttggcgga gacggtgagg     960
agggtggtga gggagttccc gcggctgcgg ctgagccagg ggaggatggt gttcgaggtg    1020
cggccgacca tcaagtggga caagggcaag gccctcgagt tcctcctcga ctcgctcggt    1080
ttcgccgact gcagcgacgt gctgccggtc tacatcggcg acgaccgcac ggacgaggac    1140
gcgttcaagg ttttgcggcg gcgtgggcag ggcgtgggga tcctggtgtc caagcacccc    1200
aaggagacga gcgcctcctt ctccctccag gagcccgccg agctgaagaa aagctcagtg    1260
cactacccac tgccggcaca acgtagcaac aggctgaacc gcatattggt caattggtgg    1320
ggtgaaggac ttaaggacaa attgtgtgct ttggagccaa gccatgagag ggaggggtgc    1380
atggcccccc actag                                                     1395
```

<210> 58
<211> 464
<212> PRT
<213> Oryza sativa

<400> 58

```
Met Ala Lys Ala Ser Val Val Val Pro Glu Gln Val Gly Ala Ala Ala
1               5                   10                  15
Ala Ala Gln Val Gly Cys Pro Cys Pro Gly Thr Thr Leu Phe Pro Tyr
            20                  25                  30
Pro Pro Pro Arg Ala Gly Ile Ala Val Arg Arg Lys Cys Leu Gln Ala
        35                  40                  45
Ala Gln Gln Leu Glu Leu Gly Ala Gly Leu Arg Gly Gly Trp Val Glu
    50                  55                  60
Ser Met Arg Ala Ser Ser Pro Thr His Ala Lys Ala Ala Ala Ala Leu
65                  70                  75                  80
Ala Ala Gly Val Asp Glu Glu His Ala Ala Trp Met Val Arg Phe Arg
                85                  90                  95
Ser Pro Ile Asp Arg Cys Ser Ser Arg Ser Trp Arg Ala Arg Ala Asp
            100                 105                 110
Thr Asp Met Asn Arg Ala Phe Pro Phe Val Phe Val Gln Ala Arg His
        115                 120                 125
Pro Ser Ala Leu Gly Glu Phe Glu Lys Val Val Ala Ala Ser Lys Gly
    130                 135                 140
Lys Gln Ile Val Met Phe Leu Asp Tyr Asp Gly Thr Leu Ser Pro Ile
145                 150                 155                 160
Val Asp Asp Pro Asp Ala Ala Phe Met Ser Glu Thr Val Ser Leu Ser
                165                 170                 175
Ser Pro Pro Leu Ser Pro Thr Leu Leu Leu His Ser Ser Ser Ser His
            180                 185                 190
Thr Ser Leu Leu Pro His Gln Met Arg Met Ala Val Arg Ser Val Ala
```

```
        195                 200                 205
Lys His Phe Pro Thr Ala Ile Val Ser Gly Arg Cys Arg Asp Lys Val
    210                 215                 220
Phe Glu Phe Val Lys Leu Ala Glu Leu Tyr Tyr Ala Gly Ser His Gly
225                 230                 235                 240
Met Asp Ile Lys Gly Pro Ala Ser Arg His Ala Ala Ala Lys Ser Pro
                245                 250                 255
Pro His Asn Lys Gly Val Leu Phe Gln Pro Ala Ser Glu Phe Leu Pro
            260                 265                 270
Met Ile Glu Gln Val His Gln Arg Leu Glu Gln Ala Thr Ser Ser Ile
        275                 280                 285
Pro Gly Ala Lys Val Glu Asn Asn Lys Phe Cys Val Ser Val His Phe
    290                 295                 300
Arg Cys Val Asp Glu Lys Ser Trp Gly Ala Leu Ala Glu Thr Val Arg
305                 310                 315                 320
Arg Val Val Arg Glu Phe Pro Arg Leu Arg Leu Ser Gln Gly Arg Met
                325                 330                 335
Val Phe Glu Val Arg Pro Thr Ile Lys Trp Asp Lys Gly Lys Ala Leu
            340                 345                 350
Glu Phe Leu Leu Asp Ser Leu Gly Phe Ala Asp Cys Ser Asp Val Leu
        355                 360                 365
Pro Val Tyr Ile Gly Asp Asp Arg Thr Asp Glu Asp Ala Phe Lys Val
    370                 375                 380
Leu Arg Arg Arg Gly Gln Gly Val Gly Ile Leu Val Ser Lys His Pro
385                 390                 395                 400
Lys Glu Thr Ser Ala Ser Phe Ser Leu Gln Glu Pro Ala Glu Leu Lys
                405                 410                 415
Lys Ser Ser Val His Tyr Pro Leu Pro Ala Gln Arg Ser Asn Arg Leu
            420                 425                 430
Asn Arg Ile Leu Val Asn Trp Trp Gly Glu Gly Leu Lys Asp Lys Leu
        435                 440                 445
Cys Ala Leu Glu Pro Ser His Glu Arg Glu Gly Cys Met Ala Pro His
    450                 455                 460
```

<210> 59
<211> 1878
<212> DNA
<213> Oryza sativa

<400> 59

```
gctccgccat cttctctcct ctcccctctc actcctccca aatcccctcc attgccttcc      60
tcgcttcgca tcgccgctcc cgccgccgcc gtcgggctgc tcgccggcat tgggtgttgg     120
ttgtgaccgt atacaattat tgtttttgga gctgagcgat cagatgcatg agccttcctt     180
ttaaatgatc tgtgtccacc atggcgatga accaaatgca ggaacttccc gcttaatggg     240
ctggatgcgt tgtatgcacg tttgcagtga caagaagaca ttgaaacgtt ggttttttat     300
tgacaagagg gttggttgag tgtctaactc ttaaggaatc ctatagtcta cttgcttgct     360
cttgtgttgt tagtaactgt tcttacaaca actctgcgga gagttctgtt gatggatttg     420
aagacaagca actctcctgt tattgctgat cctctcccta aattagcctt gccatctgct     480
gtgatgacgt acactacacc tacgagtttc ccctctaccg ggctgtactt gaacactccg     540
aaaaagaagc ctctgcctgg aaagatcgaa gaagtccgcg ccgctggatg gcttgatctc     600
atgctggcct cctcgccacc tcgcaagagg cagactaagg actttgccaa tgatgttcaa     660
gctgacgagc ttgatttgct ataccgtaat tgggtggtga accatccatc tgctttaaca     720
tcatttgagg atattgttaa tcttgccaga ggtaaaagat tggcactgtt ccttgattat     780
gatggaactc tttcaccgat tgtggacaat cctgaaaatg cagtaatgtc tgacgagatg     840
cgctctgctg tgaagcatgt ggcatcactt tttcccactg cgatcattag tggaagatct     900
cgtgataagg tttttgactt tgtgaaacta actgaactgt attacgctgg aagtcatgga     960
atggatatca tggggcctgt taggaagtct gattcgagtg gtcagcatgt ggaatgtatc    1020
aggtccactg attcagaggg taaagaggtc aacctcttcc aacctgcaag tgagttttta    1080
cctatgatta gcgaggtgta caaaaagctc agtgaaagta ttaaggacat tgatggtgca    1140
```

aggatggaag ataacaaatt ctgtgtgtcc gttcattacc gcaatgtagc accacatgac 1200
tacggagaag ttcatcaacg tgtgactgcc gtcctgaaga attacccttg tctaaggctt 1260
acccatggga gaaaggttct tgaggttcga cctgtgatag actggaacaa ggggaaagct 1320
gtggaatttt tgctggagtc acttggacta tgtgggaaag aagatgttct tcctatctat 1380
gttggagatg acaagactga tgaggatgca ttcaaggttc tgaaggcaaa cagcattggc 1440
tttggaattt tggtgtcatc tgtgcccaag gatactgatg ctttctattc cgtacgggac 1500
ccagctgagg tgatggaatt cctgaagaaa ctggcatctt ggaaggagga atccacttaa 1560
gcagaaggga ggaacataac agaatcgttt tttgaggata tggagaagta ccaactataa 1620
atacgagcaa aatatacact gctaacaaat aagtctggcc aaaatgtttt tgatttggca 1680
tcctattata taagaggctc cctaaaccac cctttttgg tctcactttc atcttatgtt 1740
tctgggattc tgagacattt cattctttta ttagttctgt tcctttagta tatataaaat 1800
atatacacat gtatgctgaa aaattttggt tcctgtccct gtcgtcttag gcaattattt 1860
attctaccgg caagttgt 1878

<210> 60
<211> 382
<212> PRT
<213> Oryza sativa

<400> 60
Met Asp Leu Lys Thr Ser Asn Ser Pro Val Ile Ala Asp Pro Leu Pro
1 5 10 15
Lys Leu Ala Leu Pro Ser Ala Val Met Thr Tyr Thr Thr Pro Thr Ser
20 25 30
Phe Pro Ser Thr Gly Leu Tyr Leu Asn Thr Pro Lys Lys Lys Pro Leu
35 40 45
Pro Gly Lys Ile Glu Glu Val Arg Ala Ala Gly Trp Leu Asp Leu Met
50 55 60
Leu Ala Ser Ser Pro Pro Arg Lys Arg Gln Thr Lys Asp Phe Ala Asn
65 70 75 80
Asp Val Gln Ala Asp Glu Leu Asp Leu Leu Tyr Arg Asn Trp Val Val
85 90 95
Asn His Pro Ser Ala Leu Thr Ser Phe Glu Asp Ile Val Asn Leu Ala
100 105 110
Arg Gly Lys Arg Leu Ala Leu Phe Leu Asp Tyr Asp Gly Thr Leu Ser
115 120 125
Pro Ile Val Asp Asn Pro Glu Asn Ala Val Met Ser Asp Glu Met Arg
130 135 140
Ser Ala Val Lys His Val Ala Ser Leu Phe Pro Thr Ala Ile Ile Ser
145 150 155 160
Gly Arg Ser Arg Asp Lys Val Phe Asp Phe Val Lys Leu Thr Glu Leu
165 170 175
Tyr Tyr Ala Gly Ser His Gly Met Asp Ile Met Gly Pro Val Arg Lys
180 185 190
Ser Asp Ser Ser Gly Gln His Val Glu Cys Ile Arg Ser Thr Asp Ser
195 200 205
Glu Gly Lys Glu Val Asn Leu Phe Gln Pro Ala Ser Glu Phe Leu Pro
210 215 220
Met Ile Ser Glu Val Tyr Lys Lys Leu Ser Glu Ser Ile Lys Asp Ile
225 230 235 240
Asp Gly Ala Arg Met Glu Asp Asn Lys Phe Cys Val Ser Val His Tyr
245 250 255
Arg Asn Val Ala Pro His Asp Tyr Gly Glu Val His Gln Arg Val Thr
260 265 270
Ala Val Leu Lys Asn Tyr Pro Cys Leu Arg Leu Thr His Gly Arg Lys
275 280 285
Val Leu Glu Val Arg Pro Val Ile Asp Trp Asn Lys Gly Lys Ala Val
290 295 300
Glu Phe Leu Leu Glu Ser Leu Gly Leu Cys Gly Lys Glu Asp Val Leu

```
305                 310                 315                 320
Pro Ile Tyr Val Gly Asp Asp Lys Thr Asp Glu Asp Ala Phe Lys Val
                325                 330                 335
Leu Lys Ala Asn Ser Ile Gly Phe Gly Ile Leu Val Ser Ser Val Pro
            340                 345                 350
Lys Asp Thr Asp Ala Phe Tyr Ser Val Arg Asp Pro Ala Glu Val Met
        355                 360                 365
Glu Phe Leu Lys Lys Leu Ala Ser Trp Lys Glu Glu Ser Thr
    370                 375                 380

<210>  61
<211>  1830
<212>  DNA
<213>  Aquilegia spp.

<400>  61
tctctctctc tctctctctc gctcgctcgc tctctgtgat ttctaggttt ttcttttcgc      60
tcttattgct tctaaaggtt agttttcgat ttcgttttca acgatttgaa gattggatca     120
agttctgtgt tgatttcttc aaagaaaaga aacagaatcg gtttctgaat attcagatat     180
cagacaatat cttcgtaagc tacatatttc ctccacttgt tttgtttaca aaatgtctgg     240
tcatcttcat taaatgctgt gtatctttgg agatgaaaag ctttcaggaa gcctgcactt     300
gatggactgc atgcccaata gcagtgataa gaaaactttg aagagaactt acaaaccgaa     360
aaaacttaat ctggatccaa tggatataaa gtcaaaccat gctgcacctg ttctcactga     420
ccctgtcccc ataaccaaat caagattagg cattcattct agcttgttgc cttactcgcc     480
gggagccacg ttttcttctg gcatgtacat aacaattcct agaaggaagg tcattcctag     540
caagcttgat gacattcgtt ccagtgggtg gctagatgcg atgaaatcct catcccctcc     600
tcgcaaaaag ctaactaaag attccaatat tgaggttgct gctgatgata gccaactcgt     660
ttaccgctca tggatgctca agtatccatc agcacttgcc tcttttgagc aaattatgaa     720
ttatgcaaag gggaagagga ttgcattgtt tctggattat gatggcacac tttcaccaat     780
cgtagatgac cctgagcgtg cttttatgtc aactgctatg cgttctgctg tgaaaaatgt     840
cgcagtgtgt ttccccactg caataattag tggaagaagt cgtgataagg tatacgagtt     900
tgtaggacta acagaactct attatgctgg cagtcatgga atggatataa tgggacctgt     960
aagacagtcc gatactggcg ataaccattc aaactgtatt agatctactg acaagcaggg    1020
taaagaagta aatcttttcc aacctgctag tgaattttta ccgatgatcg atgaggtttt    1080
tagctccctt gtcgaattta ctaaaggaat tgaaggtgca aaagttgaaa ataacaagtt    1140
ttgtgtctct gtacattacc gcaatgttga cgagaagaat tggactacag ttgcacatgg    1200
tgttcatggc atcttgaagg attaccctcg tctgcggtta actcatggga ggaaggtttt    1260
agaggttcgt cccgtgattg actgggacaa gggaaaggca gtagagtttc tgcttgattc    1320
acttggattg agtgaaagtg atgacgtgct ccccatatat gttggtgatg accggacaga    1380
tgaagatgcc tttaaggttt tgagggagaa gaatagaggt tatggcattt tagtgtcatc    1440
tgtgccaaag gaaagtaatg ctttctattc tctcaaagat ccatccgagg ttatgtcatt    1500
tctcaaatcg ttggtaagat ggaagaagtc cagaatataa aggaaggaat taaccagttt    1560
aggtagaagt tctatagtct gaactctgaa gattctaaca aataaatcaa gatggagctg    1620
cgctgttgag gtgttttctt gacactgtgg aactggatga aattgttggg gtttataagc    1680
tctcttacat ttttgtaaat cctttctgag gctcgtgttt agcgattttc ttatctatga    1740
aagttaattt ttttgttctg tgttgcaatt ttggtccatt attttaggtc tctgagggaa    1800
atttttactt aatcttataa atataagatg                                     1830

<210>  62
<211>  428
<212>  PRT
<213>  Aquilegia spp.

<400>  62
Met Leu Cys Ile Phe Gly Asp Glu Lys Leu Ser Gly Ser Leu His Leu
1               5                   10                  15
Met Asp Cys Met Pro Asn Ser Ser Asp Lys Lys Thr Leu Lys Arg Thr
            20                  25                  30
Tyr Lys Pro Lys Lys Leu Asn Leu Asp Pro Met Asp Ile Lys Ser Asn
```

```
        35                  40                  45
His Ala Ala Pro Val Leu Thr Asp Pro Val Pro Ile Thr Lys Ser Arg
    50                  55                  60
Leu Gly Ile His Ser Ser Leu Leu Pro Tyr Ser Pro Gly Ala Thr Phe
65                  70                  75                  80
Ser Ser Gly Met Tyr Ile Thr Ile Pro Arg Arg Lys Val Ile Pro Ser
                85                  90                  95
Lys Leu Asp Asp Ile Arg Ser Ser Gly Trp Leu Asp Ala Met Lys Ser
            100                 105                 110
Ser Ser Pro Pro Arg Lys Lys Leu Thr Lys Asp Ser Asn Ile Glu Val
        115                 120                 125
Ala Ala Asp Asp Ser Gln Leu Val Tyr Arg Ser Trp Met Leu Lys Tyr
    130                 135                 140
Pro Ser Ala Leu Ala Ser Phe Glu Gln Ile Met Asn Tyr Ala Lys Gly
145                 150                 155                 160
Lys Arg Ile Ala Leu Phe Leu Asp Tyr Asp Gly Thr Leu Ser Pro Ile
                165                 170                 175
Val Asp Asp Pro Glu Arg Ala Phe Met Ser Thr Ala Met Arg Ser Ala
            180                 185                 190
Val Lys Asn Val Ala Val Cys Phe Pro Thr Ala Ile Ile Ser Gly Arg
        195                 200                 205
Ser Arg Asp Lys Val Tyr Glu Phe Val Gly Leu Thr Glu Leu Tyr Tyr
    210                 215                 220
Ala Gly Ser His Gly Met Asp Ile Met Gly Pro Val Arg Gln Ser Asp
225                 230                 235                 240
Thr Gly Asp Asn His Ser Asn Cys Ile Arg Ser Thr Asp Lys Gln Gly
                245                 250                 255
Lys Glu Val Asn Leu Phe Gln Pro Ala Ser Glu Phe Leu Pro Met Ile
            260                 265                 270
Asp Glu Val Phe Ser Ser Leu Val Glu Phe Thr Lys Gly Ile Glu Gly
        275                 280                 285
Ala Lys Val Glu Asn Asn Lys Phe Cys Val Ser Val His Tyr Arg Asn
    290                 295                 300
Val Asp Glu Lys Asn Trp Thr Thr Val Ala His Gly Val His Gly Ile
305                 310                 315                 320
Leu Lys Asp Tyr Pro Arg Leu Arg Leu Thr His Gly Arg Lys Val Leu
                325                 330                 335
Glu Val Arg Pro Val Ile Asp Trp Asp Lys Gly Lys Ala Val Glu Phe
            340                 345                 350
Leu Leu Asp Ser Leu Gly Leu Ser Glu Ser Asp Asp Val Leu Pro Ile
        355                 360                 365
Tyr Val Gly Asp Asp Arg Thr Asp Glu Asp Ala Phe Lys Val Leu Arg
    370                 375                 380
Glu Lys Asn Arg Gly Tyr Gly Ile Leu Val Ser Ser Val Pro Lys Glu
385                 390                 395                 400
Ser Asn Ala Phe Tyr Ser Leu Lys Asp Pro Ser Glu Val Met Ser Phe
                405                 410                 415
Leu Lys Ser Leu Val Arg Trp Lys Lys Ser Arg Ile
            420                 425
```

<210> 63
<211> 1648
<212> DNA
<213> Aquilegia spp.

<400> 63

```
ccttcactaa gcttttctca gtttctctct ttctttctaa actctttctt tctctgttct      60
tctaattata atctttattt tggttctcat tagttgtttc ttttaactga acttcagaga     120
acttttctta atatctcttc tgtgaatctt tagtctttct tgaaagaaaa atccaatctt     180
```

```
tttacaagtt gtggtttgat ttggggtttt agaatctgaa ctaaaaacct caaatctagt     240
tgaatttcct gtttgggtgg ttatttatag tacatttggt taaagatgac taagcaaaat     300
gtggtggttt cagatgtaaa atctgggtta gcaataacag ttgcagtgtc gaattcgtct     360
ctgttttctt ctgctgtgca gaaaccatta acaacacctg gaggttatat aactatatct     420
cgtagcaagc ttttaaagaa gcttgaagaa acaggtggag ttacagaagg gagaattaat     480
gcttgggttg aatccatgag agcatcttct cctactcgta ttagaccggc agtttctttg     540
actccaaaca gagatgaaaa ctcatggatg ctcaaacatc cttcagcact taatatgttt     600
gaagaaataa caaatgcttc taaagggaag caaattgtga tgttcttaga ttatgatggt     660
actctttctc ctattgtcga cgatccagat cgtgctttca tgtctgaatc gatgagaaga     720
gctgttagag atgttgcaag atactttcct actgcaattg tgagcgggag atgcagagat     780
aaggtgtata gctttgtacg cttagcagaa ctgtactatg ctggaagtca tggtatggac     840
attaaaggac caaccaaaag ttacaaaaac aagaaaagga accaacctgt attgtttcaa     900
ccagcaaatg agtttttgcc catgattgat gaggtttaca aagcattgtt agagaaaacc     960
aaatcaactc caggtgctaa agtggagaac aataggttct gtgtatctgt gcattttaga    1020
tgtgtggatg aaaagagttg gactgaacta gcagaacaag ttagatcagt tcttaaagaa    1080
tacccaaagc ttagactgac tcaaggaaga aaggtactag agatccgtcc tactattaaa    1140
tgggacaaag ggaaggctct tgaatttttg ttagagtctc ttggattcgc caactgcaac    1200
aatgttctgc cactgtacat tggagacgat cgaaccgatg aagacgcctt taaggtcttg    1260
cgcgatagag gacaaggatt cggcattctc gtttctaaag ttccaaaaga aacaaatgct    1320
tcttattctc ttcaagaacc ttcagaggtt atggatttcc ttcaccggtt agttgaatgg    1380
aagcgtatgt cagttcgagg gcattctaga gtatagtaaa agagcaactt tcccatctag    1440
gagtgacaat gatccaagac tggggaaaat tatatgtatt ttcctttgtt caggaaaaaa    1500
gttaaaagaa aaaaaaaagg cttttctcct ttgcttttct ttgggtttgg gggcctcctt    1560
gtaagttgta actactgatg tacaagggga aatgttttca ccaaagcagc ctaatcccat    1620
tatcagggga aggcaaacat tacatttg                                       1648
```

<210> 64
<211> 376
<212> PRT
<213> Aquilegia spp.

<400> 64

```
Met Thr Lys Gln Asn Val Val Val Ser Asp Val Lys Ser Gly Leu Ala
1               5                   10                  15
Ile Thr Val Ala Val Ser Asn Ser Ser Leu Phe Ser Ser Ala Val Gln
            20                  25                  30
Lys Pro Leu Thr Thr Pro Gly Gly Tyr Ile Thr Ile Ser Arg Ser Lys
        35                  40                  45
Leu Leu Lys Lys Leu Glu Glu Thr Gly Gly Val Thr Glu Gly Arg Ile
    50                  55                  60
Asn Ala Trp Val Glu Ser Met Arg Ala Ser Ser Pro Thr Arg Ile Arg
65                  70                  75                  80
Pro Ala Val Ser Leu Thr Pro Asn Arg Asp Glu Asn Ser Trp Met Leu
                85                  90                  95
Lys His Pro Ser Ala Leu Asn Met Phe Glu Glu Ile Thr Asn Ala Ser
            100                 105                 110
Lys Gly Lys Gln Ile Val Met Phe Leu Asp Tyr Asp Gly Thr Leu Ser
        115                 120                 125
Pro Ile Val Asp Asp Pro Asp Arg Ala Phe Met Ser Glu Ser Met Arg
    130                 135                 140
Arg Ala Val Arg Asp Val Ala Arg Tyr Phe Pro Thr Ala Ile Val Ser
145                 150                 155                 160
Gly Arg Cys Arg Asp Lys Val Tyr Ser Phe Val Arg Leu Ala Glu Leu
                165                 170                 175
Tyr Tyr Ala Gly Ser His Gly Met Asp Ile Lys Gly Pro Thr Lys Ser
            180                 185                 190
Tyr Lys Asn Lys Lys Arg Asn Gln Pro Val Leu Phe Gln Pro Ala Asn
        195                 200                 205
Glu Phe Leu Pro Met Ile Asp Glu Val Tyr Lys Ala Leu Leu Glu Lys
```

210 215 220
Thr Lys Ser Thr Pro Gly Ala Lys Val Glu Asn Asn Arg Phe Cys Val
225 230 235 240
Ser Val His Phe Arg Cys Val Asp Glu Lys Ser Trp Thr Glu Leu Ala
245 250 255
Glu Gln Val Arg Ser Val Leu Lys Glu Tyr Pro Lys Leu Arg Leu Thr
260 265 270
Gln Gly Arg Lys Val Leu Glu Ile Arg Pro Thr Ile Lys Trp Asp Lys
275 280 285
Gly Lys Ala Leu Glu Phe Leu Leu Glu Ser Leu Gly Phe Ala Asn Cys
290 295 300
Asn Asn Val Leu Pro Leu Tyr Ile Gly Asp Asp Arg Thr Asp Glu Asp
305 310 315 320
Ala Phe Lys Val Leu Arg Asp Arg Gly Gln Gly Phe Gly Ile Leu Val
325 330 335
Ser Lys Val Pro Lys Glu Thr Asn Ala Ser Tyr Ser Leu Gln Glu Pro
340 345 350
Ser Glu Val Met Asp Phe Leu His Arg Leu Val Glu Trp Lys Arg Met
355 360 365
Ser Val Arg Gly His Ser Arg Val
370 375

<210> 65
<211> 1116
<212> DNA
<213> Brassica campestris

<400> 65
atggagaaac caaacaggat gtcagagagt caaaacgttg tcgtctcaga ggcggcaagg 60
tctatcatcc ccaacaactc ttcggctcct cctggtttca tctcaatctc caagaaaaag 120
cttctcaaga acctagaaat catcaatgat ggcgaaagaa tcaacgcttg ggtagattca 180
atgcgagctt cttctcctac tcatccaaaa tcactccctt cttccatctc ctcagagcaa 240
caactcagtt catggatcat gcagcatcct tctgcattag aaatgtttga gaaaatcaca 300
gaagcttcgg gagggaaaca aatcgtaata tttctagatt atgacggtac tctctctccc 360
atcgttgatg atccagacag agctttcatg tcaagcaaga tgagaagaac agtaaaaaaa 420
ctggctaagt gttttccaac tgctatagtt actggtagat gcctagacaa ggtgtataac 480
tttgtcaagc tagctgagct gtattatgct ggcagccatg gcatggacat taaagggcca 540
gcaaaaggct tctccagaca caagagggtt aaacagtctc ttctgtacca accagccagt 600
gattatcttc ccatgatcga tgaagtctat agacaacttt tggagaaaac caaatcaact 660
cctggagtca tagtagaaaa caacaagttc tgtgcttctg tgcactttcg ttgcgtcgat 720
gagaagaaat ggagcgaact ggttctacag gttcggtcgg tattaaatga ataccctagg 780
cttaaactga accaaggtcg aaaggttttc gaaatacgtc ctatgattga atgggataaa 840
ggaaaagctc ttgagttctt gttagagtca cttgggtttg gaaactctaa caacgttttc 900
ccagtttaca tcggtgatga ccggaccgac gaagatgcat ttaagctgct acgagacaga 960
ggtgaaggct gtggcgttct tgtctccaaa ttccccaaag atacggatgc ttcatattat 1020
ttgcaagatc cgtccgaggc aagtgatgaa tttcttgcaa cgattggtgg cgtggaaaca 1080
aatgcagcca agagtgtgaa gaggatgtat gtataa 1116

<210> 66
<211> 371
<212> PRT
<213> Brassica campestris

<400> 66
Met Glu Lys Pro Asn Arg Met Ser Glu Ser Gln Asn Val Val Val Ser
1 5 10 15
Glu Ala Ala Arg Ser Ile Ile Pro Asn Asn Ser Ser Ala Pro Pro Gly
20 25 30
Phe Ile Ser Ile Ser Lys Lys Lys Leu Leu Lys Asn Leu Glu Ile Ile

35 40 45
Asn Asp Gly Glu Arg Ile Asn Ala Trp Val Asp Ser Met Arg Ala Ser
50 55 60
Ser Pro Thr His Pro Lys Ser Leu Pro Ser Ser Ile Ser Ser Glu Gln
65 70 75 80
Gln Leu Ser Ser Trp Ile Met Gln His Pro Ser Ala Leu Glu Met Phe
85 90 95
Glu Lys Ile Thr Glu Ala Ser Gly Gly Lys Gln Ile Val Ile Phe Leu
100 105 110
Asp Tyr Asp Gly Thr Leu Ser Pro Ile Val Asp Asp Pro Asp Arg Ala
115 120 125
Phe Met Ser Ser Lys Met Arg Arg Thr Val Lys Lys Leu Ala Lys Cys
130 135 140
Phe Pro Thr Ala Ile Val Thr Gly Arg Cys Leu Asp Lys Val Tyr Asn
145 150 155 160
Phe Val Lys Leu Ala Glu Leu Tyr Tyr Ala Gly Ser His Gly Met Asp
165 170 175
Ile Lys Gly Pro Ala Lys Gly Phe Ser Arg His Lys Arg Val Lys Gln
180 185 190
Ser Leu Leu Tyr Gln Pro Ala Ser Asp Tyr Leu Pro Met Ile Asp Glu
195 200 205
Val Tyr Arg Gln Leu Leu Glu Lys Thr Lys Ser Thr Pro Gly Val Ile
210 215 220
Val Glu Asn Asn Lys Phe Cys Ala Ser Val His Phe Arg Cys Val Asp
225 230 235 240
Glu Lys Lys Trp Ser Glu Leu Val Leu Gln Val Arg Ser Val Leu Asn
245 250 255
Glu Tyr Pro Arg Leu Lys Leu Asn Gln Gly Arg Lys Val Phe Glu Ile
260 265 270
Arg Pro Met Ile Glu Trp Asp Lys Gly Lys Ala Leu Glu Phe Leu Leu
275 280 285
Glu Ser Leu Gly Phe Gly Asn Ser Asn Asn Val Phe Pro Val Tyr Ile
290 295 300
Gly Asp Asp Arg Thr Asp Glu Asp Ala Phe Lys Leu Leu Arg Asp Arg
305 310 315 320
Gly Glu Gly Cys Gly Val Leu Val Ser Lys Phe Pro Lys Asp Thr Asp
325 330 335
Ala Ser Tyr Tyr Leu Gln Asp Pro Ser Glu Ala Ser Asp Glu Phe Leu
340 345 350
Ala Thr Ile Gly Gly Val Glu Thr Asn Ala Ala Lys Ser Val Lys Arg
355 360 365
Met Tyr Val
370

<210> 67
<211> 1098
<212> DNA
<213> Brassica rapa

<400> 67
atggtgagct ttgtcgtgga gaaaccacag agaatgtcaa acggtgtcgt atcagagacc 60
acaaggttaa gtatcatccc taacaactct tcctctgctc agaaaacgct tctcaagaac 120
ctcgagatca tcaatggtgg acaaagagtc aacgcttggg tcgattcaat gcgggcttct 180
tctcctactc atctcaaatc actaccttct tctgtctcct cagagaaaca cctcagctca 240
tggatcatgc agcatccttc agcattagaa atgtttgaga agatcacaca ggcttcagga 300
gggaaacaaa tcgtaatgtt tcttgattat gatggtactc tctctcccat cgttgatgat 360
ccagacaaag ctttcatgtc aagcaagatg agaagaactg tgaaaaaatt ggctaaatgt 420
ttcccaactg caatagttac cggtagatgc atagacaagg tgtataactt tgtgaagctg 480
gctgagctgt attatgctgg aagccatggc atggacatta aaggtccagc aaaaggtttc 540

```
tccagacaca agagggttaa acagtctctg ttgtaccaac cagccagtga ctatcttccc    600
atgatcgatg aagtctataa acagctcttg gagaaaacta aatcaactcc tggagtcata    660
gtagaaaacc acaagtttac cgcttctgtg cactttcgtt gcgtggaaga gaagaaatgg    720
agcgaactgg ttctacaggt tcggtcggta ttagagaaat atcctacgct caaactgagc    780
caaggtcgga aggttttcga aatccgtcct atgatcgatt gggataaagg aaaagctctt    840
gagttcttgt tagagtcact tgggtttggg aactctaaca acgttttccc ggtttacatc    900
ggcgacgatc ggaccgacga agatgcattt aagatgctac gagtcagggg tgaaggcttt    960
ggcatacttg tctccaaatt tcccaaggat acagatgctt cgtattctct gcaagatccg   1020
tccgaggcaa gtccacacac gcgtatatta tacctttctg ttcctaaata taagatgttt   1080
agcagggccg atttataa                                                 1098
```

<210> 68
<211> 362
<212> PRT
<213> Brassica rapa

<400> 68

```
Met Val Ser Phe Val Val Glu Lys Pro Gln Arg Met Ser Asn Gly Val
1               5                   10                  15
Val Ser Glu Thr Thr Arg Leu Ser Ile Ile Pro Asn Asn Ser Ser Ser
            20                  25                  30
Ala Gln Lys Thr Leu Leu Lys Asn Leu Glu Ile Ile Asn Gly Gly Gln
        35                  40                  45
Arg Val Asn Ala Trp Val Asp Ser Met Arg Ala Ser Ser Pro Thr His
    50                  55                  60
Leu Lys Ser Leu Pro Ser Ser Val Ser Ser Glu Lys His Leu Ser Ser
65                  70                  75                  80
Trp Ile Met Gln His Pro Ser Ala Leu Glu Met Phe Glu Lys Ile Thr
                85                  90                  95
Gln Ala Ser Gly Gly Lys Gln Ile Val Met Phe Leu Asp Tyr Asp Gly
            100                 105                 110
Thr Leu Ser Pro Ile Val Asp Asp Pro Asp Lys Ala Phe Met Ser Ser
        115                 120                 125
Lys Met Arg Arg Thr Val Lys Lys Leu Ala Lys Cys Phe Pro Thr Ala
    130                 135                 140
Ile Val Thr Gly Arg Cys Ile Asp Lys Val Tyr Asn Phe Val Lys Leu
145                 150                 155                 160
Ala Glu Leu Tyr Tyr Ala Gly Ser His Gly Met Asp Ile Lys Gly Pro
                165                 170                 175
Ala Lys Gly Phe Ser Arg His Lys Arg Val Lys Gln Ser Leu Leu Tyr
            180                 185                 190
Gln Pro Ala Ser Asp Tyr Leu Pro Met Ile Asp Glu Val Tyr Lys Gln
        195                 200                 205
Leu Leu Glu Lys Thr Lys Ser Thr Pro Gly Val Ile Val Glu Asn His
    210                 215                 220
Lys Phe Thr Ala Ser Val His Phe Arg Cys Val Glu Glu Lys Lys Trp
225                 230                 235                 240
Ser Glu Leu Val Leu Gln Val Arg Ser Val Leu Glu Lys Tyr Pro Thr
                245                 250                 255
Leu Lys Leu Ser Gln Gly Arg Lys Val Phe Glu Ile Arg Pro Met Ile
            260                 265                 270
Asp Trp Asp Lys Gly Lys Ala Leu Glu Phe Leu Leu Glu Ser Leu Gly
        275                 280                 285
Phe Gly Asn Ser Asn Asn Val Phe Pro Val Tyr Ile Gly Asp Asp Arg
    290                 295                 300
Thr Asp Glu Asp Ala Phe Lys Met Leu Arg Val Arg Gly Glu Gly Phe
305                 310                 315                 320
Gly Ile Leu Val Ser Lys Phe Pro Lys Asp Thr Asp Ala Ser Tyr Ser
                325                 330                 335
```

```
Leu Gln Asp Pro Ser Glu Ala Ser Pro His Thr Arg Ile Leu Tyr Leu
                340                 345                 350
Ser Val Pro Lys Tyr Lys Met Phe Ser Arg
            355                 360
```

<210> 69
<211> 1280
<212> DNA
<213> Gossypium hirsutum

<400> 69

```
cgccgccggt caaaatggtc tctgatccaa actctgcaca gaaaccaccg gcaccacctg      60
ggttttatat ccatttccag aaagaaattg cttcaaaacc ttgaaatcaa tgccggagct     120
agagttaatt catgggtcga ttccatgaga gcttcttctc caactcatat gaaatcagca     180
ccgtccattg ctgatgatca aggttcctgg aatctaaacc atccatcagc actagatatg     240
ttcgaacaga taatcgatgc atcaaaaggg aaacaaatcg taatgtttct tgattacgat     300
ggcactcttt caccaatcgt agccgatcca gatcgggctt tcatgtctaa gaagatgaga     360
aagacagtga gaaagctagc caaatgtttc cctacagcta tagtgagtgg cagatgcagg     420
gacaaggtgt ataattttgt caaattagct gagctatact atgctggaag ccatggcatg     480
gacattaaag gccctgaaaa aggttccaaa tctaacaaag atactgaatc tgttcttttc     540
caaccagcta gtgaatttct tcccatgatt gatgaggttt ataaacagtt ggttgaaact     600
acaaaatcaa caccaggtgc taaagtggag aacaacaagt tttgtctctc agtacacttc     660
cgttgtgttg atgaaaagaa atggagtgaa ttggcacaac aagtgaagtc tgttttaaaa     720
gactacccca agcttcggct aactcaaggc cgaaaggttt tggaaatccg tcctacaatc     780
aaatgggaca aagggaaagc ccttgaattt ttgttagaat ctcttggatt tgctaactgt     840
accgatgtct ttcccgttta tatcggagat gatcggaccg atgaagatgc attcaagata     900
ttgagggaca gaggccaagg ttttggtatt cttgtatcca agttccccaa agacactaat     960
gcatcctatt cattacaaga accagatgag gtcatggact ttttacgacg tctggttgaa    1020
tggaaagaat tatctctaag aactcagtcg agaatgtaga agaaaataag acagtggaag    1080
taaatatttg tgcaccctag caaagaaatt gtaaaggggg gtgtgagatt aaagtagtaa    1140
aattattgtt ttaagatgag agaaatgatt ataaaagctt tgtacatctt tgtaactagt    1200
tggggtacaa agggtcaagt cttgtagctt cataacttgt ctattaatgc ataggaaagc    1260
aatggtttcg gattaatgtt                                                1280
```

<210> 70
<211> 304
<212> PRT
<213> Gossypium hirsutum

<400> 70

```
Met Arg Ala Ser Ser Pro Thr His Met Lys Ser Ala Pro Ser Ile Ala
1               5                   10                  15
Asp Asp Gln Gly Ser Trp Asn Leu Asn His Pro Ser Ala Leu Asp Met
            20                  25                  30
Phe Glu Gln Ile Ile Asp Ala Ser Lys Gly Lys Gln Ile Val Met Phe
        35                  40                  45
Leu Asp Tyr Asp Gly Thr Leu Ser Pro Ile Val Ala Asp Pro Asp Arg
    50                  55                  60
Ala Phe Met Ser Lys Lys Met Arg Lys Thr Val Arg Lys Leu Ala Lys
65                  70                  75                  80
Cys Phe Pro Thr Ala Ile Val Ser Gly Arg Cys Arg Asp Lys Val Tyr
                85                  90                  95
Asn Phe Val Lys Leu Ala Glu Leu Tyr Tyr Ala Gly Ser His Gly Met
            100                 105                 110
Asp Ile Lys Gly Pro Glu Lys Gly Ser Lys Ser Asn Lys Asp Thr Glu
        115                 120                 125
Ser Val Leu Phe Gln Pro Ala Ser Glu Phe Leu Pro Met Ile Asp Glu
    130                 135                 140
Val Tyr Lys Gln Leu Val Glu Thr Thr Lys Ser Thr Pro Gly Ala Lys
```

```
145                 150                 155                 160
Val Glu Asn Asn Lys Phe Cys Leu Ser Val His Phe Arg Cys Val Asp
                165                 170                 175
Glu Lys Lys Trp Ser Glu Leu Ala Gln Gln Val Lys Ser Val Leu Lys
            180                 185                 190
Asp Tyr Pro Lys Leu Arg Leu Thr Gln Gly Arg Lys Val Leu Glu Ile
        195                 200                 205
Arg Pro Thr Ile Lys Trp Asp Lys Gly Lys Ala Leu Glu Phe Leu Leu
    210                 215                 220
Glu Ser Leu Gly Phe Ala Asn Cys Thr Asp Val Phe Pro Val Tyr Ile
225                 230                 235                 240
Gly Asp Asp Arg Thr Asp Glu Asp Ala Phe Lys Ile Leu Arg Asp Arg
                245                 250                 255
Gly Gln Gly Phe Gly Ile Leu Val Ser Lys Phe Pro Lys Asp Thr Asn
            260                 265                 270
Ala Ser Tyr Ser Leu Gln Glu Pro Asp Glu Val Met Asp Phe Leu Arg
        275                 280                 285
Arg Leu Val Glu Trp Lys Glu Leu Ser Leu Arg Thr Gln Ser Arg Met
    290                 295                 300
```

<210> 71
<211> 1509
<212> DNA
<213> Hordeum vulgare

<400> 71

```
cagccctcca ccgcatccgg gccggagaca cctccctggc cgccatcgag catgccggtc      60
gtgtcagagg tgggcatcac ggtgaccgcg gccacggcca cggcgtgccc ctgcccgggg     120
tcgctgttcc cgtacccgcc gccgcgtgcc gggatggccg tgagccggaa gtgcctgcgg     180
gcggcgcagg cggagcttgg cgcggggatg ctcagtggcc tggtcgagtc catgcgggcg     240
tcgtccccca cgcacgccag ggccgctgcc gccctcgctg ccggcgtccg acgacgagca     300
cgcggcctgg atggccaggc acccctccgc cttggccaag ttcgaggaga tcgtggccgc     360
ctccaaaggg aagcagatcg tcatgttcct cgactacgac ggcacactgt cccccatcgt     420
cgatgacccc gacgccgcct tcatgagcga gacgatgcgg atggccgtgc gcagcgtggc     480
caagcacttc ccgacggcga tcgtcagtgg tcggtgccgc gacaaggtgt ttgagttcgt     540
gaagctggcg gagctctact acgccggcag ccacggcatg gacatcaagg gcccggccaa     600
atcctcttcc gggcacgcaa agtccaaggc caaaggagtt ctcttccaac cagcaagcga     660
gttcctgccc atgatagaag aggtgcatca acgcctgata gaggagacca agcacgtagc     720
cggcgccaag gtggagaaca acaagttctg cgtctccgtc cacttcagat gcgtcgacga     780
aaagagctgg ggcgcgctgg cggagacggt gaagggggtg atgcgggagt acccgaagct     840
gcgcatgtcg caggggcgga tggtgttcga ggtgcggccc accatcaagt gggacaaggg     900
caaggccctc gagttcctgc tcgagtcgct gggcttcgcc gactgcagca acgtgctgcc     960
cgtctacatc ggcgacgacc gcaccgacga ggacgccttc aaggtgctgc ggcggagggg    1020
ccagggcgtc gggatcctcg tctccaagca ccccaaggac accagcgcat ccttctcgct    1080
gcaggagccc gccgaggtca tggagttcct cctccgcctc gtcgagtgga agcagctctc    1140
cagggcgcgc ctcaggctgc ggcgacaggc ccacgcctga tcggacgacc acgaggatcg    1200
gccggccggg ctaattaatt cactacgcgt agcttagtta gttgatgaat cccctgctca    1260
tcgtcatcgt atgaaagcca aaggctacgt ggatgagaag aaggaaccgt accacggcga    1320
gcctgtgggg cattgtggcg ccacatgctg tgttctaacc acgcccttct tttctgggg     1380
atgtatacct accgagtaac gactatgcat gcatgtaaat taccctggta cgtccacgta    1440
caaggggagg aaccatggaa taaagaaaca gaaacctgag taaatagcaa caaccaagcc    1500
ttttgcctc                                                            1509
```

<210> 72
<211> 289
<212> PRT
<213> hordeum vulgare

<400> 72

Met Ala Arg His Pro Ser Ala Leu Ala Lys Phe Glu Glu Ile Val Ala
1 5 10 15
Ala Ser Lys Gly Lys Gln Ile Val Met Phe Leu Asp Tyr Asp Gly Thr
20 25 30
Leu Ser Pro Ile Val Asp Asp Pro Asp Ala Ala Phe Met Ser Glu Thr
35 40 45
Met Arg Met Ala Val Arg Ser Val Ala Lys His Phe Pro Thr Ala Ile
50 55 60
Val Ser Gly Arg Cys Arg Asp Lys Val Phe Glu Phe Val Lys Leu Ala
65 70 75 80
Glu Leu Tyr Tyr Ala Gly Ser His Gly Met Asp Ile Lys Gly Pro Ala
85 90 95
Lys Ser Ser Ser Gly His Ala Lys Ser Lys Ala Lys Gly Val Leu Phe
100 105 110
Gln Pro Ala Ser Glu Phe Leu Pro Met Ile Glu Glu Val His Gln Arg
115 120 125
Leu Ile Glu Glu Thr Lys His Val Ala Gly Ala Lys Val Glu Asn Asn
130 135 140
Lys Phe Cys Val Ser Val His Phe Arg Cys Val Asp Glu Lys Ser Trp
145 150 155 160
Gly Ala Leu Ala Glu Thr Val Lys Gly Val Met Arg Glu Tyr Pro Lys
165 170 175
Leu Arg Met Ser Gln Gly Arg Met Val Phe Glu Val Arg Pro Thr Ile
180 185 190
Lys Trp Asp Lys Gly Lys Ala Leu Glu Phe Leu Leu Glu Ser Leu Gly
195 200 205
Phe Ala Asp Cys Ser Asn Val Leu Pro Val Tyr Ile Gly Asp Asp Arg
210 215 220
Thr Asp Glu Asp Ala Phe Lys Val Leu Arg Arg Arg Gly Gln Gly Val
225 230 235 240
Gly Ile Leu Val Ser Lys His Pro Lys Asp Thr Ser Ala Ser Phe Ser
245 250 255
Leu Gln Glu Pro Ala Glu Val Met Glu Phe Leu Leu Arg Leu Val Glu
260 265 270
Trp Lys Gln Leu Ser Arg Ala Arg Leu Arg Leu Arg Arg Gln Ala His
275 280 285
Ala

<210> 73
<211> 1945
<212> DNA
<213> Medicago truncatula

<400> 73
tgtctctctc tagttctaaa tattccctat ctctcttttg cctcttcaat ttcccttctt 60
cctcttttgc attccattgc aattatccac ttctcacacc ccacctcttt aacctatttt 120
ctatttgttt cctcacaaac cctaagtgaa gatgactcaa aagaatgtgg ttgtgtctga 180
gaccaaaacc ggaatcaatg gtagcggtac tattaccgtg gcacagaagc caccggcagc 240
acccggtgga tacgttcaca ttccaaggag gagaattttg aagaatcttg aaatcaatgg 300
aggacaaaga atcaatacat ggattgattc tatgagagct tcttctccaa ctcatgtcaa 360
atcttctcct tctttagctg aagaatacaa ttcttggatt cttcgccatc catctgcatt 420
agatatgttt gaacaaatta tggatgctgc aaaaggaaaa cagattgtta tgtttttgga 480
ctatgatggt actttgtcac ctattgtcga tgaccctgac cgtgctttca tgtctgaatc 540
gatgaggaaa acagttaaga aacttgcaag gtgttttcct actgcaattg ttactggaag 600
atgcatagat aaagtgtaca attttgtccg tttggatgaa ctatattatg ctggaagtca 660
tggcatggat attaaagggc caacaaaaga gtccaaatac aacaaaaata acaaagctga 720
ggaagtactt tttcaacctg ccagagaatt tgttcccatg ataaatgagg tgtacgaaca 780
actagttgag aaaacaaaat caactcctgg tgccagagtt gagaaccaca agttctgcac 840

```
ctctgttcat tttagatgtg ttgatgaaaa gagatgggtt gaattggcac agcaagtaaa    900
atccgtgtta aaagagtacc cgaagcttcg tctaacacaa ggaagaaagg tattggagat    960
tcgtcccgca attaaatggg acaagggcaa ggccctcgaa tttttgctag agtcacttgg   1020
atttgccaac tgtaacgatg tatttcctgt ttatattggt gatgatagaa ccgatgaaga   1080
tgcattcaag aaattaagag acatagatca aggttttgga attctagtct ctaagtttcc   1140
aaaagacaca gctgctgcat actctttaca agagcctaat gaggttatgg agttccttca   1200
gcgtttggtg gagtggaaaa aaacatctcc aagatcacgt tctagggtgt aaatgaagac   1260
tttgtacatc cctgactggc cataaaatac atgacccata ttcatttttt aatttgtcca   1320
tcaattttct aactaataaa tcctagagtc aacaggattt gccaactgta acgatgtatt   1380
tcctgtttat attggtgatg atagaaccga tgaagatgca ttcaagaaat taagagacat   1440
agatcaaggt tttggaattc tagtctctaa gtttccaaaa gacacagctg ctgcatactc   1500
tttacaagag cctaatgagg ttatggagtt ccttcagcgt ttggtggagt ggaaaaaaac   1560
atctccaaga tcacgttcta gggtgtaaat gaagactttg tacatccctg actggccata   1620
aaatacatga cctttttat ttttctttct atgttttgtt cagattagta caaaagagca   1680
aaaatgtatg gaaaaaaaaa atgaaagaca aaagtgtctt tctgagtttg aagctcgatt   1740
gtctacattg atgtaattag cttagccaag tagtaattac taattagtac gggggaaag    1800
tatatgtact aaccatatca gatcagatca gatgaagtga ttctgaatgg gttaaaattt   1860
gtacccttat gattatgatg attaaatatt ataaggttac gtatcaaaag atatgtattt   1920
atagtgacat gcttattaag tactt                                         1945
```

```
<210>  74
<211>  366
<212>  PRT
<213>  Medicago truncatula

<400>  74
Met Thr Gln Lys Asn Val Val Val Ser Glu Thr Lys Thr Gly Ile Asn
1               5                   10                  15
Gly Ser Gly Thr Ile Thr Val Ala Gln Lys Pro Pro Ala Ala Pro Gly
            20                  25                  30
Gly Tyr Val His Ile Pro Arg Arg Arg Ile Leu Lys Asn Leu Glu Ile
        35                  40                  45
Asn Gly Gly Gln Arg Ile Asn Thr Trp Ile Asp Ser Met Arg Ala Ser
    50                  55                  60
Ser Pro Thr His Val Lys Ser Ser Pro Ser Leu Ala Glu Glu Tyr Asn
65                  70                  75                  80
Ser Trp Ile Leu Arg His Pro Ser Ala Leu Asp Met Phe Glu Gln Ile
                85                  90                  95
Met Asp Ala Ala Lys Gly Lys Gln Ile Val Met Phe Leu Asp Tyr Asp
            100                 105                 110
Gly Thr Leu Ser Pro Ile Val Asp Asp Pro Asp Arg Ala Phe Met Ser
        115                 120                 125
Glu Ser Met Arg Lys Thr Val Lys Lys Leu Ala Arg Cys Phe Pro Thr
    130                 135                 140
Ala Ile Val Thr Gly Arg Cys Ile Asp Lys Val Tyr Asn Phe Val Arg
145                 150                 155                 160
Leu Asp Glu Leu Tyr Tyr Ala Gly Ser His Gly Met Asp Ile Lys Gly
                165                 170                 175
Pro Thr Lys Glu Ser Lys Tyr Asn Lys Asn Asn Lys Ala Glu Glu Val
            180                 185                 190
Leu Phe Gln Pro Ala Arg Glu Phe Val Pro Met Ile Asn Glu Val Tyr
        195                 200                 205
Glu Gln Leu Val Glu Lys Thr Lys Ser Thr Pro Gly Ala Arg Val Glu
    210                 215                 220
Asn His Lys Phe Cys Thr Ser Val His Phe Arg Cys Val Asp Glu Lys
225                 230                 235                 240
Arg Trp Val Glu Leu Ala Gln Gln Val Lys Ser Val Leu Lys Glu Tyr
                245                 250                 255
Pro Lys Leu Arg Leu Thr Gln Gly Arg Lys Val Leu Glu Ile Arg Pro
```

```
        260                 265                 270
Ala Ile Lys Trp Asp Lys Gly Lys Ala Leu Glu Phe Leu Leu Glu Ser
    275                 280                 285
Leu Gly Phe Ala Asn Cys Asn Asp Val Phe Pro Val Tyr Ile Gly Asp
290                 295                 300
Asp Arg Thr Asp Glu Asp Ala Phe Lys Lys Leu Arg Asp Ile Asp Gln
305                 310                 315                 320
Gly Phe Gly Ile Leu Val Ser Lys Phe Pro Lys Asp Thr Ala Ala Ala
                325                 330                 335
Tyr Ser Leu Gln Glu Pro Asn Glu Val Met Glu Phe Leu Gln Arg Leu
            340                 345                 350
Val Glu Trp Lys Lys Thr Ser Pro Arg Ser Arg Ser Arg Val
        355                 360                 365
```

<210> 75
<211> 1583
<212> DNA
<213> Medicago truncatula

<400> 75

```
aacacaccca tctctctgtt cttctctgtc ttttccctct ctagtttttt ttcttattct     60
caatttctca taacaatgac taaccagaat atgttgggaa agtcacgtgt gattgtgaat    120
gagaagatat tggaattagc aatgtcgatt tcaaactcta atgttttacc aagaacttca    180
atgcctgaat taatggcttt gtttgatggg cttttaggcc agcgcaaaaa caatctcatt    240
aagcctttgg aagatgataa tgatcacaaa ggagcaacta aagttaacgc gtggattgat    300
tccatgagag cttcatctcc tactcgaacc agacacgatt cagaaaatcg tgaccagact    360
cattggactc ttttccatcc ttctgcgttg aacatgttca gtaaaataat gtacaataca    420
aatgggaaac aaattgttgt ttttcttgac tatgatggaa ctctctcccc aattgtagca    480
gatccagata aagcctacat gagcaaaaag atgagagtga cattgaagga catagcaaga    540
cattttccca ctgccattgt tagtggaagg tgcttaaaca agctatttag ctttgtaagg    600
ttggctgaat tatactatgc tggaagccat gggatggata ttaagggtcc aacaaatagg    660
agaagcacta aaaaaggtaa taatgatggg gtacttttgc agcctgctag tgaatttttg    720
cccatgatca atgaggttta taagatcttg gtggagaaaa caaagtgtgt tccaggggct    780
atggtggaaa ataacaagtt ttgtttatct gtgcattttc ggaacgttga cgaaaagagt    840
tgggaagcat tgggtgaaca agtaagctta gtgatgaatg attacccaaa actaaagcta    900
acacaaggga gaaaagtgtt ggaaattcga ccaattatta aatgggacaa aggaagggct    960
cttgaatttt tgctagagtc acttggtttt gcaaattcta aaggagtatt tccaatctat   1020
attggtgacg atagaaccga cgaagatgct tttaaggttt tacgcaatag gggccaaggg   1080
tgtgggattc ttgtttcaaa aatttcaaaa gaaactaatg cttcttacac tttgcaagat   1140
ccatctgagg ttggagaatt tttgcggcat ttggtggatt ggaaaagaac aagttctcgt   1200
tctcacaagt tgtagagaga gctaggaatc tataaatgag tttagggatt tgacaccgac   1260
ccaagaatct ggtcaagggg tagttaaaat ggcatccctt gtttgaaaat aggaaaatag   1320
tacattttat tgttccataa ttttaatatt ttagggacta gaaagtccaa atagattctc   1380
tttttctttt tttccttttc ttgtttcaat gtataattct attcttgatc tttcacacgt   1440
ttgcatgcgc atgcggatag tgaaagacat atgttttatg cctcatttgt tatatgagac   1500
attataactt tcttactctc tactgtactt aatgtacgtt tggcaaatgt aatactcata   1560
atgaaaattg ccaatttcat ctt                                           1583
```

<210> 76
<211> 379
<212> PRT
<213> Medicago truncatula

<400> 76

```
Met Thr Asn Gln Asn Met Leu Gly Lys Ser Arg Val Ile Val Asn Glu
1               5                   10                  15
Lys Ile Leu Glu Leu Ala Met Ser Ile Ser Asn Ser Asn Val Leu Pro
            20                  25                  30
Arg Thr Ser Met Pro Glu Leu Met Ala Leu Phe Asp Gly Leu Leu Gly
```

```
        35                  40                  45
Gln Arg Lys Asn Asn Leu Ile Lys Pro Leu Glu Asp Asp Asn Asp His
    50                  55                  60
Lys Gly Ala Thr Lys Val Asn Ala Trp Ile Asp Ser Met Arg Ala Ser
65                  70                  75                  80
Ser Pro Thr Arg Thr Arg His Asp Ser Glu Asn Arg Asp Gln Thr His
                85                  90                  95
Trp Thr Leu Phe His Pro Ser Ala Leu Asn Met Phe Ser Lys Ile Met
            100                 105                 110
Tyr Asn Thr Asn Gly Lys Gln Ile Val Val Phe Leu Asp Tyr Asp Gly
        115                 120                 125
Thr Leu Ser Pro Ile Val Ala Asp Pro Asp Lys Ala Tyr Met Ser Lys
    130                 135                 140
Lys Met Arg Val Thr Leu Lys Asp Ile Ala Arg His Phe Pro Thr Ala
145                 150                 155                 160
Ile Val Ser Gly Arg Cys Leu Asn Lys Leu Phe Ser Phe Val Arg Leu
                165                 170                 175
Ala Glu Leu Tyr Tyr Ala Gly Ser His Gly Met Asp Ile Lys Gly Pro
            180                 185                 190
Thr Asn Arg Arg Ser Thr Lys Lys Gly Asn Asn Asp Gly Val Leu Leu
        195                 200                 205
Gln Pro Ala Ser Glu Phe Leu Pro Met Ile Asn Glu Val Tyr Lys Ile
    210                 215                 220
Leu Val Glu Lys Thr Lys Cys Val Pro Gly Ala Met Val Glu Asn Asn
225                 230                 235                 240
Lys Phe Cys Leu Ser Val His Phe Arg Asn Val Asp Glu Lys Ser Trp
                245                 250                 255
Glu Ala Leu Gly Glu Gln Val Ser Leu Val Met Asn Asp Tyr Pro Lys
            260                 265                 270
Leu Lys Leu Thr Gln Gly Arg Lys Val Leu Glu Ile Arg Pro Ile Ile
        275                 280                 285
Lys Trp Asp Lys Gly Arg Ala Leu Glu Phe Leu Leu Glu Ser Leu Gly
    290                 295                 300
Phe Ala Asn Ser Lys Gly Val Phe Pro Ile Tyr Ile Gly Asp Asp Arg
305                 310                 315                 320
Thr Asp Glu Asp Ala Phe Lys Val Leu Arg Asn Arg Gly Gln Gly Cys
                325                 330                 335
Gly Ile Leu Val Ser Lys Ile Ser Lys Glu Thr Asn Ala Ser Tyr Thr
            340                 345                 350
Leu Gln Asp Pro Ser Glu Val Gly Glu Phe Leu Arg His Leu Val Asp
        355                 360                 365
Trp Lys Arg Thr Ser Ser Arg Ser His Lys Leu
    370                 375
```

<210> 77
<211> 1665
<212> DNA
<213> Nicotiana benthamiana

<400> 77

```
gatcttcatc cctaactcct attccaatcc caatttgtct tcctactttt tttttctag      60
cattgcgagt aaagaaacct ttcttcggct tcgtcttaac ccaactctca ttcccattcc    120
ccatttttct tttgctacaa aacagagtga aagcagagta cactatcccc tgttcttcaa    180
gattgtatta actgattagt tgaacataat ttaggagtga cgatgactca gcagaatgtg    240
gtagcgtccg accctaaatc cggtattaat ttggcaatac cagtgaaggt acaagtacca    300
tcaaactccc ccgcgctgtt cacgacagtg gcacagaagc cgccaccggc accggggagt    360
tgtatcacca tttcaagaaa aacacttgtt gaaatcaatg ggaataacac tggtgctaga    420
atcaactctt gggttgattc aatgagagct tcctctccta ctcatcacaa gtccactcct    480
cctctttctg atgacatcaa ttcttggatg gtgcaacatc catcagcact ggatatgttt    540
```

```
gagcagataa taagtgcttc aaagggaaag caaatagtga tgtttttaga ctatgacggc    600
acactctccc ccattgttga ggatcctgac caagctttca tgtctgatgc tatgagagca    660
acagtgagaa agcttgctag atatttccct actgcaatag tgagtggaag gtgcagagac    720
aaggtataca actttgtacg attggcagag ttgtactacg ctggaagcca tggaatggat    780
ataaaaggac catcaaaagg ttccaaatac aagaaaggag cagaagctgt tctttgccaa    840
ccagcaagtg aatttctacc aatgattgat gaggtttaca aagcattaat tgatgcaaca    900
aaatctacag aaggagttat agtggagaat aacaagtttt gtgcctctgt gcatttccgc    960
tgtgttgatg aaaagaaatg gggtgaacta gcacaagtcg taaggtcagt gcttaaagaa   1020
tatccaaagc tgagattaac acaaggaaga aaagtatttg agatccgtcc tactattaaa   1080
tgggacaaag gcaaagctct tgaattcttg cttgaatccc ttggatatgc taactgtact   1140
gatgtatttc ctgtatatat tggtgatgac cgaaccgatg aagatgcttt taaggttcta   1200
cgagaaagag gacagggttt tggcattctt gtctccaaaa ttccaaaaga cacacatgca   1260
tcttattctt tacaagaacc atctgaggtt atggtgtttt tacgacgctt ggtagagtgg   1320
aaaaagttgt cgttaagaag acagtttaga attcgaaggc aaattgaaga gataaaagca   1380
tctctacgga actaatgaag aattattgtc caaaagtaat ggacttaatg atgcatataa   1440
tgtattttcc ttttataaat ataacaaaag aagttgtaaa agaaaaggtg gaaaagatag   1500
gctttttaat ccttttctat ttggctttaa ggccttgtac atctttgtaa ctagctctgt   1560
acaagggtaa attgtaattt tctagtctta gctaatgcat atattggaag ttctctctct   1620
aagtacatat atactcccta aattgaaaga tgacacattt cgctt                   1665
```

<210> 78
<211> 390
<212> PRT
<213> Nicotiana benthamiana

<400> 78

```
Met Thr Gln Gln Asn Val Val Ala Ser Asp Pro Lys Ser Gly Ile Asn
1               5                   10                  15
Leu Ala Ile Pro Val Lys Val Gln Val Pro Ser Asn Ser Pro Ala Leu
            20                  25                  30
Phe Thr Thr Val Ala Gln Lys Pro Pro Pro Ala Pro Gly Ser Cys Ile
        35                  40                  45
Thr Ile Ser Arg Lys Thr Leu Val Glu Ile Asn Gly Asn Asn Thr Gly
    50                  55                  60
Ala Arg Ile Asn Ser Trp Val Asp Ser Met Arg Ala Ser Ser Pro Thr
65                  70                  75                  80
His His Lys Ser Thr Pro Pro Leu Ser Asp Asp Ile Asn Ser Trp Met
                85                  90                  95
Val Gln His Pro Ser Ala Leu Asp Met Phe Glu Gln Ile Ile Ser Ala
            100                 105                 110
Ser Lys Gly Lys Gln Ile Val Met Phe Leu Asp Tyr Asp Gly Thr Leu
        115                 120                 125
Ser Pro Ile Val Glu Asp Pro Asp Gln Ala Phe Met Ser Asp Ala Met
    130                 135                 140
Arg Ala Thr Val Arg Lys Leu Ala Arg Tyr Phe Pro Thr Ala Ile Val
145                 150                 155                 160
Ser Gly Arg Cys Arg Asp Lys Val Tyr Asn Phe Val Arg Leu Ala Glu
                165                 170                 175
Leu Tyr Tyr Ala Gly Ser His Gly Met Asp Ile Lys Gly Pro Ser Lys
            180                 185                 190
Gly Ser Lys Tyr Lys Lys Gly Ala Glu Ala Val Leu Cys Gln Pro Ala
        195                 200                 205
Ser Glu Phe Leu Pro Met Ile Asp Glu Val Tyr Lys Ala Leu Ile Asp
    210                 215                 220
Ala Thr Lys Ser Thr Glu Gly Val Ile Val Glu Asn Asn Lys Phe Cys
225                 230                 235                 240
Ala Ser Val His Phe Arg Cys Val Asp Glu Lys Lys Trp Gly Glu Leu
                245                 250                 255
Ala Gln Val Val Arg Ser Val Leu Lys Glu Tyr Pro Lys Leu Arg Leu
```

```
            260                 265                 270
Thr Gln Gly Arg Lys Val Phe Glu Ile Arg Pro Thr Ile Lys Trp Asp
        275                 280                 285
Lys Gly Lys Ala Leu Glu Phe Leu Leu Glu Ser Leu Gly Tyr Ala Asn
    290                 295                 300
Cys Thr Asp Val Phe Pro Val Tyr Ile Gly Asp Asp Arg Thr Asp Glu
305                 310                 315                 320
Asp Ala Phe Lys Val Leu Arg Glu Arg Gly Gln Gly Phe Gly Ile Leu
                325                 330                 335
Val Ser Lys Ile Pro Lys Asp Thr His Ala Ser Tyr Ser Leu Gln Glu
            340                 345                 350
Pro Ser Glu Val Met Val Phe Leu Arg Arg Leu Val Glu Trp Lys Lys
        355                 360                 365
Leu Ser Leu Arg Arg Gln Phe Arg Ile Arg Arg Gln Ile Glu Glu Ile
    370                 375                 380
Lys Ala Ser Leu Arg Asn
385                 390

<210>  79
<211>  1675
<212>  DNA
<213>  Nicotiana tabacum

<400>  79
ctggctgcag tgataaggtt acttcgaaga ggtggttttt cattgacaaa agggttggtt      60
agaagtgtgt gagacattct aattagtaaa ccgtcgtcaa ctgtttgcat agttgataaa     120
cttgtattct gccataaaaa gatattgggc acaatggacc tgaaatcaaa tacttcccca     180
gttgttactg atcctgcccc aatgactcag tccagattgg gcacccactc tgctttgatg     240
ccatactcac cgactggggc aactttctct cccacactat tccttactat cccaaggaag     300
aagccaggaa tcctagatga tgttagatca aacacttggt tggacgccat gaaatcgtca     360
tctcctacac atagcaagaa aaataaggac tccaatgctg aactaacagc aaatgaaagt     420
gatcttgcct accgcatttg gatgctcaag taccccctcag cactttcatc atttgagcaa     480
atcaccaatt atgcaaaagg caaaaggata gcactctttt tggactatga tgggacttta     540
tcaccaattg tagatgatcc agatcgagcc ttcatgtctg gtgctatgcg cgctactgtg     600
aggaatgtgg ctaaatattt tcccacagca attattagtg ggagaagccg agataaggta     660
tatgactttg tcggactagc agaactttac tatgctggta gtcatgggat ggatataatg     720
ggtcctgttc gatccgtttc tgatgactat agttgtatta gatctactaa caagcagggc     780
aaggaagtta atcttttcca acctgctggt gagttcttac caatgattga tgaggttttt     840
agatctctta ttgagctcac aaaagacatc acgggagcaa aggttgagaa caacaaattc     900
tgtgtttctg tacactatcg taacgtagat gagaagagtt ggtcagctat tggagaatct     960
gttgatgaac tgttaaaaca ctacccacgt ctgcgattga cacatggccg gaaggtttta    1020
gaagtcaggc ctgtgcttaa ctgggacaag gggaaagctg ttgagttctt acttgaatct    1080
ttgggggttga aaaattgtga tgatgttctt cccatatacg ttggagatga tcgtacagat    1140
gaagatgcat tcaaggtctt gagagaggga aataaaggct acggaatctt agtatcttct    1200
gcaccaaaag aaagcagtgc gttttactct ctgagggatc catctgaggt gatggaattc    1260
ctcaagtgct tggtatcatg gaagaagtca agtggtttta gctattaaca aggagtacca    1320
ttcaacaaca catataccccc attttgttga attcttttca tatgaagatc taatgacttg    1380
cggatggttt agggagcaga ggtgcttagt gaagcttcta gagacagcaa cagggattca    1440
agtttactgt ttctgctcag agtttttcttt tgtaaattcc ttttcgtaat tgccgcatca    1500
aggatgccct taagtcaatg tatagtcatc ttcgtatttt atttttcaag ttgtatgttg    1560
ttttaacttt ggcgtcagtt ctccccaagg gagagtgtac caacaactga aatgcttaat    1620
acatcaagta cgaacttaat ttagtaaaaa aaaaaaaaaa aaaaaaaaaa aaaaa         1675

<210>  80
<211>  384
<212>  PRT
<213>  Nicotiana tabacum

<400>  80
```

Met Asp Leu Lys Ser Asn Thr Ser Pro Val Val Thr Asp Pro Ala Pro
1               5                   10                  15
Met Thr Gln Ser Arg Leu Gly Thr His Ser Ala Leu Met Pro Tyr Ser
            20                  25                  30
Pro Thr Gly Ala Thr Phe Ser Pro Thr Leu Phe Leu Thr Ile Pro Arg
        35                  40                  45
Lys Lys Pro Gly Ile Leu Asp Asp Val Arg Ser Asn Thr Trp Leu Asp
    50                  55                  60
Ala Met Lys Ser Ser Ser Pro Thr His Ser Lys Lys Asn Lys Asp Ser
65                  70                  75                  80
Asn Ala Glu Leu Thr Ala Asn Glu Ser Asp Leu Ala Tyr Arg Ile Trp
                85                  90                  95
Met Leu Lys Tyr Pro Ser Ala Leu Ser Ser Phe Glu Gln Ile Thr Asn
            100                 105                 110
Tyr Ala Lys Gly Lys Arg Ile Ala Leu Phe Leu Asp Tyr Asp Gly Thr
        115                 120                 125
Leu Ser Pro Ile Val Asp Asp Pro Asp Arg Ala Phe Met Ser Gly Ala
    130                 135                 140
Met Arg Ala Thr Val Arg Asn Val Ala Lys Tyr Phe Pro Thr Ala Ile
145                 150                 155                 160
Ile Ser Gly Arg Ser Arg Asp Lys Val Tyr Asp Phe Val Gly Leu Ala
                165                 170                 175
Glu Leu Tyr Tyr Ala Gly Ser His Gly Met Asp Ile Met Gly Pro Val
            180                 185                 190
Arg Ser Val Ser Asp Asp Tyr Ser Cys Ile Arg Ser Thr Asn Lys Gln
        195                 200                 205
Gly Lys Glu Val Asn Leu Phe Gln Pro Ala Gly Glu Phe Leu Pro Met
    210                 215                 220
Ile Asp Glu Val Phe Arg Ser Leu Ile Glu Leu Thr Lys Asp Ile Thr
225                 230                 235                 240
Gly Ala Lys Val Glu Asn Asn Lys Phe Cys Val Ser Val His Tyr Arg
                245                 250                 255
Asn Val Asp Glu Lys Ser Trp Ser Ala Ile Gly Glu Ser Val Asp Glu
            260                 265                 270
Leu Leu Lys His Tyr Pro Arg Leu Arg Leu Thr His Gly Arg Lys Val
        275                 280                 285
Leu Glu Val Arg Pro Val Leu Asn Trp Asp Lys Gly Lys Ala Val Glu
    290                 295                 300
Phe Leu Leu Glu Ser Leu Gly Leu Lys Asn Cys Asp Asp Val Leu Pro
305                 310                 315                 320
Ile Tyr Val Gly Asp Asp Arg Thr Asp Glu Asp Ala Phe Lys Val Leu
                325                 330                 335
Arg Glu Gly Asn Lys Gly Tyr Gly Ile Leu Val Ser Ser Ala Pro Lys
            340                 345                 350
Glu Ser Ser Ala Phe Tyr Ser Leu Arg Asp Pro Ser Glu Val Met Glu
        355                 360                 365
Phe Leu Lys Cys Leu Val Ser Trp Lys Lys Ser Ser Gly Phe Ser Tyr
    370                 375                 380

<210> 81
<211> 1471
<212> DNA
<213> Nicotiana tabacum

<400> 81
ctttgttcct tacttctcta ttttctttca cttaaacaca cagaagcaga gaagtttaat 60
tattcatttc tctgactctg tgtttctcta atttcttctc ttacggattc caattcctct 120
gttttttaa tcttttcaag ggtaattttg tgttgccaaa tgacgaacca gaatgtgatt 180
gtttctgacc caagatcagg gttggagtct tctttcttat cgttctcacc ggcggtaccc 240

ggtccactac cgccgccggg gagattcatc gccgttccgg caaagaaatc atttaagaac 300
attgagtctg ctgccggaga tcatggagct aataggatta ctgctttact tgattccatg 360
agagcttctt ccccacctcg tagatcctct gaaactgaga atctcaagtc ttggattgtt 420
catcatccct cagctttgaa catgttcgag gaaattataa atgcttcaaa agggaaacaa 480
ataataatgt ttttggacta tgatggtaca ttgtctccta ttgttgatga tcctgacaaa 540
gcctttatga ctgctgagat gagggaagca gtgagagaca catccaagta ttttcctaca 600
gcaatagtga gtggaagatg cagagcaaaa gtctttaatt tcgtaaagtt atcagaactg 660
tattatgctg gaagtcatgg aatggacatt aaggcgcctg ctaaaggacg caaatataga 720
aatggaaata atcgaactgt tctctgccaa cctgccagag aatttttacc catgattgat 780
gaggtatata aatctttagt ggagaaaaca aaatctatag caggagctaa agtggaaaac 840
aacaaattct gcttatccgt acatttccgt cgtgttgaag agaaggtgtg gactgaatta 900
gctgagcaag tgaagtcagt gactaaggaa tacccaaaac ttcgattaac tcaaggaaga 960
aaggttttgg agattcgtcc cagcattaaa tgggacaagg gaaaggcact tgaatttttg 1020
ttggaatcat tagggtatgc taattcaaat gatgttctgc ctatatacat tggcgatgat 1080
cgaacagatg aagatgcttt caaggttttg cgtgacagag gacaaggttt tggaatatta 1140
gtgtccaaag cacctaaaga aacgaatgct tcctattctt tgcaagagcc attagaggtt 1200
atgtactttt taaatcgttt tgtggagtgg aaaagatcgt cctcgcaaag atatcagagg 1260
aaataaaatc cagaagagta gctcacacca agagttgacc ctttaaccct ggaagttttg 1320
ttggggatgg gtttagaaat taaagagctt taatttgtag caccttttct ttttggaaga 1380
ctcaactgta attgaaaaaa tggagaaatt tttgtaattt tccgagtata aattcaataa 1440
gaaagtattg tgcacttctc acttatatcc t 1471

<210> 82
<211> 368
<212> PRT
<213> Nicotiana tabacum

<400> 82
Met Thr Asn Gln Asn Val Ile Val Ser Asp Pro Arg Ser Gly Leu Glu
1 5 10 15
Ser Ser Phe Leu Ser Phe Ser Pro Ala Val Pro Gly Pro Leu Pro Pro
20 25 30
Pro Gly Arg Phe Ile Ala Val Pro Ala Lys Lys Ser Phe Lys Asn Ile
35 40 45
Glu Ser Ala Ala Gly Asp His Gly Ala Asn Arg Ile Thr Ala Leu Leu
50 55 60
Asp Ser Met Arg Ala Ser Ser Pro Pro Arg Arg Ser Ser Glu Thr Glu
65 70 75 80
Asn Leu Lys Ser Trp Ile Val His His Pro Ser Ala Leu Asn Met Phe
85 90 95
Glu Glu Ile Ile Asn Ala Ser Lys Gly Lys Gln Ile Ile Met Phe Leu
100 105 110
Asp Tyr Asp Gly Thr Leu Ser Pro Ile Val Asp Asp Pro Asp Lys Ala
115 120 125
Phe Met Thr Ala Glu Met Arg Glu Ala Val Arg Asp Thr Ser Lys Tyr
130 135 140
Phe Pro Thr Ala Ile Val Ser Gly Arg Cys Arg Ala Lys Val Phe Asn
145 150 155 160
Phe Val Lys Leu Ser Glu Leu Tyr Tyr Ala Gly Ser His Gly Met Asp
165 170 175
Ile Lys Ala Pro Ala Lys Gly Arg Lys Tyr Arg Asn Gly Asn Asn Arg
180 185 190
Thr Val Leu Cys Gln Pro Ala Arg Glu Phe Leu Pro Met Ile Asp Glu
195 200 205
Val Tyr Lys Ser Leu Val Glu Lys Thr Lys Ser Ile Ala Gly Ala Lys
210 215 220
Val Glu Asn Asn Lys Phe Cys Leu Ser Val His Phe Arg Arg Val Glu
225 230 235 240
Glu Lys Val Trp Thr Glu Leu Ala Glu Gln Val Lys Ser Val Thr Lys

```
                245                 250                 255
Glu Tyr Pro Lys Leu Arg Leu Thr Gln Gly Arg Lys Val Leu Glu Ile
            260                 265                 270
Arg Pro Ser Ile Lys Trp Asp Lys Gly Lys Ala Leu Glu Phe Leu Leu
        275                 280                 285
Glu Ser Leu Gly Tyr Ala Asn Ser Asn Asp Val Leu Pro Ile Tyr Ile
    290                 295                 300
Gly Asp Asp Arg Thr Asp Glu Asp Ala Phe Lys Val Leu Arg Asp Arg
305                 310                 315                 320
Gly Gln Gly Phe Gly Ile Leu Val Ser Lys Ala Pro Lys Glu Thr Asn
                325                 330                 335
Ala Ser Tyr Ser Leu Gln Glu Pro Leu Glu Val Met Tyr Phe Leu Asn
            340                 345                 350
Arg Phe Val Glu Trp Lys Arg Ser Ser Ser Gln Arg Tyr Gln Arg Lys
        355                 360                 365
```

<210> 83
<211> 1609
<212> DNA
<213> Sorghum bicolor

<220>
<221> variation
<222> (1582)..(1582)
<223> /replace = "c" /replace = "g" /replace = "t"

<400> 83

```
gcacgaggac caatcatttc tttccaacaa atctccagcc tctgctgctg ctaataaact      60
gatcgatcgc gttgttcttg ataatcatca gtcgaccgat cgcgatcggg atggcgaagc     120
ctagcgtggc ggcggtgccg gaggttgttg gcgtgccagc agcagcgcag gcgacgacgc     180
tgttcccgta cccgccaccg cgtggcgccg ggatcacagc cgccgtcgtg cgccgcaagt     240
gcctgcaggt ggagctcggc gcgggggcgg ccgggccgct gctgggcggc gcgtgctggg     300
gcgtggagtc gatgcgcgcg tcgtccccca cgcacgccaa ggccgccgcc gcgctcgccg     360
ccggcgtcga cgaggagcgc cgcgccgcct ggacggtgcg gcacccgtcg gcgctgggca     420
agttcgagca gatcgtggcg gcgtccgagg gcaagcggat cgtcatgttc ctcgactacg     480
acggcacgct gtcgcccatc gtggacgacc ccgacgccgc cttcatgagc gagacgatgc     540
ggatggccgt gcgtagcgtc gccaagcatt tcccgacggc gatcgtgagc gggcggtgcc     600
gggacaaggt gttcgagttc gtgaagctgg cggagctgta ctacgccggc agccacggca     660
tggacatcag aggcccagcc aaggcctctt cccggcacgc aaaggccaag gcaaaaggcg     720
ttctgttcca gccggcgagc gagttcctgc ccatgatcga ggaggtgcac gaccgccttg     780
tcgagacgac gcgctgcatc ccgggggcca aggtggagaa caacaagttc tgcgtctccg     840
tccacttcag atgcgtcgac gaaaagatgt ggggcgagct gtcagagtcg gtgaagggcg     900
tgctgcggga gtacccgaag ctgcggctga cacaggggcg gatggtgttc gaggtgcgtc     960
ccaccatcaa atgggacaag ggcaaggccc tcgagttcct gctcgagtcg ctcggcttcg    1020
cggactgcag caacgtcctg cccgtgtaca tcggcgacga ccgcaccgac gaggacgcct    1080
tcaaggtgct gcggcgccgg ggccagggcc acggcgtcgg gatcctcgtg tccaagcacc    1140
ccaaggagac gtccgcctcc tactccctcc aagagcccgc cgaggtgatg gagttcttgc    1200
tgcggctcgt cgagtggaag caactctcca gggcaaggct catcaggctg caatgagatt    1260
gagatggacg acgcaacgga tcaagctaac tgtcaggtgc taattaagag atcctgctac    1320
gtgaaatcgt gaatggcaga gaatctggca ctgtattgtg tcttctctac tgtcccgtag    1380
tcatctgttt tcctctccct tctctgtacg tgttccctgg gaacctctct ggcctctgct    1440
aggcgtgtgt gtaagtgttt aacaagcatg catgtacatt agtattacct aatagtagtt    1500
aacttatatg ctacatatat atatatatat atgtagtaca gtatatacaa gagggaagaa    1560
tatatgaagc aacgaagacc gaaaaaaatt atgtctcgtt caaggtttt               1609
```

<210> 84
<211> 381
<212> PRT
<213> Sorghum bicolor

<400> 84

Met Ala Lys Pro Ser Val Ala Ala Val Pro Glu Val Val Gly Val Pro
1               5                   10                  15
Ala Ala Ala Gln Ala Thr Thr Leu Phe Pro Tyr Pro Pro Pro Arg Gly
            20                  25                  30
Ala Gly Ile Thr Ala Ala Val Val Arg Arg Lys Cys Leu Gln Val Glu
        35                  40                  45
Leu Gly Ala Gly Ala Ala Gly Pro Leu Leu Gly Gly Ala Cys Trp Gly
    50                  55                  60
Val Glu Ser Met Arg Ala Ser Ser Pro Thr His Ala Lys Ala Ala Ala
65                  70                  75                  80
Ala Leu Ala Ala Gly Val Asp Glu Glu Arg Arg Ala Ala Trp Thr Val
                85                  90                  95
Arg His Pro Ser Ala Leu Gly Lys Phe Glu Gln Ile Val Ala Ala Ser
            100                 105                 110
Glu Gly Lys Arg Ile Val Met Phe Leu Asp Tyr Asp Gly Thr Leu Ser
        115                 120                 125
Pro Ile Val Asp Asp Pro Asp Ala Ala Phe Met Ser Glu Thr Met Arg
    130                 135                 140
Met Ala Val Arg Ser Val Ala Lys His Phe Pro Thr Ala Ile Val Ser
145                 150                 155                 160
Gly Arg Cys Arg Asp Lys Val Phe Glu Phe Val Lys Leu Ala Glu Leu
                165                 170                 175
Tyr Tyr Ala Gly Ser His Gly Met Asp Ile Arg Gly Pro Ala Lys Ala
            180                 185                 190
Ser Ser Arg His Ala Lys Ala Lys Ala Lys Gly Val Leu Phe Gln Pro
        195                 200                 205
Ala Ser Glu Phe Leu Pro Met Ile Glu Glu Val His Asp Arg Leu Val
    210                 215                 220
Glu Thr Thr Arg Cys Ile Pro Gly Ala Lys Val Glu Asn Asn Lys Phe
225                 230                 235                 240
Cys Val Ser Val His Phe Arg Cys Val Asp Glu Lys Met Trp Gly Glu
                245                 250                 255
Leu Ser Glu Ser Val Lys Gly Val Leu Arg Glu Tyr Pro Lys Leu Arg
            260                 265                 270
Leu Thr Gln Gly Arg Met Val Phe Glu Val Arg Pro Thr Ile Lys Trp
        275                 280                 285
Asp Lys Gly Lys Ala Leu Glu Phe Leu Leu Glu Ser Leu Gly Phe Ala
    290                 295                 300
Asp Cys Ser Asn Val Leu Pro Val Tyr Ile Gly Asp Asp Arg Thr Asp
305                 310                 315                 320
Glu Asp Ala Phe Lys Val Leu Arg Arg Arg Gly Gln Gly His Gly Val
                325                 330                 335
Gly Ile Leu Val Ser Lys His Pro Lys Glu Thr Ser Ala Ser Tyr Ser
            340                 345                 350
Leu Gln Glu Pro Ala Glu Val Met Glu Phe Leu Leu Arg Leu Val Glu
        355                 360                 365
Trp Lys Gln Leu Ser Arg Ala Arg Leu Ile Arg Leu Gln
    370                 375                 380

<210> 85
<211> 1333
<212> DNA
<213> Solanum tuberosum

<400> 85
gcccaaatcc ggtattaatc tgacaatacc ggtatcgaac tcgtcggcgt tatttacgac 60
ggcagctcag aagccaccgc cagggccggg gagttgtata actatttcaa gaaagacact 120

```
tgttgaaatt aatgggaata atagtgctag aatcaattct tgggttgaat caatgagagc    180
ttcctctcct actcatcata agtccagtcc tgctctttca gatgacttga attcttggat    240
ggtgcaacat ccatcagcac tggatatgtt tgagcagata atcagtgctt caaagggaaa    300
acaaattgtg atgtttttag actatgatgg cactctttcc cccattgttg aagatcctga    360
tcaagctttc atgtctgatg ctatgcgagc aacagtgaga aaacttgcta gatatttccc    420
tactgcaata gtgagtggaa ggtgcagaga caaggtatac agctttgtac gattggcaga    480
gttgtactat gctggtagcc atggaatgga tataaaaggg ccatcaaaag gttccaaata    540
caagaaagga gcgcaagctg ttcttttcca accagcaagt gaatttctcc ctatgattga    600
tgaggtttac aaaaaacttg tagatataac aaaatctaca gaaggagtta gagttgaaaa    660
taacaagttt tgtgcctctg tacatttccg ctgtgttgat gaaaagaaat ggggtgaact    720
agcacaagta gtaagatcag tgcttaaaga atacccaaag cttcgattga cacaaggaag    780
aaaagtatta gagattcgtc caactattaa atgggacaag ggcaaagctc tcgaattctt    840
gctcgaatca cttggatatg ctaactgtac tgatgtcttt cctgtatata ttggtgatga    900
tcgaaccgat gaagatgctt tcaaggtcct aagagaaaga gatcaaggtt ttggcattct    960
tgtctccaaa actccaaaag acacacacgc atcttattct ttgcaagaac catctgaggt   1020
tatggtgttt ctacgacgtt tggtagagtg gaaaaagttg tcattaagaa gacaatttcg   1080
aattcgaaga caaattgagg agatgaaagc atctctaagg aactaagaat ttgtccaaag   1140
tgaatatgga cttaatgatg catataatgt attttccttt tatcaaatat aacaaagaag   1200
tattaagaaa gaaaaaggta aaaaagaaat taaaagcttt ttttttatc cttttctctt    1260
tggctttaag gccttgtaca tctttgtaat tttcccagtc ttgcctaaat gcatataatg   1320
gaagttctct ctc                                                      1333
```

<210> 86
<211> 317
<212> PRT
<213> Solanum tuberosum

<400> 86

```
Met Arg Ala Ser Ser Pro Thr His His Lys Ser Ser Pro Ala Leu Ser
1               5                   10                  15
Asp Asp Leu Asn Ser Trp Met Val Gln His Pro Ser Ala Leu Asp Met
            20                  25                  30
Phe Glu Gln Ile Ile Ser Ala Ser Lys Gly Lys Gln Ile Val Met Phe
        35                  40                  45
Leu Asp Tyr Asp Gly Thr Leu Ser Pro Ile Val Glu Asp Pro Asp Gln
    50                  55                  60
Ala Phe Met Ser Asp Ala Met Arg Ala Thr Val Arg Lys Leu Ala Arg
65                  70                  75                  80
Tyr Phe Pro Thr Ala Ile Val Ser Gly Arg Cys Arg Asp Lys Val Tyr
                85                  90                  95
Ser Phe Val Arg Leu Ala Glu Leu Tyr Tyr Ala Gly Ser His Gly Met
            100                 105                 110
Asp Ile Lys Gly Pro Ser Lys Gly Ser Lys Tyr Lys Lys Gly Ala Gln
        115                 120                 125
Ala Val Leu Phe Gln Pro Ala Ser Glu Phe Leu Pro Met Ile Asp Glu
    130                 135                 140
Val Tyr Lys Lys Leu Val Asp Ile Thr Lys Ser Thr Glu Gly Val Arg
145                 150                 155                 160
Val Glu Asn Asn Lys Phe Cys Ala Ser Val His Phe Arg Cys Val Asp
                165                 170                 175
Glu Lys Lys Trp Gly Glu Leu Ala Gln Val Val Arg Ser Val Leu Lys
            180                 185                 190
Glu Tyr Pro Lys Leu Arg Leu Thr Gln Gly Arg Lys Val Leu Glu Ile
        195                 200                 205
Arg Pro Thr Ile Lys Trp Asp Lys Gly Lys Ala Leu Glu Phe Leu Leu
    210                 215                 220
Glu Ser Leu Gly Tyr Ala Asn Cys Thr Asp Val Phe Pro Val Tyr Ile
225                 230                 235                 240
Gly Asp Asp Arg Thr Asp Glu Asp Ala Phe Lys Val Leu Arg Glu Arg
```

```
                245                 250                 255
Asp Gln Gly Phe Gly Ile Leu Val Ser Lys Thr Pro Lys Asp Thr His
            260                 265                 270
Ala Ser Tyr Ser Leu Gln Glu Pro Ser Glu Val Met Val Phe Leu Arg
        275                 280                 285
Arg Leu Val Glu Trp Lys Lys Leu Ser Leu Arg Arg Gln Phe Arg Ile
    290                 295                 300
Arg Arg Gln Ile Glu Glu Met Lys Ala Ser Leu Arg Asn
305                 310                 315

<210>  87
<211>  1594
<212>  DNA
<213>  Triticum aestivum

<400>  87
ccacgcgtcc gtttcctctt cactgtctct tctcctttc tgccttccaa ccaaagcccc      60
atcccttgat tcgatcgatc gggatcctcc tctcgcgcgc ttctaggacc tgcccctcga     120
agcttctcct tctccttgga cccgtgcttg gtatggcgca gacgactgtg gtggtgccgg     180
aggtgggcat gacggctgcc acgcccactg cgtgcccctg ccctgggtcg ctgttcccgt     240
acccgccgcc gcgtgccggg atggccgtga gccgcaagtg cctgcgggcg gcgcaggcgg     300
agcttggcgc ggggatgctc agtggcctgg tcgagtccat gcgggcgtcc tcccccacgc     360
acgccagggc cgccgccgcc ctcgccgccg gcgtcgacga cgagcacgcc gcctggatgg     420
caaggcaccc gtccgccctg ggaaagttcg aggagatcgt ggccgcgtcc aaggggaagc     480
agatcgtcat gttcctggac tacgacggca cgctgtcccc catcgtcgat gaccccgacg     540
ccgccttcat gagcgagacg atgcggatgg cagtgcgcag cgtggccaag cacttcccga     600
cggcgatcgt gagcggtcgg tgccgcgaca aggtgtttga gttcgtgaag ctggcggagc     660
tctactacgc cggcagccac ggcatggaca tcaagggccc ggccaaatcc tcaaagtcca     720
aggccaaagg agttctcttc caaccagcaa gcgagttcct gcccatgata gaagaggtgc     780
atcagcggct gatagaggag acgaagcacg tagccggggc caaggtggag aacaacaagt     840
tctgcgtgtc cgtccacttc agatgcgtcg acgaaaagag ctggggcgcg ctggcggaga     900
cggtgaaggg cgtgatgcgg gagtacccga agctgcgcat gtcgcagggg cggatggtgt     960
tcgaggtgcg gcccaccatc aagtgggaca agggcaaggc cctcgagttc ctgctcgagt    1020
cgctgggctt cgccgactgc cccaacgtgc tccccgtcta catcggcgac gaccgcaccg    1080
acgaggacgc cttcaaggtg ctgcgccggc ggggccaggg cgtcgggatc ctcgtctcca    1140
agcaccccaa ggacaccagc gcctccttct cgctgcagga gccggccgag gtcatggagt    1200
tcctgctccg cctcgtcgag tggaagcagc tctccaaggc gcgcctcagg ctgcggcggc    1260
aggccgacgc ctgatcggac gacgaggatc ggccggccgg cgggaatggc cgaactaatt    1320
atttcactgc tacgtgtagc ttagctagtt aatgaaaatc ccctgcttgc tcatccgaac    1380
aaagccaaag gctacgtacg tggatgggaa gaaggaacgt actacggcga acctgcggag    1440
cattgttgcg ccacatgttg tgttctaatt aaccgtcctt ctttttctgg ggatgtatac    1500
cgtacctacc gattatgcat gcatgtaaat tacctttgtc cacgtacaag gggaggaacc    1560
atggaataaa caaagagaaa catgagtaaa tagc                                1594

<210>  88
<211>  373
<212>  PRT
<213>  Triticum aestivum

<400>  88
Met Ala Gln Thr Thr Val Val Val Pro Glu Val Gly Met Thr Ala Ala
1               5                   10                  15
Thr Pro Thr Ala Cys Pro Cys Pro Gly Ser Leu Phe Pro Tyr Pro Pro
            20                  25                  30
Pro Arg Ala Gly Met Ala Val Ser Arg Lys Cys Leu Arg Ala Ala Gln
        35                  40                  45
Ala Glu Leu Gly Ala Gly Met Leu Ser Gly Leu Val Glu Ser Met Arg
    50                  55                  60
Ala Ser Ser Pro Thr His Ala Arg Ala Ala Ala Ala Leu Ala Ala Gly
```

```
65                  70                  75                  80
Val Asp Asp Glu His Ala Ala Trp Met Ala Arg His Pro Ser Ala Leu
                85                  90                  95
Gly Lys Phe Glu Glu Ile Val Ala Ala Ser Lys Gly Lys Gln Ile Val
            100                 105                 110
Met Phe Leu Asp Tyr Asp Gly Thr Leu Ser Pro Ile Val Asp Asp Pro
        115                 120                 125
Asp Ala Ala Phe Met Ser Glu Thr Met Arg Met Ala Val Arg Ser Val
    130                 135                 140
Ala Lys His Phe Pro Thr Ala Ile Val Ser Gly Arg Cys Arg Asp Lys
145                 150                 155                 160
Val Phe Glu Phe Val Lys Leu Ala Glu Leu Tyr Tyr Ala Gly Ser His
                165                 170                 175
Gly Met Asp Ile Lys Gly Pro Ala Lys Ser Ser Lys Ser Lys Ala Lys
            180                 185                 190
Gly Val Leu Phe Gln Pro Ala Ser Glu Phe Leu Pro Met Ile Glu Glu
        195                 200                 205
Val His Gln Arg Leu Ile Glu Glu Thr Lys His Val Ala Gly Ala Lys
    210                 215                 220
Val Glu Asn Asn Lys Phe Cys Val Ser Val His Phe Arg Cys Val Asp
225                 230                 235                 240
Glu Lys Ser Trp Gly Ala Leu Ala Glu Thr Val Lys Gly Val Met Arg
                245                 250                 255
Glu Tyr Pro Lys Leu Arg Met Ser Gln Gly Arg Met Val Phe Glu Val
            260                 265                 270
Arg Pro Thr Ile Lys Trp Asp Lys Gly Lys Ala Leu Glu Phe Leu Leu
        275                 280                 285
Glu Ser Leu Gly Phe Ala Asp Cys Pro Asn Val Leu Pro Val Tyr Ile
    290                 295                 300
Gly Asp Asp Arg Thr Asp Glu Asp Ala Phe Lys Val Leu Arg Arg Arg
305                 310                 315                 320
Gly Gln Gly Val Gly Ile Leu Val Ser Lys His Pro Lys Asp Thr Ser
                325                 330                 335
Ala Ser Phe Ser Leu Gln Glu Pro Ala Glu Val Met Glu Phe Leu Leu
            340                 345                 350
Arg Leu Val Glu Trp Lys Gln Leu Ser Lys Ala Arg Leu Arg Leu Arg
        355                 360                 365
Arg Gln Ala Asp Ala
    370
```

<210> 89
<211> 1086
<212> DNA
<213> Zea mays

<400> 89

```
atgacgaagc acgccgccta ctccagcgag gacgtggtcg cggccgtggc ggcgccggcg      60
ccggccggcc ggcatttcac gtcgttccag gcgctgaagg gcgcgcccct cgactgcaag     120
aagcacgccg ccgtggacct gtccgcgtcc ggggcggccg tcgtgggcgg cggcccctgg     180
tttgagtcca tgaaggcttc gtcgccgcgg cgcgccgccg acgccgagca cggcgactgg     240
atggagaagc acccgtccgc attggcccag ttcgagccgc tgcttgccgc cgccaagggg     300
aagcagatcg tgatgttcct ggactacgac ggcaccctgt caccgatcgt cgaggacccc     360
gaccgcgccg tcatgtcgga ggagatgaga gaagccgtgc ggcgcgtcgc cgagcacttc     420
cccaccgcga ttgtgagcgg aagatgcagg gacaaggtgc tcaacttcgt gaagctgacg     480
gagctgtact acgccgggag ccatggcatg gacatccagg gccccgccgc ctgcaggcag     540
cccaaccacg tccagcaggc tgaagccgca gctgtccatt accaagctgc gagtgagttc     600
ctgccggtca tcgaagaggt gttccgcacg ctgacggcca agatggagtc catcgccggc     660
gccagggtgg agcacaacaa gtactgcctg tccgtccact tccgctgcgt ccgggaggag     720
gaatggaatg ccgtgaacga ggaggtcagg tcggtgctca gggagtaccc gaacctcaag     780
```

ctcactcacg gcagaaaggt gctggagatt cgtccgtcca tcaagtggga caagggcaag 840
gccctcgagt tcttgctcaa gtctcttggc tatgctgggc gcaacgacgt cttcccgatt 900
tacatcggag atgatcgcac tgacgaggac gctttcaagg tgctccgcaa catggggcag 960
ggcatcggaa tcctggtgtc caagcttcct aaggagacgg cggcatccta ctcgctgagt 1020
gaccctgccg aggtcaagga gttcctccgc aagctggcca ataagaaggg ggcgcgccaa 1080
ccatga 1086

<210> 90
<211> 361
<212> PRT
<213> Zea mays

<400> 90
Met Thr Lys His Ala Ala Tyr Ser Ser Glu Asp Val Val Ala Ala Val
1 5 10 15
Ala Ala Pro Ala Pro Ala Gly Arg His Phe Thr Ser Phe Gln Ala Leu
20 25 30
Lys Gly Ala Pro Leu Asp Cys Lys Lys His Ala Ala Val Asp Leu Ser
35 40 45
Ala Ser Gly Ala Ala Val Val Gly Gly Gly Pro Trp Phe Glu Ser Met
50 55 60
Lys Ala Ser Ser Pro Arg Arg Ala Ala Asp Ala Glu His Gly Asp Trp
65 70 75 80
Met Glu Lys His Pro Ser Ala Leu Ala Gln Phe Glu Pro Leu Leu Ala
85 90 95
Ala Ala Lys Gly Lys Gln Ile Val Met Phe Leu Asp Tyr Asp Gly Thr
100 105 110
Leu Ser Pro Ile Val Glu Asp Pro Asp Arg Ala Val Met Ser Glu Glu
115 120 125
Met Arg Glu Ala Val Arg Arg Val Ala Glu His Phe Pro Thr Ala Ile
130 135 140
Val Ser Gly Arg Cys Arg Asp Lys Val Leu Asn Phe Val Lys Leu Thr
145 150 155 160
Glu Leu Tyr Tyr Ala Gly Ser His Gly Met Asp Ile Gln Gly Pro Ala
165 170 175
Ala Cys Arg Gln Pro Asn His Val Gln Gln Ala Glu Ala Ala Ala Val
180 185 190
His Tyr Gln Ala Ala Ser Glu Phe Leu Pro Val Ile Glu Glu Val Phe
195 200 205
Arg Thr Leu Thr Ala Lys Met Glu Ser Ile Ala Gly Ala Arg Val Glu
210 215 220
His Asn Lys Tyr Cys Leu Ser Val His Phe Arg Cys Val Arg Glu Glu
225 230 235 240
Glu Trp Asn Ala Val Asn Glu Glu Val Arg Ser Val Leu Arg Glu Tyr
245 250 255
Pro Asn Leu Lys Leu Thr His Gly Arg Lys Val Leu Glu Ile Arg Pro
260 265 270
Ser Ile Lys Trp Asp Lys Gly Lys Ala Leu Glu Phe Leu Leu Lys Ser
275 280 285
Leu Gly Tyr Ala Gly Arg Asn Asp Val Phe Pro Ile Tyr Ile Gly Asp
290 295 300
Asp Arg Thr Asp Glu Asp Ala Phe Lys Val Leu Arg Asn Met Gly Gln
305 310 315 320
Gly Ile Gly Ile Leu Val Ser Lys Leu Pro Lys Glu Thr Ala Ala Ser
325 330 335
Tyr Ser Leu Ser Asp Pro Ala Glu Val Lys Glu Phe Leu Arg Lys Leu
340 345 350
Ala Asn Lys Lys Gly Ala Arg Gln Pro
355 360

<210> 91
<211> 1066
<212> DNA
<213> Zea mays

<400> 91
atgacgaagc gcaccgcctt cgccgcggac gacgcgatca tcgccgccgc cgccgccgtc 60
acgtcgcagc ccggccggcg gttcacgtcg tacccgccgg cgagggcgcg cggcggatgc 120
aggctggccc cggcggcggc ggcgcgccag gccacggacg accccggcgc cgctgggtcc 180
tggccagaac tagtcgtgcc gcggcacgcc gacttcgacg actggatgga gaagcacccg 240
tcggcattgg ccgcgttcga gtcggtgctg gccgccgcca aggcaagaa gatcgtcatg 300
ttcctcgact acgacggcac cctgtcgccg atcgtcaggg accccgacag cgccgtcatg 360
tccgaggaga tgcgggacgc ggtgagaggc gtggccgagc acttcccgac ggcgatcgtg 420
agcgggaggt gtagagacaa ggtgttcaac ttcgtgaagc tggcggagct gtactacgcc 480
gggagccacg gcatggacat caagggcccc acagcacagt ccaagcacac caaggcaaag 540
gccggagccg ttctatgcca acctgcgagg gcgttcctgc cggtcattga ggaggtgtac 600
cgcgcgctga cggcgagcac ggcgccgatc cccggcgcga cggtggagaa caacaagttc 660
tgcctctccg tccacttccg ctgcgtccag gaggagaaat ggcgcgctct ggaggagcag 720
gtccggtcgg tgctcaagga gtacccggac ctccgcctca ccaagggcag gaaggtcctc 780
gagatccggc cgtccatcaa gtgggacaag ggcaacgccc tgcagttctt gctcgagtct 840
ctcggttttg ctggcagtaa cagtgtcttc ccgatatata tcggagacga tagcaccgac 900
gaggacgcgt tcaaggtcct gcgcaacttg ggcaaggga tcgggatcct ggtgagcaag 960
attccgaagg agacccgcgc atcctactcc ctgcgtgaac cttctgaggt ggaggagttc 1020
ctgcgcaagt tggtcagctg gtccaaggag agcaggcaac gggact 1066

<210> 92
<211> 355
<212> PRT
<213> Zea mays

<400> 92
Met Thr Lys Arg Thr Ala Phe Ala Ala Asp Asp Ala Ile Ile Ala Ala
1 5 10 15
Ala Ala Ala Val Thr Ser Gln Pro Gly Arg Arg Phe Thr Ser Tyr Pro
20 25 30
Pro Ala Arg Ala Arg Gly Gly Cys Arg Leu Ala Pro Ala Ala Ala Ala
35 40 45
Arg Gln Ala Thr Asp Asp Pro Gly Ala Ala Gly Ser Trp Pro Glu Leu
50 55 60
Val Val Pro Arg His Ala Asp Phe Asp Asp Trp Met Glu Lys His Pro
65 70 75 80
Ser Ala Leu Ala Ala Phe Glu Ser Val Leu Ala Ala Ala Lys Gly Lys
85 90 95
Lys Ile Val Met Phe Leu Asp Tyr Asp Gly Thr Leu Ser Pro Ile Val
100 105 110
Arg Asp Pro Asp Ser Ala Val Met Ser Glu Glu Met Arg Asp Ala Val
115 120 125
Arg Gly Val Ala Glu His Phe Pro Thr Ala Ile Val Ser Gly Arg Cys
130 135 140
Arg Asp Lys Val Phe Asn Phe Val Lys Leu Ala Glu Leu Tyr Tyr Ala
145 150 155 160
Gly Ser His Gly Met Asp Ile Lys Gly Pro Thr Ala Gln Ser Lys His
165 170 175
Thr Lys Ala Lys Ala Gly Ala Val Leu Cys Gln Pro Ala Arg Ala Phe
180 185 190
Leu Pro Val Ile Glu Glu Val Tyr Arg Ala Leu Thr Ala Ser Thr Ala
195 200 205
Pro Ile Pro Gly Ala Thr Val Glu Asn Asn Lys Phe Cys Leu Ser Val

```
        210                 215                 220
His Phe Arg Cys Val Gln Glu Glu Lys Trp Arg Ala Leu Glu Glu Gln
225                 230                 235                 240
Val Arg Ser Val Leu Lys Glu Tyr Pro Asp Leu Arg Leu Thr Lys Gly
                245                 250                 255
Arg Lys Val Leu Glu Ile Arg Pro Ser Ile Lys Trp Asp Lys Gly Asn
            260                 265                 270
Ala Leu Gln Phe Leu Leu Glu Ser Leu Gly Phe Ala Gly Ser Asn Ser
        275                 280                 285
Val Phe Pro Ile Tyr Ile Gly Asp Asp Ser Thr Asp Glu Asp Ala Phe
    290                 295                 300
Lys Val Leu Arg Asn Leu Gly Gln Gly Ile Gly Ile Leu Val Ser Lys
305                 310                 315                 320
Ile Pro Lys Glu Thr Arg Ala Ser Tyr Ser Leu Arg Glu Pro Ser Glu
                325                 330                 335
Val Glu Glu Phe Leu Arg Lys Leu Val Ser Trp Ser Lys Glu Ser Arg
            340                 345                 350
Gln Arg Asp
        355
```

<210> 93
<211> 277
<212> PRT
<213> Artificial sequence

<220>
<223> consensus trehalose Ppase domain

<220>
<221> UNSURE
<222> (1)..(1)
<223> Xaa can be any naturally occurring aminao acid

<220>
<221> UNSURE
<222> (10)..(10)
<223> Xaa can be any naturally occurring aminao acid

<220>
<221> UNSURE
<222> (12)..(14)
<223> Xaa can be any naturally occurring aminao acid

<220>
<221> UNSURE
<222> (16)..(17)
<223> Xaa can be any naturally occurring aminao acid

<220>
<221> UNSURE
<222> (19)..(20)
<223> Xaa can be any naturally occurring aminao acid

<220>
<221> UNSURE
<222> (22)..(24)
<223> Xaa can be any naturally occurring aminao acid

<220>

<221> UNSURE
<222> (26)..(28)
<223> Xaa can be any naturally occurring aminao acid

<220>
<221> UNSURE
<222> (30)..(31)
<223> Xaa can be any naturally occurring aminao acid

<220>
<221> UNSURE
<222> (35)..(39)
<223> Xaa can be any naturally occurring aminao acid

<220>
<221> UNSURE
<222> (41)..(41)
<223> Xaa can be any naturally occurring aminao acid

<220>
<221> UNSURE
<222> (47)..(50)
<223> Xaa can be any naturally occurring aminao acid

<220>
<221> UNSURE
<222> (52)..(66)
<223> Xaa can be any naturally occurring aminao acid

<220>
<221> UNSURE
<222> (68)..(69)
<223> Xaa can be any naturally occurring aminao acid

<220>
<221> UNSURE
<222> (71)..(71)
<223> Xaa can be any naturally occurring aminao acid

<220>
<221> UNSURE
<222> (78)..(79)
<223> Xaa can be any naturally occurring aminao acid

<220>
<221> UNSURE
<222> (82)..(114)
<223> Xaa can be any naturally occurring aminao acid

<220>
<221> UNSURE
<222> (116)..(116)
<223> Xaa can be any naturally occurring aminao acid

<220>
<221> UNSURE
<222> (118)..(120)
<223> Xaa can be any naturally occurring aminao acid

<220>
<221> UNSURE
<222> (122)..(123)
<223> Xaa can be any naturally occurring aminao acid

<220>
<221> UNSURE
<222> (126)..(129)
<223> Xaa can be any naturally occurring aminao acid

<220>
<221> UNSURE
<222> (131)..(131)
<223> Xaa can be any naturally occurring aminao acid

<220>
<221> UNSURE
<222> (136)..(136)
<223> Xaa can be any naturally occurring aminao acid

<220>
<221> UNSURE
<222> (139)..(139)
<223> Xaa can be any naturally occurring aminao acid

<220>
<221> UNSURE
<222> (141)..(142)
<223> Xaa can be any naturally occurring aminao acid

<220>
<221> UNSURE
<222> (150)..(150)
<223> Xaa can be any naturally occurring aminao acid

<220>
<221> UNSURE
<222> (153)..(159)
<223> Xaa can be any naturally occurring aminao acid

<220>
<221> UNSURE
<222> (161)..(162)
<223> Xaa can be any naturally occurring aminao acid

<220>
<221> UNSURE
<222> (164)..(166)
<223> Xaa can be any naturally occurring aminao acid

<220>
<221> UNSURE
<222> (168)..(176)
<223> Xaa can be any naturally occurring aminao acid

<220>
<221> UNSURE
<222> (178)..(178)
<223> Xaa can be any naturally occurring aminao acid

<220>
<221> UNSURE
<222> (180)..(181)
<223> Xaa can be any naturally occurring aminao acid

<220>
<221> UNSURE
<222> (184)..(184)
<223> Xaa can be any naturally occurring aminao acid

<220>
<221> UNSURE
<222> (191)..(193)
<223> Xaa can be any naturally occurring aminao acid

<220>
<221> UNSURE
<222> (195)..(195)
<223> Xaa can be any naturally occurring aminao acid

<220>
<221> UNSURE
<222> (198)..(198)
<223> Xaa can be any naturally occurring aminao acid

<220>
<221> UNSURE
<222> (201)..(202)
<223> Xaa can be any naturally occurring aminao acid

<220>
<221> UNSURE
<222> (205)..(206)
<223> Xaa can be any naturally occurring aminao acid

<220>
<221> UNSURE
<222> (208)..(230)
<223> Xaa can be any naturally occurring aminao acid

<220>
<221> UNSURE
<222> (233)..(233)
<223> Xaa can be any naturally occurring aminao acid

<220>
<221> UNSURE
<222> (238)..(239)
<223> Xaa can be any naturally occurring aminao acid

<220>
<221> UNSURE
<222> (246)..(247)
<223> Xaa can be any naturally occurring aminao acid

<220>
<221> UNSURE
<222> (249)..(261)

<223> Xaa can be any naturally occurring aminao acid

<220>
<221> UNSURE
<222> (265)..(270)
<223> Xaa can be any naturally occurring aminao acid

<220>
<221> UNSURE
<222> (272)..(272)
<223> Xaa can be any naturally occurring aminao acid

<220>
<221> UNSURE
<222> (274)..(276)
<223> Xaa can be any naturally occurring aminao acid

<400> 93

```
Xaa Leu Asp Tyr Asp Gly Thr Leu Ser Xaa Ile Xaa Xaa Xaa Pro Xaa
1               5                   10                  15
Xaa Ala Xaa Xaa Ser Xaa Xaa Xaa Leu Xaa Xaa Xaa Leu Xaa Xaa Leu
            20                  25                  30
Ala Ser Xaa Xaa Xaa Xaa Xaa Val Xaa Ile Val Ser Gly Arg Xaa Xaa
        35                  40                  45
Xaa Xaa Leu Xaa Xaa Xaa Xaa Xaa Xaa Xaa Xaa Xaa Xaa Xaa Xaa Xaa
    50                  55                  60
Xaa Xaa Val Xaa Xaa Leu Xaa Leu Ala Ala Glu His Gly Xaa Xaa Ile
65                  70                  75                  80
Arg Xaa Xaa Xaa Xaa Xaa Xaa Xaa Xaa Xaa Xaa Xaa Xaa Xaa Xaa Xaa
                85                  90                  95
Xaa Xaa Xaa Xaa Xaa Xaa Xaa Xaa Xaa Xaa Xaa Xaa Xaa Xaa Xaa Xaa
            100                 105                 110
Xaa Xaa Leu Xaa Trp Xaa Xaa Xaa Val Xaa Xaa Ile Leu Xaa Xaa Xaa
        115                 120                 125
Xaa Glu Xaa Thr Pro Gly Ser Xaa Ile Glu Xaa Lys Xaa Xaa Ala Leu
    130                 135                 140
Ala Trp His Tyr Arg Xaa Ala Asp Xaa Xaa Xaa Xaa Xaa Xaa Xaa Ala
145                 150                 155                 160
Xaa Xaa Val Xaa Xaa Xaa Leu Xaa Xaa Xaa Xaa Xaa Xaa Xaa Xaa Xaa
                165                 170                 175
Leu Xaa Val Xaa Xaa Gly Lys Xaa Val Val Glu Val Arg Pro Xaa Xaa
            180                 185                 190
Xaa Val Xaa Lys Gly Xaa Ala Leu Xaa Xaa Ile Leu Xaa Xaa Leu Xaa
        195                 200                 205
Xaa Xaa Xaa Xaa Xaa Xaa Xaa Xaa Xaa Xaa Xaa Xaa Xaa Xaa Xaa Xaa
    210                 215                 220
Xaa Xaa Xaa Xaa Xaa Xaa Val Leu Xaa Ile Gly Asp Asp Xaa Xaa Thr
225                 230                 235                 240
Asp Glu Asp Met Phe Xaa Xaa Ile Xaa Xaa Xaa Xaa Xaa Xaa Xaa Xaa
                245                 250                 255
Xaa Xaa Xaa Xaa Xaa Ile Leu Val Xaa Xaa Xaa Xaa Xaa Xaa Thr Xaa
            260                 265                 270
Ala Xaa Xaa Xaa Leu
        275
```

<210> 94
<211> 251
<212> PRT
<213> Artificial sequence

<220>
<223> trehalose Ppase domain in SEQ ID NO 2

<400> 94
Gly Lys Gln Ile Val Met Phe Leu Asp Tyr Asp Gly Thr Leu Ser Pro
1 5 10 15
Ile Val Glu Asp Pro Asp Lys Ala Phe Ile Thr His Glu Met Arg Glu
20 25 30
Val Val Lys Asp Val Ala Ser Asn Phe Pro Thr Ala Ile Val Thr Gly
35 40 45
Arg Ser Ile Glu Lys Val Arg Ser Phe Val Gln Val Asn Glu Ile Tyr
50 55 60
Tyr Ala Gly Ser His Gly Met Asp Ile Glu Gly Pro Thr Asn Glu Asn
65 70 75 80
Ser Asn Gly Gln Ser Asn Glu Arg Val Leu Phe Gln Pro Ala Arg Glu
85 90 95
Phe Leu Pro Met Ile Glu Lys Val Val Asn Ile Leu Glu Glu Lys Thr
100 105 110
Lys Trp Ile Pro Gly Ala Met Val Glu Asn Asn Lys Phe Cys Leu Ser
115 120 125
Val His Phe Arg Arg Val Asp Glu Lys Arg Trp Pro Ala Leu Ala Glu
130 135 140
Val Val Lys Ser Val Leu Ile Asp Tyr Pro Lys Leu Lys Leu Thr Gln
145 150 155 160
Gly Arg Lys Val Leu Glu Ile Arg Pro Thr Ile Lys Trp Asp Lys Gly
165 170 175
Gln Ala Leu Asn Phe Leu Leu Lys Ser Leu Gly Tyr Glu Asn Ser Asp
180 185 190
Asp Val Val Pro Val Tyr Ile Gly Asp Asp Arg Thr Asp Glu Asp Ala
195 200 205
Phe Lys Val Leu Arg Glu Arg Gly Gln Gly Phe Gly Ile Leu Val Ser
210 215 220
Lys Val Pro Lys Asp Thr Asn Ala Ser Tyr Ser Leu Gln Asp Pro Ser
225 230 235 240
Gln Val Asn Lys Phe Leu Glu Arg Leu Val Glu
245 250

<210> 95
<211> 19
<212> PRT
<213> Artificial sequence

<220>
<223> serine-rich domain

<220>
<221> UNSURE
<222> (2)..(3)
<223> Xaa can be any naturally occurring amino acid

<220>
<221> UNSURE
<222> (16)..(17)
<223> Xaa can be any naturally occurring amino acid

<400> 95
Arg Xaa Xaa Ser Trp Val Asp Ser Met Arg Ala Ser Ser Pro Thr Xaa
1 5 10 15

Xaa Lys Ser

<210> 96
<211> 10
<212> PRT
<213> Artificial sequence

<220>
<223> B-phosphatase box

<220>
<221> VARIANT
<222> (6)..(6)
<223> /replace = "Glu"

<400> 96
Gly Asp Asp Arg Thr Asp Gln Asp Ala Phe
1               5                   10

<210> 97
<211> 12
<212> PRT
<213> Artificial sequence

<220>
<223> A-phosphatase box

<400> 97
Leu Asp Tyr Asp Gly Thr Leu Ser Pro Ile Val Asp
1               5                   10

<210> 98
<211> 2193
<212> DNA
<213> Oryza sativa

<400> 98
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc cctttccccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga atttttttc ccaaatatct taaaaagtca 1740
ctttctggtt cagttcaatg aattgattgc tacaaataat gcttttatag cgttatccta 1800
gctgtagttc agttaatagg taataccoct atagtttagt caggagaaga acttatccga 1860
tttctgatct ccatttttaa ttatatgaaa tgaactgtag cataagcagt attcatttgg 1920
attatttttt ttattagctc tcacccctttc attattctga gctgaaagtc tggcatgaac 1980
tgtcctcaat tttgttttca aattcacatc gattatctat gcattatcct cttgtatcta 2040
cctgtagaag tttcttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttc 2193

<210> 99
<211> 56
<212> DNA
<213> Artificial sequence

<220>
<223> primer: prm05451

<400> 99
ggggacaagt ttgtacaaaa aagcaggctt aaacaatgac taaccagaat gtcatc 56

<210> 100
<211> 50
<212> DNA
<213> Artificial sequence

<220>
<223> primer: prm05452

<400> 100
ggggaccact ttgtacaaga aagctgggtt gtaattatgt tgcatgtctt 50

<210> 101
<211> 32
<212> DNA
<213> Artificial sequence

<220>
<223> primer: prmAtTPPA-5

<400> 101
ggaagatcta tggacatgaa atctggtcac tc 32

<210> 102
<211> 30
<212> DNA
<213> Artificial sequence

<220>
<223> primer: prmAtTPPA-3

<400> 102

aaggcctacc cattgatctc ttccatgtca 30

<210> 103
<211> 31
<212> DNA
<213> Artificial sequence

<220>
<223> primer: prmAtTPPB-5

<400> 103
ggaattcatg actaaccaga atgtcatcgt t 31

<210> 104
<211> 29
<212> DNA
<213> Artificial sequence

<220>
<223> primer: prmAtTPPB-3

<400> 104
aaggcctctc ttctcccact gtcttcctc 29

<210> 105
<211> 31
<212> DNA
<213> Artificial sequence

<220>
<223> primer: prmAtTPPC-5

<400> 105
cgggatccat gaagattacg gatatttccg g 31

<210> 106
<211> 30
<212> DNA
<213> Artificial sequence

<220>
<223> primer: prmAtTPPC-3

<400> 106
aaggcctttc tccaagtgtt tgtttcttcc 30

<210> 107
<211> 32
<212> DNA
<213> Artificial sequence

<220>
<223> primer: prmAtTPPD-5

<400> 107
cgggatccat gacaaaccat aatgccttaa tc 32

<210> 108
<211> 32

<212> DNA
<213> Artificial sequence

<220>
<223> primer: prmAtTPPD-3

<400> 108
aaggccttct tcctcttagt gacatttgtt tc 32

<210> 109
<211> 34
<212> DNA
<213> Artificial sequence

<220>
<223> primer: prmAtTPPE-5

<400> 109
cgggatccat gttcgaagaa atacttcata aatc 34

<210> 110
<211> 29
<212> DNA
<213> Artificial sequence

<220>
<223> primer: prmAtTPPE-3

<400> 110
aaggccttgc cccacacctt gactgtttc 29

<210> 111
<211> 32
<212> DNA
<213> Artificial sequence

<220>
<223> primer: prmAtTPPF-5

<400> 111
catgccatgg atttaaactc aaaccacaaa tc 32

<210> 112
<211> 34
<212> DNA
<213> Artificial sequence

<220>
<223> primer: prmAtTPPF-3

<400> 112
tcccccggga aaaccagtag aattcttctc caac 34

<210> 113
<211> 32
<212> DNA
<213> Artificial sequence

<220>

<223> primer: prmAtTPPG-5

<400> 113
catgccatgg atttgaatat aaacaagacg ac 32

<210> 114
<211> 35
<212> DNA
<213> Artificial sequence

<220>
<223> primer: prmAtTPPG-3

<400> 114
aaggcctaaa acttgttttt gaactttcca tcttc 35

<210> 115
<211> 34
<212> DNA
<213> Artificial sequence

<220>
<223> primer: prmAtTPPH-5

<400> 115
cgggatccat ggttagattc atagaagaaa acac 34

<210> 116
<211> 30
<212> DNA
<213> Artificial sequence

<220>
<223> primer: prmAtTPPH-3

<400> 116
aaggccttgc tccagatctc aattgtttcc 30

<210> 117
<211> 32
<212> DNA
<213> Artificial sequence

<220>
<223> primer: prmAtTPPI-5

<400> 117
cgggatccat gtcagctagt caaaacattg tc 32

<210> 118
<211> 30
<212> DNA
<213> Artificial sequence

<220>
<223> primer: prmAtTPPI-3

<400> 118
aaggcctcat tcttggctgc atttgtttcc 30

<210> 119
<211> 30
<212> DNA
<213> Artificial sequence

<220>
<223> primer: prmAtTPPJ-5

<400> 119
cgggatccat ggtgagccaa aacgtcgtcg 30

<210> 120
<211> 29
<212> DNA
<213> Artificial sequence

<220>
<223> primer: prmAtTPPJ-3

<400> 120
aaggcctttg ctgcatctgt ttccactcc 29

<210> 121
<211> 1494
<212> DNA
<213> Glycine max

<400> 121
ctcttctctt ctcttcactt tgatttcatt gcaattacct ctttttgttg ggatcttctt 60
cccttctctg ttctaaaaaa gaacacggct gatctgggct cgactaaaac aaatcactaa 120
gtaaaaatga cgcagaatgc gatagtgtcc aagacaaaat cggggatcaa ccgggacata 180
accgtgccac aaaagccact ggcggcggcg gcggcggcgg gggggtacat tcccattccg 240
aggaggaggg ttttgaagaa cctggaaatc aatgcatggg ttgattccat gagatcctct 300
tctccaacca attccaaatc cacctcttct ctcgcagaag aacacagcac ttggattctt 360
cgccacccct cagcattaga catgttcgag caaattatgg atgcgtcccg gggaaagcaa 420
attgtcatgt ttctcgacta tgatggtact ttgtcgccta ttgttgatga cccagaccgt 480
gctttcatgt cggattcgat gaggagaacg gtgaggaaac ttgcaaggtg ttttcctact 540
gctatagtta ccggcagatg caaagacaag gtttacaatt ttgttcgctt ggctgagcta 600
tattatgctg gaagccatgg catggatatt caagggccaa caagagactg caaatacagc 660
aaagacaaag gagagccagt tctttttcaa cctgccagtg aatttcttcc catgattgat 720
gaggtgtacc atcaattagt tgagaaaatg aaatcaattc ctggagccat ggtggagaac 780
aacaagttct gctgttctgt tcattttcgc tgcgttgatg aaaagaaatg gagtgaactg 840
gcacaggaag tgagatcagt attaaaagag tacccgaagc ttcgtcttaa ccaaggaagg 900
aaggtattag agattcgtcc atctattaaa tgggacaaag ggaaggcgct ggaatttttg 960
ttagagtcac ttggatttgc caactgtaac gatgtctttc ctgtttacat tggagatgat 1020
aaaaccgatg aagatgcatt caagaaatta agagacagag gacaaggttt tgggattctt 1080
gtctcgaaat ttccaaagga cactactgca tcatactctt tacaagaacc aaacgaggtg 1140
atggatttcc ttcaacgttt ggtggagtgg aaacaagtat ccctccgact cagaacacgt 1200
tctcgggtgt aaatggatag aatgataatg tacatcccta ccctgccata aaaaatggag 1260
ttcagctagg gtagcatata ggaccctttt ttctatgttt tgttcagatt agaacagaga 1320
acaaaaatgt atggaaaaag gaaagacaaa agtgtctttc tgagtttgaa gcacgtctac 1380
atggatgtaa ttagccacgt agtttttgta gtacagggga aacgtctctg ttgcatcaaa 1440
tgtaattctg aatggaaaat aacatgttac gtatcgaaaa atatatatta ttgt 1494

<210> 122
<211> 361
<212> PRT
<213> Glycine max

<400> 122
Met Thr Gln Asn Ala Ile Val Ser Lys Thr Lys Ser Gly Ile Asn Arg
1 5 10 15
Asp Ile Thr Val Pro Gln Lys Pro Leu Ala Ala Ala Ala Ala Ala Gly
20 25 30
Gly Tyr Ile Pro Ile Pro Arg Arg Arg Val Leu Lys Asn Leu Glu Ile
35 40 45
Asn Ala Trp Val Asp Ser Met Arg Ser Ser Ser Pro Thr Asn Ser Lys
50 55 60
Ser Thr Ser Ser Leu Ala Glu Glu His Ser Thr Trp Ile Leu Arg His
65 70 75 80
Pro Ser Ala Leu Asp Met Phe Glu Gln Ile Met Asp Ala Ser Arg Gly
85 90 95
Lys Gln Ile Val Met Phe Leu Asp Tyr Asp Gly Thr Leu Ser Pro Ile
100 105 110
Val Asp Asp Pro Asp Arg Ala Phe Met Ser Asp Ser Met Arg Arg Thr
115 120 125
Val Arg Lys Leu Ala Arg Cys Phe Pro Thr Ala Ile Val Thr Gly Arg
130 135 140
Cys Lys Asp Lys Val Tyr Asn Phe Val Arg Leu Ala Glu Leu Tyr Tyr
145 150 155 160
Ala Gly Ser His Gly Met Asp Ile Gln Gly Pro Thr Arg Asp Cys Lys
165 170 175
Tyr Ser Lys Asp Lys Gly Glu Pro Val Leu Phe Gln Pro Ala Ser Glu
180 185 190
Phe Leu Pro Met Ile Asp Glu Val Tyr His Gln Leu Val Glu Lys Met
195 200 205
Lys Ser Ile Pro Gly Ala Met Val Glu Asn Asn Lys Phe Cys Cys Ser
210 215 220
Val His Phe Arg Cys Val Asp Glu Lys Lys Trp Ser Glu Leu Ala Gln
225 230 235 240
Glu Val Arg Ser Val Leu Lys Glu Tyr Pro Lys Leu Arg Leu Asn Gln
245 250 255
Gly Arg Lys Val Leu Glu Ile Arg Pro Ser Ile Lys Trp Asp Lys Gly
260 265 270
Lys Ala Leu Glu Phe Leu Leu Glu Ser Leu Gly Phe Ala Asn Cys Asn
275 280 285
Asp Val Phe Pro Val Tyr Ile Gly Asp Asp Lys Thr Asp Glu Asp Ala
290 295 300
Phe Lys Lys Leu Arg Asp Arg Gly Gln Gly Phe Gly Ile Leu Val Ser
305 310 315 320
Lys Phe Pro Lys Asp Thr Thr Ala Ser Tyr Ser Leu Gln Glu Pro Asn
325 330 335
Glu Val Met Asp Phe Leu Gln Arg Leu Val Glu Trp Lys Gln Val Ser
340 345 350
Leu Arg Leu Arg Thr Arg Ser Arg Val
355 360

<210> 123
<211> 1441
<212> DNA
<213> Glycine max

<400> 123
acggctgatc tgggctcgac taaaacaaat cactaagtaa aaatgacgca gaatgcgata 60
gtgtccaaga caaaatcggg gatcaaccgg gacataaccg tgccacaaaa gccactggcg 120
gcggcggcgg cggcgggggg gtacattccc attccgagga ggagggtttt gaagaacctg 180
gaaatcaatg catgggttga ttccatgaga tcctcttctc caaccaattc caaatccacc 240

```
tcttctctcg cagaagaaca cagcacttgg attcttcgcc acccctcagc attagacatg    300
ttcgagcaaa ttatggatgc gtcccgggga aagcaaattg tcatgtttct cgactatgat    360
ggtactttgt cgcctattgt tgatgaccca gaccgtgctt tcatgtcgga ttcgatgagg    420
agaacggtga ggaaacttgc aaggtgtttt cctactgcta tagttaccgg cagatgcaaa    480
gacaaggttt acaattttgt tcgcttggct gagctatatt atgctggaag ccatggcatg    540
gatattcaag ggccaacaag aacctccaaa tacagcaaca aagataaagg agagcctgtt    600
ctttttcaac ctgctagtga atttcttccc atgattgatg aggtgtacca tcaattagtt    660
gagaaaatga aatcaattcc tggagccatg gtggagaaca acaagttctg ctgttctgtt    720
cattttcgct gcgttgatga aaagaaatgg agtgaactgg cacaggaagt gagatcagta    780
ttaaaagagt acccgaagct tcgtcttaac caaggaagga aggtattaga gattcgtcca    840
tctattaaat gggacaaagg gaaggcgctg gaatttttgt tagagtcact tggatttgcc    900
aactgtaacg atgtctttcc tgtttacatt ggagatgata aaaccgatga agatgcattc    960
aagaaattaa gagacagagg acaaggtttt gggattcttg tctcgaaatt tccaaaggac   1020
actactgcat catactcttt acaagaacca aacgaggtga tggatttcct tcaacgtttg   1080
gtggagtgga aacaagtatc cctccgactc agaacacgtt ctcgggtgta aatggataga   1140
atgataatgt acatccctac cctgccataa aaaatggagt tcagctaggg tagcatatag   1200
gacccttttt tctatgtttt gttcagatta gaacagagaa caaaaatgta tggaaaaagg   1260
aaagacaaaa gtgtctttct gagtttgaag cacgtctaca tggatgtaat tagccacgta   1320
gtttttgtag tacaggggaa acgtctctgt tgcatcaaat gtaattctga atggaaaata   1380
acatgttacg tatcgaaaaa tatatattat tgtatactct cttaagaaaa aaaaaaaaaa   1440
a                                                                    1441
```

<210> 124
<211> 362
<212> PRT
<213> Glycine max

<400> 124

```
Met Thr Gln Asn Ala Ile Val Ser Lys Thr Lys Ser Gly Ile Asn Arg
1               5                   10                  15
Asp Ile Thr Val Pro Gln Lys Pro Leu Ala Ala Ala Ala Ala Ala Gly
            20                  25                  30
Gly Tyr Ile Pro Ile Pro Arg Arg Arg Val Leu Lys Asn Leu Glu Ile
        35                  40                  45
Asn Ala Trp Val Asp Ser Met Arg Ser Ser Ser Pro Thr Asn Ser Lys
    50                  55                  60
Ser Thr Ser Ser Leu Ala Glu Glu His Ser Thr Trp Ile Leu Arg His
65                  70                  75                  80
Pro Ser Ala Leu Asp Met Phe Glu Gln Ile Met Asp Ala Ser Arg Gly
                85                  90                  95
Lys Gln Ile Val Met Phe Leu Asp Tyr Asp Gly Thr Leu Ser Pro Ile
            100                 105                 110
Val Asp Asp Pro Asp Arg Ala Phe Met Ser Asp Ser Met Arg Arg Thr
        115                 120                 125
Val Arg Lys Leu Ala Arg Cys Phe Pro Thr Ala Ile Val Thr Gly Arg
    130                 135                 140
Cys Lys Asp Lys Val Tyr Asn Phe Val Arg Leu Ala Glu Leu Tyr Tyr
145                 150                 155                 160
Ala Gly Ser His Gly Met Asp Ile Gln Gly Pro Thr Arg Thr Ser Lys
                165                 170                 175
Tyr Ser Asn Lys Asp Lys Gly Glu Pro Val Leu Phe Gln Pro Ala Ser
            180                 185                 190
Glu Phe Leu Pro Met Ile Asp Glu Val Tyr His Gln Leu Val Glu Lys
        195                 200                 205
Met Lys Ser Ile Pro Gly Ala Met Val Glu Asn Asn Lys Phe Cys Cys
    210                 215                 220
Ser Val His Phe Arg Cys Val Asp Glu Lys Lys Trp Ser Glu Leu Ala
225                 230                 235                 240
Gln Glu Val Arg Ser Val Leu Lys Glu Tyr Pro Lys Leu Arg Leu Asn
```

```
                245                 250                 255
Gln Gly Arg Lys Val Leu Glu Ile Arg Pro Ser Ile Lys Trp Asp Lys
            260                 265                 270
Gly Lys Ala Leu Glu Phe Leu Leu Glu Ser Leu Gly Phe Ala Asn Cys
        275                 280                 285
Asn Asp Val Phe Pro Val Tyr Ile Gly Asp Asp Lys Thr Asp Glu Asp
    290                 295                 300
Ala Phe Lys Lys Leu Arg Asp Arg Gly Gln Gly Phe Gly Ile Leu Val
305                 310                 315                 320
Ser Lys Phe Pro Lys Asp Thr Thr Ala Ser Tyr Ser Leu Gln Glu Pro
                325                 330                 335
Asn Glu Val Met Asp Phe Leu Gln Arg Leu Val Glu Trp Lys Gln Val
            340                 345                 350
Ser Leu Arg Leu Arg Thr Arg Ser Arg Val
        355                 360

<210>  125
<211>  1729
<212>  DNA
<213>  Glycine max

<400>  125
ttcccggcct cactcacccc tccctttat ttccattatt attctgccta agcagtttct      60
tccaaacttc cttttacatt tccaatttct ctattctatc aaaagggttt gaactttgaa    120
gggaaaggaa gaaagatatg atgacgaacc aaaatgtggt gactcatgag gttattaaca    180
cgttgattgc cgtggcagct tccatttcaa actcaaccgc gttgccaagt gcaacagtgc    240
cagaatccat ggctgtgctt ggtgggtttt gggggctgcc ccataataaa aatcttgtga    300
aaaggttgga aggagctaaa gttagtgctt ggattgattc aatgagagct tcttccccaa    360
ctcgtgccaa atcagaaagc caagaaaaaa gatcttggat tctttatcac ccttcagctc    420
tgaacacgtt tgagcaaata gtatgtagtg ccaaaggaaa gcaagtcgta gtttttcttg    480
actacgatgg aactctctcc ccaattgttg cagatccgga taaagctttc atgactagaa    540
agatgagagc aacgctaaag ggcatagcaa ggcattttcc cacagcaata gtgaccggaa    600
ggtgcagaga caaggtatat aactttgtaa aattggcaga actttactat gccggaagcc    660
atggcatgga catcaagggt ccaacaaaaa gccaaagtcc aaagcaaggt aataataata    720
aagcagtgct gttccaaccc gcgagtcaat tcctgccaat gatcgatgag gtgtacaaga    780
tcttgttaga aaaaacaaag actgtcccag ggctaatgt tgagaacaat aagttttgct     840
tgtccgtgca ctttcgttgt gttgacgaaa agagttgggc agcgttggcg gagaaagtta    900
gattggtgct caatgattac ccacaactta ggctaaccca agggagaaaa gtgctagaga    960
ttcgtccaac catcaaatgg gacaagggca aggctcttga atttttgtta gaatcattag   1020
gatacgagaa ttcgaatgat gtatttccaa tatatattgg tgatgatcga actgatgagg   1080
atgcttttaa ggttttgcgc agtaggggtc aaggaattgg gattcttgtt tctagagttg   1140
caaaagaaac agatgcttcc tataccttgc aagatccatc agaggcaagt gctatatatt   1200
ccatccagta caatttattc tatataatat ttttaatgtt taattcgggc atcaatgttg   1260
tatatcttta ttgtgaatgg tgaatctgag aaatatataa tgtaattaat taacaaatat   1320
ctttatggcc acatttacag gttgagcaat tcttgcggcg tctggtggag tggaaaagac   1380
cgagtactgt gactcccaca agtgtataga gagtttgtag aatgtagatg atcacttcaa   1440
agaattgaca ccaccaccac cttagaatgg tgaagaggtg gatcgaattg tatcactttt   1500
ttttattgtt gaaaatggaa atagcactat tccataattt aaatttatta aggacaaagt   1560
ccgaacaaat agattcctac acacgtttgc atgcgcatgc ggatagggaa aggcagatgt   1620
tttatgccgc agttgcaaat ggcccgtcaa ctttgttgct aagaattgta cttatcgtac   1680
atgtggccaa tatattctga aaaagattac tacgaaaaaa aaaaaaaaa              1729

<210>  126
<211>  381
<212>  PRT
<213>  Glycine max

<400>  126
Met Met Thr Asn Gln Asn Val Val Thr His Glu Val Ile Asn Thr Leu
```

```
1               5                   10                  15
Ile Ala Val Ala Ala Ser Ile Ser Asn Ser Thr Ala Leu Pro Ser Ala
            20                  25                  30
Thr Val Pro Glu Ser Met Ala Val Leu Gly Gly Phe Trp Gly Leu Pro
        35                  40                  45
His Asn Lys Asn Leu Val Lys Arg Leu Glu Gly Ala Lys Val Ser Ala
    50                  55                  60
Trp Ile Asp Ser Met Arg Ala Ser Ser Pro Thr Arg Ala Lys Ser Glu
65                  70                  75                  80
Ser Gln Glu Lys Arg Ser Trp Ile Leu Tyr His Pro Ser Ala Leu Asn
                85                  90                  95
Thr Phe Glu Gln Ile Val Cys Ser Ala Lys Gly Lys Gln Val Val Val
            100                 105                 110
Phe Leu Asp Tyr Asp Gly Thr Leu Ser Pro Ile Val Ala Asp Pro Asp
        115                 120                 125
Lys Ala Phe Met Thr Arg Lys Met Arg Ala Thr Leu Lys Gly Ile Ala
    130                 135                 140
Arg His Phe Pro Thr Ala Ile Val Thr Gly Arg Cys Arg Asp Lys Val
145                 150                 155                 160
Tyr Asn Phe Val Lys Leu Ala Glu Leu Tyr Tyr Ala Gly Ser His Gly
                165                 170                 175
Met Asp Ile Lys Gly Pro Thr Lys Ser Gln Ser Pro Lys Gln Gly Asn
            180                 185                 190
Asn Asn Lys Ala Val Leu Phe Gln Pro Ala Ser Gln Phe Leu Pro Met
        195                 200                 205
Ile Asp Glu Val Tyr Lys Ile Leu Leu Glu Lys Thr Lys Thr Val Pro
    210                 215                 220
Gly Ala Asn Val Glu Asn Asn Lys Phe Cys Leu Ser Val His Phe Arg
225                 230                 235                 240
Cys Val Asp Glu Lys Ser Trp Ala Ala Leu Ala Glu Lys Val Arg Leu
                245                 250                 255
Val Leu Asn Asp Tyr Pro Gln Leu Arg Leu Thr Gln Gly Arg Lys Val
            260                 265                 270
Leu Glu Ile Arg Pro Thr Ile Lys Trp Asp Lys Gly Lys Ala Leu Glu
        275                 280                 285
Phe Leu Leu Glu Ser Leu Gly Tyr Glu Asn Ser Asn Asp Val Phe Pro
    290                 295                 300
Ile Tyr Ile Gly Asp Asp Arg Thr Asp Glu Asp Ala Phe Lys Val Leu
305                 310                 315                 320
Arg Ser Arg Gly Gln Gly Ile Gly Ile Leu Val Ser Arg Val Ala Lys
                325                 330                 335
Glu Thr Asp Ala Ser Tyr Thr Leu Gln Asp Pro Ser Glu Ala Ser Ala
            340                 345                 350
Ile Tyr Ser Ile Gln Tyr Asn Leu Phe Tyr Ile Ile Phe Leu Met Phe
        355                 360                 365
Asn Ser Gly Ile Asn Val Val Tyr Leu Tyr Cys Glu Trp
    370                 375                 380
```

<210> 127
<211> 1448
<212> DNA
<213> Brassica napus

<400> 127

```
ttaacttctg cctcgcctct cgttctcaac acttgtgaga aatatgatga accagaatgt     60
catcgtctcc gacagaaaag ccatcttggg ttcgaaaacc atcactgtct ctaactctcc    120
cctgttctct tctcctccca cttactttac ctttcctcgt cataagttct tggagcttct    180
cgaagcagcc gataaaaaca gcaacagcat caacaagaac aatcttggtg ctggtaagat    240
tgcatcttgg gtcgattcca tgcgtgattc ttctcctaca cgtctcagac cctcttcacg    300
```

```
tgactctgtg tcagacaacg accataaaac atcttggatc gttcgatttc catcggcttt     360
aaatatgttt gatgagattg tgaatgctgc aaaagggaaa caaattgtta tgtttcttga     420
ttacgatggg acgctctctc ccatagttga agatcctgac aaagcttaca taacacatga     480
gatgcgagaa gttgtaaaga atgtggctct aaacttccca actgctatag tcactggaag     540
atccattgat aaggttcgtg gttttgtcaa actcgatgag atttactacg ctggaagcca     600
tggcatggac attgaaggcc cgaccagcga atatgcttat ggtggcgaga gtaatcaagg     660
agtgctcttt caacctgctc gtgaatttgt acccacgatc gagaaggtgt ataagatatt     720
agaggaaaag actaaatgga tccctggggc tatggtggag aacaacaagt tttgtctgtc     780
cgtacatttt cgacgtgttg atgagaaaag atgggccgga ttagccgaac aagtaaaatc     840
agttctaatt gattatccac agctgaaact aacccaaggt agaaaggtgc ttgaaatccg     900
tcctacgatc aaatgggaca agggccaggc tctcaatttt ttgctaaaat cattagggtt     960
tgaaaagtcg gaagatgttg ttcctgtgta tattggagat gacctcaccg acgaagatgc    1020
gtttaaggtt ttacgtgagc ggggacaagg ttttgggatt ctagtctcaa aagttccaaa    1080
ggaaaccaat gcctcttact ctctccaaga cccttctcag gttaatgagt ttctgaggcg    1140
tttagtagag tggaagagga agacagtcgg ggaagcttga aaaacaaaca tgcaacatta    1200
ataacacctg agtttatttt ataaatcttg aggagaaaat aatcggtata tatagacaac    1260
cttctaaaaa actagtacta gtactagatt cgtagagggt gttttttgg aactttacct    1320
agagaggtgt cttggccaga agcatcttta cttttctaca tcgatcgaga aattgtaaat    1380
ttcgtgtaac gattcagaaa atgaagaaaa cacaactaat atcatttcca cgcaaaaaaa    1440
aaaaaaaa                                                             1448
```

<210> 128
<211> 378
<212> PRT
<213> Brassica napus

<400> 128

```
Met Met Asn Gln Asn Val Ile Val Ser Asp Arg Lys Ala Ile Leu Gly
1               5                   10                  15
Ser Lys Thr Ile Thr Val Ser Asn Ser Pro Leu Phe Ser Ser Pro Pro
            20                  25                  30
Thr Tyr Phe Thr Phe Pro Arg His Lys Phe Leu Glu Leu Leu Glu Ala
        35                  40                  45
Ala Asp Lys Asn Ser Asn Ser Ile Asn Lys Asn Asn Leu Gly Ala Gly
    50                  55                  60
Lys Ile Ala Ser Trp Val Asp Ser Met Arg Asp Ser Ser Pro Thr Arg
65                  70                  75                  80
Leu Arg Pro Ser Ser Arg Asp Ser Val Ser Asp Asn Asp His Lys Thr
                85                  90                  95
Ser Trp Ile Val Arg Phe Pro Ser Ala Leu Asn Met Phe Asp Glu Ile
            100                 105                 110
Val Asn Ala Ala Lys Gly Lys Gln Ile Val Met Phe Leu Asp Tyr Asp
        115                 120                 125
Gly Thr Leu Ser Pro Ile Val Glu Asp Pro Asp Lys Ala Tyr Ile Thr
    130                 135                 140
His Glu Met Arg Glu Val Val Lys Asn Val Ala Leu Asn Phe Pro Thr
145                 150                 155                 160
Ala Ile Val Thr Gly Arg Ser Ile Asp Lys Val Arg Gly Phe Val Lys
                165                 170                 175
Leu Asp Glu Ile Tyr Tyr Ala Gly Ser His Gly Met Asp Ile Glu Gly
            180                 185                 190
Pro Thr Ser Glu Tyr Ala Tyr Gly Gly Glu Ser Asn Gln Gly Val Leu
        195                 200                 205
Phe Gln Pro Ala Arg Glu Phe Val Pro Thr Ile Glu Lys Val Tyr Lys
    210                 215                 220
Ile Leu Glu Glu Lys Thr Lys Trp Ile Pro Gly Ala Met Val Glu Asn
225                 230                 235                 240
Asn Lys Phe Cys Leu Ser Val His Phe Arg Arg Val Asp Glu Lys Arg
                245                 250                 255
```

```
Trp Ala Gly Leu Ala Glu Gln Val Lys Ser Val Leu Ile Asp Tyr Pro
            260                 265                 270
Gln Leu Lys Leu Thr Gln Gly Arg Lys Val Leu Glu Ile Arg Pro Thr
        275                 280                 285
Ile Lys Trp Asp Lys Gly Gln Ala Leu Asn Phe Leu Leu Lys Ser Leu
    290                 295                 300
Gly Phe Glu Lys Ser Glu Asp Val Val Pro Val Tyr Ile Gly Asp Asp
305                 310                 315                 320
Leu Thr Asp Glu Asp Ala Phe Lys Val Leu Arg Glu Arg Gly Gln Gly
                325                 330                 335
Phe Gly Ile Leu Val Ser Lys Val Pro Lys Glu Thr Asn Ala Ser Tyr
            340                 345                 350
Ser Leu Gln Asp Pro Ser Gln Val Asn Glu Phe Leu Arg Arg Leu Val
        355                 360                 365
Glu Trp Lys Arg Lys Thr Val Gly Glu Ala
    370                 375
```

```
<210>  129
<211>  1448
<212>  DNA
<213>  Brassica napus

<400>  129
ttaacttctg cctcgcctct cgttctcaac acttgtgaga aatatgatga accagaatgt      60
catcgtctcc gacagaaaag ccatcttggg ttcgaaaacc atcactgtct ctaactctcc     120
cctgttctct tctcctccca cttactttac ctttcctcgt cataagttct tggagcttct     180
cgaagcagcc gataaaaaca gcaacagcat caacaagaac aatcttggtg ctggtaagat     240
tgcatcttgg gtcgattcca tgcgtgattc ttctcctaca cgtctcagac cctcttcacg     300
tgactctgtg tcagacaacg accataaaac atcttggatc gttcgatttc catcggcttt     360
aaatatgttt gatgagattg tgaatgctgc aaaagggaaa caaattgtta tgtttcttga     420
ttacgatggg acgctctctc ccatagttga agatcctgac aaagcttaca taacacatga     480
gatgcgagaa gttgtaaaga atgtggctct aaacttccca actgctatag tcactggaag     540
atccattgat aaggttcgtg gttttgtcaa actcgatgag atttactacg ctggaagcca     600
tggcatggac attgaaggcc cgaccagcga atatgcttat ggtggcgaga gtaatcaagg     660
agtgctcttt caacctgctc gtgaatttgt acccacgatc gagaaggtgt ataagatatt     720
agaggaaaag actaaatgga tccctggggc tatggtggag aacaacaagt tttgtctgtc     780
cgtacatttt cgacgtgttg atgagaaaag atgggccgga ttagccgaac aagtaaaatc     840
agttctaatt gattatccac agctgaaact aacccaaggt agaaaggtgc ttgaaatccg     900
tcctacgatc aaatgggaca agggccaggc tctcaatttt ttgctaaaat cattagggtt     960
tgaaaagtcg gaagatgttg ttcctgtgta tattggagat gaccgtaccg acgaagatgc    1020
gtttaaggtt ttacgtgagc ggggacaagg ttttgggatt ctagtctcaa agttccaaa     1080
ggaaaccaat gcctcttact ctctccaaga cccttctcag gttaatgagt ttctgaggcg    1140
tttagtagag tggaagagga agacagtcgg ggaagcttga aaaacaaaca tgcaacatta    1200
ataacacctg agtttatttt ataaatcttg aggagaaaat aatcggtata tatagacaac    1260
cttctaaaaa actagtacta gtactagatt cgtagagggt gtttttttgg aactttacct    1320
agagaggtgt cttggccaga agcatcttta cttttctaca tcgatcgaga aattgtaaat    1380
ttcgtgtaac gattcagaaa atgaagaaaa cacaactaat atcatttcca cgcaaaaaaa    1440
aaaaaaaa                                                             1448

<210>  130
<211>  378
<212>  PRT
<213>  Brassica napus

<400>  130
Met Met Asn Gln Asn Val Ile Val Ser Asp Arg Lys Ala Ile Leu Gly
1               5                   10                  15
Ser Lys Thr Ile Thr Val Ser Asn Ser Pro Leu Phe Ser Ser Pro Pro
            20                  25                  30
```

Thr Tyr Phe Thr Phe Pro Arg His Lys Phe Leu Glu Leu Leu Glu Ala
35 40 45
Ala Asp Lys Asn Ser Asn Ser Ile Asn Lys Asn Asn Leu Gly Ala Gly
50 55 60
Lys Ile Ala Ser Trp Val Asp Ser Met Arg Asp Ser Ser Pro Thr Arg
65 70 75 80
Leu Arg Pro Ser Ser Arg Asp Ser Val Ser Asp Asn Asp His Lys Thr
85 90 95
Ser Trp Ile Val Arg Phe Pro Ser Ala Leu Asn Met Phe Asp Glu Ile
100 105 110
Val Asn Ala Ala Lys Gly Lys Gln Ile Val Met Phe Leu Asp Tyr Asp
115 120 125
Gly Thr Leu Ser Pro Ile Val Glu Asp Pro Asp Lys Ala Tyr Ile Thr
130 135 140
His Glu Met Arg Glu Val Val Lys Asn Val Ala Leu Asn Phe Pro Thr
145 150 155 160
Ala Ile Val Thr Gly Arg Ser Ile Asp Lys Val Arg Gly Phe Val Lys
165 170 175
Leu Asp Glu Ile Tyr Tyr Ala Gly Ser His Gly Met Asp Ile Glu Gly
180 185 190
Pro Thr Ser Glu Tyr Ala Tyr Gly Gly Glu Ser Asn Gln Gly Val Leu
195 200 205
Phe Gln Pro Ala Arg Glu Phe Val Pro Thr Ile Glu Lys Val Tyr Lys
210 215 220
Ile Leu Glu Glu Lys Thr Lys Trp Ile Pro Gly Ala Met Val Glu Asn
225 230 235 240
Asn Lys Phe Cys Leu Ser Val His Phe Arg Arg Val Asp Glu Lys Arg
245 250 255
Trp Ala Gly Leu Ala Glu Gln Val Lys Ser Val Leu Ile Asp Tyr Pro
260 265 270
Gln Leu Lys Leu Thr Gln Gly Arg Lys Val Leu Glu Ile Arg Pro Thr
275 280 285
Ile Lys Trp Asp Lys Gly Gln Ala Leu Asn Phe Leu Leu Lys Ser Leu
290 295 300
Gly Phe Glu Lys Ser Glu Asp Val Val Pro Val Tyr Ile Gly Asp Asp
305 310 315 320
Arg Thr Asp Glu Asp Ala Phe Lys Val Leu Arg Glu Arg Gly Gln Gly
325 330 335
Phe Gly Ile Leu Val Ser Lys Val Pro Lys Glu Thr Asn Ala Ser Tyr
340 345 350
Ser Leu Gln Asp Pro Ser Gln Val Asn Glu Phe Leu Arg Arg Leu Val
355 360 365
Glu Trp Lys Arg Lys Thr Val Gly Glu Ala
370 375

<210> 131
<211> 1130
<212> DNA
<213> Oryza sativa

<400> 131
catgcggcta atgtagatgc tcactgcgct agtagtaagg tactccagta cattatggaa 60
tatacaaagc tgtaatactc gtatcagcaa gagagaggca cacaagttgt agcagtagca 120
caggattaga aaaacgggac gacaaatagt aatggaaaaa caaaaaaaaa caaggaaaca 180
catggcaata taaatggaga aatcacaaga ggaacagaat ccgggcaata cgctgcgaaa 240
gtactcgtac gtaaaaaaaa gaggcgcatt catgtgtgga cagcgtgcag cagaagcagg 300
gatttgaaac cactcaaatc caccactgca aaccttcaaa cgaggccatg gtttgaagca 360
tagaaagcac aggtaagaag cacaacgccc tcgctctcca ccctcccacc caatcgcgac 420
gcacctcgcg gatcggtgac gtggcctcgc cccccaaaaa tatcccgcgg cgtgaagctg 480

```
acaccccggg cccacccacc tgtcacgttg gcacatgttg gttatggttc ccggccgcac    540
caaaatatca acgcggcgcg gcccaaaatt tccaaaatcc cgcccaagcc cctggcgcgt    600
gccgctcttc cacccaggtc cctctcgtaa tccataatgg cgtgtgtacc ctcggctggt    660
tgtacgtggg cgggttaccc tgggggtgtg ggtggatgac gggtgggccc ggaggaggtc    720
cggccccgcg cgtcatcgcg gggcggggtg tagcgggtgc gaaaaggagg cgatcggtac    780
gaaaattcaa attaggaggt gggggcgggg gcccttggag aataagcgga atcgcagata    840
tgcccctgac ttggcttggc tcctcttctt cttatccctt gtcctcgcaa ccccgcttcc    900
ttctctcctc tcctcttctc ttctcttctc tggtggtgtg ggtgtgtccc tgtctcccct    960
ctccttcctc ctctcctttc ccctcctctc ttccccccctc tcacaagaga gagagcgcca   1020
gactctcccc aggtgaggtg agaccagtct ttttgctcga ttcgacgcgc ctttcacgcc   1080
gcctcgcgcg gatctgaccg cttccctcgg ccttctcgca ggattcagcc              1130
```

## Description

The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding a Class III Trehalose Phosphate Phosphatase (TPP) polypeptide. The present invention also concerns plants having modulated expression of a nucleic acid encoding a Class III TPP polypeptide, which plants have enhanced yield-related traits relative to control plants. The invention further concerns novel Class III TPP nucleic acid and polypeptide sequences. The invention also provides nucleic acid and polypeptide sequences, and constructs comprising the same, which are useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as, corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Another yield-related trait of importance is plant biomass, particularly in the case of forage crops, such as alfalfa, silage corn and hay. A larger plant with a greater leaf area can typically absorb more light and carbon dioxide than a smaller plant and therefore will likely gain a greater weight during the same period (Fasoula & Tollenaar 2005 Maydica 50:39).

The ability to increase plant yield would have many applications in areas such as agriculture, including in the production of ornamental plants, arboriculture, horticulture and forestry. Increasing yield may also find use in the production of algae for use in bioreactors (for the biotechnological production of substances such as pharmaceuticals, antibodies or vaccines, or for the bioconversion of organic waste) and other such areas.

Plant breeders are often interested in improving specific aspects of yield depending on the crop or plant in question, and the part of that plant or crop which is of economic value. For example, for certain crops, or for certain end uses, a plant breeder may look specifically for improvements in plant biomass (weight) of one or more parts of a plant, which may include aboveground (harvestable) parts and/or (harvestable) parts below ground. This is particularly relevant where the aboveground parts or below ground parts of a plant are for consumption. For many crops, particularly cereals, it is an improvement in seed yield that is highly desirable. Increased seed yield may manifest itself in many ways, with each individual aspect of seed yield being of varying importance to a plant breeder depending on the crop or plant in question and its end use.

It would be of great advantage to a plant breeder to be able to be able to pick and choose the aspects of yield or seed yield to be altered. It would be highly desirable to be able to pick off the shelf, so to speak, a gene suitable for altering a particular aspect, or component, of seed yield. For example an increase in the fill rate, combined with increased thousand kernel weight would be highly desirable for a crop such as corn. For rice and wheat a combination of increased fill rate, harvest index and increased thousand kernel weight would be highly desirable.

It has now been found that modulating expression in a plant of a nucleic acid encoding a Class III Trehalose Phosphate Phosphatase (TPP) polypeptide gives plants having various enhanced yield-related traits.

Trehalose, a non-reducing disaccharide consisting of two glucose molecules linked via alpha-1,1 bonds, is found in several life kingdoms. In bacteria, fungi and insects, trehalose has been shown to have a carbohydrate storage function and to play a role in stress tolerance. In plants, trehalose was initially thought to be confined to extremophites, such as the resurrection plant Selaginella lepidophylla, however it is now widely accepted that trehalose metabolism is ubiquitous in the plant kingdom.

Trehalose is synthesized from UDP-glucose and Glucose-6-phosphate in two enzymatic reactions. In a first step, UDP-glucose and Glucose-6-phosphate are converted to UDP (uridine diphosphate) and alpha, alpha-trehalose 6-phosphate (T-6-P) by the enzyme TPS, trehalose phosphate synthase. In a second step, which is catalyzed by the enzyme TPP (trehalose phosphate phosphatase), T-6-P is de-phosphorylated to produce trehalose and orthophosphate.

In yeast, the two enzymatic activities (TPS and TPP activity) reside in a large protein complex, containing the active subunits, TPS1 and TPS2, and the regulatory subunits, with TPS1 having TPS activity and TPS2 having TPP activity. In *E. coli,* the two enzymatic activities are found in separate protein complexes. In plants, the protein complex has not been characterized to date.

In *Arabidopsis thaliana,* trehalose biosynthetic enzymes have been classified into three classes:

**Class I:** containing four genes, AtTPS1 to AtTPS4 having high similarity to ScTPS1;
**Class II:** having seven members, AtTPS5 to AtTPS11, with high sequence similarity to ScTPS2; and
**Class III:**, containing 10 members, AtTPPA to AtTPPJ, encoding proteins with similarity to *E. coli* TPS2 and the C-terminus of ScTPS2 proteins.

Genes encoding proteins within these classes are also present in other plant species.

Within Class I and Class II, enzymatic activity has only been unambiguously determined for AtTPS1, which displays TPS activity (Blazquez et al. Plant J. 1998 Mar;13(5):685-9.). Surprisingly, no TPP activity has been reported to date for any of the other Class II TPS proteins. In contrast, TPP activity was previously described for A*t*TPPA and A*t*TPPB, two of the members of Class III (Vogel et al. Plant J. 1998 Mar;13(5):673-83). Plant Class III TPPs contain two phosphatase consensus sequence motifs found in all TPP enzymes described to date (Thaller et al. Protein Sci. 1998 Jul;7(7):1647-52).

The genetic manipulation of trehalose biosynthesis genes has been reported to lead to improved stress tolerance in plants, as well as causing striking developmental alterations. Overexpression of *E.coli* OtsA and OtsB genes in transgenic tobacco and potato plants was reported to cause developmental aberrations in roots and leaves, and plant stunting. Fewer seeds were produced in the OtsA transgenic tobacco plants, and the OtsB transgenic potato plants did not produced tubers (Goddijn et al. Plant Physiol. 1997 Jan;113(1):181-90). Similar results have been described by others (Holmstrom et al. Nature, 379, 683-684; Romero et al. Planta, 201, 293-297; Pilont-Smits et al. 1998; J Plant Physiol. 152:525-532; Schluepmann et al. Proc Natl Acad Sci U S A. 2003;100(11):6849-54). Mutants defective in TPS and TPP genes have also reportedly shown developmental defects. TPS1 knock out mutants in *Arabidopsis* showed impaired embryo development *(*Eastmond et a/. Plant J. 2002 Jan;29(2):225-35*).*

Published US patent application, US 20060191040, in the name of Jackson et al. mentions the isolation and characterization of a maize gene, RAMOSA3 (RA3), reported to be responsible for meristem development and inflorescence development including branching. It is suggested that the gene, gene product, and regulatory regions may be used to manipulate branching, meristem growth, inflorescence development and arrangement, and ultimately to improve yield of plants. Although altered architecture (due to a change in branching or a change in the inflorescence structure) can in some cases contribute to increased yield or increased seed yield, this is by no means certain. There are many other components which would need to be in place before an increase in yield or seed yield could be realised. For example, without seed set, seed filling, fertility of a plant etc. there would be no increase in seed yield. Furthermore, Jackson *et al.* does not mention which aspects of yield may be improved, whether that be biomass of specific parts of a plant, improved seed yield (which could be seed size, seed number, thousand kernel weight, harvest index, or other seed yield-related parameters).

It was therefore surprising to find that modulating expression in a plant of a Class III TPP polynucleotide gives plants having various enhanced yield-related traits, particularly increased seed yield relative to control plants.

Any reference hereinafter to a "protein useful in the methods of the invention" is taken to mean a Class III TPP polypeptide as defined herein. Any reference hereinafter to a "nucleic acid useful in the methods of the invention" is taken to mean a nucleic acid capable of encoding such a Class III TPP polypeptide. The terms "polypeptide" and "protein" are used interchangeably herein and refer to amino acids in a polymeric form of any length. The terms "polynucleotide (s)", "nucleic acid sequence(s)", "nucleotide sequence(s)", "polynucleotide molecule(s)" are used interchangeably herein and refer to nucleotides, either ribonucleotides or deoxyribonucleotides or a combination of both, in a polymeric form of any length.

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. A "control plant" as used herein refers not only to whole plants, but also to plant parts, including seeds and seed parts.

A preferred method for modulating (preferably, increasing) expression of a nucleic acid encoding a protein useful in the methods of the invention is by introducing and expressing in a plant a nucleic acid encoding a protein useful in the methods of the invention as defined below.

The nucleic acid to be introduced into a plant (and therefore useful in performing the methods of the invention) is any nucleic acid encoding the type of protein, which will now be described, herein also named "Class III TPP nucleic acid" or "Class III TPP gene" or a "Class III TPP polynucleotide".

A "Class III TPP polypeptide" as defined herein refers to any polypeptide with trehalose-6-phosphate phosphatase activity comprising at least one Trehalose-phosphatase (Trehalose-PPase) domain.

Trehalose-PPase domains are typically between 200 and 250 amino acids in length and typically comprise a phosphatase consensus sequence motif that is found in all TPP enzymes described to date (Thaller et al. 1998). A representative consensus sequence for the Trehalose-PPase domain is given in SEQ ID NO: 93. The amino acid sequence for the Trehalose-PPase domain comprised in SEQ ID NO: 2 is given in SEQ ID NO: 94. A person skilled in the art will readily be able to identify the presence of a Trehalose-PPase domain using tools and techniques known in the art. Examples 2, 3 and 4 herein provide further details concerning the identification of a Trehalose-PPase domain. In Class III TPP polypeptides, this phosphatase consensus sequence motif typically comprises two phosphatase boxes, named A and B-Phosphatase Box. SEQ ID NO: 96 represents a consensus sequence for Phosphatase box A and SEQ ID NO: 97 represents a consensus sequence for Phosphatase box B.

Nucleic acids useful in the methods of the invention encode Class III TPP polypeptides comprising at least one Trehalose-PPase domain, which domain preferably has, in increasing order of preference, at least 50%, 55%, 60%, 65%, 70%, 75%, 80%, 85%, 90%, 95% or 98% or more amino acid sequence identity to either SEQ ID NO: 93 or SEQ ID NO: 94. Further preferably, the Trehalose-PPase domain comprises at least one (preferably two) phosphatase box (es) having, in increasing order of preference, at least 50%, 55%, 60%, 65%, 70%, 75%, 80%, 85%, 90%, 95% or 98% or more sequence identity to the phosphatase boxes or either SEQ ID NO: 96 or SEQ ID NO: 97.

Class III TPP polypeptides may also comprise a serine-rich region, typically located in the N-terminus of the Trehalose-PPase domain. SEQ ID NO: 95 represents a consensus sequence for the serine-rich domain. Preferably, nucleic acids useful in the methods of the invention encode Class III TPP polypeptides comprising a serine-rich domain having, in increasing order of preference, at least 50%, 55%, 60%, 65%, 70%, 75%, 80%, 85%, 90%, 95% or 98% or more sequence identity to SEQ ID NO: 95.

Preferably, the nucleic acid to be introduced into a plant encodes a Class III TPP protein having, in increasing order of preference, at least 80%, 85%, 90%, 95%, 96%, 97%, 98%, 99% or 100% sequence identity to a sequence selected from SEQ ID NOs 4, 6 and 8. Most preferably, the Class III TPP nucleic acid is as represented by any of SEQ ID NOs 3, 5 and 7.

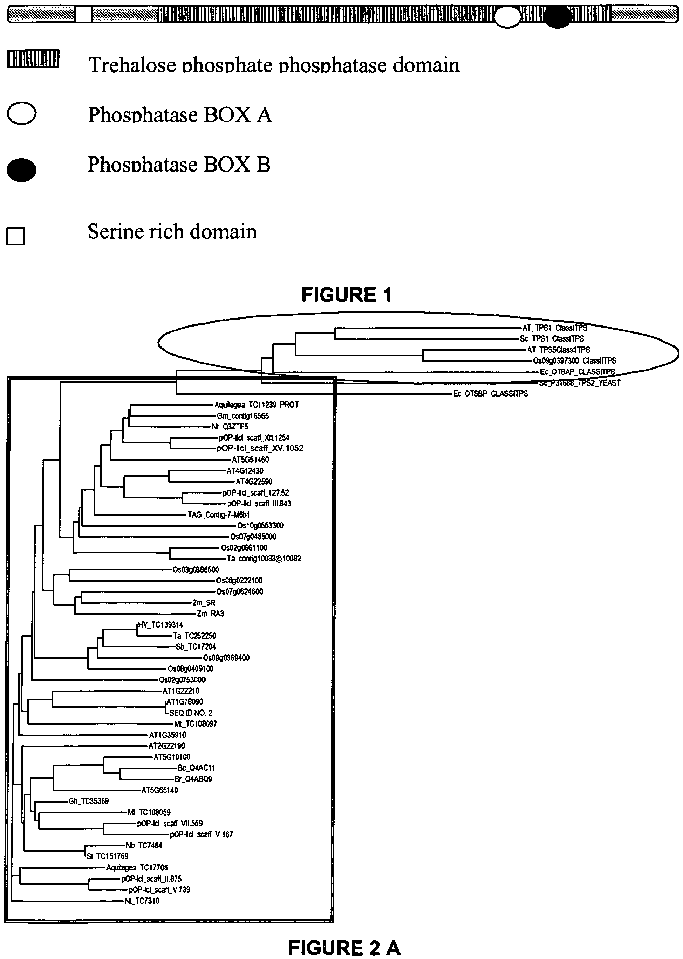

Typically, a Class III TPP polypeptide, when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

Examples of proteins useful in the methods of the invention and nucleic acids encoding the same are provided herein in Table A of Example 1.

Also useful in the methods of the invention are homologues of any of the amino acid sequences given in Table A of Example 1. "Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein.

Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gin | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols.

Also useful in the methods of the invention are derivatives of any one of the polypeptides given in Table A of Example 1 or orthologues or paralogues of any of the polypeptides given in Table A of Example 1 or derivatives of any orthologues or paralogues of any of the polypeptides given in Table A. "Derivatives" include peptides, oligopeptides, polypeptides which may, compared to the amino acid sequence of the naturally-occurring form of the protein, such as the one presented in SEQ ID NO: 2, comprise substitutions of amino acids with non-naturally occurring amino acid residues, or additions of non-naturally occurring amino acid residues. Derivatives of the polypeptides given in Table A of Example 1 are further examples which may be suitable for use in the methods of the invention. Derivatives useful in the methods of the present invention preferably have similar biological and functional activity as the unmodified protein from which they are derived.

"Derivatives" of a polypeptide include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

The invention is illustrated by transforming plants with the *Arabidopsis thaliana* nucleic acid sequence represented by SEQ ID NO: 1, encoding the polypeptide sequence of SEQ ID NO: 2, however performance of the invention is not restricted to these sequences. The methods of the invention may advantageously be performed using any nucleic acid encoding a protein useful in the methods of the invention as defined herein, including nucleic acids encoding orthologues, paralogues and homologues of SEQ ID NO: 2, such as (but not limited to) any of the nucleic acid sequences given in Table A of Example 1.

The amino acid sequences given in Table A of Example 1 are examples of orthologues and paralogues of the Class III TPP polypeptide represented by SEQ ID NO: 2, and nucleic acids encoding the same are useful in the methods of the invention. Orthologues and paralogues encompass evolutionary concepts used to describe the ancestral relationships of genes. Paralogues are genes within the same species that have originated through duplication of an ancestral gene and orthologues are genes from different organisms that have originated through speciation, and are also derived from a common ancestral gene.

Orthologues and paralogues may easily be found by performing a so-called reciprocal blast search. Typically, this involves a first BLAST involving BLASTing a query sequence (for example using any of the sequences listed in Table A of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence amongst the highest hit; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Table A of Example 1 gives examples of orthologues and paralogues of the Class III TPP protein represented by SEQ ID NO 2. Further orthologues and paralogues may readily be identified using the BLAST procedure described above.

The proteins of the invention are identifiable by the presence of the conserved Trehalose-PPase domain(s) (shown in Figure 1 and Example 4). The term "domain" refers to a set of amino acids conserved at specific positions along an alignment of sequences of evolutionarily related proteins. While amino acids at other positions can vary between homologues, amino acids that are highly conserved at specific positions indicate amino acids that are essential in the structure, stability or activity of a protein. Identified by their high degree of conservation in aligned sequences of a family of protein homologues, they can be used as identifiers to determine if any polypeptide in question belongs to a previously identified polypeptide family (in this case, the proteins useful in the methods of the invention and nucleic acids encoding the same as defined herein).

The term "motif' or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases also exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004), or Pfam (Bateman et al., Nucleic Acids Research 30(1): 276-280 (2002). A set of tools for *in silico* analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)).

Domains may also be identified using routine techniques, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains (such as the Trehalose-PPase domain, or one of the motifs defined above) may be used as well. The sequence identity values, which are indicated in Example 3 as a percentage, were determined over the entire nucleic acid or amino acid sequence and over selected domains or conserved motif(s), using the programs mentioned above using the default parameters.

Furthermore, Class III TPP proteins (at least in their native form) typically have trehalose phosphatase activity. Polypeptides with trehalose phosphatase activity belong to the enzymatic class of EC:3.1.3.12, according to the classification of the Enzyme Commission of the Nomenclature Committee of the International Union of Biochemistry and Molecular Biology (NC-IUBMB). Enzymes in class EC:3.1.3.12 catalyze the reaction: trehalose-6-phosphate + $H_2O$ = trehalose + phosphate.

The activity of a trehalose phosphatase protein may be measured by determining the levels of the substrate processed and the levels of product accumulated in an *in vitro* reaction, that is, by determining the level of trehalose-6-phosphate and trehalose accumulation from the reaction. Enzymatic methods to measure trehalose can be based on hydrolyzing trehalose to glucose, such as those described by Van Dijck et al. Biochem J. 2002 Aug 15;366(Pt 1):63-71 and Zentella et al. Plant Physiol. 1999 Apr;119(4):1473-82.

Trehalose-6-phosphate levels may also be measured by HPLC (High Performance Liquid Chromatography) methods as described by Avonce et al. Plant Physiol. 2004 Nov;136(3):3649-59; Schluepmann et al. 2003. Alternative methods based on determining the release of inorganic phosphate from trehalose-6-phosphate have also been described Klutts et al. J Biol Chem. 2003 Jan 24;278(4):2093-100. An alternative method to determine trehalose-6-phosphate levels using liquid chromatography coupled to MS-Q3 (triple quadrupole MS) has been described by Lunn et al. Biochem J. 2006 Jul 1;397(1):139-48. Further details are provided in Example 5 and Example 6.

Examples of nucleic acids suitable for use in performing the methods of the invention include the nucleic acid sequences given in Table A of Example 1, but are not limited to those sequences. Nucleic acid variants may also be useful in practising the methods of the invention. Examples of such nucleic acid variants include portions of nucleic acids encoding a protein useful in the methods of the invention, nucleic acids hybridising to nucleic acids encoding a protein useful in the methods of the invention, splice variants of nucleic acids encoding a protein useful in the methods of the invention, allelic variants of nucleic acids encoding a protein useful in the methods of the invention and variants of nucleic acids encoding a protein useful in the methods of the invention that are obtained by gene shuffling. The terms portion, hybridising sequence, splice variant, allelic variant and gene shuffling will now be described.

Nucleic acids encoding proteins useful in the methods of the invention need not be full-length nucleic acids, since performance of the methods of the invention does not rely on the use of full-length nucleic acid sequences. Portions useful in the methods of the invention, encode a polypeptide falling within the definition of a nucleic acid encoding a protein useful in the methods of the invention as defined herein and having substantially the same biological activity as the amino acid sequences given in Table A of Example 1. Preferably, the portion is a portion of any one of the nucleic acids given in Table A of Example 1. The portion is typically at least 625 consecutive nucleotides in length, preferably at least 825 consecutive nucleotides in length, more preferably at least 1025 consecutive nucleotides in length and most preferably at least 1125 consecutive nucleotides in length, the consecutive nucleotides being of any one of the nucleic acid sequences given in Table A of Example 1. Most preferably the portion is a portion of the nucleic acid of SEQ ID NO: 1. Preferably, the portion encodes an amino acid sequence which when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

A portion of a nucleic acid encoding a Class III TPP protein as defined herein may be prepared, for example, by making one or more deletions to the nucleic acid. The portions may be used in isolated form or they may be fused to other coding (or non coding) sequences in order to, for example, produce a protein that combines several activities. When fused to other coding sequences, the resultant polypeptide produced upon translation may be bigger than that predicted for the Class III TPP protein portion.

According to the present invention, there is provided a method for enhancing yield-related traits in plants, particularly increasing seed yield, comprising introducing and expressing in a plant a portion of any one of the nucleic acid sequences given in Table A of Example 1, or a portion of a nucleic acid encoding an orthologue, paralogue or homologue of any of the amino acid sequences given in Table A of Example 1.

Another nucleic acid variant useful in the methods of the invention is a nucleic acid capable of hybridising under reduced stringency conditions, preferably under stringent conditions, with a nucleic acid encoding a Class III TPP protein as defined herein, or with a portion as defined herein.

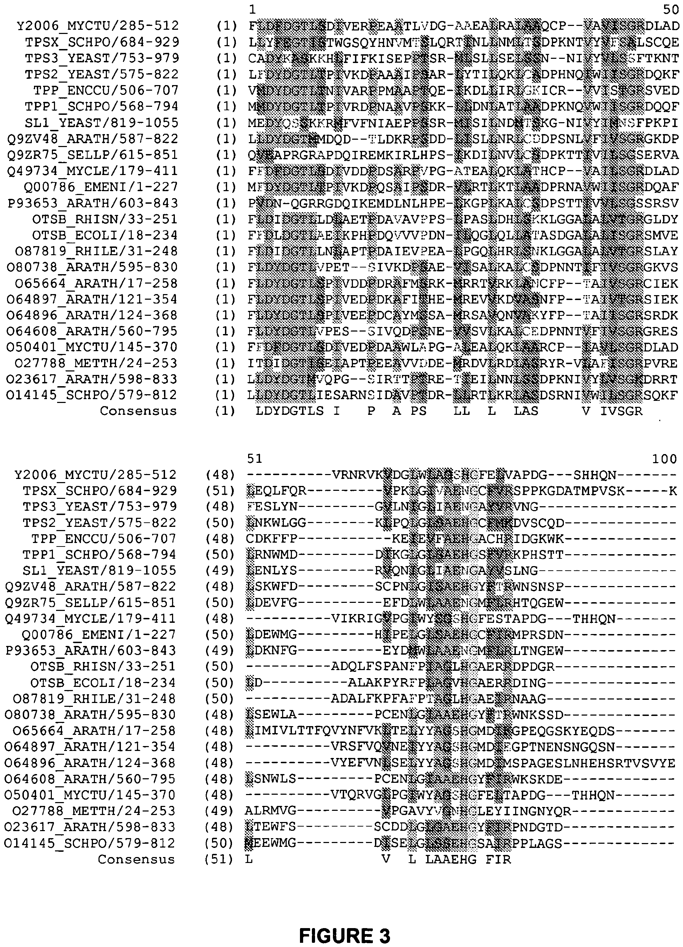

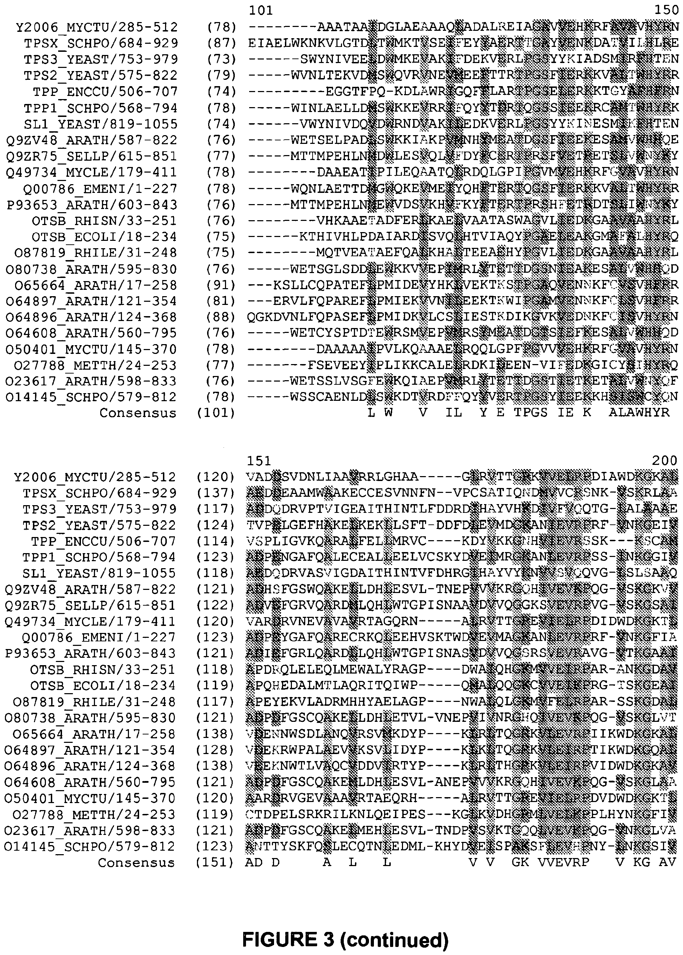

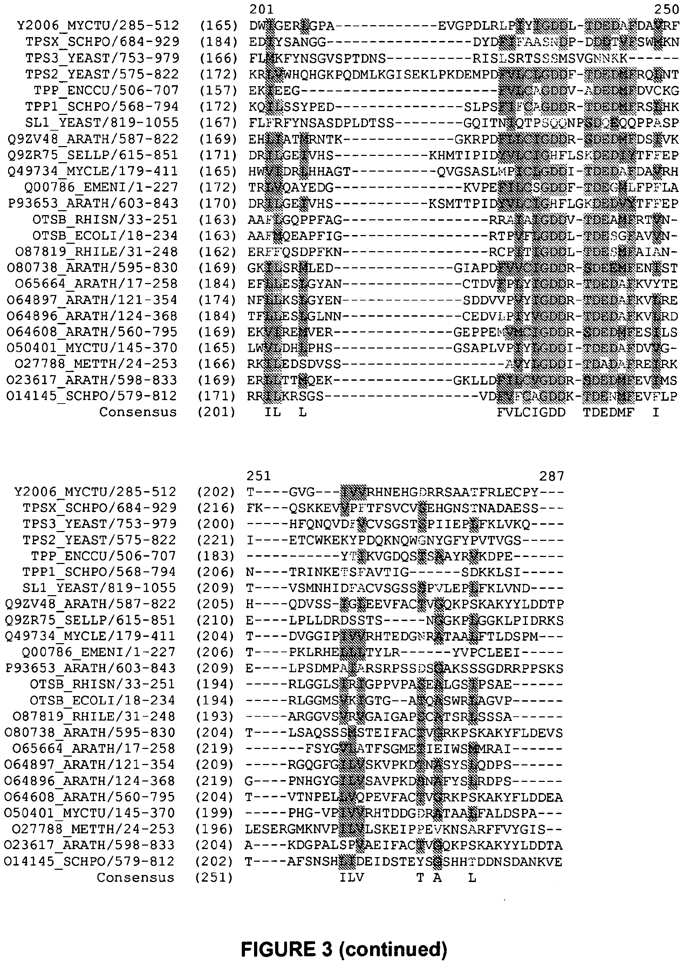

Hybridising sequences useful in the methods of the invention, encode a polypeptide having a Trehalose-PPAse domain (see the alignment of Fig. 2A and Fig. 3) and having substantially the same biological activity as Class III TPP proteins represented by any of the amino acid sequences given in Table A of Example 1. The hybridising sequence is typically at least 625 consecutive nucleotides in length, preferably at least 825 consecutive nucleotides in length, more preferably at least 1025 consecutive nucleotides in length and most preferably at least 1125 consecutive nucleotides in length, the consecutive nucleotides being of any one of the nucleic acid sequences given in Table A of Example 1. Preferably, the hybridising sequence is one that is capable of hybridising to any of the nucleic acids given in Table A of Example 1, or to a portion of any of these sequences, a portion being as defined above. Most preferably, the hybridising sequence is capable of hybridising to a nucleic acid as represented by SEQ ID NO: 1 or to a portion thereof.

Preferably, the hybridising sequence encodes an amino acid sequence which when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

Most preferably, the isolated polynucleotide molecule is capable of hybridising under stringent conditions to a sequence represented by one of SEQ ID NOs 4, 6 and 8.

According to the present invention, there is provided a method for enhancing yield-related traits in plants, particularly increasing seed yield, comprising introducing and expressing in a plant a nucleic acid capable of hybridizing to any one of the nucleic acids given in Table A of Example 1, or comprising introducing and expressing in a plant a nucleic acid capable of hybridising to a nucleic acid encoding an orthologue, paralogue or homologue of any of the nucleic acid sequences given in Table A of Example 1.

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the $T_m$ decreases about 1°C per % base mismatch. The $T_m$ may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x log_{10}[Na+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (log_{10}[Na+]^a) + 0.58 (\%G/C^b) + 11.8 (\%G/C^b)^2 - 820/L^c$$

3) oligo-DNA or oligo-RNA[d] hybrids:

For <20 nucleotides: Tm= 2 ($I_n$)
For 20-35 nucleotides: Tm= 22 + 1.46 ($I_n$)

[a] or for other monovalent cation, but only accurate in the 0.01-0.4 M range.
[b] only accurate for %GC in the 30% to 75% range.
[c] L = length of duplex in base pairs.
[d] Oligo, oligonucleotide; $I_n$, effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5 × Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989 and yearly updates).

Another nucleic acid variant useful in the methods of the invention is a splice variant encoding a Class III TPP protein as defined hereinabove. The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex, BMC Bioinformatics. 2005; 6: 25).

According to the present invention, there is provided a method for enhancing yield-related traits in plants, particularly increasing seed yield, comprising introducing and expressing in a plant a splice variant of any one of the nucleic acid sequences given in Table A of Example 1, or a splice variant of a nucleic acid encoding an orthologue, paralogue or homologue of any of the amino acid sequences given in Table A of Example 1.

Preferred splice variants are splice variants of a nucleic acid represented by SEQ ID NO: 1 or a splice variant of a nucleic acid encoding an orthologue or paralogue of SEQ ID NO: 2. Preferably, the amino acid sequence encoded by the splice variant comprises any one or more of the motifs or domains as defined herein. Preferably, the amino acid sequence encoded by the splice variant, when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

Another nucleic acid variant useful in performing the methods of the invention is an allelic variant of a nucleic acid encoding a Class III TPP protein as defined hereinabove. Alleles or allelic variants are alternative forms of a given gene, located at the same chromosomal position. Allelic variants exist in nature, and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms. The allelic variants useful in the methods of the present invention have substantially the same biological activity as the Class III TPP protein of SEQ ID NO: 2.

According to the present invention, there is provided a method for enhancing yield-related traits in plants, particularly increasing seed yield, comprising introducing and expressing in a plant an allelic variant of any one of the nucleic acids given in Table A of Example 1, or comprising introducing and expressing in a plant an allelic variant of a nucleic acid encoding an orthologue, paralogue or homologue of any of the amino acid sequences given in Table A of Example 1.

Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant comprises any one or more of the motifs or domains as defined herein. Preferably, the amino acid sequence encoded by the allelic variant, when used in the construction of a TPS/TPP phylogenetic tree, such as the one depicted in Fig. 2A, tends to cluster with the group of Class III TPP proteins comprising the amino acid sequence represented by SEQ ID NO: 2, rather than with any other group.

A further nucleic acid variant useful in the methods of the invention is a nucleic acid variant obtained by gene shuffling. Gene shuffling or directed evolution may also be used to generate variants of nucleic acids encoding Class III TPP proteins as defined above. This consists of iterations of DNA shuffling followed by appropriate screening and/or selection to generate variants of nucleic acids or portions thereof encoding Class III TPP proteins as defined above having a modified biological activity (Castle et al., (2004) Science 304(5674): 1151-4; US patents 5,811,238 and 6,395,547).

According to the present invention, there is provided a method for enhancing yield-related traits in plants, comprising introducing and expressing in a plant a variant of any one of the nucleic acid sequences given in Table A of Example 1, or comprising introducing and expressing in a plant a variant of a nucleic acid encoding an orthologue, paralogue or homologue of any of the amino acid sequences given in Table A of Example 1, which variant nucleic acid is obtained by gene shuffling.

Preferably, the variant nucleic acid obtained by gene shuffling encodes an amino acid sequence comprising any one or more of the motifs or domains as defined herein. Preferably, the amino acid encoded sequence by the variant nucleic acid obtained by gene shuffling, when used in the construction of a TPS/TPP phylogenetic tree, such as the one depicted in Fig. 2A, tends to cluster with the group of Class III TPP proteins comprising the amino acid sequence represented by SEQ ID NO: 2 rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding Class III TPP proteins may be derived from any natural or artificial source. The nucleic acid may be modified from its native form in composition and/or genomic environment through deliberate human manipulation. Preferably the Class III TPP-encoding nucleic acid is from a plant, further preferably from a dicotyledonous plant, more preferably from the Brassicaceae family, most preferably the nucleic acid is from *Arabidopsis thaliana.*

Any reference herein to a Class III TPP protein is therefore taken to mean a Class III TPP protein as defined above. Any nucleic acid encoding such a Class III TPP protein is suitable for use in performing the methods of the invention.

The present invention also encompasses plants or parts thereof (including seeds) obtainable by the methods according to the present invention. The plants or parts thereof comprise a nucleic acid transgene encoding a Class III TPP protein as defined above.

The invention also provides hitherto unknown Class III TPP nucleic acid sequences and Class III TPP protein sequences. These sequences also being useful in performing the methods of the invention.

According to a further embodiment of the present invention, there is therefore provided an isolated nucleic acid molecule comprising:

(i) a nucleic acid represented by SEQ ID NO: 3, SEQ ID NO: 5, SEQ ID NO: 7, SEQ ID NO: 121, SEQ ID NO: 123, SEQ ID NO: 125, SEQ ID NO: 127 or SEQ ID NO: 129;

(ii) the complement of any one of the SEQ ID NOs given in (i);

(iii) a nucleic acid encoding a Class III TPP protein having, in increasing order of preference, at least 80%, 85%, 90%, 95%, 96%, 97%, 98%, 99% or 100% sequence identity to any one of the amino acid sequences given in SEQ ID NO: 4, SEQ ID NO: 6, SEQ ID NO: 8, SEQ ID NO: 122, SEQ ID NO: 124, SEQ ID NO: 126, SEQ ID NO: 128 or SEQ ID NO: 130.

(iv) a nucleic acid capable of hybridizing under stringent conditions to any one of the nucleic acids given in (i), (ii) or (iii) above.

According to a further embodiment of the present invention, there is also provided an isolated polypeptide comprising:

(i) an amino acid sequence represented by any one of SEQ ID NO: 4, SEQ ID NO: 6, SEQ ID NO: 8, SEQ ID NO: 122, SEQ ID NO: 124, SEQ ID NO: 126, SEQ ID NO: 128 or SEQ ID NO: 130;

(ii) an amino acid sequence having, in increasing order of preference, at least at least 80%, 85%, 90%, 95%, 96%, 97%, 98%, 99% or 100% sequence identity to any one of the amino acid sequences given in SEQ ID NO: 4, SEQ ID NO: 6, SEQ ID NO: 8, SEQ ID NO: 122, SEQ ID NO: 124, SEQ ID NO: 126, SEQ ID NO: 128 or SEQ ID NO: 130;

(iii) derivatives of any of the amino acid sequences given in (i) or (ii) above.

The invention also provides genetic constructs and vectors to facilitate introduction and/or expression of the nucleic acid sequences useful in the methods according to the invention, in a plant. The gene constructs may be inserted into vectors, which may be commercially available, suitable for transforming into plants and suitable for expression of the gene of interest in transformed cells. The invention also provides use of a gene construct as defined herein in the methods of the invention.

More specifically, the present invention provides a construct comprising:

(a) nucleic acid encoding Class III TPP protein as defined above;

(b) one or more control sequences capable of driving expression of the nucleic acid sequence of (a); and optionally

(c) a transcription termination sequence.

Preferably the nucleic acid in the construct according to the invention is a polynucleotide molecule encoding a Class III TPP protein with an amino acid sequence in increasing order of preference of at least 80%, 85%, 90%, 95%, 96%, 97%, 98%, 99% or 100% sequence identity to a sequence represented by one of SEQ ID NO: 4, SEQ ID NO: 6, SEQ ID NO: 8, SEQ ID NO: 122, SEQ ID NO: 124, SEQ ID NO: 126, SEQ ID NO: 128 or SEQ ID NO: 130. Most preferably, the Class III TPP polynucleotide molecule is any of the nucleotide sequences of SEQ ID NO: 3, SEQ ID NO: 5, SEQ ID NO: 7, SEQ ID NO: 121, SEQ ID NO: 123, SEQ ID NO: 125, SEQ ID NO: 127 or SEQ ID NO: 129

Plants are transformed with a vector comprising the sequence of interest (i.e., a nucleic acid encoding a Class III TPP polypeptide as defined herein. The skilled artisan is well aware of the genetic elements that must be present on the vector in order to successfully transform, select and propagate host cells containing the sequence of interest. The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence. The term "promoter" refers to a nucleic acid control sequence located upstream from the transcriptional start of a gene and which is involved in recognising and binding of RNA polymerase and other proteins, thereby directing transcription of an operably linked nucleic acid. A "plant" promoter comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule is operably to a suitable promoter.

The promoter may be a constitutive promoter, which refers to a promoter that is transcriptionally active during most, but not necessarily all, phases of its growth and development and under most environmental conditions, in at least one cell, tissue or organ. Alternatively, the promoter may be an inducible promoter, i.e. having induced or increased transcription initiation in response to a chemical (for a review see Gatz 1997, Annu. Rev. Plant Physiol. Plant Mol. Biol., 48:89-108), environmental or physical stimulus. Another example of an inducible promoter is a stress-inducible promoter, i.e. a promoter activated when a plant is exposed to various stress conditions, or a pathogen-induced promoter.

Additionally or alternatively, the promoter may be an organ-specific or tissue-specific promoter, i.e. one that is capable of preferentially initiating transcription in certain organs or tissues, such as the leaves, roots, seed tissue etc.; or the promoter may be a ubiquitous promoter, which is active in substantially all tissues or cells of an organism, or the promoter may be developmentally regulated, thereby being active during certain developmental stages or in parts of the plant that undergo developmental changes. Promoters able to initiate transcription in certain organs or tissues only are referred to herein as "organ-specific" or "tissue-specific" respectively, similarly, promoters able to initiate transcription in certain cells only are referred to herein as "cell-specific".

Preferably, the Class III TPP nucleic acid or variant thereof is operably linked to a constitutive promoter. A preferred constitutive promoter is one that is also substantially ubiquitously expressed. Further preferably the promoter is derived from a plant, more preferably from a monocotyledonous plant. Most preferred is the use of a GOS2 promoter, preferably a GOS2 promoter from rice, most preferably a promoter substantially as represented by SEQ ID NO: 98. It should be clear that the applicability of the present invention is not restricted to the Class III TPP nucleic acid represented by SEQ ID NO: 1, nor is the applicability of the invention restricted to expression of a Class III TPP nucleic acid when driven by a GOS2 promoter. According to another preferred feature of the invention, the constitutive promoter is a High Mobility Group Protein (HMGP) promoter, preferably a HMGP promoter from rice, more preferably substantially similar to SEQ ID NO: 131, most preferably identical to SEQ ID NO: 131. Examples of other constitutive promoters which may also be used to drive expression of a Class III TPP nucleic acid are shown in Table 2 below.

**Table 2:** Examples of constitutive promoters

| Gene Source | Reference |
|---|---|
| Actin | McElroy et al, Plant Cell, 2: 163-171, 1990 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| ocs | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |

(continued)

| Gene Source | Reference |
|---|---|
| V-ATPase | WO 01/14572 |
| Super promoter | WO 95/14098 |
| G-box proteins | WO 94/12015 |

For the identification of functionally equivalent promoters, the promoter strength and/or expression pattern of a candidate promoter may be analysed for example by operably linking the promoter to a reporter gene and assay the expression level and pattern of the reporter gene in various tissues of the plant. Suitable well-known reporter genes include for example beta-glucuronidase or beta galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally, the term "weak promoter" refers to a promoter that drives expression of a coding sequence at a low level, levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1,000 transcripts per cell.

Optionally, one or more terminator sequences may be used in the construct introduced into a plant. The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

An intron sequence may also be added to the 5' untranslated region (UTR) or in the coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg, Mol. Cell Biol. 8:4395-4405 (1988); Callis et al., Genes Dev. 1: 1183-1200 (1987)). Such intron enhancement of gene expression is typically greatest when placed near the 5' end of the transcription unit. Use of the maize introns Adh1-S intron 1, 2, and 6, the Bronze-1 intron are known in the art. For general information, see The Maize Handbook, Chapter 116, Freeling and Walbot, Eds., Springer, N.Y. (1994).

Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. As used herein, the term "selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as *nptII* that phosphorylates neomycin and kanamycin, or *hpt,* phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example *bar* which provides resistance to Basta®; *aroA* or *gox* providing resistance against glyphosate, or the genes conferring resistance to, for example, imidazolinone, phosphinothricin or sulfonylurea), or genes that provide a metabolic trait (such as *manA* that allows plants to use mannose as sole carbon source or xlose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with *Agrobacteria,* the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems, which have the advantage that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

The invention also provides a method for the production of transgenic plants having enhanced yield-related traits, particularly increased seed yield, relative to control plants, comprising introduction and expression in a plant of any nucleic acid encoding a Class III TPP protein as defined hereinabove.

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

More specifically, the present invention provides a method for the production of transgenic plants having increased yield, which method comprises:

(i) introducing and expressing in a plant, plant part or plant cell a *Class III* TPP nucleic acid or variant thereof; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known *inter alia* from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the centre of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the"floral dip" method the developing floral tissue is incubated briefly with a surfactant-treated agrobacterial suspension [Clough, SJ und Bent, AF (1998). The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid encoding a Class III TPP protein as defined hereinabove. Preferred host cells according to the invention are plant cells.

Host plants for the nucleic acids or the vector used in the method according to the invention, the expression cassette or construct or vector are, in principle, advantageously all plants, which are capable of synthesizing the polypeptides used in the inventive method.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression. For example, endogenous promoters may be altered *in vivo* by mutation, deletion, and/or substitution (see, Kmiec, U.S. Pat. No. 5,565,350; Zarling et al., PCT/US93/03868), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added as described above.

Other control sequences (besides promoter, enhancer, silencer, intron sequences, 3'UTR and/or 5'UTR regions, micro-RNA target sites, may be protein and/or RNA stabilizing elements.

As mentioned above, a preferred method for modulating (preferably, increasing) expression of a nucleic acid encoding a Class III TPP protein is by introducing and expressing in a plant a nucleic acid encoding a Class III TPP protein; however the effects of performing the method, i.e. enhancing yield-related traits may also be achieved using other well known techniques. A description of some of these techniques will now follow.

One such technique is T-DNA activation tagging (Hayashi et al. Science (1992) 1350-1353), which involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

The effects of the invention may also be reproduced using the technique of TILLING (Targeted Induced Local Lesions In Genomes). This is a mutagenesis technology useful to generate and/or identify a nucleic acid encoding a Class III TPP protein with modified expression and/or activity. TILLING also allows selection of plants carrying such mutant variants. These mutant variants may exhibit modified expression, either in strength or in location or in timing (if the mutations affect the promoter for example). These mutant variants may exhibit higher Class III TPP protein activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

The effects of the invention may also be reproduced using homologous recombination, which allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Reference herein to enhanced yield-related traits is taken to mean an increase in biomass (weight) of one or more parts of a plant, which may include aboveground (harvestable) parts and/or (harvestable) parts below ground.

In particular, such harvestable parts are seeds, and performance of the methods of the invention results in plants having increased seed yield relative to the seed yield of suitable control plants.

The term "yield" in general means a measurable produce of economic value, necessarily related to a specified crop, to an area, and to a period of time. Individual plant parts directly contribute to yield based on their number, size and/or weight, whereas the actual yield is the yield per acre for a crop and year, which is determined by dividing total production (includes both harvested and appraised production) by planted acres.

The terms "increase", "improving" or "improve" are interchangeable and shall mean in the sense of the application at least a 5%, 6%, 7%, 8%, 9% or 10%, preferably at least 15% or 20%, more preferably 25%, 30%, 35% or 40% more yield and/or growth in comparison to the wild type plant as defined herein.

Increased seed yield may manifest itself as one or more of the following: a) an increase in seed biomass (total seed weight) which may be on an individual seed basis and/or per plant and/or per hectare or acre; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Since the transgenic plants according to the present invention have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a Class III TPP protein as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects. Another abiotic stress may result from a nutrient deficiency, such as a shortage of nitrogen , phosphorus and potassium.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to suitable control plants grown under comparable conditions. Therefore, according to the present invention, there is provided a method for enhancing yield-related traits in plants grown under non-stress conditions or under mild drought conditions, which method comprises increasing expression in a plant of a nucleic acid encoding a Class III TPP polypeptide.

In a preferred embodiment of the invention, the enhanced yield-related trait is manifested as an increase in one or more of the following: total number of seeds per plant, number of filled seeds per plant and seed weight per plant. Preferably, these increases are found in plants grown under non-stress conditions.

The methods of the invention are advantageously applicable to any plant.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agropyron* spp., *Allium* spp., *Amaranthus* spp., *Ananas comosus, Annona* spp., *Apium graveolens, Arachis* spp, *Artocarpus* spp., *Asparagus officinalis, Avena* spp. (e.g. *Avena sativa, Avena fatua, Avena byzantina, Avena fatua* var. sativa, *Avena hybrida*), *Averrhoa carambola, Benincasa hispida, Bertholletia excelsea, Beta vulgaris, Brassica* spp. (e.g. *Brassica napus, Brassica rapa* ssp. [canola, oilseed rape, turnip rape]), *Cadaba farinosa, Camellia sinensis, Canna indica, Capsicum* spp., *Carex elata, Carica papaya, Carissa macrocarpa, Carya* spp., *Carthamus tinctorius, Castanea* spp., *Cichorium endivia, Cinnamomum* spp., *Citrullus lanatus, Citrus* spp., *Cocos* spp., *Coffea* spp., *Colocasia esculenta, Cola* spp., *Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., Elaeis (e.g. *Elaeis guineensis, Elaeis oleifera*), *Eleusine coracana, Eriobotrya japonica, Eugenia uniflora, Fagopyrum* spp., *Fagus* spp., *Ficus carica, Fortunella* spp., *Fragaria* spp., *Ginkgo biloba, Glycine* spp. (e.g. *Glycine max, Soja hispida* or *Soja max*), *Gossypium hirsutum, Helianthus* spp. (e.g. Helianthus annuus), *Hemerocallis fulva, Hibiscus* spp., *Hordeum* spp. (e.g. *Hordeum vulgare*), *Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. (e.g. *Lycopersicon esculentum, Lycopersicon lycopersicum, Lycopersicon pyriforme*), *Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus spp., Momordica* spp., *Morus nigra, Musa* spp., *Nicotiana* spp., *Olea* spp., *Opuntia* spp., *Omithopus* spp., *Oryza* spp. (e.g. *Oryza sativa, Oryza latifolia), Panicum miliaceum, Passiflora edulis, Pastinaca sativa, Persea* spp., *Petroselinum crispum, Phaseolus* spp., *Phoenix* spp., *Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Sambucus* spp., *Secale cereale, Sesamum* spp., *Sinapis* sp., *Solanum* spp. (e.g. *Solanum tuberosum, Solanum integrifolium* or *Solanum lycopersicum*), *Sorghum bicolor, Spinacia* spp., *Syzygium* spp., *Tagetes* spp., *Tamarindus indica, Theobroma cacao, Trifolium* spp., *Triticosecale rimpaui, Triticum* spp. (e.g. *Triticum aestivum, Triticum durum, Triticum turgidum, Triticum hybernum, Triticum macha, Triticum sativum* or *Triticum vulgare*), *Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, sorghum and oats.

The present invention also encompasses use of nucleic acids encoding the Class III TPP protein described herein and use of these Class III TPP proteins in enhancing yield-related traits in plants.

Nucleic acids encoding the Class III TPP protein described herein, or the Class III TPP proteins themselves, may find use in breeding programmes in which a DNA marker is identified which may be genetically linked to a Class III TPP encoding gene. The nucleic acids/genes, or the Class III TPP proteins themselves may be used to define a molecular marker. This DNA or protein marker may then be used in breeding programmes to select plants having enhanced yield-related traits as defined hereinabove in the methods of the invention.

Allelic variants of a Class III TPP protein-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding Class III TPP proteins may also be used as probes for genetically and physically mapping the genes that they are a part of, and as markers for traits linked to those genes. Such information may be useful in plant breeding in order to develop lines with desired phenotypes. Such use of Class III TPP protein-encoding nucleic acids requires only a nucleic acid sequence of at least 15 nucleotides in length. The Class III TPP protein-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the Class III TPP protein-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the Class III TPP protein-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

**Description of figures**

The present invention will now be described with reference to the following figures in which:

**Fig. 1** is a schematic representation of the structural elements present in Class III TPP polypeptides. The position of the characteristic elements: trehalose phosphate phosphatase domain, phosphatase BOX A and BOX B and serine-rich domain are indicated.

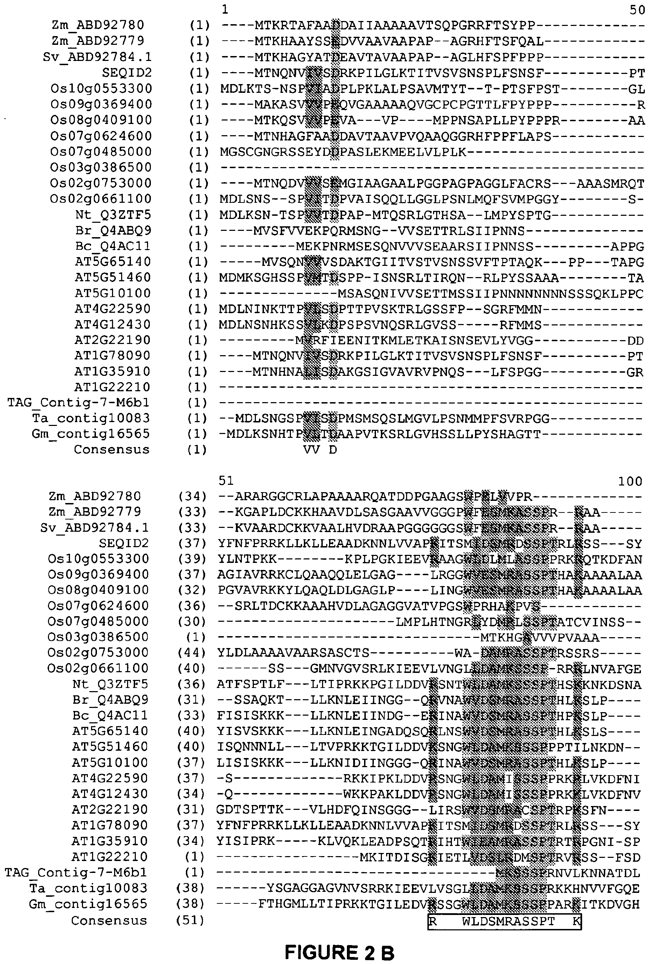

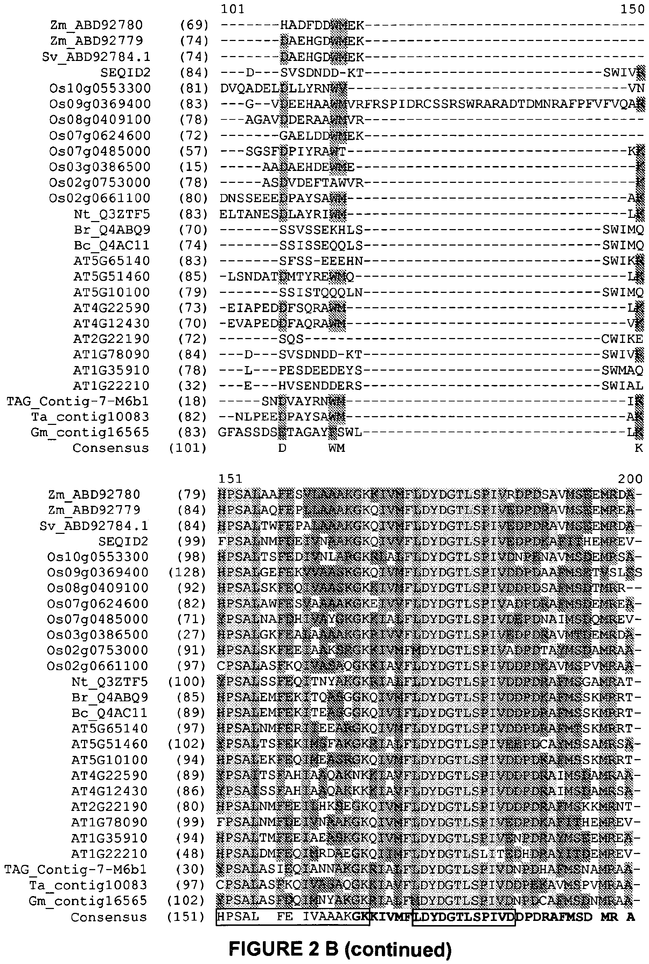

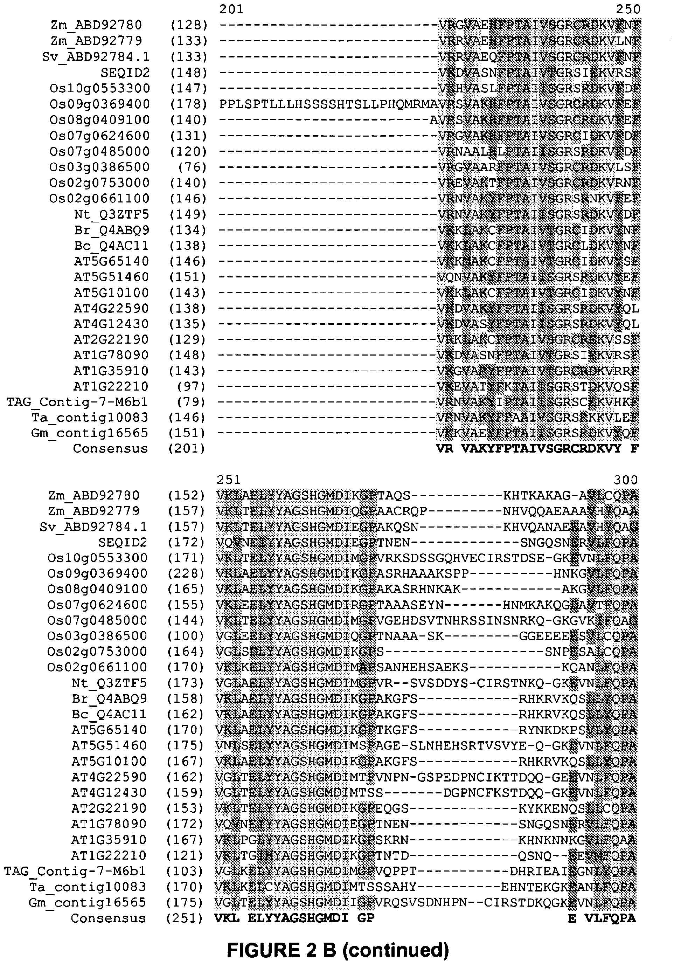

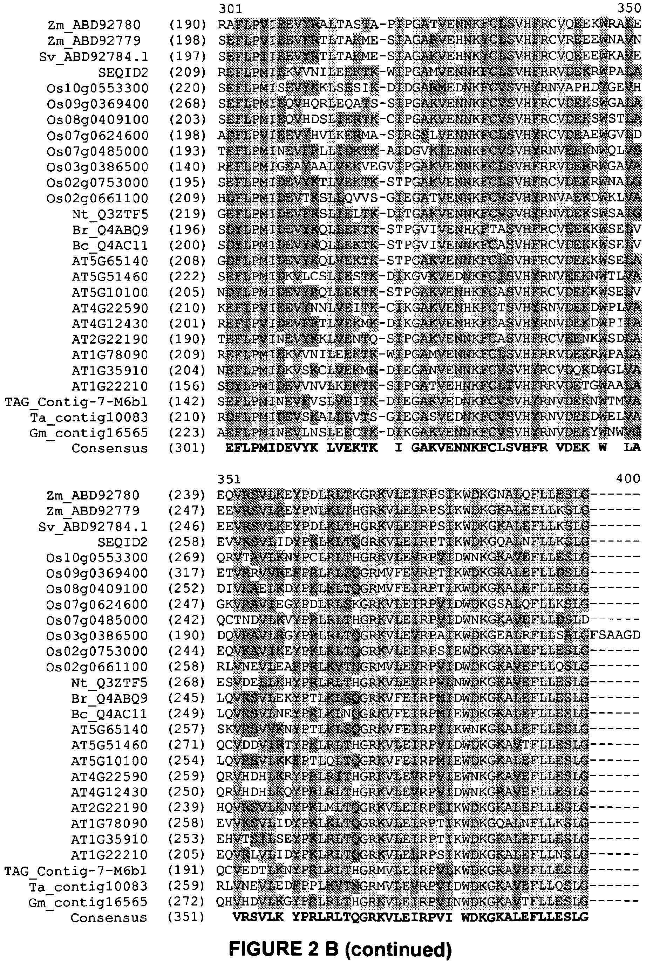

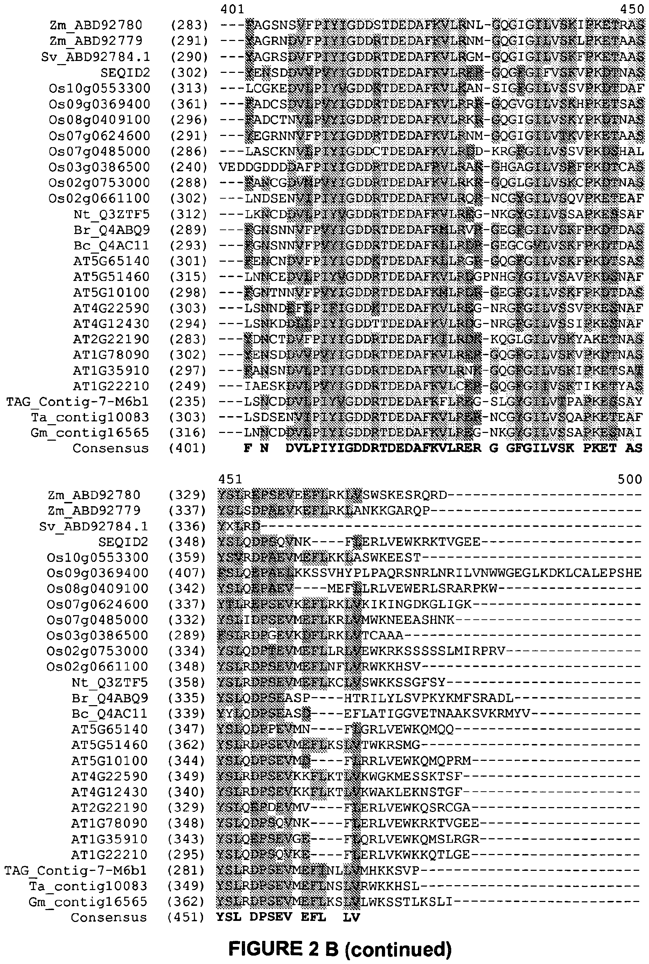

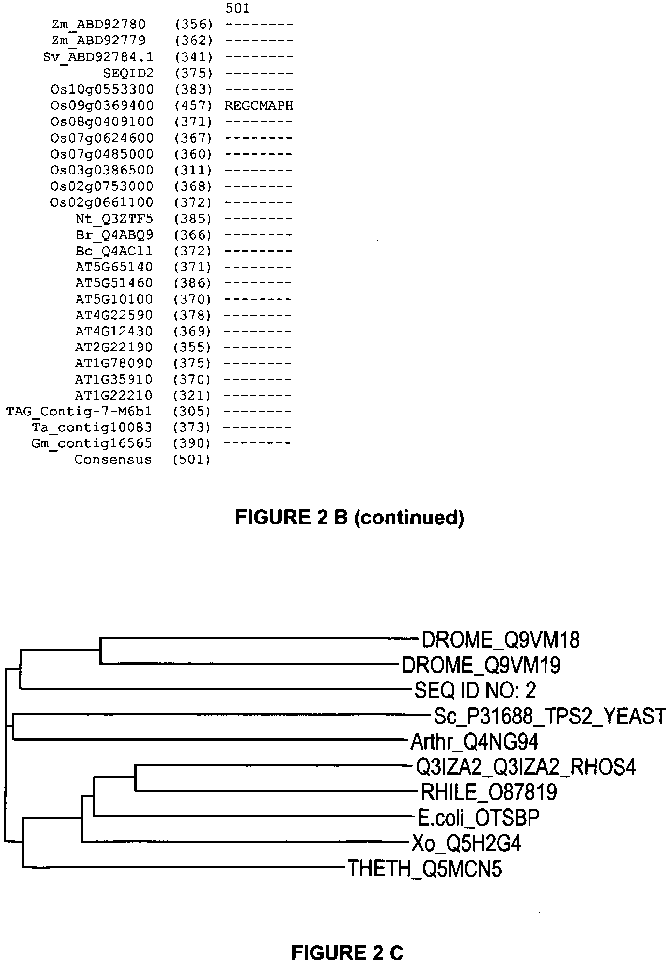

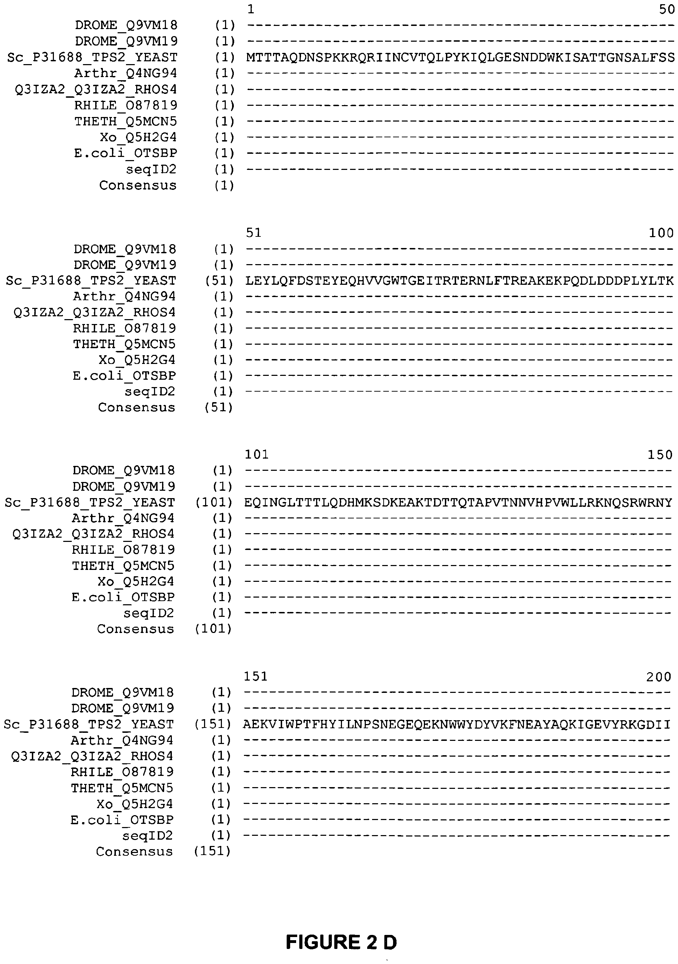

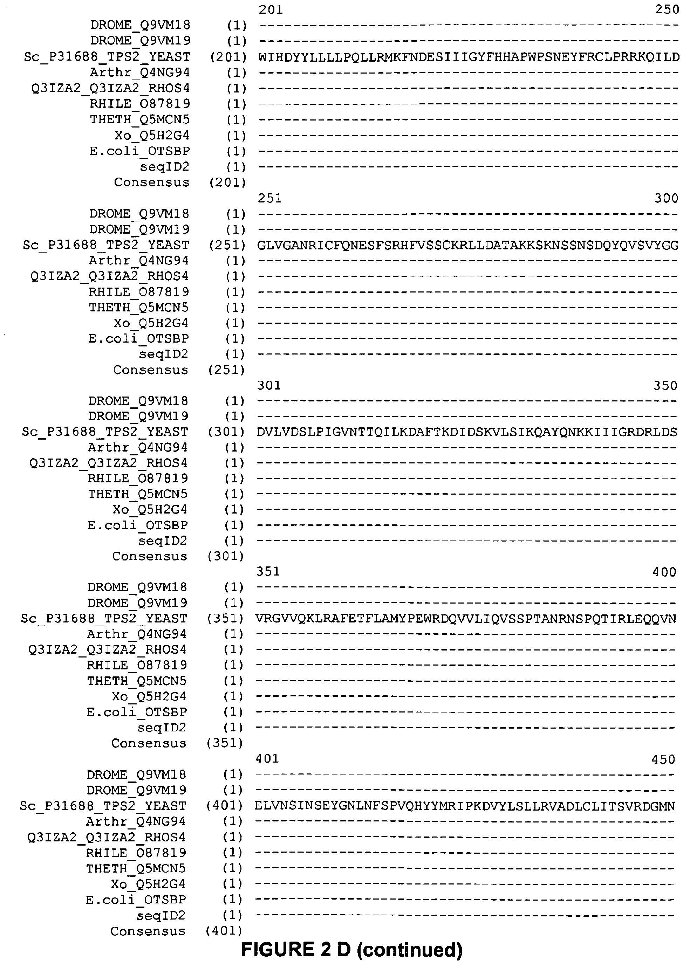

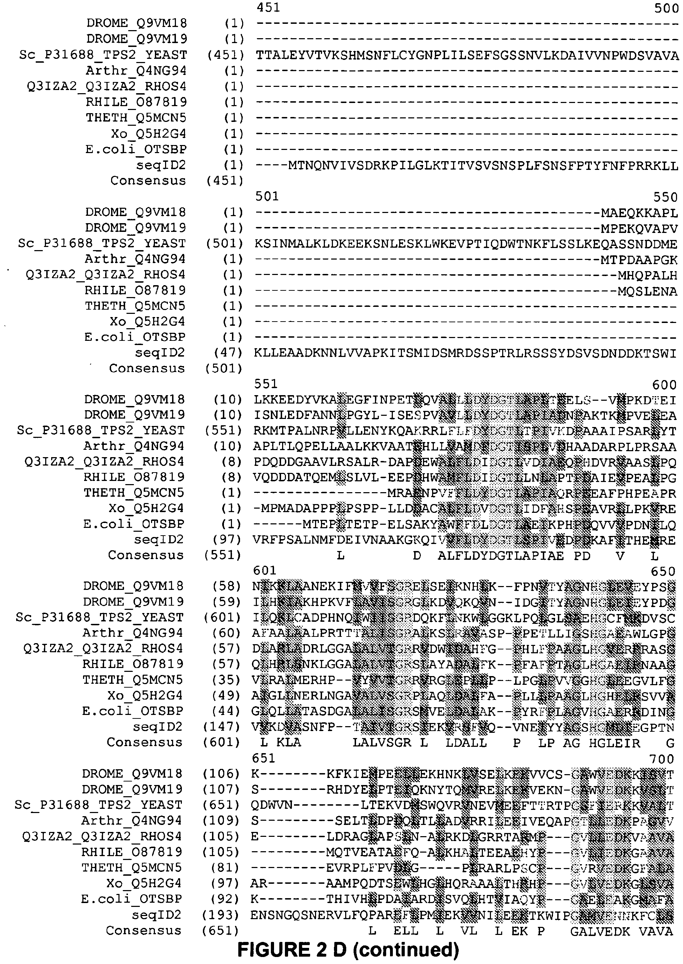

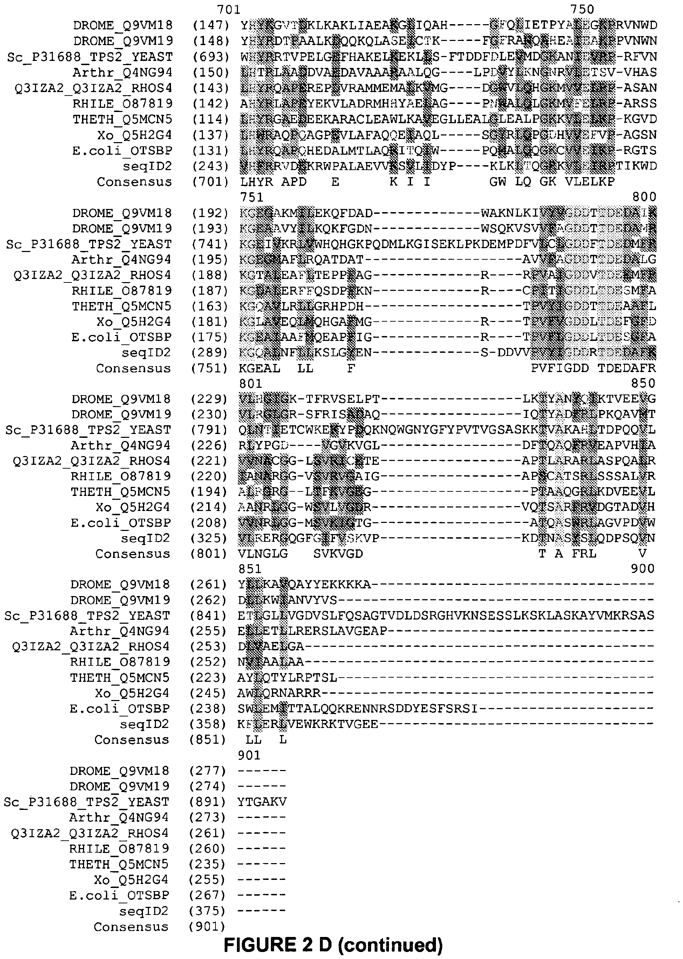

**Fig. 2** is a sequence alignment and phylogenetic tree of TPS/TPP polypeptides as described in Example 2. Figure 2A shows a phylogenetic tree of TPS/TPP polypeptides. Clades encircled in the oval contain proteins of Class I and Class II TPS proteins. Class III TPP proteins (within the rectangle) cluster apart from the Class I and Class II TPS group. Figure 2B shows an alignment of Class III TPP polypeptides of plant origin. Region of the Trehalose-PPase domain is highlighted in bold. Position of the serine-rich and Phosphatase A and B boxes are highlighted by a rectangle. Figure 2C shows a phylogenetic tree of Class III TPP polypeptides of non-plant origin. Figure 2D shows an alignment of Class III TPP polypeptides of non-plant origin.

**Fig. 3** is an alignment of Trehalose-PPase domains found in Class III TPP polypeptides.

**Fig. 4** shows TPP activity as measured by HPLC. The first graph of Fig. 4A corresponds to the control sample pGEX; the second graph of Fig. 4B corresponds to the TPPI sample.

**Fig. 5** is a bar chart showing quantification of TPP activity as detected by HPLC for the control sample, pGEX, and for the TPP sample.

**Fig. 6** shows the complementation of the growth defect on the yeast strain YSH448 (tps2Δ) by *Arabidopsis thaliana* Class III TPP polypeptides. Picture in Fig. 6A corresponds to a plate of a drop assay at 30 C, showing that all strains are viable at the non-restrictive temperature. Fig. 6B shows a plate at 37 C, showing that only strains transformed with a Class III TPP polynucleotide grow well at the restrictive temperature of 37 C. Fig. 6C shows a plate of a drop assay at 39 C.

**Fig. 7** Binary vector for increased expression in *Oryza sativa* of an *Arabidopsis thaliana* Class III TPP protein-encoding nucleic acid under the control of a GOS2 promoter.

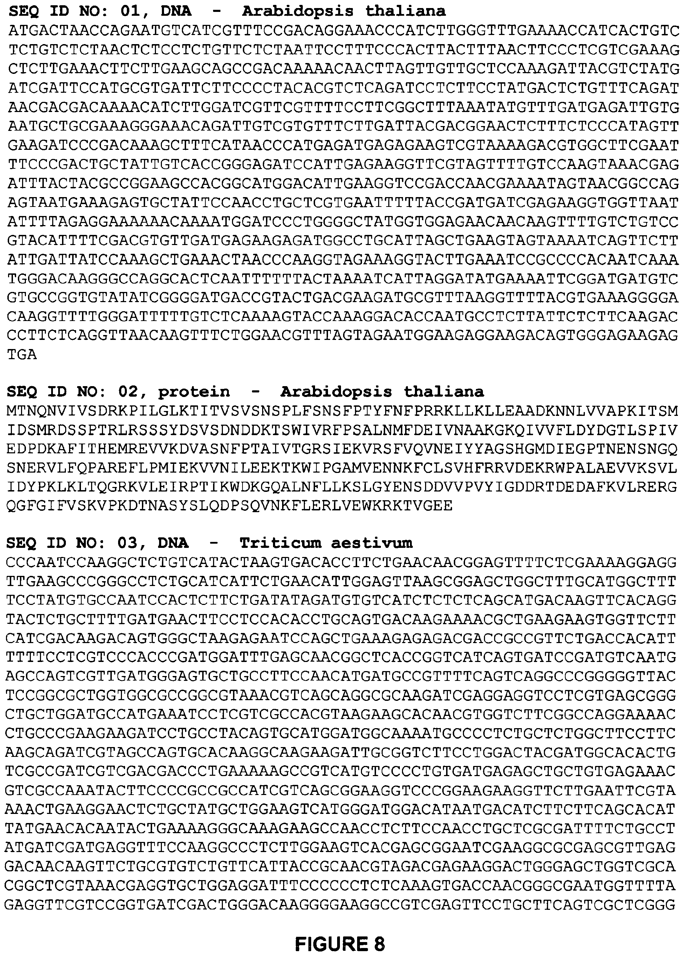

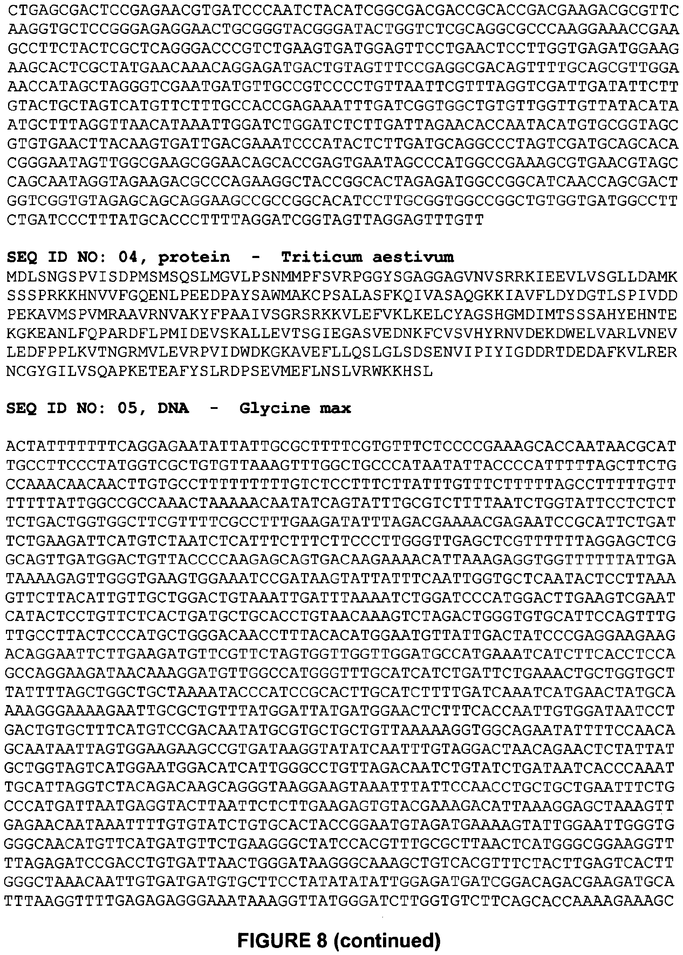

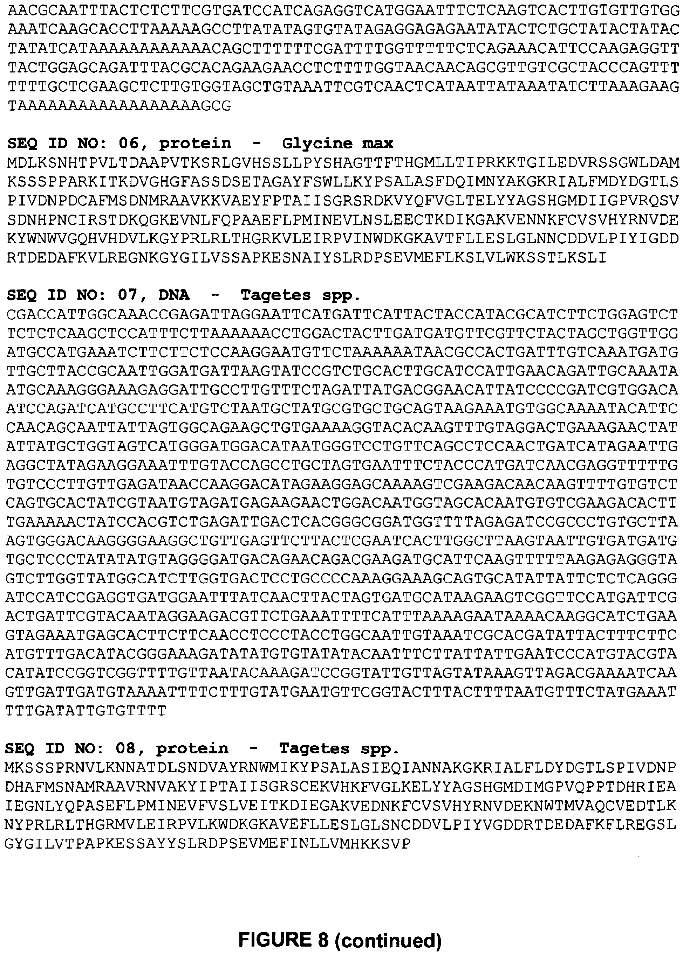

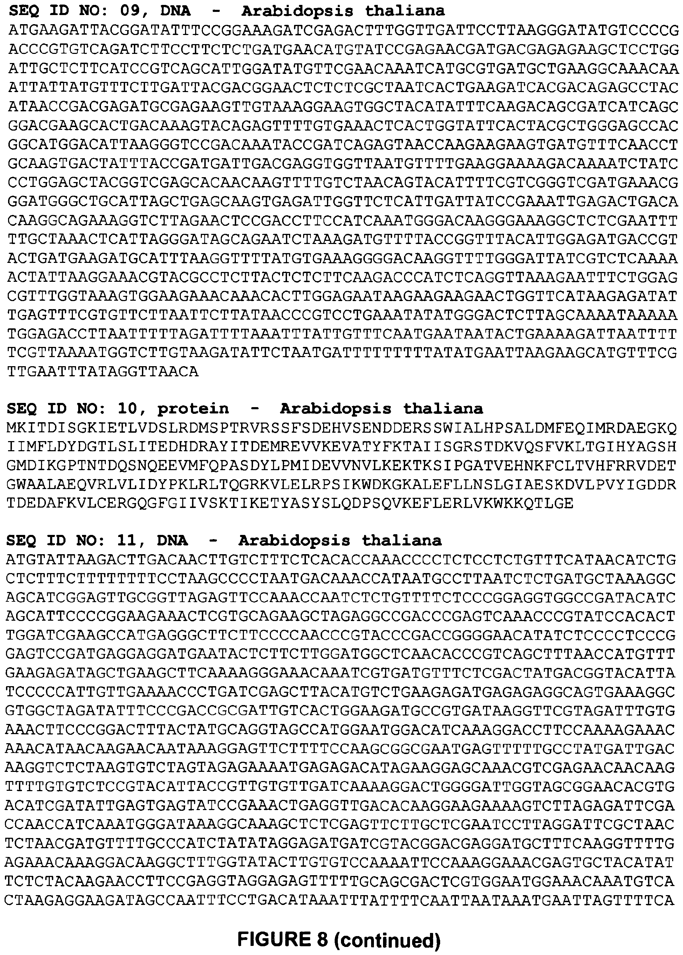

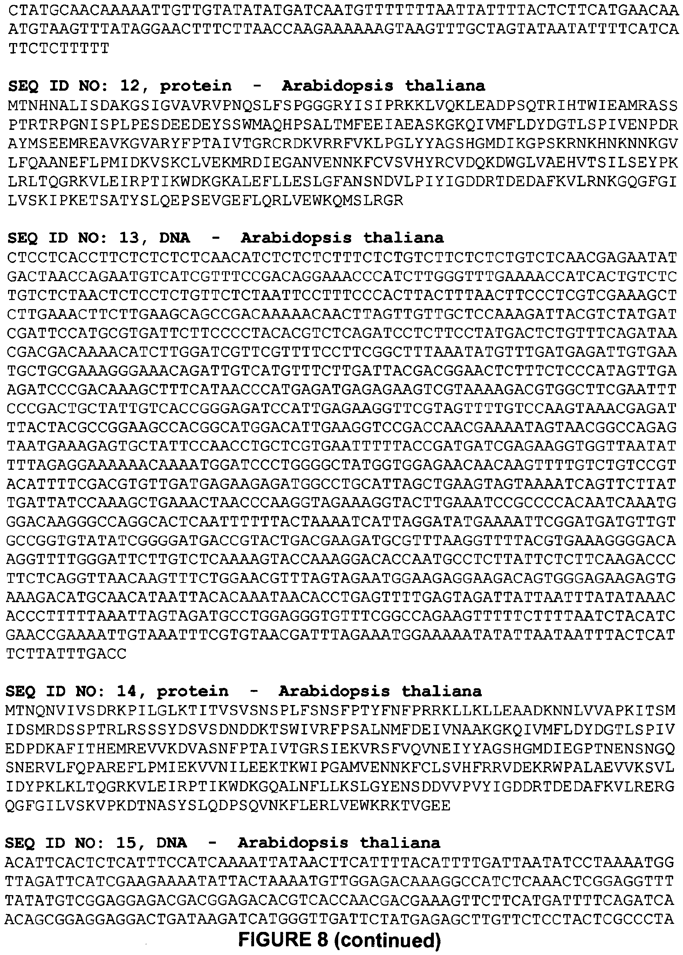

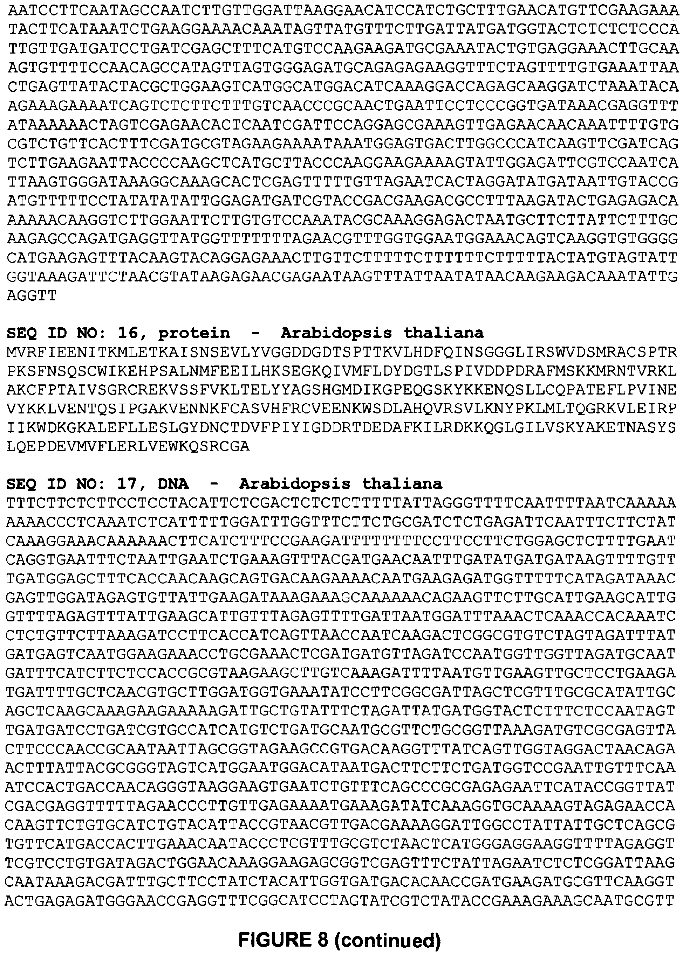

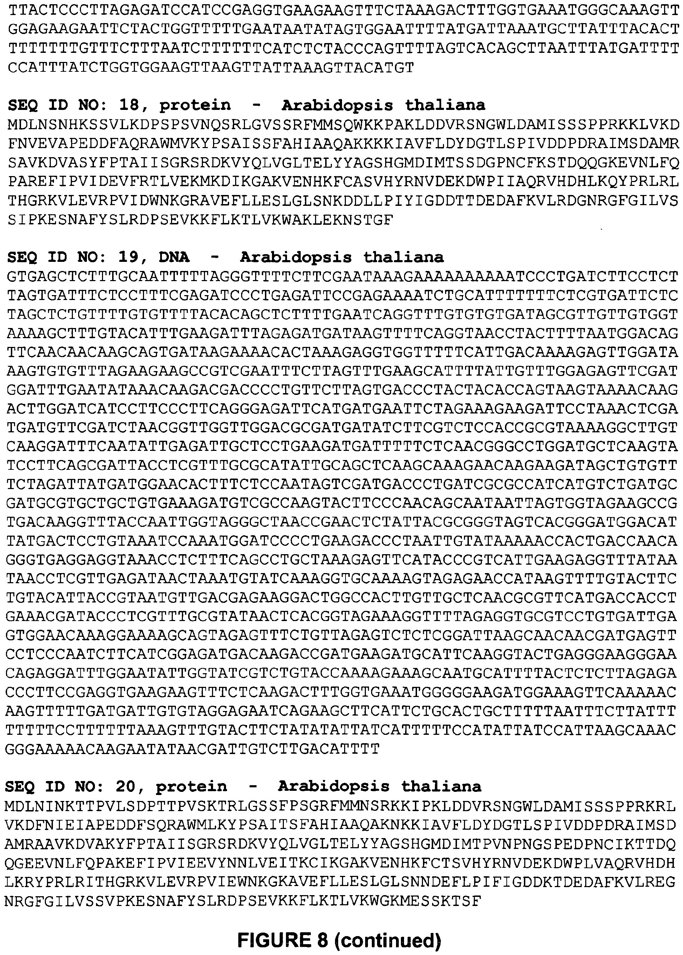

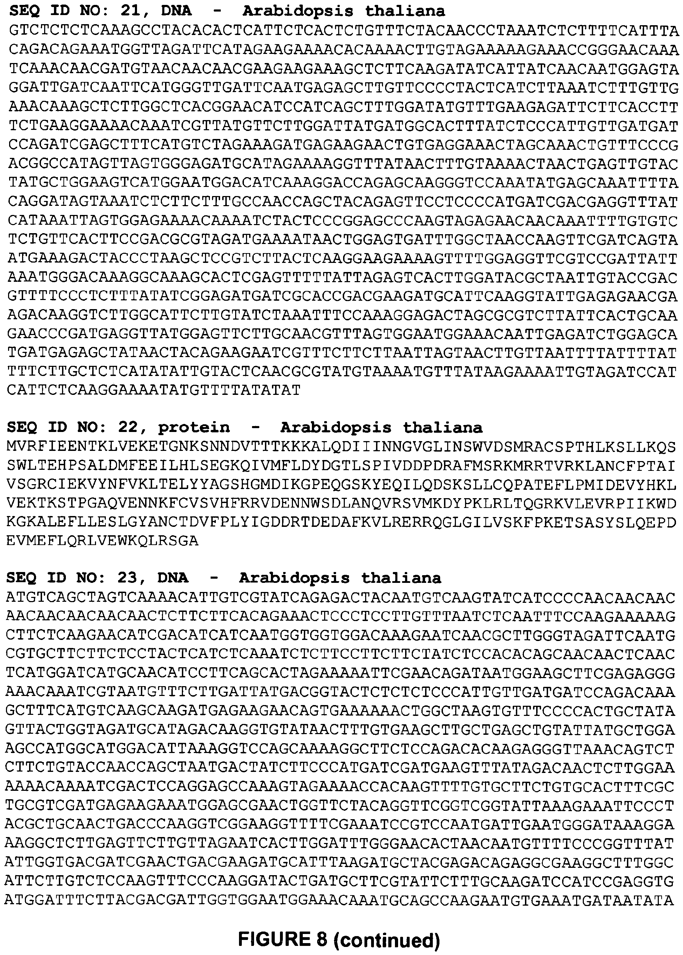

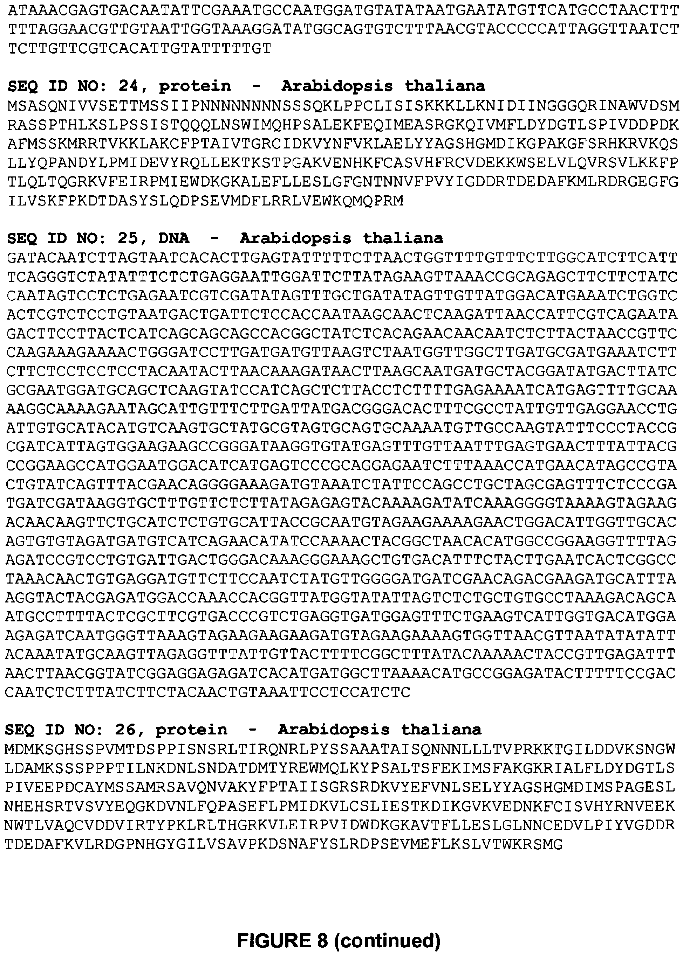

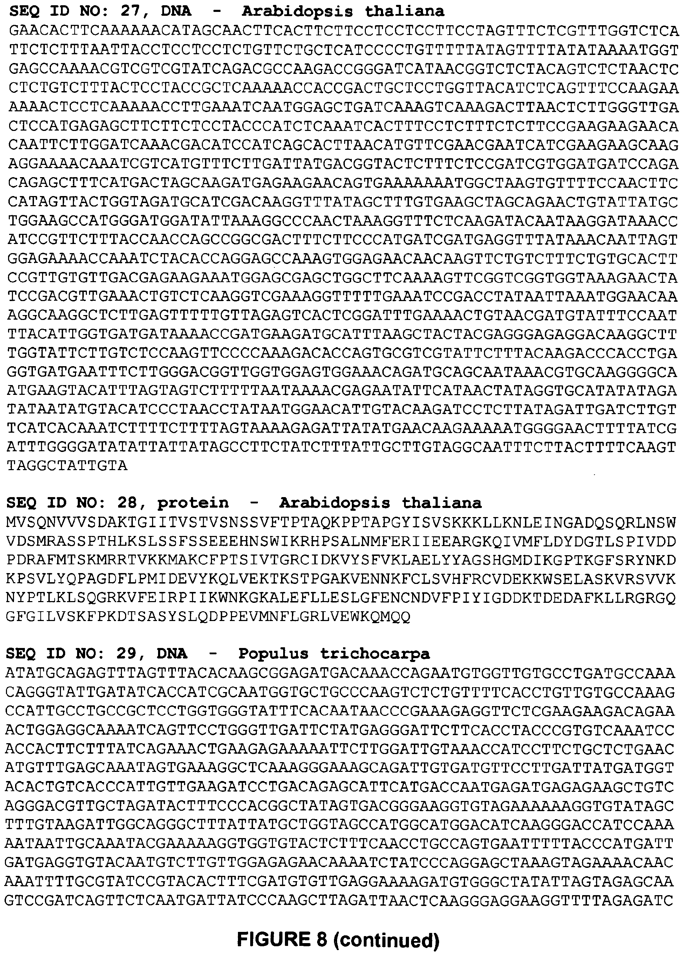

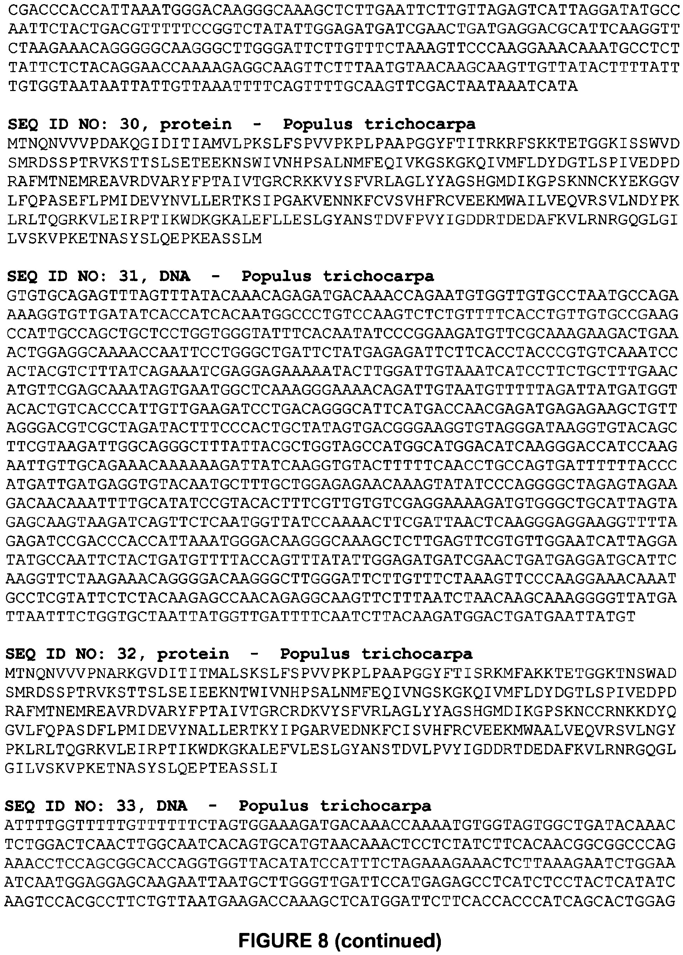

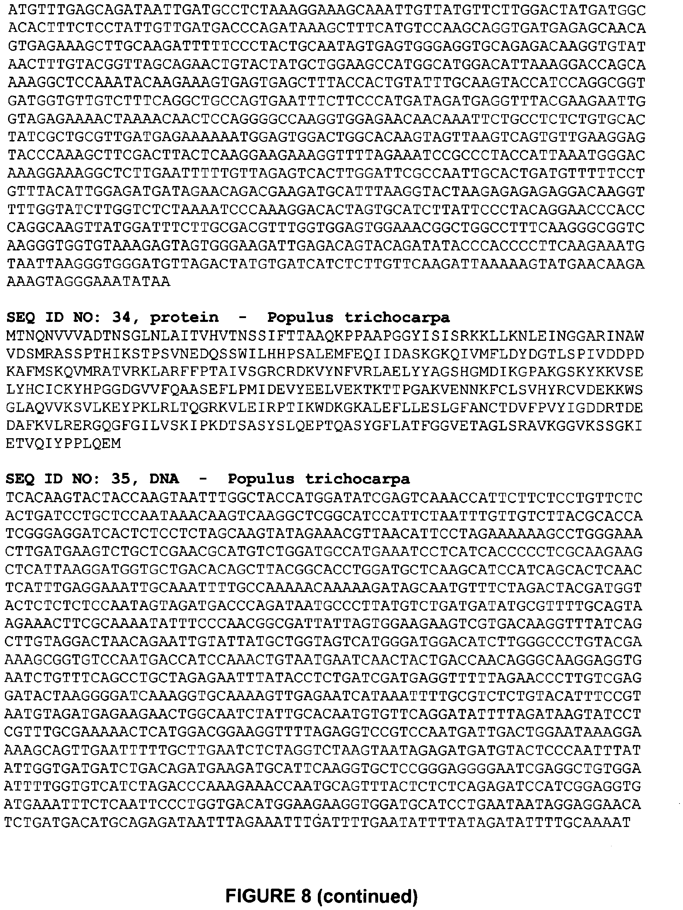

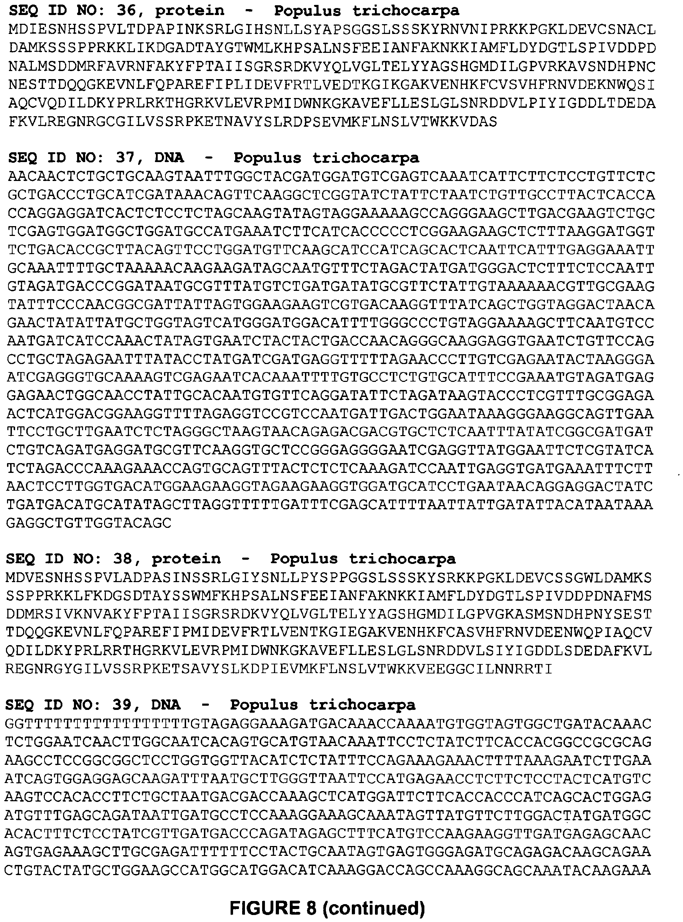

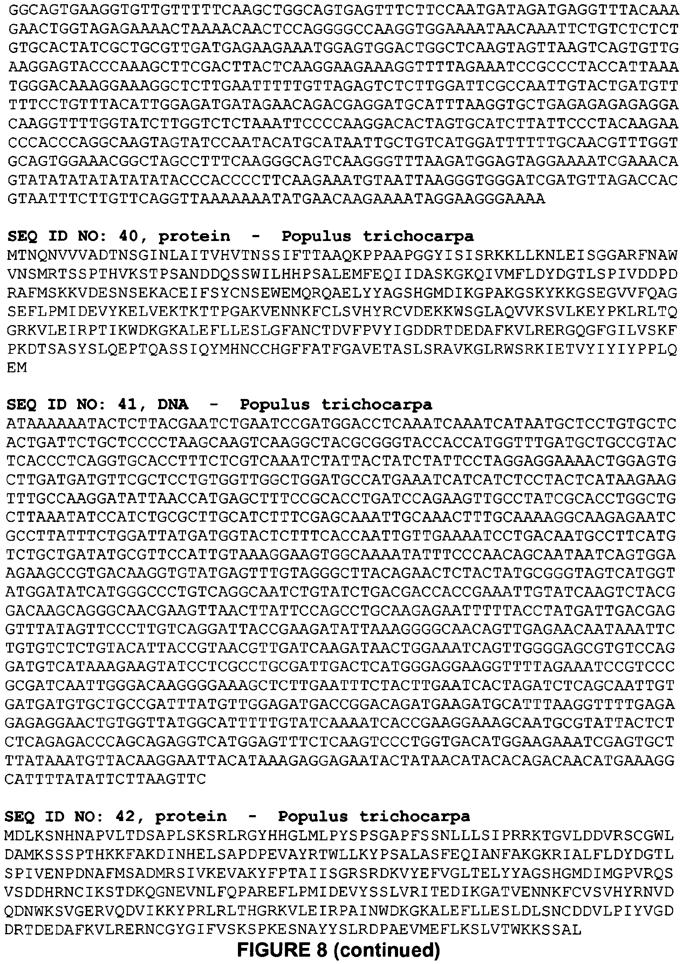

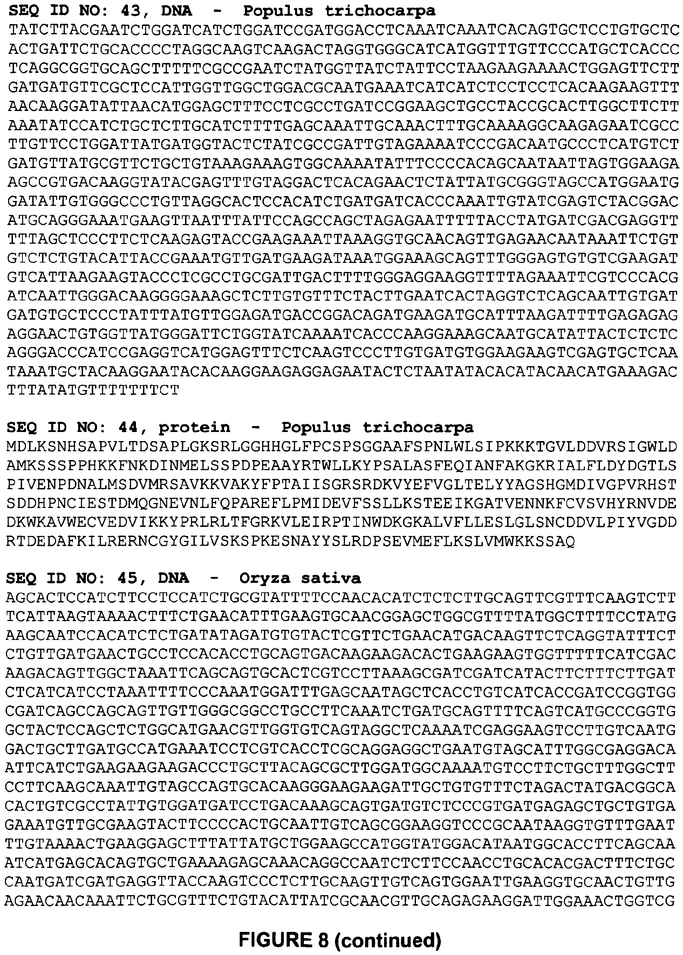

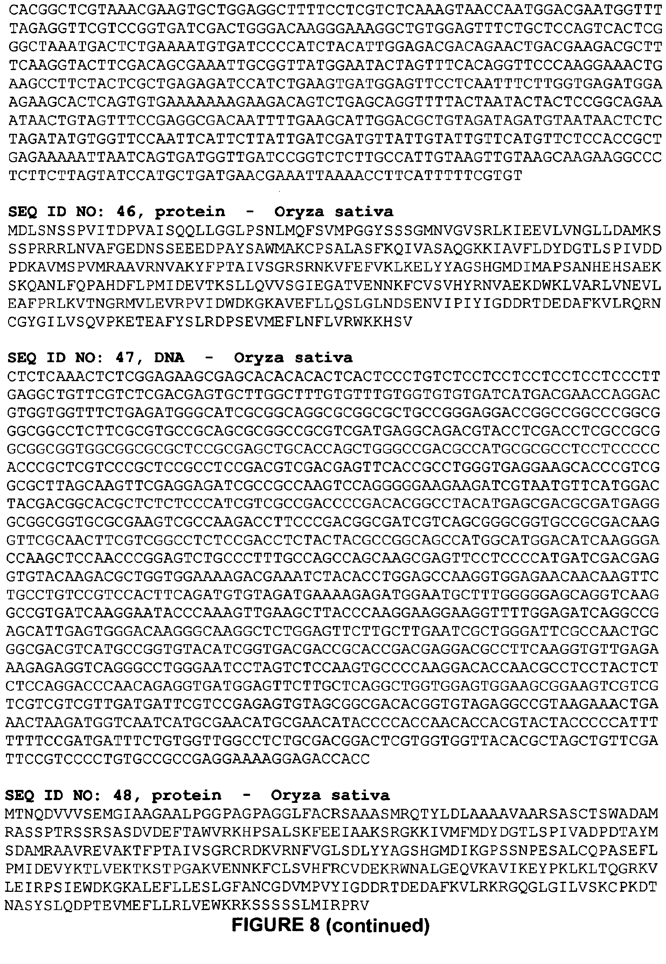

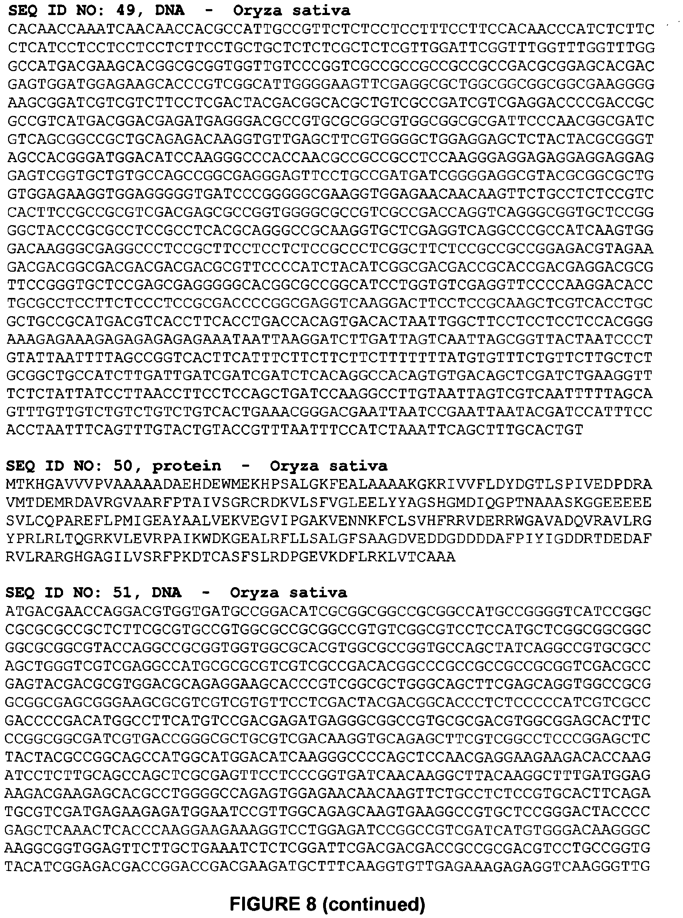

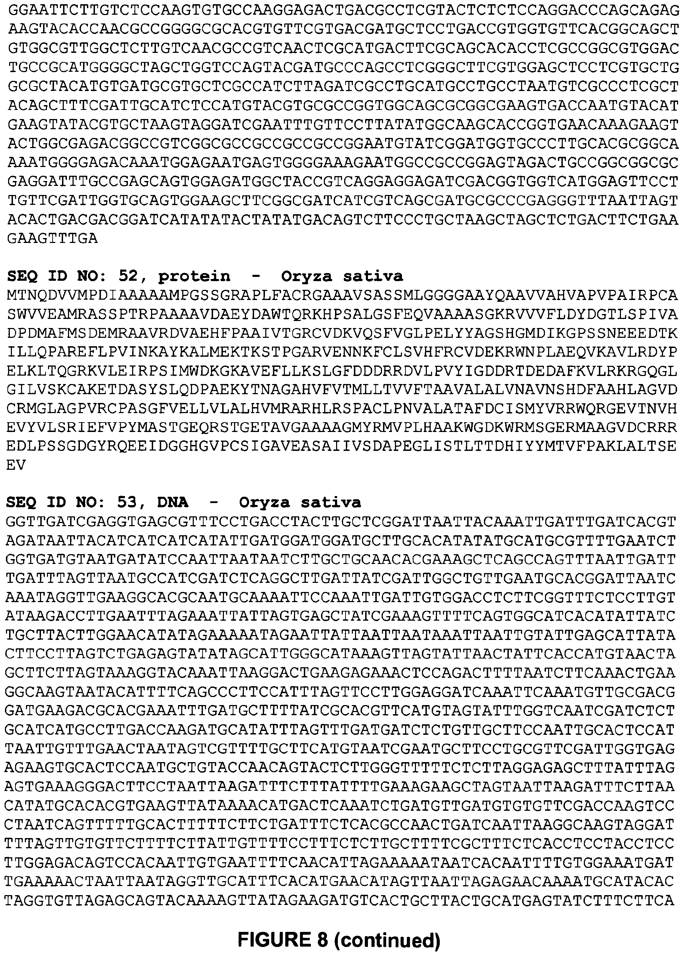

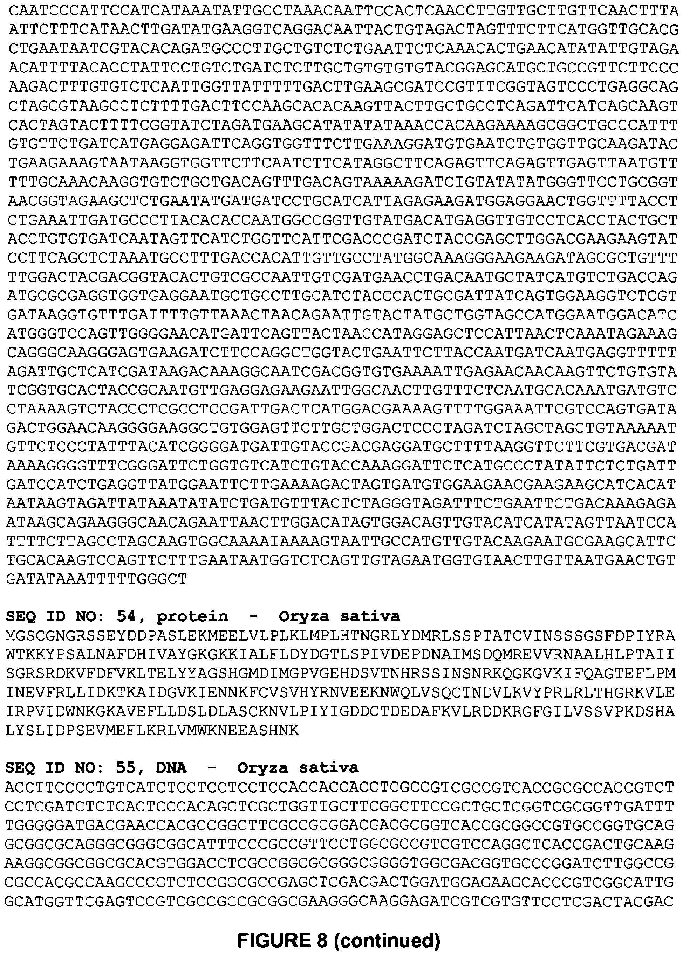

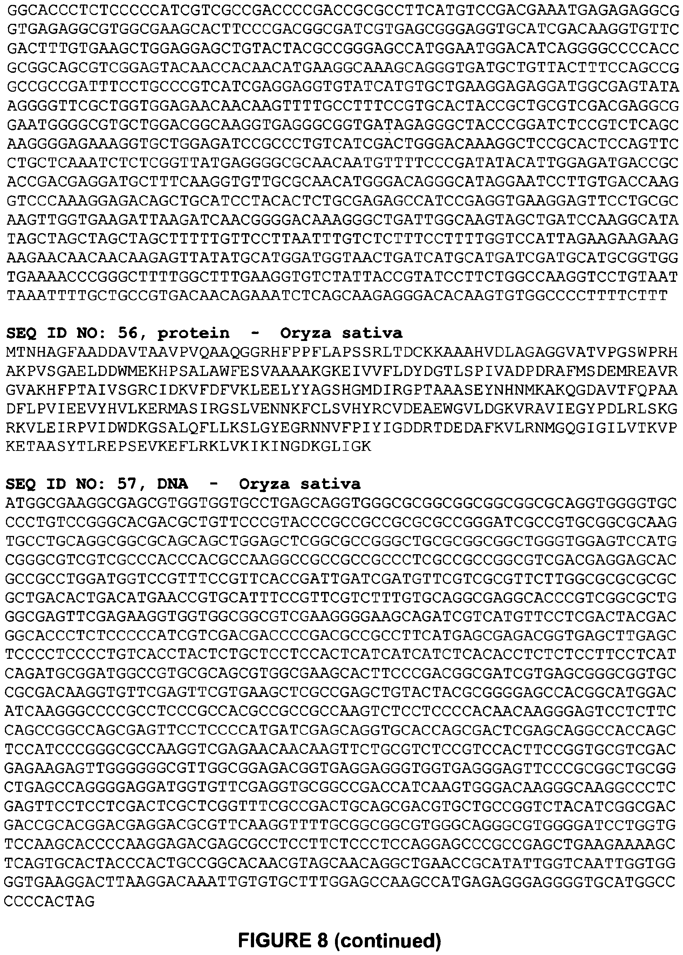

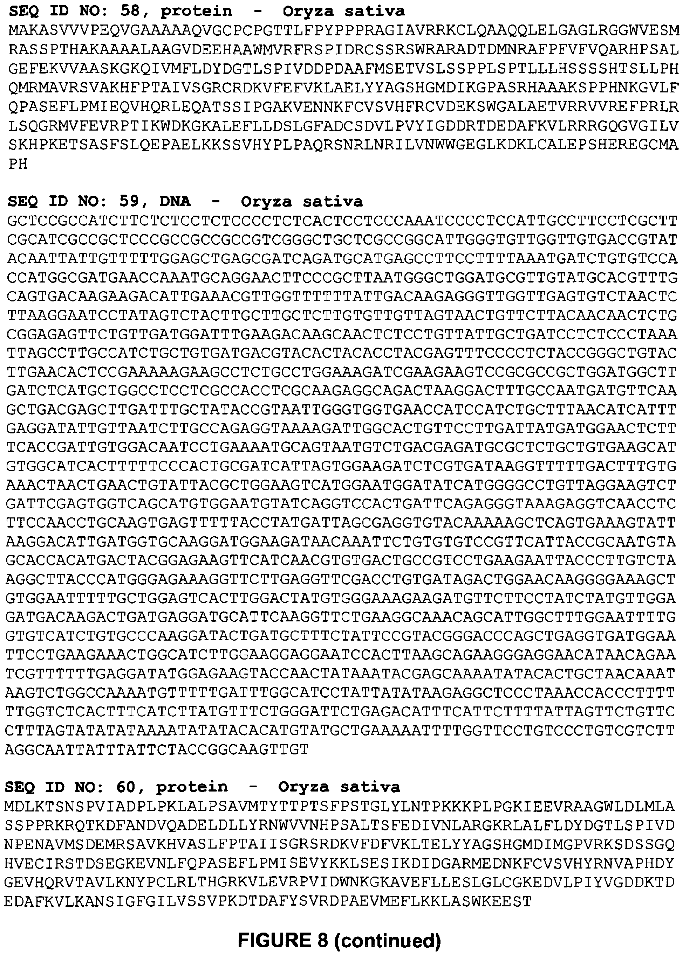

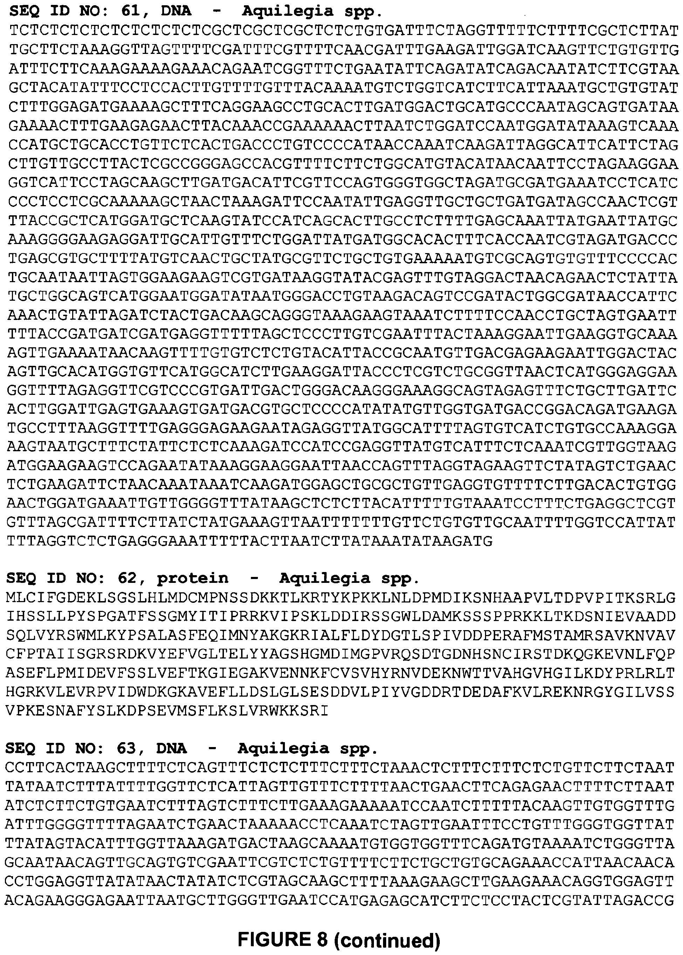

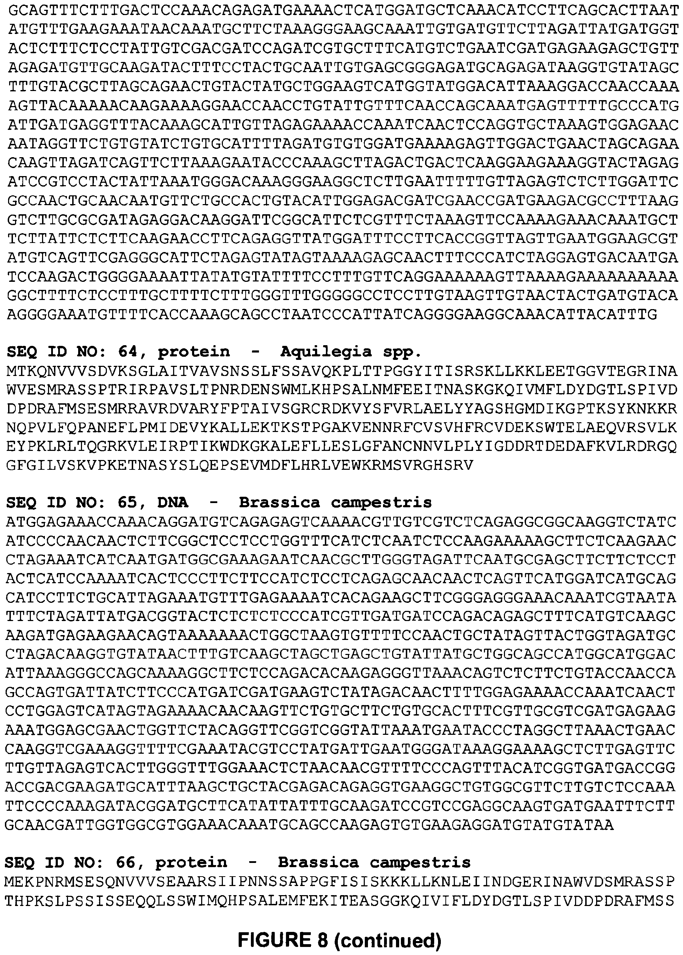

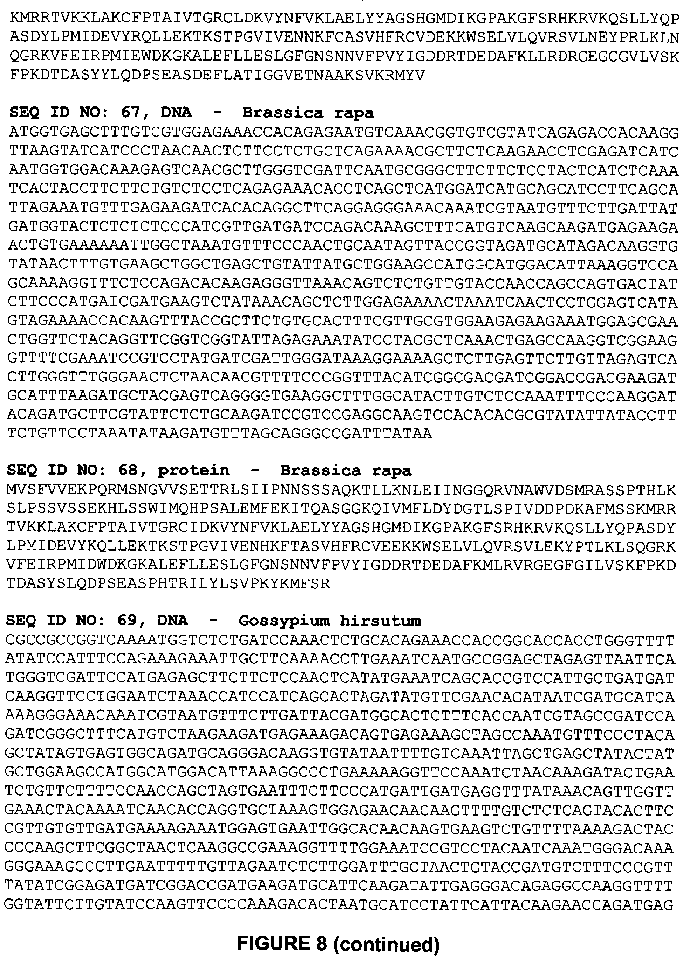

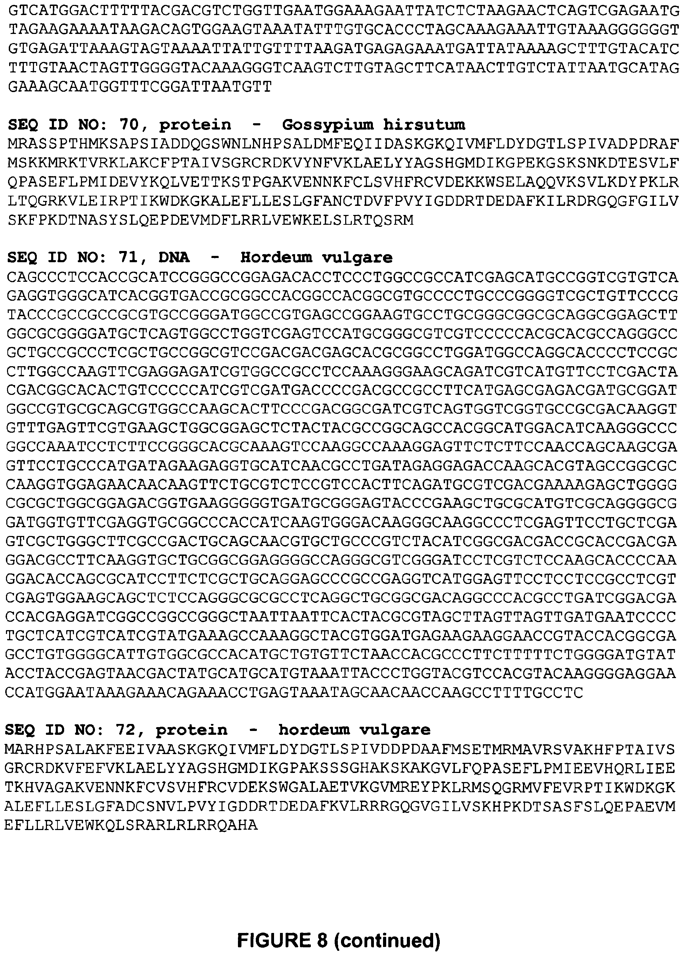

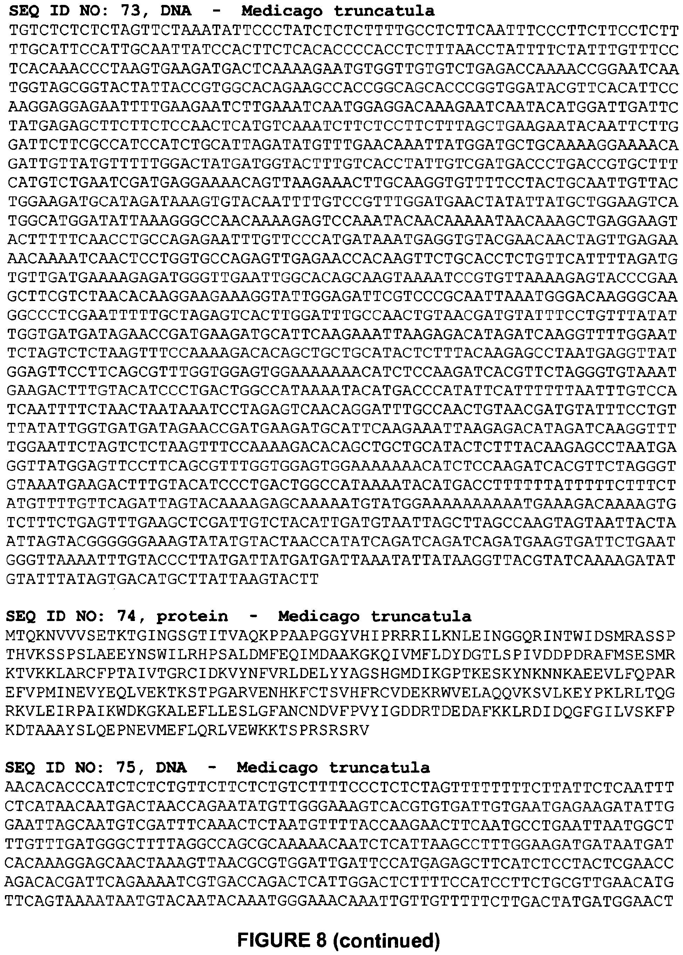

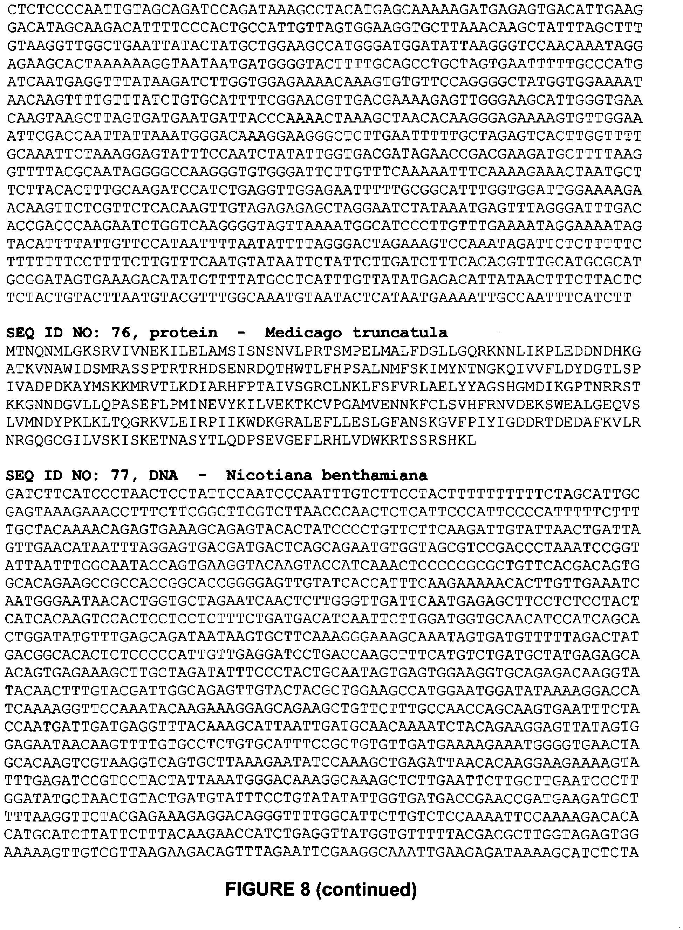

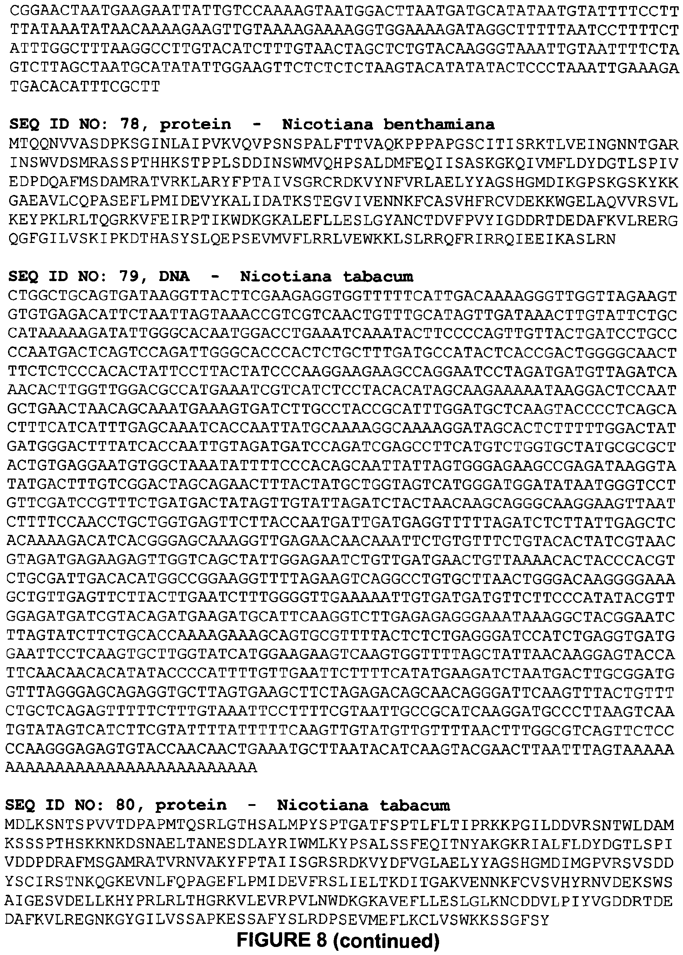

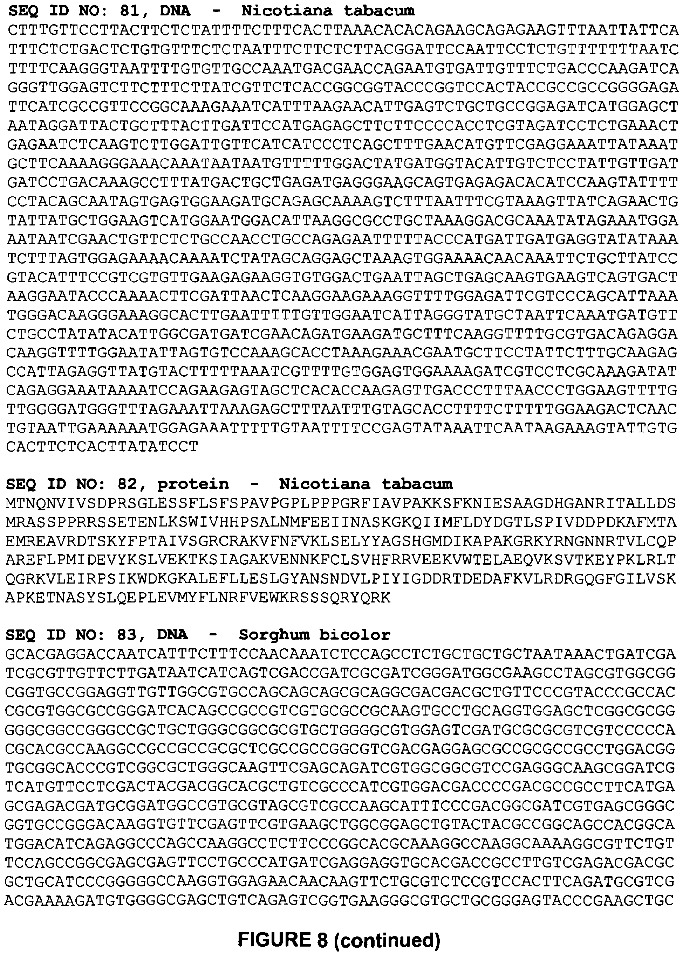

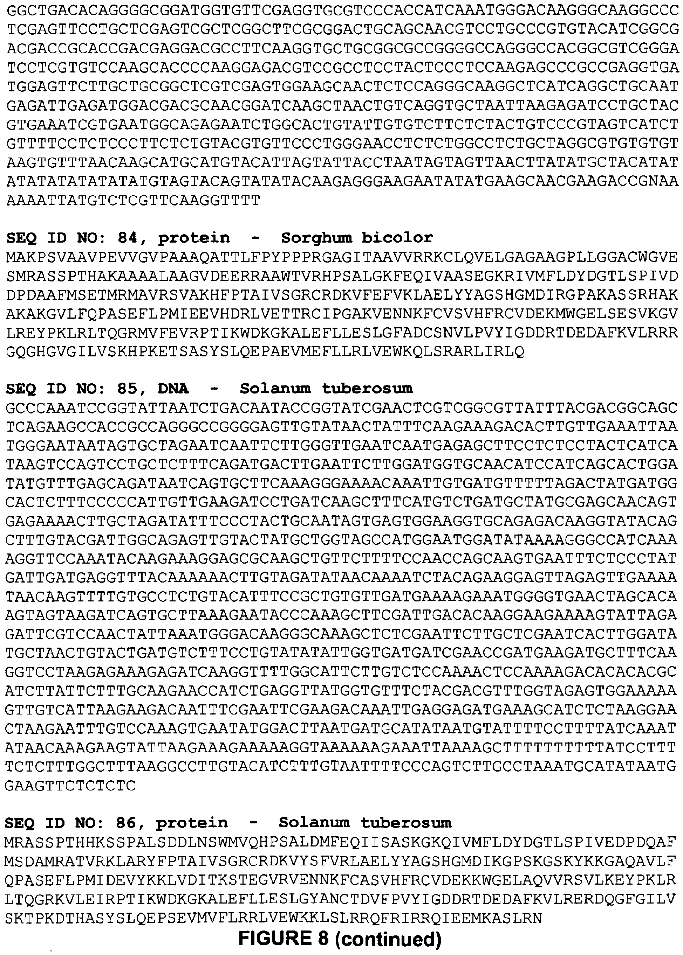

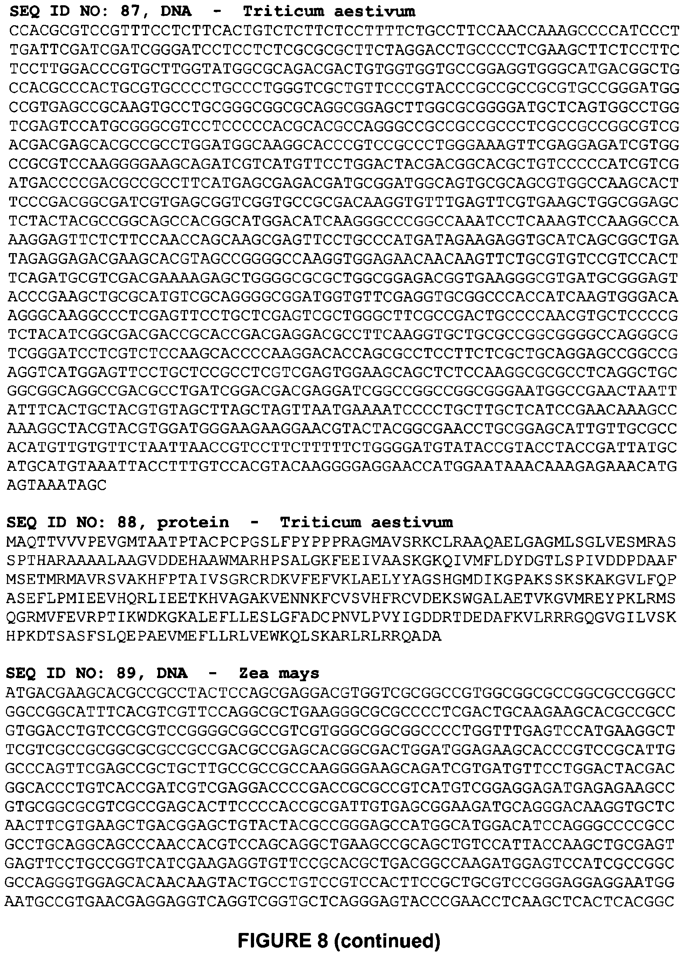

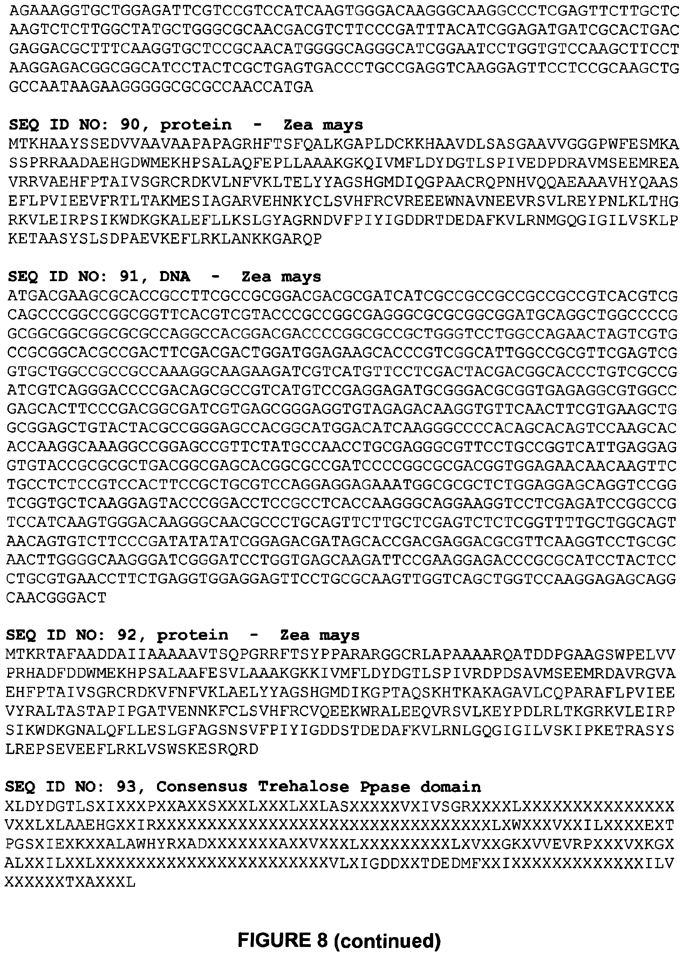

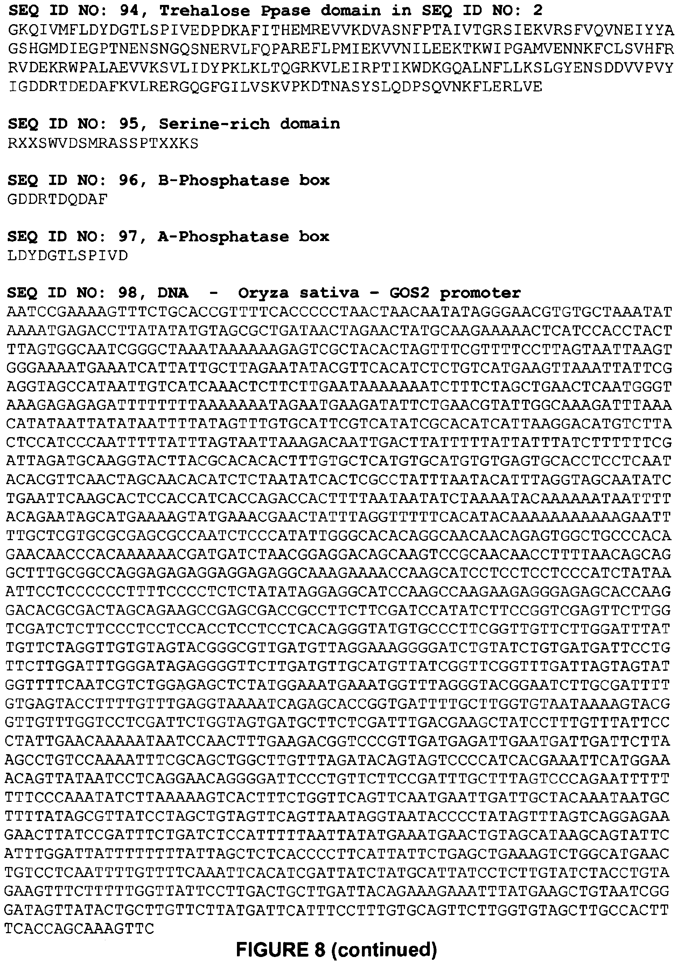

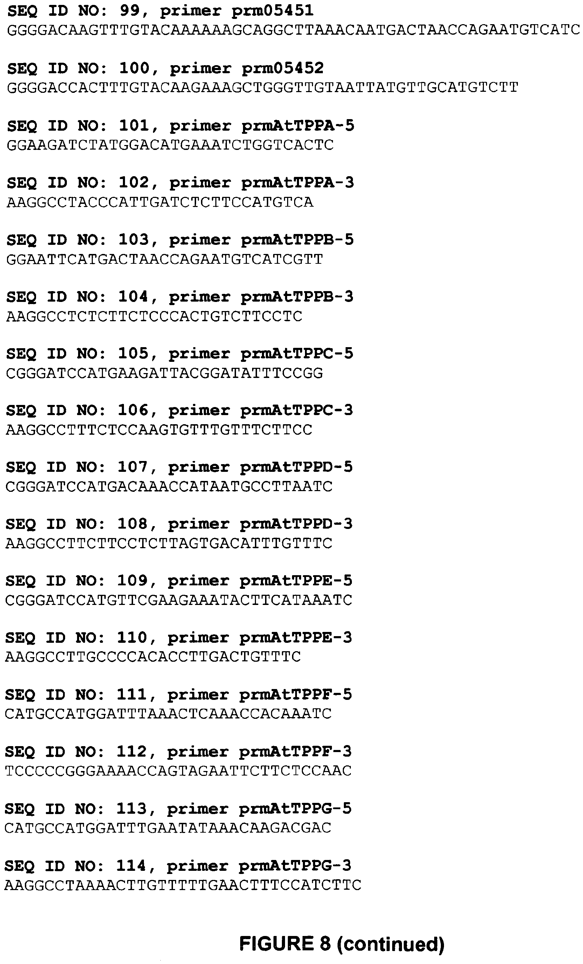

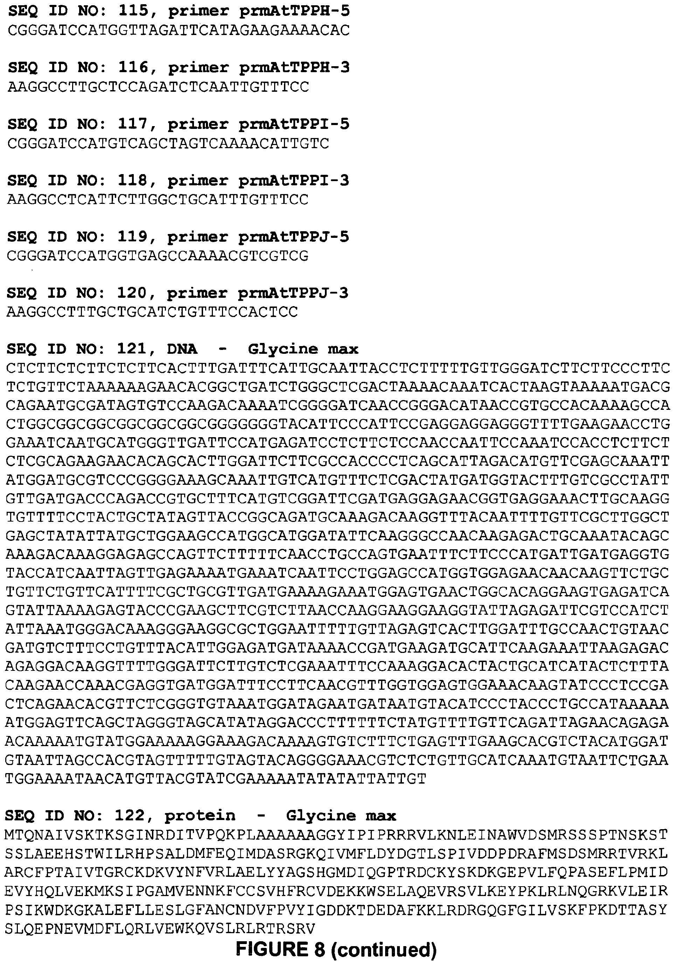

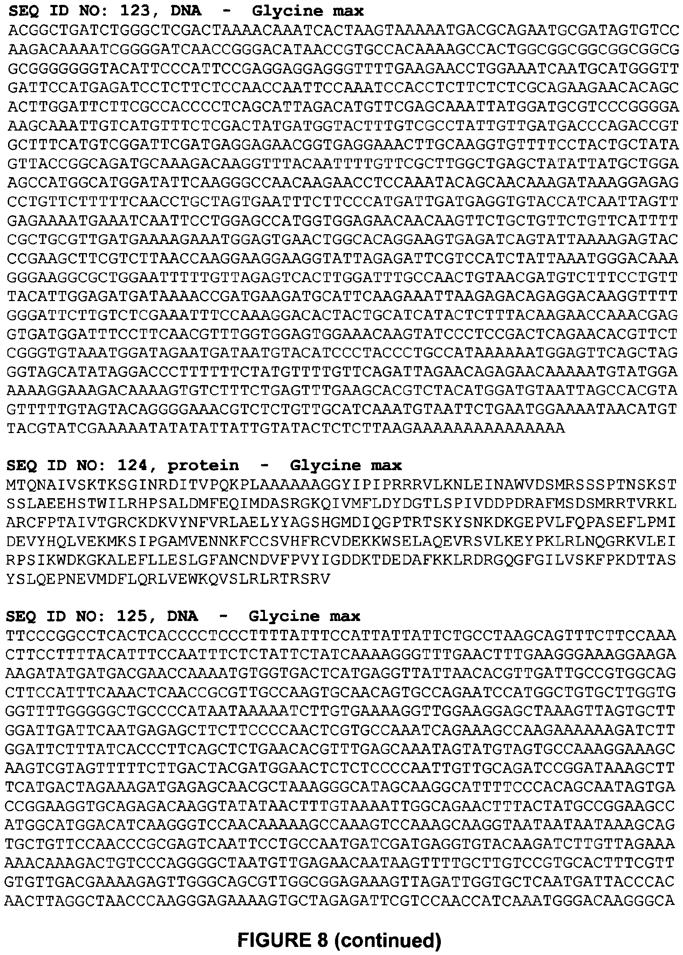

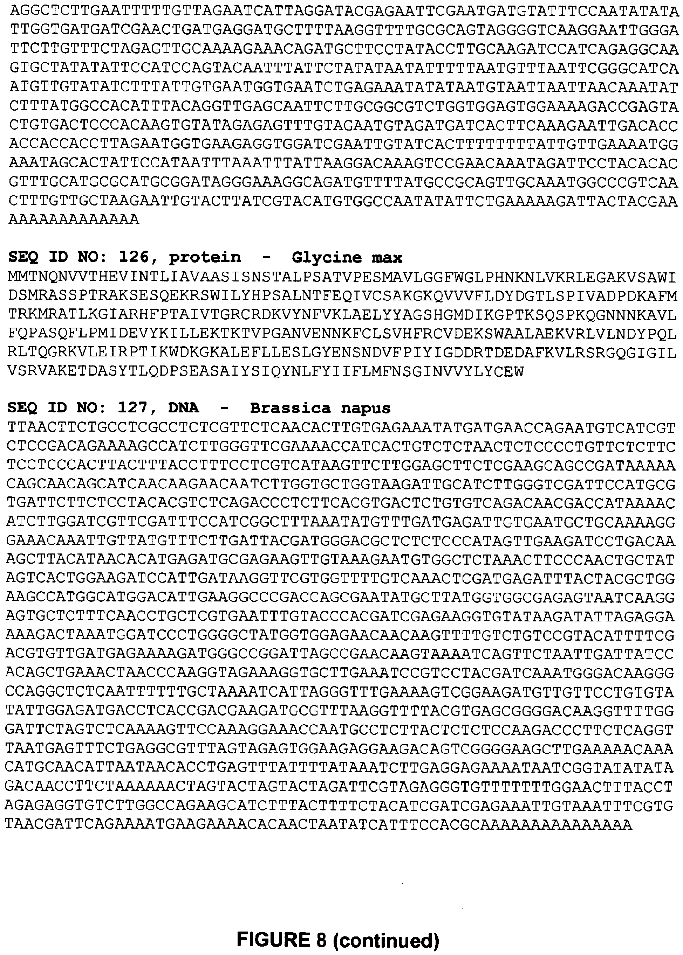

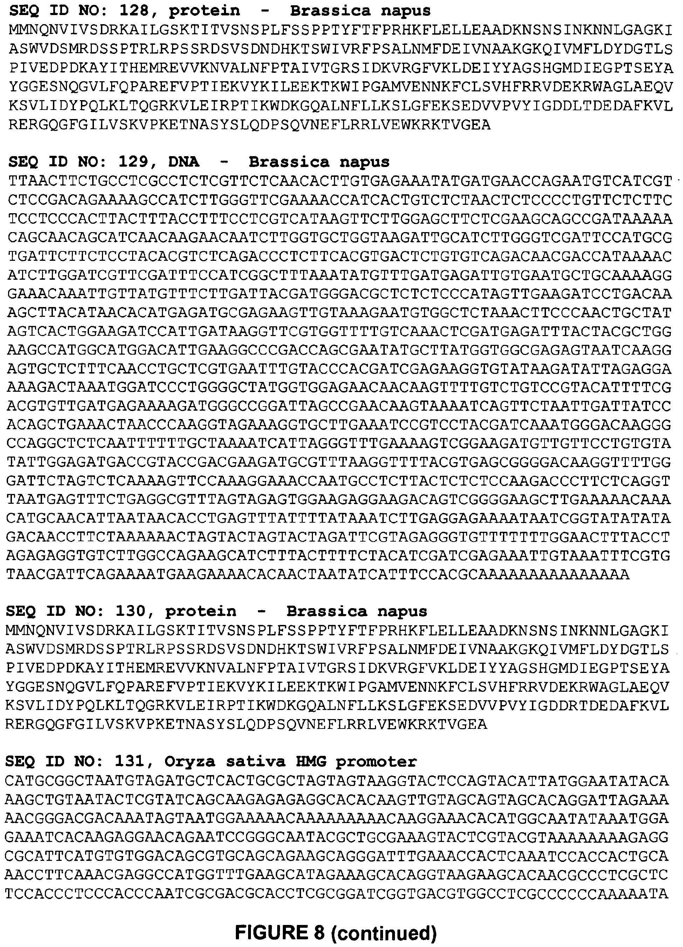

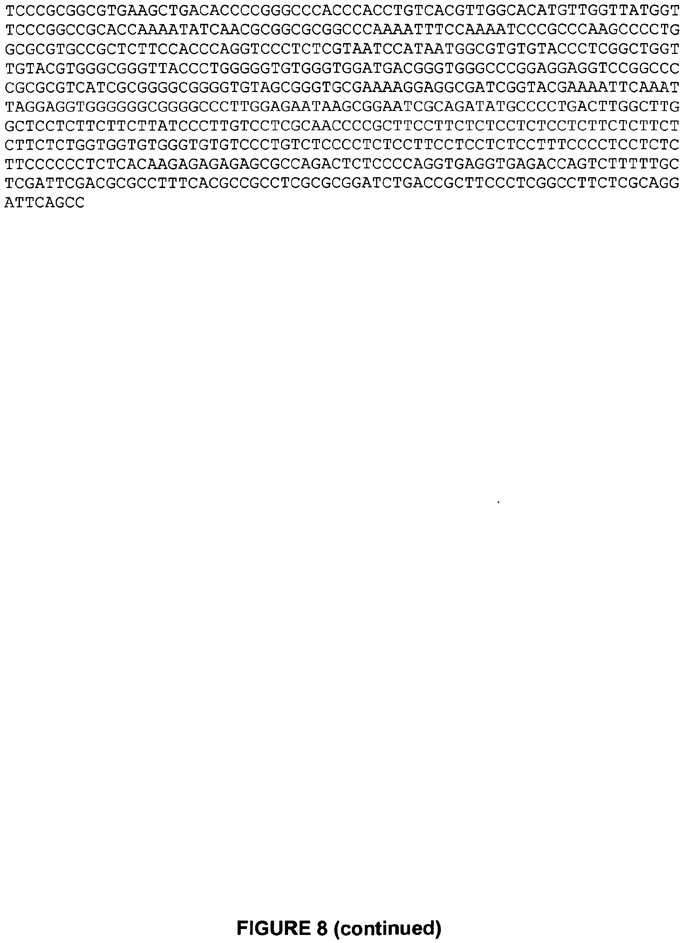

**Fig. 8** Sequence listing.

## Examples

The present invention will now be described with reference to the following examples, which are by way of illustration alone. The following examples are not intended to completely define or otherwise limit the scope of the invention.

### *Example 1: Identification of sequences related to SEQ ID NO: 1 and SEQ ID NO: 2*

Sequences (full length cDNA, ESTs or genomic) related to SEQ ID NO: 1 and/or protein sequences related to SEQ ID NO: 2 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program was used to find regions of local similarity between sequences by comparing nucleic acid or polypeptide sequences to sequence databases and by calculating the statistical significance of matches. The polypeptide encoded by SEQ ID NO: 1 was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters were adjusted to modify the stringency of the search.

Table A provides a list of nucleic acid and protein sequences related to the nucleic acid sequence as represented by SEQ ID NO: 1 and the protein sequence represented by SEQ ID NO: 2.

**Table A:** Nucleic acid sequences related to the nucleic acid sequence represented by SEQ ID NO: 1 useful in the methods of the present invention, and the corresponding deduced polypeptides. For each sequence the database accession number of either the protein or the nucleic acid encoding such protein is given.

| Name | Source organism | NT/PROT | SEQ ID NO: | Database accession number | Status |
|---|---|---|---|---|---|
| >SEQ ID NO: 2 | *Arabidopsis thaliana* | nt | 1 | NA | Full length |
| >SEQ ID NO: 2 | *Arabidopsis thaliana* | PROT | 2 | NA | Full length |
| >Ta_ contig10083@1008 | *Triticum_ aestivum* | nt | 3 | NA | Full length |
| >Ta_ contig10083@1008 | *Triticum_ aestivum* | PROT | 4 | NA | Full length |
| >Gm_contig16565 | *Glycine_max* | nt | 5 | NA | Full length |
| >Gm_contig16565 | *Glycine_max* | PROT | 6 | NA | Full length |
| >TAG_Contig-7-M6b1 | *Tagetes ssp* | nt | 7 | NA | Full length |
| >TAG_Contig-7-M6b1 | *Tagetes ssp* | PROT | 8 | NA | Full length |
| >AT1G22210 | *Arabidopsis thaliana* | nt | 9 | AT1G22210 | Full length |
| >AT1G22210 | *Arabidopsis thaliana* | PROT | 10 | AT1G22210 | Full length |
| >AT1G35910 | *Arabidopsis thaliana* | nt | 11 | AT1G35910 | Full length |
| >AT1G35910 | *Arabidopsis thaliana* | PROT | 12 | AT1G35910 | Full length |

(continued)

| Name | Source organism | NT/PROT | SEQ ID NO: | Database accession number | Status |
|---|---|---|---|---|---|
| >AT1 G78090 | *Arabidopsis thaliana* | nt | 13 | AT1G78090 | Full length |
| >AT1G78090 | *Arabidopsis thaliana* | PROT | 14 | AT1G78090 | Full length |
| >AT2G22190 | *Arabidopsis thaliana* | nt | 15 | AT2G22190 | Full length |
| >AT2G22190 | *Arabidopsis thaliana* | PROT | 16 | AT2G22190 | Full length |
| >AT4G12430 | *Arabidopsis thaliana* | nt | 17 | AT4G12430 | Full length |
| >AT4G12430 | *Arabidopsis thaliana* | PROT | 18 | AT4G12430 | Full length |
| >AT4G22590 | *Arabidopsis thaliana* | nt | 19 | AT4G22590 | Full length |
| >AT4G22590 | *Arabidopsis thaliana* | PROT | 20 | AT4G22590 | Full length |
| >At4g39770 | *Arabidopsis thaliana* | nt | 21 | At4g39770 | Full length |
| >At4g39770 | *Arabidopsis thaliana* | PROT | 22 | At4g39770 | Full length |
| >AT5G10100 | *Arabidopsis thaliana* | nt | 23 | AT5G10100 | Full length |
| >AT5G10100 | *Arabidopsis thaliana* | PROT | 24 | AT5G10100 | Full length |
| >AT5G51460 | *Arabidopsis thaliana* | nt | 25 | AT5G51460 | Full length |
| >AT5G51460 | *Arabidopsis thaliana* | PROT | 26 | AT5G51460 | Full length |
| >AT5G65140 | *Arabidopsis thaliana* | nt | 27 | AT5G65140 | Full length |
| >AT5G65140 | *Arabidopsis thaliana* | PROT | 28 | AT5G65140 | Full length |
| >pOP-Icl\|scaff_II. 875 | *Populus trichocarpa* | nt | 29 | pOP-Icl\|scaff_II. 875 | Full length |
| >pOP-Icl\|scaff_II. 875 | *Populus trichocarpa* | PROT | 30 | pOP-Icl\|scaff_II. 875 | Full length |
| >pOP-Icl\|scaff_V. 739 | *Populus trichocarpa* | nt | 31 | pOP-Icl\|scaff_V. 739 | Full length |
| >pOP-Icl\|scaff_V. 739 | *Populus trichocarpa* | PROT | 32 | pOP-Icl\|scaff_V. 739 | Full length |
| >pOP-Icl\|scaff_VII. 559 | *Populus trichocarpa* | nt | 33 | pOP-Icl\|scaff_ VII.559 | Full length |

(continued)

| Name | Source organism | NT/PROT | SEQ ID NO: | Database accession number | Status |
|---|---|---|---|---|---|
| >pOP-Icl\|scaff_VII.559 | *Populus trichocarpa* | PROT | 34 | pOP-Icl\|scaff_VII.559 | Full length |
| >pOP-IIcl\|scaff_127.52 | *Populus trichocarpa* | nt | 35 | pOP-IIcl\|scaff_127.52 | Full length |
| >pOP-IIcl\|scaff_127.52 | *Populus trichocarpa* | PROT | 36 | pOP-IIcl\|scaff_127.52 | Full length |
| >pOP-IIcl\|scaff_III.843 | *Populus trichocarpa* | nt | 37 | pOP-IIcl\|scaff_III.843 | Full length |
| >pOP-IIcl\|scaff_III.843 | *Populus trichocarpa* | PROT | 38 | pOP-IIcl\|scaff_III.843 | Full length |
| >pOP-IIcl\|scaff_V.167 | *Populus trichocarpa* | nt | 39 | pOP-IIcl\|scaff_V.167 | Full length |
| >pOP-IIcl\|scaff_V.167 | *Populus trichocarpa* | PROT | 40 | pOP-IIcl\|scaff_V.167 | Full length |
| >pOP-IIcl\|scaff_XII.1254 | *Populus trichocarpa* | nt | 41 | pOP-IIcl\|scaff_XII.1254 | Full length |
| >pOP-IIcl\|scaff_XII.1254 | *Populus trichocarpa* | PROT | 42 | pOP-IIcl\|scaff_XII.1254 | Full length |
| >pOP-IIcl\|scaff_XV.1052 | *Populus trichocarpa* | nt | 43 | pOP-IIcl\|scaff_XV.1052 | Full length |
| >pOP-IIcl\|scaff_XV.1052 | *Populus trichocarpa* | PROT | 44 | pOP-IIcl\|scaff_XV.1052 | Full length |
| >Os02g0661100 | *Oryza sativa* | nt | 45 | Os02g0661100 | Full length |
| >Os02g0661100 | *Oryza sativa* | PROT | 46 | Os02g0661100 | Full length |
| >Os02g0753000 | *Oryza sativa* | nt | 47 | Os02g0753000 | Full length |
| >Os02g0753000 | *Oryza sativa* | PROT | 48 | Os02g0753000 | Full length |
| >OsO3gO386500 | *Oryza sativa* | nt | 49 | Os03g0386500 | Full length |
| >Os03g0386500 | *Oryza sativa* | PROT | 50 | Os03g0386500 | Full length |
| >Os06g0222100 | *Oryza sativa* | nt | 51 | Os06g0222100 | Full length |
| >Os06g0222100 | *Oryza sativa* | PROT | 52 | Os06g0222100 | Full length |
| >Os07g0485000 | *Oryza sativa* | nt | 53 | Os07g0485000 | Full length |
| >Os07g0485000 | *Oryza sativa* | PROT | 54 | Os07g0485000 | Full length |
| >Os07g0624600 | *Oryza sativa* | nt | 55 | Os07g0624600 | Full length |
| >Os07g0624600 | *Oryza sativa* | PROT | 56 | Os07g0624600 | Full length |
| >Os09g0369400 | *Oryza sativa* | nt | 57 | Os09g0369400 | Full length |
| >Os09g0369400 | *Oryza sativa* | PROT | 58 | Os09g0369400 | Full length |
| >Os10g0553300 | *Oryza sativa* | nt | 59 | Os10g0553300 | Full length |
| >Os10g0553300 | *Oryza sativa* | PROT | 60 | Os10g0553300 | Full length |
| >Aquilegia_TC11239 | *Aquilegia ssp* | nt | 61 | Aquilegia_TC11239 | Full length |

(continued)

| Name | Source organism | NT/PROT | SEQ ID NO: | Database accession number | Status |
|---|---|---|---|---|---|
| >Aquilegia_ TC11239 | *Aquilegia ssp* | PROT | 62 | Aquilegia_ TC11239 | Full length |
| >Aquilegia_ TC17706 | *Aquilegia ssp* | nt | 63 | Aquilegia_ TC17706 | Full length |
| >Aquilegia_ TC17706 | *Aquilegia ssp* | PROT | 64 | Aquilegia_ TC17706 | Full length |
| >Bc_Q4AC11 | *Brassica campestris* | nt | 65 | Bc_Q4AC11 | Full length |
| >Bc_Q4AC11 | *Brassica campestris* | PROT | 66 | Bc_Q4AC11 | Full length |
| >Br_Q4ABQ9 | *Brassica rapa* | nt | 67 | Br_Q4ABQ9 | Full length |
| >Br_Q4ABQ9 | *Brassica rapa* | PROT | 68 | Br_Q4ABQ9 | Full length |
| >Gh_TC35369 | *Gossypium-hirsutum* | nt | 69 | Gh_TC35369 | Full length |
| >Gh_TC35369 | *Gossypium_ hirsutum* | PROT | 70 | Gh_TC35369 | Full length |
| >Hv_TC139314 | *Hordeum vulgare* | nt | 71 | HV_TC139314 | Full length |
| >Hv_TC139314 | *Hordeum vulgare* | PROT | 72 | HV_TC139314 | Full length |
| >Mt_TC108059 | *Medicago_ truncatula* | nt | 73 | Mt_TC108059 | Full length |
| >Mt_TC108059 | *Medicago_ truncatula* | PROT | 74 | Mt_TC108059 | Full length |
| >Mt_TC108097 | *Medicago_ truncatula* | nt | 75 | Mt_TC108097 | Full length |
| >Mt_TC108097 | *Medicago_ truncatula* | PROT | 76 | Mt_TC108097 | Full length |
| >Nb_TC7464 | *Nicotiana benthamiana* | nt | 77 | Nb_TC7464 | Full length |
| >Nb_TC7464 | *Nicotiana benthamiana* | PROT | 78 | Nb_TC7464 | Full length |
| >Nt_Q3ZTF5 | *Nicotiana tabacum* | nt | 79 | Nt_Q3ZTF5 | Full length |
| >Nt_Q3ZTF5 | *Nicotiana tabacum* | PROT | 80 | Nt_Q3ZTF5 | Full length |
| >Nt_TC7310 | *Nicotiana tabacum* | nt | 81 | Nt_TC7310 | Full length |
| >Nt_TC7310 | *Nicotiana tabacum* | PROT | 82 | Nt_TC7310 | Full length |
| >Sb_TC17204 | *Sorghum_ bicolor* | nt | 83 | Sb_TC17204 | Full length |

(continued)

| Name | Source organism | NT/PROT | SEQ ID NO: | Database accession number | Status |
|---|---|---|---|---|---|
| >Sb_TC17204 | *Sorghum_ bicolor* | PROT | 84 | Sb_TC17204 | Full length |
| >St_TC151769 | *Solanum-tuberosum* | nt | 85 | St_TC151769 | Full length |
| >St_TC151769 | *Solanum_ tuberosum* | PROT | 86 | St_TC151769 | Full length |
| >Ta_TC252250 | *Triticum_ aestivum* | nt | 87 | Ta_TC252250 | Full length |
| >Ta_TC252250 | *Triticum_ aestivum* | PROT | 88 | Ta_TC252250 | Full length |
| >Zm_ABD92779 | *Zea mays* | nt | 89 | Zm_ABD92779 | Full length |
| >Zm_ABD92779 | *Zea mays* | PROT | 90 | Zm_ABD92779 | Full length |
| >Zm_ABD92780 | *Zea mays* | nt | 91 | Zm_ABD92780 | Full length |
| >Zm_ABD92780 | *Zea mays* | PROT | 92 | Zm_ABD92780 | Full length |
| NA. It does not apply. Not found in public databases.<br>nt: nucleotide sequence<br>PROT: protein sequence | | | | | |

***Example 2: Alignment of* Class III TPP polypeptide *sequences***

Alignment of Class III TPP polypeptide sequences was performed using the AlignX program from the Vector NTI (Invitrogen) is based on the popular Clustal algorithm of progressive alignment (Thompson et al. (1997) Nucleic Acids Res 25:4876-4882; Chenna et al. (2003). Nucleic Acids Res 31:3497-3500). A phylogenetic tree was constructed using a neighbour-joining clustering algorithm, using default values (gap open penalty of 10, gap extension penalty of 0,1 and selected weight matrix is Blosum 62 (if polypeptides are aligned)).

***Example 3: Calculation of global percentage identity between polypeptide sequences useful in performing the methods of the invention***

Global percentages of similarity and identity between some of the full length polypeptide sequences given in Table A of Example 1 were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program was used to perform a series of pair-wise alignments using the Myers and Miller global alignment algorithm (with a gap opening penalty of 12, and a gap extension penalty of 2), and to calculate similarity and identity using Blosum 62 (for polypeptides) and to place the results in a distance matrix. Sequence similarity is shown in the bottom half of the dividing line and sequence identity is shown in the top half of the diagonal dividing line.

Parameters used in the comparison were:

**Scoring matrix: Blosum62**

First Gap: 12

Extending gap: 2

Results of the software analysis are shown in Table B for the global similarity and identity as displayed below and above the diagonal respectively. Global similarity of SEQ ID NO:2 to the paralogous proteins shown Table B1 is above 48% identity. Similarity of SEQ ID NO:2 to the plant homeologous sequences given in Tables B2 and B3 is above 40 % identity for proteins of dicotyledoneous plant origin and above 45% identity for monocotyledoneous.

**Table B:** MatGAT results for global similarity and identity over the full length of the polypeptide sequences.

**Table B1.** Global similarity and identity amongst paralogous Class III TPP polypeptides in *Arabidopsis thaliana.*

| | 1 | 2 | 3 | 4 | 5 | 6 | 7 | 8 | 9 |
|---|---|---|---|---|---|---|---|---|---|
| 1. AT2G22190 | | 48.5 | 64 | 61.8 | 57.2 | 48.6 | 48.8 | 51.9 | 55.3 |
| 2. AT5G51460 | 65.2 | | 48.6 | 48.6 | 51.5 | 59.1 | 61 | 45 | 48.2 |
| 3. AT5G65140 | 78.1 | 69.1 | | 74.1 | 60.2 | 47.1 | 47.3 | 51.1 | 60.1 |
| 4. AT5G10100 | 75.6 | 66.5 | 88.4 | | 57.3 | 45.7 | 47.2 | 50.3 | 55.1 |
| 5. AT1G35910 | 72.1 | 70.4 | 78.1 | 76.7 | | 50.9 | 52 | 54.2 | 59.7 |
| 6. AT4G22590 | 65.5 | 77.1 | 67.9 | 67.9 | 69.5 | | 81.2 | 45.8 | 47.7 |
| 7. AT4G12430 | 66.3 | 76.1 | 67.6 | 69.4 | 72.1 | 89.7 | | 46.7 | 49.4 |
| 8. AT1G22210 | 67.5 | 60.3 | 66.8 | 65.3 | 68.8 | 61.5 | 63.3 | | 58.5 |
| 9. AT1 G78090 | 69.5 | 68.6 | 77 | 74.1 | 77.3 | 68.4 | 67.9 | 73.5 | |
| 10. SEQ ID NO: 2 | 69.5 | 68.6 | 77 | 74.1 | 77.3 | 68.4 | 67.9 | 73.3 | 100 |

**Table B2.** Global similarity and identity amongst homologous Class III TPP polypeptides from dicotyledonous plant origin.

| **Protein** | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| 1. Nt_Q3ZTF5 | | 49.7 | 49.4 | 50.5 | 50.8 | 50.6 | 44 | 73.7 | 74.6 | 68.2 | 50.4 | 65 | 75.6 | 60.7 |
| 2. Bc_Q4AC11 | 68.2 | | 81.7 | 57.7 | 57.2 | 62.5 | 56.2 | 47.4 | 46 | 47.3 | 60.6. | 47.8 | 46.9 | 44.4 |
| 3. Br_Q4ABQ9 | 67.7 | 88.7 | | 57.7 | 57.7 | 58.5 | 53.2 | 46 | 45.6 | 47.5 | 60.7 | 46.7 | 45.4 | 43.8 |
| 4. pOP-IcI\|scaff_II.875 | 65.6 | 75.7 | 75.1 | | 89.9 | 62 | 58.1 | 49.5 | 49.6 | 49.1 | 59.1 | 47.9 | 50.1 | 46.4 |
| 5. pOP-IcI\|scaff_V.739 | 66.9 | 75.5 | 74.3 | 94.7 | | 60.1 | 55.9 | 50.1 | 49.5 | 50 | 57 | 48.1 | 50 | 46 |
| 6. pOP-IcI\|scaff_VII.559 | 66.4 | 75.7 | 71.8 | 74.5 | 74.3 | | 81.9 | 48.3 | 47.6 | 46.5 | 61.5 | 47.3 | 49.1 | 44.1 |
| 7. pOP-IIcI\|scaff_V.167 | 63.1 | 72.9 | 68.6 | 72.1 | 71.9 | 88 | | 42 | 41.7 | 41.8 | 57.4 | 42.7 | 42.6 | 40.5 |
| 8. pOP-IIcI\|scaff_XV.1052 | 85 | 66.6 | 63 | 63.2 | 64.8 | 66.7 | 62.3 | | 87.1 | 69.1 | 49.1 | 64.6 | 74.4 | 57.8 |
| 9. pOP-IIcI\|scaff_XII.1254 | 87.1 | 66.4 | 63.6 | 63.8 | 65.4 | 66.2 | 61.8 | 92.5 | | 68.5. | 49.2 | 64.7 | 75 | 57.1 |
| 10. pOP-IIcI\|scaff_127.52 | 83.1 | 66.8 | 64.2 | 63.4 | 66.1 | 64.2 | 63.3 | 82.1 | 82.9 | | 48.6 | 85.2 | 67.2 | 54.7 |
| 11. Gh_TC35369 | 62.8 | 70.4 | 70.7 | 70.4 | 70.6 | 67.6 | 64.3 | 60.9 | 61 | 60 | | 48.2. | 49.9 | 57.1 |
| 12. pOP-IIcI\|scaff_III.843 | 80.8 | 64.5 | 63.7 | 65.3 | 64.2 | 63.7 | 62.3 | 80.3 | 81.4 | 91.5 | 59.6 | | 64.9 | 53.4 |
| 13. Gm_contig16565 | 85.9 | 67.4 | 63 | 63.5 | 64.5 | 65.4 | 63.8 | 85.9 | 86.4 | 83 | 61.4 | 81.7 | | 57.8 |
| 14. TAG_Contig-7-M6b1 | 68 | 60.6 | 60.8 | 61.4 | 62.5 | 56.6 | 53.3 | 67.6 | 65.9 | 65.8 | 76 | 64 | 66.1 | |
| 15. SEQ ID NO: 2 | 69.3 | 71.9 | 70.1 | 74.9 | 76.7 | 70.8 | 69.1 | 69.2 | 69.5 | 68.4 | 66 | 66.1 | 68.4 | 61.5 |

**Table B3.** Global similarity and identity amongst homologous Class III TPP polypeptides from monocotyledonous plant origin.

| | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| 1. Os02g0753000 | | 63 | 42.9 | 51.9 | 48 | 51.8 | 49.9 | 47.3 | 47.3 | 62.1 | 54.3 | 48.7 | 57.1 | 57.1 |
| 2. Os08g0409100 | 77 | | 38.2 | 56.2 | 59.9 | 50.8 | 52.5 | 51.6 | 49.4 | 77 | 63.7 | 52.6 | 59.4 | 56.4 |
| 3. Os06g0222100 | 50.8 | 46.3 | | 34.2 | 35 | 33.4 | 31.2 | 28.6 | 29.8 | 37.1 | 31.9 | 31.5 | 34.9 | 35.1 |
| 4. Os07g0624600 | 71.1 | 71.4 | 43.5 | | 45 | 52.9 | 50.4 | 48.3 | 47.3 | 53.9 | 49.3 | 48.4 | 64.7 | 63.4 |
| 5. Os09g0369400 | 58.8 | 69.2 | 48.7 | 57.8 | | 41.5 | 42 | 39.3 | 37.5 | 65.7 | 50.9 | 39.9 | 47.2 | 46.5 |
| 6. Os03g0386500 | 66.2 | 64.9 | 40.3 | 64.5 | 51.1 | | 45.3 | 43.6 | 44.9 | 50.5 | 56.5 | 45 | 55.9 | 55.3 |
| 7. Os02g0661100 | 66.6 | 70.6 | 41.9 | 65.5 | 55.8 | 58.2 | | 51.9 | 55.7 | 50.4 | 46.1 | 84.5 | 50.4 | 49.6 |
| 8. Os07g0485000 | 65.7 | 67 | 40.1 | 63.4 | 51.7 | 60.2 | 68.5 | | 53.9 | 48.1 | 46.7 | 50.6 | 47.2 | 48.5 |
| 9. Os10g0553300 | 65.4 | 66.5 | 40.4 | 64.4 | 53.4 | 58.1 | 72 | 66.5 | | 47.8 | 43.3 | 55.5 | 47.7 | 49.1 |
| 10. Ta_TC252250 | 75.9 | 88.5 | 47.1 | 69.7 | 71.3 | 63.5 | 69.2 | 64.6 | 66.2 | | 74.5 | 50.5 | 57.7 | 55.8 |
| 11. HV_TC139314 | 65.7 | 71.9 | 39.8 | 61.7 | 55.6 | 72.3 | 59 | 60.7 | 56.5 | 75.6 | | 45.7 | 53.6 | 50.4 |
| 12. Ta_contig10083 | 66.9 | 68 | 41.8 | 64.2 | 54.7 | 58.1 | 91.7 | 67.2 | 71.5 | 69.2 | 58.3 | | 48.3 | 47.2 |
| 13. Zm_SRA ABD92780 | 71.4 | 71.4 | 42.4 | 74.3 | 57.5 | 68.5 | 63.6 | 65.5 | 64.1 | 69.2 | 65.1 | 62.9 | | 65.5 |
| 14. Zm_RA3 | 74.1 | 74.3 | 43.8 | 75.7 | 57.3 | 66.5 | 65.5 | 66.8 | 66.2 | 71.6 | 64.5 | 62.6 | 78.1 | |
| 15. SEQ ID NO: 2 | 73.3 | 74.3 | 46.5 | 68.7 | 58.2 | 61.5 | 69.8 | 65.2 | 68.3 | 72.5 | 61.2 | 70.1 | 67.1 | 66.3 |

**Table B4.** Global similarity and identity amongst homologous Class III TPP polypeptides from non-plant origin.

| | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| 1. E.coli_OTSBP | | 36.8 | 36.4 | 35.8 | 29.2 | 30.7 | 23.5 | 26.1 | 11.3 | 36.8 | 36.4 | 29.2 | 30.7 | 23.5 |
| 2. Xo_Q5H2G4 | 54.1 | | 38 | 38.9 | 33.5 | 28.8 | 24.5 | 25.1 | 10.8 | 100 | 38 | 33.5 | 28.8 | 24.5 |
| 3. Q3IZA2_RHOS4 | 55.3 | 53.1 | | 46.5 | 32 | 28.6 | 23.3 | 25.2 | 10.8 | 38 | 100 | 32 | 28.6 | 23.3 |
| 4. RHILE_O87819 | 53.4 | 57.1 | 61.2 | | 31 | 26.4 | 23.3 | 24.9 | 11.3 | 38.9 | 46.5 | 31 | 26.4 | 23.3 |
| 5. THETH_Q5MCN5 | 47.4 | 48.4 | 45.8. | 44.4 | | 27.1 | 30.3 | 27 | 10.5 | 32.8 | 32 | 100 | 27.1 | 30.3 |
| 3. Arthr_Q4NG94 | 46.7 | 41.2 | 42.3 | 40.1 | 43.4 | | 26.3 | 27.7 | 9.6 | 28.7 | 28.3 | 27.1 | 100 | 26.3 |
| 7. DROME_Q9VM18 | 44.6 | 42 | 43.5 | 44.6 | 46.7 | 45.7 | | 40.6 | 10.9 | 24.5 | 23.3 | 30.3 | 26.4 | 100 |
| 8. DROME_Q9VM19 | 42.9 | 39.9 | 45.1 | 44 | 45.8 | 46.5 | 34.9 | | 10.6 | 25.1 | 25.2. | 27 | 27.7 | 40.6 |
| 9. Sc_P31688\|TPS2 | 17.5 | 16.9 | 17 | 16.4 | 15.2 | 15.1 | 17.7 | 16.6 | | 11.1 | 10.8 | 10.5 | 9.8 | 10.9 |
| 10. Xo_Q5H2G4 | 54.1 | 100 | 53.1 | 57.1 | 47.6 | 41.5 | 42 | 39.9 | 17 | | 38 | 33.5 | 28.8 | 24.5 |
| 11. Q3IZA2\|Q3IZA2_RHOS4 | 55.3 | 53.1 | 100 | 61.2 | 45.8 | 41.9 | 43.5 | 45.1 | 17 | 53.1 | | 32 | 28.6 | 23.3 |
| 12. THETH_Q5MCN5 | 47.4 | 48.4 | 45.8 | 44.4 | 100 | 43.4 | 46.7 | 45.8 | 15.6 | 48.4 | 45.8 | | 27.1 | 30.3 |
| 13. Arthr_Q4NG94 | 46.7 | 41.2 | 42.3 | 40.1 | 43.4 | 100 | 44.2 | 46.5 | 15.1 | 41.2 | 42.3 | 43.4 | | 26.3 |
| 14. DROME_Q9VM18 | 44.6 | 42 | 43.5 | 44.6 | 46.7 | 45.7 | 100 | 64.9 | 17.7 | 42 | 43.5 | 46.7 | 45.7 | |
| 15. SEQ ID NO: 2 | 37.4 | 34 | 37.2 | 34 | 36.9 | 38.5 | 41.7 | 41.2 | 23.7 | 34 | 37.2 | 36.9 | 38.5 | 41.7 |

*Example 4: Identification of domains comprised in Class III TPP polypeptides*

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.
The results of the InterPro scan of the polypeptide sequence as represented by *SEQ ID NO: 14* are presented in Table C.

**Table C:** InterPro scan results of the polypeptide sequence as represented by *SEQ ID NO: 14.*

| Database | Accession number | Accession name | Region of homology to the domain |
|---|---|---|---|
| TIGRFAMs | TIGR01484 | HAD-SF-IIB: HAD-superfamily hydrolase | 119-332 |
| Pfam | PF02358 | Trehalose_PPase | 121-354 |
| TIGRFAMs | TIGR00685 | T6PP: trehalose-phosphatase | 115-365 |

*Example 5: In vitro activity assay for Trehalose Phosphate Phosphatase activity*

The *Arabidopsis* Class III TPP polynucleotide corresponding to the coding region of AtTPPI isoform was cloned into the pGEX-4T-1 (Pharmacia) plasmid, designed to create GST-fusion proteins. The vector was introduced into *E. coli.* The expressed recombinant protein produced in the *E. coli* strain BL21 was purified and the TPP activity determined by HPLC.

*Expression in E. coli*

Supercompetent *E. coli* strain BL21 Star™(DE3) One Shot® (Invitrogen) cells were transformed with the pGEX-GST construct and incubated overnight on Luria Bertani (LB) medium + ampicilline (Amp) plates at 37°C. Colonies were collected and transferred to200 ml LB+Amp and incubated shaking at 37°C for 4h before induction of expression by adding isopropyl-β-thiogalactoside (IPTG, 0.3 mM final concentration). This was followed by an another incubation period of 4h at 30°C. Cells were collected by centrifugation at 4°C (30 min, 3000 rpm) and resuspended in 5 ml ice-cold 1x Phosphate-buffered saline (PBS) buffer (140 mM NaCl; 2.7 mM KCl; 10 mM $Na_2HPO_4$; 1.8 mM $KH_2PO_4$; pH 7.3), aliquoted in 5 chilled screw cap tubes. After centrifugation at 4°C (5 min, 6000 rpm), pellets were frozen in liquid nitrogen, and stored at -80°C.

*Protein purification*

The cells were washed once with 5 ml ice-cold lysis buffer (1x PBS; 0.4% Triton; 2 mM $MgCl_2$, 1 mM EDTA, 2 mM dithiothreitol (DTT); 0.2 mg/ml lysozyme and 1 tablet EDTA protease-inhibitor mix (Roche)/10 ml lysis buffer), incubated for 15 min on ice, and sonicated by three 15-s pulses of sonication (Sartorius Labsonic®P). Lysates were cleared at 4°C by centrifugation at 12,000g. The supernatant fraction was mixed with 200 μl glutathion-sepharose beads (Amersham Biosciences) that had been pre-equilibrated in wash buffer (1x PBS; 0.1% Triton; 2 mM $MgCl_2$, 1 mM EDTA, 1 mM DTT), and incubated for 1h at 4°C in a rollerdrum. Beads were collected by centrifugation at 4°C (1 min, 1800 rpm), washed five times with 1 ml ice-cold wash buffer and stored at -20°C before proceeding with TPP activity measurements.

*TPP activity measurement*

Trehalose-6-phosphate phosphatase (TPP) activity was detected by measuring the produced trehalose (T) after adding Trehalose-6-phosphate (T6P) to the protein samples. Samples were added to 150 μl assay buffer with T6P (2 mM T6P; 2 mM $MgCl_2$; 10 μM Validamycin A and 45 mM Tris-HCl, pH 7.5) or without T6P (2 mM $MgCl_2$; 10 μM Validamycin A and 45 mM Tris-HCl, pH 7.5). After incubation for 1-1.5h at 37°C, the beads were collected by centrifugation (10 min; 1,200g) and the supernatant was injected into the HPLC, together with a T standard dilution. Protein concentrations were measured by the Bradford method.

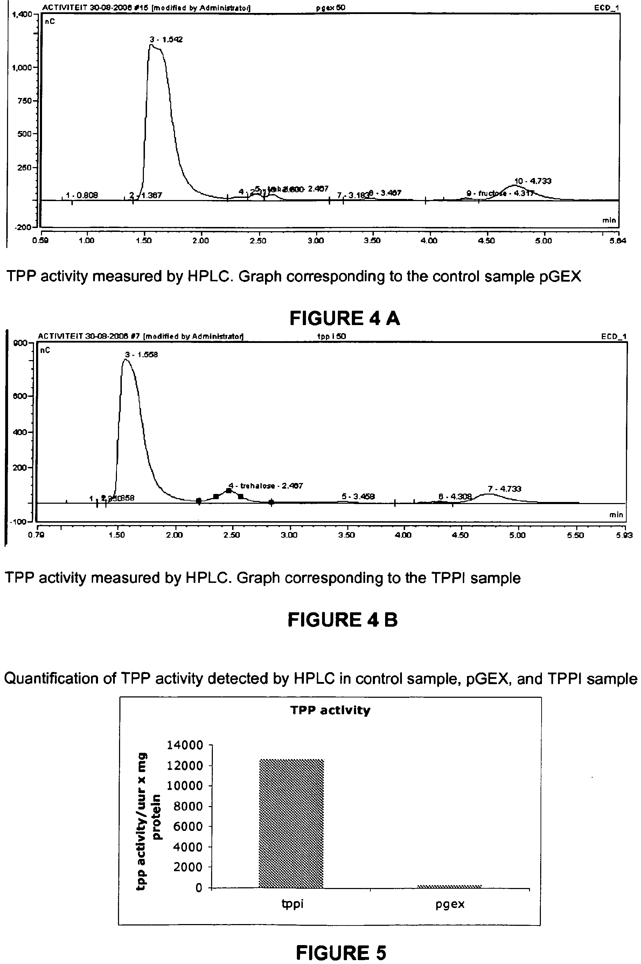

The results as indicated in Figure 4 showed that TPPI has trehalose-6-phosphate phosphatase activity.

***Example* 6: *In vivo activity assay for Trehalose Phosphate Phosphatase***

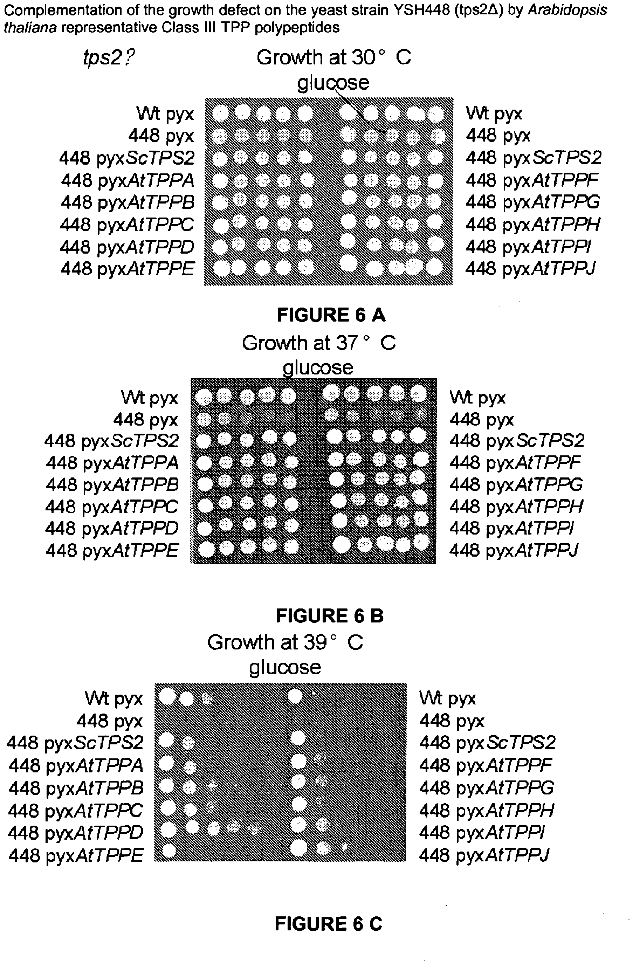

The activity of the enzyme represented by SEQ ID NO: 2 and that of its paralogous proteins having sequences represented by SEQ ID NOs: 10, 12, 16, 18, 20, 22, 24, 26 and 28 was determined by functional complementation of the yeast tps2 mutant according to the protocol described by Vogel et al. 1998 with minor modifications. Briefly, a DNA fragment comprising the coding regions of any of the sequences represented by SEQ ID No:1, 9, 11, 15, 17, 19, 21, 23, 25 and 27 amplified from *Arabidopsis thaliana* cDNA by the polymerase chain reaction using the primers listed in Table D. The DNA fragments subsequently cloned in the yeast expression vector pYX212. The constructs were introduced into the thermosensitive yeast tps2-deficient strain YSH448. The transformed yeast strains were grown at the the permissive temperature of 30 C and the restrictive conditions of 38 C and 39 C in SD-URA glucose medium. As shown in the figure 6 only constructs comprising the coding region of any of SEQ ID No: 2, 10, 12, 16, 18, 20, 22, 24, 26 and 28 were able to restore the growth of the yeast at the restrictive temperatures of 37 C and 39 C. The yeast transformants containing the empty vector pYX212 did not restored the growth of the tps2 deficient strain YSH448. The yeast transformants expressing the wild type yeast TPS2 gene from the vector pYX212 and the empty pYX212 was used as positive and negative controls respectively.

**Table D:** List of primers used in the PCR to amplify Class III Polypeptides from Arabidopsis thaliana. Stop codon was provided in the cloning vector.

| prm name | origin | SEQ ID NO: | Sequence |
|---|---|---|---|
| >PrmAtTPPA-5 | Synthetic | 101 | GGAAGATCTATGGACATGAAATCTGGTCACTC |
| >PrmAtTPPA-3 | Synthetic | 102 | AAGGCCTACCCATTGATCTCTTCCATGTCA |
| >PrmAtTPPB-5 | Synthetic | 103 | GGAATTCATGACTAACCAGAATGTCATCGTT |
| >PrmAtTPPB-3 | Synthetic | 104 | AAGGCCTCTCTTCTCCCACTGTCTTCCTC |
| >PrmAtTPPC-5 | Synthetic | 105 | CGGGATCCATGAAGATTACGGATATTTCCGG |
| >PrmAtTPPC-3 | Synthetic | 106 | AAGGCCTTTCTCCAAGTGTTTGTTTCTTCC |
| >PrmAtTPPD-5 | Synthetic | 107 | CGGGATCCATGACAAACCATAATGCCTTAATC |
| >PrmAtTPPD-3 | Synthetic | 108 | AAGGCCTTCTTCCTCTTAGTGACATTTGTTTC |
| >PrmAtTPPE-5 | Synthetic | 109 | CGGGATCCATGTTCGAAGAAATACTTCATAAATC |
| >PrmAtTPPE-3 | Synthetic | 110 | AAGGCCTTGCCCCACACCTTGACTGTTTC |
| >PrmAtTPPF-5 | Synthetic | 111 | CATGCCATGGATTTAAACTCAAACCACAAATC |
| >PrmAtTPPF-3 | Synthetic | 112 | TCCCCCGGGAAAACCAGTAGAATTCTTCTCCAAC |
| >PrmAtTPPG-5 | Synthetic | 113 | CATGCCATGGATTTGAATATAAACAAGACGAC |
| >PrmAtTPPG-3 | Synthetic | 114 | AAGGCCTAAAACTTGTTTTTGAACTTTCCATCTTC |
| >PrmAtTPPH-5 | Synthetic | 115 | CGGGATCCATGGTTAGATTCATAGAAGAAAACAC |
| >PrmAtTPPH-3 | Synthetic | 116 | AAGGCCTTGCTCCAGATCTCAATTGTTTCC |
| >PrmAtTPPI-5 | Synthetic | 117 | CGGGATCCATGTCAGCTAGTCAAAACATTGTC |
| >PrmAtTPPI-3 | Synthetic | 118 | AAGGCCTCATTCTTGGCTGCATTTGTTTCC |
| >PrmAtTPPJ-5 | Synthetic | 119 | CGGGATCCATGGTGAGCCAAAACGTCGTCG |
| >PrmAtTPPJ-3 | Synthetic | 120 | AAGGCCTTTGCTGCATCTGTTTCCACTCC |

***Example 7: Cloning of nucleic acid sequence* as *represented by SEQ ID NO: 1***

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The *Arabidopsis thaliana Class III TPP* gene was amplified by PCR using as template an *Arabidopsis thaliana* seedling cDNA library (Invitrogen, Paisley, UK). Primers prm05451 (SEQ ID NO: 99, primer; sense, start codon in bold, AttB1 site in italic: 5'-ggggacaagtttgtacaa aaaagcaggcttaaacaa**tg**actaaccagaatgtcatc-3') and prm05452 (SEQ ID NO: 100, primer2; reverse, complementary, AttB2 site in italic: 5'-*ggggaccactttgtacaagaaagctgggt*tgtaattatgtt gcatgtctt-3'), which include the AttB sites for Gateway recombination, were used for PCR amplification. PCR was performed using Hifi Taq DNA polymerase in standard conditions. A PCR fragment of the expected length was amplified and purified also using standard methods. The first step of the Gateway procedure, the BP reaction, was then performed, during which the PCR fragment recombines in vivo with the pDONR201 plasmid to produce, according to the Gateway terminology, an "entry clone", pEC_*Class III TPP*. Plasmid pDONR201 was purchased from Invitrogen, as part of the Gateway® technology.

### *Example 8: Expression vector construction using the nucleic acid sequence as represented by SEQ ID NO: 1*

The entry clone pEC_*Class III TPP* was subsequently used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 98) for constitutive expression was located upstream of this Gateway cassette.

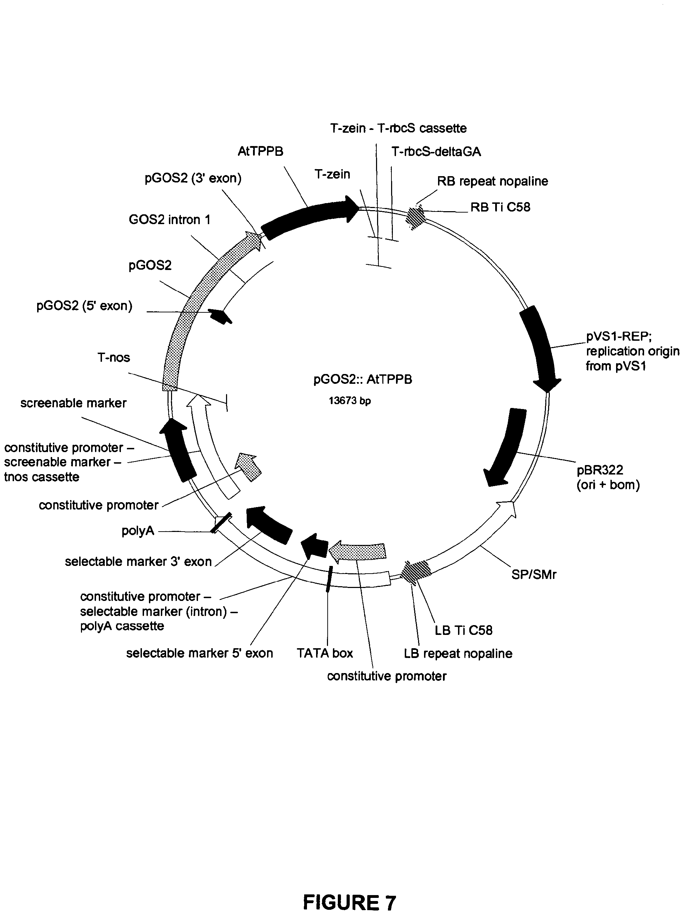

After the LR recombination step, the resulting expression vector pXC_*Class III TPP* (Figure 7) was transformed into *Agrobacterium* strain LBA4044 according to methods well known in the art.

### *Example 9: Plant transformation*

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for cocultivation. *Agrobacterium* was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

*Com transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well.

Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 — 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839—847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

***Example 10: Phenotypic evaluation procedure***

<u>10.1 Evaluation setup</u>

Approximately 35 independent T0 rice transformants were generated. The primary transformants were transferred from a tissue culture chamber to a greenhouse for growing and harvest of T1 seed. Five events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds were grown in potting soil under normal conditions until they approached the heading stage. They were then transferred to a "dry" section where irrigation was withheld. Humidity probes were inserted into randomly chosen pots to monitor the soil water content (SWC). When SWC went below certain thresholds, the plants were automatically watered until a normal level was reached. The plants were then transferred back to normal conditions. The rest of the cultivation (plant maturation, seed harvest) was the same as for plants not grown under abiotic stress conditions. Growth and yield parameters were recorded as detailed for growth under normal conditions.

*Nitrogen use efficiency screen*

Rice plants from T2 seeds were grown in potting soil under normal conditions except for the nutrient solution. The pots were watered from transplantation to maturation with a specific nutrient solution containing reduced N nitrogen (N) content, usually between 7 to 8 times less than normal (control) plants. The rest of the cultivation (plant maturation, seed harvest) was the same as for plants not grown under abiotic stress. Growth and yield parameters are recorded as detailed for growth under normal conditions.

10.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

10.3 Parameters measured

**Biomass-related parameter measurement**

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

Plant early vigour is estimated from the plant (seedling) aboveground area three weeks post-germination. Increase in root biomass is expressed as an increase in total root biomass (measured as maximum biomass of roots observed during the exponential growth phase of the roots); or as an increase in the root/shoot index (measured as the ratio between root mass and shoot mass in the period of active growth of root and shoot).

**Seed-related parameter measurements**

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 11: Results of the phenotypic evaluation of the transgenic plants***

The results of the evaluation of transgenic rice plants expressing the nucleic acid sequence of SEQ ID NO: 1 under the control of a GOS2 promoter are presented in Table E. The percentage difference between the transgenics and the corresponding nullizygotes is also shown, with a P value from the F test below 0.05.

Total seed yield, number of filled seeds, seed fill rate and harvest index were significantly increased in the transgenic plants expressing the nucleic acid sequence of SEQ ID NO: 1, compared to the control plants (in this case, the nullizygotes).

**Table E:** Results of the evaluation of transgenic rice plants expressing the nucleic acid sequence of SEQ ID NO: 1 under the control of a GOS2 promoter

| **Trait** | **% Increase in the transgenic versus control plants** |
|---|---|
| Total seed yield | 15 |
| Number of filled seeds | 17 |
| Number of total seeds | 11 |
| Harvest index | 7 |
| Flowers per panicle | 5 |
| Aboveground Biomass | 6 |

Transgenic rice plants expressing the nucleic acid sequence of SEQ ID NO: 1 under the control of a GOS2 promoter were also subjected to a drought screen. The following parameters were increased compared to corresponding control plants and parameters marked * gave a P value from the F test of below 0.05. *Total seed yield (11% increase compared to control plants), *number of filled seeds (10% increase compared to control plants), *harvest index (11% increase compared to control plants), fill rate (8% increase compared to control plants).

**Table F**: Results of the evaluation of transgenic rice plants subjected to the nitrogen use efficiency screen and expressing the nucleic acid sequence of SEQ ID NO: 1 under the control of a GOS2 promoter.

Plants subjected to the nitrogen use efficiency screen showed an increase in the parameters described in the table below compared to corresponding control plants.

| Parameter | % increase |
|---|---|
| Aboveground biomass | 9.8 |
| Root biomass | 11.4 |
| Total seed yield | 11.2 |
| No. filled seeds | 25.9 |
| No. flowers per panicle | 6.1 |
| Plant Height | 13.4 |
| Total no. seeds | 9.5 |

**Table G**: Results of the evaluation of transgenic rice plants subjected to the nitrogen screen and expressing the nucleic acid sequence of SEQ ID NO: 1 under the control of an HMG promoter (High Mobility Group).

Plants subjected to the nitrogen use efficiency screen showed an increase in the parameters described in the table below compared to corresponding control plants.

| Parameter | % percentage increase |
|---|---|
| Emergence Vigour | 18.2 |
| No. filled seeds | 32.2 |

## Claims

1. A method for increasing yield in plants relative to control plants, comprising modulating expression in a plant of a nucleic acid encoding a Class III Trehalose Phosphate Phosphatase (TPP) polypeptide, wherein said modulated expression is effected by introducing and expressing in a plant a nucleic acid encoding a Class III TPP polypeptide, wherein said increased yield is any one or more of increased seed yield, increased root biomass, increased above-ground biomass.

2. Method according to claim 1, wherein said Class III TPP polypeptide comprises:

   (i) a Trehalose-PPase domain having in increasing order of preference at least 50%, 55%, 60%, 65%, 70%, 75%, 80%, 85%, 90%, 95%, 98% or 100% amino acid sequence identity to SEQ ID NO: 93 or SEQ ID NO: 94, wherein said Trehalose-PPase domain comprises:

      a. a Phosphatase Box A having in increasing order of preference at least 50%, 55%, 60%, 65%, 70%, 75%, 80%, 85%, 90%, 95%, 98% or 100 % sequence identity to SEQ ID NO: 96; and/or
      b. a Phosphatase Box B having in increasing order of preference at least 50%, 55%, 60%, 65%, 70%, 75%, 80%, 85%, 90%, 95%, 98% or 100 % sequence identity to SEQ ID NO: 97; and/or

   (ii) a Serine-rich domain having in increasing order of preference at least 50%, 55%, 60%, 65%, 70%, 75%, 80%, 85%, 90%, 95%, 98% or 100% sequence identity to SEQ ID NO: 95.

3. Method according to claim 1 or 2, wherein said nucleic acid encoding a Class III TPP protein is represented by the nucleic acid of SEQ ID NO: 25 or a portion thereof, or a sequence capable of hybridising with the nucleic acid of SEQ ID NO: 25 or a portion thereof.

4. Method according to any one of claims 1 to 3, wherein said nucleic acid sequence encodes Class III TPP polypeptide given in SEQ ID NO: 26 or derivative thereof.

5. Method according to claim 4, wherein said derivative is an orthologue or paralogue of the Class III TPP polypeptide given in SEQ ID NO: 26.

6. Method according to any one of claims 1 to 5, wherein said nucleic acid is operably linked to a constitutive promoter, preferably to a GOS2 promoter.

7. Method according to any one of claims 1 to 6, wherein said nucleic acid encoding a Class III TPP protein is of plant origin, preferably from a dicotyledonous plant, further preferably from the genus *Arabidopsis,* most preferably from *Arabidopsis thaliana.*

8. Plant or part thereof, including seeds, obtainable by a method according to any one of claims 1 to 6, wherein said plant or part thereof comprises a recombinant nucleic acid encoding a Class III TPP protein as defined in any one of claims 1 to 5.

**9.** Construct comprising:

(a) nucleic acid encoding a Class III TPP polypeptide as defined in any one of claims 1 to 5;
(b) one or more control sequences capable of driving expression of the nucleic acid sequence of (a), preferably said control sequences is at least a constitutive promoter, more preferably a GOS2 promoter; and optionally
(c) a transcription termination sequence.

**10.** Use of a construct according to claim 9 in a method for making plants having increased yield relative to control plants, wherein said increased yield is any one or more of increased seed yield, increased root biomass, increased above-ground biomass, and wherein seed yield is one or more of the following:

(i) Increased number of seeds per plant;
(ii) Increased number of filled seeds per plant;
(iii) Increased seed weight per plant;
(iv) Increased fill rate;
(v) Increased flowers per panicle;
(vi) Increased harvest index.

**11.** Plant, plant part or plant cell transformed with a construct according to claim 9.

**12.** Method for the production of a transgenic plant having increased yield, comprising:

(i) Introducing and expressing in a plant a nucleic acid encoding a Class III TPP protein as defined in any one of claims 1 to 5, and
(ii) cultivating the plant cell under conditions promoting plant growth and development.

**13.** Transgenic plant having increased yield resulting from increased expression of a nucleic acid encoding a Class III TPP protein as defined in any one of claims 1 to 5.

**14.** Transgenic plant cell derived from the transgenic plant according to claim 13, or products derived thereof, wherein said transgenic plant is a crop plant or a monocot or a cereal, such as rice, maize, wheat, barley, millet, rye, sorghum and oats.

**15.** Harvestable parts of a plant according to claim 13 or 14, or products derived from said harvested parts, wherein said harvestable parts are seeds.

**16.** Use of a nucleic acid encoding a Class III TPP protein as defined in any one of claims 1 to 5 in increasing yield in plants relative to control plants, wherein said increased yield is any one or more of increased seed yield, increased root biomass, increased above-ground biomass, and wherein seed yield is one or more of the following:

(i) Increased number of seeds per plant;
(ii) Increased number of filled seeds per plant;
(iii) Increased seed weight per plant;
(iv) Increased fill rate;
(v) Increased flowers per panicle;
(vi) Increased harvest index.

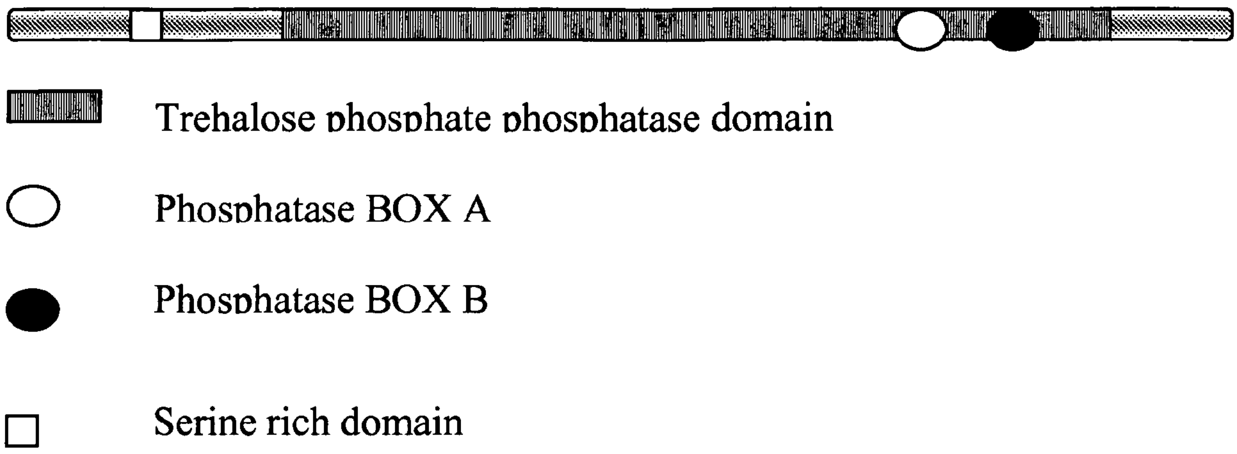


**FIGURE 1**

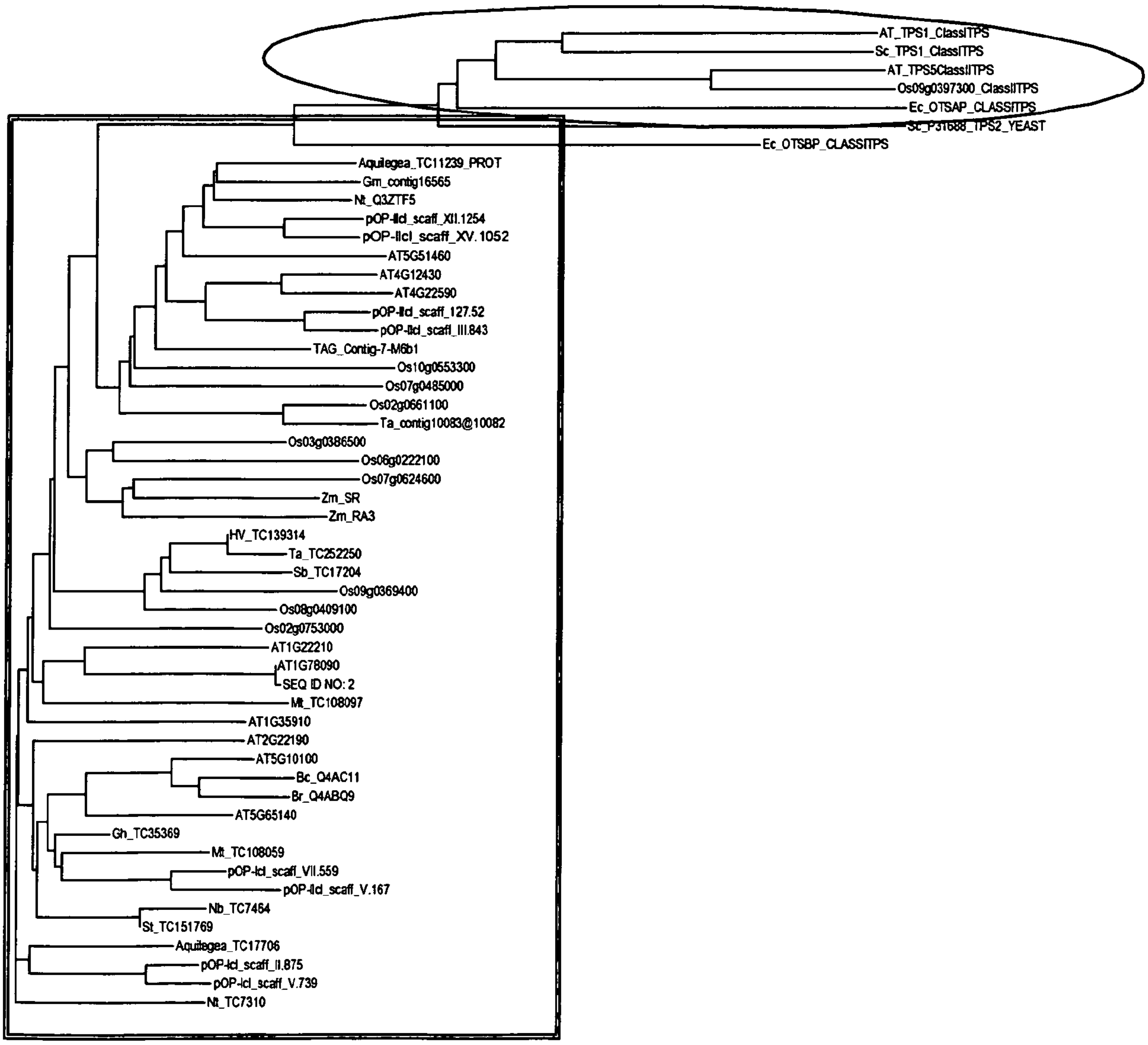


**FIGURE 2 A**

```
                                1                                                50
        Zm_ABD92780     (1) ----MTKRTAFAADDAIIAAAAAVTSQPGRRFTSYPP-------------
        Zm_ABD92779     (1) ----MTKHAAYSSEDVVAAVAAPAP--AGRHFTSFQAL------------
      Sv_ABD92784.1     (1) ----MTKHAGYATDEAVTAVAAPAP--AGLHFSPFPPP------------
             SEQID2     (1) ----MTNQNVIVSDRKPILGLKTITVSVSNSPLFSNSF----------PT
       Os10g0553300     (1) MDLKTS-NSPVIADPLPKLALPSAVMTYT--T-PTSFPST--------GL
       Os09g0369400     (1) ----MAKASVVVPEQVGAAAAAQVGCPCPGTTLFPYPPP----------R
       Os08g0409100     (1) ----MTKQSVVVPEVA---VP----MPPNSAPLLPYPPPR--------AA
       Os07g0624600     (1) ----MTNHAGFAADDAVTAAVPVQAAQGGRHFPPFLAPS-----------
       Os07g0485000     (1) MGSCGNGRSSEYDDPASLEKMEELVLPLK---------------------
       Os03g0386500     (1) --------------------------------------------------
       Os02g0753000     (1) ----MTNQDVVVSEMGIAAGAALPGGPAGPAGGLFACRS---AAASMRQT
       Os02g0661100     (1) MDLSNS--SPVITDPVAISQQLLGGLPSNLMQFSVMPGGY--------S-
          Nt_Q3ZTF5     (1) MDLKSN-TSPVVTDPAP-MTQSRLGTHSA--LMPYSPTG-----------
          Br_Q4ABQ9     (1) ----MVSFVVEKPQRMSNG--VVSETTRLSIIPNNS--------------
          Bc_Q4AC11     (1) ---------MEKPNRMSESQNVVVSEAARSIIPNNSS---------APPG
          AT5G65140     (1) -----MVSQNVVVSDAKTGIITVSTVSNSSVFTPTAQK---PP---TAPG
          AT5G51460     (1) MDMKSGHSSPVMTDSPP-ISNSRLTIRQN--RLPYSSAAA--------TA
          AT5G10100     (1) --------------MSASQNIVVSETTMSSIIPNNNNNNNSSSQKLPPC
          AT4G22590     (1) MDLNINKTTPVLSDPTTPVSKTRLGSSFP--SGRFMMN------------
          AT4G12430     (1) MDLNSNHKSSVLKDPSPSVNQSRLGVSS-----RFMMS------------
          AT2G22190     (1) ---------MVRFIEENITKMLETKAISNSEVLYVGG-----------DD
          AT1G78090     (1) ----MTNQNVIVSDRKPILGLKTITVSVSNSPLFSNSF----------PT
          AT1G35910     (1) ----MTNHNALISDAKGSIGVAVRVPNQS---LFSPGG----------GR
          AT1G22210     (1) --------------------------------------------------
TAG_Contig-7-M6b1     (1) --------------------------------------------------
    Ta_contig10083     (1) --MDLSNGSPVVSDPMSMSQSLMGVLPSNMMPFSVRPGG-----------
    Gm_contig16565     (1) -MDLKSNHTPVVTDAAPVTKSRLGVHSSLLPYSHAGTT------------
          Consensus     (1)           VV D
```

```
                                51                                              100
        Zm_ABD92780    (34) --ARARGGCRLAPAAAARQATDDPGAAGSWPRLWVPR-------------
        Zm_ABD92779    (33) --KGAPLDCKKHAAVDLSASGAAVVGGGPWFESMKASSPR--KAA-----
      Sv_ABD92784.1    (33) --KVAARDCKKVAALHVDRAAPGGGGGGSWFESMKASSPR--KAA-----
             SEQID2    (37) YFNFPRRKLLKLLEAADKNNLVVAPKITSMIDSMRDSSPTRLKSS---SY
       Os10g0553300    (39) YLNTPKK--------KPLPGKIEEVRAAGWSDLMLASSPPRKRQTKDFAN
       Os09g0369400    (37) AGIAVRRKCLQAAQQLELGAG----LRGGWVESMRASSPTHAKAAAALAA
       Os08g0409100    (32) PGVAVRKKYLQAQLDLGAGLP----LINGWVESMRASSPTHAKAAAALAA
       Os07g0624600    (36) --SRLTDCKKAAAHVDLAGAGGVATVPGSWPRHAKPVS------------
       Os07g0485000    (30) ---------------------LMPLHTNGRSYDMRLSSPTATCVINSS--
       Os03g0386500     (1) -------------------------------MTKHGKVVVPVAAA-----
       Os02g0753000    (44) YLDLAAAAVAARSASCTS----------WA-DAMRASSPTRSSRS-----
       Os02g0661100    (40) ------SS---GMNVGVSRLKIEEVLVNGLSDAMKSSSP-RRKLNVAFGE
          Nt_Q3ZTF5    (36) ATFSPTLF---LTIPRKKPGILDDVKSNTWLDAMKSSSPTHSKKNKDSNA
          Br_Q4ABQ9    (31) --SSAQKT---LLKNLEIINGG--QKVNAWVDSMRASSPTHLKSLP----
          Bc_Q4AC11    (33) FISISKKK---LLKNLEIINDG--EKINAWVDSMRASSPTHPKSLP----
          AT5G65140    (40) YISVSKKK---LLKNLEINGADQSQKLNSWVDSMRASSPTHLKSLS----
          AT5G51460    (40) ISQNNNLL---LTVPRKKTGILDDVKSNGWLDAMKSSSPPPTILNKDN--
          AT5G10100    (37) LISISKKK---LLKNIDIINGGG-QKINAWVDSMRASSPTHLKSLP----
          AT4G22590    (37) -S-------------RKKIPKLDDVKSNGWLDAMISSSPPRKRLVKDFNI
          AT4G12430    (34) -Q-------------WKKPAKLDDVKSNGWLDAMISSSPPRKKLVKDFNV
          AT2G22190    (31) GDTSPTTK---VLHDFQINSGGG--LIRSWVDSMRACSPTRPKSFN----
          AT1G78090    (37) YFNFPRRKLLKLLEAADKNNLVVAPKITSMIDSMRDSSPTRLKSS---SY
          AT1G35910    (34) YISIPRK-----KLVQKLEADPSQTKIHTWIEAMRASSPTRTKPGNI-SP
          AT1G22210     (1) -----------------MKITDISGKIETLVDSLRDMSPTRVKSS--FSD
TAG_Contig-7-M6b1     (1) ----------------------------------MKSSSPRNVLKNNATDL
    Ta_contig10083    (38) ------YSGAGGAGVNVSRRKIEEVLVSGLSDAMKSSSPRKKHNVVFGQE
    Gm_contig16565    (38) -----FTHGMLLTIPRKKTGILEDVKSSGWLDAMKSSSPPARKITKDVGH
          Consensus    (51)                          R  WLDSMRASSPT  K
```

FIGURE 2 B

```
                          101                                                150
Zm_ABD92780         (69)  -------HADFDDWMEK---------------------------------
Zm_ABD92779         (74)  -------DAEHGDWMEK---------------------------------
Sv_ABD92784.1       (74)  -------DAEHGDWMEK---------------------------------
SEQID2              (84)  ---D---SVSDNDD-KT-----------------------------SWIVK
Os10g0553300        (81)  DVQADELDLLYRNWV-----------------------------------VN
Os09g0369400        (83)  ---G--VDEEHAAWMVRFRSPIDRCSSRSWRARADTDMNRAFPFVFVQAK
Os08g0409100        (78)  ---AGAVDDERAAWMVR---------------------------------
Os07g0624600        (72)  -------GAELDDWMEK---------------------------------
Os07g0485000        (57)  ---SGSFDPIYRAWT-----------------------------------KK
Os03g0386500        (15)  -----AADAEHDEWME----------------------------------K
Os02g0753000        (78)  -----ASDVDEFTAWVR---------------------------------K
Os02g0661100        (80)  DNSSEEEDPAYSAWM-----------------------------------AK
Nt_Q3ZTF5           (83)  ELTANESDLAYRIWM-----------------------------------LK
Br_Q4ABQ9           (70)  -------SSVSSEKHLS-----------------------------SWIMQ
Bc_Q4AC11           (74)  -------SSISSEQQLS-----------------------------SWIMQ
AT5G65140           (83)  -------SFSS-EEEHN-----------------------------SWIKK
AT5G51460           (85)  -LSNDATDMTYREWMQ----------------------------------LK
AT5G10100           (79)  -------SSISTQQQLN-----------------------------SWIMQ
AT4G22590           (73)  -EIAPEDDFSQRAWM-----------------------------------LK
AT4G12430           (70)  -EVAPEDDFAQRAWM-----------------------------------VK
AT2G22190           (72)  -------SQS------------------------------------CWIKE
AT1G78090           (84)  ---D---SVSDNDD-KT-----------------------------SWIVK
AT1G35910           (78)  ---L---PESDEEDEYS-----------------------------SWMAQ
AT1G22210           (32)  ---E---HVSENDDERS-----------------------------SWIAL
TAG_Contig-7-M6b1   (18)  -----SNDVAYRNWM-----------------------------------IK
Ta_contig10083      (82)  --NLPEEDPAYSAWM-----------------------------------AK
Gm_contig16565      (83)  GFASSDSDTAGAYWSWL---------------------------------LK
Consensus          (101)         D      WM                                  K

                          151                                                200
Zm_ABD92780         (79)  HPSALAAFESVLAAAKGKKIVMFLDYDGTLSPIVRDPDSAVMSEEMRDA-
Zm_ABD92779         (84)  HPSALAQFEPLLAAAKGKQIVMFLDYDGTLSPIVEDPDRAVMSEEMREA-
Sv_ABD92784.1       (84)  HPSALTWFEPALAAAKGKQIVMFLDYDGTLSPIVEDPDRAVMSEEMRDA-
SEQID2              (99)  FPSALNMFDEIVNAAKGKQIVVFLDYDGTLSPIVEDPDKAFITHEMREV-
Os10g0553300        (98)  HPSALTSFEDIVNLARGKRLALFLDYDGTLSPIVDNPENAVMSDEMRSA-
Os09g0369400       (128)  HPSALGEFEKVVAASKGKQIVMFLDYDGTLSPIVDDPDAAFMSRTVSLSS
Os08g0409100        (92)  HPSALSKFEQIVAASKGKKIVMFLDYDGTLSPIVDDPDSAFMSDTMRR--
Os07g0624600        (82)  HPSALAWFESVAAAAKGKEIVVFLDYDGTLSPIVADPDRAFMSDEMREA-
Os07g0485000        (71)  YPSALNAFDHIVAYGKGKKIALFLDYDGTLSPIVDEPDNAIMSDQMREV-
Os03g0386500        (27)  HPSALGKFEALAAAAKGKRIVVFLDYDGTLSPIVEDPDRAVMTDEMRDA-
Os02g0753000        (91)  HPSALSKFEEIAAKSRGKKIVMFMDYDGTLSPIVADPDTAYMSDAMRAA-
Os02g0661100        (97)  CPSALASFKQIVASAQGKKIAVFLDYDGTLSPIVDDPDKAVMSPVMRAA-
Nt_Q3ZTF5          (100)  YPSALSSFEQITNYAKGKRIALFLDYDGTLSPIVDDPDRAFMSGAMRAT-
Br_Q4ABQ9           (85)  HPSALEMFEKITQASGGKQIVMFLDYDGTLSPIVDDPDKAFMSSKMRRT-
Bc_Q4AC11           (89)  HPSALEMFEKITEASGGKQIVIFLDYDGTLSPIVDDPDRAFMSSKMRRT-
AT5G65140           (97)  HPSALNMFERIIEEARGKQIVMFLDYDGTLSPIVDDPDRAFMTSKMRRT-
AT5G51460          (102)  YPSALTSFEKIMSFAKGKRIALFLDYDGTLSPIVEEPDCAYMSSAMRSA-
AT5G10100           (94)  HPSALEKFEQIMEASRGKQIVMFLDYDGTLSPIVDDPDKAFMSSKMRRT-
AT4G22590           (89)  YPSAITSFAHIAAQAKNKKIAVFLDYDGTLSPIVDDPDRAIMSDAMRAA-
AT4G12430           (86)  YPSAISSFAHIAAQAKKKKIAVFLDYDGTLSPIVDDPDRAIMSDAMRSA-
AT2G22190           (80)  HPSALNMFEEIIHKSEGKQIVMFLDYDGTLSPIVDDPDRAFMSKKMRNT-
AT1G78090           (99)  FPSALNMFDEIVNAAKGKQIVMFLDYDGTLSPIVEDPDKAFITHEMREV-
AT1G35910           (94)  HPSALTMFEEIAEASKGKQIVMFLDYDGTLSPIVENPDRAYMSEEMREA-
AT1G22210           (48)  HPSALDMFEQIMRDAEGKQIIMFLDYDGTLSLITEDHDRAYITDEMREV-
TAG_Contig-7-M6b1   (30)  YPSALASIEQIANNAKGKRIALFLDYDGTLSPIVDNPDHAFMSNAMRAA-
Ta_contig10083      (97)  CPSALASFKQIVASAQGKKIAVFLDYDGTLSPIVDDPEKAVMSPVMRAA-
Gm_contig16565     (102)  YPSALASFDQIMNYAKGKRIALFMDYDGTLSPIVDNPDCAFMSDNMRAA-
Consensus          (151)  HPSAL  FE IVAAAKGKKIVMFLDYDGTLSPIVDDPDRAFMSD MR A
```

FIGURE 2 B (continued)

```
                              201                                                250
         Zm_ABD92780   (128)  --------------------------VRGVAEHFPTAIVSGRCRDKVYNF
         Zm_ABD92779   (133)  --------------------------VRRVAEHFPTAIVSGRCRDKVLNF
       Sv_ABD92784.1   (133)  --------------------------VRRVAEQFPTAIVSGRCRDKVYNF
              SEQID2   (148)  --------------------------VKDVASNFPTAIVTGRSIEKVRSF
         Os10g0553300  (147)  --------------------------VKHVASLFPTAIISGRSRDKVYDF
         Os09g0369400  (178)  PPLSPTLLLHSSSSHTSLLPHQMRMAVRSVAKHFPTAIVSGRCRDKVYEF
         Os08g0409100  (140)  -------------------------AVRSVAKHFPTAIVSGRCRDKVYEF
         Os07g0624600  (131)  --------------------------VRGVAKHFPTAIVSGRCIDKVYDF
         Os07g0485000  (120)  --------------------------VRNAALHLPTAIISGRSRDKVYDF
         Os03g0386500   (76)  --------------------------VRGVAARFPTAIVSGRCRDKVLSF
         Os02g0753000  (140)  --------------------------VREVAKTFPTAIVSGRCRDKVRNF
         Os02g0661100  (146)  --------------------------VRNVAKYFPTAIVSGRSRNKVYEF
           Nt_Q3ZTF5   (149)  --------------------------VRNVAKYFPTAIISGRSRDKVYDF
           Br_Q4ABQ9   (134)  --------------------------VKKLAKCFPTAIVTGRCIDKVYNF
           Bc_Q4AC11   (138)  --------------------------VKKLAKCFPTAIVTGRCLDKVYNF
           AT5G65140   (146)  --------------------------VKKMAKCFPTSIVTGRCIDKVYSF
           AT5G51460   (151)  --------------------------VQNVAKYFPTAIISGRSRDKVYEF
           AT5G10100   (143)  --------------------------VKKLAKCFPTAIVTGRCIDKVYNF
           AT4G22590   (138)  --------------------------VKDVAKYFPTAIISGRSRDKVYQL
           AT4G12430   (135)  --------------------------VKDVASYFPTAIISGRSRDKVYQL
           AT2G22190   (129)  --------------------------VRKLAKCFPTAIVSGRCREKVSSF
           AT1G78090   (148)  --------------------------VKDVASNFPTAIVTGRSIEKVRSF
           AT1G35910   (143)  --------------------------VKGVARYFPTAIVTGRCRDKVRRF
           AT1G22210    (97)  --------------------------VKEVATYFKTAIISGRSTDKVQSF
  TAG_Contig-7-M6b1    (79)  --------------------------VRNVAKYIPTAIISGRSCEKVHKF
     Ta_contig10083   (146)  --------------------------VRNVAKYFPAAIVSGRSRKKVLEF
     Gm_contig16565   (151)  --------------------------VKKVAEYFPTAIISGRSRDKVYQF
           Consensus   (201)                            VR VAKYFPTAIVSGRCRDKVY F

                              251                                                300
         Zm_ABD92780   (152)  VKLAELYYAGSHGMDIKGPTAQS-----------KHTKAKAG-AVLCQPA
         Zm_ABD92779   (157)  VKLTELYYAGSHGMDIQGPAACRQP---------NHVQQAEAAAVHYQAA
       Sv_ABD92784.1   (157)  VKLTELYYAGSHGMDIEGPAKQSN----------KHVQANAEEAVHYQAG
              SEQID2   (172)  VQVNEIYYAGSHGMDIEGPTNEN-------------SNGQSNERVLFQPA
         Os10g0553300  (171)  VKLTELYYAGSHGMDIMGPVRKSDSSGQHVECIRSTDSE-GKEVNLFQPA
         Os09g0369400  (228)  VKLAELYYAGSHGMDIKGPASRHAAAKSPP----------HNKGVLFQPA
         Os08g0409100  (165)  VKLAELYYAGSHGMDIKGPAKASRHNKAK-----------AKGVLFQPA
         Os07g0624600  (155)  VKLEELYYAGSHGMDIRGPTAAASEYN-------HNMKAKQGEAVTFQPA
         Os07g0485000  (144)  VKLTELYYAGSHGMDIMGPVGEHDSVTNHRSSINSNRKQ-GKGVKLFQAG
         Os03g0386500  (100)  VGLEELYYAGSHGMDIQGPTNAAA-SK---------GGEEEESVLCQPA
         Os02g0753000  (164)  VGLSDLYYAGSHGMDIKGPS------------------SNPESALCQPA
         Os02g0661100  (170)  VKLKELYYAGSHGMDIMAPSANHEHSAEKS-----------KQANLFQPA
           Nt_Q3ZTF5   (173)  VGLAELYYAGSHGMDIMGPVR--SVSDDYS-CIRSTNKQ-GKEVNLFQPA
           Br_Q4ABQ9   (158)  VKLAELYYAGSHGMDIKGPAKGFS------------RHKRVKQSLLYQPA
           Bc_Q4AC11   (162)  VKLAELYYAGSHGMDIKGPAKGFS------------RHKRVKQSLLYQPA
           AT5G65140   (170)  VKLAELYYAGSHGMDIKGPTKGFS------------RYNKDKPSVLYQPA
           AT5G51460   (175)  VNLSELYYAGSHGMDIMSPAGE-SLNHEHSRTVSVYE-Q-GKEVNLFQPA
           AT5G10100   (167)  VKLAELYYAGSHGMDIKGPAKGFS------------RHKRVKQSLLYQPA
           AT4G22590   (162)  VGLTELYYAGSHGMDIMTPVNPN-GSPEDPNCIKTTDQQ-GEEVNLFQPA
           AT4G12430   (159)  VGLTELYYAGSHGMDIMTSS-------DGPNCFKSTDQQ-GKEVNLFQPA
           AT2G22190   (153)  VKLTELYYAGSHGMDIKGPEQGS-------------KYKKENQSLLCQPA
           AT1G78090   (172)  VQVNEIYYAGSHGMDIEGPTNEN-------------SNGQSNERVLFQPA
           AT1G35910   (167)  VKLPGLYYAGSHGMDIKGPSKRN-------------KHNKNNKGVLFQAA
           AT1G22210   (121)  VKLTGIHYAGSHGMDIKGPTNTD-------------QSNQ--EEVMFQPA
  TAG_Contig-7-M6b1   (103)  VGLKELYYAGSHGMDIMGPVQPPT-----------DHRIEAISGNLYQPA
     Ta_contig10083   (170)  VKLKELCYAGSHGMDIMTSSSAHY----------EHNTEKGKEANLFQPA
     Gm_contig16565   (175)  VGLTELYYAGSHGMDIIGPVRQSVSDNHPN--CIRSTDKQGKEVNLFQPA
           Consensus   (251)  VKL ELYYAGSHGMDI GP                           E VLFQPA
```

**FIGURE 2 B (continued)**

```
                             301                                               350
Zm_ABD92780          (190)  RAFLPVIEEVYRALTASTA-PIPGATVENNKFCLSVHFRCVQEKWRALE
Zm_ABD92779          (198)  SEFLPVIEEVTRTLTAKME-SIAGARVEHNKYCLSVHFRCVREEEWNAVN
Sv_ABD92784.1        (197)  SEFLPIIEEVYRTLTAKME-SIAGAKVEHNKYCLSVHFRCVQEEEWKAVE
SEQID2               (209)  REFLPMIEKVVNILEEKTK-WIPGAMVENNKFCLSVHFRRVDEKRWPALA
Os10g0553300         (220)  SEFLPMISEVYKKLSESIK-DIDGARMEDNKFCVSVHYRNVAPHDYGEVH
Os09g0369400         (268)  SEFLPMIEQVHQRLEQATS-SIPGAKVENNKFCVSVHFRCVDEKSWGALA
Os08g0409100         (203)  SEFLPMIEQVHDSLIERTK-CIPGAKVENNKFCVSVHFRCVDEKSWSTLA
Os07g0624600         (198)  ADFLPVIEEVYHVLKERMA-SIRGSLVENNKFCLSVHYRCVDEAEWGVLD
Os07g0485000         (193)  TEFLPMINEVFRLLIDKTK-AIDGVKIENNKFCVSVHYRNVEEKNWQLVA
Os03g0386500         (140)  REFLPMIGEAYAALVEKVEGVIPGAKVENNKFCLSVHFRRVDERRWGAVA
Os02g0753000         (195)  SEFLPMIDEVYKTLVEKTK-STPGAKVENNKFCLSVHFRCVDEKRWNALG
Os02g0661100         (209)  HGFLPMIDEVTKSLLQVVS-GIEGATVENNKFCVSVHYRNVAEKDWKLVA
Nt_Q3ZTF5            (219)  GEFLPMIDEVFKSLIELTK-DITGAKVENNKFCVSVHYRNVDEKSWSAIG
Br_Q4ABQ9            (196)  SDYLPMIDEVYKQLLEKTK-STPGVIVENHKFTASVHFRCVDEKKWSELV
Bc_Q4AC11            (200)  SDYLPMIDEVYRQLLEKTK-STPGVIVENNKFCASVHFRCVDEKKWSELV
AT5G65140            (208)  GDFLPMIDEVYKQLVEKTK-STPGAKVENNKFCLSVHFRCVDEKKWSELA
AT5G51460            (222)  SEFLPMIDKVLCSLIESTK-DIKGVKVEDNKFCISVHYRNVEEKNWTLVA
AT5G10100            (205)  NDYLPMIDEVYRQLLEKTK-STPGAKVENHKFCASVHFRCVDEKKWSELV
AT4G22590            (210)  KEFIPVIEEVYNNLVEITK-CIKGAKVENHKFCTSVHYRNVDEKDWPLVA
AT4G12430            (201)  REFIPVIDEVFRTLVEKMK-DIKGAKVENHKFCASVHYRNVDEKDWPIIA
AT2G22190            (190)  TEFLPVINEVYKKLVENTQ-SIPGAKVENNKFCASVHFRCVEENKWSDLA
AT1G78090            (209)  REFLPMIEKVVNILEEKTK-WIPGAMVENNKFCLSVHFRRVDEKRWPALA
AT1G35910            (204)  NEFLPMIDKVSKCLVEKMK-DIEGANVENNKFCVSVHYRCVDQKDWGLVA
AT1G22210            (156)  SDYLPMIDEVVNVLKEKTK-SIPGATVEHNKFCLTVHFRRVDETGWAALA
TAG_Contig-7-M6b1    (142)  SEFLPMINEVFVSLVEITK-DIEGAKVEDNKFCVSVHYRNVDEKNWTMVA
Ta_contig10083       (210)  RDFLPMIDEVSKALLEVTS-GIEGASVEDNKFCVSVHYRNVDEKDWELVA
Gm_contig16565       (223)  AEFLPMINEVLNSLEECTK-DIKGAKVENNKFCVSVHYRNVDEKYWNWVG
Consensus            (301)   EFLPMIDEVYK LVEKTK  I GAKVENNKFCLSVHFR VDEK W  LA

                             351                                               400
Zm_ABD92780          (239)  EQVRSVLKEYPDLRLTKGRKVLEIRPSIKWDKGNALQFLLESLG------
Zm_ABD92779          (247)  EEVRSVLREYPNLKLTHGRKVLEIRPSIKWDKGKALEFLLKSLG------
Sv_ABD92784.1        (246)  EEVRSVLKEYPDLKLTHGRKVLEIRPSIKWDKGKALEFLLKSLG------
SEQID2               (258)  EVVKSVLIDYPKLKLTQGRKVLEIRPTIKWDKGQALNFLLKSLG------
Os10g0553300         (269)  QRVTAVLKNYPCLRLTHGRKVLEVRPVIDWNKGKAVEFLLESLG------
Os09g0369400         (317)  ETVRRVVREFPRLRLSQGRMVFEVRPTIKWDKGKALEFLLDSLG------
Os08g0409100         (252)  DIVKAELKDYPKLKLTQGRMVFEIRPTIKWDKGKALEFLLESLG------
Os07g0624600         (247)  GKVRAVIEGYPDLRLSKGRKVLEIRPVIDWDKGSALQFLLKSLG------
Os07g0485000         (242)  QCTNDVLKVYPRLRLTHGRKVLEIRPVIDWNKGKAVEFLLDSLD------
Os03g0386500         (190)  DQVRAVLRGYPRLRLTQGRKVLEVRPAIKWDKGEALRFLLSALGFSAAGD
Os02g0753000         (244)  EQVKAVIKEYPKLKLTQGRKVLEIRPSIEWDKGKALEFLLESLG------
Os02g0661100         (258)  RLVNEVLEAFPRLKVTNGRMVLEVRPVIDWDKGKAVEFLLQSLG------
Nt_Q3ZTF5            (268)  ESVDEILKHYPRLRLTHGRKVLEVRPVLNWDKGKAVEFLLESLG------
Br_Q4ABQ9            (245)  LQVRSVLEKYPTLKLSQGRKVFEIRPMIDWDKGKALEFLLESLG------
Bc_Q4AC11            (249)  LQVRSVLNEYPRLKLNQGRKVFEIRPMIEWDKGKALEFLLESLG------
AT5G65140            (257)  SKVRSVVKNYPTLKLSQGRKVFEIRPIIKWNKGKALEFLLESLG------
AT5G51460            (271)  QCVDDVIRTYPKLRLTHGRKVLEIRPVIDWDKGKAVTFLLESLG------
AT5G10100            (254)  LQVRSVLKKFPTLQLTQGRKVFEIRPMIEWDKGKALEFLLESLG------
AT4G22590            (259)  QRVHDHLKRYPRLRITHGRKVLEVRPVIEWNKGKAVEFLLESLG------
AT4G12430            (250)  QRVHDHLKQYPRLRLTHGRKVLEVRPVIDWNKGRAVEFLLESLG------
AT2G22190            (239)  HQVRSVLKNYPKLMLTQGRKVLEIRPIIKWDKGKALEFLLESLG------
AT1G78090            (258)  EVVKSVLIDYPKLKLTQGRKVLEIRPTIKWDKGQALNFLLKSLG------
AT1G35910            (253)  EHVTSILSEYPKLRLTQGRKVLEIRPTIKWDKGKALEFLLESLG------
AT1G22210            (205)  EQVRLVLIDYPKLRLTQGRKVLEIRPSIKWDKGKALEFLLNSLG------
TAG_Contig-7-M6b1    (191)  QCVEDTLKNYPRLRLTHGRMVLEIRPVLKWDKGKAVEFLLESLG------
Ta_contig10083       (259)  RLVNEVLEDFPPLKVTNGRMVLEVRPVIDWDKGKAVEFLLQSLG------
Gm_contig16565       (272)  QHVHDVLKGYPRLRLTHGRKVLEIRPVINWDKGKAVTFLLESLG------
Consensus            (351)    VRSVLK YPRLRLTQGRKVLEIRPVI WDKGKALEFLLESLG
```

**FIGURE 2 B (continued)**

```
                                 401                                              450
       Zm_ABD92780     (283)   ---FAGSNSVFPIYIGDDSTDEDAFKVLRNL-GQGIGILVSKIPKETRAS
       Zm_ABD92779     (291)   ---YAGRNDVFPIYIGDDRTDEDAFKVLRNM-GQGIGILVSKLPKETAAS
     Sv_ABD92784.1     (290)   ---YAGRSDVFPIYIGDDRTDEDAFKVLRGM-GQGIGILVSKFPKETAAS
            SEQID2     (302)   ---FENSDDVVPVYIGDDRTDEDAFKVLRER-GQGFGIFVSKVPKDTNAS
      Os10g0553300     (313)   ---LCGKEDVLPIYVGDDRTDEDAFKVLKAN-SIGFGILVSSVPKDTDAF
      Os09g0369400     (361)   ---FADCSDVLPVYIGDDRTDEDAFKVLRRR-GQGVGILVSKHPKETSAS
      Os08g0409100     (296)   ---FADCTNVLPVYIGDDRTDEDAFKVLRKR-GQGIGILVSKYPKDTNAS
      Os07g0624600     (291)   ---YEGRNNVFPIYIGDDRTDEDAFKVLRNM-GQGIGILVTKVPKETAAS
      Os07g0485000     (286)   ---LASCKNVLPIYIGDDCTDEDAFKVLRED-KRGFGILVSSVPKDSHAL
      Os03g0386500     (240)   VEDDGDDDDAFPIYIGDDRTDEDAFRVLRAR-GHGAGILVSRFPKDTCAS
      Os02g0753000     (288)   ---FANCGDVMPVYIGDDRTDEDAFKVLRKR-GQGLGILVSKCPKDTNAS
      Os02g0661100     (302)   ---LNDSENVIPIYIGDDRTDEDAFKVLRQR-NCGYGILVSQVPKETEAF
         Nt_Q3ZTF5     (312)   ---LKNCDDVLPIYVGDDRTDEDAFKVLRRG-NKGYGILVSSAPKESSAF
         Br_Q4ABQ9     (289)   ---FGNSNNVFPVYIGDDRTDEDAFKMLRVR-GEGFGILVSKFPKDTDAS
         Bc_Q4AC11     (293)   ---FGNSNNVFPVYIGDDRTDEDAFKLLRER-GEGCGVLVSKFPKDTDAS
         AT5G65140     (301)   ---FENCNDVFPIYIGDDRTDEDAFKLLRGR-GQGFGILVSKFPKDTSAS
         AT5G51460     (315)   ---LNNCEDVLPIYVGDDRTDEDAFKVLRGGPNHGYGILVSAVPKDSNAF
         AT5G10100     (298)   ---FGNTNNVFPVYIGDDRTDEDAFKMLRDR-GEGFGILVSKFPKDTDAS
         AT4G22590     (303)   ---LSNNDEFLPIFIGDDRTDEDAFKVLRRG-NRGFGILVSSVPKESNAF
         AT4G12430     (294)   ---LSNKDDDLPIYIGDDTTDEDAFKVLRDG-NRGFGILVSSIPKESNAF
         AT2G22190     (283)   ---YDNCTDVFPIYIGDDRTDEDAFKILRDR-KQGLGILVSKYAKETNAS
         AT1G78090     (302)   ---FENSDDVVPVYIGDDRTDEDAFKVLRER-GQGFGILVSKVPKDTNAS
         AT1G35910     (297)   ---FANSNDVLPIYIGDDRTDEDAFKVLRNK-GQGFGILVSKIPKETSAS
         AT1G22210     (249)   ---IAESKDVLPVYIGDDRTDEDAFKVLCER-GQGFGIIVSKTIKETYAS
 TAG_Contig-7-M6b1     (235)   ---LSNCDDVLPIYVGDDRTDEDAFKFLRRG-SLGYGILVTPAPKESSAY
    Ta_contig10083     (303)   ---LSDSENVIPIYIGDDRTDEDAFKVLRER-NCGYGILVSQAPKETEAF
    Gm_contig16565     (316)   ---LNNCDDVLPIYIGDDRTDEDAFKVLRRG-NKGYGILVSSAPKESNAI
         Consensus     (401)      F N  DVLPIYIGDDRTDEDAFKVLRER G GFGILVSK PKET AS

                                 451                                              500
       Zm_ABD92780     (329)   YSLREPSEVEEFLRKLASWSKESRQRD-----------------------
       Zm_ABD92779     (337)   YSLSDPREVKEFLRKLANKKGARQP-------------------------
     Sv_ABD92784.1     (336)   YXLRD---------------------------------------------
            SEQID2     (348)   YSLQDPSQVNK----FLERLVEWKRKTVGEE-------------------
      Os10g0553300     (359)   YSVRDPAEVMEFLKKLASWKEEST--------------------------
      Os09g0369400     (407)   FSLQEPAELKKSSVHYPLPAQRSNRLNRILVNWWGEGLKDKLCALEPSHE
      Os08g0409100     (342)   YSLQEPAEV----MEFLLRLVEWERLSRARPKW-----------------
      Os07g0624600     (337)   YTLREPSEVKEFLRKLVKIKINGDKGLIGK--------------------
      Os07g0485000     (332)   YSLIDPSEVMEFLKRLVMWKNEEASHNK----------------------
      Os03g0386500     (289)   FSLRDPGEVKDFLRKLVTCAAA----------------------------
      Os02g0753000     (334)   YSLQDPTEVMEFLLRLVEWKRKSSSSSLMIRPRV----------------
      Os02g0661100     (348)   YSLRDPSEVMEFLNFLVRWKKHSV--------------------------
         Nt_Q3ZTF5     (358)   YSLRDPSEVMEFLKCLVSWKKSSGFSY-----------------------
         Br_Q4ABQ9     (335)   YSLQDPSEASP----HTRILYLSVPKYKMFSRADL---------------
         Bc_Q4AC11     (339)   YYLQDPSEASS----EFLATIGGVETNAAKSVKRMYV-------------
         AT5G65140     (347)   YSLQDPPEVMN----FLGRLVEWKQMQQ----------------------
         AT5G51460     (362)   YSLRDPSEVMEFLKSLVTWKRSMG--------------------------
         AT5G10100     (344)   YSLQDPSEVMS----FLRRLVEWKQMQPRM--------------------
         AT4G22590     (349)   YSLRDPSEVKKFLKTLVKWGKMESSKTSF---------------------
         AT4G12430     (340)   YSLRDPSEVKKFLKTLVKWAKLEKNSTGF---------------------
         AT2G22190     (329)   YSLQEPDEVMV----FLERLVEWKQSRCGA--------------------
         AT1G78090     (348)   YSLQDPSQVNK----FLERLVEWKRKTVGEE-------------------
         AT1G35910     (343)   YSLQEPSEVGE----FLQRLVEWKQMSLRGR-------------------
         AT1G22210     (295)   YSLQDPSQVKE----FLERLVKWKKQTLGE--------------------
 TAG_Contig-7-M6b1     (281)   YSLRDPSEVMEFFNLLVMHKKSVP--------------------------
    Ta_contig10083     (349)   YSLRDPSEVMEFLNSLVRWKKHSL--------------------------
    Gm_contig16565     (362)   YSLRDPSEVMEFLKSLVLWKSSTLKSLI----------------------
         Consensus     (451)   YSL DPSEV EFL  LV
```

**FIGURE 2 B (continued)**

```
                            501
       Zm_ABD92780   (356)  --------
       Zm_ABD92779   (362)  --------
     Sv_ABD92784.1   (341)  --------
            SEQID2   (375)  --------
      Os10g0553300   (383)  --------
      Os09g0369400   (457)  REGCMAPH
      Os08g0409100   (371)  --------
      Os07g0624600   (367)  --------
      Os07g0485000   (360)  --------
      Os03g0386500   (311)  --------
      Os02g0753000   (368)  --------
      Os02g0661100   (372)  --------
         Nt_Q3ZTF5   (385)  --------
         Br_Q4ABQ9   (366)  --------
         Bc_Q4AC11   (372)  --------
         AT5G65140   (371)  --------
         AT5G51460   (386)  --------
         AT5G10100   (370)  --------
         AT4G22590   (378)  --------
         AT4G12430   (369)  --------
         AT2G22190   (355)  --------
         AT1G78090   (375)  --------
         AT1G35910   (370)  --------
         AT1G22210   (321)  --------
TAG_Contig-7-M6b1    (305)  --------
    Ta_contig10083   (373)  --------
    Gm_contig16565   (390)  --------
         Consensus   (501)
```

**FIGURE 2 B (continued)**

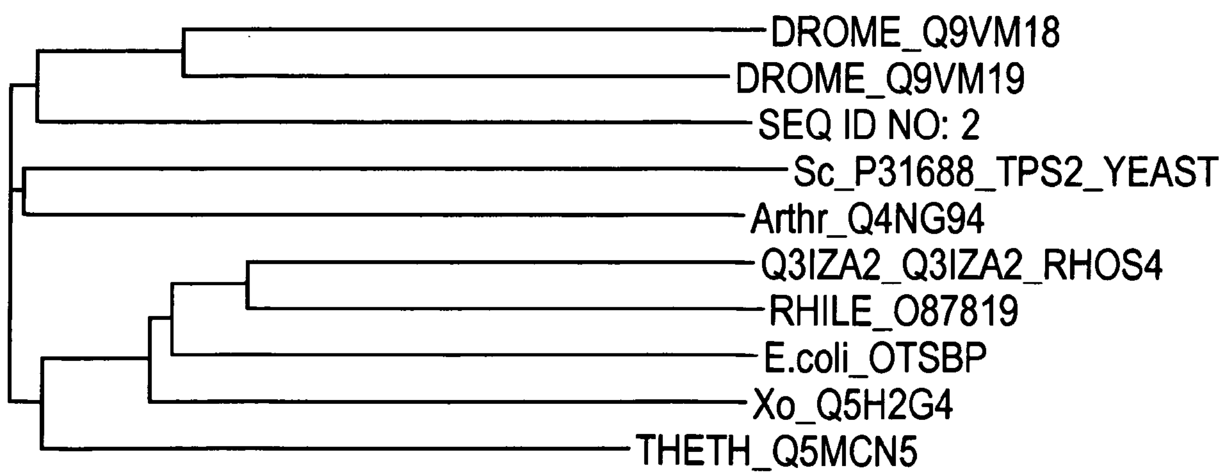


**FIGURE 2 C**

```
                                1                                                50
         DROME_Q9VM18    (1)   --------------------------------------------------
         DROME_Q9VM19    (1)   --------------------------------------------------
  Sc_P31688_TPS2_YEAST   (1)   MTTTAQDNSPKKRQRIINCVTQLPYKIQLGESNDDWKISATTGNSALFSS
         Arthr_Q4NG94    (1)   --------------------------------------------------
   Q3IZA2_Q3IZA2_RHOS4   (1)   --------------------------------------------------
         RHILE_O87819    (1)   --------------------------------------------------
         THETH_Q5MCN5    (1)   --------------------------------------------------
            Xo_Q5H2G4    (1)   --------------------------------------------------
         E.coli_OTSBP    (1)   --------------------------------------------------
               seqID2    (1)   --------------------------------------------------
            Consensus    (1)

                                51                                              100
         DROME_Q9VM18    (1)   --------------------------------------------------
         DROME_Q9VM19    (1)   --------------------------------------------------
  Sc_P31688_TPS2_YEAST  (51)   LEYLQFDSTEYEQHVVGWTGEITRTERNLFTREAKEKPQDLDDDPLYLTK
         Arthr_Q4NG94    (1)   --------------------------------------------------
   Q3IZA2_Q3IZA2_RHOS4   (1)   --------------------------------------------------
         RHILE_O87819    (1)   --------------------------------------------------
         THETH_Q5MCN5    (1)   --------------------------------------------------
            Xo_Q5H2G4    (1)   --------------------------------------------------
         E.coli_OTSBP    (1)   --------------------------------------------------
               seqID2    (1)   --------------------------------------------------
            Consensus   (51)

                                101                                             150
         DROME_Q9VM18    (1)   --------------------------------------------------
         DROME_Q9VM19    (1)   --------------------------------------------------
  Sc_P31688_TPS2_YEAST (101)   EQINGLTTTLQDHMKSDKEAKTDTTQTAPVTNNVHPVWLLRKNQSRWRNY
         Arthr_Q4NG94    (1)   --------------------------------------------------
   Q3IZA2_Q3IZA2_RHOS4   (1)   --------------------------------------------------
         RHILE_O87819    (1)   --------------------------------------------------
         THETH_Q5MCN5    (1)   --------------------------------------------------
            Xo_Q5H2G4    (1)   --------------------------------------------------
         E.coli_OTSBP    (1)   --------------------------------------------------
               seqID2    (1)   --------------------------------------------------
            Consensus  (101)

                                151                                             200
         DROME_Q9VM18    (1)   --------------------------------------------------
         DROME_Q9VM19    (1)   --------------------------------------------------
  Sc_P31688_TPS2_YEAST (151)   AEKVIWPTFHYILNPSNEGEQEKNWWYDYVKFNEAYAQKIGEVYRKGDII
         Arthr_Q4NG94    (1)   --------------------------------------------------
   Q3IZA2_Q3IZA2_RHOS4   (1)   --------------------------------------------------
         RHILE_O87819    (1)   --------------------------------------------------
         THETH_Q5MCN5    (1)   --------------------------------------------------
            Xo_Q5H2G4    (1)   --------------------------------------------------
         E.coli_OTSBP    (1)   --------------------------------------------------
               seqID2    (1)   --------------------------------------------------
            Consensus  (151)
```

**FIGURE 2 D**

```
                                  201                                              250
        DROME_Q9VM18       (1)  --------------------------------------------------
        DROME_Q9VM19       (1)  --------------------------------------------------
Sc_P31688_TPS2_YEAST     (201)  WIHDYYLLLLPQLLRMKFNDESIIIGYFHHAPWPSNEYFRCLPRRKQILD
        Arthr_Q4NG94       (1)  --------------------------------------------------
  Q3IZA2_Q3IZA2_RHOS4      (1)  --------------------------------------------------
        RHILE_O87819       (1)  --------------------------------------------------
        THETH_Q5MCN5       (1)  --------------------------------------------------
           Xo_Q5H2G4       (1)  --------------------------------------------------
        E.coli_OTSBP       (1)  --------------------------------------------------
              seqID2       (1)  --------------------------------------------------
           Consensus     (201)
                                  251                                              300
        DROME_Q9VM18       (1)  --------------------------------------------------
        DROME_Q9VM19       (1)  --------------------------------------------------
Sc_P31688_TPS2_YEAST     (251)  GLVGANRICFQNESFSRHFVSSCKRLLDATAKKSKNSSNSDQYQVSVYGG
        Arthr_Q4NG94       (1)  --------------------------------------------------
  Q3IZA2_Q3IZA2_RHOS4      (1)  --------------------------------------------------
        RHILE_O87819       (1)  --------------------------------------------------
        THETH_Q5MCN5       (1)  --------------------------------------------------
           Xo_Q5H2G4       (1)  --------------------------------------------------
        E.coli_OTSBP       (1)  --------------------------------------------------
              seqID2       (1)  --------------------------------------------------
           Consensus     (251)
                                  301                                              350
        DROME_Q9VM18       (1)  --------------------------------------------------
        DROME_Q9VM19       (1)  --------------------------------------------------
Sc_P31688_TPS2_YEAST     (301)  DVLVDSLPIGVNTTQILKDAFTKDIDSKVLSIKQAYQNKKIIIGRDRLDS
        Arthr_Q4NG94       (1)  --------------------------------------------------
  Q3IZA2_Q3IZA2_RHOS4      (1)  --------------------------------------------------
        RHILE_O87819       (1)  --------------------------------------------------
        THETH_Q5MCN5       (1)  --------------------------------------------------
           Xo_Q5H2G4       (1)  --------------------------------------------------
        E.coli_OTSBP       (1)  --------------------------------------------------
              seqID2       (1)  --------------------------------------------------
           Consensus     (301)
                                  351                                              400
        DROME_Q9VM18       (1)  --------------------------------------------------
        DROME_Q9VM19       (1)  --------------------------------------------------
Sc_P31688_TPS2_YEAST     (351)  VRGVVQKLRAFETFLAMYPEWRDQVVLIQVSSPTANRNSPQTIRLEQQVN
        Arthr_Q4NG94       (1)  --------------------------------------------------
  Q3IZA2_Q3IZA2_RHOS4      (1)  --------------------------------------------------
        RHILE_O87819       (1)  --------------------------------------------------
        THETH_Q5MCN5       (1)  --------------------------------------------------
           Xo_Q5H2G4       (1)  --------------------------------------------------
        E.coli_OTSBP       (1)  --------------------------------------------------
              seqID2       (1)  --------------------------------------------------
           Consensus     (351)
                                  401                                              450
        DROME_Q9VM18       (1)  --------------------------------------------------
        DROME_Q9VM19       (1)  --------------------------------------------------
Sc_P31688_TPS2_YEAST     (401)  ELVNSINSEYGNLNFSPVQHYYMRIPKDVYLSLLRVADLCLITSVRDGMN
        Arthr_Q4NG94       (1)  --------------------------------------------------
  Q3IZA2_Q3IZA2_RHOS4      (1)  --------------------------------------------------
        RHILE_O87819       (1)  --------------------------------------------------
        THETH_Q5MCN5       (1)  --------------------------------------------------
           Xo_Q5H2G4       (1)  --------------------------------------------------
        E.coli_OTSBP       (1)  --------------------------------------------------
              seqID2       (1)  --------------------------------------------------
           Consensus     (401)
```

**FIGURE 2 D (continued)**

```
                                 451                                              500
         DROME_Q9VM18      (1)   --------------------------------------------------
         DROME_Q9VM19      (1)   --------------------------------------------------
  Sc_P31688_TPS2_YEAST   (451)   TTALEYVTVKSHMSNFLCYGNPLILSEFSGSSNVLKDAIVVNPWDSVAVA
          Arthr_Q4NG94     (1)   --------------------------------------------------
   Q3IZA2_Q3IZA2_RHOS4     (1)   --------------------------------------------------
          RHILE_O87819     (1)   --------------------------------------------------
          THETH_Q5MCN5     (1)   --------------------------------------------------
             Xo_Q5H2G4     (1)   --------------------------------------------------
          E.coli_OTSBP     (1)   --------------------------------------------------
                seqID2     (1)   ----MTNQNVIVSDRKPILGLKTITVSVSNSPLFSNSFPTYFNFPRRKLL
             Consensus   (451)
                                 501                                              550
         DROME_Q9VM18      (1)   ----------------------------------------MAEQKKAPL
         DROME_Q9VM19      (1)   ----------------------------------------MPEKQVAPV
  Sc_P31688_TPS2_YEAST   (501)   KSINMALKLDKEEKSNLESKLWKEVPTIQDWTNKFLSSLKEQASSNDDME
          Arthr_Q4NG94     (1)   ----------------------------------------MTPDAAPGK
   Q3IZA2_Q3IZA2_RHOS4     (1)   -------------------------------------------MHQPALH
          RHILE_O87819     (1)   -------------------------------------------MQSLENA
          THETH_Q5MCN5     (1)   --------------------------------------------------
             Xo_Q5H2G4     (1)   --------------------------------------------------
          E.coli_OTSBP     (1)   --------------------------------------------------
                seqID2    (47)   KLLEAADKNNLVVAPKITSMIDSMRDSSPTRLRSSSYDSVSDNDDKTSWI
             Consensus   (501)
                                 551                                              600
         DROME_Q9VM18     (10)   LKKEEDYVKALEGFINPETDQVALLLDYDGTLAPITEELS--VMPKDTEI
         DROME_Q9VM19     (10)   ISNLEDFANNLPGYL-ISESPVAVLLDYDGTLAPIADNPAKTKMPVELEA
  Sc_P31688_TPS2_YEAST   (551)   RKMTPALNRPVLLENYKQAKRRLFLFDYDGTLTPIVKDPAAAIPSARLYT
          Arthr_Q4NG94    (10)   APLTLQPELLAALKKVAATSHLLVAMDFDGTISPLVDHAADARPLPRSAA
   Q3IZA2_Q3IZA2_RHOS4     (8)   PDQDDGAAVLRSALR-DAPDEWALFLDIDGTLVDIAEQPHDVRVAASLPQ
          RHILE_O87819     (8)   VQDDDATQEMLSLVL-EEPDHWAMFLDIDGTLLNLAPTPDAIEVPEALPG
          THETH_Q5MCN5     (1)   ----------------MRAENPVFFLDYDGTLAPIAQRPEEAFPHPEAPR
             Xo_Q5H2G4     (1)   -MPMADAPPPLPSPP-LLDDACALFLDVDGTLIDFAHSPEAVRELPKVRE
          E.coli_OTSBP     (1)   ------MTEPLTETP-ELSAKYAWFFDLDGTLAEIKPHPDQVVVPDNILQ
                seqID2    (97)   VRFPSALNMFDEIVNAAKGKQIVVFLDYDGTLSPIVEDPDKAFITHEMRE
             Consensus   (551)             L         D  ALFLDYDGTLAPIAE PD    V  L
                                 601                                              650
         DROME_Q9VM18     (58)   NIKKLAANEKIFMVVFSGRELSEIKNHLK--FPNVTYAGNHGLEVEYPSG
         DROME_Q9VM19     (59)   ILHKIAKHPKVFLAVISGRGLKDVQKQVN--IDGITYAGNHGLEIEYPDG
  Sc_P31688_TPS2_YEAST   (601)   ILQKLCADPHNQIWIISGRDQKFLNKWLGGKLPQLGLSAEHGCFMKDVSC
          Arthr_Q4NG94    (60)   AFAALAALPRTTTALISGRALKSLRAVASP-PPETLLIGSHGAEAWLGPG
   Q3IZA2_Q3IZA2_RHOS4    (57)   DLARLADRLGGALALVTGRRVDWIDAHFG--PHLFPAAGLHGVERPRASG
          RHILE_O87819    (57)   QLHRLSNKLGGALALVTGRSLAYADALFK--PFAFPTAGLHGAEIRNAAG
          THETH_Q5MCN5    (35)   VLRALMERHP--VYVVTGRRVRGLEPLLP--LPGLPVVGGHGLEEGVLFG
             Xo_Q5H2G4    (49)   AIGLLNERLNGAVALVSGRPLAQLDALFA--PLLLPAAGLHGHELRSVVR
          E.coli_OTSBP    (44)   GLQLLATASDGALALISGRSMVELDALAK--PYRFPLAGVHGAERRDING
                seqID2   (147)   VVKDVASNFP--TAIVTGRSIEKVRSFVQ--VNEIYYAGSHGMDIEGPTN
             Consensus   (601)    L KLA        LALVSGR L  LDALL    P  LP AG HGLEIR   G
                                 651                                              700
         DROME_Q9VM18    (106)   K--------KFKIEMPEELLEKHNKLVSELKEKVVCS-GAWVEDKKISVT
         DROME_Q9VM19    (107)   S--------RHDYELPTEIQKNYTQMVRELKEKVEKN-GAWVEDKKVSLT
  Sc_P31688_TPS2_YEAST   (651)   QDWVN--------LTEKVDMSWQVRVNEVMEEFTTRTPGSFIERKKVALT
          Arthr_Q4NG94   (109)   S---------SELTLDPDQLTLLADVRRILEEIVEQAPGTLLEDKPAGVV
   Q3IZA2_Q3IZA2_RHOS4   (105)   E--------LDRAGLAPSLN-ALRKDLGRRTARMP---GVLLEDKVAAVA
          RHILE_O87819   (105)   ---------MQTVEATAEFQ-ALKHALTEEAEHYP---GVLIEDKGAAVA
          THETH_Q5MCN5    (81)   ---------EVRPLFPVDLG-----PLRARLPSCP---GVRVEDKGFALA
             Xo_Q5H2G4    (97)   AR-------AAMPQDTSEWSHGLHQRAAALTHRHP---GVLVEDKGLSVA
          E.coli_OTSBP    (92)   K--------THIVHLPDAIARDISVQLHTVIAQYP---GAELEAKGMAFA
                seqID2   (193)   ENSNGQSNERVLFQPAREFLPMIEKVVNILEEKTKWIPGAMVENNKFCLS
             Consensus   (651)                 L  ELL  L  VL  L EK P   GALVEDK VAVA
```

**FIGURE 2 D (continued)**

```
                          701                                                750
       DROME_Q9VM18 (147) YHYKGVTDKLKAKLIAEARGEIQAH-----GFQLIETPYALEGKPRVNWD
       DROME_Q9VM19 (148) YHYRDTPAALKDQQKQLASEICTK-----FGFRANQAHEALEAKPPVNWN
 Sc_P31688_TPS2_YEAST (693) WHYRRTVPELGEFHAKELKEKLLS-FTDDFDLEVMDGKANIEVRP-RFVN
        Arthr_Q4NG94 (150) LHTRLAADDVAEDAVAAARAALQG----LPDVYLKNGNRVLETSV-VHAS
  Q3IZA2_Q3IZA2_RHOS4 (143) LHYRQAPEREPDVRAMMEMALKVMG----DGWVLQHGKMVVELRP-ASAN
        RHILE_O87819 (142) AHYRLAPEYEKVLADRMHHYAELAG----PNWALQLGKMVFELRP-ARSS
        THETH_Q5MCN5 (114) LHYRGAEDEEKARACLEAWLKAVEGLLEALGLEALPGKKVLELKP-KGVD
           Xo_Q5H2G4 (137) LHWRAQPQAGPSVLAFAQQEIAQL-----SGYRLQPGDHVVEFVP-AGSN
         E.coli_OTSBP (131) LHYRQAPQHEDALMTLAQRITQIW-----PQWALQQGKCVVEIKP-RGTS
              seqID2 (243) VHFRRVDSKRWPALAEVVKSVLIDYP----KLKLTQGRKVLEIRPTIKWD
           Consensus (701) LHYR APD   E       K I I        GW LQ GK VLELKP
                          751                                                800
       DROME_Q9VM18 (192) KGEGAKMILEKQFDAD-------------WAKNLKIVYVGDDTTDEDAIK
       DROME_Q9VM19 (193) KGEAAVYILKQKFGDN-------------WSQKVSVVFAGDDTTDEDAMR
 Sc_P31688_TPS2_YEAST (741) KGEIVKRLVWHQHGKPQDMLKGISEKLPKDEMPDFVLCLGDDFTDEDMFR
        Arthr_Q4NG94 (195) KGEGMAFLRQATDAT-------------------AVVFAGDDTTDEDALG
  Q3IZA2_Q3IZA2_RHOS4 (188) KGTALEAFLTEPPFAG-------------R----RFVALGDDVTDEEMFR
        RHILE_O87819 (187) KGDALERFFQSDPFKN-------------R----CFITLGDDLTDESMFA
        THETH_Q5MCN5 (163) KGQAVLRLLGRHPDH-------------------TPVYIGDDTTDEAAFL
           Xo_Q5H2G4 (181) KGLAVEQLMQHGAPMG-------------R----TPVFVGDDLTDEFGFD
         E.coli_OTSBP (175) KGEALAAFMQEAPFIG-------------R----TPVFLGDDLTDESGFA
              seqID2 (289) KGQALNFLLKSLGYEN-------------S-DDVVPVYIGDDRTDEDAFK
           Consensus (751) KGEAL   LL     F                    PVFIGDD TDEDAFR
                          801                                                850
       DROME_Q9VM18 (229) VLHGIGK-TFRVSELPT-----------------LKTYANKQIKTVEEVG
       DROME_Q9VM19 (230) VLRGLGR-SFRISADAQ-----------------IQTYADFRLPKQAVMT
 Sc_P31688_TPS2_YEAST (791) QLNTIETCWKEKYPDQKNQWGNYGFYPVTVGSASKKTVAKAHLTDPQQVL
        Arthr_Q4NG94 (226) RLYPGD----VGVKVGL-----------------DFTQAQFRVEAPVHIA
  Q3IZA2_Q3IZA2_RHOS4 (221) VVNACGG-LSVRICRTE-----------------APTLARARLASPQALR
        RHILE_O87819 (220) TANARGG-VSVRVGAIG-----------------APSCATSRLSSSALVR
        THETH_Q5MCN5 (194) ALFGRG--LTFKVGEG-------------------PTAAQGRLKDVEEVL
           Xo_Q5H2G4 (214) AANRLGG-WSVLVGDR------------------VQTSARFRVDGTADVH
         E.coli_OTSBP (208) VVNRLGG-MSVKIGTG-------------------ATQASNRLAGVPDVW
              seqID2 (325) VIRERGQGFGIFVSKVP-----------------KDTNASYSLQDPSQVN
           Consensus (801) VLNGLG   SVKVGD                     T A FRL     V
                          851                                                900
       DROME_Q9VM18 (261) YLLKAVQAYYEKKKKA----------------------------------
       DROME_Q9VM19 (262) DLLKWLANVYVS--------------------------------------
 Sc_P31688_TPS2_YEAST (841) ETLGLLVGDVSLFQSAGTVDLDSRGHVKNSESSLKSKLASKAYVMKRSAS
        Arthr_Q4NG94 (255) ELLETLLRERSLAVGEAP--------------------------------
  Q3IZA2_Q3IZA2_RHOS4 (253) DLVAELGA------------------------------------------
        RHILE_O87819 (252) NVLAALAA------------------------------------------
        THETH_Q5MCN5 (223) AYLQTYLRPTSL--------------------------------------
           Xo_Q5H2G4 (245) AWLQRNARRR----------------------------------------
         E.coli_OTSBP (238) SWLEMLTTALQQKRENNRSDDYESFSRSI---------------------
              seqID2 (358) KFLERLVEWKRKTVGEE---------------------------------
           Consensus (851)  LL  L
                          901
       DROME_Q9VM18 (277) ------
       DROME_Q9VM19 (274) ------
 Sc_P31688_TPS2_YEAST (891) YTGAKV
        Arthr_Q4NG94 (273) ------
  Q3IZA2_Q3IZA2_RHOS4 (261) ------
        RHILE_O87819 (260) ------
        THETH_Q5MCN5 (235) ------
           Xo_Q5H2G4 (255) ------
         E.coli_OTSBP (267) ------
              seqID2 (375) ------
           Consensus (901)
```

**FIGURE 2 D (continued)**

```
                              1                                                50
Y2006_MYCTU/285-512     (1)   FLDFDGTLSDIVERPEAATLVDG-AAEALRALAAQCP--VAVISGRDLAD
TPSX_SCHPO/684-929      (1)   LLYFEGTISTWGSQYHNVMTSLQRTSNLLNMLTSDPKNTVYVFSSLSCQE
TPS3_YEAST/753-979      (1)   CADYKASKKHAFIFKISEPPTSR-MASLLSELSSN--NIVYVLSSFTKNT
TPS2_YEAST/575-822      (1)   LFDYDGTLTPIVKDPAAAIPSAR-LYTILQKLCADPHNQIWIISGRDQKF
TPP_ENCCU/506-707       (1)   VMDYDGTLTNIVARPPMAAPTQE-LKDLLIRLGKICR--VVISTGRSVED
TPP1_SCHPO/568-794      (1)   MMDYDGTLTPIVRDPNAAVPSKK-LRDNLATLAADPKNQVWIISGRDQQF
SL1_YEAST/819-1055      (1)   MEDYQSSKKRMFVFNIAEPPSSR-MISILNDMTSKG-NIVYIMNSFPKPI
Q9ZV48_ARATH/587-822    (1)   LLDYDGTMMDQD--TLDKRPSDD-LISLLNRLCDDPSNLVFIVSGRGKDP
Q9ZR75_SELLP/615-851    (1)   QVMAPRGRAPDQIREMKIRLHPS-IKDILNVLCSDPKTTIVILSGSERVA
Q49734_MYCLE/179-411    (1)   FFTFDGTLSDIVDDPDSARPVPG-ATEALQKLATHCP--VAIISGRDLAD
Q00786_EMENI/1-227      (1)   MFDYDGTLSPIVKDPQSAIPSDR-VLRTLKTIAADPRNAVWIISGRDQAF
P93653_ARATH/603-843    (1)   PVDN-QGRRGDQIKEMDLNLHPE-LKGPLKALCSDPSTTIVVLSGSSRSV
OTSB_RHISN/33-251       (1)   FLDIDGTLLDIAETPDAVAVPPS-LPASLDHLSKKLGGALALVTGRGLDY
OTSB_ECOLI/18-234       (1)   FFDLDGTLAEIKPHPDQVVVPDN-ILQGLQLLATASDGALALISGRSMVE
087819_RHILE/31-248     (1)   FLDIDGTLLNSAPTPDAIEVPEA-LPGQLHRLSNKLGGALALVTGRSLAY
080738_ARATH/595-830    (1)   FLDYDGTLVPET--SIVKDPSAE-VISALKALCSDPNNTIFIVSGRGKVS
065664_ARATH/17-258     (1)   FLDYDGTLSPIVDDPDRAFMSRK-MRRTVRKLAKCFP--TAIVSGRCIEK
064897_ARATH/121-354    (1)   FLDYDGTLSPIVEDPDKAFISHE-MREVVKDVASNFP--TAIVTGRSIEK
064896_ARATH/124-368    (1)   FLDYDGTLSPIVEEPDCAYMSSA-MRSAVQNVAKYFP--TAIISGRSRDK
064608_ARATH/560-795    (1)   FLDYDGTLVPES--SIVQDPSNE-VVSVLKALCEDPNNTVFIVSGRGRES
050401_MYCTU/145-370    (1)   FFDFDGTLSDIVEDPDAAWLAPG-AAEALQKLAARCP--IAVLSGRDLAD
027788_METTH/24-253     (1)   ITDIDGTISEIAPTPEEAVVDDE-MRDVLRDLASRYR-VAAFISGRPVRE
023617_ARATH/598-833    (1)   LLDYDGTMVQPG--SIRTTPSRE-TIEILNNLSSDPKNIVYIVSGKDRRT
014145_SCHPO/579-812    (1)   LLDYDGTLIESARNSIDAVPSDR-LLRTLKRLASDSRNIVWILSGRSQKF
Consensus               (1)    LDYDGTLS I    P  A PS     LL  L  LAS      V IVSGR

                              51                                               100
Y2006_MYCTU/285-512    (48)   ----------VRNRVKVDGLWLAGSHGFELVAPDG---SHHQN-------
TPSX_SCHPO/684-929     (51)   LEQLFQR---------VPKLGIVAENGCFVRSPPKGDATMPVSK-----K
TPS3_YEAST/753-979     (48)   FESLYN---------GVLNIGLIAENGAYVRVNG----------------
TPS2_YEAST/575-822     (50)   LNKWLGG--------KLPQLGLSAEHGCFMKDVSCQD-------------
TPP_ENCCU/506-707      (48)   CDKFFP-----------KELEVFAEHGACHRIDGKWK-------------
TPP1_SCHPO/568-794     (50)   LRNWMD---------DLKGLGLSAEHGSFVRKPHSTT-------------
SL1_YEAST/819-1055     (49)   LENLYS---------RVQNIGLIAENGAYVSLNG----------------
Q9ZV48_ARATH/587-822   (48)   LSKWFD---------SCPNLGISAEHGYFTRWNSNSP-------------
Q9ZR75_SELLP/615-851   (50)   LDEVFG----------EFDLWLAAENGMFLRHTQGEW-------------
Q49734_MYCLE/179-411   (48)   ----------VIKRIGVPGLWYSGSHGFESTAPDG---THHQN-------
Q00786_EMENI/1-227     (50)   LDEWMG---------HLPELGLSAEHGCFIRMPRSDN-------------
P93653_ARATH/603-843   (49)   LDKNFG----------EYDMWLAAENGMFLRLTNGEW-------------
OTSB_RHISN/33-251      (50)   ----------ADQLFSPANFPIAGLHGAERRDPDGR--------------
OTSB_ECOLI/18-234      (50)   LD----------ALAKPYRFPLAGVHGAERRDING---------------
087819_RHILE/31-248    (50)   ----------ADALFKPFAFPTAGLHGAELRNAAG---------------
080738_ARATH/595-830   (48)   LSEWLA---------PCENLGIAAEHGYFTRWNKSSD-------------
065664_ARATH/17-258    (48)   LIMIVLTTFQVYNFVKLTELYYAGSHGMDIKGPEQGSKYEQDS-------
064897_ARATH/121-354   (48)   ----------VRSFVQVNELYYAGSHGMDIEGPTNENSNGQSN-------
064896_ARATH/124-368   (48)   ----------VYEFVNLSELYYAGSHGMDIMSPAGESLNHEHSRTVSVYE
064608_ARATH/560-795   (48)   LSNWLS---------PCENLGIAAEHGYFIRWKSKDE-------------
050401_MYCTU/145-370   (48)   ----------VTQRVGLPGLWYAGSHGFELTAPDG---THHQN-------
027788_METTH/24-253    (49)   ALRMVG----------VPGAVYVGNHGLEYIINGNYQR------------
023617_ARATH/598-833   (48)   LTEWFS---------SCDDLGLGAEHGYFIRPNDGTD-------------
014145_SCHPO/579-812   (50)   MEEWMG---------DLSELGLSSEHGSAIRPPLAGS-------------
Consensus              (51)   L                V  L LAAEHG FIR
```

# FIGURE 3

```
                                 101                                              150
Y2006_MYCTU/285-512      (78)  --------AAATAALDGLAEAAAQWADALREIAGAVVEHKRFAVAVHYRN
TPSX_SCHPO/684-929       (87)  EIAELWKNKVLGTDLTWMKTVSEIFEYYAERTTGAYVENKDATVILHLRE
TPS3_YEAST/753-979       (73)  ------SWYNIVEELDWMKEVAKIFDEKVERLPGSYYKIADSMIRFHTEN
TPS2_YEAST/575-822       (79)  -----WVNLTEKVDMSWQVRVNEVMEEFTTRTPGSFIERKKVALTWHYRR
TPP_ENCCU/506-707        (74)  ---------EGGTFPQ-KDLAWRIGQFYLARIPGSEIERKKTGYAFHFRN
TPP1_SCHPO/568-794       (78)  -----WINLAELLDMSWKKEVRRIFQYYTDRIQGSSIEEKRCAMTWHYRK
SL1_YEAST/819-1055       (74)  ------VWYNIVDQVDWRNDVAKILEDKVERLPGSYYKINESMIKFHTEN
Q9ZV48_ARATH/587-822     (76)  -----WETSELPADLSWKKIAKPVMNHYMEATDGSFIEEKESALVWHHQE
Q9ZR75_SELLP/615-851     (77)  -----MTTMPEHLNMDWLESVQLVFDYFCERIPRSFVETRETSLVWNYKY
Q49734_MYCLE/179-411     (78)  --------DAAEATLPILEQAATQWRDQLGPIPGVMVEHKRFGVAVHYRN
Q00786_EMENI/1-227       (78)  -----WQNLAETTDMGWQKEVMEIYQHFTERIQGSFIERKKVALTWHYRR
P93653_ARATH/603-843     (76)  -----MTTMPEHLNMEWVDSVKHVFKYFTERTPRSHFETRDTSLIWNYKY
OTSB_RHISN/33-251        (76)  --------VHKAAETADFERLKAELVAATASWAGVLIEDKGAAVAAHYRL
OTSB_ECOLI/18-234        (75)  ------KTHIVHLPDAIARDISVQLHTVIAQYPGAELEAKGMAFALHYRQ
O87819_RHILE/31-248      (75)  --------MQTVEATAEFQALKHALTEEAEHYPGVLIEDKGAAVAAHYRL
O80738_ARATH/595-830     (76)  -----WETSGLSDDLEWKKVVEPIMRLYTETTDGSNIEAKESALVWHHQD
O65664_ARATH/17-258      (91)  ---KSLLCQPATEFLPMIDEVYHKLVEKTKSTPGAQVENNKFCVSVHFRR
O64897_ARATH/121-354     (81)  ---ERVLFQPAREFLPMIEKVVNILEEKTKWIPGAMVENNKFCLSVHFRR
O64896_ARATH/124-368     (88)  QGKDVNLFQPASEFLPMIDKVLCSLIESTKDIKGVKVEDNKFCISVHYRN
O64608_ARATH/560-795     (76)  -----WETCYSPTDTEWRSMVEPVMRSYMEATDGTSIEFKESALVWHHQD
O50401_MYCTU/145-370     (78)  --------DAAAAALPVLKQAAAEWRQQLGPFPGVVVEHKRFGVAVHYRN
O27788_METTH/24-253      (77)  -------FSEVEEYLPLIKKCALELRDKIIEEN-VIFEDKGICYLIHYRQ
O23617_ARATH/598-833     (76)  -----WETSSLVSGFEWKQIAEPVMRLYTETTDGSTIETKETALVWNYQF
O14145_SCHPO/579-812     (78)  -----WSSCAENLDLSWKDTVRDFFQYLVERIPGSYIEEKKHSISWCYQN
Consensus               (101)                L W   V  IL  Y E TPGS IE K  ALAWHYR

                                 151                                              200
Y2006_MYCTU/285-512     (120)  VADDSVDNLIAALRRLGHAA-----GVRVTTGRKVVELRPDIAWDKGKAL
TPSX_SCHPO/684-929      (137)  AEDLEAAMWAAKECCESVNNFN--VPCSATIQNDMVVCKSNK-VSKRLAA
TPS3_YEAST/753-979      (117)  ADDQDRVPTVIGEAITHINTLFDDRDIHAYVHKDIVFVQQTG-VALAAAE
TPS2_YEAST/575-822      (124)  TVPDLGEFHAKELKEKLLSFT-DDFDVEVMDGKANLEVRPRF-VNKGEIV
TPP_ENCCU/506-707       (114)  VSPLIGVKQARALFELLMRVC----KDYVKKGNHVIEVRSSK---KSCAM
TPP1_SCHPO/568-794      (123)  ADPDNGAFQALECEALLEELVCSKYDVEVMRGKANLEVRPSS-INKGGIV
SL1_YEAST/819-1055      (118)  ADDQDRVASVIGDAITHINTVFDHRGIHAYVYKNVVSVQQVG-LSLSAAQ
Q9ZV48_ARATH/587-822    (121)  ADHSFGSWQAKELLDHLESVL-TNEPVVVKRGQHIVEVKPQG-VSKGKVV
Q9ZR75_SELLP/615-851    (122)  ADVEFGRVQARDLLQHLWTGPISNAAVDVVQGGKSVEVRPVG-VSKGSAI
Q49734_MYCLE/179-411    (120)  VARDRVNEVAVALRTAGQRN-----ALRVTTGREVIELRPDIDWDKGKTL
Q00786_EMENI/1-227      (123)  ADPEYGAFQARECRKQLEEHVSKTWDVEVMAGKANLEVRPRF-VNKGFIA
P93653_ARATH/603-843    (121)  ADIEFGRLQARDLLQHLWTGPISNASVDVVQGSRSVEVRAVG-VTKGAAI
OTSB_RHISN/33-251       (118)  APDRQLELEQLMEWALYRAGP----DWALQHGKMVVEIRPAR-ANKGDAV
OTSB_ECOLI/18-234       (119)  APQHEDALMTLAQRITQIWP-----QMALQQGKCVVEIKPRG-TSKGEAI
O87819_RHILE/31-248     (117)  APEYEKVLADRMHHYAELAGP----NWALQLGKMVFELRPAR-SSKGDAL
O80738_ARATH/595-830    (121)  ADPDFGSCQAKELLDHLETVL-VNEPVIVNRGHQIVEVKPQG-VSKGLVT
O65664_ARATH/17-258     (138)  VDENNWSDLANQVRSVLKDYP----KLRLTQGRKVLEVRPIIKWDKGKAL
O64897_ARATH/121-354    (128)  VDEKRWPALAEVVKSVLIDYP----KLKLTQGRKVLEIRPTIKWDKGQAL
O64896_ARATH/124-368    (138)  VEEKNWTLVAQCVDDVLRTYP----KLRLTHGRKVLEIRPVIDWDKGKAV
O64608_ARATH/560-795    (121)  ADPDFGSCQAKELLDHLESVL-ANEPVVVKRGQHIVEVKPQG-VSKGLAA
O50401_MYCTU/145-370    (120)  AARDRVGEVAAALRTAEQRH-----ALRVTTGREVIELRPDVDWDKGKTL
O27788_METTH/24-253     (119)  CTDPELSRKRILKNLQEIPES---KGLKVDHGRMLVELKPPLHYNKGFIV
O23617_ARATH/598-833    (121)  ADPDFGSCQAKELMEHLESVL-TNDPVSVKTGQQLVEVKPQG-VNKGLVA
O14145_SCHPO/579-812    (123)  ANTTYSKFQALECQTNLEDML-KHYDVEISPAKSFLEVHPNY-LNKGSIV
Consensus               (151)  AD D     A  L   L         V V  GK VVEVRP   V KG AV
```

## FIGURE 3 (continued)

```
                                    201                                              250
  Y2006_MYCTU/285-512   (165)  DWIGERLGPA------------EVGPDLRLPIYIGDDL-TDEDAFDAVRF
   TPSX_SCHPO/684-929   (184)  EDIYSANGG-----------------DYDLIFAASNDP-DDDTVFSWLKN
   TPS3_YEAST/753-979   (166)  FLLKFYNSGVSPTDNS----------RISLSRTSSSMSVGNNKK------
   TPS2_YEAST/575-822   (172)  KRIVWHQHGKPQDMLKGISEKLPKDEMPDFVLCLGDDF-TDEDMFRQLNT
    TPP_ENCCU/506-707   (157)  EKIEEG----------------------FVLCAGDDV-ADEDMFDVCKG
   TPP1_SCHPO/568-794   (172)  KQILSSYPED---------------SLPSFTFCAGDDR-TDEDMFRSLHK
   SL1_YEAST/819-1055   (167)  FLFRFYNSASDPLDTSS--------GQITNIQTPSQQNPSDQEQQPPASP
  Q9ZV48_ARATH/587-822  (169)  EHILATMRNTK-------------GKRPDFILCIGDDR-SDEDMFDSLVK
  Q9ZR75_SELLP/615-851  (171)  DRILGEIVHS-----------KHMTIPIDYVLCIGHFLSKDEDIYTFFEP
  Q49734_MYCLE/179-411  (165)  HWVIDRLHHAGT----------QVGSASLMPICLGDDI-TDEDAFDAVRH
  Q00786_EMENI/1-227    (172)  TRLVQAYEDG---------------KVPEFILCSGDDF-TDEGMLFPFLA
  P93653_ARATH/603-843  (170)  DRILGEIVHS-----------KSMTTPIDYVLCIGHFLGKDEDVYTFFEP
   OTSB_RHISN/33-251    (163)  AAFLGQPPFAG-----------------RRALALGDDV-TDEAMFRTVN-
   OTSB_ECOLI/18-234    (163)  AAFMQEAPFIG-----------------RTPVFLGDDL-TDESGFAVVN-
  O87819_RHILE/31-248   (162)  ERFFQSDPFKN-----------------RCPLTIGDDL-TDESMFAIAN-
  O80738_ARATH/595-830  (169)  GKILSRMLED--------------GIAPDFVVCIGDDR-SDEEMFENISI
  O65664_ARATH/17-258   (184)  EFLLESLGYAN--------------CTDVIPIYIGDDR-TDEDAFKVYTE
  O64897_ARATH/121-354  (174)  NFLLKSLGYEN--------------SDDVVPIYIGDDR-TDEDAFKVLRE
  O64896_ARATH/124-368  (184)  TFLLESLGLNN--------------CEDVLPIYVGDDR-TDEDAFKVLRD
  O64608_ARATH/560-795  (169)  EKVIREMVER---------------GEPPEMVMCIGDDR-SDEDMFESILS
  O50401_MYCTU/145-370  (165)  LWVLDHLPHS--------------GSAPLVPIYLGDDI-TDEDAFDVLG-
  O27788_METTH/24-253   (166)  RKILEDSDVSS-------------------AIYLGDDI-TDADAFRELRK
  O23617_ARATH/598-833  (169)  ERILTTMQEK--------------GKLLDFILCVGDDR-SDEDMFEVIMS
  O14145_SCHPO/579-812  (171)  RRILKRSGS------------------VDIVFCAGDDK-TDENMFEVFLP
              Consensus (201)    IL  L                      FVLCIGDD  TDEDMF  I
```

```
                                   251                                 287
  Y2006_MYCTU/285-512   (202)  T----GVG---IVVRHNEHGDRRSAATFRLECPY---
   TPSX_SCHPO/684-929   (216)  FK---QSKKEVLPFTFSVCVLEHGNSTNADAESS---
   TPS3_YEAST/753-979   (200)  -----HFQNQVDFLCVSGSTLPIIEPLFKLVKQ----
   TPS2_YEAST/575-822   (221)  I----ETCWKEKYPDQKNQWGNYGFYPVTVGS-----
    TPP_ENCCU/506-707   (183)  -----------YTLKVGDQSLSLAYRLKDPE------
   TPP1_SCHPO/568-794   (206)  N----TRINKETSFAVTIG------SDKKLSI-----
   SL1_YEAST/819-1055   (209)  T----VSMNHIDFACVSGSSLPVLEPLFKLVND----
  Q9ZV48_ARATH/587-822  (205)  H----QDVSS-LGLEEVFACLVLQKPSKAKYYLDDTP
  Q9ZR75_SELLP/615-851  (210)  E----LPLLDRDSSTS-----NLGKPLGGKLPIDRKS
  Q49734_MYCLE/179-411  (204)  T----DVGGIPIVVRHTEDGNRLTAALFTLDSPM---
  Q00786_EMENI/1-227    (206)  T----PKLRHELLITYLR------YVPCLEEI-----
  P93653_ARATH/603-843  (209)  E----LPSDMPALARSRPSSDSLAKSSSGDRRPPSKS
   OTSB_RHISN/33-251    (194)  -----RLGGLSLRLGPPVPALELLGSLPSAE------
   OTSB_ECOLI/18-234    (194)  -----RLGGMSVKIGTG--ALQASWRLAGVP------
  O87819_RHILE/31-248   (193)  -----ARGGVSLRLGAIGAPLCATSRLSSSA------
  O80738_ARATH/595-830  (204)  T----LSAQSSSLSTEIFACLVLRKPSKAKYFLDEVS
  O65664_ARATH/17-258   (219)  -------FSYGLLATFSGMELIEIWSLMRAI------
  O64897_ARATH/121-354  (209)  -----RGQGFGILVSKVPKDLNLSYSLQDPS------
  O64896_ARATH/124-368  (219)  G----PNHGYGILVSAVPKDLNLFYSLRDPS------
  O64608_ARATH/560-795  (204)  T----VTNPELLVQPEVFACLVLRKPSKAKYFLDDEA
  O50401_MYCTU/145-370  (199)  -----PHG-VPIVVRHTDDGDRLTAALFALDSPA---
  O27788_METTH/24-253   (196)  LESERGMKNVPILVLSKEIPPEVKNSARFFVYGIS--
  O23617_ARATH/598-833  (204)  A----KDGPALSPLAEIFACLVLQKPSKAKYYLDDTA
  O14145_SCHPO/579-812  (202)  T----AFSNSHLLDEIDSTEYSLSHHTDDNSDANKVE
              Consensus (251)             ILV       T A   L
```

**FIGURE 3 (continued)**

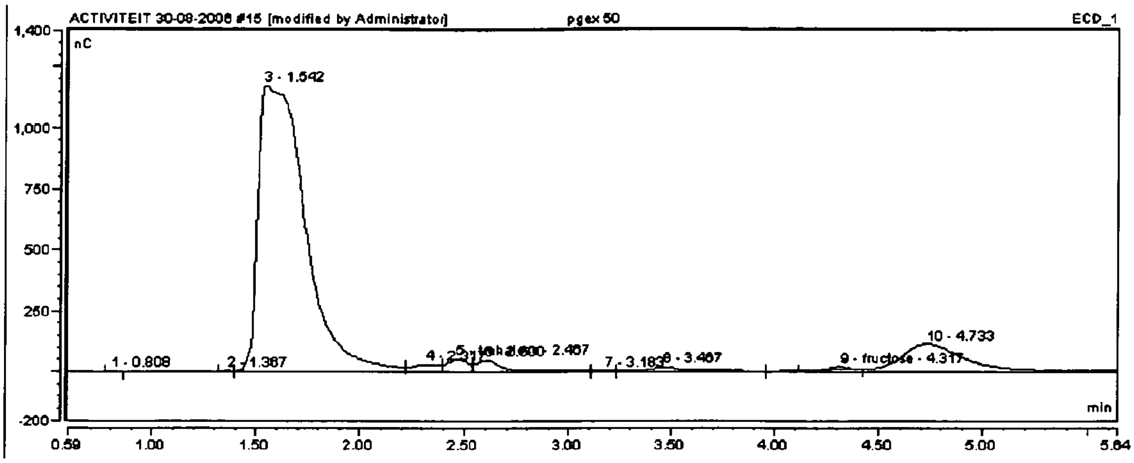


TPP activity measured by HPLC. Graph corresponding to the control sample pGEX

## FIGURE 4 A

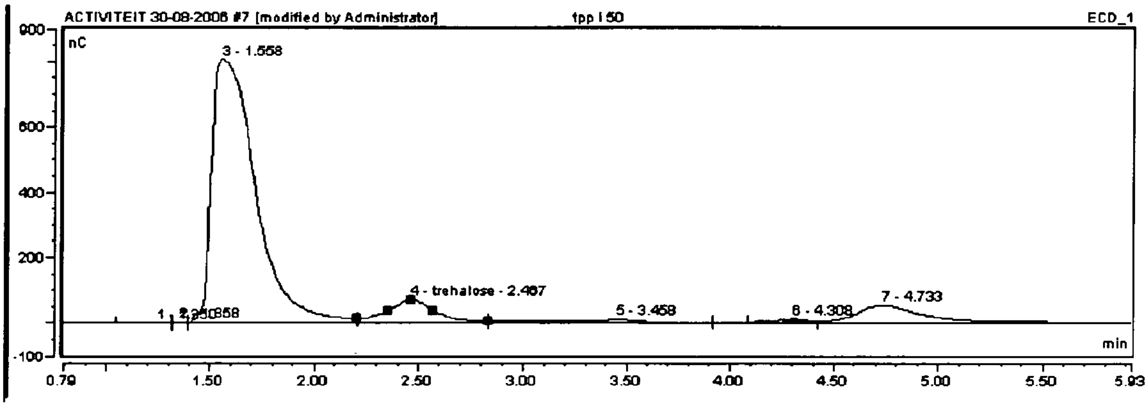


TPP activity measured by HPLC. Graph corresponding to the TPPI sample

## FIGURE 4 B

Quantification of TPP activity detected by HPLC in control sample, pGEX, and TPPI sample

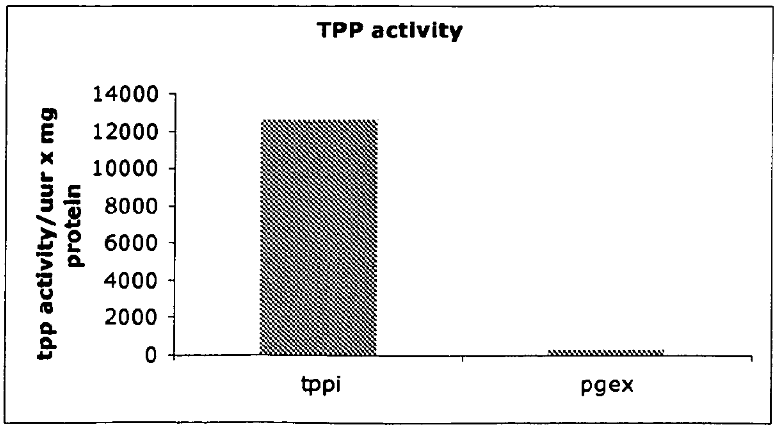


## FIGURE 5

Complementation of the growth defect on the yeast strain YSH448 (tps2Δ) by *Arabidopsis thaliana* representative Class III TPP polypeptides

*tps2?* Growth at 30° C

glucose

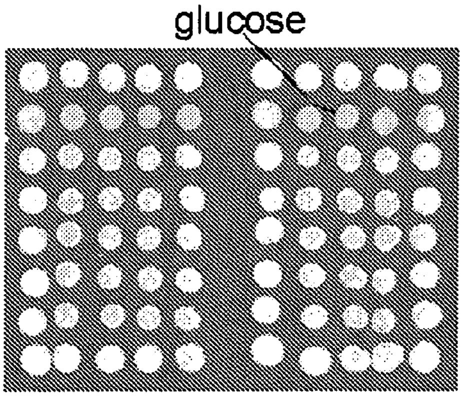


**FIGURE 6 A**

Growth at 37 ° C

glucose

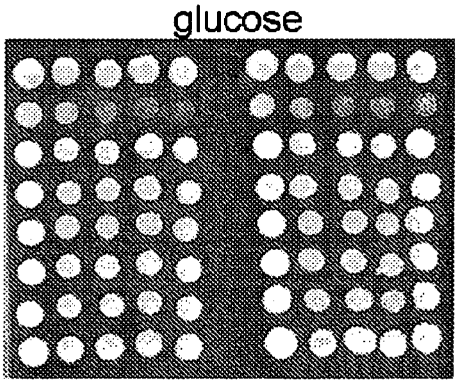


**FIGURE 6 B**

Growth at 39° C

glucose

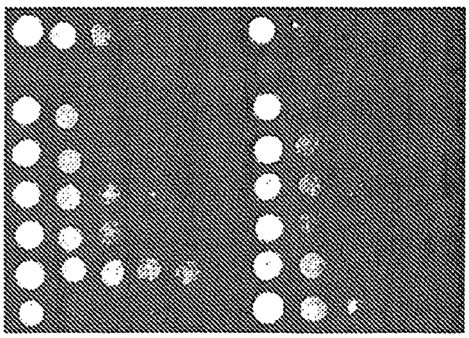


**FIGURE 6 C**

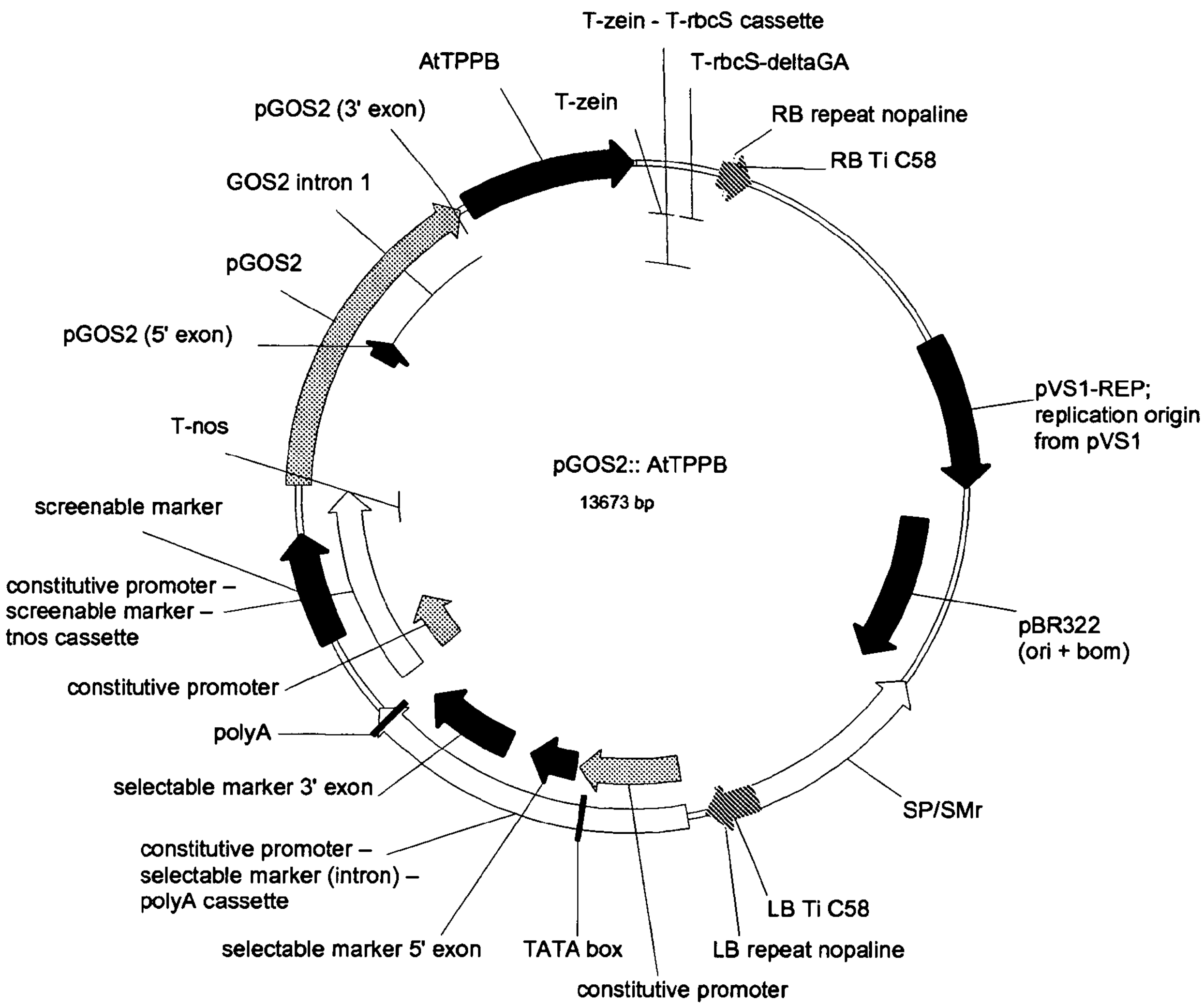
T-zein - T-rbcS cassette
AtTPPB
T-rbcS-deltaGA
T-zein
pGOS2 (3' exon)
RB repeat nopaline
RB Ti C58
GOS2 intron 1
pGOS2
pGOS2 (5' exon)
pVS1-REP;
replication origin
from pVS1
T-nos
pGOS2:: AtTPPB
13673 bp
screenable marker
constitutive promoter –
screenable marker –
tnos cassette
pBR322
(ori + bom)
constitutive promoter
polyA
selectable marker 3' exon
SP/SMr
constitutive promoter –
selectable marker (intron) –
polyA cassette
LB Ti C58
selectable marker 5' exon
TATA box
LB repeat nopaline
constitutive promoter

FIGURE 7

**SEQ ID NO: 01, DNA - Arabidopsis thaliana**

ATGACTAACCAGAATGTCATCGTTTCCGACAGGAAACCCATCTTGGGTTTGAAAACCATCACTGTC
TCTGTCTCTAACTCTCCTCTGTTCTCTAATTCCTTTCCCACTTACTTTAACTTCCCTCGTCGAAAG
CTCTTGAAACTTCTTGAAGCAGCCGACAAAAACAACTTAGTTGTTGCTCCAAAGATTACGTCTATG
ATCGATTCCATGCGTGATTCTTCCCCTACACGTCTCAGATCCTCTTCCTATGACTCTGTTTCAGAT
AACGACGACAAAACATCTTGGATCGTTCGTTTTCCTTCGGCTTTAAATATGTTTGATGAGATTGTG
AATGCTGCGAAAGGGAAACAGATTGTCGTGTTTCTTGATTACGACGGAACTCTTTCTCCCATAGTT
GAAGATCCCGACAAAGCTTTCATAACCCATGAGATGAGAGAAGTCGTAAAAGACGTGGCTTCGAAT
TTCCCGACTGCTATTGTCACCGGGAGATCCATTGAGAAGGTTCGTAGTTTTGTCCAAGTAAACGAG
ATTTACTACGCCGGAAGCCACGGCATGGACATTGAAGGTCCGACCAACGAAAATAGTAACGGCCAG
AGTAATGAAAGAGTGCTATTCCAACCTGCTCGTGAATTTTTACCGATGATCGAGAAGGTGGTTAAT
ATTTTAGAGGAAAAAACAAAATGGATCCCTGGGGCTATGGTGGAGAACAACAAGTTTTGTCTGTCC
GTACATTTTCGACGTGTTGATGAGAAGAGATGGCCTGCATTAGCTGAAGTAGTAAAATCAGTTCTT
ATTGATTATCCAAAGCTGAAACTAACCCAAGGTAGAAAGGTACTTGAAATCCGCCCCACAATCAAA
TGGGACAAGGGCCAGGCACTCAATTTTTTACTAAAATCATTAGGATATGAAAATTCGGATGATGTC
GTGCCGGTGTATATCGGGGATGACCGTACTGACGAAGATGCGTTTAAGGTTTTACGTGAAAGGGGA
CAAGGTTTTGGGATTTTTGTCTCAAAAGTACCAAAGGACACCAATGCCTCTTATTCTCTTCAAGAC
CCTTCTCAGGTTAACAAGTTTCTGGAACGTTTAGTAGAATGGAAGAGGAAGACAGTGGGAGAAGAG
TGA

**SEQ ID NO: 02, protein - Arabidopsis thaliana**

MTNQNVIVSDRKPILGLKTITVSVSNSPLFSNSFPTYFNFPRRKLLKLLEAADKNNLVVAPKITSM
IDSMRDSSPTRLRSSSYDSVSDNDDKTSWIVRFPSALNMFDEIVNAAKGKQIVVFLDYDGTLSPIV
EDPDKAFITHEMREVVKDVASNFPTAIVTGRSIEKVRSFVQVNEIYYAGSHGMDIEGPTNENSNGQ
SNERVLFQPAREFLPMIEKVVNILEEKTKWIPGAMVENNKFCLSVHFRRVDEKRWPALAEVVKSVL
IDYPKLKLTQGRKVLEIRPTIKWDKGQALNFLLKSLGYENSDDVVPVYIGDDRTDEDAFKVLRERG
QGFGIFVSKVPKDTNASYSLQDPSQVNKFLERLVEWKRKTVGEE

**SEQ ID NO: 03, DNA - Triticum aestivum**

CCCAATCCAAGGCTCTGTCATACTAAGTGACACCTTCTGAACAACGGAGTTTTCTCGAAAAGGAGG
TTGAAGCCCGGGCCTCTGCATCATTCTGAACATTGGAGTTAAGCGGAGCTGGCTTTGCATGGCTTT
TCCTATGTGCCAATCCACTCTTCTGATATAGATGTGTCATCTCTCTCAGCATGACAAGTTCACAGG
TACTCTGCTTTTGATGAACTTCCTCCACACCTGCAGTGACAAGAAAACGCTGAAGAAGTGGTTCTT
CATCGACAAGACAGTGGGCTAAGAGAATCCAGCTGAAAGAGAGACGACCGCCGTTCTGACCACATT
TTTTCCTCGTCCCACCCGATGGATTTGAGCAACGGCTCACCGGTCATCAGTGATCCGATGTCAATG
AGCCAGTCGTTGATGGGAGTGCTGCCTTCCAACATGATGCCGTTTTCAGTCAGGCCCGGGGGTTAC
TCCGGCGCTGGTGGCGCCGGCGTAAACGTCAGCAGGCGCAAGATCGAGGAGGTCCTCGTGAGCGGG
CTGCTGGATGCCATGAAATCCTCGTCGCCACGTAAGAAGCACAACGTGGTCTTCGGCCAGGAAAAC
CTGCCCGAAGAAGATCCTGCCTACAGTGCATGGATGGCAAAATGCCCCTCTGCTCTGGCTTCCTTC
AAGCAGATCGTAGCCAGTGCACAAGGCAAGAAGATTGCGGTCTTCCTGGACTACGATGGCACACTG
TCGCCGATCGTCGACGACCCTGAAAAAGCCGTCATGTCCCCTGTGATGAGAGCTGCTGTGAGAAAC
GTCGCCAAATACTTCCCCGCCGCCATCGTCAGCGGAAGGTCCCGGAAGAAGGTTCTTGAATTCGTA
AAACTGAAGGAACTCTGCTATGCTGGAAGTCATGGGATGGACATAATGACATCTTCTTCAGCACAT
TATGAACACAATACTGAAAAGGGCAAAGAAGCCAACCTCTTCCAACCTGCTCGCGATTTTCTGCCT
ATGATCGATGAGGTTTCCAAGGCCCTCTTGGAAGTCACGAGCGGAATCGAAGGCGCGAGCGTTGAG
GACAACAAGTTCTGCGTGTCTGTTCATTACCGCAACGTAGACGAGAAGGACTGGGAGCTGGTCGCA
CGGCTCGTAAACGAGGTGCTGGAGGATTTCCCCCCTCTCAAAGTGACCAACGGGCGAATGGTTTTA
GAGGTTCGTCCGGTGATCGACTGGGACAAGGGGAAGGCCGTCGAGTTCCTGCTTCAGTCGCTCGGG

## FIGURE 8

CTGAGCGACTCCGAGAACGTGATCCCAATCTACATCGGCGACGACCGCACCGACGAAGACGCGTTC
AAGGTGCTCCGGGAGAGGAACTGCGGGTACGGGATACTGGTCTCGCAGGCGCCCAAGGAAACCGAA
GCCTTCTACTCGCTCAGGGACCCGTCTGAAGTGATGGAGTTCCTGAACTCCTTGGTGAGATGGAAG
AAGCACTCGCTATGAACAAACAGGAGATGACTGTAGTTTCCGAGGCGACAGTTTTGCAGCGTTGGA
AACCATAGCTAGGGTCGAATGATGTTGCCGTCCCCTGTTAATTCGTTTAGGTCGATTGATATTCTT
GTACTGCTAGTCATGTTCTTTGCCACCGAGAAATTTGATCGGTGGCTGTGTTGGTTGTTATACATA
ATGCTTTAGGTTAACATAAATTGGATCTGGATCTCTTGATTAGAACACCAATACATGTGCGGTAGC
GTGTGAACTTACAAGTGATTGACGAAATCCCATACTCTTGATGCAGGCCCTAGTCGATGCAGCACA
CGGGAATAGTTGGCGAAGCGGAACAGCACCGAGTGAATAGCCCATGGCCGAAAGCGTGAACGTAGC
CAGCAATAGGTAGAAGACGCCCAGAAGGCTACCGGCACTAGAGATGGCCGGCATCAACCAGCGACT
GGTCGGTGTAGAGCAGCAGGAAGCCGCCGGCACATCCTTGCGGTGGCCGGCTGTGGTGATGGCCTT
CTGATCCCTTTATGCACCCTTTTAGGATCGGTAGTTAGGAGTTTGTT

**SEQ ID NO: 04, protein - Triticum aestivum**

MDLSNGSPVISDPMSMSQSLMGVLPSNMMPFSVRPGGYSGAGGAGVNVSRRKIEEVLVSGLLDAMK
SSSPRKKHNVVFGQENLPEEDPAYSAWMAKCPSALASFKQIVASAQGKKIAVFLDYDGTLSPIVDD
PEKAVMSPVMRAAVRNVAKYFPAAIVSGRSRKKVLEFVKLKELCYAGSHGMDIMTSSSAHYEHNTE
KGKEANLFQPARDFLPMIDEVSKALLEVTSGIEGASVEDNKFCVSVHYRNVDEKDWELVARLVNEV
LEDFPPLKVTNGRMVLEVRPVIDWDKGKAVEFLLQSLGLSDSENVIPIYIGDDRTDEDAFKVLRER
NCGYGILVSQAPKETEAFYSLRDPSEVMEFLNSLVRWKKHSL

**SEQ ID NO: 05, DNA - Glycine max**

ACTATTTTTTCAGGAGAATATTATTGCGCTTTTCGTGTTTCTCCCCGAAAGCACCAATAACGCAT
TGCCTTCCCTATGGTCGCTGTGTTAAAGTTTGGCTGCCCATAATATTACCCCATTTTTAGCTTCTG
CCAAACAACAACTTGTGCCTTTTTTTTTGTCTCCTTTCTTATTTGTTTCTTTTTAGCCTTTTTGTT
TTTTTATTGGCCGCCAAACTAAAAACAATATCAGTATTTGCGTCTTTTAATCTGGTATTCCTCTCT
TCTGACTGGTGGCTTCGTTTTCGCCTTTGAAGATATTTAGACGAAAACGAGAATCCGCATTCTGAT
TCTGAAGATTCATGTCTAATCTCATTTCTTTCTTCCCTTGGGTTGAGCTCGTTTTTTAGGAGCTCG
GCAGTTGATGGACTGTTACCCCAAGAGCAGTGACAAGAAAACATTAAAGAGGTGGTTTTTTATTGA
TAAAAGAGTTGGGTGAAGTGGAAATCCGATAAGTATTATTTCAATTGGTGCTCAATACTCCTTAAA
GTTCTTACATTGTTGCTGGACTGTAAATTGATTTAAAATCTGGATCCCATGGACTTGAAGTCGAAT
CATACTCCTGTTCTCACTGATGCTGCACCTGTAACAAAGTCTAGACTGGGTGTGCATTCCAGTTTG
TTGCCTTACTCCCATGCTGGGACAACCTTTACACATGGAATGTTATTGACTATCCCGAGGAAGAAG
ACAGGAATTCTTGAAGATGTTCGTTCTAGTGGTTGGTTGGATGCCATGAAATCATCTTCACCTCCA
GCCAGGAAGATAACAAAGGATGTTGGCCATGGGTTTGCATCATCTGATTCTGAAACTGCTGGTGCT
TATTTTAGCTGGCTGCTAAAATACCCATCCGCACTTGCATCTTTTGATCAAATCATGAACTATGCA
AAAGGGAAAAGAATTGCGCTGTTTATGGATTATGATGGAACTCTTTCACCAATTGTGGATAATCCT
GACTGTGCTTTCATGTCCGACAATATGCGTGCTGCTGTTAAAAAGGTGGCAGAATATTTTCCAACA
GCAATAATTAGTGGAAGAAGCCGTGATAAGGTATATCAATTTGTAGGACTAACAGAACTCTATTAT
GCTGGTAGTCATGGAATGGACATCATTGGGCCTGTTAGACAATCTGTATCTGATAATCACCCAAAT
TGCATTAGGTCTACAGACAAGCAGGGTAAGGAAGTAAATTTATTCCAACCTGCTGCTGAATTTCTG
CCCATGATTAATGAGGTACTTAATTCTCTTGAAGAGTGTACGAAAGACATTAAAGGAGCTAAAGTT
GAGAACAATAAATTTTGTGTATCTGTGCACTACCGGAATGTAGATGAAAAGTATTGGAATTGGGTG
GGGCAACATGTTCATGATGTTCTGAAGGGCTATCCACGTTTGCGCTTAACTCATGGGCGGAAGGTT
TTAGAGATCCGACCTGTGATTAACTGGGATAAGGGCAAAGCTGTCACGTTTCTACTTGAGTCACTT
GGGCTAAACAATTGTGATGATGTGCTTCCTATATATATTGGAGATGATCGGACAGACGAAGATGCA
TTTAAGGTTTTGAGAGAGGGAAATAAAGGTTATGGGATCTTGGTGTCTTCAGCACCAAAAGAAAGC

**FIGURE 8 (continued)**

AACGCAATTTACTCTCTTCGTGATCCATCAGAGGTCATGGAATTTCTCAAGTCACTTGTGTTGTGG
AAATCAAGCACCTTAAAAAGCCTTATATAGTGTATAGAGGAGAGAATATACTCTGCTATACTATAC
TATATCATAAAAAAAAAAAAACAGCTTTTTCGATTTTGGTTTTTCTCAGAAACATTCCAAGAGGTT
TACTGGAGCAGATTTACGCACAGAAGAACCTCTTTGGTAACAACAGCGTTGTCGCTACCCAGTTT
TTTTGCTCGAAGCTCTTGTGGTAGCTGTAAATTCGTCAACTCATAATTATAAATATCTTAAAGAAG
TAAAAAAAAAAAAAAAAAAGCG

**SEQ ID NO: 06, protein - Glycine max**
MDLKSNHTPVLTDAAPVTKSRLGVHSSLLPYSHAGTTFTHGMLLTIPRKKTGILEDVRSSGWLDAM
KSSSPPARKITKDVGHGFASSDSETAGAYFSWLLKYPSALASFDQIMNYAKGKRIALFMDYDGTLS
PIVDNPDCAFMSDNMRAAVKKVAEYFPTAIISGRSRDKVYQFVGLTELYYAGSHGMDIIGPVRQSV
SDNHPNCIRSTDKQGKEVNLFQPAAEFLPMINEVLNSLEECTKDIKGAKVENNKFCVSVHYRNVDE
KYWNWVGQHVHDVLKGYPRLRLTHGRKVLEIRPVINWDKGKAVTFLLESLGLNNCDDVLPIYIGDD
RTDEDAFKVLREGNKGYGILVSSAPKESNAIYSLRDPSEVMEFLKSLVLWKSSTLKSLI

**SEQ ID NO: 07, DNA - Tagetes spp.**
CGACCATTGGCAAACCGAGATTAGGAATTCATGATTCATTACTACCATACGCATCTTCTGGAGTCT
TCTCTCAAGCTCCATTTCTTAAAAAACCTGGACTACTTGATGATGTTCGTTCTACTAGCTGGTTGG
ATGCCATGAAATCTTCTTCTCCAAGGAATGTTCTAAAAAATAACGCCACTGATTTGTCAAATGATG
TTGCTTACCGCAATTGGATGATTAAGTATCCGTCTGCACTTGCATCCATTGAACAGATTGCAAATA
ATGCAAAGGGAAAGAGGATTGCCTTGTTTCTAGATTATGACGGAACATTATCCCCGATCGTGGACA
ATCCAGATCATGCCTTCATGTCTAATGCTATGCGTGCTGCAGTAAGAAATGTGGCAAAATACATTC
CAACAGCAATTATTAGTGGCAGAAGCTGTGAAAAGGTACACAAGTTTGTAGGACTGAAAGAACTAT
ATTATGCTGGTAGTCATGGGATGGACATAATGGGTCCTGTTCAGCCTCCAACTGATCATAGAATTG
AGGCTATAGAAGGAAATTTGTACCAGCCTGCTAGTGAATTTCTACCCATGATCAACGAGGTTTTTG
TGTCCCTTGTTGAGATAACCAAGGACATAGAAGGAGCAAAAGTCGAAGACAACAAGTTTTGTGTCT
CAGTGCACTATCGTAATGTAGATGAGAAGAACTGGACAATGGTAGCACAATGTGTCGAAGACACTT
TGAAAAACTATCCACGTCTGAGATTGACTCACGGGCGGATGGTTTTAGAGATCCGCCCTGTGCTTA
AGTGGGACAAGGGGAAGGCTGTTGAGTTCTTACTCGAATCACTTGGCTTAAGTAATTGTGATGATG
TGCTCCCTATATATGTAGGGGATGACAGAACAGACGAAGATGCATTCAAGTTTTTAAGAGAGGGTA
GTCTTGGTTATGGCATCTTGGTGACTCCTGCCCCAAAGGAAAGCAGTGCATATTATTCTCTCAGGG
ATCCATCCGAGGTGATGGAATTTATCAACTTACTAGTGATGCATAAGAAGTCGGTTCCATGATTCG
ACTGATTCGTACAATAGGAAGACGTTCTGAAATTTTCATTTAAAAGAATAAAACAAGGCATCTGAA
GTAGAAATGAGCACTTCTTCAACCTCCCTACCTGGCAATTGTAAATCGCACGATATTACTTTCTTC
ATGTTTGACATACGGGAAAGATATATGTGTATATACAATTTCTTATTATTGAATCCCATGTACGTA
CATATCCGGTCGGTTTTGTTAATACAAAGATCCGGTATTGTTAGTATAAAGTTAGACGAAAATCAA
GTTGATTGATGTAAAATTTTCTTTGTATGAATGTTCGGTACTTTACTTTTAATGTTTCTATGAAAT
TTTGATATTGTGTTTT

**SEQ ID NO: 08, protein - Tagetes spp.**
MKSSSPRNVLKNNATDLSNDVAYRNWMIKYPSALASIEQIANNAKGKRIALFLDYDGTLSPIVDNP
DHAFMSNAMRAAVRNVAKYIPTAIISGRSCEKVHKFVGLKELYYAGSHGMDIMGPVQPPTDHRIEA
IEGNLYQPASEFLPMINEVFVSLVEITKDIEGAKVEDNKFCVSVHYRNVDEKNWTMVAQCVEDTLK
NYPRLRLTHGRMVLEIRPVLKWDKGKAVEFLLESLGLSNCDDVLPIYVGDDRTDEDAFKFLREGSL
GYGILVTPAPKESSAYYSLRDPSEVMEFINLLVMHKKSVP

**FIGURE 8 (continued)**

**SEQ ID NO: 09, DNA - Arabidopsis thaliana**

ATGAAGATTACGGATATTTCCGGAAAGATCGAGACTTTGGTTGATTCCTTAAGGGATATGTCCCCG
ACCCGTGTCAGATCTTCCTTCTCTGATGAACATGTATCCGAGAACGATGACGAGAGAAGCTCCTGG
ATTGCTCTTCATCCGTCAGCATTGGATATGTTCGAACAAATCATGCGTGATGCTGAAGGCAAACAA
ATTATTATGTTTCTTGATTACGACGGAACTCTCTCGCTAATCACTGAAGATCACGACAGAGCCTAC
ATAACCGACGAGATGCGAGAAGTTGTAAAGGAAGTGGCTACATATTTCAAGACAGCGATCATCAGC
GGACGAAGCACTGACAAAGTACAGAGTTTTGTGAAACTCACTGGTATTCACTACGCTGGGAGCCAC
GGCATGGACATTAAGGGTCCGACAAATACCGATCAGAGTAACCAAGAAGAAGTGATGTTTCAACCT
GCAAGTGACTATTTACCGATGATTGACGAGGTGGTTAATGTTTTGAAGGAAAAGACAAAATCTATC
CCTGGAGCTACGGTCGAGCACAACAAGTTTTGTCTAACAGTACATTTTCGTCGGGTCGATGAAACG
GGATGGGCTGCATTAGCTGAGCAAGTGAGATTGGTTCTCATTGATTATCCGAAATTGAGACTGACA
CAAGGCAGAAAGGTCTTAGAACTCCGACCTTCCATCAAATGGGACAAGGGAAAGGCTCTCGAATTT
TTGCTAAACTCATTAGGGATAGCAGAATCTAAAGATGTTTTACCGGTTTACATTGGAGATGACCGT
ACTGATGAAGATGCATTTAAGGTTTTATGTGAAAGGGGACAAGGTTTTGGGATTATCGTCTCAAAA
ACTATTAAGGAAACGTACGCCTCTTACTCTCTTCAAGACCCATCTCAGGTTAAAGAATTTCTGGAG
CGTTTGGTAAAGTGGAAGAAACAAACACTTGGAGAATAAGAAGAAGAACTGGTTCATAAGAGATAT
TGAGTTTCGTGTTCTTAATTCTTATAACCCGTCCTGAAATATATGGGACTCTTAGCAAAATAAAAA
TGGAGACCTTAATTTTTAGATTTTAAATTTATTGTTTCAATGAATAATACTGAAAAGATTAATTTT
TCGTTAAAATGGTCTTGTAAGATATTCTAATGATTTTTTTTATATGAATTAAGAAGCATGTTTCG
TTGAATTTATAGGTTAACA

**SEQ ID NO: 10, protein - Arabidopsis thaliana**

MKITDISGKIETLVDSLRDMSPTRVRSSFSDEHVSENDDERSSWIALHPSALDMFEQIMRDAEGKQ
IIMFLDYDGTLSLITEDHDRAYITDEMREVVKEVATYFKTAIISGRSTDKVQSFVKLTGIHYAGSH
GMDIKGPTNTDQSNQEEVMFQPASDYLPMIDEVVNVLKEKTKSIPGATVEHNKFCLTVHFRRVDET
GWAALAEQVRLVLIDYPKLRLTQGRKVLELRPSIKWDKGKALEFLLNSLGIAESKDVLPVYIGDDR
TDEDAFKVLCERGQGFGIIVSKTIKETYASYSLQDPSQVKEFLERLVKWKKQTLGE

**SEQ ID NO: 11, DNA - Arabidopsis thaliana**

ATGTATTAAGACTTGACAACTTGTCTTTCTCACACCAAACCCCTCTCCTCTGTTTCATAACATCTG
CTCTTTCTTTTTTTTCCTAAGCCCCTAATGACAAACCATAATGCCTTAATCTCTGATGCTAAAGGC
AGCATCGGAGTTGCGGTTAGAGTTCCAAACCAATCTCTGTTTTCTCCCGGAGGTGGCCGATACATC
AGCATTCCCCGGAAGAAACTCGTGCAGAAGCTAGAGGCCGACCCGAGTCAAACCCGTATCCACACT
TGGATCGAAGCCATGAGGGCTTCTTCCCCAACCCGTACCCGACCGGGGAACATATCTCCCCTCCCG
GAGTCCGATGAGGAGGATGAATACTCTTCTTGGATGGCTCAACACCCGTCAGCTTTAACCATGTTT
GAAGAGATAGCTGAAGCTTCAAAAGGGAAACAAATCGTGATGTTTCTCGACTATGACGGTACATTA
TCCCCCATTGTTGAAAACCCTGATCGAGCTTACATGTCTGAAGAGATGAGAGAGGCAGTGAAAGGC
GTGGCTAGATATTTCCCGACCGCGATTGTCACTGGAAGATGCCGTGATAAGGTTCGTAGATTTGTG
AAACTTCCCGGACTTTACTATGCAGGTAGCCATGGAATGGACATCAAAGGACCTTCCAAAAGAAAC
AAACATAACAAGAACAATAAAGGAGTTCTTTTCCAAGCGGCGAATGAGTTTTTGCCTATGATTGAC
AAGGTCTCTAAGTGTCTAGTAGAGAAAATGAGAGACATAGAAGGAGCAAACGTCGAGAACAACAAG
TTTTGTGTCTCCGTACATTACCGTTGTGTTGATCAAAAGGACTGGGGATTGGTAGCGGAACACGTG
ACATCGATATTGAGTGAGTATCCGAAACTGAGGTTGACACAAGGAAGAAAAGTCTTAGAGATTCGA
CCAACCATCAAATGGGATAAAGGCAAAGCTCTCGAGTTCTTGCTCGAATCCTTAGGATTCGCTAAC
TCTAACGATGTTTTGCCCATCTATATAGGAGATGATCGTACGGACGAGGATGCTTTCAAGGTTTTG
AGAAACAAAGGACAAGGCTTTGGTATACTTGTGTCCAAAATTCCAAAGGAAACGAGTGCTACATAT
TCTCTACAAGAACCTTCCGAGGTAGGAGAGTTTTTGCAGCGACTCGTGGAATGGAAACAAATGTCA
CTAAGAGGAAGATAGCCAATTTCCTGACATAAATTTATTTTCAATTAATAAATGAATTAGTTTTCA

**FIGURE 8 (continued)**

CTATGCAACAAAAATTGTTGTATATATGATCAATGTTTTTTAATTATTTACTCTTCATGAACAA
ATGTAAGTTTATAGGAACTTTCTTAACCAAGAAAAAAGTAAGTTTGCTAGTATAATATTTTCATCA
TTCTCTTTTT

**SEQ ID NO: 12, protein - Arabidopsis thaliana**

MTNHNALISDAKGSIGVAVRVPNQSLFSPGGGRYISIPRKKLVQKLEADPSQTRIHTWIEAMRASS
PTRTRPGNISPLPESDEEDEYSSWMAQHPSALTMFEEIAEASKGKQIVMFLDYDGTLSPIVENPDR
AYMSEEMREAVKGVARYFPTAIVTGRCRDKVRRFVKLPGLYYAGSHGMDIKGPSKRNKHNKNNKGV
LFQAANEFLPMIDKVSKCLVEKMRDIEGANVENNKFCVSVHYRCVDQKDWGLVAEHVTSILSEYPK
LRLTQGRKVLEIRPTIKWDKGKALEFLLESLGFANSNDVLPIYIGDDRTDEDAFKVLRNKGQGFGI
LVSKIPKETSATYSLQEPSEVGEFLQRLVEWKQMSLRGR

**SEQ ID NO: 13, DNA - Arabidopsis thaliana**

CTCCTCACCTTCTCTCTCTCAACATCTCTCTCTTTCTCTGTCTTCTCTCTGTCTCAACGAGAATAT
GACTAACCAGAATGTCATCGTTTCCGACAGGAAACCCATCTTGGGTTTGAAAACCATCACTGTCTC
TGTCTCTAACTCTCCTCTGTTCTCTAATTCCTTTCCCACTTACTTTAACTTCCCTCGTCGAAAGCT
CTTGAAACTTCTTGAAGCAGCCGACAAAAACAACTTAGTTGTTGCTCCAAAGATTACGTCTATGAT
CGATTCCATGCGTGATTCTTCCCCTACACGTCTCAGATCCTCTTCCTATGACTCTGTTTCAGATAA
CGACGACAAAACATCTTGGATCGTTCGTTTTCCTTCGGCTTTAAATATGTTTGATGAGATTGTGAA
TGCTGCGAAAGGGAAACAGATTGTCATGTTTCTTGATTACGACGGAACTCTTTCTCCCATAGTTGA
AGATCCCGACAAAGCTTTCATAACCCATGAGATGAGAGAAGTCGTAAAAGACGTGGCTTCGAATTT
CCCGACTGCTATTGTCACCGGGAGATCCATTGAGAAGGTTCGTAGTTTTGTCCAAGTAAACGAGAT
TTACTACGCCGGAAGCCACGGCATGGACATTGAAGGTCCGACCAACGAAAATAGTAACGGCCAGAG
TAATGAAAGAGTGCTATTCCAACCTGCTCGTGAATTTTTACCGATGATCGAGAAGGTGGTTAATAT
TTTAGAGGAAAAAACAAAATGGATCCCTGGGGCTATGGTGGAGAACAACAAGTTTTGTCTGTCCGT
ACATTTTCGACGTGTTGATGAGAAGAGATGGCCTGCATTAGCTGAAGTAGTAAAATCAGTTCTTAT
TGATTATCCAAAGCTGAAACTAACCCAAGGTAGAAAGGTACTTGAAATCCGCCCCACAATCAAATG
GGACAAGGGCCAGGCACTCAATTTTTTACTAAAATCATTAGGATATGAAAATTCGGATGATGTTGT
GCCGGTGTATATCGGGGATGACCGTACTGACGAAGATGCGTTTAAGGTTTTACGTGAAAGGGGACA
AGGTTTTGGGATTCTTGTCTCAAAAGTACCAAAGGACACCAATGCCTCTTATTCTCTTCAAGACCC
TTCTCAGGTTAACAAGTTTCTGGAACGTTTAGTAGAATGGAAGAGGAAGACAGTGGGAGAAGAGTG
AAAGACATGCAACATAATTACACAAATAACACCTGAGTTTTGAGTAGATTATTAATTTATATAAAC
ACCCTTTTTAAATTAGTAGATGCCTGGAGGGTGTTTCGGCCAGAAGTTTTTCTTTTAATCTACATC
GAACCGAAAATTGTAAATTTCGTGTAACGATTTAGAAATGGAAAAATATATTAATAATTTACTCAT
TCTTATTTGACC

**SEQ ID NO: 14, protein - Arabidopsis thaliana**

MTNQNVIVSDRKPILGLKTITVSVSNSPLFSNSFPTYFNFPRRKLLKLLEAADKNNLVVAPKITSM
IDSMRDSSPTRLRSSSYDSVSDNDDKTSWIVRFPSALNMFDEIVNAAKGKQIVMFLDYDGTLSPIV
EDPDKAFITHEMREVVKDVASNFPTAIVTGRSIEKVRSFVQVNEIYYAGSHGMDIEGPTNENSNGQ
SNERVLFQPAREFLPMIEKVVNILEEKTKWIPGAMVENNKFCLSVHFRRVDEKRWPALAEVVKSVL
IDYPKLKLTQGRKVLEIRPTIKWDKGQALNFLLKSLGYENSDDVVPVYIGDDRTDEDAFKVLRERG
QGFGILVSKVPKDTNASYSLQDPSQVNKFLERLVEWKRKTVGEE

**SEQ ID NO: 15, DNA - Arabidopsis thaliana**

ACATTCACTCTCATTTCCATCAAAATTATAACTTCATTTTACATTTTGATTAATATCCTAAAATGG
TTAGATTCATCGAAGAAAATATTACTAAAATGTTGGAGACAAAGGCCATCTCAAACTCGGAGGTTT
TATATGTCGGAGGAGACGACGGAGACACGTCACCAACGACGAAAGTTCTTCATGATTTTCAGATCA
ACAGCGGAGGAGGACTGATAAGATCATGGGTTGATTCTATGAGAGCTTGTTCTCCTACTCGCCCTA

**FIGURE 8 (continued)**

AATCCTTCAATAGCCAATCTTGTTGGATTAAGGAACATCCATCTGCTTTGAACATGTTCGAAGAAA
TACTTCATAAATCTGAAGGAAAACAAATAGTTATGTTTCTTGATTATGATGGTACTCTCTCTCCCA
TTGTTGATGATCCTGATCGAGCTTTCATGTCCAAGAAGATGCGAAATACTGTGAGGAAACTTGCAA
AGTGTTTTCCAACAGCCATAGTTAGTGGGAGATGCAGAGAGAAGGTTTCTAGTTTTGTGAAATTAA
CTGAGTTATACTACGCTGGAAGTCATGGCATGGACATCAAAGGACCAGAGCAAGGATCTAAATACA
AGAAAGAAAATCAGTCTCTTCTTTGTCAACCCGCAACTGAATTCCTCCCGGTGATAAACGAGGTTT
ATAAAAAACTAGTCGAGAACACTCAATCGATTCCAGGAGCGAAAGTTGAGAACAACAAATTTTGTG
CGTCTGTTCACTTTCGATGCGTAGAAGAAAATAAATGGAGTGACTTGGCCCATCAAGTTCGATCAG
TCTTGAAGAATTACCCCAAGCTCATGCTTACCCAAGGAAGAAAAGTATTGGAGATTCGTCCAATCA
TTAAGTGGGATAAAGGCAAAGCACTCGAGTTTTTGTTAGAATCACTAGGATATGATAATTGTACCG
ATGTTTTTCCTATATATATTGGAGATGATCGTACCGACGAAGACGCCTTTAAGATACTGAGAGACA
AAAAACAAGGTCTTGGAATTCTTGTGTCCAAATACGCAAAGGAGACTAATGCTTCTTATTCTTTGC
AAGAGCCAGATGAGGTTATGGTTTTTTTAGAACGTTTGGTGGAATGGAAACAGTCAAGGTGTGGGG
CATGAAGAGTTTACAAGTACAGGAGAAACTTGTTCTTTTTCTTTTTTCTTTTTACTATGTAGTATT
GGTAAAGATTCTAACGTATAAGAGAACGAGAATAAGTTTATTAATATAACAAGAAGACAAATATTG
AGGTT

**SEQ ID NO: 16, protein - Arabidopsis thaliana**
MVRFIEENITKMLETKAISNSEVLYVGGDDGDTSPTTKVLHDFQINSGGGLIRSWVDSMRACSPTR
PKSFNSQSCWIKEHPSALNMFEEILHKSEGKQIVMFLDYDGTLSPIVDDPDRAFMSKKMRNTVRKL
AKCFPTAIVSGRCREKVSSFVKLTELYYAGSHGMDIKGPEQGSKYKKENQSLLCQPATEFLPVINE
VYKKLVENTQSIPGAKVENNKFCASVHFRCVEENKWSDLAHQVRSVLKNYPKLMLTQGRKVLEIRP
IIKWDKGKALEFLLESLGYDNCTDVFPIYIGDDRTDEDAFKILRDKKQGLGILVSKYAKETNASYS
LQEPDEVMVFLERLVEWKQSRCGA

**SEQ ID NO: 17, DNA - Arabidopsis thaliana**
TTTCTTCTCTTCCTCCTACATTCTCGACTCTCTCTTTTTATTAGGGTTTTCAATTTAATCAAAAA
AAAACCCTCAAATCTCATTTTTGGATTTGGTTTCTTCTGCGATCTCTGAGATTCAATTTCTTCTAT
CAAAGGAAACAAAAAACTTCATCTTTCCGAAGATTTTTTTTCCTTCCTTCTGGAGCTCTTTTGAAT
CAGGTGAATTTCTAATTGAATCTGAAAGTTTACGATGAACAATTTGATATGATGATAAGTTTTGTT
TGATGGAGCTTTCACCAACAAGCAGTGACAAGAAAACAATGAAGAGATGGTTTTTCATAGATAAAC
GAGTTGGATAGAGTGTTATTGAAGATAAAGAAAGCAAAAAACAGAAGTTCTTGCATTGAAGCATTG
GTTTTAGAGTTTATTGAAGCATTGTTTAGAGTTTTGATTAATGGATTTAAACTCAAACCACAAATC
CTCTGTTCTTAAAGATCCTTCACCATCAGTTAACCAATCAAGACTCGGCGTGTCTAGTAGATTTAT
GATGAGTCAATGGAAGAAACCTGCGAAACTCGATGATGTTAGATCCAATGGTTGGTTAGATGCAAT
GATTTCATCTTCTCCACCGCGTAAGAAGCTTGTCAAAGATTTTAATGTTGAAGTTGCTCCTGAAGA
TGATTTTGCTCAACGTGCTTGGATGGTGAAATATCCTTCGGCGATTAGCTCGTTTGCGCATATTGC
AGCTCAAGCAAAGAAGAAAAAGATTGCTGTATTTCTAGATTATGATGGTACTCTTTCTCCAATAGT
TGATGATCCTGATCGTGCCATCATGTCTGATGCAATGCGTTCTGCGGTTAAAGATGTCGCGAGTTA
CTTCCCAACCGCAATAATTAGCGGTAGAAGCCGTGACAAGGTTTATCAGTTGGTAGGACTAACAGA
ACTTTATTACGCGGGTAGTCATGGAATGGACATAATGACTTCTTCTGATGGTCCGAATTGTTTCAA
ATCCACTGACCAACAGGGTAAGGAAGTGAATCTGTTTCAGCCCGCGAGAGAATTCATACCGGTTAT
CGACGAGGTTTTTAGAACCCTTGTTGAGAAAATGAAAGATATCAAAGGTGCAAAAGTAGAGAACCA
CAAGTTCTGTGCATCTGTACATTACCGTAACGTTGACGAAAAGGATTGGCCTATTATTGCTCAGCG
TGTTCATGACCACTTGAAACAATACCCTCGTTTGCGTCTAACTCATGGGAGGAAGGTTTTAGAGGT
TCGTCCTGTGATAGACTGGAACAAAGGAAGAGCGGTCGAGTTTCTATTAGAATCTCTCGGATTAAG
CAATAAAGACGATTTGCTTCCTATCTACATTGGTGATGACACAACCGATGAAGATGCGTTCAAGGT
ACTGAGAGATGGGAACCGAGGTTTCGGCATCCTAGTATCGTCTATACCGAAAGAAAGCAATGCGTT

## FIGURE 8 (continued)

TTACTCCCTTAGAGATCCATCCGAGGTGAAGAAGTTTCTAAAGACTTTGGTGAAATGGGCAAAGTT
GGAGAAGAATTCTACTGGTTTTTGAATAATATAGTGGAATTTTATGATTAAATGCTTATTTACACT
TTTTTTTGTTTCTTTAATCTTTTTTCATCTCTACCCAGTTTTAGTCACAGCTTAATTTATGATTTT
CCATTTATCTGGTGGAAGTTAAGTTATTAAAGTTACATGT

**SEQ ID NO: 18, protein - Arabidopsis thaliana**
MDLNSNHKSSVLKDPSPSVNQSRLGVSSRFMMSQWKKPAKLDDVRSNGWLDAMISSSPPRKKLVKD
FNVEVAPEDDFAQRAWMVKYPSAISSFAHIAAQAKKKKIAVFLDYDGTLSPIVDDPDRAIMSDAMR
SAVKDVASYFPTAIISGRSRDKVYQLVGLTELYYAGSHGMDIMTSSDGPNCFKSTDQQGKEVNLFQ
PAREFIPVIDEVFRTLVEKMKDIKGAKVENHKFCASVHYRNVDEKDWPIIAQRVHDHLKQYPRLRL
THGRKVLEVRPVIDWNKGRAVEFLLESLGLSNKDDLLPIYIGDDTTDEDAFKVLRDGNRGFGILVS
SIPKESNAFYSLRDPSEVKKFLKTLVKWAKLEKNSTGF

**SEQ ID NO: 19, DNA - Arabidopsis thaliana**
GTGAGCTCTTTGCAATTTTTAGGGTTTTCTTCGAATAAAGAAAAAAAAAATCCCTGATCTTCCTCT
TAGTGATTTCTCCTTTCGAGATCCCTGAGATTCCGAGAAAATCTGCATTTTTTTCTCGTGATTCTC
TAGCTCTGTTTTGTGTTTTACACAGCTCTTTTGAATCAGGTTTGTGTGTGATAGCGTTGTTGTGGT
AAAAGCTTTGTACATTTGAAGATTTAGAGATGATAAGTTTTCAGGTAACCTACTTTTAATGGACAG
TTCAACAACAAGCAGTGATAAGAAAACACTAAAGAGGTGGTTTTTCATTGACAAAAGAGTTGGATA
AAGTGTGTTTAGAAGAAGCCGTCGAATTTCTTAGTTTGAAGCATTTTATTGTTTGGAGAGTTCGAT
GGATTTGAATATAAACAAGACGACCCCTGTTCTTAGTGACCCTACTACACCAGTAAGTAAAACAAG
ACTTGGATCATCCTTCCCTTCAGGGAGATTCATGATGAATTCTAGAAAGAAGATTCCTAAACTCGA
TGATGTTCGATCTAACGGTTGGTTGGACGCGATGATATCTTCGTCTCCACCGCGTAAAAGGCTTGT
CAAGGATTTCAATATTGAGATTGCTCCTGAAGATGATTTTTCTCAACGGGCCTGGATGCTCAAGTA
TCCTTCAGCGATTACCTCGTTTGCGCATATTGCAGCTCAAGCAAAGAACAAGAAGATAGCTGTGTT
TCTAGATTATGATGGAACACTTTCTCCAATAGTCGATGACCCTGATCGCGCCATCATGTCTGATGC
GATGCGTGCTGCTGTGAAAGATGTCGCCAAGTACTTCCCAACAGCAATAATTAGTGGTAGAAGCCG
TGACAAGGTTTACCAATTGGTAGGGCTAACCGAACTCTATTACGCGGGTAGTCACGGGATGGACAT
TATGACTCCTGTAAATCCAAATGGATCCCCTGAAGACCCTAATTGTATAAAAACCACTGACCAACA
GGGTGAGGAGGTAAACCTCTTTCAGCCTGCTAAAGAGTTCATACCCGTCATTGAAGAGGTTTATAA
TAACCTCGTTGAGATAACTAAATGTATCAAAGGTGCAAAAGTAGAGAACCATAAGTTTTGTACTTC
TGTACATTACCGTAATGTTGACGAGAAGGACTGGCCACTTGTTGCTCAACGCGTTCATGACCACCT
GAAACGATACCCTCGTTTGCGTATAACTCACGGTAGAAAGGTTTTAGAGGTGCGTCCTGTGATTGA
GTGGAACAAAGGAAAAGCAGTAGAGTTTCTGTTAGAGTCTCTCGGATTAAGCAACAACGATGAGTT
CCTCCCAATCTTCATCGGAGATGACAAGACCGATGAAGATGCATTCAAGGTACTGAGGGAAGGGAA
CAGAGGATTTGGAATATTGGTATCGTCTGTACCAAAAGAAAGCAATGCATTTTACTCTCTTAGAGA
CCCTTCCGAGGTGAAGAAGTTTCTCAAGACTTTGGTGAAATGGGGGAAGATGGAAAGTTCAAAAAC
AAGTTTTTGATGATTGTGTAGGAGAATCAGAAGCTTCATTCTGCACTGCTTTTTAATTTCTTATTT
TTTTTCCTTTTTTAAAGTTTGTACTTCTATATATTATCATTTTTCCATATTATCCATTAAGCAAAC
GGGAAAAACAAGAATATAACGATTGTCTTGACATTTT

**SEQ ID NO: 20, protein - Arabidopsis thaliana**
MDLNINKTTPVLSDPTTPVSKTRLGSSFPSGRFMMNSRKKIPKLDDVRSNGWLDAMISSSPPRKRL
VKDFNIEIAPEDDFSQRAWMLKYPSAITSFAHIAAQAKNKKIAVFLDYDGTLSPIVDDPDRAIMSD
AMRAAVKDVAKYFPTAIISGRSRDKVYQLVGLTELYYAGSHGMDIMTPVNPNGSPEDPNCIKTTDQ
QGEEVNLFQPAKEFIPVIEEVYNNLVEITKCIKGAKVENHKFCTSVHYRNVDEKDWPLVAQRVHDH
LKRYPRLRITHGRKVLEVRPVIEWNKGKAVEFLLESLGLSNNDEFLPIFIGDDKTDEDAFKVLREG
NRGFGILVSSVPKESNAFYSLRDPSEVKKFLKTLVKWGKMESSKTSF

## FIGURE 8 (continued)

**SEQ ID NO: 21, DNA - Arabidopsis thaliana**

GTCTCTCTCAAAGCCTACACACTCATTCTCACTCTGTTTCTACAACCCTAAATCTCTTTTCATTTA
CAGACAGAAATGGTTAGATTCATAGAAGAAAACACAAAACTTGTAGAAAAAGAAACCGGGAACAAA
TCAAACAACGATGTAACAACAACGAAGAAGAAAGCTCTTCAAGATATCATTATCAACAATGGAGTA
GGATTGATCAATTCATGGGTTGATTCAATGAGAGCTTGTTCCCCTACTCATCTTAAATCTTTGTTG
AAACAAAGCTCTTGGCTCACGGAACATCCATCAGCTTTGGATATGTTTGAAGAGATTCTTCACCTT
TCTGAAGGAAAACAAATCGTTATGTTCTTGGATTATGATGGCACTTTATCTCCCATTGTTGATGAT
CCAGATCGAGCTTTCATGTCTAGAAAGATGAGAAGAACTGTGAGGAAACTAGCAAACTGTTTCCCG
ACGGCCATAGTTAGTGGGAGATGCATAGAAAAGGTTTATAACTTTGTAAAACTAACTGAGTTGTAC
TATGCTGGAAGTCATGGAATGGACATCAAAGGACCAGAGCAAGGGTCCAAATATGAGCAAATTTTA
CAGGATAGTAAATCTCTTCTTTGCCAACCAGCTACAGAGTTCCTCCCCATGATCGACGAGGTTTAT
CATAAATTAGTGGAGAAAACAAAATCTACTCCCGGAGCCCAAGTAGAGAACAACAAATTTTGTGTC
TCTGTTCACTTCCGACGCGTAGATGAAAATAACTGGAGTGATTTGGCTAACCAAGTTCGATCAGTA
ATGAAAGACTACCCTAAGCTCCGTCTTACTCAAGGAAGAAAAGTTTTGGAGGTTCGTCCGATTATT
AAATGGGACAAAGGCAAAGCACTCGAGTTTTTATTAGAGTCACTTGGATACGCTAATTGTACCGAC
GTTTTCCCTCTTTATATCGGAGATGATCGCACCGACGAAGATGCATTCAAGGTATTGAGAGAACGA
AGACAAGGTCTTGGCATTCTTGTATCTAAATTTCCAAAGGAGACTAGCGCGTCTTATTCACTGCAA
GAACCCGATGAGGTTATGGAGTTCTTGCAACGTTTAGTGGAATGGAAACAATTGAGATCTGGAGCA
TGATGAGAGCTATAACTACAGAAGAATCGTTTCTTCTTAATTAGTAACTTGTTAATTTTATTTTAT
TTTCTTGCTCTCATATATTGTACTCAACGCGTATGTAAAATGTTTATAAGAAAATTGTAGATCCAT
CATTCTCAAGGAAAATATGTTTTATATAT

**SEQ ID NO: 22, protein - Arabidopsis thaliana**

MVRFIEENTKLVEKETGNKSNNDVTTTKKKALQDIIINNGVGLINSWVDSMRACSPTHLKSLLKQS
SWLTEHPSALDMFEEILHLSEGKQIVMFLDYDGTLSPIVDDPDRAFMSRKMRRTVRKLANCFPTAI
VSGRCIEKVYNFVKLTELYYAGSHGMDIKGPEQGSKYEQILQDSKSLLCQPATEFLPMIDEVYHKL
VEKTKSTPGAQVENNKFCVSVHFRRVDENNWSDLANQVRSVMKDYPKLRLTQGRKVLEVRPIIKWD
KGKALEFLLESLGYANCTDVFPLYIGDDRTDEDAFKVLRERRQGLGILVSKFPKETSASYSLQEPD
EVMEFLQRLVEWKQLRSGA

**SEQ ID NO: 23, DNA - Arabidopsis thaliana**

ATGTCAGCTAGTCAAAACATTGTCGTATCAGAGACTACAATGTCAAGTATCATCCCCAACAACAAC
AACAACAACAACAACTCTTCTTCACAGAAACTCCCTCCTTGTTTAATCTCAATTTCCAAGAAAAAG
CTTCTCAAGAACATCGACATCATCAATGGTGGTGGACAAAGAATCAACGCTTGGGTAGATTCAATG
CGTGCTTCTTCTCCTACTCATCTCAAATCTCTTCCTTCTTCTATCTCCACACAGCAACAACTCAAC
TCATGGATCATGCAACATCCTTCAGCACTAGAAAAATTCGAACAGATAATGGAAGCTTCGAGAGGG
AAACAAATCGTAATGTTTCTTGATTATGACGGTACTCTCTCTCCCATTGTTGATGATCCAGACAAA
GCTTTCATGTCAAGCAAGATGAGAAGAACAGTGAAAAAACTGGCTAAGTGTTTCCCCACTGCTATA
GTTACTGGTAGATGCATAGACAAGGTGTATAACTTTGTGAAGCTTGCTGAGCTGTATTATGCTGGA
AGCCATGGCATGGACATTAAAGGTCCAGCAAAAGGCTTCTCCAGACACAAGAGGGTTAAACAGTCT
CTTCTGTACCAACCAGCTAATGACTATCTTCCCATGATCGATGAAGTTTATAGACAACTCTTGGAA
AAAACAAAATCGACTCCAGGAGCCAAAGTAGAAAACCACAAGTTTTGTGCTTCTGTGCACTTTCGC
TGCGTCGATGAGAAGAAATGGAGCGAACTGGTTCTACAGGTTCGGTCGGTATTAAAGAAATTCCCT
ACGCTGCAACTGACCCAAGGTCGGAAGGTTTTCGAAATCCGTCCAATGATTGAATGGGATAAAGGA
AAGGCTCTTGAGTTCTTGTTAGAATCACTTGGATTTGGGAACACTAACAATGTTTTCCCGGTTTAT
ATTGGTGACGATCGAACTGACGAAGATGCATTTAAGATGCTACGAGACAGAGGCGAAGGCTTTGGC
ATTCTTGTCTCCAAGTTTCCCAAGGATACTGATGCTTCGTATTCTTTGCAAGATCCATCCGAGGTG
ATGGATTTCTTACGACGATTGGTGGAATGGAAACAAATGCAGCCAAGAATGTGAAATGATAATATA

**FIGURE 8 (continued)**

ATAAACGAGTGACAATATTCGAAATGCCAATGGATGTATATAATGAATATGTTCATGCCTAACTTT
TTTAGGAACGTTGTAATTGGTAAAGGATATGGCAGTGTCTTTAACGTACCCCCATTAGGTTAATCT
TCTTGTTCGTCACATTGTATTTTTGT

**SEQ ID NO: 24, protein - Arabidopsis thaliana**
MSASQNIVVSETTMSSIIPNNNNNNNNSSSQKLPPCLISISKKKLLKNIDIINGGGQRINAWVDSM
RASSPTHLKSLPSSISTQQQLNSWIMQHPSALEKFEQIMEASRGKQIVMFLDYDGTLSPIVDDPDK
AFMSSKMRRTVKKLAKCFPTAIVTGRCIDKVYNFVKLAELYYAGSHGMDIKGPAKGFSRHKRVKQS
LLYQPANDYLPMIDEVYRQLLEKTKSTPGAKVENHKFCASVHFRCVDEKKWSELVLQVRSVLKKFP
TLQLTQGRKVFEIRPMIEWDKGKALEFLLESLGFGNTNNVFPVYIGDDRTDEDAFKMLRDRGEGFG
ILVSKFPKDTDASYSLQDPSEVMDFLRRLVEWKQMQPRM

**SEQ ID NO: 25, DNA - Arabidopsis thaliana**
GATACAATCTTAGTAATCACACTTGAGTATTTTTCTTAACTGGTTTGTTTCTTGGCATCTTCATT
TCAGGGTCTATATTTCTCTGAGGAATTGGATTCTTATAGAAGTTAAACCGCAGAGCTTCTTCTATC
CAATAGTCCTCTGAGAATCGTCGATATAGTTTGCTGATATAGTTGTTATGGACATGAAATCTGGTC
ACTCGTCTCCTGTAATGACTGATTCTCCACCAATAAGCAACTCAAGATTAACCATTCGTCAGAATA
GACTTCCTTACTCATCAGCAGCAGCCACGGCTATCTCACAGAACAACAATCTCTTACTAACCGTTC
CAAGAAAGAAAACTGGGATCCTTGATGATGTTAAGTCTAATGGTTGGCTTGATGCGATGAAATCTT
CTTCTCCTCCTCCTACAATACTTAACAAAGATAACTTAAGCAATGATGCTACGGATATGACTTATC
GCGAATGGATGCAGCTCAAGTATCCATCAGCTCTTACCTCTTTTGAGAAAATCATGAGTTTTGCAA
AAGGCAAAAGAATAGCATTGTTTCTTGATTATGACGGGACACTTTCGCCTATTGTTGAGGAACCTG
ATTGTGCATACATGTCAAGTGCTATGCGTAGTGCAGTGCAAAATGTTGCCAAGTATTTCCCTACCG
CGATCATTAGTGGAAGAAGCCGGGATAAGGTGTATGAGTTTGTTAATTTGAGTGAACTTTATTACG
CCGGAAGCCATGGAATGGACATCATGAGTCCCGCAGGAGAATCTTTAAACCATGAACATAGCCGTA
CTGTATCAGTTTACGAACAGGGGAAAGATGTAAATCTATTCCAGCCTGCTAGCGAGTTTCTCCCGA
TGATCGATAAGGTGCTTTGTTCTCTTATAGAGAGTACAAAAGATATCAAAGGGGTAAAAGTAGAAG
ACAACAAGTTCTGCATCTCTGTGCATTACCGCAATGTAGAAGAAAAGAACTGGACATTGGTTGCAC
AGTGTGTAGATGATGTCATCAGAACATATCCAAAACTACGGCTAACACATGGCCGGAAGGTTTTAG
AGATCCGTCCTGTGATTGACTGGGACAAAGGGAAAGCTGTGACATTTCTACTTGAATCACTCGGCC
TAAACAACTGTGAGGATGTTCTTCCAATCTATGTTGGGGATGATCGAACAGACGAAGATGCATTTA
AGGTACTACGAGATGGACCAAACCACGGTTATGGTATATTAGTCTCTGCTGTGCCTAAAGACAGCA
ATGCCTTTTACTCGCTTCGTGACCCGTCTGAGGTGATGGAGTTTCTGAAGTCATTGGTGACATGGA
AGAGATCAATGGGTTAAAGTAGAAGAAGAAGATGTAGAAGAAAAGTGGTTAACGTTAATATATATT
ACAAATATGCAAGTTAGAGGTTTATTGTTACTTTTCGGCTTTATACAAAAACTACCGTTGAGATTT
AACTTAACGGTATCGGAGGAGAGATCACATGATGGCTTAAAACATGCCGGAGATACTTTTTCCGAC
CAATCTCTTTATCTTCTACAACTGTAAATTCCTCCATCTC

**SEQ ID NO: 26, protein - Arabidopsis thaliana**
MDMKSGHSSPVMTDSPPISNSRLTIRQNRLPYSSAAATAISQNNNLLLTVPRKKTGILDDVKSNGW
LDAMKSSSPPPTILNKDNLSNDATDMTYREWMQLKYPSALTSFEKIMSFAKGKRIALFLDYDGTLS
PIVEEPDCAYMSSAMRSAVQNVAKYFPTAIISGRSRDKVYEFVNLSELYYAGSHGMDIMSPAGESL
NHEHSRTVSVYEQGKDVNLFQPASEFLPMIDKVLCSLIESTKDIKGVKVEDNKFCISVHYRNVEEK
NWTLVAQCVDDVIRTYPKLRLTHGRKVLEIRPVIDWDKGKAVTFLLESLGLNNCEDVLPIYVGDDR
TDEDAFKVLRDGPNHGYGILVSAVPKDSNAFYSLRDPSEVMEFLKSLVTWKRSMG

## FIGURE 8 (continued)

**SEQ ID NO: 27, DNA - Arabidopsis thaliana**
GAACACTTCAAAAAACATAGCAACTTCACTTCTTCCTCCTCCTTCCTAGTTTCTCGTTTGGTCTCA
TTCTCTTTAATTACCTCCTCCTCTGTTCTGCTCATCCCCTGTTTTTATAGTTTTATATAAAATGGT
GAGCCAAAACGTCGTCGTATCAGACGCCAAGACCGGGATCATAACGGTCTCTACAGTCTCTAACTC
CTCTGTCTTTACTCCTACCGCTCAAAAACCACCGACTGCTCCTGGTTACATCTCAGTTTCCAAGAA
AAAACTCCTCAAAAACCTTGAAATCAATGGAGCTGATCAAAGTCAAAGACTTAACTCTTGGGTTGA
CTCCATGAGAGCTTCTTCTCCTACCCATCTCAAATCACTTTCCTCTTTCTCTTCCGAAGAAGAACA
CAATTCTTGGATCAAACGACATCCATCAGCACTTAACATGTTCGAACGAATCATCGAAGAAGCAAG
AGGAAAACAAATCGTCATGTTTCTTGATTATGACGGTACTCTTTCTCCGATCGTGGATGATCCAGA
CAGAGCTTTCATGACTAGCAAGATGAGAAGAACAGTGAAAAAAATGGCTAAGTGTTTTCCAACTTC
CATAGTTACTGGTAGATGCATCGACAAGGTTTATAGCTTTGTGAAGCTAGCAGAACTGTATTATGC
TGGAAGCCATGGGATGGATATTAAAGGCCCAACTAAAGGTTTCTCAAGATACAATAAGGATAAACC
ATCCGTTCTTTACCAACCAGCCGGCGACTTTCTTCCCATGATCGATGAGGTTTATAAACAATTAGT
GGAGAAAACCAAATCTACACCAGGAGCCAAAGTGGAGAACAACAAGTTCTGTCTTTCTGTGCACTT
CCGTTGTGTTGACGAGAAGAAATGGAGCGAGCTGGCTTCAAAAGTTCGGTCGGTGGTAAAGAACTA
TCCGACGTTGAAACTGTCTCAAGGTCGAAAGGTTTTTGAAATCCGACCTATAATTAAATGGAACAA
AGGCAAGGCTCTTGAGTTTTTGTTAGAGTCACTCGGATTTGAAAACTGTAACGATGTATTTCCAAT
TTACATTGGTGATGATAAAACCGATGAAGATGCATTTAAGCTACTACGAGGGAGAGGACAAGGCTT
TGGTATTCTTGTCTCCAAGTTCCCCAAAGACACCAGTGCGTCGTATTCTTTACAAGACCCACCTGA
GGTGATGAATTTCTTGGGACGGTTGGTGGAGTGGAAACAGATGCAGCAATAAACGTGCAAGGGGCA
ATGAAGTACATTTAGTAGTCTTTTTAATAAAACGAGAATATTCATAACTATAGGTGCATATATAGA
TATAATATGTACATCCCTAACCTATAATGGAACATTGTACAAGATCCTCTTATAGATTGATCTTGT
TCATCACAAATCTTTTCTTTTAGTAAAAGAGATTATATGAACAAGAAAAATGGGGAACTTTTATCG
ATTTGGGGATATATTATTATAGCCTTCTATCTTTATTGCTTGTAGGCAATTTCTTACTTTTCAAGT
TAGGCTATTGTA

**SEQ ID NO: 28, protein - Arabidopsis thaliana**
MVSQNVVVSDAKTGIITVSTVSNSSVFTPTAQKPPTAPGYISVSKKKLLKNLEINGADQSQRLNSW
VDSMRASSPTHLKSLSSFSSEEEHNSWIKRHPSALNMFERIIEEARGKQIVMFLDYDGTLSPIVDD
PDRAFMTSKMRRTVKKMAKCFPTSIVTGRCIDKVYSFVKLAELYYAGSHGMDIKGPTKGFSRYNKD
KPSVLYQPAGDFLPMIDEVYKQLVEKTKSTPGAKVENNKFCLSVHFRCVDEKKWSELASKVRSVVK
NYPTLKLSQGRKVFEIRPIIKWNKGKALEFLLESLGFENCNDVFPIYIGDDKTDEDAFKLLRGRGQ
GFGILVSKFPKDTSASYSLQDPPEVMNFLGRLVEWKQMQQ

**SEQ ID NO: 29, DNA - Populus trichocarpa**
ATATGCAGAGTTTAGTTTACACAAGCGGAGATGACAAACCAGAATGTGGTTGTGCCTGATGCCAAA
CAGGGTATTGATATCACCATCGCAATGGTGCTGCCCAAGTCTCTGTTTTCACCTGTTGTGCCAAAG
CCATTGCCTGCCGCTCCTGGTGGGTATTTCACAATAACCCGAAAGAGGTTCTCGAAGAAGACAGAA
ACTGGAGGCAAAATCAGTTCCTGGGTTGATTCTATGAGGGATTCTTCACCTACCCGTGTCAAATCC
ACCACTTCTTTATCAGAAACTGAAGAGAAAAATTCTTGGATTGTAAACCATCCTTCTGCTCTGAAC
ATGTTTGAGCAAATAGTGAAAGGCTCAAAGGGAAAGCAGATTGTGATGTTCCTTGATTATGATGGT
ACACTGTCACCCATTGTTGAAGATCCTGACAGAGCATTCATGACCAATGAGATGAGAGAAGCTGTC
AGGGACGTTGCTAGATACTTTCCCACGGCTATAGTGACGGGAAGGTGTAGAAAAAAGGTGTATAGC
TTTGTAAGATTGGCAGGGCTTTATTATGCTGGTAGCCATGGCATGGACATCAAGGGACCATCCAAA
AATAATTGCAAATACGAAAAAGGTGGTGTACTCTTTCAACCTGCCAGTGAATTTTTACCCATGATT
GATGAGGTGTACAATGTCTTGTTGGAGAGAACAAAATCTATCCCAGGAGCTAAAGTAGAAAACAAC
AAATTTTGCGTATCCGTACACTTTCGATGTGTTGAGGAAAAGATGTGGGCTATATTAGTAGAGCAA
GTCCGATCAGTTCTCAATGATTATCCCAAGCTTAGATTAACTCAAGGGAGGAAGGTTTTAGAGATC

**FIGURE 8 (continued)**

CGACCCACCATTAAATGGGACAAGGGCAAAGCTCTTGAATTCTTGTTAGAGTCATTAGGATATGCC
AATTCTACTGACGTTTTTCCGGTCTATATTGGAGATGATCGAACTGATGAGGACGCATTCAAGGTT
CTAAGAAACAGGGGGCAAGGGCTTGGGATTCTTGTTTCTAAAGTTCCCAAGGAAACAAATGCCTCT
TATTCTCTACAGGAACCAAAAGAGGCAAGTTCTTTAATGTAACAAGCAAGTTGTTATACTTTTATT
TGTGGTAATAATTATTGTTAAATTTTCAGTTTTGCAAGTTCGACTAATAAATCATA

**SEQ ID NO: 30, protein - Populus trichocarpa**
MTNQNVVVPDAKQGIDITIAMVLPKSLFSPVVPKPLPAAPGGYFTITRKRFSKKTETGGKISSWVD
SMRDSSPTRVKSTTSLSETEEKNSWIVNHPSALNMFEQIVKGSKGKQIVMFLDYDGTLSPIVEDPD
RAFMTNEMREAVRDVARYFPTAIVTGRCRKKVYSFVRLAGLYYAGSHGMDIKGPSKNNCKYEKGGV
LFQPASEFLPMIDEVYNVLLERTKSIPGAKVENNKFCVSVHFRCVEEKMWAILVEQVRSVLNDYPK
LRLTQGRKVLEIRPTIKWDKGKALEFLLESLGYANSTDVFPVYIGDDRTDEDAFKVLRNRGQGLGI
LVSKVPKETNASYSLQEPKEASSLM

**SEQ ID NO: 31, DNA - Populus trichocarpa**
GTGTGCAGAGTTTAGTTTATACAAACAGAGATGACAAACCAGAATGTGGTTGTGCCTAATGCCAGA
AAAGGTGTTGATATCACCATCACAATGGCCCTGTCCAAGTCTCTGTTTTCACCTGTTGTGCCGAAG
CCATTGCCAGCTGCTCCTGGTGGGTATTTCACAATATCCCGGAAGATGTTCGCAAAGAAGACTGAA
ACTGGAGGCAAAACCAATTCCTGGGCTGATTCTATGAGAGATTCTTCACCTACCCGTGTCAAATCC
ACTACGTCTTTATCAGAAATCGAGGAGAAAAATACTTGGATTGTAAATCATCCTTCTGCTTTGAAC
ATGTTCGAGCAAATAGTGAATGGCTCAAAGGGAAAACAGATTGTAATGTTTTTAGATTATGATGGT
ACACTGTCACCCATTGTTGAAGATCCTGACAGGGCATTCATGACCAACGAGATGAGAGAAGCTGTT
AGGGACGTCGCTAGATACTTTCCCACTGCTATAGTGACGGGAAGGTGTAGGGATAAGGTGTACAGC
TTCGTAAGATTGGCAGGGCTTTATTACGCTGGTAGCCATGGCATGGACATCAAGGGACCATCCAAG
AATTGTTGCAGAAACAAAAAAGATTATCAAGGTGTACTTTTTCAACCTGCCAGTGATTTTTTACCC
ATGATTGATGAGGTGTACAATGCTTTGCTGGAGAGAACAAAGTATATCCCAGGGGCTAGAGTAGAA
GACAACAAATTTTGCATATCCGTACACTTTCGTTGTGTCGAGGAAAAGATGTGGGCTGCATTAGTA
GAGCAAGTAAGATCAGTTCTCAATGGTTATCCAAAACTTCGATTAACTCAAGGGAGGAAGGTTTTA
GAGATCCGACCCACCATTAAATGGGACAAGGGCAAAGCTCTTGAGTTCGTGTTGGAATCATTAGGA
TATGCCAATTCTACTGATGTTTTACCAGTTTATATTGGAGATGATCGAACTGATGAGGATGCATTC
AAGGTTCTAAGAAACAGGGGACAAGGGCTTGGGATTCTTGTTTCTAAAGTTCCCAAGGAAACAAAT
GCCTCGTATTCTCTACAAGAGCCAACAGAGGCAAGTTCTTTAATCTAACAAGCAAAGGGGTTATGA
TTAATTTCTGGTGCTAATTATGGTTGATTTTCAATCTTACAAGATGGACTGATGAATTATGT

**SEQ ID NO: 32, protein - Populus trichocarpa**
MTNQNVVVPNARKGVDITITMALSKSLFSPVVPKPLPAAPGGYFTISRKMFAKKTETGGKTNSWAD
SMRDSSPTRVKSTTSLSEIEEKNTWIVNHPSALNMFEQIVNGSKGKQIVMFLDYDGTLSPIVEDPD
RAFMTNEMREAVRDVARYFPTAIVTGRCRDKVYSFVRLAGLYYAGSHGMDIKGPSKNCCRNKKDYQ
GVLFQPASDFLPMIDEVYNALLERTKYIPGARVEDNKFCISVHFRCVEEKMWAALVEQVRSVLNGY
PKLRLTQGRKVLEIRPTIKWDKGKALEFVLESLGYANSTDVLPVYIGDDRTDEDAFKVLRNRGQGL
GILVSKVPKETNASYSLQEPTEASSLI

**SEQ ID NO: 33, DNA - Populus trichocarpa**
ATTTTGGTTTTTGTTTTTTCTAGTGGAAAGATGACAAACCAAAATGTGGTAGTGGCTGATACAAAC
TCTGGACTCAACTTGGCAATCACAGTGCATGTAACAAACTCCTCTATCTTCACAACGGCGGCCCAG
AAACCTCCAGCGGCACCAGGTGGTTACATATCCATTTCTAGAAAGAAACTCTTAAAGAATCTGGAA
ATCAATGGAGGAGCAAGAATTAATGCTTGGGTTGATTCCATGAGAGCCTCATCTCCTACTCATATC
AAGTCCACGCCTTCTGTTAATGAAGACCAAAGCTCATGGATTCTTCACCACCCATCAGCACTGGAG

**FIGURE 8 (continued)**

ATGTTTGAGCAGATAATTGATGCCTCTAAAGGAAAGCAAATTGTTATGTTCTTGGACTATGATGGC
ACACTTTCTCCTATTGTTGATGACCCAGATAAAGCTTTCATGTCCAAGCAGGTGATGAGAGCAACA
GTGAGAAAGCTTGCAAGATTTTTCCCTACTGCAATAGTGAGTGGGAGGTGCAGAGACAAGGTGTAT
AACTTTGTACGGTTAGCAGAACTGTACTATGCTGGAAGCCATGGCATGGACATTAAAGGACCAGCA
AAAGGCTCCAAATACAAGAAAGTGAGTGAGCTTTACCACTGTATTTGCAAGTACCATCCAGGCGGT
GATGGTGTTGTCTTTCAGGCTGCCAGTGAATTTCTTCCCATGATAGATGAGGTTTACGAAGAATTG
GTAGAGAAAACTAAAACAACTCCAGGGGCCAAGGTGGAGAACAACAAATTCTGCCTCTCTGTGCAC
TATCGCTGCGTTGATGAGAAAAAATGGAGTGGACTGGCACAAGTAGTTAAGTCAGTGTTGAAGGAG
TACCCAAAGCTTCGACTTACTCAAGGAAGAAAGGTTTTAGAAATCCGCCCTACCATTAAATGGGAC
AAAGGAAAGGCTCTTGAATTTTTGTTAGAGTCACTTGGATTCGCCAATTGCACTGATGTTTTTCCT
GTTTACATTGGAGATGATAGAACAGACGAAGATGCATTTAAGGTACTAAGAGAGAGAGGACAAGGT
TTTGGTATCTTGGTCTCTAAAATCCCAAAGGACACTAGTGCATCTTATTCCCTACAGGAACCCACC
CAGGCAAGTTATGGATTTCTTGCGACGTTTGGTGGAGTGGAAACGGCTGGCCTTTCAAGGGCGGTC
AAGGGTGGTGTAAAGAGTAGTGGGAAGATTGAGACAGTACAGATATACCCACCCCTTCAAGAAATG
TAATTAAGGGTGGGATGTTAGACTATGTGATCATCTCTTGTTCAAGATTAAAAAGTATGAACAAGA
AAAGTAGGGAAATATAA

**SEQ ID NO: 34, protein - Populus trichocarpa**
MTNQNVVVADTNSGLNLAITVHVTNSSIFTTAAQKPPAAPGGYISISRKKLLKNLEINGGARINAW
VDSMRASSPTHIKSTPSVNEDQSSWILHHPSALEMFEQIIDASKGKQIVMFLDYDGTLSPIVDDPD
KAFMSKQVMRATVRKLARFFPTAIVSGRCRDKVYNFVRLAELYYAGSHGMDIKGPAKGSKYKKVSE
LYHCICKYHPGGDGVVFQAASEFLPMIDEVYEELVEKTKTTPGAKVENNKFCLSVHYRCVDEKKWS
GLAQVVKSVLKEYPKLRLTQGRKVLEIRPTIKWDKGKALEFLLESLGFANCTDVFPVYIGDDRTDE
DAFKVLRERGQGFGILVSKIPKDTSASYSLQEPTQASYGFLATFGGVETAGLSRAVKGGVKSSGKI
ETVQIYPPLQEM

**SEQ ID NO: 35, DNA - Populus trichocarpa**
TCACAAGTACTACCAAGTAATTTGGCTACCATGGATATCGAGTCAAACCATTCTTCTCCTGTTCTC
ACTGATCCTGCTCCAATAAACAAGTCAAGGCTCGGCATCCATTCTAATTTGTTGTCTTACGCACCA
TCGGGAGGATCACTCTCCTCTAGCAAGTATAGAAACGTTAACATTCCTAGAAAAAAGCCTGGGAAA
CTTGATGAAGTCTGCTCGAACGCATGTCTGGATGCCATGAAATCCTCATCACCCCCTCGCAAGAAG
CTCATTAAGGATGGTGCTGACACAGCTTACGGCACCTGGATGCTCAAGCATCCATCAGCACTCAAC
TCATTTGAGGAAATTGCAAATTTTGCCAAAAACAAAAAGATAGCAATGTTTCTAGACTACGATGGT
ACTCTCTCTCCAATAGTAGATGACCCAGATAATGCCCTTATGTCTGATGATATGCGTTTTGCAGTA
AGAAACTTCGCAAAATATTTCCCAACGGCGATTATTAGTGGAAGAAGTCGTGACAAGGTTTATCAG
CTTGTAGGACTAACAGAATTGTATTATGCTGGTAGTCATGGGATGGACATCTTGGGCCCTGTACGA
AAAGCGGTGTCCAATGACCATCCAAACTGTAATGAATCAACTACTGACCAACAGGGCAAGGAGGTG
AATCTGTTTCAGCCTGCTAGAGAATTTATACCTCTGATCGATGAGGTTTTTAGAACCCTTGTCGAG
GATACTAAGGGGATCAAAGGTGCAAAAGTTGAGAATCATAAATTTTGCGTCTCTGTACATTTCCGT
AATGTAGATGAGAAGAACTGGCAATCTATTGCACAATGTGTTCAGGATATTTTAGATAAGTATCCT
CGTTTGCGAAAAACTCATGGACGGAAGGTTTTAGAGGTCCGTCCAATGATTGACTGGAATAAAGGA
AAAGCAGTTGAATTTTTGCTTGAATCTCTAGGTCTAAGTAATAGAGATGATGTACTCCCAATTTAT
ATTGGTGATGATCTGACAGATGAAGATGCATTCAAGGTGCTCCGGGAGGGGAATCGAGGCTGTGGA
ATTTTGGTGTCATCTAGACCCAAAGAAACCAATGCAGTTTACTCTCTCAGAGATCCATCGGAGGTG
ATGAAATTTCTCAATTCCCTGGTGACATGGAAGAAGGTGGATGCATCCTGAATAATAGGAGGAACA
TCTGATGACATGCAGAGATAATTTAGAAATTTGATTTTGAATATTTTATAGATATTTTGCAAAAT

**FIGURE 8 (continued)**

**SEQ ID NO: 36, protein - Populus trichocarpa**

MDIESNHSSPVLTDPAPINKSRLGIHSNLLSYAPSGGSLSSSKYRNVNIPRKKPGKLDEVCSNACL
DAMKSSSPPRKKLIKDGADTAYGTWMLKHPSALNSFEEIANFAKNKKIAMFLDYDGTLSPIVDDPD
NALMSDDMRFAVRNFAKYFPTAIISGRSRDKVYQLVGLTELYYAGSHGMDILGPVRKAVSNDHPNC
NESTTDQQGKEVNLFQPAREFIPLIDEVFRTLVEDTKGIKGAKVENHKFCVSVHFRNVDEKNWQSI
AQCVQDILDKYPRLRKTHGRKVLEVRPMIDWNKGKAVEFLLESLGLSNRDDVLPIYIGDDLTDEDA
FKVLREGNRGCGILVSSRPKETNAVYSLRDPSEVMKFLNSLVTWKKVDAS

**SEQ ID NO: 37, DNA - Populus trichocarpa**

AACAACTCTGCTGCAAGTAATTTGGCTACGATGGATGTCGAGTCAAATCATTCTTCTCCTGTTCTC
GCTGACCCTGCATCGATAAACAGTTCAAGGCTCGGTATCTATTCTAATCTGTTGCCTTACTCACCA
CCAGGAGGATCACTCTCCTCTAGCAAGTATAGTAGGAAAAAGCCAGGGAAGCTTGACGAAGTCTGC
TCGAGTGGATGGCTGGATGCCATGAAATCTTCATCACCCCCTCGGAAGAAGCTCTTTAAGGATGGT
TCTGACACCGCTTACAGTTCCTGGATGTTCAAGCATCCATCAGCACTCAATTCATTTGAGGAAATT
GCAAATTTTGCTAAAAACAAGAAGATAGCAATGTTTCTAGACTATGATGGGACTCTTTCTCCAATT
GTAGATGACCCGGATAATGCGTTTATGTCTGATGATATGCGTTCTATTGTAAAAAACGTTGCGAAG
TATTTCCCAACGGCGATTATTAGTGGAAGAAGTCGTGACAAGGTTTATCAGCTGGTAGGACTAACA
GAACTATATTATGCTGGTAGTCATGGGATGGACATTTTGGGCCCTGTAGGAAAAGCTTCAATGTCC
AATGATCATCCAAACTATAGTGAATCTACTACTGACCAACAGGGCAAGGAGGTGAATCTGTTCCAG
CCTGCTAGAGAATTTATACCTATGATCGATGAGGTTTTTAGAACCCTTGTCGAGAATACTAAGGGA
ATCGAGGGTGCAAAAGTCGAGAATCACAAATTTTGTGCCTCTGTGCATTTCCGAAATGTAGATGAG
GAGAACTGGCAACCTATTGCACAATGTGTTCAGGATATTCTAGATAAGTACCCTCGTTTGCGGAGA
ACTCATGGACGGAAGGTTTTAGAGGTCCGTCCAATGATTGACTGGAATAAAGGGAAGGCAGTTGAA
TTCCTGCTTGAATCTCTAGGGCTAAGTAACAGAGACGACGTGCTCTCAATTTATATCGGCGATGAT
CTGTCAGATGAGGATGCGTTCAAGGTGCTCCGGGAGGGGAATCGAGGTTATGGAATTCTCGTATCA
TCTAGACCCAAAGAAACCAGTGCAGTTTACTCTCTCAAAGATCCAATTGAGGTGATGAAATTTCTT
AACTCCTTGGTGACATGGAAGAAGGTAGAAGAAGGTGGATGCATCCTGAATAACAGGAGGACTATC
TGATGACATGCATATAGCTTAGGTTTTTGATTTCGAGCATTTTAATTATTGATATTACATAATAAA
GAGGCTGTTGGTACAGC

**SEQ ID NO: 38, protein - Populus trichocarpa**

MDVESNHSSPVLADPASINSSRLGIYSNLLPYSPPGGSLSSSKYSRKKPGKLDEVCSSGWLDAMKS
SSPPRKKLFKDGSDTAYSSWMFKHPSALNSFEEIANFAKNKKIAMFLDYDGTLSPIVDDPDNAFMS
DDMRSIVKNVAKYFPTAIISGRSRDKVYQLVGLTELYYAGSHGMDILGPVGKASMSNDHPNYSEST
TDQQGKEVNLFQPAREFIPMIDEVFRTLVENTKGIEGAKVENHKFCASVHFRNVDEENWQPIAQCV
QDILDKYPRLRRTHGRKVLEVRPMIDWNKGKAVEFLLESLGLSNRDDVLSIYIGDDLSDEDAFKVL
REGNRGYGILVSSRPKETSAVYSLKDPIEVMKFLNSLVTWKKVEEGGCILNNRRTI

**SEQ ID NO: 39, DNA - Populus trichocarpa**

GGTTTTTTTTTTTTTTTTTGTAGAGGAAAGATGACAAACCAAAATGTGGTAGTGGCTGATACAAAC
TCTGGAATCAACTTGGCAATCACAGTGCATGTAACAAATTCCTCTATCTTCACCACGGCCGCGCAG
AAGCCTCCGGCGGCTCCTGGTGGTTACATCTCTATTTCCAGAAAGAAACTTTTAAAGAATCTTGAA
ATCAGTGGAGGAGCAAGATTTAATGCTTGGGTTAATTCCATGAGAACCTCTTCTCCTACTCATGTC
AAGTCCACACCTTCTGCTAATGACGACCAAAGCTCATGGATTCTTCACCACCCATCAGCACTGGAG
ATGTTTGAGCAGATAATTGATGCCTCCAAAGGAAAGCAAATAGTTATGTTCTTGGACTATGATGGC
ACACTTTCTCCTATCGTTGATGACCCAGATAGAGCTTTCATGTCCAAGAAGGTTGATGAGAGCAAC
AGTGAGAAAGCTTGCGAGATTTTTTCCTACTGCAATAGTGAGTGGGAGATGCAGAGACAAGCAGAA
CTGTACTATGCTGGAAGCCATGGCATGGACATCAAAGGACCAGCCAAAGGCAGCAAATACAAGAAA

**FIGURE 8 (continued)**

GGCAGTGAAGGTGTTGTTTTTCAAGCTGGCAGTGAGTTTCTTCCAATGATAGATGAGGTTTACAAA
GAACTGGTAGAGAAAACTAAAACAACTCCAGGGGCCAAGGTGGAAAATAACAAATTCTGTCTCTCT
GTGCACTATCGCTGCGTTGATGAGAAGAAATGGAGTGGACTGGCTCAAGTAGTTAAGTCAGTGTTG
AAGGAGTACCCAAAGCTTCGACTTACTCAAGGAAGAAAGGTTTTAGAAATCCGCCCTACCATTAAA
TGGGACAAAGGAAAGGCTCTTGAATTTTTGTTAGAGTCTCTTGGATTCGCCAATTGTACTGATGTT
TTTCCTGTTTACATTGGAGATGATAGAACAGACGAGGATGCATTTAAGGTGCTGAGAGAGAGAGGA
CAAGGTTTTGGTATCTTGGTCTCTAAATTCCCCAAGGACACTAGTGCATCTTATTCCCTACAAGAA
CCCACCCAGGCAAGTAGTATCCAATACATGCATAATTGCTGTCATGGATTTTTTGCAACGTTTGGT
GCAGTGGAAACGGCTAGCCTTTCAAGGGCAGTCAAGGGTTTAAGATGGAGTAGGAAAATCGAAACA
GTATATATATATATATACCCACCCCTTCAAGAAATGTAATTAAGGGTGGGATCGATGTTAGACCAC
GTAATTTCTTGTTCAGGTTAAAAAAATATGAACAAGAAAATAGGAAGGGAAAA

**SEQ ID NO: 40, protein - Populus trichocarpa**
MTNQNVVVADTNSGINLAITVHVTNSSIFTTAAQKPPAAPGGYISISRKKLLKNLEISGGARFNAW
VNSMRTSSPTHVKSTPSANDDQSSWILHHPSALEMFEQIIDASKGKQIVMFLDYDGTLSPIVDDPD
RAFMSKKVDESNSEKACEIFSYCNSEWEMQRQAELYYAGSHGMDIKGPAKGSKYKKGSEGVVFQAG
SEFLPMIDEVYKELVEKTKTTPGAKVENNKFCLSVHYRCVDEKKWSGLAQVVKSVLKEYPKLRLTQ
GRKVLEIRPTIKWDKGKALEFLLESLGFANCTDVFPVYIGDDRTDEDAFKVLRERGQGFGILVSKF
PKDTSASYSLQEPTQASSIQYMHNCCHGFFATFGAVETASLSRAVKGLRWSRKIETVYIYIYPPLQ
EM

**SEQ ID NO: 41, DNA - Populus trichocarpa**
ATAAAAAATACTCTTACGAATCTGAATCCGATGGACCTCAAATCAAATCATAATGCTCCTGTGCTC
ACTGATTCTGCTCCCCTAAGCAAGTCAAGGCTACGCGGGTACCACCATGGTTTGATGCTGCCGTAC
TCACCCTCAGGTGCACCTTTCTCGTCAAATCTATTACTATCTATTCCTAGGAGGAAAACTGGAGTG
CTTGATGATGTTCGCTCCTGTGGTTGGCTGGATGCCATGAAATCATCATCTCCTACTCATAAGAAG
TTTGCCAAGGATATTAACCATGAGCTTTCCGCACCTGATCCAGAAGTTGCCTATCGCACCTGGCTG
CTTAAATATCCATCTGCGCTTGCATCTTTCGAGCAAATTGCAAACTTTGCAAAAGGCAAGAGAATC
GCCTTATTTCTGGATTATGATGGTACTCTTTCACCAATTGTTGAAAATCCTGACAATGCCTTCATG
TCTGCTGATATGCGTTCCATTGTAAAGGAAGTGGCAAAATATTTCCCAACAGCAATAATCAGTGGA
AGAAGCCGTGACAAGGTGTATGAGTTTGTAGGGCTTACAGAACTCTACTATGCGGGTAGTCATGGT
ATGGATATCATGGGCCCTGTCAGGCAATCTGTATCTGACGACCACCGAAATTGTATCAAGTCTACG
GACAAGCAGGGCAACGAAGTTAACTTATTCCAGCCTGCAAGAGAATTTTTACCTATGATTGACGAG
GTTTATAGTTCCCTTGTCAGGATTACCGAAGATATTAAAGGGGCAACAGTTGAGAACAATAAATTC
TGTGTCTCTGTACATTACCGTAACGTTGATCAAGATAACTGGAAATCAGTTGGGGAGCGTGTCCAG
GATGTCATAAAGAAGTATCCTCGCCTGCGATTGACTCATGGGAGGAAGGTTTTAGAAATCCGTCCC
GCGATCAATTGGGACAAGGGGAAAGCTCTTGAATTTCTACTTGAATCACTAGATCTCAGCAATTGT
GATGATGTGCTGCCGATTTATGTTGGAGATGACCGGACAGATGAAGATGCATTTAAGGTTTTGAGA
GAGAGGAACTGTGGTTATGGCATTTTTGTATCAAAATCACCGAAGGAAAGCAATGCGTATTACTCT
CTCAGAGACCCAGCAGAGGTCATGGAGTTTCTCAAGTCCCTGGTGACATGGAAGAAATCGAGTGCT
TTATAAATGTTACAAGGAATTACATAAAGAGGAGAATACTATAACATACACAGACAACATGAAAGG
CATTTTATATTCTTAAGTTC

**SEQ ID NO: 42, protein - Populus trichocarpa**
MDLKSNHNAPVLTDSAPLSKSRLRGYHHGLMLPYSPSGAPFSSNLLLSIPRRKTGVLDDVRSCGWL
DAMKSSSPTHKKFAKDINHELSAPDPEVAYRTWLLKYPSALASFEQIANFAKGKRIALFLDYDGTL
SPIVENPDNAFMSADMRSIVKEVAKYFPTAIISGRSRDKVYEFVGLTELYYAGSHGMDIMGPVRQS
VSDDHRNCIKSTDKQGNEVNLFQPAREFLPMIDEVYSSLVRITEDIKGATVENNKFCVSVHYRNVD
QDNWKSVGERVQDVIKKYPRLRLTHGRKVLEIRPAINWDKGKALEFLLESLDLSNCDDVLPIYVGD
DRTDEDAFKVLRERNCGYGIFVSKSPKESNAYYSLRDPAEVMEFLKSLVTWKKSSAL

**FIGURE 8 (continued)**

**SEQ ID NO: 43, DNA - Populus trichocarpa**

TATCTTACGAATCTGGATCATCTGGATCCGATGGACCTCAAATCAAATCACAGTGCTCCTGTGCTC
ACTGATTCTGCACCCCTAGGCAAGTCAAGACTAGGTGGGCATCATGGTTTGTTCCCATGCTCACCC
TCAGGCGGTGCAGCTTTTTCGCCGAATCTATGGTTATCTATTCCTAAGAAGAAAACTGGAGTTCTT
GATGATGTTCGCTCCATTGGTTGGCTGGACGCAATGAAATCATCATCTCCTCCTCACAAGAAGTTT
AACAAGGATATTAACATGGAGCTTTCCTCGCCTGATCCGGAAGCTGCCTACCGCACTTGGCTTCTT
AAATATCCATCTGCTCTTGCATCTTTTGAGCAAATTGCAAACTTTGCAAAAGGCAAGAGAATCGCC
TTGTTCCTGGATTATGATGGTACTCTATCGCCGATTGTAGAAAATCCCGACAATGCCCTCATGTCT
GATGTTATGCGTTCTGCTGTAAAGAAAGTGGCAAAATATTTCCCCACAGCAATAATTAGTGGAAGA
AGCCGTGACAAGGTATACGAGTTTGTAGGACTCACAGAACTCTATTATGCGGGTAGCCATGGAATG
GATATTGTGGGCCCTGTTAGGCACTCCACATCTGATGATCACCCAAATTGTATCGAGTCTACGGAC
ATGCAGGGAAATGAAGTTAATTTATTCCAGCCAGCTAGAGAATTTTTACCTATGATCGACGAGGTT
TTTAGCTCCCTTCTCAAGAGTACCGAAGAAATTAAAGGTGCAACAGTTGAGAACAATAAATTCTGT
GTCTCTGTACATTACCGAAATGTTGATGAAGATAAATGGAAAGCAGTTTGGGAGTGTGTCGAAGAT
GTCATTAAGAAGTACCCTCGCCTGCGATTGACTTTTGGGAGGAAGGTTTTAGAAATTCGTCCCACG
ATCAATTGGGACAAGGGGAAAGCTCTTGTGTTTCTACTTGAATCACTAGGTCTCAGCAATTGTGAT
GATGTGCTCCCTATTTATGTTGGAGATGACCGGACAGATGAAGATGCATTTAAGATTTTGAGAGAG
AGGAACTGTGGTTATGGGATTCTGGTATCAAAATCACCCAAGGAAAGCAATGCATATTACTCTCTC
AGGGACCCATCCGAGGTCATGGAGTTTCTCAAGTCCCTTGTGATGTGGAAGAAGTCGAGTGCTCAA
TAAATGCTACAAGGAATACACAAGGAAGAGGAGAATACTCTAATATACACATACAACATGAAAGAC
TTTATATGTTTTTTTCT

**SEQ ID NO: 44, protein - Populus trichocarpa**

MDLKSNHSAPVLTDSAPLGKSRLGGHHGLFPCSPSGGAAFSPNLWLSIPKKKTGVLDDVRSIGWLD
AMKSSSPPHKKFNKDINMELSSPDPEAAYRTWLLKYPSALASFEQIANFAKGKRIALFLDYDGTLS
PIVENPDNALMSDVMRSAVKKVAKYFPTAIISGRSRDKVYEFVGLTELYYAGSHGMDIVGPVRHST
SDDHPNCIESTDMQGNEVNLFQPAREFLPMIDEVFSSLLKSTEEIKGATVENNKFCVSVHYRNVDE
DKWKAVWECVEDVIKKYPRLRLTFGRKVLEIRPTINWDKGKALVFLLESLGLSNCDDVLPIYVGDD
RTDEDAFKILRERNCGYGILVSKSPKESNAYYSLRDPSEVMEFLKSLVMWKKSSAQ

**SEQ ID NO: 45, DNA - Oryza sativa**

AGCACTCCATCTTCCTCCATCTGCGTATTTTCCAACACATCTCTCTTGCAGTTCGTTTCAAGTCTT
TCATTAAGTAAAACTTTCTGAACATTTGAAGTGCAACGGAGCTGGCGTTTTATGGCTTTTCCTATG
AAGCAATCCACATCTCTGATATAGATGTGTACTCGTTCTGAACATGACAAGTTCTCAGGTATTTCT
CTGTTGATGAACTGCCTCCACACCTGCAGTGACAAGAAGACACTGAAGAAGTGGTTTTTCATCGAC
AAGACAGTTGGCTAAATTCAGCAGTGCACTCGTCCTTAAAGCGATCGATCATACTTCTTTCTTGAT
CTCATCATCCTAAATTTTCCCAAATGGATTTGAGCAATAGCTCACCTGTCATCACCGATCCGGTGG
CGATCAGCCAGCAGTTGTTGGGCGGCCTGCCTTCAAATCTGATGCAGTTTTCAGTCATGCCCGGTG
GCTACTCCAGCTCTGGCATGAACGTTGGTGTCAGTAGGCTCAAAATCGAGGAAGTCCTTGTCAATG
GACTGCTTGATGCCATGAAATCCTCGTCACCTCGCAGGAGGCTGAATGTAGCATTTGGCGAGGACA
ATTCATCTGAAGAAGAAGACCCTGCTTACAGCGCTTGGATGGCAAAATGTCCTTCTGCTTTGGCTT
CCTTCAAGCAAATTGTAGCCAGTGCACAAGGGAAGAAGATTGCTGTGTTTCTAGACTATGACGGCA
CACTGTCGCCTATTGTGGATGATCCTGACAAAGCAGTGATGTCTCCCGTGATGAGAGCTGCTGTGA
GAAATGTTGCGAAGTACTTCCCCACTGCAATTGTCAGCGGAAGGTCCCGCAATAAGGTGTTTGAAT
TTGTAAAACTGAAGGAGCTTTATTATGCTGGAAGCCATGGTATGGACATAATGGCACCTTCAGCAA
ATCATGAGCACAGTGCTGAAAAGAGCAAACAGGCCAATCTCTTCCAACCTGCACACGACTTTCTGC
CAATGATCGATGAGGTTACCAAGTCCCTCTTGCAAGTTGTCAGTGGAATTGAAGGTGCAACTGTTG
AGAACAACAAATTCTGCGTTTCTGTACATTATCGCAACGTTGCAGAGAAGGATTGGAAACTGGTCG

## FIGURE 8 (continued)

CACGGCTCGTAAACGAAGTGCTGGAGGCTTTTCCTCGTCTCAAAGTAACCAATGGACGAATGGTTT
TAGAGGTTCGTCCGGTGATCGACTGGGACAAGGGAAAGGCTGTGGAGTTTCTGCTCCAGTCACTCG
GGCTAAATGACTCTGAAAATGTGATCCCCATCTACATTGGAGACGACAGAACTGACGAAGACGCTT
TCAAGGTACTTCGACAGCGAAATTGCGGTTATGGAATACTAGTTTCACAGGTTCCCAAGGAAACTG
AAGCCTTCTACTCGCTGAGAGATCCATCTGAAGTGATGGAGTTCCTCAATTTCTTGGTGAGATGGA
AGAAGCACTCAGTGTGAAAAAAAGAAGACAGTCTGAGCAGGTTTTACTAATACTACTCCGGCAGAA
ATAACTGTAGTTTCCGAGGCGACAATTTTGAAGCATTGGACGCTGTAGATAGATGTAATAACTCTC
TAGATATGTGGTTCCAATTCATTCTTATTGATCGATGTTATTGTATTGTTCATGTTCTCCACCGCT
GAGAAAAATTAATCAGTGATGGTTGATCCGGTCTCTTGCCATTGTAAGTTGTAAGCAAGAAGGCCC
TCTTCTTAGTATCCATGCTGATGAACGAAATTAAAACCTTCATTTTTCGTGT

**SEQ ID NO: 46, protein - Oryza sativa**
MDLSNSSPVITDPVAISQQLLGGLPSNLMQFSVMPGGYSSSGMNVGVSRLKIEEVLVNGLLDAMKS
SSPRRRLNVAFGEDNSSEEEDPAYSAWMAKCPSALASFKQIVASAQGKKIAVFLDYDGTLSPIVDD
PDKAVMSPVMRAAVRNVAKYFPTAIVSGRSRNKVFEFVKLKELYYAGSHGMDIMAPSANHEHSAEK
SKQANLFQPAHDFLPMIDEVTKSLLQVVSGIEGATVENNKFCVSVHYRNVAEKDWKLVARLVNEVL
EAFPRLKVTNGRMVLEVRPVIDWDKGKAVEFLLQSLGLNDSENVIPIYIGDDRTDEDAFKVLRQRN
CGYGILVSQVPKETEAFYSLRDPSEVMEFLNFLVRWKKHSV

**SEQ ID NO: 47, DNA - Oryza sativa**
CTCTCAAACTCTCGGAGAAGCGAGCACACACACTCACTCCCTGTCTCCTCCTCCTCCTCCTCCCTT
GAGGCTGTTCGTCTCGACGAGTGCTTGGCTTTGTGTTTGTGGTGTGTGATCATGACGAACCAGGAC
GTGGTGGTTTCTGAGATGGGCATCGCGGCAGGCGCGGCGCTGCCGGGAGGACCGGCCGGCCCGGCG
GGCGGCCTCTTCGCGTGCCGCAGCGCGGCCGCGTCGATGAGGCAGACGTACCTCGACCTCGCCGCG
GCGGCGGTGGCGGCGCGCTCCGCGAGCTGCACCAGCTGGGCCGACGCCATGCGCGCCTCCTCCCCC
ACCCGCTCGTCCCGCTCCGCCTCCGACGTCGACGAGTTCACCGCCTGGGTGAGGAAGCACCCGTCG
GCGCTTAGCAAGTTCGAGGAGATCGCCGCCAAGTCCAGGGGGAAGAAGATCGTAATGTTCATGGAC
TACGACGGCACGCTCTCTCCCATCGTCGCCGACCCCGACACGGCCTACATGAGCGACGCGATGAGG
GCGGCGGTGCGCGAAGTCGCCAAGACCTTCCCGACGGCGATCGTCAGCGGGCGGTGCCGCGACAAG
GTTCGCAACTTCGTCGGCCTCTCCGACCTCTACTACGCCGGCAGCCATGGCATGGACATCAAGGGA
CCAAGCTCCAACCCGGAGTCTGCCCTTTGCCAGCCAGCAAGCGAGTTCCTCCCCATGATCGACGAG
GTGTACAAGACGCTGGTGGAAAAGACGAAATCTACACCTGGAGCCAAGGTGGAGAACAACAAGTTC
TGCCTGTCCGTCCACTTCAGATGTGTAGATGAAAAGAGATGGAATGCTTTGGGGGAGCAGGTCAAG
GCCGTGATCAAGGAATACCCAAAGTTGAAGCTTACCCAAGGAAGGAAGGTTTTGGAGATCAGGCCG
AGCATTGAGTGGGACAAGGGCAAGGCTCTGGAGTTCTTGCTTGAATCGCTGGGATTCGCCAACTGC
GGCGACGTCATGCCGGTGTACATCGGTGACGACCGCACCGACGAGGACGCCTTCAAGGTGTTGAGA
AAGAGAGGTCAGGGCCTGGGAATCCTAGTCTCCAAGTGCCCCAAGGACACCAACGCCTCCTACTCT
CTCCAGGACCCAACAGAGGTGATGGAGTTCTTGCTCAGGCTGGTGGAGTGGAAGCGGAAGTCGTCG
TCGTCGTCGTTGATGATTCGTCCGAGAGTGTAGCGGCGACACGGTGTAGAGGCCGTAAGAAACTGA
AACTAAGATGGTCAATCATGCGAACATGCGAACATACCCCACCAACACCACGTACTACCCCCATTT
TTTTCCGATGATTTCTGTGGTTGGCCTCTGCGACGGACTCGTGGTGGTTACACGCTAGCTGTTCGA
TTCCGTCCCCTGTGCCGCCGAGGAAAAGGAGACCACC

**SEQ ID NO: 48, protein - Oryza sativa**
MTNQDVVVSEMGIAAGAALPGGPAGPAGGLFACRSAAASMRQTYLDLAAAAVAARSASCTSWADAM
RASSPTRSSRSASDVDEFTAWVRKHPSALSKFEEIAAKSRGKKIVMFMDYDGTLSPIVADPDTAYM
SDAMRAAVREVAKTFPTAIVSGRCRDKVRNFVGLSDLYYAGSHGMDIKGPSSNPESALCQPASEFL
PMIDEVYKTLVEKTKSTPGAKVENNKFCLSVHFRCVDEKRWNALGEQVKAVIKEYPKLKLTQGRKV
LEIRPSIEWDKGKALEFLLESLGFANCGDVMPVYIGDDRTDEDAFKVLRKRGQGLGILVSKCPKDT
NASYSLQDPTEVMEFLLRLVEWKRKSSSSSLMIRPRV

FIGURE 8 (continued)

**SEQ ID NO: 49, DNA - Oryza sativa**
CACAACCAAATCAACAACCACGCCATTGCCGTTCTCTCCTCCTTTCCTTCCACAACCCATCTCTTC
CTCATCCTCCTCCTCCTCTTCCTGCTGCTCTCTCGCTCTCGTTGGATTCGGTTTGGTTTGGTTTGG
GCCATGACGAAGCACGGCGCGGTGGTTGTCCCGGTCGCCGCCGCCGCCGCCGACGCGGAGCACGAC
GAGTGGATGGAGAAGCACCCGTCGGCATTGGGGAAGTTCGAGGCGCTGGCGGCGGCGGCGAAGGGG
AAGCGGATCGTCGTCTTCCTCGACTACGACGGCACGCTGTCGCCGATCGTCGAGGACCCCGACCGC
GCCGTCATGACGGACGAGATGAGGGACGCCGTGCGCGGCGTGGCGGCGCGATTCCCAACGGCGATC
GTCAGCGGCCGCTGCAGAGACAAGGTGTTGAGCTTCGTGGGGCTGGAGGAGCTCTACTACGCGGGT
AGCCACGGGATGGACATCCAAGGGCCCACCAACGCCGCCGCCTCCAAGGGAGGAGAGGAGGAGGAG
GAGTCGGTGCTGTGCCAGCCGGCGAGGGAGTTCCTGCCGATGATCGGGGAGGCGTACGCGGCGCTG
GTGGAGAAGGTGGAGGGGGTGATCCCGGGGGCGAAGGTGGAGAACAACAAGTTCTGCCTCTCCGTC
CACTTCCGCCGCGTCGACGAGCGCCGGTGGGGCGCCGTCGCCGACCAGGTCAGGGCGGTGCTCCGG
GGCTACCCGCGCCTCCGCCTCACGCAGGGCCGCAAGGTGCTCGAGGTCAGGCCCGCCATCAAGTGG
GACAAGGGCGAGGCCCTCCGCTTCCTCCTCTCCGCCCTCGGCTTCTCCGCCGCCGGAGACGTAGAA
GACGACGGCGACGACGACGACGCGTTCCCCATCTACATCGGCGACGACCGCACCGACGAGGACGCG
TTCCGGGTGCTCCGAGCGAGGGGGCACGGCGCCGGCATCCTGGTGTCGAGGTTCCCCAAGGACACC
TGCGCCTCCTTCTCCCTCCGCGACCCCGGCGAGGTCAAGGACTTCCTCCGCAAGCTCGTCACCTGC
GCTGCCGCATGACGTCACCTTCACCTGACCACAGTGACACTAATTGGCTTCCTCCTCCTCCACGGG
AAAGAGAAAGAGAGAGAGAGAGAAATAATTAAGGATCTTGATTAGTCAATTAGCGGTTACTAATCCCT
GTATTAATTTTAGCCGGTCACTTCATTTCTTCTTCTTCTTTTTTTATGTGTTTCTGTTCTTGCTCT
GCGGCTGCCATCTTGATTGATCGATCGATCTCACAGGCCACAGTGTGACAGCTCGATCTGAAGGTT
TCTCTATTATCCTTAACCTTCCTCCAGCTGATCCAAGGCCTTGTAATTAGTCGTCAATTTTTAGCA
GTTTGTTGTCTGTCTGTCTGTCACTGAAACGGGACGAATTAATCCGAATTAATACGATCCATTTCC
ACCTAATTTCAGTTTGTACTGTACCGTTTAATTTCCATCTAAATTCAGCTTTGCACTGT

**SEQ ID NO: 50, protein - Oryza sativa**
MTKHGAVVVPVAAAAADAEHDEWMEKHPSALGKFEALAAAAKGKRIVVFLDYDGTLSPIVEDPDRA
VMTDEMRDAVRGVAARFPTAIVSGRCRDKVLSFVGLEELYYAGSHGMDIQGPTNAAASKGGEEEEE
SVLCQPAREFLPMIGEAYAALVEKVEGVIPGAKVENNKFCLSVHFRRVDERRWGAVADQVRAVLRG
YPRLRLTQGRKVLEVRPAIKWDKGEALRFLLSALGFSAAGDVEDDGDDDDAFPIYIGDDRTDEDAF
RVLRARGHGAGILVSRFPKDTCASFSLRDPGEVKDFLRKLVTCAAA

**SEQ ID NO: 51, DNA - Oryza sativa**
ATGACGAACCAGGACGTGGTGATGCCGGACATCGCGGCGGCCGCGGCCATGCCGGGGTCATCCGGC
CGCGCGCCGCTCTTCGCGTGCCGTGGCGCCGCGGCCGTGTCGGCGTCCTCCATGCTCGGCGGCGGC
GGCGCGGCGTACCAGGCCGCGGTGGTGGCGCACGTGGCGCCGGTGCCAGCTATCAGGCCGTGCGCC
AGCTGGGTCGTCGAGGCCATGCGCGCGTCGTCGCCGACACGGCCCGCCGCCGCCGCGGTCGACGCC
GAGTACGACGCGTGGACGCAGAGGAAGCACCCGTCGGCGCTGGGCAGCTTCGAGCAGGTGGCCGCG
GCGGCGAGCGGGAAGCGCGTCGTCGTGTTCCTCGACTACGACGGCACCCTCTCCCCCATCGTCGCC
GACCCCGACATGGCCTTCATGTCCGACGAGATGAGGGCGGCCGTGCGCGACGTGGCGGAGCACTTC
CCGGCGGCGATCGTGACCGGGCGCTGCGTCGACAAGGTGCAGAGCTTCGTCGGCCTCCCGGAGCTC
TACTACGCCGGCAGCCATGGCATGGACATCAAGGGCCCCAGCTCCAACGAGGAAGAAGACACCAAG
ATCCTCTTGCAGCCAGCTCGCGAGTTCCTCCCGGTGATCAACAAGGCTTACAAGGCTTTGATGGAG
AAGACGAAGAGCACGCCTGGGGCCAGAGTGGAGAACAACAAGTTCTGCCTCTCCGTGCACTTCAGA
TGCGTCGATGAGAAGAGATGGAATCCGTTGGCAGAGCAAGTGAAGGCCGTGCTCCGGGACTACCCC
GAGCTCAAACTCACCCAAGGAAGAAAGGTCCTGGAGATCCGGCCGTCGATCATGTGGGACAAGGGC
AAGGCGGTGGAGTTCTTGCTGAAATCTCTCGGATTCGACGACGACCGCCGCGACGTCCTGCCGGTG
TACATCGGAGACGACCGGACCGACGAAGATGCTTTCAAGGTGTTGAGAAAGAGAGGTCAAGGGTTG

**FIGURE 8 (continued)**

GGAATTCTTGTCTCCAAGTGTGCCAAGGAGACTGACGCCTCGTACTCTCTCCAGGACCCAGCAGAG
AAGTACACCAACGCCGGGGCGCACGTGTTCGTGACGATGCTCCTGACCGTGGTGTTCACGGCAGCT
GTGGCGTTGGCTCTTGTCAACGCCGTCAACTCGCATGACTTCGCAGCACACCTCGCCGGCGTGGAC
TGCCGCATGGGGCTAGCTGGTCCAGTACGATGCCCAGCCTCGGGCTTCGTGGAGCTCCTCGTGCTG
GCGCTACATGTGATGCGTGCTCGCCATCTTAGATCGCCTGCATGCCTGCCTAATGTCGCCCTCGCT
ACAGCTTTCGATTGCATCTCCATGTACGTGCGCCGGTGGCAGCGCGGCGAAGTGACCAATGTACAT
GAAGTATACGTGCTAAGTAGGATCGAATTTGTTCCTTATATGGCAAGCACCGGTGAACAAAGAAGT
ACTGGCGAGACGGCCGTCGGCGCCGCCGCCGCCGGAATGTATCGGATGGTGCCCTTGCACGCGGCA
AAATGGGGAGACAAATGGAGAATGAGTGGGGAAAGAATGGCCGCCGGAGTAGACTGCCGGCGGCGC
GAGGATTTGCCGAGCAGTGGAGATGGCTACCGTCAGGAGGAGATCGACGGTGGTCATGGAGTTCCT
TGTTCGATTGGTGCAGTGGAAGCTTCGGCGATCATCGTCAGCGATGCGCCCGAGGGTTTAATTAGT
ACACTGACGACGGATCATATATACTATATGACAGTCTTCCCTGCTAAGCTAGCTCTGACTTCTGAA
GAAGTTTGA

**SEQ ID NO: 52, protein - Oryza sativa**
MTNQDVVMPDIAAAAAMPGSSGRAPLFACRGAAAVSASSMLGGGGAAYQAAVVAHVAPVPAIRPCA
SWVVEAMRASSPTRPAAAAVDAEYDAWTQRKHPSALGSFEQVAAAASGKRVVVFLDYDGTLSPIVA
DPDMAFMSDEMRAAVRDVAEHFPAAIVTGRCVDKVQSFVGLPELYYAGSHGMDIKGPSSNEEEDTK
ILLQPAREFLPVINKAYKALMEKTKSTPGARVENNKFCLSVHFRCVDEKRWNPLAEQVKAVLRDYP
ELKLTQGRKVLEIRPSIMWDKGKAVEFLLKSLGFDDDRRDVLPVYIGDDRTDEDAFKVLRKRGQGL
GILVSKCAKETDASYSLQDPAEKYTNAGAHVFVTMLLTVVFTAAVALALVNAVNSHDFAAHLAGVD
CRMGLAGPVRCPASGFVELLVLALHVMRARHLRSPACLPNVALATAFDCISMYVRRWQRGEVTNVH
EVYVLSRIEFVPYMASTGEQRSTGETAVGAAAAGMYRMVPLHAAKWGDKWRMSGERMAAGVDCRRR
EDLPSSGDGYRQEEIDGGHGVPCSIGAVEASAIIVSDAPEGLISTLTTDHIYYMTVFPAKLALTSE
EV

**SEQ ID NO: 53, DNA - Oryza sativa**
GGTTGATCGAGGTGAGCGTTTCCTGACCTACTTGCTCGGATTAATTACAAATTGATTTGATCACGT
AGATAATTACATCATCATCATATTGATGGATGGATGCTTGCACATATATGCATGCGTTTTGAATCT
GGTGATGTAATGATATCCAATTAATAATCTTGCTGCAACACGAAAGCTCAGCCAGTTTAATTGATT
TGATTTAGTTAATGCCATCGATCTCAGGCTTGATTATCGATTGGCTGTTGAATGCACGGATTAATC
AAATAGGTTGAAGGCACGCAATGCAAAATTCCAAATTGATTGTGGACCTCTTCGGTTTCTCCTTGT
ATAAGACCTTGAATTTAGAAATTATTAGTGAGCTATCGAAAGTTTTCAGTGGCATCACATATTATC
TGCTTACTTGGAACATATAGAAAAATAGAATTATTAATTAATAAATTAATTGTATTGAGCATTATA
CTTCCTTAGTCTGAGAGTATATAGCATTGGGCATAAAGTTAGTATTAACTATTCACCATGTAACTA
GCTTCTTAGTAAAGGTACAAATTAAGGACTGAAGAGAAACTCCAGACTTTTAATCTTCAAACTGAA
GGCAAGTAATACATTTTCAGCCCTTCCATTTAGTTCCTTGGAGGATCAAATTCAAATGTTGCGACG
GATGAAGACGCACGAAATTTGATGCTTTTATCGCACGTTCATGTAGTATTTGGTCAATCGATCTCT
GCATCATGCCTTGACCAAGATGCATATTTAGTTTGATGATCTCTGTTGCTTCCAATTGCACTCCAT
TAATTGTTTGAACTAATAGTCGTTTTGCTTCATGTAATCGAATGCTTCCTGCGTTCGATTGGTGAG
AGAAGTGCACTCCAATGCTGTACCAACAGTACTCTTGGGTTTTTCTCTTAGGAGAGCTTTATTTAG
AGTGAAAGGGACTTCCTAATTAAGATTTCTTTATTTTGAAAGAAGCTAGTAATTAAGATTTCTTAA
CATATGCACACGTGAAGTTATAAAACATGACTCAAATCTGATGTTGATGTGTGTTCGACCAAGTCC
CTAATCAGTTTTTGCACTTTTTCTTCTGATTTCTCACGCCAACTGATCAATTAAGGCAAGTAGGAT
TTTAGTTGTGTTCTTTTCTTATTGTTTTCCTTTCTCTTGCTTTTCGCTTTCTCACCTCCTACCTCC
TTGGAGACAGTCCACAATTGTGAATTTTCAACATTAGAAAAATAATCACAATTTTGTGGAAATGAT
TGAAAAACTAATTAATAGGTTGCATTTCACATGAACATAGTTAATTAGAGAACAAAATGCATACAC
TAGGTGTTAGAGCAGTACAAAAGTTATAGAAGATGTCACTGCTTACTGCATGAGTATCTTTCTTCA

**FIGURE 8 (continued)**

CAATCCCATTCCATCATAAATATTGCCTAAACAATTCCACTCAACCTTGTTGCTTGTTCAACTTTA
ATTCTTTCATAACTTGATATGAAGGTCAGGACAATTACTGTAGACTAGTTTCTTCATGGTTGCACG
CTGAATAATCGTACACAGATGCCCTTGCTGTCTCTGAATTCTCAAACACTGAACATATATTGTAGA
ACATTTTACACCTATTCCTGTCTGATCTCTTGCTGTGTGTGTACGGAGCATGCTGCCGTTCTTCCC
AAGACTTTGTGTCTCAATTGGTTATTTTTGACTTGAAGCGATCCGTTTCGGTAGTCCCTGAGGCAG
CTAGCGTAAGCCTCTTTTGACTTCCAAGCACACAAGTTACTTGCTGCCTCAGATTCATCAGCAAGT
CACTAGTACTTTTCGGTATCTAGATGAAGCATATATATAAACCACAAGAAAAGCGGCTGCCCATTT
GTGTTCTGATCATGAGGAGATTCAGGTGGTTTCTTGAAAGGATGTGAATCTGTGGTTGCAAGATAC
TGAAGAAAGTAATAAGGTGGTTCTTCAATCTTCATAGGCTTCAGAGTTCAGAGTTGAGTTAATGTT
TTTGCAAACAAGGTGTCTGCTGACAGTTTGACAGTAAAAAGATCTGTATATATGGGTTCCTGCGGT
AACGGTAGAAGCTCTGAATATGATGATCCTGCATCATTAGAGAAGATGGAGGAACTGGTTTTACCT
CTGAAATTGATGCCCTTACACACCAATGGCCGGTTGTATGACATGAGGTTGTCCTCACCTACTGCT
ACCTGTGTGATCAATAGTTCATCTGGTTCATTCGACCCGATCTACCGAGCTTGGACGAAGAAGTAT
CCTTCAGCTCTAAATGCCTTTGACCACATTGTTGCCTATGGCAAAGGGAAGAAGATAGCGCTGTTT
TTGGACTACGACGGTACACTGTCGCCAATTGTCGATGAACCTGACAATGCTATCATGTCTGACCAG
ATGCGCGAGGTGGTGAGGAATGCTGCCTTGCATCTACCCACTGCGATTATCAGTGGAAGGTCTCGT
GATAAGGTGTTTGATTTTGTTAAACTAACAGAATTGTACTATGCTGGTAGCCATGGAATGGACATC
ATGGGTCCAGTTGGGGAACATGATTCAGTTACTAACCATAGGAGCTCCATTAACTCAAATAGAAAG
CAGGGCAAGGGAGTGAAGATCTTCCAGGCTGGTACTGAATTCTTACCAATGATCAATGAGGTTTTT
AGATTGCTCATCGATAAGACAAAGGCAATCGACGGTGTGAAAATTGAGAACAACAAGTTCTGTGTA
TCGGTGCACTACCGCAATGTTGAGGAGAAGAATTGGCAACTTGTTTCTCAATGCACAAATGATGTC
CTAAAAGTCTACCCTCGCCTCCGATTGACTCATGGACGAAAAGTTTTGGAAATTCGTCCAGTGATA
GACTGGAACAAGGGGAAGGCTGTGGAGTTCTTGCTGGACTCCCTAGATCTAGCTAGCTGTAAAAAT
GTTCTCCCTATTTACATCGGGGATGATTGTACCGACGAGGATGCTTTTAAGGTTCTTCGTGACGAT
AAAAGGGGTTTCGGGATTCTGGTGTCATCTGTACCAAAGGATTCTCATGCCCTATATTCTCTGATT
GATCCATCTGAGGTTATGGAATTCTTGAAAAGACTAGTGATGTGGAAGAACGAAGAAGCATCACAT
AATAAGTAGATTATAAATATATCTGATGTTTACTCTAGGGTAGATTTCTGAATTCTGACAAAGAGA
ATAAGCAGAAGGGCAACAGAATTAACTTGGACATAGTGGACAGTTGTACATCATATAGTTAATCCA
TTTTCTTAGCCTAGCAAGTGGCAAAATAAAAGTAATTGCCATGTTGTACAAGAATGCGAAGCATTC
TGCACAAGTCCAGTTCTTTGAATAATGGTCTCAGTTGTAGAATGGTGTAACTTGTTAATGAACTGT
GATATAAATTTTTGGGCT

**SEQ ID NO: 54, protein - Oryza sativa**
MGSCGNGRSSEYDDPASLEKMEELVLPLKLMPLHTNGRLYDMRLSSPTATCVINSSSGSFDPIYRA
WTKKYPSALNAFDHIVAYGKGKKIALFLDYDGTLSPIVDEPDNAIMSDQMREVVRNAALHLPTAII
SGRSRDKVFDFVKLTELYYAGSHGMDIMGPVGEHDSVTNHRSSINSNRKQGKGVKIFQAGTEFLPM
INEVFRLLIDKTKAIDGVKIENNKFCVSVHYRNVEEKNWQLVSQCTNDVLKVYPRLRLTHGRKVLE
IRPVIDWNKGKAVEFLLDSLDLASCKNVLPIYIGDDCTDEDAFKVLRDDKRGFGILVSSVPKDSHA
LYSLIDPSEVMEFLKRLVMWKNEEASHNK

**SEQ ID NO: 55, DNA - Oryza sativa**
ACCTTCCCCTGTCATCTCCTCCTCCTCCACCACCACCTCGCCGTCGCCGTCACCGCGCCACCGTCT
CCTCGATCTCTCACTCCCACAGCTCGCTGGTTGCTTCGGCTTCCGCTGCTCGGTCGCGGTTGATTT
TGGGGGATGACGAACCACGCCGGCTTCGCCGCGGACGACGCGGTCACCGCGGCCGTGCCGGTGCAG
GCGGCGCAGGGCGGGCGGCATTTCCCGCCGTTCCTGGCGCCGTCGTCCAGGCTCACCGACTGCAAG
AAGGCGGCGGCGCACGTGGACCTCGCCGGCGCGGGCGGGGTGGCGACGGTGCCCGGATCTTGGCCG
CGCCACGCCAAGCCCGTCTCCGGCGCCGAGCTCGACGACTGGATGGAGAAGCACCCGTCGGCATTG
GCATGGTTCGAGTCCGTCGCCGCCGCGGCGAAGGGCAAGGAGATCGTCGTGTTCCTCGACTACGAC

## FIGURE 8 (continued)

GGCACCCTCTCCCCCATCGTCGCCGACCCCGACCGCGCCTTCATGTCCGACGAAATGAGAGAGGCG
GTGAGAGGCGTGGCGAAGCACTTCCCGACGGCGATCGTGAGCGGGAGGTGCATCGACAAGGTGTTC
GACTTTGTGAAGCTGGAGGAGCTGTACTACGCCGGGAGCCATGGAATGGACATCAGGGGCCCCACC
GCGGCAGCGTCGGAGTACAACCACAACATGAAGGCAAAGCAGGGTGATGCTGTTACTTTCCAGCCG
GCCGCCGATTTCCTGCCCGTCATCGAGGAGGTGTATCATGTGCTGAAGGAGAGGATGGCGAGTATA
AGGGGTTCGCTGGTGGAGAACAACAAGTTTTGCCTTTCCGTGCACTACCGCTGCGTCGACGAGGCG
GAATGGGGCGTGCTGGACGGCAAGGTGAGGGCGGTGATAGAGGGCTACCCGGATCTCCGTCTCAGC
AAGGGGAGAAAGGTGCTGGAGATCCGCCCTGTCATCGACTGGGACAAAGGCTCCGCACTCCAGTTC
CTGCTCAAATCTCTCGGTTATGAGGGGCGCAACAATGTTTTCCCGATATACATTGGAGATGACCGC
ACCGACGAGGATGCTTTCAAGGTGTTGCGCAACATGGGACAGGGCATAGGAATCCTTGTGACCAAG
GTCCCAAAGGAGACAGCTGCATCCTACACTCTGCGAGAGCCATCCGAGGTGAAGGAGTTCCTGCGC
AAGTTGGTGAAGATTAAGATCAACGGGGACAAAGGGCTGATTGGCAAGTAGCTGATCCAAGGCATA
TAGCTAGCTAGCTAGCTTTTTGTTCCTTAATTTGTCTCTTTCCTTTTGGTCCATTAGAAGAAGAAG
AAGAACAACAACAAGAGTTATATGCATGGATGGTAACTGATCATGCATGATCGATGCATGCGGTGG
TGAAAACCCGGGCTTTTGGCTTTGAAGGTGTCTATTACCGTATCCTTCTGGCCAAGGTCCTGTAAT
TAAATTTTGCTGCCGTGACAACAGAAATCTCAGCAAGAGGGACACAAGTGTGGCCCCTTTTCTTT

**SEQ ID NO: 56, protein - Oryza sativa**
MTNHAGFAADDAVTAAVPVQAAQGGRHFPPFLAPSSRLTDCKKAAAHVDLAGAGGVATVPGSWPRH
AKPVSGAELDDWMEKHPSALAWFESVAAAAKGKEIVVFLDYDGTLSPIVADPDRAFMSDEMREAVR
GVAKHFPTAIVSGRCIDKVFDFVKLEELYYAGSHGMDIRGPTAAASEYNHNMKAKQGDAVTFQPAA
DFLPVIEEVYHVLKERMASIRGSLVENNKFCLSVHYRCVDEAEWGVLDGKVRAVIEGYPDLRLSKG
RKVLEIRPVIDWDKGSALQFLLKSLGYEGRNNVFPIYIGDDRTDEDAFKVLRNMGQGIGILVTKVP
KETAASYTLREPSEVKEFLRKLVKIKINGDKGLIGK

**SEQ ID NO: 57, DNA - Oryza sativa**
ATGGCGAAGGCGAGCGTGGTGGTGCCTGAGCAGGTGGGCGCGGCGGCGGCGGCGCAGGTGGGGTGC
CCCTGTCCGGGCACGACGCTGTTCCCGTACCCGCCGCCGCGCGCCGGGATCGCCGTGCGGCGCAAG
TGCCTGCAGGCGGCGCAGCAGCTGGAGCTCGGCGCCGGGCTGCGCGGCGGCTGGGTGGAGTCCATG
CGGGCGTCGTCGCCCACCCACGCCAAGGCCGCCGCCGCCCTCGCCGCCGGCGTCGACGAGGAGCAC
GCCGCCTGGATGGTCCGTTTCCGTTCACCGATTGATCGATGTTCGTCGCGTTCTTGGCGCGCGCGC
GCTGACACTGACATGAACCGTGCATTTCCGTTCGTCTTTGTGCAGGCGAGGCACCCGTCGGCGCTG
GGCGAGTTCGAGAAGGTGGTGGCGGCGTCGAAGGGGAAGCAGATCGTCATGTTCCTCGACTACGAC
GGCACCCTCTCCCCCATCGTCGACGACCCCGACGCCGCCTTCATGAGCGAGACGGTGAGCTTGAGC
TCCCCTCCCCTGTCACCTACTCTGCTCCTCCACTCATCATCATCTCACACCTCTCTCCTTCCTCAT
CAGATGCGGATGGCCGTGCGCAGCGTGGCGAAGCACTTCCCGACGGCGATCGTGAGCGGGCGGTGC
CGCGACAAGGTGTTCGAGTTCGTGAAGCTCGCCGAGCTGTACTACGCGGGGAGCCACGGCATGGAC
ATCAAGGGCCCCGCCTCCCGCCACGCCGCCGCCAAGTCTCCTCCCCACAACAAGGGAGTCCTCTTC
CAGCCGGCCAGCGAGTTCCTCCCCATGATCGAGCAGGTGCACCAGCGACTCGAGCAGGCCACCAGC
TCCATCCCGGGCGCCAAGGTCGAGAACAACAAGTTCTGCGTCTCCGTCCACTTCCGGTGCGTCGAC
GAGAAGAGTTGGGGGGCGTTGGCGGAGACGGTGAGGAGGGTGGTGAGGGAGTTCCCGCGGCTGCGG
CTGAGCCAGGGGAGGATGGTGTTCGAGGTGCGGCCGACCATCAAGTGGGACAAGGGCAAGGCCCTC
GAGTTCCTCCTCGACTCGCTCGGTTTCGCCGACTGCAGCGACGTGCTGCCGGTCTACATCGGCGAC
GACCGCACGGACGAGGACGCGTTCAAGGTTTTGCGGCGGCGTGGGCAGGGCGTGGGGATCCTGGTG
TCCAAGCACCCCAAGGAGACGAGCGCCTCCTTCTCCCTCCAGGAGCCCGCCGAGCTGAAGAAAAGC
TCAGTGCACTACCCACTGCCGGCACAACGTAGCAACAGGCTGAACCGCATATTGGTCAATTGGTGG
GGTGAAGGACTTAAGGACAAATTGTGTGCTTTGGAGCCAAGCCATGAGAGGGAGGGGTGCATGGCC
CCCCACTAG

## FIGURE 8 (continued)

**SEQ ID NO: 58, protein - Oryza sativa**

MAKASVVVPEQVGAAAAAQVGCPCPGTTLFPYPPPRAGIAVRRKCLQAAQQLELGAGLRGGWVESM
RASSPTHAKAAAALAAGVDEEHAAWMVRFRSPIDRCSSRSWRARADTDMNRAFPFVFVQARHPSAL
GEFEKVVAASKGKQIVMFLDYDGTLSPIVDDPDAAFMSETVSLSSPPLSPTLLLHSSSSHTSLLPH
QMRMAVRSVAKHFPTAIVSGRCRDKVFEFVKLAELYYAGSHGMDIKGPASRHAAAKSPPHNKGVLF
QPASEFLPMIEQVHQRLEQATSSIPGAKVENNKFCVSVHFRCVDEKSWGALAETVRRVVREFPRLR
LSQGRMVFEVRPTIKWDKGKALEFLLDSLGFADCSDVLPVYIGDDRTDEDAFKVLRRRGQGVGILV
SKHPKETSASFSLQEPAELKKSSVHYPLPAQRSNRLNRILVNWWGEGLKDKLCALEPSHEREGCMA
PH

**SEQ ID NO: 59, DNA - Oryza sativa**

GCTCCGCCATCTTCTCTCCTCTCCCCTCTCACTCCTCCCAAATCCCCTCCATTGCCTTCCTCGCTT
CGCATCGCCGCTCCCGCCGCCGCCGTCGGGCTGCTCGCCGGCATTGGGTGTTGGTTGTGACCGTAT
ACAATTATTGTTTTTGGAGCTGAGCGATCAGATGCATGAGCCTTCCTTTTAAATGATCTGTGTCCA
CCATGGCGATGAACCAAATGCAGGAACTTCCCGCTTAATGGGCTGGATGCGTTGTATGCACGTTTG
CAGTGACAAGAAGACATTGAAACGTTGGTTTTTATTGACAAGAGGGTTGGTTGAGTGTCTAACTC
TTAAGGAATCCTATAGTCTACTTGCTTGCTCTTGTGTTGTTAGTAACTGTTCTTACAACAACTCTG
CGGAGAGTTCTGTTGATGGATTTGAAGACAAGCAACTCTCCTGTTATTGCTGATCCTCTCCCTAAA
TTAGCCTTGCCATCTGCTGTGATGACGTACACTACACCTACGAGTTTCCCCTCTACCGGGCTGTAC
TTGAACACTCCGAAAAAGAAGCCTCTGCCTGGAAAGATCGAAGAAGTCCGCGCCGCTGGATGGCTT
GATCTCATGCTGGCCTCCTCGCCACCTCGCAAGAGGCAGACTAAGGACTTTGCCAATGATGTTCAA
GCTGACGAGCTTGATTTGCTATACCGTAATTGGGTGGTGAACCATCCATCTGCTTTAACATCATTT
GAGGATATTGTTAATCTTGCCAGAGGTAAAAGATTGGCACTGTTCCTTGATTATGATGGAACTCTT
TCACCGATTGTGGACAATCCTGAAAATGCAGTAATGTCTGACGAGATGCGCTCTGCTGTGAAGCAT
GTGGCATCACTTTTTCCCACTGCGATCATTAGTGGAAGATCTCGTGATAAGGTTTTTGACTTTGTG
AAACTAACTGAACTGTATTACGCTGGAAGTCATGGAATGGATATCATGGGGCCTGTTAGGAAGTCT
GATTCGAGTGGTCAGCATGTGGAATGTATCAGGTCCACTGATTCAGAGGGTAAAGAGGTCAACCTC
TTCCAACCTGCAAGTGAGTTTTTACCTATGATTAGCGAGGTGTACAAAAAGCTCAGTGAAAGTATT
AAGGACATTGATGGTGCAAGGATGGAAGATAACAAATTCTGTGTGTCCGTTCATTACCGCAATGTA
GCACCACATGACTACGGAGAAGTTCATCAACGTGTGACTGCCGTCCTGAAGAATTACCCTTGTCTA
AGGCTTACCCATGGGAGAAAGGTTCTTGAGGTTCGACCTGTGATAGACTGGAACAAGGGGAAAGCT
GTGGAATTTTTGCTGGAGTCACTTGGACTATGTGGGAAAGAAGATGTTCTTCCTATCTATGTTGGA
GATGACAAGACTGATGAGGATGCATTCAAGGTTCTGAAGGCAAACAGCATTGGCTTTGGAATTTTG
GTGTCATCTGTGCCCAAGGATACTGATGCTTTCTATTCCGTACGGGACCCAGCTGAGGTGATGGAA
TTCCTGAAGAAACTGGCATCTTGGAAGGAGGAATCCACTTAAGCAGAAGGGAGGAACATAACAGAA
TCGTTTTTTGAGGATATGGAGAAGTACCAACTATAAATACGAGCAAAATATACACTGCTAACAAAT
AAGTCTGGCCAAAATGTTTTTGATTTGGCATCCTATTATATAAGAGGCTCCCTAAACCACCCTTTT
TTGGTCTCACTTTCATCTTATGTTTCTGGGATTCTGAGACATTTCATTCTTTTATTAGTTCTGTTC
CTTTAGTATATATAAAATATATACACATGTATGCTGAAAAATTTTGGTTCCTGTCCCTGTCGTCTT
AGGCAATTATTTATTCTACCGGCAAGTTGT

**SEQ ID NO: 60, protein - Oryza sativa**

MDLKTSNSPVIADPLPKLALPSAVMTYTTPTSFPSTGLYLNTPKKKPLPGKIEEVRAAGWLDLMLA
SSPPRKRQTKDFANDVQADELDLLYRNWVVNHPSALTSFEDIVNLARGKRLALFLDYDGTLSPIVD
NPENAVMSDEMRSAVKHVASLFPTAIISGRSRDKVFDFVKLTELYYAGSHGMDIMGPVRKSDSSGQ
HVECIRSTDSEGKEVNLFQPASEFLPMISEVYKKLSESIKDIDGARMEDNKFCVSVHYRNVAPHDY
GEVHQRVTAVLKNYPCLRLTHGRKVLEVRPVIDWNKGKAVEFLLESLGLCGKEDVLPIYVGDDKTD
EDAFKVLKANSIGFGILVSSVPKDTDAFYSVRDPAEVMEFLKKLASWKEEST

## FIGURE 8 (continued)

**SEQ ID NO: 61, DNA - Aquilegia spp.**

TCTCTCTCTCTCTCTCTCTCGCTCGCTCGCTCTCTGTGATTTCTAGGTTTTTCTTTTCGCTCTTAT
TGCTTCTAAAGGTTAGTTTTCGATTTCGTTTTCAACGATTTGAAGATTGGATCAAGTTCTGTGTTG
ATTTCTTCAAAGAAAAGAAACAGAATCGGTTTCTGAATATTCAGATATCAGACAATATCTTCGTAA
GCTACATATTTCCTCCACTTGTTTTGTTTACAAAATGTCTGGTCATCTTCATTAAATGCTGTGTAT
CTTTGGAGATGAAAAGCTTTCAGGAAGCCTGCACTTGATGGACTGCATGCCCAATAGCAGTGATAA
GAAAACTTTGAAGAGAACTTACAAACCGAAAAAACTTAATCTGGATCCAATGGATATAAAGTCAAA
CCATGCTGCACCTGTTCTCACTGACCCTGTCCCCATAACCAAATCAAGATTAGGCATTCATTCTAG
CTTGTTGCCTTACTCGCCGGGAGCCACGTTTTCTTCTGGCATGTACATAACAATTCCTAGAAGGAA
GGTCATTCCTAGCAAGCTTGATGACATTCGTTCCAGTGGGTGGCTAGATGCGATGAAATCCTCATC
CCCTCCTCGCAAAAAGCTAACTAAAGATTCCAATATTGAGGTTGCTGCTGATGATAGCCAACTCGT
TTACCGCTCATGGATGCTCAAGTATCCATCAGCACTTGCCTCTTTTGAGCAAATTATGAATTATGC
AAAGGGGAAGAGGATTGCATTGTTTCTGGATTATGATGGCACACTTTCACCAATCGTAGATGACCC
TGAGCGTGCTTTTATGTCAACTGCTATGCGTTCTGCTGTGAAAAATGTCGCAGTGTGTTTCCCCAC
TGCAATAATTAGTGGAAGAAGTCGTGATAAGGTATACGAGTTTGTAGGACTAACAGAACTCTATTA
TGCTGGCAGTCATGGAATGGATATAATGGGACCTGTAAGACAGTCCGATACTGGCGATAACCATTC
AAACTGTATTAGATCTACTGACAAGCAGGGTAAAGAAGTAAATCTTTTCCAACCTGCTAGTGAATT
TTTACCGATGATCGATGAGGTTTTTAGCTCCCTTGTCGAATTTACTAAAGGAATTGAAGGTGCAAA
AGTTGAAAATAACAAGTTTTGTGTCTCTGTACATTACCGCAATGTTGACGAGAAGAATTGGACTAC
AGTTGCACATGGTGTTCATGGCATCTTGAAGGATTACCCTCGTCTGCGGTTAACTCATGGGAGGAA
GGTTTTAGAGGTTCGTCCCGTGATTGACTGGGACAAGGGAAAGGCAGTAGAGTTTCTGCTTGATTC
ACTTGGATTGAGTGAAAGTGATGACGTGCTCCCCATATATGTTGGTGATGACCGGACAGATGAAGA
TGCCTTTAAGGTTTTGAGGGAGAAGAATAGAGGTTATGGCATTTTAGTGTCATCTGTGCCAAAGGA
AAGTAATGCTTTCTATTCTCTCAAAGATCCATCCGAGGTTATGTCATTTCTCAAATCGTTGGTAAG
ATGGAAGAAGTCCAGAATATAAAGGAAGGAATTAACCAGTTTAGGTAGAAGTTCTATAGTCTGAAC
TCTGAAGATTCTAACAAATAAATCAAGATGGAGCTGCGCTGTTGAGGTGTTTTCTTGACACTGTGG
AACTGGATGAAATTGTTGGGGTTTATAAGCTCTCTTACATTTTTGTAAATCCTTTCTGAGGCTCGT
GTTTAGCGATTTTCTTATCTATGAAAGTTAATTTTTTTGTTCTGTGTTGCAATTTTGGTCCATTAT
TTTAGGTCTCTGAGGGAAATTTTTACTTAATCTTATAAATATAAGATG

**SEQ ID NO: 62, protein - Aquilegia spp.**

MLCIFGDEKLSGSLHLMDCMPNSSDKKTLKRTYKPKKLNLDPMDIKSNHAAPVLTDPVPITKSRLG
IHSSLLPYSPGATFSSGMYITIPRRKVIPSKLDDIRSSGWLDAMKSSSPPRKKLTKDSNIEVAADD
SQLVYRSWMLKYPSALASFEQIMNYAKGKRIALFLDYDGTLSPIVDDPERAFMSTAMRSAVKNVAV
CFPTAIISGRSRDKVYEFVGLTELYYAGSHGMDIMGPVRQSDTGDNHSNCIRSTDKQGKEVNLFQP
ASEFLPMIDEVFSSLVEFTKGIEGAKVENNKFCVSVHYRNVDEKNWTTVAHGVHGILKDYPRLRLT
HGRKVLEVRPVIDWDKGKAVEFLLDSLGLSESDDVLPIYVGDDRTDEDAFKVLREKNRGYGILVSS
VPKESNAFYSLKDPSEVMSFLKSLVRWKKSRI

**SEQ ID NO: 63, DNA - Aquilegia spp.**

CCTTCACTAAGCTTTTCTCAGTTTCTCTCTTTCTTTCTAAACTCTTTCTTTCTCTGTTCTTCTAAT
TATAATCTTTATTTTGGTTCTCATTAGTTGTTTCTTTTAACTGAACTTCAGAGAACTTTTCTTAAT
ATCTCTTCTGTGAATCTTTAGTCTTTCTTGAAAGAAAAATCCAATCTTTTTACAAGTTGTGGTTTG
ATTTGGGGTTTTAGAATCTGAACTAAAAACCTCAAATCTAGTTGAATTTCCTGTTTGGGTGGTTAT
TTATAGTACATTTGGTTAAAGATGACTAAGCAAAATGTGGTGGTTTCAGATGTAAAATCTGGGTTA
GCAATAACAGTTGCAGTGTCGAATTCGTCTCTGTTTTCTTCTGCTGTGCAGAAACCATTAACAACA
CCTGGAGGTTATATAACTATATCTCGTAGCAAGCTTTTAAAGAAGCTTGAAGAAACAGGTGGAGTT
ACAGAAGGGAGAATTAATGCTTGGGTTGAATCCATGAGAGCATCTTCTCCTACTCGTATTAGACCG

**FIGURE 8 (continued)**

GCAGTTTCTTTGACTCCAAACAGAGATGAAAACTCATGGATGCTCAAACATCCTTCAGCACTTAAT
ATGTTTGAAGAAATAACAAATGCTTCTAAAGGGAAGCAAATTGTGATGTTCTTAGATTATGATGGT
ACTCTTTCTCCTATTGTCGACGATCCAGATCGTGCTTTCATGTCTGAATCGATGAGAAGAGCTGTT
AGAGATGTTGCAAGATACTTTCCTACTGCAATTGTGAGCGGGAGATGCAGAGATAAGGTGTATAGC
TTTGTACGCTTAGCAGAACTGTACTATGCTGGAAGTCATGGTATGGACATTAAAGGACCAACCAAA
AGTTACAAAAACAAGAAAAGGAACCAACCTGTATTGTTTCAACCAGCAAATGAGTTTTTGCCCATG
ATTGATGAGGTTTACAAAGCATTGTTAGAGAAAACCAAATCAACTCCAGGTGCTAAAGTGGAGAAC
AATAGGTTCTGTGTATCTGTGCATTTTAGATGTGTGGATGAAAAGAGTTGGACTGAACTAGCAGAA
CAAGTTAGATCAGTTCTTAAAGAATACCCAAAGCTTAGACTGACTCAAGGAAGAAAGGTACTAGAG
ATCCGTCCTACTATTAAATGGGACAAAGGGAAGGCTCTTGAATTTTTGTTAGAGTCTCTTGGATTC
GCCAACTGCAACAATGTTCTGCCACTGTACATTGGAGACGATCGAACCGATGAAGACGCCTTTAAG
GTCTTGCGCGATAGAGGACAAGGATTCGGCATTCTCGTTTCTAAAGTTCCAAAAGAAACAAATGCT
TCTTATTCTCTTCAAGAACCTTCAGAGGTTATGGATTTCCTTCACCGGTTAGTTGAATGGAAGCGT
ATGTCAGTTCGAGGGCATTCTAGAGTATAGTAAAAGAGCAACTTTCCCATCTAGGAGTGACAATGA
TCCAAGACTGGGGAAAATTATATGTATTTTCCTTTGTTCAGGAAAAAAGTTAAAAGAAAAAAAAAA
GGCTTTTCTCCTTTGCTTTTCTTTGGGTTTGGGGGCCTCCTTGTAAGTTGTAACTACTGATGTACA
AGGGGAAATGTTTTCACCAAAGCAGCCTAATCCCATTATCAGGGGAAGGCAAACATTACATTTG

**SEQ ID NO: 64, protein - Aquilegia spp.**
MTKQNVVVSDVKSGLAITVAVSNSSLFSSAVQKPLTTPGGYITISRSKLLKKLEETGGVTEGRINA
WVESMRASSPTRIRPAVSLTPNRDENSWMLKHPSALNMFEEITNASKGKQIVMFLDYDGTLSPIVD
DPDRAFMSESMRRAVRDVARYFPTAIVSGRCRDKVYSFVRLAELYYAGSHGMDIKGPTKSYKNKKR
NQPVLFQPANEFLPMIDEVYKALLEKTKSTPGAKVENNRFCVSVHFRCVDEKSWTELAEQVRSVLK
EYPKLRLTQGRKVLEIRPTIKWDKGKALEFLLESLGFANCNNVLPLYIGDDRTDEDAFKVLRDRGQ
GFGILVSKVPKETNASYSLQEPSEVMDFLHRLVEWKRMSVRGHSRV

**SEQ ID NO: 65, DNA - Brassica campestris**
ATGGAGAAACCAAACAGGATGTCAGAGAGTCAAAACGTTGTCGTCTCAGAGGCGGCAAGGTCTATC
ATCCCCAACAACTCTTCGGCTCCTCCTGGTTTCATCTCAATCTCCAAGAAAAAGCTTCTCAAGAAC
CTAGAAATCATCAATGATGGCGAAAGAATCAACGCTTGGGTAGATTCAATGCGAGCTTCTTCTCCT
ACTCATCCAAAATCACTCCCTTCTTCCATCTCCTCAGAGCAACAACTCAGTTCATGGATCATGCAG
CATCCTTCTGCATTAGAAATGTTTGAGAAAATCACAGAAGCTTCGGGAGGGAAACAAATCGTAATA
TTTCTAGATTATGACGGTACTCTCTCTCCCATCGTTGATGATCCAGACAGAGCTTTCATGTCAAGC
AAGATGAGAAGAACAGTAAAAAAACTGGCTAAGTGTTTTCCAACTGCTATAGTTACTGGTAGATGC
CTAGACAAGGTGTATAACTTTGTCAAGCTAGCTGAGCTGTATTATGCTGGCAGCCATGGCATGGAC
ATTAAAGGGCCAGCAAAAGGCTTCTCCAGACACAAGAGGGTTAAACAGTCTCTTCTGTACCAACCA
GCCAGTGATTATCTTCCCATGATCGATGAAGTCTATAGACAACTTTTGGAGAAAACCAAATCAACT
CCTGGAGTCATAGTAGAAAACAACAAGTTCTGTGCTTCTGTGCACTTTCGTTGCGTCGATGAGAAG
AAATGGAGCGAACTGGTTCTACAGGTTCGGTCGGTATTAAATGAATACCCTAGGCTTAAACTGAAC
CAAGGTCGAAAGGTTTTCGAAATACGTCCTATGATTGAATGGGATAAAGGAAAAGCTCTTGAGTTC
TTGTTAGAGTCACTTGGGTTTGGAAACTCTAACAACGTTTTCCCAGTTTACATCGGTGATGACCGG
ACCGACGAAGATGCATTTAAGCTGCTACGAGACAGAGGTGAAGGCTGTGGCGTTCTTGTCTCCAAA
TTCCCCAAAGATACGGATGCTTCATATTATTTGCAAGATCCGTCCGAGGCAAGTGATGAATTTCTT
GCAACGATTGGTGGCGTGGAAACAAATGCAGCCAAGAGTGTGAAGAGGATGTATGTATAA

**SEQ ID NO: 66, protein - Brassica campestris**
MEKPNRMSESQNVVVSEAARSIIPNNSSAPPGFISISKKKLLKNLEIINDGERINAWVDSMRASSP
THPKSLPSSISSEQQLSSWIMQHPSALEMFEKITEASGGKQIVIFLDYDGTLSPIVDDPDRAFMSS

**FIGURE 8 (continued)**

KMRRTVKKLAKCFPTAIVTGRCLDKVYNFVKLAELYYAGSHGMDIKGPAKGFSRHKRVKQSLLYQP
ASDYLPMIDEVYRQLLEKTKSTPGVIVENNKFCASVHFRCVDEKKWSELVLQVRSVLNEYPRLKLN
QGRKVFEIRPMIEWDKGKALEFLLESLGFGNSNNVFPVYIGDDRTDEDAFKLLRDRGEGCGVLVSK
FPKDTDASYYLQDPSEASDEFLATIGGVETNAAKSVKRMYV

**SEQ ID NO: 67, DNA - Brassica rapa**

ATGGTGAGCTTTGTCGTGGAGAAACCACAGAGAATGTCAAACGGTGTCGTATCAGAGACCACAAGG
TTAAGTATCATCCCTAACAACTCTTCCTCTGCTCAGAAAACGCTTCTCAAGAACCTCGAGATCATC
AATGGTGGACAAAGAGTCAACGCTTGGGTCGATTCAATGCGGGCTTCTTCTCCTACTCATCTCAAA
TCACTACCTTCTTCTGTCTCCTCAGAGAAACACCTCAGCTCATGGATCATGCAGCATCCTTCAGCA
TTAGAAATGTTTGAGAAGATCACACAGGCTTCAGGAGGGAAACAAATCGTAATGTTTCTTGATTAT
GATGGTACTCTCTCTCCCATCGTTGATGATCCAGACAAAGCTTTCATGTCAAGCAAGATGAGAAGA
ACTGTGAAAAAATTGGCTAAATGTTTCCCAACTGCAATAGTTACCGGTAGATGCATAGACAAGGTG
TATAACTTTGTGAAGCTGGCTGAGCTGTATTATGCTGGAAGCCATGGCATGGACATTAAAGGTCCA
GCAAAAGGTTTCTCCAGACACAAGAGGGTTAAACAGTCTCTGTTGTACCAACCAGCCAGTGACTAT
CTTCCCATGATCGATGAAGTCTATAAACAGCTCTTGGAGAAAACTAAATCAACTCCTGGAGTCATA
GTAGAAAACCACAAGTTTACCGCTTCTGTGCACTTTCGTTGCGTGGAAGAGAAGAAATGGAGCGAA
CTGGTTCTACAGGTTCGGTCGGTATTAGAGAAATATCCTACGCTCAAACTGAGCCAAGGTCGGAAG
GTTTTCGAAATCCGTCCTATGATCGATTGGGATAAAGGAAAAGCTCTTGAGTTCTTGTTAGAGTCA
CTTGGGTTTGGGAACTCTAACAACGTTTTCCCGGTTTACATCGGCGACGATCGGACCGACGAAGAT
GCATTTAAGATGCTACGAGTCAGGGGTGAAGGCTTTGGCATACTTGTCTCCAAATTTCCCAAGGAT
ACAGATGCTTCGTATTCTCTGCAAGATCCGTCCGAGGCAAGTCCACACACGCGTATATTATACCTT
TCTGTTCCTAAATATAAGATGTTTAGCAGGGCCGATTTATAA

**SEQ ID NO: 68, protein - Brassica rapa**

MVSFVVEKPQRMSNGVVSETTRLSIIPNNSSSAQKTLLKNLEIINGGQRVNAWVDSMRASSPTHLK
SLPSSVSSEKHLSSWIMQHPSALEMFEKITQASGGKQIVMFLDYDGTLSPIVDDPDKAFMSSKMRR
TVKKLAKCFPTAIVTGRCIDKVYNFVKLAELYYAGSHGMDIKGPAKGFSRHKRVKQSLLYQPASDY
LPMIDEVYKQLLEKTKSTPGVIVENHKFTASVHFRCVEEKKWSELVLQVRSVLEKYPTLKLSQGRK
VFEIRPMIDWDKGKALEFLLESLGFGNSNNVFPVYIGDDRTDEDAFKMLRVRGEGFGILVSKFPKD
TDASYSLQDPSEASPHTRILYLSVPKYKMFSR

**SEQ ID NO: 69, DNA - Gossypium hirsutum**

CGCCGCCGGTCAAAATGGTCTCTGATCCAAACTCTGCACAGAAACCACCGGCACCACCTGGGTTTT
ATATCCATTTCCAGAAAGAAATTGCTTCAAAACCTTGAAATCAATGCCGGAGCTAGAGTTAATTCA
TGGGTCGATTCCATGAGAGCTTCTTCTCCAACTCATATGAAATCAGCACCGTCCATTGCTGATGAT
CAAGGTTCCTGGAATCTAAACCATCCATCAGCACTAGATATGTTCGAACAGATAATCGATGCATCA
AAAGGGAAACAAATCGTAATGTTTCTTGATTACGATGGCACTCTTTCACCAATCGTAGCCGATCCA
GATCGGGCTTTCATGTCTAAGAAGATGAGAAAGACAGTGAGAAAGCTAGCCAAATGTTTCCCTACA
GCTATAGTGAGTGGCAGATGCAGGGACAAGGTGTATAATTTTGTCAAATTAGCTGAGCTATACTAT
GCTGGAAGCCATGGCATGGACATTAAAGGCCCTGAAAAAGGTTCCAAATCTAACAAAGATACTGAA
TCTGTTCTTTTCCAACCAGCTAGTGAATTTCTTCCCATGATTGATGAGGTTTATAAACAGTTGGTT
GAAACTACAAAATCAACACCAGGTGCTAAAGTGGAGAACAACAAGTTTTGTCTCTCAGTACACTTC
CGTTGTGTTGATGAAAAGAAATGGAGTGAATTGGCACAACAAGTGAAGTCTGTTTTAAAAGACTAC
CCCAAGCTTCGGCTAACTCAAGGCCGAAAGGTTTTGGAAATCCGTCCTACAATCAAATGGGACAAA
GGGAAAGCCCTTGAATTTTTGTTAGAATCTCTTGGATTTGCTAACTGTACCGATGTCTTTCCCGTT
TATATCGGAGATGATCGGACCGATGAAGATGCATTCAAGATATTGAGGGACAGAGGCCAAGGTTTT
GGTATTCTTGTATCCAAGTTCCCCAAAGACACTAATGCATCCTATTCATTACAAGAACCAGATGAG

**FIGURE 8 (continued)**

GTCATGGACTTTTTACGACGTCTGGTTGAATGGAAAGAATTATCTCTAAGAACTCAGTCGAGAATG
TAGAAGAAAATAAGACAGTGGAAGTAAATATTTGTGCACCCTAGCAAAGAAATTGTAAAGGGGGGT
GTGAGATTAAAGTAGTAAAATTATTGTTTTAAGATGAGAGAAATGATTATAAAAGCTTTGTACATC
TTTGTAACTAGTTGGGGTACAAAGGGTCAAGTCTTGTAGCTTCATAACTTGTCTATTAATGCATAG
GAAAGCAATGGTTTCGGATTAATGTT

**SEQ ID NO: 70, protein - Gossypium hirsutum**
MRASSPTHMKSAPSIADDQGSWNLNHPSALDMFEQIIDASKGKQIVMFLDYDGTLSPIVADPDRAF
MSKKMRKTVRKLAKCFPTAIVSGRCRDKVYNFVKLAELYYAGSHGMDIKGPEKGSKSNKDTESVLF
QPASEFLPMIDEVYKQLVETTKSTPGAKVENNKFCLSVHFRCVDEKKWSELAQQVKSVLKDYPKLR
LTQGRKVLEIRPTIKWDKGKALEFLLESLGFANCTDVFPVYIGDDRTDEDAFKILRDRGQGFGILV
SKFPKDTNASYSLQEPDEVMDFLRRLVEWKELSLRTQSRM

**SEQ ID NO: 71, DNA - Hordeum vulgare**
CAGCCCTCCACCGCATCCGGGCCGGAGACACCTCCCTGGCCGCCATCGAGCATGCCGGTCGTGTCA
GAGGTGGGCATCACGGTGACCGCGGCCACGGCCACGGCGTGCCCCTGCCCGGGGTCGCTGTTCCCG
TACCCGCCGCCGCGTGCCGGGATGGCCGTGAGCCGGAAGTGCCTGCGGGCGGCGCAGGCGGAGCTT
GGCGCGGGGATGCTCAGTGGCCTGGTCGAGTCCATGCGGGCGTCGTCCCCCACGCACGCCAGGGCC
GCTGCCGCCCTCGCTGCCGGCGTCCGACGACGAGCACGCGGCCTGGATGGCCAGGCACCCCTCCGC
CTTGGCCAAGTTCGAGGAGATCGTGGCCGCCTCCAAAGGGAAGCAGATCGTCATGTTCCTCGACTA
CGACGGCACACTGTCCCCCATCGTCGATGACCCCGACGCCGCCTTCATGAGCGAGACGATGCGGAT
GGCCGTGCGCAGCGTGGCCAAGCACTTCCCGACGGCGATCGTCAGTGGTCGGTGCCGCGACAAGGT
GTTTGAGTTCGTGAAGCTGGCGGAGCTCTACTACGCCGGCAGCCACGGCATGGACATCAAGGGCCC
GGCCAAATCCTCTTCCGGGCACGCAAAGTCCAAGGCCAAAGGAGTTCTCTTCCAACCAGCAAGCGA
GTTCCTGCCCATGATAGAAGAGGTGCATCAACGCCTGATAGAGGAGACCAAGCACGTAGCCGGCGC
CAAGGTGGAGAACAACAAGTTCTGCGTCTCCGTCCACTTCAGATGCGTCGACGAAAAGAGCTGGGG
CGCGCTGGCGGAGACGGTGAAGGGGGTGATGCGGGAGTACCCGAAGCTGCGCATGTCGCAGGGGCG
GATGGTGTTCGAGGTGCGGCCCACCATCAAGTGGGACAAGGGCAAGGCCCTCGAGTTCCTGCTCGA
GTCGCTGGGCTTCGCCGACTGCAGCAACGTGCTGCCCGTCTACATCGGCGACGACCGCACCGACGA
GGACGCCTTCAAGGTGCTGCGGCGGAGGGGCCAGGGCGTCGGGATCCTCGTCTCCAAGCACCCCAA
GGACACCAGCGCATCCTTCTCGCTGCAGGAGCCCGCCGAGGTCATGGAGTTCCTCCTCCGCCTCGT
CGAGTGGAAGCAGCTCTCCAGGGCGCGCCTCAGGCTGCGGCGACAGGCCCACGCCTGATCGGACGA
CCACGAGGATCGGCCGGCCGGGCTAATTAATTCACTACGCGTAGCTTAGTTAGTTGATGAATCCCC
TGCTCATCGTCATCGTATGAAAGCCAAAGGCTACGTGGATGAGAAGAAGGAACCGTACCACGGCGA
GCCTGTGGGGCATTGTGGCGCCACATGCTGTGTTCTAACCACGCCCTTCTTTTTCTGGGGATGTAT
ACCTACCGAGTAACGACTATGCATGCATGTAAATTACCCTGGTACGTCCACGTACAAGGGGAGGAA
CCATGGAATAAAGAAACAGAAACCTGAGTAAATAGCAACAACCAAGCCTTTTGCCTC

**SEQ ID NO: 72, protein - hordeum vulgare**
MARHPSALAKFEEIVAASKGKQIVMFLDYDGTLSPIVDDPDAAFMSETMRMAVRSVAKHFPTAIVS
GRCRDKVFEFVKLAELYYAGSHGMDIKGPAKSSSGHAKSKAKGVLFQPASEFLPMIEEVHQRLIEE
TKHVAGAKVENNKFCVSVHFRCVDEKSWGALAETVKGVMREYPKLRMSQGRMVFEVRPTIKWDKGK
ALEFLLESLGFADCSNVLPVYIGDDRTDEDAFKVLRRRGQGVGILVSKHPKDTSASFSLQEPAEVM
EFLLRLVEWKQLSRARLRLRRQAHA

## FIGURE 8 (continued)

**SEQ ID NO: 73, DNA - Medicago truncatula**

TGTCTCTCTCTAGTTCTAAATATTCCCTATCTCTCTTTTGCCTCTTCAATTTCCCTTCTTCCTCTT
TTGCATTCCATTGCAATTATCCACTTCTCACACCCCACCTCTTTAACCTATTTTCTATTTGTTTCC
TCACAAACCCTAAGTGAAGATGACTCAAAAGAATGTGGTTGTGTCTGAGACCAAAACCGGAATCAA
TGGTAGCGGTACTATTACCGTGGCACAGAAGCCACCGGCAGCACCCGGTGGATACGTTCACATTCC
AAGGAGGAGAATTTTGAAGAATCTTGAAATCAATGGAGGACAAAGAATCAATACATGGATTGATTC
TATGAGAGCTTCTTCTCCAACTCATGTCAAATCTTCTCCTTCTTTAGCTGAAGAATACAATTCTTG
GATTCTTCGCCATCCATCTGCATTAGATATGTTTGAACAAATTATGGATGCTGCAAAAGGAAAACA
GATTGTTATGTTTTTGGACTATGATGGTACTTTGTCACCTATTGTCGATGACCCTGACCGTGCTTT
CATGTCTGAATCGATGAGGAAAACAGTTAAGAAACTTGCAAGGTGTTTTCCTACTGCAATTGTTAC
TGGAAGATGCATAGATAAAGTGTACAATTTTGTCCGTTTGGATGAACTATATTATGCTGGAAGTCA
TGGCATGGATATTAAAGGGCCAACAAAAGAGTCCAAATACAACAAAAATAACAAAGCTGAGGAAGT
ACTTTTTCAACCTGCCAGAGAATTTGTTCCCATGATAAATGAGGTGTACGAACAACTAGTTGAGAA
AACAAAATCAACTCCTGGTGCCAGAGTTGAGAACCACAAGTTCTGCACCTCTGTTCATTTTAGATG
TGTTGATGAAAAGAGATGGGTTGAATTGGCACAGCAAGTAAAATCCGTGTTAAAAGAGTACCCGAA
GCTTCGTCTAACACAAGGAAGAAAGGTATTGGAGATTCGTCCCGCAATTAAATGGGACAAGGGCAA
GGCCCTCGAATTTTTGCTAGAGTCACTTGGATTTGCCAACTGTAACGATGTATTTCCTGTTTATAT
TGGTGATGATAGAACCGATGAAGATGCATTCAAGAAATTAAGAGACATAGATCAAGGTTTTGGAAT
TCTAGTCTCTAAGTTTCCAAAAGACACAGCTGCTGCATACTCTTTACAAGAGCCTAATGAGGTTAT
GGAGTTCCTTCAGCGTTTGGTGGAGTGGAAAAAAACATCTCCAAGATCACGTTCTAGGGTGTAAAT
GAAGACTTTGTACATCCCTGACTGGCCATAAAATACATGACCCATATTCATTTTTTAATTTGTCCA
TCAATTTTCTAACTAATAAATCCTAGAGTCAACAGGATTTGCCAACTGTAACGATGTATTTCCTGT
TTATATTGGTGATGATAGAACCGATGAAGATGCATTCAAGAAATTAAGAGACATAGATCAAGGTTT
TGGAATTCTAGTCTCTAAGTTTCCAAAAGACACAGCTGCTGCATACTCTTTACAAGAGCCTAATGA
GGTTATGGAGTTCCTTCAGCGTTTGGTGGAGTGGAAAAAAACATCTCCAAGATCACGTTCTAGGGT
GTAAATGAAGACTTTGTACATCCCTGACTGGCCATAAAATACATGACCTTTTTTATTTTTCTTTCT
ATGTTTTGTTCAGATTAGTACAAAAGAGCAAAAATGTATGGAAAAAAAAAAATGAAAGACAAAAGTG
TCTTTCTGAGTTTGAAGCTCGATTGTCTACATTGATGTAATTAGCTTAGCCAAGTAGTAATTACTA
ATTAGTACGGGGGGAAAGTATATGTACTAACCATATCAGATCAGATCAGATGAAGTGATTCTGAAT
GGGTTAAAATTTGTACCCTTATGATTATGATGATTAAATATTATAAGGTTACGTATCAAAAGATAT
GTATTTATAGTGACATGCTTATTAAGTACTT

**SEQ ID NO: 74, protein - Medicago truncatula**

MTQKNVVVSETKTGINGSGTITVAQKPPAAPGGYVHIPRRRILKNLEINGGQRINTWIDSMRASSP
THVKSSPSLAEEYNSWILRHPSALDMFEQIMDAAKGKQIVMFLDYDGTLSPIVDDPDRAFMSESMR
KTVKKLARCFPTAIVTGRCIDKVYNFVRLDELYYAGSHGMDIKGPTKESKYNKNNKAEEVLFQPAR
EFVPMINEVYEQLVEKTKSTPGARVENHKFCTSVHFRCVDEKRWVELAQQVKSVLKEYPKLRLTQG
RKVLEIRPAIKWDKGKALEFLLESLGFANCNDVFPVYIGDDRTDEDAFKKLRDIDQGFGILVSKFP
KDTAAAYSLQEPNEVMEFLQRLVEWKKTSPRSRSRV

**SEQ ID NO: 75, DNA - Medicago truncatula**

AACACACCCATCTCTCTGTTCTTCTCTGTCTTTTCCCTCTCTAGTTTTTTTCTTATTCTCAATTT
CTCATAACAATGACTAACCAGAATATGTTGGGAAAGTCACGTGTGATTGTGAATGAGAAGATATTG
GAATTAGCAATGTCGATTTCAAACTCTAATGTTTTACCAAGAACTTCAATGCCTGAATTAATGGCT
TTGTTTGATGGGCTTTTAGGCCAGCGCAAAAACAATCTCATTAAGCCTTTGGAAGATGATAATGAT
CACAAAGGAGCAACTAAAGTTAACGCGTGGATTGATTCCATGAGAGCTTCATCTCCTACTCGAACC
AGACACGATTCAGAAAATCGTGACCAGACTCATTGGACTCTTTTCCATCCTTCTGCGTTGAACATG
TTCAGTAAAATAATGTACAATACAAATGGGAAACAAATTGTTGTTTTTCTTGACTATGATGGAACT

## FIGURE 8 (continued)

CTCTCCCCAATTGTAGCAGATCCAGATAAAGCCTACATGAGCAAAAAGATGAGAGTGACATTGAAG
GACATAGCAAGACATTTTCCCACTGCCATTGTTAGTGGAAGGTGCTTAAACAAGCTATTTAGCTTT
GTAAGGTTGGCTGAATTATACTATGCTGGAAGCCATGGGATGGATATTAAGGGTCCAACAAATAGG
AGAAGCACTAAAAAAGGTAATAATGATGGGGTACTTTTGCAGCCTGCTAGTGAATTTTTGCCCATG
ATCAATGAGGTTTATAAGATCTTGGTGGAGAAAACAAAGTGTGTTCCAGGGGCTATGGTGGAAAAT
AACAAGTTTTGTTTATCTGTGCATTTTCGGAACGTTGACGAAAAGAGTTGGGAAGCATTGGGTGAA
CAAGTAAGCTTAGTGATGAATGATTACCCAAAACTAAAGCTAACACAAGGGAGAAAAGTGTTGGAA
ATTCGACCAATTATTAAATGGGACAAAGGAAGGGCTCTTGAATTTTTGCTAGAGTCACTTGGTTTT
GCAAATTCTAAAGGAGTATTTCCAATCTATATTGGTGACGATAGAACCGACGAAGATGCTTTTAAG
GTTTTACGCAATAGGGGCCAAGGGTGTGGGATTCTTGTTTCAAAAATTTCAAAAGAAACTAATGCT
TCTTACACTTTGCAAGATCCATCTGAGGTTGGAGAATTTTTGCGGCATTTGGTGGATTGGAAAAGA
ACAAGTTCTCGTTCTCACAAGTTGTAGAGAGAGCTAGGAATCTATAAATGAGTTTAGGGATTTGAC
ACCGACCCAAGAATCTGGTCAAGGGGTAGTTAAAATGGCATCCCTTGTTTGAAAATAGGAAAATAG
TACATTTTATTGTTCCATAATTTTAATATTTTAGGGACTAGAAAGTCCAAATAGATTCTCTTTTTC
TTTTTTTCCTTTTCTTGTTTCAATGTATAATTCTATTCTTGATCTTTCACACGTTTGCATGCGCAT
GCGGATAGTGAAAGACATATGTTTTATGCCTCATTTGTTATATGAGACATTATAACTTTCTTACTC
TCTACTGTACTTAATGTACGTTTGGCAAATGTAATACTCATAATGAAAATTGCCAATTTCATCTT

**SEQ ID NO: 76, protein - Medicago truncatula**
MTNQNMLGKSRVIVNEKILELAMSISNSNVLPRTSMPELMALFDGLLGQRKNNLIKPLEDDNDHKG
ATKVNAWIDSMRASSPTRTRHDSENRDQTHWTLFHPSALNMFSKIMYNTNGKQIVVFLDYDGTLSP
IVADPDKAYMSKKMRVTLKDIARHFPTAIVSGRCLNKLFSFVRLAELYYAGSHGMDIKGPTNRRST
KKGNNDGVLLQPASEFLPMINEVYKILVEKTKCVPGAMVENNKFCLSVHFRNVDEKSWEALGEQVS
LVMNDYPKLKLTQGRKVLEIRPIIKWDKGRALEFLLESLGFANSKGVFPIYIGDDRTDEDAFKVLR
NRGQGCGILVSKISKETNASYTLQDPSEVGEFLRHLVDWKRTSSRSHKL

**SEQ ID NO: 77, DNA - Nicotiana benthamiana**
GATCTTCATCCCTAACTCCTATTCCAATCCCAATTTGTCTTCCTACTTTTTTTTTTCTAGCATTGC
GAGTAAAGAAACCTTTCTTCGGCTTCGTCTTAACCCAACTCTCATTCCCATTCCCCATTTTTCTTT
TGCTACAAAACAGAGTGAAAGCAGAGTACACTATCCCCTGTTCTTCAAGATTGTATTAACTGATTA
GTTGAACATAATTTAGGAGTGACGATGACTCAGCAGAATGTGGTAGCGTCCGACCCTAAATCCGGT
ATTAATTTGGCAATACCAGTGAAGGTACAAGTACCATCAAACTCCCCCGCGCTGTTCACGACAGTG
GCACAGAAGCCGCCACCGGCACCGGGGAGTTGTATCACCATTTCAAGAAAAACACTTGTTGAAATC
AATGGGAATAACACTGGTGCTAGAATCAACTCTTGGGTTGATTCAATGAGAGCTTCCTCTCCTACT
CATCACAAGTCCACTCCTCCTCTTTCTGATGACATCAATTCTTGGATGGTGCAACATCCATCAGCA
CTGGATATGTTTGAGCAGATAATAAGTGCTTCAAAGGGAAAGCAAATAGTGATGTTTTTAGACTAT
GACGGCACACTCTCCCCCATTGTTGAGGATCCTGACCAAGCTTTCATGTCTGATGCTATGAGAGCA
ACAGTGAGAAAGCTTGCTAGATATTTCCCTACTGCAATAGTGAGTGGAAGGTGCAGAGACAAGGTA
TACAACTTTGTACGATTGGCAGAGTTGTACTACGCTGGAAGCCATGGAATGGATATAAAAGGACCA
TCAAAAGGTTCCAAATACAAGAAAGGAGCAGAAGCTGTTCTTTGCCAACCAGCAAGTGAATTTCTA
CCAATGATTGATGAGGTTTACAAAGCATTAATTGATGCAACAAAATCTACAGAAGGAGTTATAGTG
GAGAATAACAAGTTTTGTGCCTCTGTGCATTTCCGCTGTGTTGATGAAAAGAAATGGGGTGAACTA
GCACAAGTCGTAAGGTCAGTGCTTAAAGAATATCCAAAGCTGAGATTAACACAAGGAAGAAAAGTA
TTTGAGATCCGTCCTACTATTAAATGGGACAAAGGCAAAGCTCTTGAATTCTTGCTTGAATCCCTT
GGATATGCTAACTGTACTGATGTATTTCCTGTATATATTGGTGATGACCGAACCGATGAAGATGCT
TTTAAGGTTCTACGAGAAAGAGGACAGGGTTTTGGCATTCTTGTCTCCAAAATTCCAAAAGACACA
CATGCATCTTATTCTTTACAAGAACCATCTGAGGTTATGGTGTTTTTACGACGCTTGGTAGAGTGG
AAAAAGTTGTCGTTAAGAAGACAGTTTAGAATTCGAAGGCAAATTGAAGAGATAAAAGCATCTCTA

## FIGURE 8 (continued)

CGGAACTAATGAAGAATTATTGTCCAAAAGTAATGGACTTAATGATGCATATAATGTATTTTCCTT
TTATAAATATAACAAAAGAAGTTGTAAAAGAAAAGGTGGAAAAGATAGGCTTTTTAATCCTTTTCT
ATTTGGCTTTAAGGCCTTGTACATCTTTGTAACTAGCTCTGTACAAGGGTAAATTGTAATTTTCTA
GTCTTAGCTAATGCATATATTGGAAGTTCTCTCTCTAAGTACATATATACTCCCTAAATTGAAAGA
TGACACATTTCGCTT

**SEQ ID NO: 78, protein - Nicotiana benthamiana**
MTQQNVVASDPKSGINLAIPVKVQVPSNSPALFTTVAQKPPPAPGSCITISRKTLVEINGNNTGAR
INSWVDSMRASSPTHHKSTPPLSDDINSWMVQHPSALDMFEQIISASKGKQIVMFLDYDGTLSPIV
EDPDQAFMSDAMRATVRKLARYFPTAIVSGRCRDKVYNFVRLAELYYAGSHGMDIKGPSKGSKYKK
GAEAVLCQPASEFLPMIDEVYKALIDATKSTEGVIVENNKFCASVHFRCVDEKKWGELAQVVRSVL
KEYPKLRLTQGRKVFEIRPTIKWDKGKALEFLLESLGYANCTDVFPVYIGDDRTDEDAFKVLRERG
QGFGILVSKIPKDTHASYSLQEPSEVMVFLRRLVEWKKLSLRRQFRIRRQIEEIKASLRN

**SEQ ID NO: 79, DNA - Nicotiana tabacum**
CTGGCTGCAGTGATAAGGTTACTTCGAAGAGGTGGTTTTTCATTGACAAAAGGGTTGGTTAGAAGT
GTGTGAGACATTCTAATTAGTAAACCGTCGTCAACTGTTTGCATAGTTGATAAACTTGTATTCTGC
CATAAAAAGATATTGGGCACAATGGACCTGAAATCAAATACTTCCCCAGTTGTTACTGATCCTGCC
CCAATGACTCAGTCCAGATTGGGCACCCACTCTGCTTTGATGCCATACTCACCGACTGGGGCAACT
TTCTCTCCCACACTATTCCTTACTATCCCAAGGAAGAAGCCAGGAATCCTAGATGATGTTAGATCA
AACACTTGGTTGGACGCCATGAAATCGTCATCTCCTACACATAGCAAGAAAAATAAGGACTCCAAT
GCTGAACTAACAGCAAATGAAAGTGATCTTGCCTACCGCATTTGGATGCTCAAGTACCCCTCAGCA
CTTTCATCATTTGAGCAAATCACCAATTATGCAAAAGGCAAAAGGATAGCACTCTTTTTGGACTAT
GATGGGACTTTATCACCAATTGTAGATGATCCAGATCGAGCCTTCATGTCTGGTGCTATGCGCGCT
ACTGTGAGGAATGTGGCTAAATATTTTCCCACAGCAATTATTAGTGGGAGAAGCCGAGATAAGGTA
TATGACTTTGTCGGACTAGCAGAACTTTACTATGCTGGTAGTCATGGGATGGATATAATGGGTCCT
GTTCGATCCGTTTCTGATGACTATAGTTGTATTAGATCTACTAACAAGCAGGGCAAGGAAGTTAAT
CTTTTCCAACCTGCTGGTGAGTTCTTACCAATGATTGATGAGGTTTTTAGATCTCTTATTGAGCTC
ACAAAAGACATCACGGGAGCAAAGGTTGAGAACAACAAATTCTGTGTTTCTGTACACTATCGTAAC
GTAGATGAGAAGAGTTGGTCAGCTATTGGAGAATCTGTTGATGAACTGTTAAAACACTACCCACGT
CTGCGATTGACACATGGCCGGAAGGTTTTAGAAGTCAGGCCTGTGCTTAACTGGGACAAGGGGAAA
GCTGTTGAGTTCTTACTTGAATCTTTGGGGTTGAAAAATTGTGATGATGTTCTTCCCATATACGTT
GGAGATGATCGTACAGATGAAGATGCATTCAAGGTCTTGAGAGAGGGAAATAAAGGCTACGGAATC
TTAGTATCTTCTGCACCAAAAGAAAGCAGTGCGTTTTACTCTCTGAGGGATCCATCTGAGGTGATG
GAATTCCTCAAGTGCTTGGTATCATGGAAGAAGTCAAGTGGTTTTAGCTATTAACAAGGAGTACCA
TTCAACAACACATATACCCCATTTTGTTGAATTCTTTTCATATGAAGATCTAATGACTTGCGGATG
GTTTAGGGAGCAGAGGTGCTTAGTGAAGCTTCTAGAGACAGCAACAGGGATTCAAGTTTACTGTTT
CTGCTCAGAGTTTTTCTTTGTAAATTCCTTTTCGTAATTGCCGCATCAAGGATGCCCTTAAGTCAA
TGTATAGTCATCTTCGTATTTTATTTTTCAAGTTGTATGTTGTTTTAACTTTGGCGTCAGTTCTCC
CCAAGGGAGAGTGTACCAACAACTGAAATGCTTAATACATCAAGTACGAACTTAATTTAGTAAAAA
AAAAAAAAAAAAAAAAAAAAAAAAA

**SEQ ID NO: 80, protein - Nicotiana tabacum**
MDLKSNTSPVVTDPAPMTQSRLGTHSALMPYSPTGATFSPTLFLTIPRKKPGILDDVRSNTWLDAM
KSSSPTHSKKNKDSNAELTANESDLAYRIWMLKYPSALSSFEQITNYAKGKRIALFLDYDGTLSPI
VDDPDRAFMSGAMRATVRNVAKYFPTAIISGRSRDKVYDFVGLAELYYAGSHGMDIMGPVRSVSDD
YSCIRSTNKQGKEVNLFQPAGEFLPMIDEVFRSLIELTKDITGAKVENNKFCVSVHYRNVDEKSWS
AIGESVDELLKHYPRLRLTHGRKVLEVRPVLNWDKGKAVEFLLESLGLKNCDDVLPIYVGDDRTDE
DAFKVLREGNKGYGILVSSAPKESSAFYSLRDPSEVMEFLKCLVSWKKSSGFSY

**FIGURE 8 (continued)**

**SEQ ID NO: 81, DNA - Nicotiana tabacum**

CTTTGTTCCTTACTTCTCTATTTCTTTCACTTAAACACACAGAAGCAGAGAAGTTTAATTATTCA
TTTCTCTGACTCTGTGTTTCTCTAATTTCTTCTCTTACGGATTCCAATTCCTCTGTTTTTTAATC
TTTTCAAGGGTAATTTTGTGTTGCCAAATGACGAACCAGAATGTGATTGTTTCTGACCCAAGATCA
GGGTTGGAGTCTTCTTTCTTATCGTTCTCACCGGCGGTACCCGGTCCACTACCGCCGCCGGGGAGA
TTCATCGCCGTTCCGGCAAAGAAATCATTTAAGAACATTGAGTCTGCTGCCGGAGATCATGGAGCT
AATAGGATTACTGCTTTACTTGATTCCATGAGAGCTTCTTCCCCACCTCGTAGATCCTCTGAAACT
GAGAATCTCAAGTCTTGGATTGTTCATCATCCCTCAGCTTTGAACATGTTCGAGGAAATTATAAAT
GCTTCAAAAGGGAAACAAATAATAATGTTTTTGGACTATGATGGTACATTGTCTCCTATTGTTGAT
GATCCTGACAAAGCCTTTATGACTGCTGAGATGAGGGAAGCAGTGAGAGACACATCCAAGTATTTT
CCTACAGCAATAGTGAGTGGAAGATGCAGAGCAAAAGTCTTTAATTTCGTAAAGTTATCAGAACTG
TATTATGCTGGAAGTCATGGAATGGACATTAAGGCGCCTGCTAAAGGACGCAAATATAGAAATGGA
AATAATCGAACTGTTCTCTGCCAACCTGCCAGAGAATTTTTACCCATGATTGATGAGGTATATAAA
TCTTTAGTGGAGAAAACAAAATCTATAGCAGGAGCTAAAGTGGAAAACAACAAATTCTGCTTATCC
GTACATTTCCGTCGTGTTGAAGAGAAGGTGTGGACTGAATTAGCTGAGCAAGTGAAGTCAGTGACT
AAGGAATACCCAAAACTTCGATTAACTCAAGGAAGAAAGGTTTTGGAGATTCGTCCCAGCATTAAA
TGGGACAAGGGAAAGGCACTTGAATTTTTGTTGGAATCATTAGGGTATGCTAATTCAAATGATGTT
CTGCCTATATACATTGGCGATGATCGAACAGATGAAGATGCTTTCAAGGTTTTGCGTGACAGAGGA
CAAGGTTTTGGAATATTAGTGTCCAAAGCACCTAAAGAAACGAATGCTTCCTATTCTTTGCAAGAG
CCATTAGAGGTTATGTACTTTTTAAATCGTTTTGTGGAGTGGAAAAGATCGTCCTCGCAAAGATAT
CAGAGGAAATAAAATCCAGAAGAGTAGCTCACACCAAGAGTTGACCCTTTAACCCTGGAAGTTTTG
TTGGGGATGGGTTTAGAAATTAAAGAGCTTTAATTTGTAGCACCTTTTCTTTTTGGAAGACTCAAC
TGTAATTGAAAAAATGGAGAAATTTTTGTAATTTTCCGAGTATAAATTCAATAAGAAAGTATTGTG
CACTTCTCACTTATATCCT

**SEQ ID NO: 82, protein - Nicotiana tabacum**

MTNQNVIVSDPRSGLESSFLSFSPAVPGPLPPPGRFIAVPAKKSFKNIESAAGDHGANRITALLDS
MRASSPPRRSSETENLKSWIVHHPSALNMFEEIINASKGKQIIMFLDYDGTLSPIVDDPDKAFMTA
EMREAVRDTSKYFPTAIVSGRCRAKVFNFVKLSELYYAGSHGMDIKAPAKGRKYRNGNNRTVLCQP
AREFLPMIDEVYKSLVEKTKSIAGAKVENNKFCLSVHFRRVEEKVWTELAEQVKSVTKEYPKLRLT
QGRKVLEIRPSIKWDKGKALEFLLESLGYANSNDVLPIYIGDDRTDEDAFKVLRDRGQGFGILVSK
APKETNASYSLQEPLEVMYFLNRFVEWKRSSSQRYQRK

**SEQ ID NO: 83, DNA - Sorghum bicolor**

GCACGAGGACCAATCATTTCTTTCCAACAAATCTCCAGCCTCTGCTGCTGCTAATAAACTGATCGA
TCGCGTTGTTCTTGATAATCATCAGTCGACCGATCGCGATCGGGATGGCGAAGCCTAGCGTGGCGG
CGGTGCCGGAGGTTGTTGGCGTGCCAGCAGCAGCGCAGGCGACGACGCTGTTCCCGTACCCGCCAC
CGCGTGGCGCCGGGATCACAGCCGCCGTCGTGCGCCGCAAGTGCCTGCAGGTGGAGCTCGGCGCGG
GGGCGGCCGGGCCGCTGCTGGGCGGCGCGTGCTGGGGCGTGGAGTCGATGCGCGCGTCGTCCCCCA
CGCACGCCAAGGCCGCCGCCGCGCTCGCCGCCGGCGTCGACGAGGAGCGCCGCGCCGCCTGGACGG
TGCGGCACCCGTCGGCGCTGGGCAAGTTCGAGCAGATCGTGGCGGCGTCCGAGGGCAAGCGGATCG
TCATGTTCCTCGACTACGACGGCACGCTGTCGCCCATCGTGGACGACCCCGACGCCGCCTTCATGA
GCGAGACGATGCGGATGGCCGTGCGTAGCGTCGCCAAGCATTTCCCGACGGCGATCGTGAGCGGGC
GGTGCCGGGACAAGGTGTTCGAGTTCGTGAAGCTGGCGGAGCTGTACTACGCCGGCAGCCACGGCA
TGGACATCAGAGGCCCAGCCAAGGCCTCTTCCCGGCACGCAAAGGCCAAGGCAAAAGGCGTTCTGT
TCCAGCCGGCGAGCGAGTTCCTGCCCATGATCGAGGAGGTGCACGACCGCCTTGTCGAGACGACGC
GCTGCATCCCGGGGGCCAAGGTGGAGAACAACAAGTTCTGCGTCTCCGTCCACTTCAGATGCGTCG
ACGAAAAGATGTGGGGCGAGCTGTCAGAGTCGGTGAAGGGCGTGCTGCGGGAGTACCCGAAGCTGC

## FIGURE 8 (continued)

GGCTGACACAGGGGCGGATGGTGTTCGAGGTGCGTCCCACCATCAAATGGGACAAGGGCAAGGCCC
TCGAGTTCCTGCTCGAGTCGCTCGGCTTCGCGGACTGCAGCAACGTCCTGCCCGTGTACATCGGCG
ACGACCGCACCGACGAGGACGCCTTCAAGGTGCTGCGGCGCCGGGGCCAGGGCCACGGCGTCGGGA
TCCTCGTGTCCAAGCACCCCAAGGAGACGTCCGCCTCCTACTCCCTCCAAGAGCCCGCCGAGGTGA
TGGAGTTCTTGCTGCGGCTCGTCGAGTGGAAGCAACTCTCCAGGGCAAGGCTCATCAGGCTGCAAT
GAGATTGAGATGGACGACGCAACGGATCAAGCTAACTGTCAGGTGCTAATTAAGAGATCCTGCTAC
GTGAAATCGTGAATGGCAGAGAATCTGGCACTGTATTGTGTCTTCTCTACTGTCCCGTAGTCATCT
GTTTTCCTCTCCCTTCTCTGTACGTGTTCCCTGGGAACCTCTCTGGCCTCTGCTAGGCGTGTGTGT
AAGTGTTTAACAAGCATGCATGTACATTAGTATTACCTAATAGTAGTTAACTTATATGCTACATAT
ATATATATATATATGTAGTACAGTATATACAAGAGGGAAGAATATATGAAGCAACGAAGACCGNAA
AAAATTATGTCTCGTTCAAGGTTTT

**SEQ ID NO: 84, protein - Sorghum bicolor**
MAKPSVAAVPEVVGVPAAAQATTLFPYPPPRGAGITAAVVRRKCLQVELGAGAAGPLLGGACWGVE
SMRASSPTHAKAAAALAAGVDEERRAAWTVRHPSALGKFEQIVAASEGKRIVMFLDYDGTLSPIVD
DPDAAFMSETMRMAVRSVAKHFPTAIVSGRCRDKVFEFVKLAELYYAGSHGMDIRGPAKASSRHAK
AKAKGVLFQPASEFLPMIEEVHDRLVETTRCIPGAKVENNKFCVSVHFRCVDEKMWGELSESVKGV
LREYPKLRLTQGRMVFEVRPTIKWDKGKALEFLLESLGFADCSNVLPVYIGDDRTDEDAFKVLRRR
GQGHGVGILVSKHPKETSASYSLQEPAEVMEFLLRLVEWKQLSRARLIRLQ

**SEQ ID NO: 85, DNA - Solanum tuberosum**
GCCCAAATCCGGTATTAATCTGACAATACCGGTATCGAACTCGTCGGCGTTATTTACGACGGCAGC
TCAGAAGCCACCGCCAGGGCCGGGGAGTTGTATAACTATTTCAAGAAAGACACTTGTTGAAATTAA
TGGGAATAATAGTGCTAGAATCAATTCTTGGGTTGAATCAATGAGAGCTTCCTCTCCTACTCATCA
TAAGTCCAGTCCTGCTCTTTCAGATGACTTGAATTCTTGGATGGTGCAACATCCATCAGCACTGGA
TATGTTTGAGCAGATAATCAGTGCTTCAAAGGGAAAACAAATTGTGATGTTTTTAGACTATGATGG
CACTCTTTCCCCCATTGTTGAAGATCCTGATCAAGCTTTCATGTCTGATGCTATGCGAGCAACAGT
GAGAAAACTTGCTAGATATTTCCCTACTGCAATAGTGAGTGGAAGGTGCAGAGACAAGGTATACAG
CTTTGTACGATTGGCAGAGTTGTACTATGCTGGTAGCCATGGAATGGATATAAAAGGGCCATCAAA
AGGTTCCAAATACAAGAAAGGAGCGCAAGCTGTTCTTTTCCAACCAGCAAGTGAATTTCTCCCTAT
GATTGATGAGGTTTACAAAAAACTTGTAGATATAACAAAATCTACAGAAGGAGTTAGAGTTGAAAA
TAACAAGTTTTGTGCCTCTGTACATTTCCGCTGTGTTGATGAAAAGAAATGGGGTGAACTAGCACA
AGTAGTAAGATCAGTGCTTAAAGAATACCCAAAGCTTCGATTGACACAAGGAAGAAAAGTATTAGA
GATTCGTCCAACTATTAAATGGGACAAGGGCAAAGCTCTCGAATTCTTGCTCGAATCACTTGGATA
TGCTAACTGTACTGATGTCTTTCCTGTATATATTGGTGATGATCGAACCGATGAAGATGCTTTCAA
GGTCCTAAGAGAAAGAGATCAAGGTTTTGGCATTCTTGTCTCCAAAACTCCAAAAGACACACACGC
ATCTTATTCTTTGCAAGAACCATCTGAGGTTATGGTGTTTCTACGACGTTTGGTAGAGTGGAAAAA
GTTGTCATTAAGAAGACAATTTCGAATTCGAAGACAAATTGAGGAGATGAAAGCATCTCTAAGGAA
CTAAGAATTTGTCCAAAGTGAATATGGACTTAATGATGCATATAATGTATTTTCCTTTTATCAAAT
ATAACAAAGAAGTATTAAGAAAGAAAAAGGTAAAAAAGAAATTAAAAGCTTTTTTTTTTATCCTTT
TCTCTTTGGCTTTAAGGCCTTGTACATCTTTGTAATTTTCCCAGTCTTGCCTAAATGCATATAATG
GAAGTTCTCTCTC

**SEQ ID NO: 86, protein - Solanum tuberosum**
MRASSPTHHKSSPALSDDLNSWMVQHPSALDMFEQIISASKGKQIVMFLDYDGTLSPIVEDPDQAF
MSDAMRATVRKLARYFPTAIVSGRCRDKVYSFVRLAELYYAGSHGMDIKGPSKGSKYKKGAQAVLF
QPASEFLPMIDEVYKKLVDITKSTEGVRVENNKFCASVHFRCVDEKKWGELAQVVRSVLKEYPKLR
LTQGRKVLEIRPTIKWDKGKALEFLLESLGYANCTDVFPVYIGDDRTDEDAFKVLRERDQGFGILV
SKTPKDTHASYSLQEPSEVMVFLRRLVEWKKLSLRRQFRIRRQIEEMKASLRN

**FIGURE 8 (continued)**

**SEQ ID NO: 87, DNA - Triticum aestivum**
CCACGCGTCCGTTTCCTCTTCACTGTCTCTTCTCCTTTTCTGCCTTCCAACCAAAGCCCCATCCCT
TGATTCGATCGATCGGGATCCTCCTCTCGCGCGCTTCTAGGACCTGCCCCTCGAAGCTTCTCCTTC
TCCTTGGACCCGTGCTTGGTATGGCGCAGACGACTGTGGTGGTGCCGGAGGTGGGCATGACGGCTG
CCACGCCCACTGCGTGCCCCTGCCCTGGGTCGCTGTTCCCGTACCCGCCGCCGCGTGCCGGGATGG
CCGTGAGCCGCAAGTGCCTGCGGGCGGCGCAGGCGGAGCTTGGCGCGGGGATGCTCAGTGGCCTGG
TCGAGTCCATGCGGGCGTCCTCCCCCACGCACGCCAGGGCCGCCGCCGCCCTCGCCGCCGGCGTCG
ACGACGAGCACGCCGCCTGGATGGCAAGGCACCCGTCCGCCCTGGGAAAGTTCGAGGAGATCGTGG
CCGCGTCCAAGGGGAAGCAGATCGTCATGTTCCTGGACTACGACGGCACGCTGTCCCCCATCGTCG
ATGACCCCGACGCCGCCTTCATGAGCGAGACGATGCGGATGGCAGTGCGCAGCGTGGCCAAGCACT
TCCCGACGGCGATCGTGAGCGGTCGGTGCCGCGACAAGGTGTTTGAGTTCGTGAAGCTGGCGGAGC
TCTACTACGCCGGCAGCCACGGCATGGACATCAAGGGCCCGGCCAAATCCTCAAAGTCCAAGGCCA
AAGGAGTTCTCTTCCAACCAGCAAGCGAGTTCCTGCCCATGATAGAAGAGGTGCATCAGCGGCTGA
TAGAGGAGACGAAGCACGTAGCCGGGGCCAAGGTGGAGAACAACAAGTTCTGCGTGTCCGTCCACT
TCAGATGCGTCGACGAAAAGAGCTGGGGCGCGCTGGCGGAGACGGTGAAGGGCGTGATGCGGGAGT
ACCCGAAGCTGCGCATGTCGCAGGGGCGGATGGTGTTCGAGGTGCGGCCCACCATCAAGTGGGACA
AGGGCAAGGCCCTCGAGTTCCTGCTCGAGTCGCTGGGCTTCGCCGACTGCCCCAACGTGCTCCCCG
TCTACATCGGCGACGACCGCACCGACGAGGACGCCTTCAAGGTGCTGCGCCGGCGGGGCCAGGGCG
TCGGGATCCTCGTCTCCAAGCACCCCAAGGACACCAGCGCCTCCTTCTCGCTGCAGGAGCCGGCCG
AGGTCATGGAGTTCCTGCTCCGCCTCGTCGAGTGGAAGCAGCTCTCCAAGGCGCGCCTCAGGCTGC
GGCGGCAGGCCGACGCCTGATCGGACGACGAGGATCGGCCGGCCGGCGGGAATGGCCGAACTAATT
ATTTCACTGCTACGTGTAGCTTAGCTAGTTAATGAAAATCCCCTGCTTGCTCATCCGAACAAAGCC
AAAGGCTACGTACGTGGATGGGAAGAAGGAACGTACTACGGCGAACCTGCGGAGCATTGTTGCGCC
ACATGTTGTGTTCTAATTAACCGTCCTTCTTTTTCTGGGGATGTATACCGTACCTACCGATTATGC
ATGCATGTAAATTACCTTTGTCCACGTACAAGGGGAGGAACCATGGAATAAACAAAGAGAAACATG
AGTAAATAGC

**SEQ ID NO: 88, protein - Triticum aestivum**
MAQTTVVVPEVGMTAATPTACPCPGSLFPYPPPRAGMAVSRKCLRAAQAELGAGMLSGLVESMRAS
SPTHARAAAALAAGVDDEHAAWMARHPSALGKFEEIVAASKGKQIVMFLDYDGTLSPIVDDPDAAF
MSETMRMAVRSVAKHFPTAIVSGRCRDKVFEFVKLAELYYAGSHGMDIKGPAKSSKSKAKGVLFQP
ASEFLPMIEEVHQRLIEETKHVAGAKVENNKFCVSVHFRCVDEKSWGALAETVKGVMREYPKLRMS
QGRMVFEVRPTIKWDKGKALEFLLESLGFADCPNVLPVYIGDDRTDEDAFKVLRRRGQGVGILVSK
HPKDTSASFSLQEPAEVMEFLLRLVEWKQLSKARLRLRRQADA

**SEQ ID NO: 89, DNA - Zea mays**
ATGACGAAGCACGCCGCCTACTCCAGCGAGGACGTGGTCGCGGCCGTGGCGGCGCCGGCGCCGGCC
GGCCGGCATTTCACGTCGTTCCAGGCGCTGAAGGGCGCGCCCCTCGACTGCAAGAAGCACGCCGCC
GTGGACCTGTCCGCGTCCGGGGCGGCCGTCGTGGGCGGCGGCCCCTGGTTTGAGTCCATGAAGGCT
TCGTCGCCGCGGCGCGCCGCCGACGCCGAGCACGGCGACTGGATGGAGAAGCACCCGTCCGCATTG
GCCCAGTTCGAGCCGCTGCTTGCCGCCGCCAAGGGGAAGCAGATCGTGATGTTCCTGGACTACGAC
GGCACCCTGTCACCGATCGTCGAGGACCCCGACCGCGCCGTCATGTCGGAGGAGATGAGAGAAGCC
GTGCGGCGCGTCGCCGAGCACTTCCCCACCGCGATTGTGAGCGGAAGATGCAGGGACAAGGTGCTC
AACTTCGTGAAGCTGACGGAGCTGTACTACGCCGGGAGCCATGGCATGGACATCCAGGGCCCCGCC
GCCTGCAGGCAGCCCAACCACGTCCAGCAGGCTGAAGCCGCAGCTGTCCATTACCAAGCTGCGAGT
GAGTTCCTGCCGGTCATCGAAGAGGTGTTCCGCACGCTGACGGCCAAGATGGAGTCCATCGCCGGC
GCCAGGGTGGAGCACAACAAGTACTGCCTGTCCGTCCACTTCCGCTGCGTCCGGGAGGAGGAATGG
AATGCCGTGAACGAGGAGGTCAGGTCGGTGCTCAGGGAGTACCCGAACCTCAAGCTCACTCACGGC

## FIGURE 8 (continued)

AGAAAGGTGCTGGAGATTCGTCCGTCCATCAAGTGGGACAAGGGCAAGGCCCTCGAGTTCTTGCTC
AAGTCTCTTGGCTATGCTGGGCGCAACGACGTCTTCCCGATTTACATCGGAGATGATCGCACTGAC
GAGGACGCTTTCAAGGTGCTCCGCAACATGGGGCAGGGCATCGGAATCCTGGTGTCCAAGCTTCCT
AAGGAGACGGCGGCATCCTACTCGCTGAGTGACCCTGCCGAGGTCAAGGAGTTCCTCCGCAAGCTG
GCCAATAAGAAGGGGGCGCGCCAACCATGA

**SEQ ID NO: 90, protein - Zea mays**
MTKHAAYSSEDVVAAVAAPAPAGRHFTSFQALKGAPLDCKKHAAVDLSASGAAVVGGGPWFESMKA
SSPRRAADAEHGDWMEKHPSALAQFEPLLAAAKGKQIVMFLDYDGTLSPIVEDPDRAVMSEEMREA
VRRVAEHFPTAIVSGRCRDKVLNFVKLTELYYAGSHGMDIQGPAACRQPNHVQQAEAAAVHYQAAS
EFLPVIEEVFRTLTAKMESIAGARVEHNKYCLSVHFRCVREEEWNAVNEEVRSVLREYPNLKLTHG
RKVLEIRPSIKWDKGKALEFLLKSLGYAGRNDVFPIYIGDDRTDEDAFKVLRNMGQGIGILVSKLP
KETAASYSLSDPAEVKEFLRKLANKKGARQP

**SEQ ID NO: 91, DNA - Zea mays**
ATGACGAAGCGCACCGCCTTCGCCGCGGACGACGCGATCATCGCCGCCGCCGCCGCCGTCACGTCG
CAGCCCGGCCGGCGGTTCACGTCGTACCCGCCGGCGAGGGCGCGCGGCGGATGCAGGCTGGCCCCG
GCGGCGGCGGCGCGCCAGGCCACGGACGACCCCGGCGCCGCTGGGTCCTGGCCAGAACTAGTCGTG
CCGCGGCACGCCGACTTCGACGACTGGATGGAGAAGCACCCGTCGGCATTGGCCGCGTTCGAGTCG
GTGCTGGCCGCCGCCAAAGGCAAGAAGATCGTCATGTTCCTCGACTACGACGGCACCCTGTCGCCG
ATCGTCAGGGACCCCGACAGCGCCGTCATGTCCGAGGAGATGCGGGACGCGGTGAGAGGCGTGGCC
GAGCACTTCCCGACGGCGATCGTGAGCGGGAGGTGTAGAGACAAGGTGTTCAACTTCGTGAAGCTG
GCGGAGCTGTACTACGCCGGGAGCCACGGCATGGACATCAAGGGCCCCACAGCACAGTCCAAGCAC
ACCAAGGCAAAGGCCGGAGCCGTTCTATGCCAACCTGCGAGGGCGTTCCTGCCGGTCATTGAGGAG
GTGTACCGCGCGCTGACGGCGAGCACGGCGCCGATCCCCGGCGCGACGGTGGAGAACAACAAGTTC
TGCCTCTCCGTCCACTTCCGCTGCGTCCAGGAGGAGAAATGGCGCGCTCTGGAGGAGCAGGTCCGG
TCGGTGCTCAAGGAGTACCCGGACCTCCGCCTCACCAAGGGCAGGAAGGTCCTCGAGATCCGGCCG
TCCATCAAGTGGGACAAGGGCAACGCCCTGCAGTTCTTGCTCGAGTCTCTCGGTTTTGCTGGCAGT
AACAGTGTCTTCCCGATATATATCGGAGACGATAGCACCGACGAGGACGCGTTCAAGGTCCTGCGC
AACTTGGGGCAAGGGATCGGGATCCTGGTGAGCAAGATTCCGAAGGAGACCCGCGCATCCTACTCC
CTGCGTGAACCTTCTGAGGTGGAGGAGTTCCTGCGCAAGTTGGTCAGCTGGTCCAAGGAGAGCAGG
CAACGGGACT

**SEQ ID NO: 92, protein - Zea mays**
MTKRTAFAADDAIIAAAAAVTSQPGRRFTSYPPARARGGCRLAPAAAARQATDDPGAAGSWPELVV
PRHADFDDWMEKHPSALAAFESVLAAAKGKKIVMFLDYDGTLSPIVRDPDSAVMSEEMRDAVRGVA
EHFPTAIVSGRCRDKVFNFVKLAELYYAGSHGMDIKGPTAQSKHTKAKAGAVLCQPARAFLPVIEE
VYRALTASTAPIPGATVENNKFCLSVHFRCVQEEKWRALEEQVRSVLKEYPDLRLTKGRKVLEIRP
SIKWDKGNALQFLLESLGFAGSNSVFPIYIGDDSTDEDAFKVLRNLGQGIGILVSKIPKETRASYS
LREPSEVEEFLRKLVSWSKESRQRD

**SEQ ID NO: 93, Consensus Trehalose Ppase domain**
XLDYDGTLSXIXXXPXXAXXSXXXLXXXLXXLASXXXXXVXIVSGRXXXXLXXXXXXXXXXXXXX
VXXLXLAAEHGXXIRXXXXXXXXXXXXXXXXXXXXXXXXXXXXXXXXLXWXXXVXXILXXXXEXT
PGSXIEXKXXALAWHYRXADXXXXXXXAXXVXXXLXXXXXXXXXLXVXXGKXVVEVRPXXXVXKGX
ALXXILXXLXXXXXXXXXXXXXXXXXXXXXXXVLXIGDDXXTDEDMFXXIXXXXXXXXXXXXXILV
XXXXXXTXAXXXL

## FIGURE 8 (continued)

**SEQ ID NO: 94, Trehalose Ppase domain in SEQ ID NO: 2**
GKQIVMFLDYDGTLSPIVEDPDKAFITHEMREVVKDVASNFPTAIVTGRSIEKVRSFVQVNEIYYA
GSHGMDIEGPTNENSNGQSNERVLFQPAREFLPMIEKVVNILEEKTKWIPGAMVENNKFCLSVHFR
RVDEKRWPALAEVVKSVLIDYPKLKLTQGRKVLEIRPTIKWDKGQALNFLLKSLGYENSDDVVPVY
IGDDRTDEDAFKVLRERGQGFGILVSKVPKDTNASYSLQDPSQVNKFLERLVE

**SEQ ID NO: 95, Serine-rich domain**
RXXSWVDSMRASSPTXXKS

**SEQ ID NO: 96, B-Phosphatase box**
GDDRTDQDAF

**SEQ ID NO: 97, A-Phosphatase box**
LDYDGTLSPIVD

**SEQ ID NO: 98, DNA - Oryza sativa - GOS2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTCCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTTCGATCCATATCTTCCGGTCGAGTTCTTGG
TCGATCTCTTCCCTCCTCCACCTCCTCCTCACAGGGTATGTGCCCTTCGGTTGTTCTTGGATTTAT
TGTTCTAGGTTGTGTAGTACGGGCGTTGATGTTAGGAAAGGGGATCTGTATCTGTGATGATTCCTG
TTCTTGGATTTGGGATAGAGGGGTTCTTGATGTTGCATGTTATCGGTTCGGTTTGATTAGTAGTAT
GGTTTTCAATCGTCTGGAGAGCTCTATGGAAATGAAATGGTTTAGGGTACGGAATCTTGCGATTTT
GTGAGTACCTTTTGTTTGAGGTAAAATCAGAGCACCGGTGATTTTGCTTGGTGTAATAAAAGTACG
GTTGTTTGGTCCTCGATTCTGGTAGTGATGCTTCTCGATTTGACGAAGCTATCCTTTGTTTATTCC
CTATTGAACAAAAATAATCCAACTTTGAAGACGGTCCCGTTGATGAGATTGAATGATTGATTCTTA
AGCCTGTCCAAAATTTCGCAGCTGGCTTGTTTAGATACAGTAGTCCCCATCACGAAATTCATGGAA
ACAGTTATAATCCTCAGGAACAGGGGATTCCCTGTTCTTCCGATTTGCTTTAGTCCCAGAATTTTT
TTTCCCAAATATCTTAAAAAGTCACTTTCTGGTTCAGTTCAATGAATTGATTGCTACAAATAATGC
TTTTATAGCGTTATCCTAGCTGTAGTTCAGTTAATAGGTAATACCCCTATAGTTTAGTCAGGAGAA
GAACTTATCCGATTTCTGATCTCCATTTTTAATTATATGAAATGAACTGTAGCATAAGCAGTATTC
ATTTGGATTATTTTTTTTATTAGCTCTCACCCCTTCATTATTCTGAGCTGAAAGTCTGGCATGAAC
TGTCCTCAATTTTGTTTTCAAATTCACATCGATTATCTATGCATTATCCTCTTGTATCTACCTGTA
GAAGTTTCTTTTTGGTTATTCCTTGACTGCTTGATTACAGAAAGAAATTTATGAAGCTGTAATCGG
GATAGTTATACTGCTTGTTCTTATGATTCATTTCCTTTGTGCAGTTCTTGGTGTAGCTTGCCACTT
TCACCAGCAAAGTTC

**FIGURE 8 (continued)**

**SEQ ID NO: 99, primer prm05451**
GGGGACAAGTTTGTACAAAAAAGCAGGCTTAAACAATGACTAACCAGAATGTCATC

**SEQ ID NO: 100, primer prm05452**
GGGGACCACTTTGTACAAGAAAGCTGGGTTGTAATTATGTTGCATGTCTT

**SEQ ID NO: 101, primer prmAtTPPA-5**
GGAAGATCTATGGACATGAAATCTGGTCACTC

**SEQ ID NO: 102, primer prmAtTPPA-3**
AAGGCCTACCCATTGATCTCTTCCATGTCA

**SEQ ID NO: 103, primer prmAtTPPB-5**
GGAATTCATGACTAACCAGAATGTCATCGTT

**SEQ ID NO: 104, primer prmAtTPPB-3**
AAGGCCTCTCTTCTCCCACTGTCTTCCTC

**SEQ ID NO: 105, primer prmAtTPPC-5**
CGGGATCCATGAAGATTACGGATATTTCCGG

**SEQ ID NO: 106, primer prmAtTPPC-3**
AAGGCCTTTCTCCAAGTGTTTGTTTCTTCC

**SEQ ID NO: 107, primer prmAtTPPD-5**
CGGGATCCATGACAAACCATAATGCCTTAATC

**SEQ ID NO: 108, primer prmAtTPPD-3**
AAGGCCTTCTTCCTCTTAGTGACATTTGTTTC

**SEQ ID NO: 109, primer prmAtTPPE-5**
CGGGATCCATGTTCGAAGAAATACTTCATAAATC

**SEQ ID NO: 110, primer prmAtTPPE-3**
AAGGCCTTGCCCCACACCTTGACTGTTTC

**SEQ ID NO: 111, primer prmAtTPPF-5**
CATGCCATGGATTTAAACTCAAACCACAAATC

**SEQ ID NO: 112, primer prmAtTPPF-3**
TCCCCCGGGAAAACCAGTAGAATTCTTCTCCAAC

**SEQ ID NO: 113, primer prmAtTPPG-5**
CATGCCATGGATTTGAATATAAACAAGACGAC

**SEQ ID NO: 114, primer prmAtTPPG-3**
AAGGCCTAAAACTTGTTTTTGAACTTTCCATCTTC

**FIGURE 8 (continued)**

**SEQ ID NO: 115, primer prmAtTPPH-5**
CGGGATCCATGGTTAGATTCATAGAAGAAAACAC

**SEQ ID NO: 116, primer prmAtTPPH-3**
AAGGCCTTGCTCCAGATCTCAATTGTTTCC

**SEQ ID NO: 117, primer prmAtTPPI-5**
CGGGATCCATGTCAGCTAGTCAAAACATTGTC

**SEQ ID NO: 118, primer prmAtTPPI-3**
AAGGCCTCATTCTTGGCTGCATTTGTTTCC

**SEQ ID NO: 119, primer prmAtTPPJ-5**
CGGGATCCATGGTGAGCCAAAACGTCGTCG

**SEQ ID NO: 120, primer prmAtTPPJ-3**
AAGGCCTTTGCTGCATCTGTTTCCACTCC

**SEQ ID NO: 121, DNA - Glycine max**
CTCTTCTCTTCTCTTCACTTTGATTTCATTGCAATTACCTCTTTTTGTTGGGATCTTCTTCCCTTC
TCTGTTCTAAAAAAGAACACGGCTGATCTGGGCTCGACTAAAACAAATCACTAAGTAAAAATGACG
CAGAATGCGATAGTGTCCAAGACAAAATCGGGGATCAACCGGGACATAACCGTGCCACAAAAGCCA
CTGGCGGCGGCGGCGGCGGCGGGGGGGTACATTCCCATTCCGAGGAGGAGGGTTTTGAAGAACCTG
GAAATCAATGCATGGGTTGATTCCATGAGATCCTCTTCTCCAACCAATTCCAAATCCACCTCTTCT
CTCGCAGAAGAACACAGCACTTGGATTCTTCGCCACCCCTCAGCATTAGACATGTTCGAGCAAATT
ATGGATGCGTCCCGGGGAAAGCAAATTGTCATGTTTCTCGACTATGATGGTACTTTGTCGCCTATT
GTTGATGACCCAGACCGTGCTTTCATGTCGGATTCGATGAGGAGAACGGTGAGGAAACTTGCAAGG
TGTTTTCCTACTGCTATAGTTACCGGCAGATGCAAAGACAAGGTTTACAATTTTGTTCGCTTGGCT
GAGCTATATTATGCTGGAAGCCATGGCATGGATATTCAAGGGCCAACAAGAGACTGCAAATACAGC
AAAGACAAAGGAGAGCCAGTTCTTTTTCAACCTGCCAGTGAATTTCTTCCCATGATTGATGAGGTG
TACCATCAATTAGTTGAGAAAATGAAATCAATTCCTGGAGCCATGGTGGAGAACAACAAGTTCTGC
TGTTCTGTTCATTTTCGCTGCGTTGATGAAAAGAAATGGAGTGAACTGGCACAGGAAGTGAGATCA
GTATTAAAAGAGTACCCGAAGCTTCGTCTTAACCAAGGAAGGAAGGTATTAGAGATTCGTCCATCT
ATTAAATGGGACAAAGGGAAGGCGCTGGAATTTTTGTTAGAGTCACTTGGATTTGCCAACTGTAAC
GATGTCTTTCCTGTTTACATTGGAGATGATAAAACCGATGAAGATGCATTCAAGAAATTAAGAGAC
AGAGGACAAGGTTTTGGGATTCTTGTCTCGAAATTTCCAAAGGACACTACTGCATCATACTCTTTA
CAAGAACCAAACGAGGTGATGGATTTCCTTCAACGTTTGGTGGAGTGGAAACAAGTATCCCTCCGA
CTCAGAACACGTTCTCGGGTGTAAATGGATAGAATGATAATGTACATCCCTACCCTGCCATAAAAA
ATGGAGTTCAGCTAGGGTAGCATATAGGACCCTTTTTTCTATGTTTTGTTCAGATTAGAACAGAGA
ACAAAAATGTATGGAAAAAGGAAAGACAAAAGTGTCTTTCTGAGTTTGAAGCACGTCTACATGGAT
GTAATTAGCCACGTAGTTTTTGTAGTACAGGGGAAACGTCTCTGTTGCATCAAATGTAATTCTGAA
TGGAAAATAACATGTTACGTATCGAAAAATATATATTATTGT

**SEQ ID NO: 122, protein - Glycine max**
MTQNAIVSKTKSGINRDITVPQKPLAAAAAAGGYIPIPRRRVLKNLEINAWVDSMRSSSPTNSKST
SSLAEEHSTWILRHPSALDMFEQIMDASRGKQIVMFLDYDGTLSPIVDDPDRAFMSDSMRRTVRKL
ARCFPTAIVTGRCKDKVYNFVRLAELYYAGSHGMDIQGPTRDCKYSKDKGEPVLFQPASEFLPMID
EVYHQLVEKMKSIPGAMVENNKFCCSVHFRCVDEKKWSELAQEVRSVLKEYPKLRLNQGRKVLEIR
PSIKWDKGKALEFLLESLGFANCNDVFPVYIGDDKTDEDAFKKLRDRGQGFGILVSKFPKDTTASY
SLQEPNEVMDFLQRLVEWKQVSLRLRTRSRV

**FIGURE 8 (continued)**

**SEQ ID NO: 123, DNA - Glycine max**
ACGGCTGATCTGGGCTCGACTAAAACAAATCACTAAGTAAAAATGACGCAGAATGCGATAGTGTCC
AAGACAAAATCGGGGATCAACCGGGACATAACCGTGCCACAAAAGCCACTGGCGGCGGCGGCGGCG
GCGGGGGGGTACATTCCCATTCCGAGGAGGAGGGTTTTGAAGAACCTGGAAATCAATGCATGGGTT
GATTCCATGAGATCCTCTTCTCCAACCAATTCCAAATCCACCTCTTCTCTCGCAGAAGAACACAGC
ACTTGGATTCTTCGCCACCCCTCAGCATTAGACATGTTCGAGCAAATTATGGATGCGTCCCGGGGA
AAGCAAATTGTCATGTTTCTCGACTATGATGGTACTTTGTCGCCTATTGTTGATGACCCAGACCGT
GCTTTCATGTCGGATTCGATGAGGAGAACGGTGAGGAAACTTGCAAGGTGTTTTCCTACTGCTATA
GTTACCGGCAGATGCAAAGACAAGGTTTACAATTTTGTTCGCTTGGCTGAGCTATATTATGCTGGA
AGCCATGGCATGGATATTCAAGGGCCAACAAGAACCTCCAAATACAGCAACAAAGATAAAGGAGAG
CCTGTTCTTTTTCAACCTGCTAGTGAATTTCTTCCCATGATTGATGAGGTGTACCATCAATTAGTT
GAGAAAATGAAATCAATTCCTGGAGCCATGGTGGAGAACAACAAGTTCTGCTGTTCTGTTCATTTT
CGCTGCGTTGATGAAAAGAAATGGAGTGAACTGGCACAGGAAGTGAGATCAGTATTAAAAGAGTAC
CCGAAGCTTCGTCTTAACCAAGGAAGGAAGGTATTAGAGATTCGTCCATCTATTAAATGGGACAAA
GGGAAGGCGCTGGAATTTTTGTTAGAGTCACTTGGATTTGCCAACTGTAACGATGTCTTTCCTGTT
TACATTGGAGATGATAAAACCGATGAAGATGCATTCAAGAAATTAAGAGACAGAGGACAAGGTTTT
GGGATTCTTGTCTCGAAATTTCCAAAGGACACTACTGCATCATACTCTTTACAAGAACCAAACGAG
GTGATGGATTTCCTTCAACGTTTGGTGGAGTGGAAACAAGTATCCCTCCGACTCAGAACACGTTCT
CGGGTGTAAATGGATAGAATGATAATGTACATCCCTACCCTGCCATAAAAAATGGAGTTCAGCTAG
GGTAGCATATAGGACCCTTTTTTCTATGTTTTGTTCAGATTAGAACAGAGAACAAAAATGTATGGA
AAAAGGAAAGACAAAAGTGTCTTTCTGAGTTTGAAGCACGTCTACATGGATGTAATTAGCCACGTA
GTTTTTGTAGTACAGGGGAAACGTCTCTGTTGCATCAAATGTAATTCTGAATGGAAAATAACATGT
TACGTATCGAAAAATATATATTATTGTATACTCTCTTAAGAAAAAAAAAAAAAAA

**SEQ ID NO: 124, protein - Glycine max**
MTQNAIVSKTKSGINRDITVPQKPLAAAAAAGGYIPIPRRRVLKNLEINAWVDSMRSSSPTNSKST
SSLAEEHSTWILRHPSALDMFEQIMDASRGKQIVMFLDYDGTLSPIVDDPDRAFMSDSMRRTVRKL
ARCFPTAIVTGRCKDKVYNFVRLAELYYAGSHGMDIQGPTRTSKYSNKDKGEPVLFQPASEFLPMI
DEVYHQLVEKMKSIPGAMVENNKFCCSVHFRCVDEKKWSELAQEVRSVLKEYPKLRLNQGRKVLEI
RPSIKWDKGKALEFLLESLGFANCNDVFPVYIGDDKTDEDAFKKLRDRGQGFGILVSKFPKDTTAS
YSLQEPNEVMDFLQRLVEWKQVSLRLRTRSRV

**SEQ ID NO: 125, DNA - Glycine max**
TTCCCGGCCTCACTCACCCCTCCCTTTTATTTCCATTATTATTCTGCCTAAGCAGTTTCTTCCAAA
CTTCCTTTTACATTTCCAATTTCTCTATTCTATCAAAAGGGTTTGAACTTTGAAGGGAAAGGAAGA
AAGATATGATGACGAACCAAAATGTGGTGACTCATGAGGTTATTAACACGTTGATTGCCGTGGCAG
CTTCCATTTCAAACTCAACCGCGTTGCCAAGTGCAACAGTGCCAGAATCCATGGCTGTGCTTGGTG
GGTTTTGGGGGCTGCCCCATAATAAAAATCTTGTGAAAAGGTTGGAAGGAGCTAAAGTTAGTGCTT
GGATTGATTCAATGAGAGCTTCTTCCCCAACTCGTGCCAAATCAGAAAGCCAAGAAAAAAGATCTT
GGATTCTTTATCACCCTTCAGCTCTGAACACGTTTGAGCAAATAGTATGTAGTGCCAAAGGAAAGC
AAGTCGTAGTTTTTCTTGACTACGATGGAACTCTCTCCCCAATTGTTGCAGATCCGGATAAAGCTT
TCATGACTAGAAAGATGAGAGCAACGCTAAAGGGCATAGCAAGGCATTTTCCCACAGCAATAGTGA
CCGGAAGGTGCAGAGACAAGGTATATAACTTTGTAAAATTGGCAGAACTTTACTATGCCGGAAGCC
ATGGCATGGACATCAAGGGTCCAACAAAAAGCCAAAGTCCAAAGCAAGGTAATAATAATAAAGCAG
TGCTGTTCCAACCCGCGAGTCAATTCCTGCCAATGATCGATGAGGTGTACAAGATCTTGTTAGAAA
AAACAAAGACTGTCCCAGGGGCTAATGTTGAGAACAATAAGTTTTGCTTGTCCGTGCACTTTCGTT
GTGTTGACGAAAAGAGTTGGGCAGCGTTGGCGGAGAAAGTTAGATTGGTGCTCAATGATTACCCAC
AACTTAGGCTAACCCAAGGGAGAAAAGTGCTAGAGATTCGTCCAACCATCAAATGGGACAAGGGCA

## FIGURE 8 (continued)

AGGCTCTTGAATTTTTGTTAGAATCATTAGGATACGAGAATTCGAATGATGTATTTCCAATATATA
TTGGTGATGATCGAACTGATGAGGATGCTTTTAAGGTTTTGCGCAGTAGGGGTCAAGGAATTGGGA
TTCTTGTTTCTAGAGTTGCAAAAGAAACAGATGCTTCCTATACCTTGCAAGATCCATCAGAGGCAA
GTGCTATATATTCCATCCAGTACAATTTATTCTATATAATATTTTTAATGTTTAATTCGGGCATCA
ATGTTGTATATCTTTATTGTGAATGGTGAATCTGAGAAATATATAATGTAATTAATTAACAAATAT
CTTTATGGCCACATTTACAGGTTGAGCAATTCTTGCGGCGTCTGGTGGAGTGGAAAAGACCGAGTA
CTGTGACTCCCACAAGTGTATAGAGAGTTTGTAGAATGTAGATGATCACTTCAAAGAATTGACACC
ACCACCACCTTAGAATGGTGAAGAGGTGGATCGAATTGTATCACTTTTTTTATTGTTGAAAATGG
AAATAGCACTATTCCATAATTTAAATTTATTAAGGACAAAGTCCGAACAAATAGATTCCTACACAC
GTTTGCATGCGCATGCGGATAGGGAAAGGCAGATGTTTTATGCCGCAGTTGCAAATGGCCCGTCAA
CTTTGTTGCTAAGAATTGTACTTATCGTACATGTGGCCAATATATTCTGAAAAAGATTACTACGAA
AAAAAAAAAAAAA

**SEQ ID NO: 126, protein - Glycine max**

MMTNQNVVTHEVINTLIAVAASISNSTALPSATVPESMAVLGGFWGLPHNKNLVKRLEGAKVSAWI
DSMRASSPTRAKSESQEKRSWILYHPSALNTFEQIVCSAKGKQVVVFLDYDGTLSPIVADPDKAFM
TRKMRATLKGIARHFPTAIVTGRCRDKVYNFVKLAELYYAGSHGMDIKGPTKSQSPKQGNNNKAVL
FQPASQFLPMIDEVYKILLEKTKTVPGANVENNKFCLSVHFRCVDEKSWAALAEKVRLVLNDYPQL
RLTQGRKVLEIRPTIKWDKGKALEFLLESLGYENSNDVFPIYIGDDRTDEDAFKVLRSRGQGIGIL
VSRVAKETDASYTLQDPSEASAIYSIQYNLFYIIFLMFNSGINVVYLYCEW

**SEQ ID NO: 127, DNA - Brassica napus**

TTAACTTCTGCCTCGCCTCTCGTTCTCAACACTTGTGAGAAATATGATGAACCAGAATGTCATCGT
CTCCGACAGAAAAGCCATCTTGGGTTCGAAAACCATCACTGTCTCTAACTCTCCCCTGTTCTCTTC
TCCTCCCACTTACTTTACCTTTCCTCGTCATAAGTTCTTGGAGCTTCTCGAAGCAGCCGATAAAAA
CAGCAACAGCATCAACAAGAACAATCTTGGTGCTGGTAAGATTGCATCTTGGGTCGATTCCATGCG
TGATTCTTCTCCTACACGTCTCAGACCCTCTTCACGTGACTCTGTGTCAGACAACGACCATAAAAC
ATCTTGGATCGTTCGATTTCCATCGGCTTTAAATATGTTTGATGAGATTGTGAATGCTGCAAAAGG
GAAACAAATTGTTATGTTTCTTGATTACGATGGGACGCTCTCTCCCATAGTTGAAGATCCTGACAA
AGCTTACATAACACATGAGATGCGAGAAGTTGTAAAGAATGTGGCTCTAAACTTCCCAACTGCTAT
AGTCACTGGAAGATCCATTGATAAGGTTCGTGGTTTTGTCAAACTCGATGAGATTTACTACGCTGG
AAGCCATGGCATGGACATTGAAGGCCCGACCAGCGAATATGCTTATGGTGGCGAGAGTAATCAAGG
AGTGCTCTTTCAACCTGCTCGTGAATTTGTACCCACGATCGAGAAGGTGTATAAGATATTAGAGGA
AAAGACTAAATGGATCCCTGGGGCTATGGTGGAGAACAACAAGTTTTGTCTGTCCGTACATTTTCG
ACGTGTTGATGAGAAAAGATGGGCCGGATTAGCCGAACAAGTAAAATCAGTTCTAATTGATTATCC
ACAGCTGAAACTAACCCAAGGTAGAAAGGTGCTTGAAATCCGTCCTACGATCAAATGGGACAAGGG
CCAGGCTCTCAATTTTTTGCTAAAATCATTAGGGTTTGAAAAGTCGGAAGATGTTGTTCCTGTGTA
TATTGGAGATGACCTCACCGACGAAGATGCGTTTAAGGTTTTACGTGAGCGGGGACAAGGTTTTGG
GATTCTAGTCTCAAAAGTTCCAAAGGAAACCAATGCCTCTTACTCTCTCCAAGACCCTTCTCAGGT
TAATGAGTTTCTGAGGCGTTTAGTAGAGTGGAAGAGGAAGACAGTCGGGGAAGCTTGAAAAACAAA
CATGCAACATTAATAACACCTGAGTTTATTTTATAAATCTTGAGGAGAAAATAATCGGTATATATA
GACAACCTTCTAAAAAACTAGTACTAGTACTAGATTCGTAGAGGGTGTTTTTTTGGAACTTTACCT
AGAGAGGTGTCTTGGCCAGAAGCATCTTTACTTTTCTACATCGATCGAGAAATTGTAAATTTCGTG
TAACGATTCAGAAAATGAAGAAAACACAACTAATATCATTTCCACGCAAAAAAAAAAAAAAA

**FIGURE 8 (continued)**

**SEQ ID NO: 128, protein - Brassica napus**
MMNQNVIVSDRKAILGSKTITVSNSPLFSSPPTYFTFPRHKFLELLEAADKNSNSINKNNLGAGKI
ASWVDSMRDSSPTRLRPSSRDSVSDNDHKTSWIVRFPSALNMFDEIVNAAKGKQIVMFLDYDGTLS
PIVEDPDKAYITHEMREVVKNVALNFPTAIVTGRSIDKVRGFVKLDEIYYAGSHGMDIEGPTSEYA
YGGESNQGVLFQPAREFVPTIEKVYKILEEKTKWIPGAMVENNKFCLSVHFRRVDEKRWAGLAEQV
KSVLIDYPQLKLTQGRKVLEIRPTIKWDKGQALNFLLKSLGFEKSEDVVPVYIGDDLTDEDAFKVL
RERGQGFGILVSKVPKETNASYSLQDPSQVNEFLRRLVEWKRKTVGEA

**SEQ ID NO: 129, DNA - Brassica napus**
TTAACTTCTGCCTCGCCTCTCGTTCTCAACACTTGTGAGAAATATGATGAACCAGAATGTCATCGT
CTCCGACAGAAAAGCCATCTTGGGTTCGAAAACCATCACTGTCTCTAACTCTCCCCTGTTCTCTTC
TCCTCCCACTTACTTTACCTTTCCTCGTCATAAGTTCTTGGAGCTTCTCGAAGCAGCCGATAAAAA
CAGCAACAGCATCAACAAGAACAATCTTGGTGCTGGTAAGATTGCATCTTGGGTCGATTCCATGCG
TGATTCTTCTCCTACACGTCTCAGACCCTCTTCACGTGACTCTGTGTCAGACAACGACCATAAAAC
ATCTTGGATCGTTCGATTTCCATCGGCTTTAAATATGTTTGATGAGATTGTGAATGCTGCAAAAGG
GAAACAAATTGTTATGTTTCTTGATTACGATGGGACGCTCTCTCCCATAGTTGAAGATCCTGACAA
AGCTTACATAACACATGAGATGCGAGAAGTTGTAAAGAATGTGGCTCTAAACTTCCCAACTGCTAT
AGTCACTGGAAGATCCATTGATAAGGTTCGTGGTTTTGTCAAACTCGATGAGATTTACTACGCTGG
AAGCCATGGCATGGACATTGAAGGCCCGACCAGCGAATATGCTTATGGTGGCGAGAGTAATCAAGG
AGTGCTCTTTCAACCTGCTCGTGAATTTGTACCCACGATCGAGAAGGTGTATAAGATATTAGAGGA
AAAGACTAAATGGATCCCTGGGGCTATGGTGGAGAACAACAAGTTTTGTCTGTCCGTACATTTTCG
ACGTGTTGATGAGAAAAGATGGGCCGGATTAGCCGAACAAGTAAAATCAGTTCTAATTGATTATCC
ACAGCTGAAACTAACCCAAGGTAGAAAGGTGCTTGAAATCCGTCCTACGATCAAATGGGACAAGGG
CCAGGCTCTCAATTTTTTGCTAAAATCATTAGGGTTTGAAAAGTCGGAAGATGTTGTTCCTGTGTA
TATTGGAGATGACCGTACCGACGAAGATGCGTTTAAGGTTTTACGTGAGCGGGGACAAGGTTTTGG
GATTCTAGTCTCAAAAGTTCCAAAGGAAACCAATGCCTCTTACTCTCTCCAAGACCCTTCTCAGGT
TAATGAGTTTCTGAGGCGTTTAGTAGAGTGGAAGAGGAAGACAGTCGGGGAAGCTTGAAAAACAAA
CATGCAACATTAATAACACCTGAGTTTATTTTATAAATCTTGAGGAGAAAATAATCGGTATATATA
GACAACCTTCTAAAAAACTAGTACTAGTACTAGATTCGTAGAGGGTGTTTTTTTGGAACTTTACCT
AGAGAGGTGTCTTGGCCAGAAGCATCTTTACTTTTCTACATCGATCGAGAAATTGTAAATTTCGTG
TAACGATTCAGAAAATGAAGAAAACACAACTAATATCATTTCCACGCAAAAAAAAAAAAAAA

**SEQ ID NO: 130, protein - Brassica napus**
MMNQNVIVSDRKAILGSKTITVSNSPLFSSPPTYFTFPRHKFLELLEAADKNSNSINKNNLGAGKI
ASWVDSMRDSSPTRLRPSSRDSVSDNDHKTSWIVRFPSALNMFDEIVNAAKGKQIVMFLDYDGTLS
PIVEDPDKAYITHEMREVVKNVALNFPTAIVTGRSIDKVRGFVKLDEIYYAGSHGMDIEGPTSEYA
YGGESNQGVLFQPAREFVPTIEKVYKILEEKTKWIPGAMVENNKFCLSVHFRRVDEKRWAGLAEQV
KSVLIDYPQLKLTQGRKVLEIRPTIKWDKGQALNFLLKSLGFEKSEDVVPVYIGDDRTDEDAFKVL
RERGQGFGILVSKVPKETNASYSLQDPSQVNEFLRRLVEWKRKTVGEA

**SEQ ID NO: 131, Oryza sativa HMG promoter**
CATGCGGCTAATGTAGATGCTCACTGCGCTAGTAGTAAGGTACTCCAGTACATTATGGAATATACA
AAGCTGTAATACTCGTATCAGCAAGAGAGAGGCACACAAGTTGTAGCAGTAGCACAGGATTAGAAA
AACGGGACGACAAATAGTAATGGAAAAACAAAAAAAAACAAGGAAACACATGGCAATATAAATGGA
GAAATCACAAGAGGAACAGAATCCGGGCAATACGCTGCGAAAGTACTCGTACGTAAAAAAAAGAGG
CGCATTCATGTGTGGACAGCGTGCAGCAGAAGCAGGGATTTGAAACCACTCAAATCCACCACTGCA
AACCTTCAAACGAGGCCATGGTTTGAAGCATAGAAAGCACAGGTAAGAAGCACAACGCCCTCGCTC
TCCACCCTCCCACCCAATCGCGACGCACCTCGCGGATCGGTGACGTGGCCTCGCCCCCCAAAAATA

**FIGURE 8 (continued)**

TCCCGCGGCGTGAAGCTGACACCCCGGGCCCACCCACCTGTCACGTTGGCACATGTTGGTTATGGT
TCCCGGCCGCACCAAAATATCAACGCGGCGCGGCCCAAAATTTCCAAAATCCCGCCCAAGCCCCTG
GCGCGTGCCGCTCTTCCACCCAGGTCCCTCTCGTAATCCATAATGGCGTGTGTACCCTCGGCTGGT
TGTACGTGGGCGGGTTACCCTGGGGGTGTGGGTGGATGACGGGTGGGCCCGGAGGAGGTCCGGCCC
CGCGCGTCATCGCGGGGCGGGGTGTAGCGGGTGCGAAAAGGAGGCGATCGGTACGAAAATTCAAAT
TAGGAGGTGGGGGGCGGGGCCCTTGGAGAATAAGCGGAATCGCAGATATGCCCCTGACTTGGCTTG
GCTCCTCTTCTTCTTATCCCTTGTCCTCGCAACCCCGCTTCCTTCTCTCCTCTCCTCTTCTCTTCT
CTTCTCTGGTGGTGTGGGTGTGTCCCTGTCTCCCCTCTCCTTCCTCCTCTCCTTTCCCCTCCTCTC
TTCCCCCCTCTCACAAGAGAGAGAGCGCCAGACTCTCCCCAGGTGAGGTGAGACCAGTCTTTTTGC
TCGATTCGACGCGCCTTTCACGCCGCCTCGCGCGGATCTGACCGCTTCCCTCGGCCTTCTCGCAGG
ATTCAGCC

**FIGURE 8 (continued)**

Europäisches Patentamt
European Patent Office
Office européen des brevets

# EUROPEAN SEARCH REPORT

Application Number
EP 12 15 1921

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE Geneseq [Online]<br><br>21 January 2003 (2003-01-21),<br>Harper et al.: "Arabidopsis thaliana stress regulated gene SEQ ID NO",<br>XP002673992,<br>retrieved from EBI<br>Database accession no. ABZ13932<br>* the whole document *<br>& WO 02/16655 A2 (SCRIPPS RESEARCH INST [US]; SYNGENTA PARTICIPATIONS AG [CH]; HARPER JE) 28 February 2002 (2002-02-28)<br>----- | 1-16 | INV.<br>C12N15/82 |
| X | US 2004/229364 A1 (CAIMI PERRY G [US] ET AL) 18 November 2004 (2004-11-18)<br>* abstract *<br>* paragraph [0006] - paragraph [0011] *<br>* paragraph [0017] - paragraph [0018] *<br>* example 5 *<br>* sequence 37 *<br>----- | 1-16 | |
| Y | WO 97/42326 A (MOGEN INT [NL]; GODDIJN OSCAR JOHANNES MARIA [NL]; PEN JAN [NL]; SMEEK) 13 November 1997 (1997-11-13)<br>* abstract *<br>* page 3, line 1 - line 27 *<br>* page 4, line 5 - line 14 *<br>* page 27, line 17 - line 26 *<br>* example 2 *<br>* examples 4,5 *<br>* example 7 *<br>* example 10 *<br>* examples 19-21 *<br>-----<br>-/-- | 1-16 | TECHNICAL FIELDS SEARCHED (IPC)<br><br>C12N |

The present search report has been drawn up for all claims

2

| Place of search | Date of completion of the search | Examiner |
|---|---|---|
| Munich | 17 April 2012 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 1921

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | VOGEL GUIDO ET AL: "Trehalose-6-phosphate phosphatases from Arabidopsis thaliana: Identification by functional complementation of the yeast tps2 mutant", PLANT JOURNAL, vol. 13, no. 5, March 1998 (1998-03), pages 673-683, XP002673948, ISSN: 0960-7412<br>* abstract *<br>* page 673, right-hand column, line 29 - line 34 *<br>* page 678, left-hand column, line 22 - right-hand column, line 2 *<br>* page 680, left-hand column, line 9 - line 14 *<br>----- | 1-16 | |
| | | | TECHNICAL FIELDS SEARCHED (IPC) |

The present search report has been drawn up for all claims

| Place of search | Date of completion of the search | Examiner |
|---|---|---|
| Munich | 17 April 2012 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

**ANNEX TO THE EUROPEAN SEARCH REPORT ON EUROPEAN PATENT APPLICATION NO.** EP 12 15 1921

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2012

| Patent document cited in search report | Publication date | Patent family member(s) | Publication date |
|---|---|---|---|
| US 2004229364 A1 | 18-11-2004 | NONE | |
| WO 9742326 A | 13-11-1997 | AT 301718 T | 15-08-2005 |
| | | AU 718379 B2 | 13-04-2000 |
| | | AU 2898897 A | 26-11-1997 |
| | | AU 4514800 A | 14-09-2000 |
| | | BR 9710959 A | 15-06-2004 |
| | | CA 2253348 A1 | 13-11-1997 |
| | | CN 1221454 A | 30-06-1999 |
| | | CZ 9803450 A3 | 12-01-2000 |
| | | DE 69733945 D1 | 15-09-2005 |
| | | DE 69733945 T2 | 13-04-2006 |
| | | DK 0901527 T3 | 19-12-2005 |
| | | EP 0901527 A2 | 17-03-1999 |
| | | ES 2243997 T3 | 01-12-2005 |
| | | JP 2000510691 A | 22-08-2000 |
| | | KR 20000010758 A | 25-02-2000 |
| | | MA 24248 A1 | 01-04-1998 |
| | | NO 985097 A | 30-12-1998 |
| | | NZ 332677 A | 27-04-2001 |
| | | PL 329913 A1 | 26-04-1999 |
| | | SK 148498 A3 | 16-05-2000 |
| | | TR 9802220 T2 | 22-03-1999 |
| | | TR 9902035 T2 | 21-10-1999 |
| | | TR 9902036 T2 | 21-10-1999 |
| | | TR 9902037 T2 | 23-10-2000 |
| | | US 6833490 B1 | 21-12-2004 |
| | | US 2008138903 A1 | 12-06-2008 |
| | | WO 9742326 A2 | 13-11-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060191040 A, Jackson
- US 5811238 A
- US 6395547 A
- WO 2004065596 A
- US 4962028 A
- WO 0114572 A
- WO 9514098 A
- WO 9412015 A
- US 5565350 A
- WO 0015815 A
- EP 1198985 A1
- US 9303868 W, Zarling
- US 5164310 A
- US 5159135 A


### Non-patent literature cited in the description


- **WANG et al.** *Planta,* 2003, vol. 218, 1-14
- **FASOULA ; TOLLENAAR.** *Maydica,* 2005, vol. 50, 39
- **BLAZQUEZ et al.** *Plant J.,* March 1998, vol. 13 (5), 685-9
- **VOGEL et al.** *Plant J.,* March 1998, vol. 13 (5), 673-83
- **THALLER et al.** *Protein Sci.,* July 1998, vol. 7 (7), 1647-52
- **GODDIJN et al.** *Plant Physiol.,* January 1997, vol. 113 (1), 181-90
- **HOLMSTROM et al.** *Nature,* vol. 379, 683-684
- **ROMERO et al.** *Planta,* vol. 201, 293-297
- **PILONT-SMITS et al.** *J Plant Physiol.,* 1998, vol. 152, 525-532
- **SCHLUEPMANN et al.** *Proc Natl Acad Sci U S A.,* 2003, vol. 100 (11), 6849-54
- **EASTMOND.** *Plant J.,* January 2002, vol. 29 (2), 225-35
- **CREIGHTON.** Proteins. W.H. Freeman and Company, 1984
- **TERPE.** *Appl. Microbiol. Biotechnol.,* 2003, vol. 60, 523-533
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAIPress, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics,* 10 July 2003, vol. 4, 29
- **VAN DIJCK et al.** *Biochem J.,* 15 August 2002, vol. 366, 63-71
- **ZENTELLA et al.** *Plant Physiol.,* April 1999, vol. 119 (4), 1473-82
- **AVONCE et al.** *Plant Physiol.,* November 2004, vol. 136 (3), 3649-59
- **KLUTTS et al.** *J Biol Chem.,* 24 January 2003, vol. 278 (4), 2093-100
- **LUNN et al.** *Biochem J.,* 01 July 2006, vol. 397 (1), 139-48
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- Current Protocols in Molecular Biology. Wiley

- **GATZ.** *Annu. Rev. Plant Physiol. Plant Mol. Biol.,* 1997, vol. 48, 89-108
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **HEID et al.** *Genome Methods,* 1996, vol. 6, 986-994
- **BUCHMAN ; BERG.** *Mol. Cell Biol.,* 1988, vol. 8, 4395-4405
- **CALLIS et al.** *Genes Dev.,* 1987, vol. 1, 1183-1200
- The Maize Handbook. Springer, 1994
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT, AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet,* 1980, vol. 32, 314-331

- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet,* 1997, vol. 7, 22-28
- **DEAR ; COOK.** *Nucleic Acid Res.,* 1989, vol. 17, 6795-6807
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **CAMPANELLA JJ ; BITINCKA L ; SMALLEY J.** MatGAT: an application that generates similarity/identity matrices using protein or DNA sequences. *BMC Bioinformatics,* 2003, vol. 4, 29
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfax. BIOS Scientific Publications Ltd (UK, 1993
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158